# EUROPEAN PATENT APPLICATION

(11) **EP 4 644 377 A1**
(43) Date of publication of application: **05.11.2025**
(21) Application number: 24736940.8
(22) Date of filing: 02.01.2024
(51) Int. Cl.: C07D 295/13, C07C 237/22, C07D 207/09, C07C 323/52, C07D 307/14, A61K 9/127, A61K 9/14, A61K 45/00, A61P 3/00

(54) **LIPID COMPOUND FOR GENE DELIVERY AND LIPID NANOPARTICLE COMPRISING SAME**

(30) Priority: 30.12.2022 CN 202211727565; 30.06.2023 CN 202310803747
(71) Applicant: NANJING CYBERNAX BIOMEDICAL TECHNOLOGY CO., LTD., Nanjing, Jiangsu 211112 (CN)
(72) Inventor: YUAN, Zhefan, Nanjing, Jiangsu 211112 (CN); CHEN, Bo, Nanjing, Jiangsu 211112 (CN); QIAN, Sunxiang, Nanjing, Jiangsu 211112 (CN); LI, Qingfeng, Nanjing, Jiangsu 211112 (CN); JIA, Kan, Nanjing, Jiangsu 211112 (CN)
(74) Representative: Schüssler, Andrea
(86) International application number: PCT/CN2024/070147
(87) International publication number: WO 2024/141105

(57) **Abstract**

Disclosed herein are a lipid compound comprising an isonitrile or a carboxylic acid terminal group, and a preparation method therefor and the use thereof in the preparation of a lipid compound that can be used for gene delivery. Also disclosed herein are a lipid compound that can be used for gene delivery, and a preparation method therefor and the use thereof in gene delivery. Further disclosed herein are a liposome and a lipid nanoparticle comprising the lipid compound that can be used for gene delivery, and a gene delivery composition comprising the lipid compound, the liposome, or the lipid nanoparticle. The lipid compound, the liposome, the lipid nanoparticle and the gene delivery composition herein that can be used for gene delivery enable efficient compounding, protection, and intracellular and targeted delivery and release of biomolecules, such as nucleic acids in tissues and organs *in vitro* and *in vivo.*

## Description

### Cross-Reference to Related Application

This application claims the priority to Chinese Patent Application No. 202211727565.0, filed on December 30, 2022, and Chinese Patent Application No. 202310803747.X, filed on June 30, 2023, the entire contents of which are incorporated herein by reference.

### Technical Field

The present disclosure relates to the field of gene loading and delivery, and particularly to a lipid compound for gene delivery, a preparation method therefor, and use thereof in gene delivery. The present disclosure further relates to a liposome and a lipid nanoparticle comprising the lipid compound, and to a gene delivery composition comprising the lipid compound, the liposome, or the lipid nanoparticle.

### Background

At present, a wide variety of nucleic acids are being developed as therapeutic agents for the treatment of various diseases. These nucleic acids include DNA in gene therapy, plasmid-based interfering nucleic acids, and small interfering nucleic acids for RNA interference (RNAi), including siRNA, miRNA, antisense molecules, ribozymes, and aptamers.

Effective delivery of nucleic acid drugs requires the delivery of therapeutic nucleic acid molecules into target cells. *In vivo,* naked nucleic acid molecules are degraded and cleared by a large number of nucleases. Moreover, due to their inherent negative charge, nucleic acid molecules are difficult to directly penetrate a negatively charged cell membrane. Therefore, specialized delivery systems are required to protect the nucleic acid molecules and facilitate their entry into the target cells. Lipid nanoparticles are currently the most effective delivery vehicles for nucleic acid drugs, and have been verified in various marketed drugs, including COVID-19 mRNA vaccines. A classical lipid nanoparticle is composed of four types of lipids encapsulating the nucleic acid molecules, such as an ionizable lipid, cholesterol, a neutral phospholipid, and a polyethylene glycol (PEG) lipid. Among these, the ionizable lipid determines the *in vitro* and *in vivo* nucleic acid molecule delivery efficiency of the lipid nanoparticle. The delivery of the therapeutic nucleic acid molecules to the target is important for the therapeutic effects, which is often hindered by a limited ability of the ionizable lipid to reach targeted cells and tissues. The expansion and optimization of the ionizable lipid structure is crucial for entry into the targeted tissues and cells.

One of the fundamental challenges that medical practitioners are currently facing is that a wide variety of nucleic acids are being developed as therapeutic agents for the treatment of various diseases. These nucleic acids include DNA in gene therapy, plasmid-based small interfering nucleic acids (iNA) for RNA interference (RNAi), antisense molecules, ribozymes, antagomirs, microRNAs, aptamers, etc. With the development of these nucleic acids, there is a widespread need to produce a lipid formulation that is easy to prepare and can be easily delivered to target tissues. People are looking forward to developing a novel ionizable lipid to meet the needs for effective delivery of the nucleic acid drugs in gene therapy.

Isonitrile is one of the most unusual, fascinating, and unique functional groups in organic chemistry. Its high reactivity enables its carbon atoms to participate in many organic reactions. Specifically, the isonitrile can act as a nucleophile attacking activated electrophiles, as an electrophile being attacked by different nucleophiles, as a carbene participating in common [4+1] cycloadditions, and as a radical acceptor forming an imino radical reaction intermediate. Finally, the lone pair electrons on a terminal carbon atom explain its strong metal-coordination characteristics, enabling the preparation of countless coordination complexes. However, the current researches mainly focus on short-chain and aromatic isonitriles, while research on lipid-based isonitriles comprising biodegradable groups remains nearly nonexistent.

### Summary

To solve the above-mentioned problems, an objective of the present disclosure is to provide various lipid compounds, preparation methods therefor, and use thereof in gene delivery. Another objective of the present disclosure is to provide a novel class of lipid compounds comprising isonitrile or carboxylic acid terminal groups, preparation methods therefor, and use thereof.

Daniel Anderson's research group at MIT (Nature Biotechnology, 2019) reported the use of a Ugi three-component reaction to prepare an ionizable lipid with symmetrical hydrophobic long chains. However, the structural diversity of this lipid is relatively limited due to the restricted number of components and the symmetry. Moreover, a rigid cyclic linker also affects the freedom of a lipid during fusion with a target cell membrane, thereby limiting its ability to fuse with the cell membrane to a certain extent. According to the technical solution of the present disclosure, a series of ionizable lipid compounds with diverse topological structures can be produced at high throughput based on a classical Ugi four-component reaction and its variants. The ionizable lipid compound produced in the present disclosure features asymmetric hydrophobic long chains and enhanced molecular flexibility, enabling improved binding with the target cell membrane, and thereby achieving efficient intracellular delivery of nucleic acids to different target cells. Lipid nanoparticles (LNPs) prepared from these lipid compounds exhibit varying particle sizes and cell-type-specific preferences in nucleic acid molecule delivery efficiency.

According to one aspect of the present disclosure, there is provided a lipid compound of formula (I), or an N-oxide, stereoisomer, or pharmaceutically acceptable salt thereof, 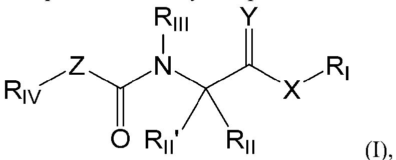 wherein X is O or NH; Y is O or S; Z is absent, or Z is O, NH, or S;
R_{II}' is hydrogen, or substituted or unsubstituted hydrocarbyl, substituted or unsubstituted heterohydrocarbyl, substituted or unsubstituted aryl, or substituted or unsubstituted heteroaryl having 1 to 11 carbon atoms; and
R_{I}, R_{II}, R_{III}, and R_{IV} are each independently selected from a group (A) comprising a group containing at least one ionizable tertiary amine structure, a group (B) comprising a substituted or unsubstituted aliphatic or substituted or unsubstituted heteroaliphatic group having 11 to 30 carbon atoms, and a group (C) comprising a substituted or unsubstituted hydrocarbyl, substituted or unsubstituted heterohydrocarbyl, substituted or unsubstituted aryl, or substituted or unsubstituted heteroaryl group having fewer than 11 carbon atoms,
provided that:
   (1) when any one of R_{I}, R_{II}, R_{III}, and R_{IV} is a group containing at least one ionizable tertiary amine structure, any two of the remaining three are identical or different substituted or unsubstituted hydrocarbyl, substituted or unsubstituted heterohydrocarbyl, substituted or unsubstituted aryl, or substituted or unsubstituted heteroaryl groups having fewer than 11 carbon atoms, and the other is a substituted or unsubstituted aliphatic or substituted or unsubstituted heteroaliphatic group having 11 to 30 carbon atoms; or any two of the remaining three are identical or different substituted or unsubstituted aliphatic or substituted or unsubstituted heteroaliphatic groups having 11 to 30 carbon atoms, and the other is a substituted or unsubstituted hydrocarbyl, substituted or unsubstituted heterohydrocarbyl, substituted or unsubstituted aryl, or substituted or unsubstituted heteroaryl group having fewer than 11 carbon atoms;
      or
   (2) when any two of R_{I}, R_{II}, R_{III}, and R_{IV} are identical or different groups containing at least one ionizable tertiary amine structure, one of the remaining two is a substituted or unsubstituted hydrocarbyl, substituted or unsubstituted heterohydrocarbyl, substituted or unsubstituted aryl, or substituted or unsubstituted heteroaryl group having fewer than 11 carbon atoms, and the other is a substituted or unsubstituted aliphatic or substituted or unsubstituted heteroaliphatic group having 11 to 30 carbon atoms.

In some embodiments, only one of R_{I}, R_{II}, R_{III}, and R_{IV} is a group containing at least one ionizable tertiary amine structure.

In some embodiments, the group A comprises a group containing at least one ionizable tertiary amine structure, wherein the group containing at least one ionizable tertiary amine structure has 3 to 11 carbon atoms and is represented by the following general formula, 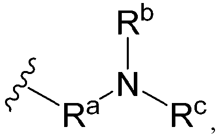 wherein R^{a} is substituted or unsubstituted C₁-C₆ alkylene; R^{b} and R^{c} are each independently substituted or unsubstituted C₁-C₆ alkyl, substituted or unsubstituted C₂-C₆ alkenyl, or substituted or unsubstituted C₂-C₆ alkynyl, which are optionally substituted with 1, 2, or 3 substituents selected from -OH, -SH, -NR^{d}R^{d}', or phenyl, wherein R^{d} and R^{d}' are each independently hydrogen or C₁-C₃ alkyl; or R^{b} and R^{c}, together with the N atom to which they are attached, form a 5- to 12-membered heterocyclic ring or heteroaromatic ring, the 5- to 12-membered heterocyclic ring or heteroaromatic ring containing 1, 2, or 3 heteroatoms independently selected from N, O, and S, wherein at least one heteroatom is N, and the 5- to 12-membered heterocyclic ring or heteroaromatic ring is optionally substituted with one or more C₁-C₆ alkyls or oxo (=O); or R^{a} and R^{b}, together with the N atom to which they are attached, form a 5- to 12-membered heterocyclic ring or heteroaromatic ring optionally substituted with C₁-C₆ alkylene, the 5- to 12-membered heterocyclic ring or heteroaromatic ring optionally substituted with C₁-C₆ alkylene containing 1, 2, or 3 heteroatoms independently selected from N, O, and S, wherein at least one heteroatom is N, and the 5- to 12-membered heterocyclic ring or heteroaromatic ring optionally substituted with C₁-C₆ alkylene is optionally substituted with one or more C₁-C₆ alkyls or oxo (=O); or R^{a}, R^{b}, and R^{c}, together with the N atom to which they are attached, form a 5- to 12-membered heterocyclic ring or heteroaromatic ring optionally substituted with C₁-C₆ alkylene, the 5- to 12-membered heterocyclic ring or heteroaromatic ring optionally substituted with C₁-C₆ alkylene containing 1, 2, or 3 heteroatoms independently selected from N, O, and S, wherein at least one heteroatom is N, and the 5- to 12-membered heterocyclic ring or heteroaromatic ring optionally substituted with C₁-C₆ alkylene is optionally substituted with one or more C₁-C₆ alkyls or oxo (=O).

In some embodiments, the group B comprises a substituted or unsubstituted aliphatic or substituted or unsubstituted heteroaliphatic group having 11 to 30 carbon atoms, wherein the substituted or unsubstituted aliphatic or substituted or unsubstituted heteroaliphatic group having 11 to 30 carbon atoms optionally contains 1, 2, 3, 4, 5, 6, 7, 8, 9, 10, 11, or 12 groups independently selected from -C=C-, -C≡C-, 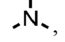 , -NH-, -NH₂, -OH, -OR^{m}, -O-, -C(O)-, -C(ORⁿ)-, -C(O)O-, -SH, -SR^{o}, -S-, -C(S)-, -C(SR^{p})-, -C(S)O-, and -P(O)-, wherein R^{m}, Rⁿ, R^{o}, and R^{p} are each independently substituted or unsubstituted C₁-C₁₄ aliphatic hydrocarbyl.

In some embodiments, the group C comprises a substituted or unsubstituted hydrocarbyl, substituted or unsubstituted heterohydrocarbyl, substituted or unsubstituted aryl, or substituted or unsubstituted heteroaryl group having 1 to 11 carbon atoms, wherein the substituted or unsubstituted hydrocarbyl, substituted or unsubstituted heterohydrocarbyl, substituted or unsubstituted aryl, or substituted or unsubstituted heteroaryl group having 1 to 11 carbon atoms optionally contains 1, 2, 3, 4, 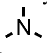 or 5 groups independently selected from deuterium, halogen, -C=C-, -C=C-, , -NH-, -NH₂, -OH, -OR^{m}', -O-, -C(O)-, -C(ORⁿ')-, -C(O)O-, -SH, -SR^{o}', -S-, -C(S)-, -C(SR^{p}'), -C(S)O-, and -P(O)-, wherein R^{m}', Rⁿ', R^{o}', and R^{p}' are each independently substituted or unsubstituted C₁-C₁₄ aliphatic hydrocarbyl; and the hydrocarbyl, heterohydrocarbyl, aryl, or heteroaryl group is optionally substituted with one or more substituents selected from the following group: deuterium, halogen, -NO₂, and -OH.

In some embodiments, R_{II}' is hydrogen, or is selected from the group C.

In some embodiments, R_{I}, R_{II}, R_{III}, and R_{IV} satisfy the following conditions:
R_{I} is selected from the group A, R_{II} and R_{III} are selected from the group B, and R_{IV} is selected from the group C; or
R_{I} is selected from the group A, R_{II} and R_{IV} are selected from the group B, and R_{III} is selected from the group C; or
R_{I} is selected from the group A, R_{III} and R_{IV} are selected from the group B, and R_{II} is selected from the group C; or
R_{II} is selected from the group A, R_{I} and R_{III} are selected from the group B, and R_{IV} is selected from the group C; or
R_{II} is selected from the group A, R_{I} and R_{IV} are selected from the group B, and R_{III} is selected from the group C; or
R_{II} is selected from the group A, R_{III} and R_{IV} are selected from the group B, and R_{I} is selected from the group C; or
R_{III} is selected from the group A, R_{I} and R_{II} are selected from the group B, and R_{IV} is selected from the group C; or
R_{III} is selected from the group A, R_{I} and R_{IV} are selected from the group B, and R_{II} is selected from the group C; or
R_{III} is selected from the group A, R_{II} and R_{IV} are selected from the group B, and R_{I} is selected from the group C; or
R_{IV} is selected from the group A, R_{I} and R_{II} are selected from the group B, and R_{III} is selected from the group C; or
R_{IV} is selected from the group A, R_{I} and R_{III} are selected from the group B, and R_{II} is selected from the group C; or
R_{IV} is selected from the group A, R_{II} and R_{III} are selected from the group B, and R_{I} is selected from the group C.

In some embodiments, R_{I}, R_{II}, R_{III}, and R_{IV} each independently optionally comprise at least one degradable group.

In some embodiments, when R_{I}, R_{II}, R_{III}, and R_{IV} are each independently selected from the group B, each optionally comprises at least one degradable group.

In some embodiments, the degradable group is selected from -C(O)O-, -OC(O)-, -OC(O)O-, -S-S-, -C(O)NH-, -NHC(O)-, -NHC(O)O-, -NR¹C(O)-, -C(O)NR²-, -NR³C(O)O-, -OP(O)OR⁴O-, -OCR⁵(OR⁶)O-, -CR⁷(OR⁸)O-, and -CH(OR⁹)O-, wherein R¹, R ², R³, R⁴, R⁵, R⁶, R⁷, R⁸, and R⁹ are each independently substituted or unsubstituted C₁-C₁₄ aliphatic hydrocarbyl.

In some embodiments,
when R_{III} and R_{IV} are selected from the group B, one of R_{III} and R_{IV} has 16 to 30 carbon atoms, and the other has 11 to 25 carbon atoms;
when R_{II} and R_{IV} are selected from the group B, one of R_{II} and R_{IV} has 16 to 30 carbon atoms, and the other has 11 to 25 carbon atoms;
when R_{II} and R_{III} are selected from the group B, one of R_{II} and R_{III} has 16 to 30 carbon atoms, and the other has 11 to 25 carbon atoms;
when R_{I} and R_{IV} are selected from the group B, one of R_{I} and R_{IV} has 16 to 30 carbon atoms, and the other has 11 to 25 carbon atoms;
when R_{I} and R_{II} are selected from the group B, one of R_{I} and R_{II} has 16 to 30 carbon atoms, and the other has 11 to 25 carbon atoms; and/or
when R_{I} and R_{III} are selected from the group B, one of R_{I} and R_{III} has 16 to 30 carbon atoms, and the other has 11 to 25 carbon atoms.

In some embodiments,
when R_{III} and R_{IV} are selected from the group B, one of R_{III} and R_{IV} has 16 to 25 carbon atoms, and the other has 11 to 16 carbon atoms;
when R_{II} and R_{IV} are selected from the group B, one of R_{II} and R_{IV} has 16 to 25 carbon atoms, and the other has 11 to 16 carbon atoms;
when R_{II} and R_{III} are selected from the group B, one of R_{II} and R_{III} has 16 to 25 carbon atoms, and the other has 11 to 16 carbon atoms;
when R_{I} and R_{IV} are selected from the group B, one of R_{I} and R_{IV} has 16 to 25 carbon atoms, and the other has 11 to 16 carbon atoms;
when R_{I} and R_{II} are selected from the group B, one of R_{I} and R_{II} has 16 to 25 carbon atoms, and the other has 11 to 16 carbon atoms; and/or
when R_{I} and R_{III} are selected from the group B, one of R_{I} and R_{III} has 16 to 25 carbon atoms, and the other has 11 to 16 carbon atoms.

In some embodiments,
when R_{III} and R_{IV} are selected from the group B, at least one of R_{III} and R_{IV} comprises at least one degradable group;
when R_{II} and R_{IV} are selected from the group B, at least one of R_{II} and R_{IV} comprises at least one degradable group;
when R_{II} and R_{III} are selected from the group B, at least one of R_{II} and R_{III} comprises at least one degradable group;
when R_{I} and R_{IV} are selected from the group B, at least one of R_{I} and R_{IV} comprises at least one degradable group;
when R_{I} and R_{II} are selected from the group B, at least one of R_{I} and R_{II} comprises at least one degradable group; and/or
when R_{I} and R_{III} are selected from the group B, at least one of R_{I} and R_{III} comprises at least one degradable group.

In some embodiments,
when R_{III} and R_{IV} are selected from the group B, only one of R_{III} and R_{IV} comprises one degradable group;
when R_{II} and R_{IV} are selected from the group B, only one of R_{II} and R_{IV} comprises at least one degradable group;
when R_{II} and R_{III} are selected from the group B, only one of R_{II} and R_{III} comprises at least one degradable group;
when R_{I} and R_{IV} are selected from the group B, only one of R_{I} and R_{IV} comprises one degradable group;
when R_{I} and R_{II} are selected from the group B, only one of R_{I} and R_{II} comprises one degradable group; and/or
when R_{I} and R_{III} are selected from the group B, only one of R_{I} and R_{III} comprises one degradable group.

In some embodiments, there is provided a lipid compound of formula (I_{A}), or an N-oxide, stereoisomer, or pharmaceutically acceptable salt thereof, 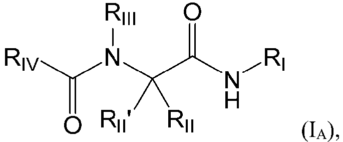 wherein R_{II}', R_{I}, R_{II}, R_{III}, and R_{IV} are as defined in the present disclosure.

The group A, the group B, and the group C are as defined in the present disclosure.

In some embodiments, R_{II}' is hydrogen, or substituted or unsubstituted hydrocarbyl, substituted or unsubstituted heterohydrocarbyl, substituted or unsubstituted aryl, or substituted or unsubstituted heteroaryl having 1 to 11 carbon atoms. For example, R_{II}' is hydrogen or is selected from the group C, such as hydrocarbyl having 1 to 8, 1 to 6, or 1 to 4 carbon atoms, specifically, for example, C₁-C₆ alkyl and C₁-C₄ alkyl, including but not limited to methyl, ethyl, propyl, isopropyl, n-butyl, sec-butyl, tert-butyl, pentyl, hexyl, etc.

In some embodiments, R_{III} is selected from the group A, R_{I} and R_{IV} are selected from the group B, and R_{II} is selected from the group C.

In some embodiments, R_{III} is selected from the group A, R_{I} and R_{IV} are selected from the group B, R_{II} is selected from the group C, and R_{II}' is hydrogen or is selected from the group C.

In some embodiments, there is provided a lipid compound of formula (I_{A-1}), or an N-oxide, stereoisomer, or pharmaceutically acceptable salt thereof, 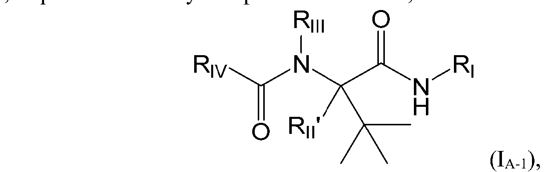 wherein R_{II}', R_{I}, R_{III}, and R_{IV} are as defined in the present disclosure.

The group A, the group B, and the group C are as defined in the present disclosure.

In some embodiments, R_{II}' is hydrogen, or substituted or unsubstituted hydrocarbyl, substituted or unsubstituted heterohydrocarbyl, substituted or unsubstituted aryl, or substituted or unsubstituted heteroaryl having 1 to 11 carbon atoms. For example, R_{II}' is hydrogen or is selected from the group C, such as hydrocarbyl having 1 to 8, 1 to 6, or 1 to 4 carbon atoms, specifically, for example, C₁-C₆ alkyl and C₁-C₄ alkyl, including but not limited to methyl, ethyl, propyl, isopropyl, n-butyl, sec-butyl, tert-butyl, pentyl, hexyl, etc.

In some embodiments, R_{III} is selected from the group A, and R_{I} and R_{IV} are selected from the group B.

In some embodiments, R_{III} is selected from the group A, R_{I} and R_{IV} are selected from the group B, and R_{II}' is hydrogen or is selected from the group C.

In some embodiments, there is provided a lipid compound of formula (I_{A-2}), or an N-oxide, stereoisomer, or pharmaceutically acceptable salt thereof, 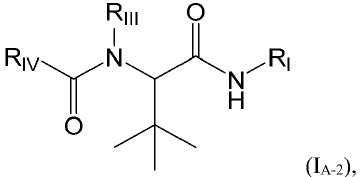 wherein, R_{I}, R_{III}, and R_{IV} are as defined in the present disclosure.

The group A, the group B, and the group C are as defined in the present disclosure.

In some embodiments, R_{III} is selected from the group A, and R_{I} and R_{IV} are selected from the group B.

In some embodiments, there is provided a lipid compound of formula (I_{B}), or an N-oxide, stereoisomer, or pharmaceutically acceptable salt thereof, 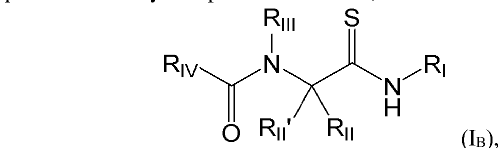 wherein R_{II}', R_{I}, R_{II}, R_{III}, and R_{IV} are as defined in the present disclosure.

The group A, the group B, and the group C are as defined in the present disclosure.

In some embodiments, R_{II}' is hydrogen, or substituted or unsubstituted hydrocarbyl, substituted or unsubstituted heterohydrocarbyl, substituted or unsubstituted aryl, or substituted or unsubstituted heteroaryl having 1 to 11 carbon atoms. For example, R_{II}' is hydrogen or is selected from the group C, such as hydrocarbyl having 1 to 8, 1 to 6, or 1 to 4 carbon atoms, specifically, for example, C₁-C₆ alkyl and C₁-C₄ alkyl, including but not limited to methyl, ethyl, propyl, isopropyl, n-butyl, sec-butyl, tert-butyl, pentyl, hexyl, etc.

In some embodiments, R_{III} is selected from the group A, R_{I} and R_{IV} are selected from the group B, and R_{II} is selected from the group C.

In some embodiments, R_{III} is selected from the group A, R_{I} and R_{IV} are selected from the group B, R_{II} is selected from the group C, and R_{II}' is hydrogen or is selected from the group C.

According to another aspect of the present disclosure, there is provided a method for preparing a lipid compound of formula (I) according to the present disclosure, the method comprising the following steps: 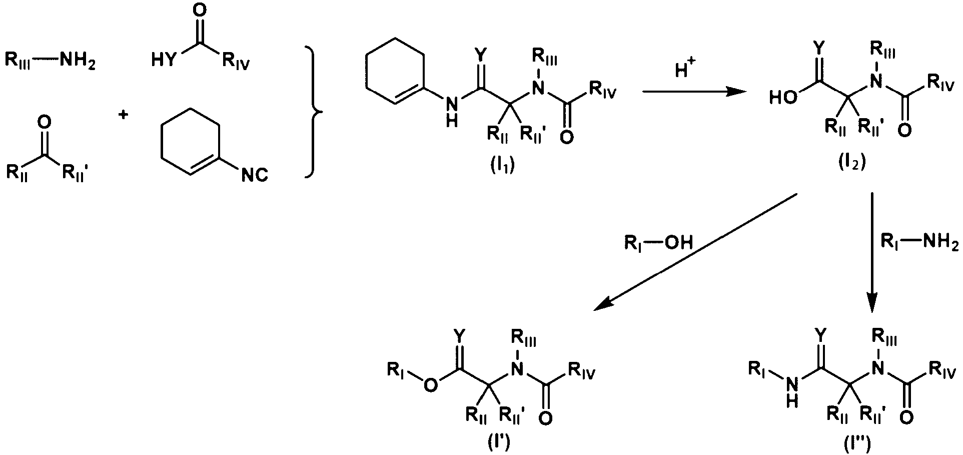
(i) dissolving a first reactant (R_{III}-NH₂) and a second reactant (R_{II}-C(O)-R_{II}') in ethanol before reacting, to form a first mixture;
(ii) adding a third reactant (HY-C(O)-R_{IV}) to the first mixture obtained in step (i) before reacting, to form a second mixture;
(iii) adding 1-isocyanato-1-cyclohexene to the second mixture obtained in step (ii) before reacting and purifying, to obtain a compound of formula (I₁);
(iv) removing a cycloalkenyl amide group from the product (compound of formula I₁) obtained in the above step under acidic conditions, to obtain a compound of formula (I₂), which is an acid; and
(v) mixing the product (compound of formula (I₂)) obtained in the above step with a fourth reactant (R_{I}-OH or R_{I}-NH₂), followed by a dehydration condensation reaction, and then purifying to obtain a lipid compound of formula (I') or (I").

The first three steps of the reaction constitute a classical Ugi four-component reaction using a cycloalkenyl isonitrile compound as a fixed component. A variable R_{I} group can be conveniently introduced by selective removal of a cycloalkenyl amide structure, and the type of the X group in the structure of formula (I) can be modified by selecting the structure of the fourth reactant.

According to an alternative embodiment of the present disclosure, the third reactant is replaced with a combination of carbon dioxide and an alcohol. The lipid compound prepared by the method has the following general structural formula (I₃), in which -O-R_{IV} is incorporated into an amide structure to form a carbamate structure.

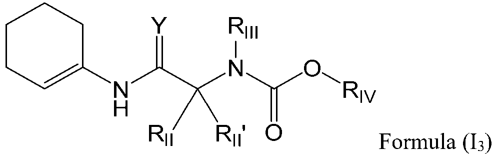

According to an alternative embodiment of the present disclosure, the above-mentioned alternative solution can be further replaced by a combination of the following steps: (I) replacing the third reactant with carbon dioxide and tert-butanol to generate a t-butyloxycarbonyl (BOC)-protected amine structure; (II) simultaneously removing a BOC protecting group and the cycloalkenyl amide group under acidic conditions to obtain an intermediate with an amino acid-like structure; and (iii) further introducing an amide comprising an R_{IV} group into the secondary amine of the intermediate to prepare a target lipid compound, and modifying the type of the X group in the structure of formula (I) by selecting the structure of the fourth reactant. Compared with the classical Ugi four-component reaction, the above-mentioned alternative solution prepares a modular lipid precursor that enables convenient introduction of linker groups such as -O-, -S-, and -NH- linked to carbonyl at an R_{IV} group of formula (I), thereby allowing further modulation of the biological degradability of the lipid compound of formula (I) at the R_{IV} group. This feature is difficult to achieve using the classical Ugi four-component reaction employing carboxylic acid as the third reactant.

According to another aspect of the present disclosure, there is provided a method for preparing a lipid compound of formula (I) according to the present disclosure, the method comprising the following steps: 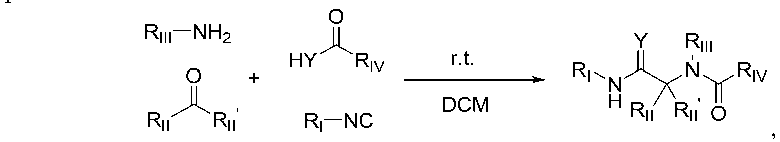 mixing a first reactant (R_{I}-NC), a second reactant (R_{II}-C(O)-R_{II}'), a third reactant (R_{III}-NH₂), and a fourth reactant (HY-C(O)-R_{IV}), and then subjecting the mixture to a one-step reaction at room temperature to obtain a lipid compound of formula (I").

According to another aspect of the present disclosure, there is provided use of a lipid compound of formula (I) according to the present disclosure, or an N-oxide, stereoisomer, or pharmaceutically acceptable salt thereof in preparation of a liposome and/or lipid nanoparticle.

According to another aspect of the present disclosure, there is provided a liposome. The liposome of the present disclosure comprises the lipid compound of formula (I), or the N-oxide, stereoisomer, or pharmaceutically acceptable salt thereof.

In some embodiments, the liposome of the present disclosure further comprises a phospholipid and cholesterol.

According to another aspect of the present disclosure, there is provided a lipid nanoparticle. The lipid nanoparticle of the present disclosure comprises the lipid compound of formula (I), or the N-oxide, stereoisomer, or pharmaceutically acceptable salt thereof.

In some embodiments, the lipid nanoparticle of the present disclosure further comprises a phospholipid, a structural lipid, a polyethylene glycol (PEG) lipid, and optionally, a nucleic acid.

The delivery efficiency of the nucleic acid in the present disclosure refers to, for example, increased accumulation of nucleic acids, higher transcription of nucleic acids (e.g., for DNA delivery), enhanced translation of target proteins (e.g., for mRNA delivery), or more efficient inhibition of target mRNA transcription and translation (e.g., for miRNA delivery) and the like in target cells, tissues, or organs. According to some embodiments of the present disclosure, the lipid nanoparticle exhibits higher delivery efficiency compared with direct delivery of the nucleic acid without using a delivery vehicle. In some embodiments, the delivery efficiency is reflected by the amount of protein expressed from the delivered mRNA in target cells or in an animal body.

According to another aspect of the present disclosure, there is provided a pharmaceutical composition comprising a lipid compound of formula (I) according to the present disclosure and a pharmaceutically acceptable carrier or excipients. In some embodiments, the pharmaceutical composition of the present disclosure comprises a liposome comprising the lipid compound of formula (I) and a pharmaceutically acceptable carrier or excipients. In some embodiments, the pharmaceutical composition of the present disclosure comprises a lipid nanoparticle comprising the lipid compound of formula (I) and a pharmaceutically acceptable carrier or excipients.

Another objective of the present disclosure is to provide a lipid compound comprising an isonitrile or carboxylic acid terminal group, a preparation method therefor, and use thereof in preparation of a lipid compound for gene delivery. The lipid compound comprising the isonitrile or carboxylic acid terminal group of the present disclosure can be synthesized into an ionizable lipid compound that is more degradable and more efficient for nucleic acid (DNA, RNA, etc.) or plasmid delivery via Ugi, Passerini, and other isonitrile-based specific multicomponent reactions.

Compared with the prior art, the lipid compound comprising the isonitrile or carboxylic acid terminal group of the present disclosure can be used to prepare an ionizable lipid-NCL via a one-step isonitrile-based specific multicomponent reaction. The resulting ionizable lipid-NCL can be used together with other existing lipid molecules to formulate lipid nanoparticles (LNPs) encapsulating nucleic acids or plasmids, thereby achieving efficient RNA delivery. Due to the presence of ester bonds inherently introduced by the lipid isonitrile, the ionizable lipid-NCL is more readily biodegradable *in vivo* and exhibits significantly lower toxicity compared with that of the traditional amide bond. The method of the present disclosure features a simple synthetic technology, low-cost raw materials, and suitability for large-scale production.

According to another aspect of the present disclosure, there is provided a lipid compound of formula (II), or a stereoisomer or pharmaceutically acceptable salt thereof, 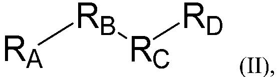
wherein R_{A} is selected from a substituted or unsubstituted aliphatic and heteroaliphatic group, and R_{A} is optionally substituted with one or more substituents selected from the following group: deuterium, halogen, -NO₂, and -OH;
R_{B} is a degradable group comprising a degradable bond selected from the following group consisting of 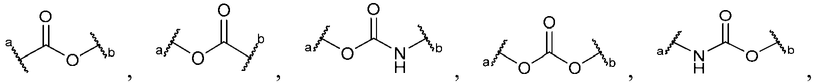 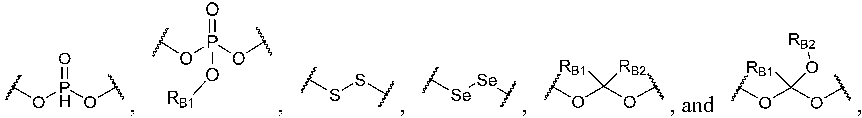 wherein a represents a position linked to R_{A}; b represents a position linked to R_{C}; and R_{B1} and R_{B2} are each independently selected from H and substituted or unsubstituted C₁₋₆ alkyl, and the alkyl is optionally substituted with one or more substituents selected from the following group: deuterium, halogen, -NO₂, and -OH;
R_{C} is selected from a substituted or unsubstituted aliphatic and heteroaliphatic group, and R_{C} is optionally substituted with one or more substituents selected from the following group: deuterium, halogen, -NO₂, and -OH; and
R_{D} is selected from -NC or -COOH.

In some embodiments, the R_{A} group of the lipid compound of formula (II) of the present disclosure comprises a branching center carbon atom; further, the branching center carbon atom is located at the α-, β-, or γ-position of the R_{B} group.

According to another aspect of the present disclosure, there is provided a preparation method for a lipid compound of formula (II) according to the present disclosure, the preparation method comprising the following synthesis route:
when R_{B} is selected from 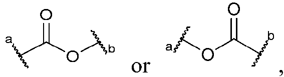 and R_{D} is selected from -NC, 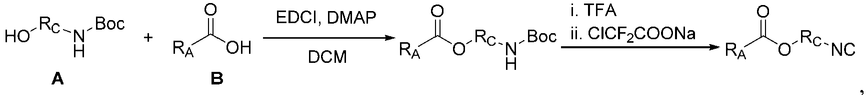 or 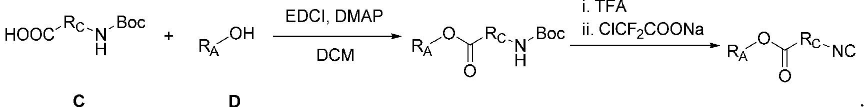 or 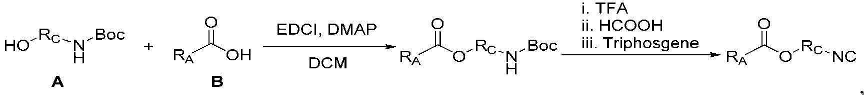 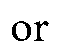 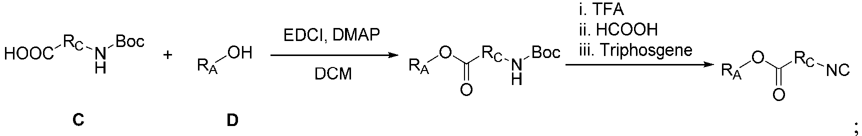 or 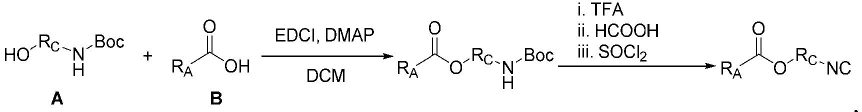 or 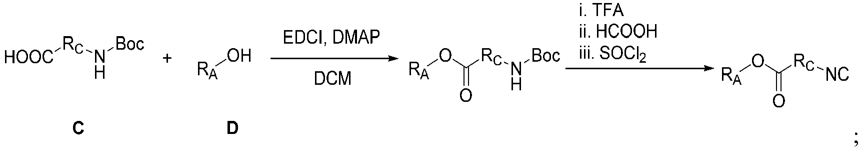 or 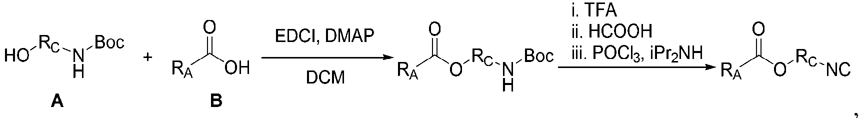 or 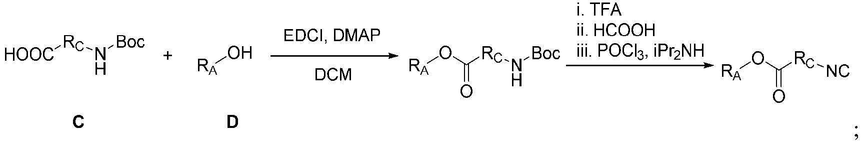 or 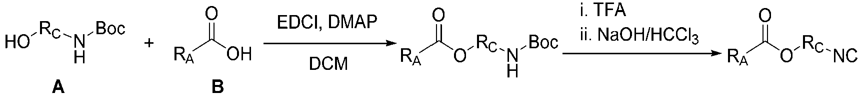 or 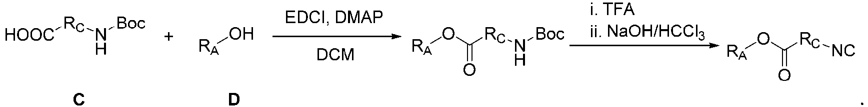 or 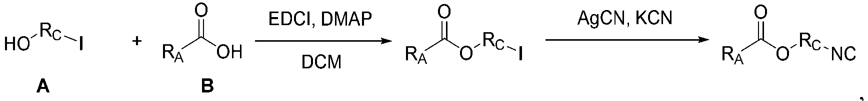 or 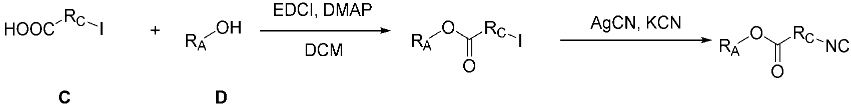 ; and
when R_{B} is selected from 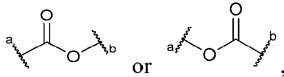 and R_{D} is selected from -COOH, 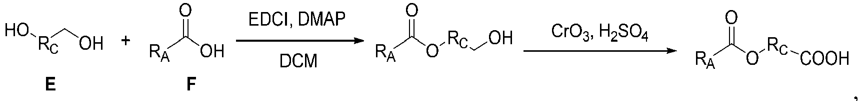 or 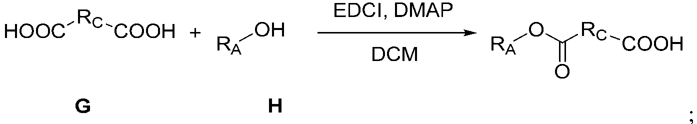 or 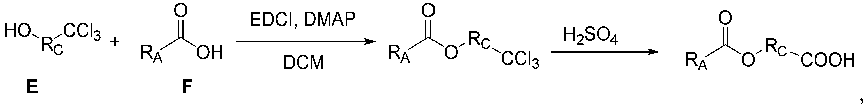 or 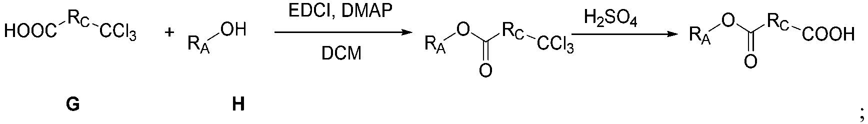 or 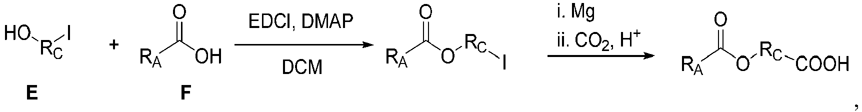 or 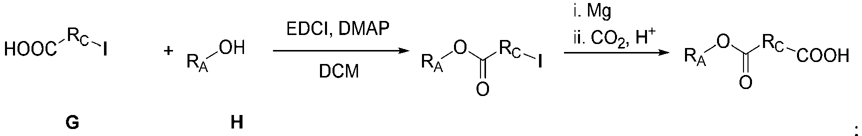 or

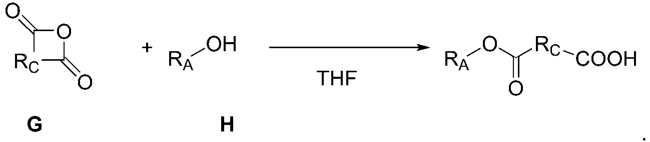

According to another aspect of the present disclosure, there is provided use of a lipid compound of formula (II) according to the present disclosure in preparation of ionizable lipids, wherein the ionizable lipids comprise lipid compounds of formulas (I), (III), (IV), (V), and (VI) of the present disclosure as shown below: 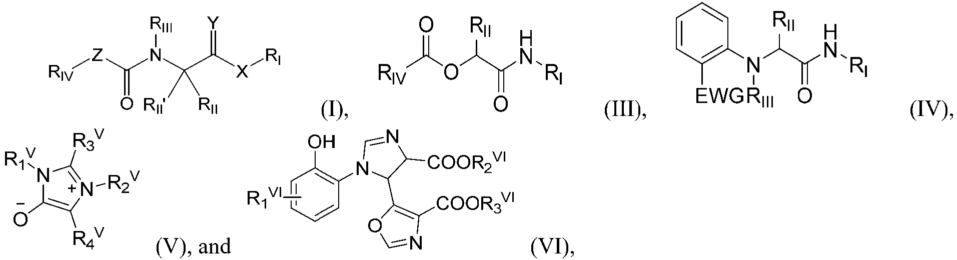 wherein substituents in the formulas (I), (III), (IV), (V), and (VI) are as defined herein.

According to another aspect of the present disclosure, there are provided lipid compounds of formulas (III), (IV), (V), and (VI) according to the present disclosure, or N-oxides, stereoisomers, or pharmaceutically acceptable salts thereof, 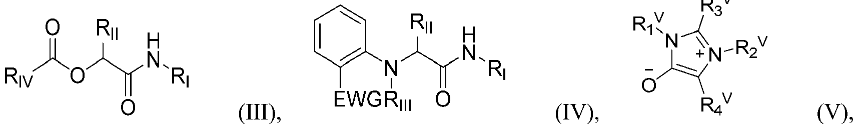 and 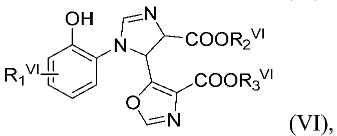 wherein substituents in the formulas (III), (IV), (V), and (VI) are as defined herein.

According to another aspect of the present disclosure, there is provided a liposome comprising the following three components: cholesterol, a phospholipid, and one or more lipid compounds selected from formulas (I), (III), (IV), (V), and (VI).

According to another aspect of the present disclosure, there is provided a drug delivery system comprising the liposome of the present disclosure as a delivery vehicle.

According to another aspect of the present disclosure, there is provided a lipid nanoparticle comprising the following four components: one or more lipid compounds selected from formulas (I), (III), (IV), (V), and (VI), a phospholipid, a structural lipid, and a PEG lipid.

According to another aspect of the present disclosure, there is provided a preparation method for a lipid nanoparticle, the preparation method comprising co-assembling one or more lipid compounds selected from formulas (I), (III), (IV), (V), and (VI) with other lipid molecules, wherein the other lipids are selected from one or more of the following group consisting of a phospholipid, a structural lipid, a PEG lipid, etc.

According to another aspect of the present disclosure, there is provided a lipid nanoparticle encapsulating a nucleic acid molecule, which uses the lipid nanoparticle of the present disclosure as a delivery vehicle.

According to another aspect of the present disclosure, there is provided a lipid nanoparticle encapsulating a plasmid, which uses the lipid nanoparticle of the present disclosure as a delivery vehicle.

According to another aspect of the present disclosure, there is provided a preparation method for a lipid nanoparticle encapsulating a nucleic acid molecule or plasmid, the method comprising the following steps:
1. dissolving a plurality of lipid compounds in an organic solvent miscible with water, such as ethanol;
2. dispersing nucleic acid molecules or plasmids in an acidic aqueous solution with pH < 7; and
3. rapidly mixing the above two solutions at a specific volume ratio to prepare the lipid nanoparticle, wherein the rapid mixing can be performed by methods including microfluidics, impinging stream, or direct mixing via pipetting devices.

### Brief Description of the Drawings

FIGS. 1 to 6 show transfection results of LNPs composed of lipid compounds of the present disclosure in 293T cells in examples.
FIGS. 7 to 12 show transfection results of LNPs composed of lipid compounds of the present disclosure in A549 cells in examples.
FIG. 13 shows transfection results of LNPs composed of lipid compounds of the present disclosure in 293T cells (top) and A549 cells (bottom) in examples.
FIG. 14 shows one-dimensional proton nuclear magnetic resonance (¹H NMR) spectrum of lipid 175.
FIG. 15 shows proton NMR splitting peaks of different R_{II} substituents in several lipid compounds based on a lipid 175 skeleton.
FIG. 16 shows proton NMR splitting peaks of different R_{II} substituents in several lipid compounds based on a lipid 284 skeleton.
FIG. 17 shows eGFP expression after LNPs encapsulating eGFP-expressing plasmid DNA are prepared using lipids 284 and 424 of the present disclosure and control lipids, and administered to cells.
FIG. 18 shows a neutralizing antibody titer against respiratory syncytial virus (RSV) euvirus obtained by detection after LNPs comprising RSV-mRNA are prepared using lipid 174 and SM102 of the present disclosure, and administered to mice via intramuscular injection.

### Detailed Description of the Embodiments

As illustrated and exemplified in the drawings, the above-mentioned and other objectives, components and advantages will become obvious through more detailed description of the following specific embodiments, where the same reference numbers markers and the like represent the same parts, componnets or features in specific embodiments. It is pointed that if there are processes that are not described in detail, they are all achieved or understood by those skilled in the art with reference to the existing technology. Used reagents or instruments, which do not indicate the manufacturers, are considered as conventional products that can be commercially available.

In terms of scopes of use, the terms "comprising", "containing" and "having" or any variations thereof, as used in Claims and specification herein, shall be deemed to indicate opening groups that may include unspecified other elements. The terms "at least one" and "one or more" can be interchangeably used. The term "single" is applied to indicate one and only one object. Similarly, when a specific quantity of objects is needed, other specific integer values such as "two" will be used. The terms "preferably", "preferable", "prefer", "optionally", "may" and similar terms are used to indicate that referenced items, conditions or steps are optional (i.e., not necessary) features in embodiments. Unless otherwise specified, a range that is described as "between a and b" includes the values of "a" and "b".

Although various improvements have been made in the description herein with reference to specific embodiments of the present disclosure, it should be understood that such the description is merely illustrative and should not be construed as limiting any claimed scope of the present disclosure. Therefore, any claimed scope and content of the present disclosure will only be limited by the terms of the appended claims in their current forms or as modified during the examination period or as implemented in any continuing application. Furthermore, it should be understood that unless otherwise stated, the features of any specific embodiments discussed herein may be combined with one or more features of any one or more embodiments discussed or considered in other ways herein.

The definitions of specific functional groups and chemical terminologies are described in more detail below. For the purposes of the present disclosure, chemical elements are identified based on the periodic table of elements, CAS edition, Handbook of Chemistry and Physics, 75th edition, inner cover, and specific functional groups are typically defined as described therein. In addition, general principles of organic chemistry, specific functional group moieties and reactivity are described in Organic Chemistry, Thomas Sorrell, University Science Books, Sausalito, 1999; Smith and March, March's Advanced Organic Chemistry, 5th edition, John Wiley & Sons, Inc., New York, 2001; Larock, Comprehensive Organic Transformations, VCH Publishers, Inc., New York, 1989; Carruthers, Some Modem Methods of Organic Synthesis, 3rd edition, Cambridge University Press, Cambridge, 1987.

Unless otherwise clearly specified, all scopes cited herein are included.

When a series of values are listed, it is intended to cover each value and subranges in this range. For example, "C₁₋₆" is intended to cover C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅ and C₅₋₆. In some embodiments, C₀ represents 0 carbon atom, i.e., the group is absent.

When any variable appears more than once in any component or in formula (I) or in any other formulas depicting and describing the compounds of the present disclosure, its definition at each occurrence is independent of its definition at every other occurrence. In addition, the use of combinations of substituents and/or variables is allowed only when such combinations generate stable compounds.

### Definition

As used herein, the term "hydrocarbyl" refers to a chemical group containing hydrogen and carbon. The hydrocarbyl may be substituted or unsubstituted. The hydrocarbyl may be unsaturated, saturated, linear, non-linear, cyclic, polycyclic or heterocyclic, and the hydrocarbyl includes alkyl, alkenyl, alkynyl, etc. The hydrocarbyl may be completely saturated, monounsaturated or polyunsaturated, and may include bivalent and multivalent groups having a specified carbon atom number (i.e., C₁-C₁₀ means 1 to 10 carbon atoms, including 1, 2, 3, 4, 5, 6, 7, 8, 9 and 10 carbon atoms). When the hydrocarbyl contains heteroatoms such as N, O and S, it is also referred to as "heterohydrocarbyl". As used herein, the term "hydrocarbylene" refers to a bivalent substituent which is monovalent hydrocarbyl with one hydrogen atom substituted by a valence. As used herein, the term "heterohydrocarbylene" refers to a bivalent substituent which is monovalent heterohydrocarbyl with one hydrogen atom substituted by a valence.

As used herein, the term "alkyl" refers to linear or branched saturated hydrocarbyl. The term "Cᵢ₋ⱼ alkyl" refers to alkyl having i to j carbon atoms. Unless otherwise specified, alkyl may contain 1 to 10 carbon atoms. In some embodiments, alkyl contains 1 to 6 carbon atoms, for example 1 to 5 carbon atoms, 1 to 4 carbon atoms, 1 to 3 carbon atoms, or 1 to 2 carbon atoms. Non-limiting examples of alkyl include methyl, ethyl, n-propyl and isopropyl, n-butyl, sec-butyl, isobutyl and tert-butyl, neopentyl, etc. As used herein, the term "alkylene" refers to a bivalent substituent which is monovalent alkyl with one hydrogen atom substituted by a valence.

As used herein, the term "alkenyl" refers to a linear or branched hydrocarbyl having at least one carbon-carbon double bond, and including groups having "cis" and "trans" orientations, or optionally, "E" and "Z" directions. Unless otherwise specified, the alkenyl may contain 2 to 10 carbon atoms. In some embodiments, the alkenyl may contain 2 to 6 carbon atoms, for example 2 to 5 carbon atoms, 2 to 4 carbon atoms and 2 to 3 carbon atoms. In some embodiments, the alkenyl group contains 2 carbon atoms. Non-limiting examples of the alkenyl include ethylidene (vinyl), allyl, butenyl, pentenyl, 1-methyl-2-buten-1-yl, 5-hexenyl, etc. As used herein, the term "alkenylene" refers to a bivalent substituent which is a monovalent alkenyl with one hydrogen atom substituted by a valence.

As used herein, the term "alkynyl" refers to a linear or branched hydrocarbyl having at least one carbon-carbon triple bond. Unless otherwise specified, the alkynyl may contain 2 to 10 carbon atoms. In some embodiments, the alkynyl group may contain 2 to 8 carbon atoms, 2 to 6 carbon atoms, 2 to 4 carbon atoms and 2 to 3 carbon atoms. In some embodiments, the alkenyl group contains 2 carbon atoms. Non-limiting examples of the alkynyl include ethynyl, 1-propinyl, 2-propinyl, etc. As used herein, the term "alkynylene" refers to a bivalent substituent which is a monovalent alkynyl with one hydrogen atom substituted by a valence.

As used herein, the term "alkoxy" refers to a group -O- alkyl in which the alkyl has a meaning as defined herein.

As used herein, the term "cycloalkyl" refers to non-aromatic, saturated monocyclic and multicyclic ring systems in which all ring forming atoms are carbon. Unless otherwise specified, the cycloalkyl group may contain 3 to 10 ring forming carbon atoms (i.e., C₃₋₁₀ cycloalkyl). In some embodiments, the cycloalkyl group may contain 3 to 9, 3 to 8, 3 to 7, 3 to 6, 4 to 10, 4 to 9, 4 to 8, 4 to 7, 4 to 6, 4 to 5, 5 to 10, 5 to 9, 5 to 8, 5 to 7, 5 to 6 ring forming carbon atoms, etc. Specifically, the cycloalkyl may be monocyclic or bicyclic. Optionally, a bicyclic cycloalkyl group may comprise fused, spirocyclic and bridged cycloalkyl structures.

In another aspect, also included is a cycloalkyl ring in which 1, 2 or 3 heteroatoms are replaced by cyclocarbon atoms. Such the group is referred to as "heterocyclyl" or "heterocyclic ring" which refers to a cycloalkyl group that is as defined above but has at least one heteroatom selected from N, O and S as a ring forming atom. Unless otherwise specified, the heterocyclyl group may contain 3 to 10 ring forming atoms (i.e., 3-to 10-membered heterocyclyl). In some embodiments, the heterocyclyl group may contain 3 to 9, 3 to 8, 3 to 7, 3 to 6, 4 to 10, 4 to 9, 4 to 8, 4 to 7, 4 to 6, 4 to 5, 5 to 10, 5 to 9, 5 to 8, 5 to 7, 5 to 6 ring forming atoms, etc. Specifically, the heterocyclyl group may be monocyclic or bicyclic. Optionally, a bicyclic heterocyclyl group may comprise fused, spirocyclic and bridged heterocyclyl structures. Non-limiting examples of the heterocyclyl group include epoxyethyl, pyrrolidinyl, piperidinyl, tetrahydropyranyl, piperazinyl, pyrrolidinyl and morpholinyl. The heterocyclyl group may also be described by using a carbon number. For example, C₃₋₆ heterocyclyl refers to a heterocyclyl group containing 3 to 6 ring forming carbon atoms, and also containing at least one heteroatom such as 1, 2 or 3 heteroatoms as ring forming atoms. In some embodiments, the heterocyclyl group or heterocyclic ring contains 1 or 2 heteroatoms as ring forming atoms. In some embodiments, the heterocyclyl group may be monocyclic or bicyclic, for example, a fused bicyclic ring and a spirobicyclic ring. Under the context of the present disclosure, the terms "heterocyclyl" and "heterocyclic ring" can be interchangeably used.

As used herein, the term "aliphatic group" refers to a substituted or unsubstituted linear and/or branched, saturated and/or unsaturated hydrocarbon group, including linear, branched or cyclic alkyl, alkenyl and alkynyl. In some embodiments, the term "aliphatic group" and "hydrocarbyl" can be interchangeably used. In some embodiments, the aliphatic group comprises one or more for example 1, 2, 3, 4, 5, 6, 7, 8, 9, 10, 11 or 12 unsaturated carbon-carbon double bond (-C=C-) and carbon-carbon triple bond (-C=C-) groups, and/or any combinations thereof.

As used herein, the term "heteroaliphatic group" refers to a substituted or unsubstituted linear and/or branched, saturated or unsaturated hydrocarbon group containing a heteroatom selected from N, O and S, including linear, branched or cyclic alkyl, alkenyl and alkynyl. In some embodiments, the term "heteroaliphatic group" and "heterohydrocarbyl" can be interchangeably used. In some embodiments, the heteroatom contained in the heteroaliphatic group" together with carbon atoms forms a main chain of the heteroaliphatic group, for example but not limited to group structures such as -C-N-C-, -C-O-C-, -C-O-O-C, -C-S-C- and -C-S-S-C, or any combinations thereof. In some embodiments, the heteroatoms contained in the heteroaliphatic group may be substituents that are linked to the carbon atom, for example but not limited to substituent structures such as -C≡N, -C=N-, -C-N=, -C=O, -C-OH, -C=S and -C-SH. In some embodiments, the heteroatoms contained in the heteroaliphatic group may be any combinations of the above-mentioned listed group structures. In some embodiments, the heteroaliphatic group contains one or more for example 1, 2, 3, 4, 5, 6, 7, 8, 9, 10, 11 or 12 unsaturated carbon-carbon 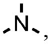 double bonds (-C=C-), carbon-carbon triple bonds (-C=C-), , -NH-, -NH₂-, OH, -OR^{m}, -O-, -C(O)-, -C(ORⁿ)-, -C(O)O-, -SH, -SR^{o}, -S-, -C(S)-, -C(SR^{p})-, -C(S)O-, -P(O)-, and/or any combinations thereof, wherein R^{m}, Rⁿ, R^{o} and R^{p} are each independently substituted or unsubstituted C₁-C₁₄ aliphatic hydrocarbyl, for example C₁-C₁₂, C₁-C₁₀, C₁-C₈, C₁-C₆ and C₁-C₄ aliphatic hydrocarbyl. Non-limiting specific examples of substituted or unsubstituted C₁-C₁₄ aliphatic hydrocarbyl include but are not limited to methyl, ethyl, n-propyl and isopropyl, n-butyl, sec-butyl, isobutyl, tert-butyl, neopentyl, cyclopropyl, cyclobutyl, cyclopentyl, cyclohexyl, ethylidene (vinyl), propenyl, butenyl, pentenyl, 1-methyl-2-buten-1-yl, 5-hexenyl, ethynyl, 1-propynyl, 2-propynyl, etc.

As used herein, the term "aryl" or "aryl ring" refers to a monocyclic, bicyclic or polycyclic carbon ring system having at least one aryl ring. Unless otherwise specified, the aryl may be 6- to 10-membered. In some embodiments, the aryl group may contain 6 ring forming carbon atoms. All the atoms in the carbon ring aryl group are carbon atoms. Non-limiting examples of the aryl group include phenyl, naphthyl, 1,2-dihydronaphthyl, 1,2,3,4-tetrahydronaphthyl, fluorenyl, indanyl, indenyl, etc. Under the context of the present disclosure, the terms "aryl" and "aryl ring" can be interchangeably used.

As used herein, the term "heteroaryl" or "heteroaryl ring" refers to a monocyclic system, or a fused or bridged bicyclic system, wherein the cyclic system contains one, two, three or four heteroatoms independently selected from the group consisting of nitrogen, oxide and sulfur; and at least one ring is an aryl ring. Unless otherwise specified, the heteroaryl group may be 5- to 10-membered. In some embodiments, the heteroaryl group may be 5-membered or 6-membered. In some embodiments, the heteroaryl group may contain one, two or three heteroatoms. In some embodiments, the heteroaryl group may contain one or two heteroatoms. Non-limiting examples of the heteroaryl group include benzoimidazolyl, benzofuranyl, benzothiazolyl, benzothiophenyl, benzoxazolyl, furyl, imidazolyl, indolyl, isoindazolyl, isoquinolyl, isothiazolyl, isothiazolyl, isoxazolyl, oxadiazolyl, oxazolyl, purinyl, pyrrolyl, pyridyl, pyrazinyl, pyrimidyl, quinolyl, quinolyl, thiadiazolyl, thiazolyl, thienyl, triazolyl, tetrazyl, dihydroindolyl, tetrahydroquinolyl, tetrahydroisoquinolyl, etc. The heteroaryl group comprises at least one ring having at least one heteroatom as described above and at least one aryl ring. For example, the ring having at least one heteroatom may be fused to one, two or three carboncyclic rings such as an aryl ring, a cyclohexane ring, a cyclohexene ring, a cyclopentane ring and a cyclopentene ring, or another monocyclic heterocyclic ring. Non-limiting examples of the fused heteroaryl group include 1,2,3,5,8,8a-hexahydroindolizine, 2,3-dihydrobenzofuran, 2,3-dihydroindoline, 2,3-dihydrobenzothiophene, etc. Under the context of the present disclosure, the terms "heteroaryl" and "heteroaryl ring" can be interchangeably used.

As used herein, the term "oxo" refers to a divalent oxygen atom and the structure of oxo may be displayed as=O.

As used herein, the term "halo" or "halogen" refers to fluorine, chlorine, bromine and iodine, or fluorides, chlorides, bromides and iodides. In some embodiments, non-limiting examples of halo include fluorine, chlorine and bromine, or fluorides, chlorides and bromides, more specifically fluorine and chlorine, or fluorides and chlorides.

As used herein, the term "heteroatom" refers to nitrogen (N), oxygen (O) and sulfur (S), and may include any oxidation forms of nitrogen and sulfur, and any quaternization forms of alkaline nitrogen, unless otherwise specified.

As used herein, the term "substitutive", "substituted" or "substituted with" refers to a fact that when referring to a chemical group, the chemical group has one or more hydrogen atoms which are removed and replaced by substituents. As used herein, the term "substituent" has common meaning known in the art and refers to a chemical moiety that is covalently attached to a mother group, or if appropriate, fused to the mother group. It can be understood that the substitution of a given atom is limited by a valence. It should be understood that the substituent may be further substituted.

When it is indicated in formula (I) or any of its embodiments that a moiety is "optionally" substituted, it means that formula (I) or its embodiments encompass compounds substituted with a substituent indicated on the moiety and compounds that do not contain the indicated substituent on the moiety (i.e., wherein the moiety is not substituted).

Compounds provided herein are described with reference to general formulas and specific compounds. In addition, the compounds of the present disclosure may be present in multiple different forms or in a form of its derivatives, all of which are included within the scope of the present disclosure. These include for example pharmaceutically acceptable salts, tautomers, stereoisomers, racemic mixtures, positional isomers, prodrugs, solvated forms, different crystal forms or polymorphic substances, and active metabolites, etc.

As used herein, unless otherwise specified, the term "pharmaceutically acceptable salt" includes salts that maintain biological effectiveness of free acid/alkali forms of specific compounds and are not unexpected in biology or in other aspects. The pharmaceutically acceptable salt may include salts formed with inorganic alkali or acids and organic alkali or acids. Under the condition that the compound of the present disclosure contains one or more acidic or alkaline groups, the present disclosure also comprises their corresponding pharmaceutically acceptable salts. Therefore, the compound containing the acidic group (such as a carboxyl group) of the present disclosure may be present in a form of salt, and used according to the present disclosure, for example, alkali metal salts, alkaline earth metal salts, aluminum salts or ammonium salts. More non-limiting examples of such the salts include lithium salts, potassium salts, calcium salts, magnesium salts, barium salts, or salts of ammonia or organic amine (such as ethylamine, ethanolamine, diethanolamine, triethanolamine, piperidine, N-methylglutamine, or amino acids). For example, these salts are easily obtained by reacting compounds having acidic groups with appropriate alkali (such as lithium hydroxide, sodium hydroxide, sodium propoxide, potassium hydroxide, potassium ethoxide, magnesium hydroxide, calcium hydroxide or barium hydroxide). Other alkali salts of the compound of the present disclosure include but are not limited to salts of copper (I), copper (II), iron (II), iron (III), manganese (II) and zinc. The compound of the present disclosure contains one or more alkaline groups, for example groups that may be protonated, which may be present in a form of salts, and in a form of addition salts of the alkaline groups and inorganic acids or organic acids. Examples of appropriate acids include hydrogen chloride, hydrogen bromide, hydrogen iodide, phosphoric acid, sulfuric acid, nitric acid, methanesulfonic acid, p-toluenesulfonic acid, naphthalene disulfonic acid, sulfoacetic acid, trifluoroacetic acid, oxalic acid, acetic acid, tartaric acid, lactic acid, salicylic acid, benzoic acid, carbonic acid, formic acid, propionic acid, pivalic acid, diethylacetic acid, malonic acid, succinic acid, heptanedioic acid, fumaric acid, malonic acid, maleic acid, malic acid, pamoic acid, mandelic acid, sulphamic acid, phenylpropionic acid, gluconic acid, ascorbic acid, isonicotinic acid, citric acid, adipic acid, taurocholic acid, glutaric acid, stearic acid, glutamic acid or aspartic acid, and other acids known to those skilled in the art. The formed salts are particularly hydrochlorides, chlorides, hydrobromates, bromides, iodides, sulfates, phosphates, methylsulfonates (methanesulfonates), toluenesulfonates, carbonates, bicarbonates, formates, acetates, sulfoacetates, trifluoromethanesulfonates, oxalates, malonates, maleates, succinates, tartrates, malates, pamoates, mandelates, fumarates, lactates, citrates, glutarates, stearates, aspartates and glutamates. In addition, the chemometry of the salt formed by the compound of the present disclosure may be an integer multiple or a non-integer multiple of 1.

The compound containing an alkaline nitrogen-containing group of the present disclosure can be quaternized by using a reagent, for example C₁₋₄ haloalkane such as methyl, ethyl, isopropyl and tert-butyl chloride, bromine and iodine; di(C₁₋₄) alkyl sulfates such as dimethyl sulfate, diethyl sulfate and dipentyl sulfate; C₁₀₋₁₈ alkyl halides such as decyl, dodecyl, laurel, myristyl and stearyl chlorides, bromides and iodides; and aryl C₁₋₄ alkyl halides such as benzyl chloride and phenethyl bromide.

If the compound of the present disclosure simultaneously contains an acidic group and an alkaline group in a molecule, in addition to the above-mentioned salt forms, the present disclosure also comprises an internal salt or betaine (zwitterion). Corresponding salts may be obtained by a conventional method known by those skilled in the art, for example, by contacting them with an organic or inorganic acid or alkali in a solvent or a dispersing agent, or by performing anion exchange or cation exchange with other salts. The present disclosure also comprises all the salts of the compound of the present disclosure, due to low physiological compatibility, they are not directly applicable to drugs, but can be used as, for example, an intermediate of a chemical reaction or used for preparing pharmaceutically acceptable salts. Reviews on more suitable salts are seen in Stahl and Wermuth, Manual of Medicinal Salts: Characteristics, selection and use (Wiley-VCH, 2002).

The lipid compound of the present disclosure or the pharmaceutically acceptable salt thereof may be present in non-solvated and solvated forms. As used herein, the term "solvate" refers to a molecular composite comprising the lipid compound of the present disclosure or the pharmaceutically acceptable salt thereof and one or more pharmaceutically acceptable solvent molecules. For example, when the solvent is water, the term "hydrate" is used.

The lipid compound of the present disclosure may have one or more chiral (asymmetrical) centers. The present disclosure encompasses all the stereoisomeric forms of the lipid compound of the present disclosure. The asymmetrical centers existing in the lipid compounds of the present disclosure may independently have (R) or (S) configurations. When a bond to a chiral carbon is described as a straight line in the structural formula of the present disclosure, or when the name of the compound is described without the (R) or (S) chiral name of the chiral carbon, it should be understood that the (R) and (S) configurations of each of such the chiral carbon and therefore each enantiomer or diastereomer and mixtures thereof are all contained in this formula or name. The generation of specific stereoisomers and mixtures thereof can be identified in examples where such the stereoisomers or mixtures thereof are obtained, but absolutely does not limit the conclusion of all the stereoisomers and mixtures thereof in the scope of the present disclosure.

The present disclosure comprises all possible enantiomers and diastereomers as well as mixtures of two or more stereoisomers, for example mixtures of eneantiomers and/or diastereomers in all proportions. Therefore, the enantiomers are enantiomeric pure forms (as left and right handed eneantiomers) of the subject of the present disclosure, racemic forms, and mixture forms of two enantiomers in all proportions. Under the condition of cis/trans isomers, the present disclosure comprises cis forms and trans forms as well as mixtures of these forms in all proportions. If necessary, the mixtures can be separated by a conventional method (for example, by chromatography or crystallization, by using stereochemically homogeneous synthesis starting materials, or by stereoselective synthesis) to prepare single stereoisomers. Optionally, derivation can be performed prior to separation of stereoisomers. The separation of mixtures of stereoisomers can be performed in a middle step during the synthesis of the lipid compound of the present disclosure, or on the final racemic product. Absolute stereochemistry can be determined by X-ray crystallography of crystalline products or crystalline intermediates, if necessary, these crystalline products or crystalline intermediates are derived by using a reagent containing a stereocenter with a known configuration. Optionally, absolute stereochemistry can be determined by vibration circular dichroism (VCD) spectral analysis.

Unless otherwise specified, the structure described herein is also intended to include different compounds only in the presence of one or more isotope-enriched atoms, in other words, compounds in which one or more atoms are replaced by atoms having the same atomic number, but the atomic mass or mass number is different from a dominant atomic mass or mass number in nature. Such the compounds are called "isotopic variants". The present disclosure is intended to include all pharmaceutically acceptable isotopic variants of the compound of formula (I). Examples of isotopes suitably included in the compound of the present disclosure include but are not limited to isotopes of hydrogen, for example ²H (i.e., D) and ³H; carbon, for example ¹¹C, ¹³C and ¹⁴C; chlorine, for example ³⁶Cl; fluorine, for example ¹⁸F; iodine, for example ¹²³I and ¹²⁵I; nitrogen, for example ¹³N and ¹⁵N; oxygen, for example ¹⁵O, ¹⁷O and ¹⁸O; phosphorus, for example ³²P; and sulfur, for example ³⁵S. Some isotopic variants of the compound of formula (I), for example those admixed with radioactive isotopes, can be used for researches on drug and/or substrate tissue distribution. Specifically, compounds having different described structures only in replacement with heavier isotopes (for example hydrogen is replaced with deuterium (²H or D)) can provide some therapeutic advantages, for example, the above-mentioned compounds can be used in some specific cases due to higher metabolic stability, increased *in vivo* half-life period or reduced dose requirements. The isotopic variants of the compound of formula (I) can be typically synthesized and prepared through conventional technologies known by those skilled in the art or through methods similar to those described in appended examples and by replacing previously used non-labeling reagents with proper isotope labeling reagents.

Pharmaceutically acceptable solvates according to the present disclosure may comprise those in which crystalline solvents can be replaced by isotopes, for example D₂O, d₆-acetone and d₆-dimethylsulfoxide (DMSO).

As used herein, the term "lipid" refers to a class of organic compounds which are derivatives (for example esters) of a fatty acid and are typically characterized by being insoluble in water but soluble in many organic solvents. The lipid is typically divided into at least three classes: (1) a "simple lipid" comprising fat and oil as well as wax; (2) a "composite lipid" comprising phospholipids and glycolipids; and (3) a "derived lipid" such as steroid.

As used herein, the term "phospholipid" refers to a lipid molecule consisting of two hydrophobic fatty acid "tails" and a hydrophilic "head" formed by phosphate groups. The two components are most often bonded through a glycerol molecule, and therefore, herein, the phospholipid is preferably a glycerol-phospholipid. In addition, the phosphate group is often modified by a simple organic molecule for example choline (i.e., generating choline phosphate) or ethanolamine (i.e., generating phosphoethanolamine). In some embodiments, the phospholipid can be selected from the following group which includes but is not limited to 1,2-dilinoleoyl-sn-glycero-3-phosphocholine (DLPC), , 2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-di-undecyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-bisdocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0PE), 2-distearoyl-sn-glycero-3-phosphoethanolamine, 1,2-dilinoleoyl -sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-bisdocosahexaenoyl-sn-glycero-3-phosphoethanolamine, 1,2-dioleoyl-sn-glycero-3-phospho-rac-(1-glycero) sodium salts (DOPG), sphingomyelin and mixtures thereof.

As used herein, the term "structural lipid" refers to sterol and also means a lipid containing a sterol moiety. In some embodiments, the structural lipid can be selected from the following group which includes but is not limited to cholesterol, fecal sterol, sitosterol, ergosterol, campesterol, stigmasterol, brassinosteroid, tomatidine, tomatin, ursolic acid, α-tocopherol, hopane, phytosterol, steroid and mixtures thereof. In some embodiments, the structural lipid is cholesterol.

As used herein, the term "PEG lipid", or alternatively called a PEGylated lipid, refers to any proper lipid modified with a PEG (polyethylene glycol) group. In some embodiments, the PEG lipid can be selected from the following group which includes but is not limited to PEG modified phosphatidylethanolamine, PEG modified phosphatidic acid, PEG modified ceramide, PEG modified dialkylamine, PEG modified diacylglycerol, PEG modified dialkylglycerol and mixtures thereof. In some embodiments, specific examples of the PEG lipid include but are not limited to C14-PEG2000 (1,2-dimyristoyl-rac-glycerol, methoxypolyethylene glycol-2000 (DMG-PEG2000)) and C18-PEG5000 (1,2-distearoyl-rac-glycerol and methoxypolyethylene glycol-5000 (DSG-PEG5000)).

As used herein, the term "hydrophobic lipid" refers to a compound having non-polar groups which include (but are not limited to) long-chain saturated and unsaturated aliphatic hydrocarbyl, and such the groups are optionally substituted with one or more aromatic groups, cycloaliphatic groups or heterocyclic groups. Proper examples include (but are not limited to) diacylglycerol, dialkylglycerol, N,N-dialkylamino, 1,2-diacyloxyl-3-aminopropane and 1,2-dialkyl-3-aminopropane.

As used herein, the terms "cationic lipid" and "ionizable lipid" can be interchangeably used herein for lipids comprising those amino head groups (for example, alkylamino or dialkylamino head groups) having one, two, three or more fatty acids or fatty alkyl chains and titratable pH, and salts thereof. The cationic lipid is typically protonated (i.e., positively charged) at a pH lower than its pKa, and is basically neutral at a pH higher than its pKa. The cationic lipid of the present disclosure is also referred to as a titratable cationic lipid. In some embodiments, the cationic lipid contains: an ionizable tertiary amine (for example, titratable pH) head group; C18 alkyl chains, wherein each alkyl chain independently has 0 to 3 (for example, 0, 1, 2 or 3) double bonds; and an ether, ester or ketal bond between the head group and the alkyl chains. Such the catonic lipids include (but are not limited to) DSDMA, DODMA, DLinDMA, DLenDMA, γ-DLenDMA, DLin-K-DMA, DLin-K-C2-DMA (also referred to as DLin-C2K-DMA, XTC2 and C2K), DLin-K-C3-DMA, DLin-K-C4-DMA, DLen-C2K-DMA, y-DLen-C2K-DMA, DLin-M-C2-DMA (also referred to as MC2), DLin-M-C3-DMA (also referred to MC3) and (DLin-MP-DMA) (also referred to as1-B11). In some embodiments, the ionizable lipid is heptadec-9-yl-8-((2-hydroxyethyl) (6-oxo-6-((undecyloxy)hexyl)amino) octanoate (SM102).

As used herein, "nucleic acid" comprises double-stranded RNA, single-stranded RNA, isolated RNA (such as partially purified RNA), basically pure RNA, synthetic RNA, RNA generated by recombination, and altered RNA that differs from naturally occurring RNA through addition, deletion, substitution and/or alternation of one or more nucleotides. Such the alternation can include addition of non-nucleotide substances into the end or internal (for example one or more nucleotides of RNA) of interfering RNA. The nucleotide in the RNA molecule of the present disclosure can also comprise non-standard nucleotides, such as a non-naturally occurring nucleotide, a chemically synthesized nucleotide or deoxyribonucleotide. These altered RNAs can be referred to as analogues or naturally occurring RNA analogues. As used herein, the terms "ribonucleic acid" and "RNA" refer to a molecule containing at least one ribonucleotide residue, including siRNA, anti-sense RNA, single-stranded RNA, microRNA, mRNA, non-coding RNA and multivalent RNA. The ribonucleotide is a nucleotide having hydroxyl at the position 2' of a β-D-ribofuranose moiety. These terms include double-stranded RNA (dsRNA), single-stranded RNA (ssRNA), isolated RNA (such as partially purified RNA), basically pure RNA, synthetic RNA, RNA generated by recombination, and modified and changed RNA that differs from naturally occurring RNA through addition, deletion, substitution, modification and/or alternation of one or more nucleotides. The alternation of RNA can include for example addition of non-nucleotide substances into the end or internal (for example one or more nucleotides of RNA) of interfering RNA, and the nucleotide in the RNA molecule comprises non-standard nucleotides, such as non-naturally occurring nucleotides or chemically synthesized nucleotides or deoxyribonucleotides. These altered RNAs can be referred to as analogues.

As used herein, the term "liposome" refers to a vesicle having at least one lipid double layer, preferably a spherical vesicle. The liposome is capable of carrying an aqueous solution, a compound, a drug or other substances in a compartment (i.e., an internal cavity or space) surrounded by at least one lipid double layer.

As used herein, the term "lipid nanoparticle" refers to a lipid preparation for delivering a therapeutic nucleic acid (for example, mRNA) to a concerned target site (for example, cells, tissues and organs). In some embodiments, the lipid nanoparticle is a nucleic acid-lipid particle which is typically composed of a nucleic acid, a cationic lipid (for example, the lipid compound of the present disclosure), a non-cationic lipid (for example, a phospholipid), a PEG-lipid and an optional structural lipid (for example, cholesterol). Typically, the therapeutic nucleic acid (for example, mRNA) can be encapsuled in the lipid moiety of the lipid nanoparticle, thereby protecting the therapeutic nucleic acid from enzymatic degradation.

### Lipid compound

The lipid compound of the present disclosure can be used for gene delivery, particularly in a liposome and a lipid nanoparticle to deliver a therapeutic agent and/or a preventive agent such as a nucleic acid to cells or organs. When used in the preparation of the liposome and the lipid nanoparticle, the lipid compound of the present disclosure can also be referred to as an "ionizable lipid", an "ionizable lipid compound", a "cationic lipid", or a "cationic lipid compound".

In one aspect, the lipid compound of the present disclosure is a lipid compound having a structure of formula (I), or an N-oxide, a stereoisomer or a pharmaceutically acceptable salt thereof, 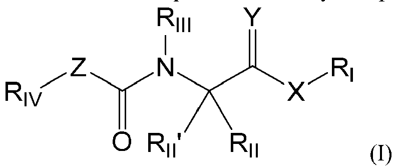
wherein X is O or NH; Y is O or S, Z is absent, or Z is O, NH or S;
R_{II}' is hydrogen, or substituted or unsubstituted hydrocarbyl, substituted or unsubstituted heterohydrocarbyl, substituted or unsubstituted aryl, or substituted or unsubstituted heteroaryl having 1 to 11 carbon atoms;
R_{I}, R_{II}, R_{III} and R_{IV} are each independently selected from a group (A) comprising a group containing at least one ionizable tertiary amine structure, a group (B) comprising a substituted or unsubstituted aliphatic or substituted or unsubstituted heteroaliphatic group having 11 to 30 carbon atoms, and a group (C) comprising a substituted or unsubstituted hydrocarbyl, substituted or unsubstituted heterohydrocarbyl, substituted or unsubstituted aryl, or substituted or unsubstituted heteroaryl group having fewer than 11 carbon atoms,
provided that:
   (1) when any one of R_{I}, R_{II}, R_{III} and R_{IV} is a group containing at least one ionizable tertiary amine structure, any two of the remaining three are identical or different substituted or unsubstituted hydrocarbyl, substituted or unsubstituted heterohydrocarbyl, substituted or unsubstituted aryl, or substituted or unsubstituted heteroaryl groups having fewer than 11 carbon atoms, and the other is a substituted or unsubstituted aliphatic or substituted or unsubstituted heteroaliphatic group having 11 to 30 carbon atoms, or, any two of the remaining three are identical or different substituted or unsubstituted aliphatic or substituted or unsubstituted heteroaliphatic groups having 11 to 30 carbon atoms, and the other is a substituted or unsubstituted hydrocarbyl, substituted or unsubstituted heterohydrocarbyl, substituted or unsubstituted aryl, or substituted or unsubstituted heteroaryl group having fewer than 11 carbon atoms;
      or
   (2) when any two of R_{I}, R_{II}, R_{III} and R_{IV} are identical or different groups containing at least one ionizable tertiary amine structure, one of the remaining two is a substituted or unsubstituted hydrocarbyl, substituted or unsubstituted heterohydrocarbyl, substituted or unsubstituted aryl, or substituted or unsubstituted heteroaryl group having fewer than 11 carbon atoms, and the other is a substituted or unsubstituted aliphatic or substituted or unsubstituted heteroaliphatic group having 11 to 30 carbon atoms.

In some embodiments, R_{II}' is substituted or unsubstituted hydrocarbyl, substituted or unsubstituted heterohydrocarbyl, substituted or unsubstituted aryl, or substituted or unsubstituted heteroaryl having 1 to 11 carbon atoms. In some embodiments, "substituted" refers to being optionally substituted with 1, 2 or 3 substituents selected from C₁-C₆ alkoxy, -OH, -SH or -NR^{d}R^{d'}, wherein R^{d} and R^{d}' are each independently C₁-C₃ alkyl. In some embodiments, R_{II}' is selected from the group C, specific limitation descriptions about the group C are all suitable for R_{II}'. R_{II}' is hydrogen or is selected from the group C, for example hydrocarbyl having 1 to 8 such as 1 to 6 and 1-4 carbon atoms, specifically for example C₁-C₆ alkyl and C₁-C₄ alkyl, including but not limited to methyl, ethyl, propryl, isopropyl, n-butyl, p-butyl, sec-butyl, tert-butyl, pentyl, hexyl, etc. In some embodiments, R_{II}' and R_{II} are identical. In some embodiments, R_{II}' and R_{II} are different.

Herein, groups containing at least one ionizable tertiary amine structure can be collectively referred to as a set of groups, i.e., group A. Therefore, in the lipid compound of formula (I), R_{I,} R_{II,} R_{III} and R_{IV} can be each independently a group containing at least one ionizable tertiary amine structure and selected from the group A. When any two of R_{I,} R_{II,} R_{III} and R_{IV} are groups containing at least one ionizable tertiary amine structure, they are identical, or different. In some embodiments, only one of R_{I,} R_{II,} R_{III} and R_{IV} is a group containing at least one ionizable tertiary amine structure.

In some embodiments, the group containing at least one ionizable tertiary amine structure is a group having 3 to 11 total carbon atoms. In some embodiments, the group containing at least one ionizable tertiary amine structure is a group having the following general formula: 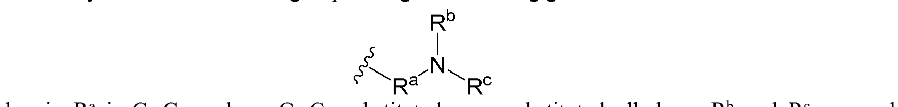 wherein R^{a} is C₁-C₆ such as C₁-C₄ substituted or unsubstituted alkylene; R^{b} and R^{c} are each independently C₁-C₆ such as C₁-C₄ substituted or unsubstituted alkyl, C₂-C₆ such as C₂-C₄ substituted or unsubstituted alkenyl and C₂-C₆ such as C₂-C₄ substituted or unsubstituted alkynyl, which are optionally substituted with 1, 2 or 3 substituents selected from -OH, -SH, -NR^{d}R^{d'}, or phenyl, wherein R^{d} and R^{d}' are each independently hydrogen or C₁-C₃ alkyl; or R^{b} and R^{c} together with the N atom to which they are attached form a 5- to 12-membered heterocyclic ring or heteroaromatic ring, the 5- to 12-membered heterocyclic ring or heteroaromatic ring containing 1, 2 or 3 heteroatoms independently selected from N, O and S, wherein at least one heteroatom is N, and the 5- to 12-membered heterocyclic ring or heteroaromatic ring is optionally substituted with one or more C₁-C₆ such as C₁-C₄ alkyls or oxo (=O); or R^{a} and R^{b} together with the N atom to which they are attached form a 5- to 12-membered heterocyclic ring or heteroaromatic ring optionally substituted with C₁-C₆ such as C₁-C₄ alkylene, the 5-to 12-membered heterocyclic ring or heteroaromatic ring optionally substituted with C₁-C₆ such as C₁-C₄ alkylene contains 1, 2 or 3 heteroatoms selected from N, O and S, wherein at least one heteroatom is N, and the 5- to 12-membered heterocyclic ring or heteroaromatic ring optionally substituted with alkylene is optionally substituted with one or more C₁-C₆ such as C₁-C₄ alkyls or oxo (=O); or R^{a}, R^{b} and R^{c} together with the N atom to which they are attached form a 5- to 12-membered heterocyclic ring or heteroaromatic ring optionally substituted with C₁-C₆ such as C₁-C₄ alkylene, the 5-to 12-membered heterocyclic ring or heteroaromatic ring optionally substituted with C₁-C₆ such as C₁-C₄ alkylene contains 1, 2 or 3 heteroatoms selected from N, O and S, wherein at least one heteroatom is N, and the 5- to 12-membered heterocyclic ring or heteroaromatic ring optionally substituted with alkylene is optionally substituted with one or more C₁-C₆ such as C₁-C₄ alkyls or oxo (=O).

In some embodiments, R^{b} and R^{c} together with the N atom to which they are attached form a 5- to 12- such as 5- to 6-membered heterocyclic ring or heteroaromatic ring, the 5- to 12- such as 5- to 6-membered heterocyclic ring or heteroaromatic ring can be monocyclic, fused bicyclic, spirocyclic bicyclic or bridged bicyclic, etc. In some embodiments, R^{b} and R^{c} together with the N atom to which they are attached form a 5-, 6-, 7- or 8-membered monocyclic heterocyclic ring or heteroaromatic ring which contains 1, 2 or 3 heteroatoms selected from N, O and S, wherein at least one heteroatom is N. In some embodiments, non-limiting examples of the heterocyclic ring or heteroaromatic ring formed by R^{b} and R^{c} together with the N atom to which they are attached include but are not limited to the following heterocyclic rings and heteroaromatic rings:

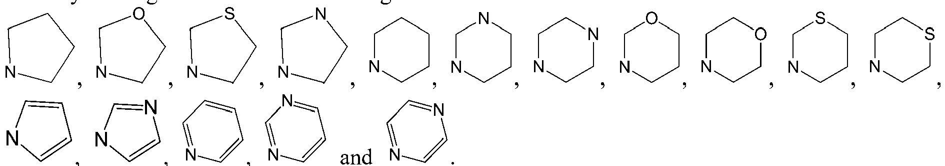

In some embodiments, R^{a} and R^{b} together with the N atom to which they are attached form a 5- to 12- such as 5- to 6-membered heterocyclic ring or heteroaromatic ring optionally substituted with C₁-C₆ such as C₁-C₄ alkylene, the 5- to 12- such as 5- to 6-membered heterocyclic ring or heteroaromatic ring optionally substituted with alkylene can be monocyclic, fused bicyclic, spirocyclic bicyclic or bridged bicyclic, etc. In some embodiments, R^{a} and R^{b} together with the N atom to which they are attached form a 5-, 6-, 7- or 8-membered monocyclic heterocyclic ring or heteroaromatic ring optionally substituted with C₁-C₆ such as C₁-C₄ alkylene, which contains 1, 2 or 3 heteroatoms selected from N, O and S, wherein at least one heteroatom is N. In some embodiments, non-limiting examples of the 5- to 12-such as 5- to 6-membered heterocyclic ring or heteroaromatic ring optionally substituted with C₁-C₆ such as C₁-C₄ alkylene formed by R^{a} and R^{b} together with the N atom to which they are attached include but are not limited to the following heterocyclic rings: * represents a position linked to other parts in a compound molecule,

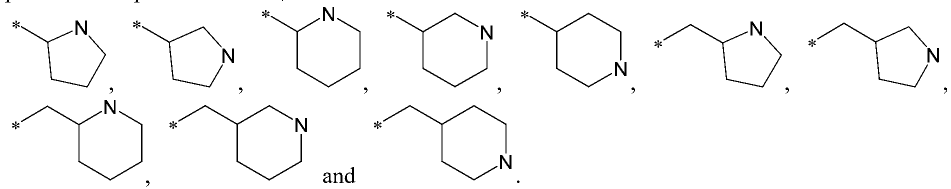

In some embodiments, R^{a}, R^{b} and R^{c} together with the N atom to which they are attached form a 5-to 12- such as 5- to 6-membered heterocyclic ring or heteroaromatic ring optionally substituted with C₁-C₆ such as C₁-C₄ alkylene, the 5- to 12- such as 5- to 6-membered heterocyclic ring or heteroaromatic ring optionally substituted with alkylene can be monocyclic, fused bicyclic, spirocyclic bicyclic or bridged bicyclic, etc. In some embodiments, R^{a}, R^{b} and R^{c} together with the N atom to which they are attached form a 5-, 6-, 7- or 8-membered monocyclic heterocyclic ring or heteroaromatic ring optionally substituted with C₁-C₆ such as C₁-C₄ alkylene, which contains 1, 2 or 3 heteroatoms selected from N, O and S, wherein at least one heteroatom is N. In some embodiments, non-limiting examples of the 5- to 12- such as 5- to 6-membered heterocyclic ring or heteroaromatic ring optionally substituted with C₁-C₆ such as C₁-C₄ alkylene formed by R^{a} and R^{b} together with the N atom to which they are attached include but are not limited to the following heteroaromatic rings: * represents a position that is linked to other parts in a compound molecule,

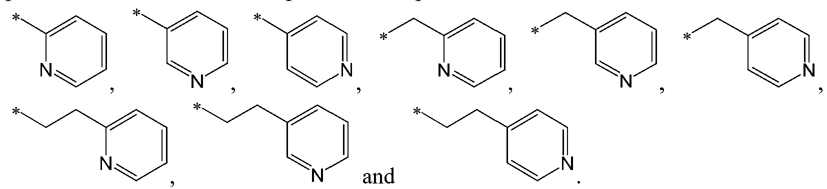

In some embodiments, the group A comprises groups containing at least one ionizable tertiary amine structure as shown in the following structures:

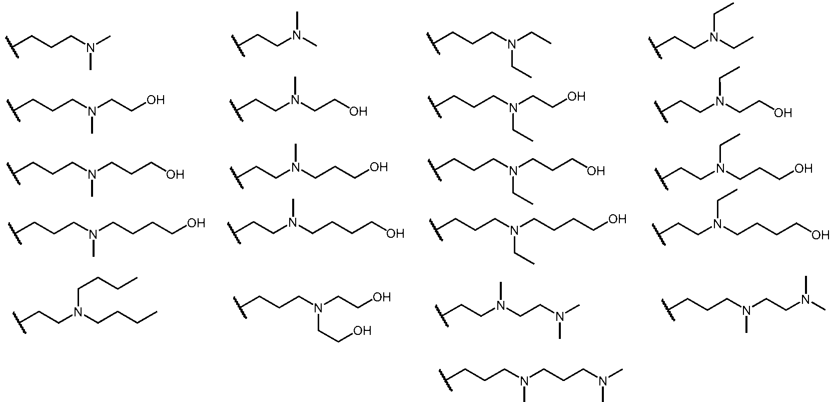

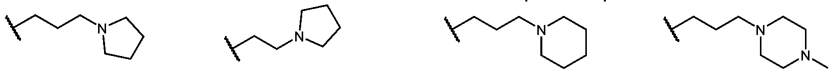

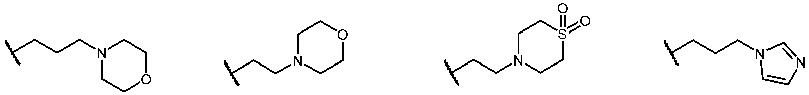

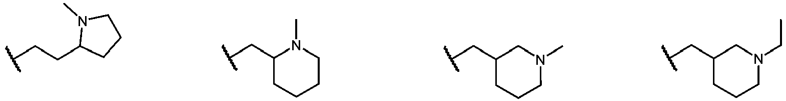

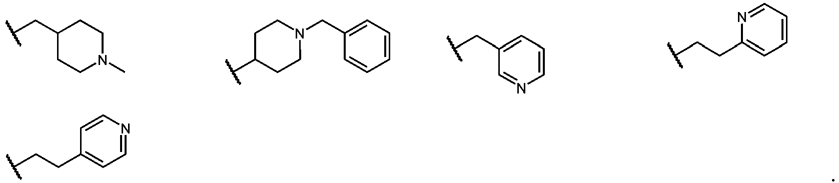

Herein, substituted or unsubstituted aliphatic or substituted or unsubstituted heteroaliphatic groups having 11 to 30 carbon atoms can be collectively referred to as a set of groups, i.e., group B. Therefore, in the lipid compound of formula (I), R_{I,} R_{II,} R_{III} and R_{IV} can be each independently substituted or unsubstituted aliphatic or substituted or unsubstituted heteroaliphatic groups having 11 to 30 carbon atoms and selected from the group B. When any two of R_{I,} R_{II,} R_{III} and R_{IV} are substituted or unsubstituted aliphatic or substituted or unsubstituted heteroaliphatic groups having 11 to 30 carbon atoms and selected from the group B, they can be identical or different.

In some embodiments, the substituted or unsubstituted aliphatic or substituted or unsubstituted heteroaliphatic group selected from the group B has 11 to 30 carbon atoms, for example 11, 12, 13, 14, 15,16, 17, 18, 19, 20, 21, 22, 23, 24, 25, 26, 27, 28, 29 and 30 carbon atoms. In some embodiments, the substituted or unsubstituted aliphatic or substituted or unsubstituted heteroaliphatic group selected from the group B has for example 11 to 16 carbon atoms, 11 to 25 carbon atoms, 16 to 25 carbon atoms or 16 to 30 carbon atoms, or the number of carbon atoms it has is in a range between any two numerical values within a range of 11 to 30.

In some embodiments, the substituted or unsubstituted aliphatic or substituted or unsubstituted heteroaliphatic group selected from the group B contains 1 to 8 heteroatoms independently selected from N, O and S, for example 1, 2, 3, 4, 5, 6, 7 and 8 heteroatoms. In some embodiments, the substituted or unsubstituted aliphatic or substituted or unsubstituted heteroaliphatic group selected from the group B contains 1 to 2 heteroatoms, 1 to 3 heteroatoms, 1 to 4 heteroatoms or 1 to 5 heteroatoms, or the number of heteroatoms it has is in a range between any two numerical values within a range of 1 to 8.

In some embodiments, the substituted or unsubstituted aliphatic or substituted or unsubstituted heteroaliphatic group selected from the group B has 11 to 30 atoms including carbon atoms and heteroatoms such as N, O and S, for example 11,12, 13, 14, 15, 16, 17, 18, 19, 20, 21, 22, 23, 24, 25, 26, 27, 28, 29 and 30 atoms. In some embodiments, the substituted or unsubstituted aliphatic or substituted or unsubstituted heteroaliphatic group selected from the group B has for example 11 to 16 atoms, 11 to 25 atoms, 16 to 25 atoms, or 16-30 atoms, or the number of atoms it has is in a range between any two numerical values within a range of 11 to 30.

In some embodiments, the group B comprises substituted or unsubstituted aliphatic or substituted or unsubstituted heteroaliphatic groups having 11 to 30 carbon atoms as shown in the following structures:

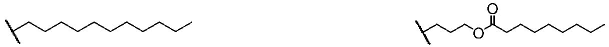

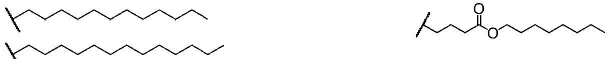

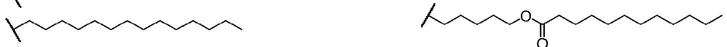

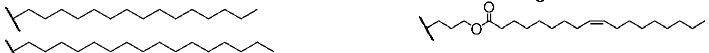

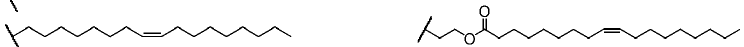

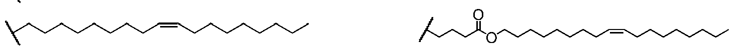

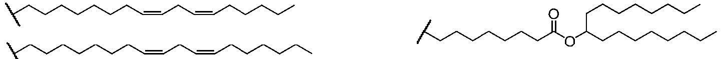

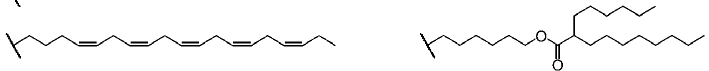

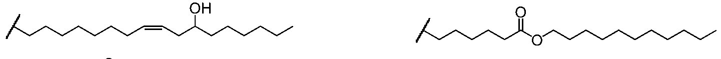

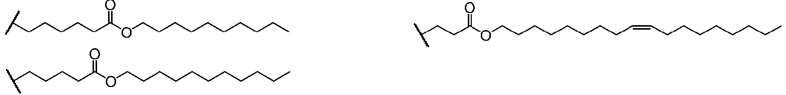

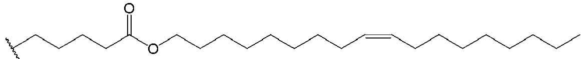

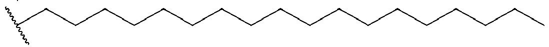

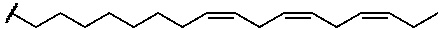

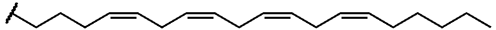

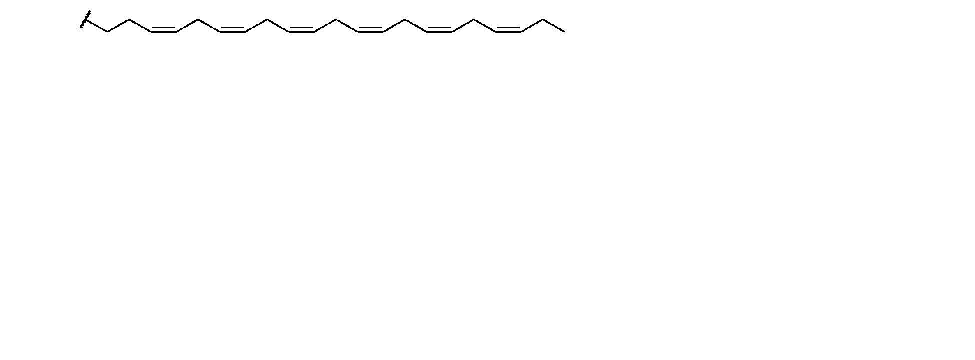

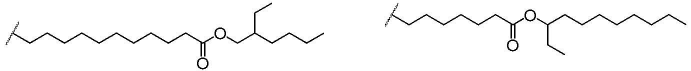

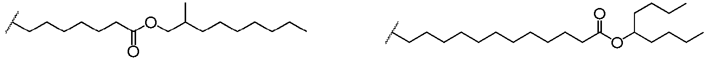

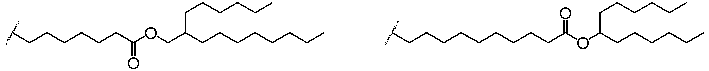

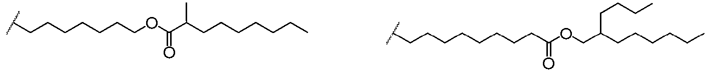

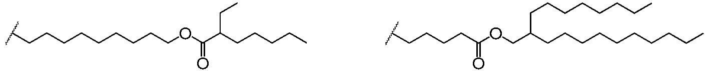

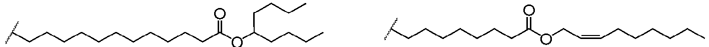

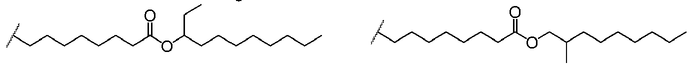

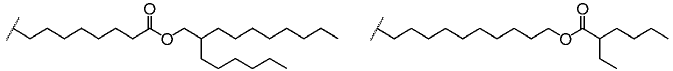

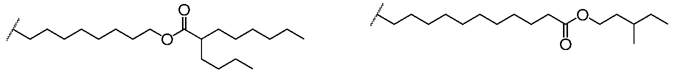

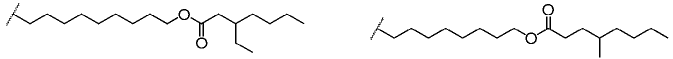

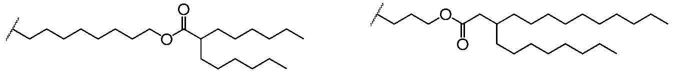

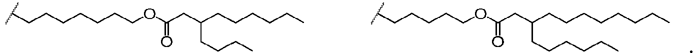

Herein, the substituted or unsubstituted hydrocarbyl, substituted or unsubstituted heterohydrocarbyl, substituted or unsubstituted aryl or substituted or unsubstituted heteroaryl groups having fewer than 11 carbon atoms can be collectively referred to as a set of groups, i.e., group C. Therefore, in the lipid compounds of formula (I), R_{I,} R_{II,} R_{III} and R_{IV} can be each independently a substituted or unsubstituted hydrocarbyl, substituted or unsubstituted heterohydrocarbyl, substituted or unsubstituted aryl or substituted or unsubstituted heteroaryl group having fewer than 11 carbon atoms and selected from the group C. When any two of R_{I,} R_{II,} R_{III} and R_{IV} are substituted or unsubstituted hydrocarbyl, substituted or unsubstituted heterohydrocarbyl, substituted or unsubstituted aryl or substituted or unsubstituted heteroaryl groups having fewer than 11 carbon atoms and selected from the group B, they can be identical or different.

In some embodiments, the substituted or unsubstituted hydrocarbyl, substituted or unsubstituted heterohydrocarbyl, substituted or unsubstituted aryl or substituted or unsubstituted heteroaryl group selected from the group C has 1 to 11 carbon atoms, for example 1, 2, 3, 4, 5, 6, 7, 8, 9, 10 and 11 carbon atoms. In some embodiments, the substituted or unsubstituted hydrocarbyl, substituted or unsubstituted heterohydrocarbyl, substituted or unsubstituted aryl or substituted or unsubstituted heteroaryl group selected from the group C has for example 1 to 6 carbon atoms, 1 to 7 carbon atoms, 1 to 8 carbon atoms, 1 to 9 carbon atoms, 1 to 10 carbon atoms or 1 to 11 carbon atoms, or the number of carbon atoms it has in a range between any two numerical values within a range of 1 to 11.

In some embodiments, the substituted or unsubstituted hydrocarbyl, substituted or unsubstituted heterohydrocarbyl, substituted or unsubstituted aryl or substituted or unsubstituted heteroaryl group selected from the group C contains 1 to 5 heteroatoms independently selected from N, O and S, for example 1, 2, 3, 4 and 5 heteroatoms. In some embodiments, the substituted or unsubstituted hydrocarbyl, substituted or unsubstituted heterohydrocarbyl, substituted or unsubstituted aryl or substituted or unsubstituted heteroaryl group selected from the group C contains for example 1 to 2 heteroatoms, 1 to 3 heteroatoms or 1 to 4 heteroatoms, or the number of heteroatoms it has is in a range between any two numerical values of 1 to 5.

In some embodiments, the substituted or unsubstituted hydrocarbyl, substituted or unsubstituted heterohydrocarbyl, substituted or unsubstituted aryl or substituted or unsubstituted heteroaryl group selected from the group C has 1 to 11 atoms including carbon atoms and heteroatoms such as N, O and S, for example 1, 2, 3, 4, 5, 6, 7, 8, 9, 10 and 11 atoms. In some embodiments, the substituted or unsubstituted hydrocarbyl, substituted or unsubstituted heterohydrocarbyl, substituted or unsubstituted aryl or substituted or unsubstituted heteroaryl group selected from the group C has for example 1 to 6 atoms, 1 to 7 atoms, 1 to 8 atoms, 1 to 9 atoms, 1 to 10 atoms or 1 to 11 atoms, or the number of atoms it has is in a range between any two numerical values within a range of 1 to 11.

In some embodiments, the substituted or unsubstituted hydrocarbyl, substituted or unsubstituted heterohydrocarbyl, substituted or unsubstituted aryl or substituted or unsubstituted heteroaryl group selected from the group C is optionally substituted with one or more substituents selected from the following group: deuterium, halogen, -NO₂ and -OH.

In some embodiments, the substituted or unsubstituted hydrocarbyl comprises linear, branched or cyclic alkyl, alkenyl and alkynyl. In some embodiments, the substituted or unsubstituted hydrocarbyl contains one or more for example 1, 2, 3, 4 or 5 unsaturated carbon-carbon double bond (-C=C-) and carbon-carbon triple bond (-C=C-) groups, and/or any combinations thereof. In some embodiments, the substituted or unsubstituted heterohydrocarbyl comprises linear, branched or cyclic heteroalkyl, heteroalkenyl and heteroalkynyl containing heteroatoms selected from N, O and S. In some embodiments, the heteroatom contained in the substituted or unsubstituted heterohydrocarbyl together with carbon atoms forms a main chain of a heteroaliphatic group, for example but not limited to group structures such as -C-N-C-, -C-O-C-, -C-O-O-C, -C-S-C- and -C-S-S-C, or any combinations thereof. In some embodiments, the heteroatom contained in the substituted or unsubstituted heterohydrocarbyl can be a substituent linked to the carbon atom, for example but not limited to substituent structures such as -C≡N, -C=N-, -C-N=, -C=O, -C-OH, -C=S and -C-SH. In some of the embodiments, the heteroatoms contained in the substituted or unsubstituted heterohydrocarbyl can be any combinations of the above listed group structures. In some embodiments, the heteroaliphatic group contains one or more for example 1, 2, 3, 4 or 5 unsaturated carbon-carbon double bond (-C=C-), carbon-carbon triple bond 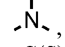 (-C≡C-), , -NH-, -NH₂, -OH, -OR^{m}', -O-, -C(O)-, -C(ORⁿ')-, -C(O)O-, -SH, -SR^{o}', -S-, -C(S)-, -C(SR^{p}')-, -C(S)O- and -P(O)- groups, wherein R^{m}', Rⁿ', R^{o}' and R^{p}' are each independently substituted or unsubstituted C₁-C₁₄ aliphatic hydrocarbyl, for example C₁-C₁₂, C₁-C₁₀, C₁-C₈, C₁-C₆ and C₁-C₄ aliphatic hydrocarbyl. Non-limiting specific examples of the substituted or unsubstituted C₁-C₁₄ aliphatic hydrocarbyl include but are not limited to methyl, ethyl, n-propyl and isopropyl, n-butyl, p-butyl, isobutyl, tert-butyl, neopentyl, cyclopropyl, cyclobutyl, cyclopentyl, cyclohexyl, ethylidene (vinyl), allyl, butenyl, pentenyl, 1-methyl-2-buten-1-yl, 5-hexenyl, ethynyl, 1-propinyl, 2-propinyl, etc.

In some embodiments, the group C comprises substituted or unsubstituted hydrocarbyl, substituted or unsubstituted heterohydrocarbyl, substituted or unsubstituted aryl or substituted or unsubstituted heteroaryl groups having fewer than 11 as shown in the following structures:

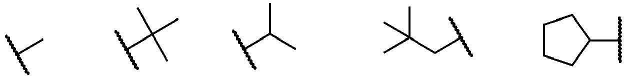

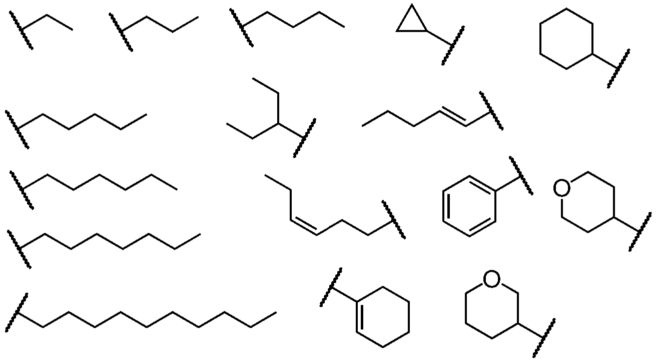

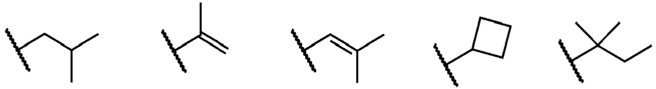

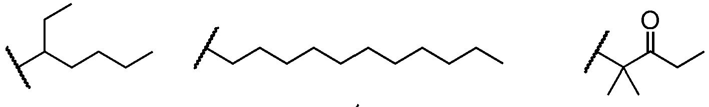

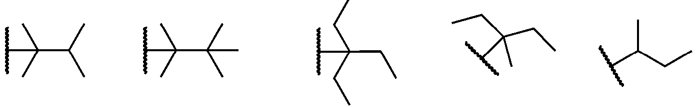

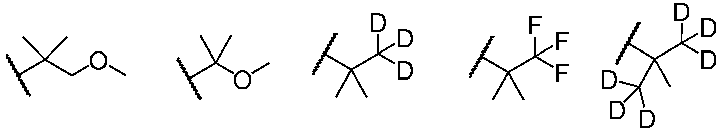

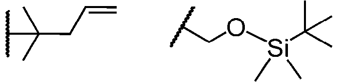

In some embodiments, R_{I} is selected from the group A, R_{III} and R_{IV} are selected from the group B, and R_{II} is selected from the group C; or
R_{I} is selected from the group A, R_{II} and R_{IV} are selected from the group B, and R_{III} is selected from the group C; or
R_{II} is selected from the group A, R_{I} and R_{IV} are selected from the group B, and R_{III} is selected from the group C; or
R_{III} is selected from the group A, R_{I} and R_{IV} are selected from the group B, and R_{II} is selected from the group C; or
R_{IV} is selected from the group A, R_{I} and R_{II} are selected from the group B, and R_{III} is selected from the group C; or
R_{IV} is selected from the group A, R_{I} and R_{III} are selected from the group B, and R_{II} is selected from the group C.

In some embodiments, R_{I} is selected from the group A, R_{II} and R_{III} are selected from the group B, and R_{IV} is selected from the group C; or
R_{II} is selected from the group A, R_{I} and R_{III} are selected from the group B, and R_{IV} is selected from the group C; or
R_{II} is selected from the group A, R_{III} and R_{IV} are selected from the group B, and R_{I} is selected from the group C; or
R_{III} is selected from the group A, R_{I} and R_{II} are selected from the group B, and R_{IV} is selected from the group C; or
R_{III} is selected from the group A, R_{II} and R_{IV} are selected from the group B, and R_{I} is selected from the group C; or
R_{IV} is selected from the group A, R_{II} and R_{III} are selected from the group B, and R_{I} is selected from the group C.

In some embodiments, R_{I}, R_{II}, R_{III} and R_{IV} each independently and optionally contain at least one degradable group. The lipid compound of the present disclosure preferably contains a degradable group. When entering cells, tissues or organs, the degradable group contained in the lipid compound of the present disclosure is broken, so that the lipid compound is partially or completely degraded, thereby reducing or completely eliminating the toxicity of the lipid compound on cells. The introduction of the degradable group can accelerate the metabolism of lipids in organs such as liver, thereby reducing the accumulation of lipids in the body and decreasing the potential toxicity.

In some embodiments, the degradable group is selected from -C(O)O-, -OC(O)-, -OC(O)O-, -S-S-, -C(O)NH-, -NHC(O)-, -NHC(O)O-, -NR¹C(O)-, -C(O)NR²-, -NR³C(O)O-, -OP(O)OR⁴O-, -OCR⁵(OR⁶)O-, -CR⁷(OR⁸)O- and -CH(OR⁹)O-, wherein R¹, R ², R³, R⁴, R⁵, R⁶, R⁷, R⁸ and R⁹ are each independently substituted or unsubstituted C₁-C₁₄ aliphatic hydrocarbyl, comprising linear, branched and cyclic alkyl, alkenyl, alkynyl or polyunsaturated hydrocarbyl, for example C₁-C₁₂, C₁-C₁₀, C₁-C₈, C₁-C₆ and C₁-C₄ aliphatic hydrocarbyl. Non-limiting specific examples of the substituted or unsubstituted C₁-C₁₄ aliphatic hydrocarbyl include but are not limited to methyl, ethyl, n-propyl and isopropyl, n-butyl, p-butyl, isobutyl, tert-butyl, neopentyl, cyclopropyl, cyclobutyl, cyclopentyl, cyclohexyl, ethylidene (vinyl), allyl, butenyl, pentenyl, 1-methyl-2-buten-1-yl, 5-hexenyl, acetenyl, 1-propinyl, 2-propinyl, etc.

In some embodiments, at least one of R_{I}, R_{II}, R_{III} and R_{IV} contains at least one degradable group. In some embodiments, when R_{III} and R_{IV} are selected from the group B, at least one of R_{III} and R_{IV} contains at least one degradable group; when R_{II} and R_{IV} are selected from the group B, at least one of R_{II} and R_{IV} contains at least one degradable group; when R_{I} and R_{IV} are selected from the group B, at least one of R_{I} and R_{IV} contains at least one degradable group; when R_{I} and R_{II} are selected from the group B, at least one of R_{I} and R_{II} contains at least one degradable group; when R_{I} and R_{III} are selected from the group B, at least one of R_{I} and R_{III} contains at least one degradable group; and/or, when R_{II} and R_{III} are selected from the group B, at least one of R_{II} and R_{III} contains at least one degradable group.

In some embodiments, only one of R_{I}, R_{II}, R_{III} and R_{IV} contains at least one degradable group. In some embodiments, when R_{III} and R_{IV} are selected from the group B, only one of R_{III} and R_{IV} contains at least one degradable group; when R_{II} and R_{IV} are selected from the group B, only one of R_{II} and R_{IV} contains at least one degradable group; when R_{I} and R_{IV} are selected from the group B, only one of R_{I} and R_{IV} contains at least one degradable group; when R_{I} and R_{II} are selected from the group B, only one of R_{I} and R_{II} contains at least one degradable group; when R_{I} and R_{III} are selected from the group B, only one of R_{I} and R_{III} contains at least one degradable group; and/or, when R_{II} and R_{III} are selected from the group B, only one of R_{II} and R_{III} contains at least one degradable group. In some embodiments, the degradable group is an ester group, i.e., -C(O)-O-; or the degradable group comprises an ester bond.

In some embodiments, only one of R_{I}, R_{II}, R_{III} and R_{IV} contains one degradable group. In some embodiments, when R_{III} and R_{IV} are selected from the group B, only one of R_{III} and R_{IV} contains one degradable group; when R_{II} and R_{IV} are selected from the group B, only one of R_{II} and R_{IV} contains one degradable group; when R_{I} and R_{IV} are selected from the group B, only one of R_{I} and R_{IV} contains one degradable group; when R_{I} and R_{II} are selected from the group B, only one of R_{I} and R_{II} contains one degradable group; when R_{I} and R_{III} are selected from the group B, only one of R_{I} and R_{III} contains one degradable group; and/or, when R_{II} and R_{III} are selected from the group B, only one of R_{II} and R_{III} contains one degradable group. In some embodiments, the degradable group is an ester group, i.e., -C(O)-O-; or the degradable group comprises an ester bond.

In some embodiments, when being selected from the group B, at least one of R_{I}, R_{II}, R_{III} and/or R_{IV} contains at least one ester group or ester bond. In some embodiments, when R_{I} and R_{IV} are selected from the group B, R_{I} and/or R_{IV} contains at least one ester group or ester bond. In some embodiments, when being selected from the group B, only one of R_{I}, R_{II}, R_{III} and/or R_{IV} contains one ester group or ester bond. In some embodiments, when R_{I} and R_{IV} are selected from the group B, only one of R_{I} and R_{IV} contains one ester group or ester bond, for example R_{I} contains one ester group or ester bond, or R_{IV} contains one ester group or ester bond.

In some embodiments, R_{II}' is hydrogen, or is selected from the group C, R_{III} is selected from the group A, R_{II} is selected from the group C, R_{I} and R_{IV} are selected from the group B, and the group A, the group B and the group C are as defined herein.

Preferably, at least one or only one of R_{I} and R_{IV} contains a degradable group, for example the degradable group is an ester group, i.e., -C(O)-O-; or the degradable group comprises an ester bond.

Preferably, when R_{I} and/or R_{IV} contains no degradable group, R_{I} and/or R_{IV} is selected from groups as shown in the following structures:

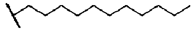

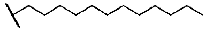

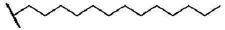

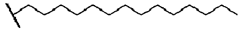

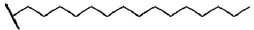

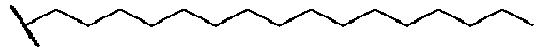

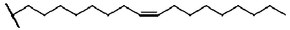

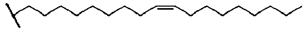

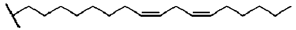

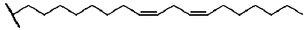

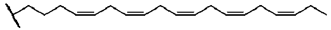

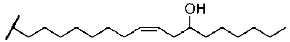

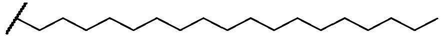

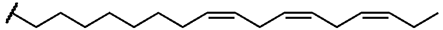

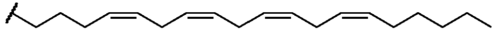

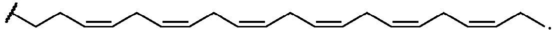

Preferably, when R_{I} and/or R_{IV} contains a degradable group, R_{I} and/or R_{IV} is selected from groups as shown in the following structures:

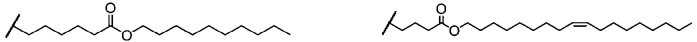

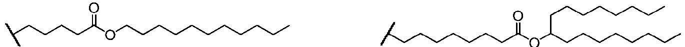

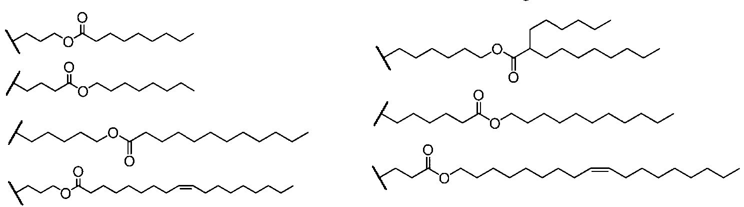

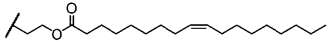

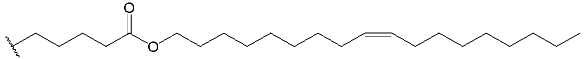

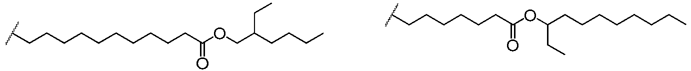

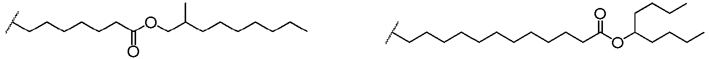

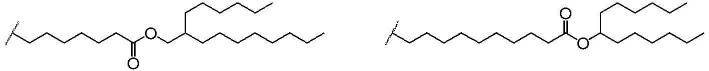

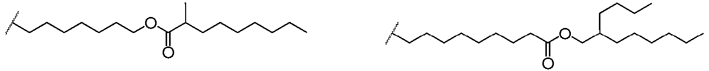

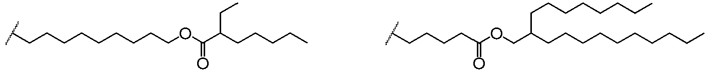

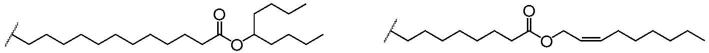

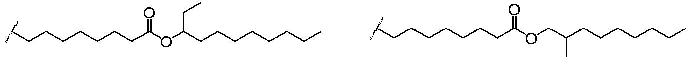

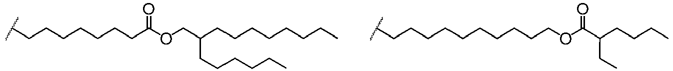

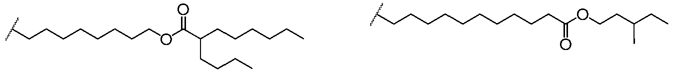

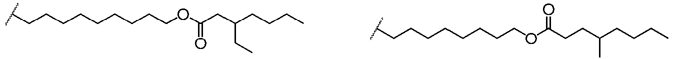

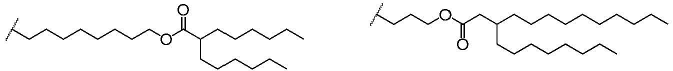

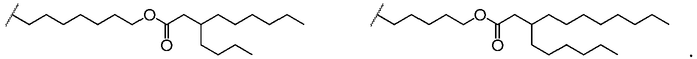

Preferably, when R^{I} and/or R_{IV} contains a degradable group, R_{I} and/or R_{IV} has a structure as shown below: 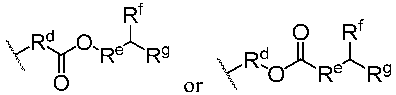
wherein R^{d} is substituted or unsubstituted hydrocarbylene or substituted or unsubstituted heterohydrocarbylene having 1 to 11 carbon atoms, the hydrocarbylene or heterohydrocarbylene is optionally substituted with one or more substituents selected from the following group: deuterium, halogen, -NO₂ and -OH, preferably R^{d} is substituted or unsubstituted hydrocarbylene having 1 to 11 carbon atoms, for example alkylene, more preferably R^{d} is substituted or unsubstituted linear hydrocarbylene having 1 to 11 carbon atoms, for example linear alkylene, and the carbon atom number of the linear alkylene is for example 1 to 11, 2 to 11, 2 to 10, 3 to 10, etc.
R^{e} is absent, or is substituted or unsubstituted hydrocarbylene or substituted or unsubstituted heterohydrocarbylene having 1 to 4 carbon atoms, and the hydrocarbylene or heterohydrocarbylene is optionally substituted with one or more substituents selected from the following group: deuterium, halogen, -NO₂ and -OH, preferably R^{e} is absent, or is substituted or unsubstituted linear hydrocarbylene having 1 to 4 carbon atoms, for example linear alkylene, the carbon atom number of the linear alkylene is for example 1 to 4, 1 to 3 and 1 to 2, for example R^{e} is absent, or is methylidene, ethylidene, propylidene or butylidene, preferably R^{e} is absent, or is methylidene or ethylidene.
R^{f} and R^{g} are each independently substituted or unsubstituted hydrocarbyl or substituted or unsubstituted heterohydrocarbyl having 1 to 11 carbon atoms, and the hydrocarbyl or heterohydrocarbyl is optionally substituted with one or more substituents selected from the following group: deuterium, halogen, -NO₂ and -OH, preferably R^{f} and R^{g} are each independently substituted or unsubstituted hydrocarbyl having 1 to 11 carbon atoms, for example alkyl, more preferably R^{f} and R^{g} are each independently substituted or unsubstituted linear hydrocarbyl having 1 to 11 carbon atoms, for example linear alkyl, the carbon atom number of the linear alkyl is for example 1 to 11, 1 to 10, 1 to 9, 1 to 8, etc., for example R^{f} and R^{g} are each independently methyl, ethyl, propyl, butyl, pentyl, hexyl, heptyl, octyl, nonyl, decyl or hendecyl, and R^{f} and R^{g} can be identical or different.

Preferably, at least one or only one, such as R_{I} or R_{IV}, of R_{I} and R_{IV} is selected from the group consisting of the following groups:

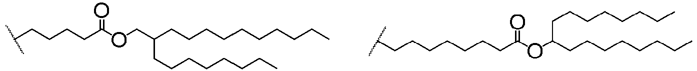

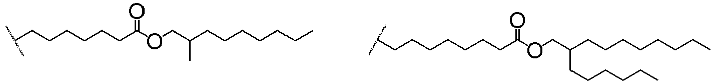

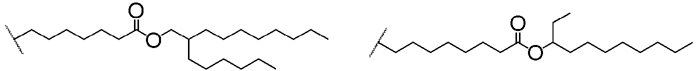

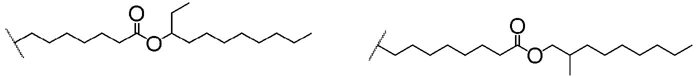

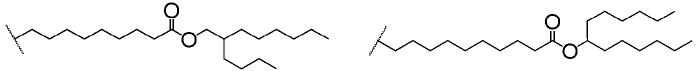

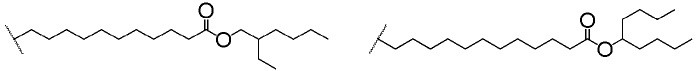

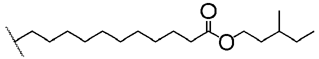

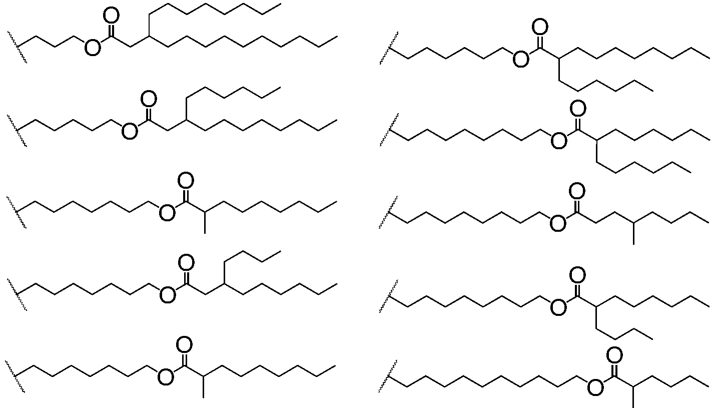

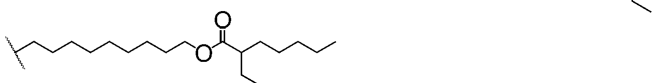

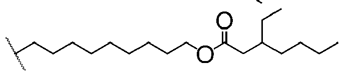

In some embodiments, the lipid compound of the present disclosure has the following structural formula (I_{A}), 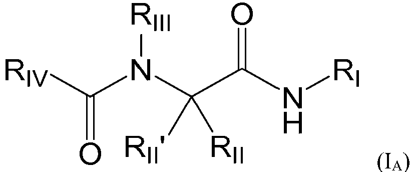 wherein R_{II}', R_{I}, R_{II}, R_{III} and R_{IV} are as defined in the present disclosure.

The group A, the group B and the group C are as defined in the present disclosure.

In some embodiments, R_{II}' is hydrogen, or substituted or unsubstituted hydrocarbyl, substituted or unsubstituted heterohydrocarbyl, substituted or unsubstituted aryl or substituted or unsubstituted heteroaryl having 1 to 11 carbon atoms, for example R_{II}' is hydrogen or is selected from the group C, for example hydrocarbyl having 1 to 8 such as 1 to 6 and 1 to 4 carbon atoms, specifically for example C₁-C₆ alkyl and C₁-C₄ alkyl, including but not limited to methyl, ethyl, propyl, isopropyl, n-butyl, p-butyl, sec-butyl, tert-butyl, pentyl, hexyl, etc.

In some embodiments, R_{II}' is hydrogen.

In some embodiments, R_{II}' is selected from the group C, for example hydrocarbyl having 1 to 8 such as 1 to 6 and 1 to 4 carbon atoms, specifically for example C₁-C₆ alkyl and C₁-C₄ alkyl, including but not limited to methyl, ethyl, propyl, isopropyl, n-butyl, p-butyl, sec-butyl, tert-butyl, pentyl, hexyl, etc.

In some embodiments, R_{III} is selected from the group A.

In some embodiments, R_{III} is selected from the group A, and the group containing at least one ionizable tertiary amine structure in the group A is a group having the following general formula: 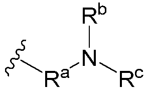 wherein R^{a} is substituted or unsubstituted C₁-C₆ such as C₁-C₄ alkylene; R^{b} and R^{c} are each independently substituted or unsubstituted C₁-C₆ such as C₁-C₄ alkyl, substituted or unsubstituted C₂-C₆ such as C₂-C₄ alkenyl and substituted or unsubstituted C₂-C₆ such as C₂-C₄ alkynyl, which are optionally substituted with 1, 2 or 3 substituents selected from -OH, -SH, -NR^{d}R^{d'}, or phenyl, wherein R^{d} and R^{d}' are each independently hydrogen or C₁-C₃ alkyl; or R^{b} and R^{c} together with the N atom to which they are attached form a 5- to 12-membered heterocyclic ring or heteroaromatic ring, the 5- to 12-membered heterocyclic ring or heteroaromatic ring containing 1, 2 or 3 heteroatoms independently selected from N, O and S, wherein at least one heteroatom is N, and the 5- to 12-membered heterocyclic ring or heteroaromatic ring is optionally substituted with one or more C₁-C₆ such as C₁-C₄ alkyls or oxo (=O); or R^{a} and R^{b} together with the N atom to which they are attached form a 5- to 12-membered heterocyclic ring or heteroaromatic ring optionally substituted with C₁-C₆ such as C₁-C₄ alkylene, the 5-to 12-membered heterocyclic ring or heteroaromatic ring optionally substituted with alkylene containing 1, 2 or 3 heteroatoms independently selected from N, O and S, wherein at least one heteroatom is N, and the 5- to 12-membered heterocyclic ring or heteroaromatic ring optionally substituted with alkylene is optionally substituted with one or more C₁-C₆ such as C₁-C₄ alkyls or oxo (=O); or R^{a}, R^{b} and R^{c} together with the N atom to which they are attached form a 5- to 12-membered heterocyclic ring or heteroaromatic ring optionally substituted with C₁-C₆ such as C₁-C₄ alkylene, the 5-to 12-membered heterocyclic ring or heteroaromatic ring optionally substituted with alkylene containing 1, 2 or 3 heteroatoms selected from N, O and S, wherein at least one heteroatom is N, and the 5- to 12-membered heterocyclic ring or heteroaromatic ring optionally substituted with alkylene is optionally substituted with one or more C₁-C₆ such as C₁-C₄ alkyls or oxo (=O).

In some embodiments, R^{b} and R^{c} together with the N atom to which they are attached form a 5- to 12- such as 5- to 6-membered heterocyclic ring or heteroaromatic ring, the 5- to 12- such as 5- to 6-membered heterocyclic ring or heteroaromatic ring can be monocyclic, fused bicyclic, spirocyclic bicyclic or bridged bicyclic, etc. In some embodiments, R^{b} and R^{c} together with the N atom to which they are attached form a 5-, 6-, 7- or 8-membered monocyclic heterocyclic ring or heteroaromatic ring which contains 1, 2 or 3 heteroatoms selected from N, O and S, wherein at least one heteroatom is N. In some embodiments, non-limiting examples of the heterocyclic ring or heteroaromatic ring formed by R^{b} and R^{c} together with the N atom to which they are attached include but are not limited to the following heterocyclic rings and heteroaromatic rings:

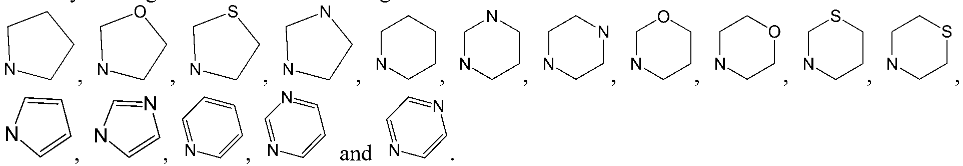

In some embodiments, R^{a} and R^{b} together with the N atom to which they are attached form a 5- to 12- such as 5- to 6-membered heterocyclic ring or heteroaromatic ring optionally substituted with C₁-C₆ such as C₁-C₄ alkylene, the 5- to 12- such as 5- to 6-membered heterocyclic ring or heteroaromatic ring optionally substituted with alkylene can be monocyclic, fused bicyclic, spirocyclic bicyclic or bridged bicyclic, etc. In some embodiments, R^{a} and R^{b} together with the N atom to which they are attached form a 5-, 6-, 7- or 8-membered monocyclic heterocyclic ring or heteroaromatic ring optionally substituted with C₁-C₆ such as C₁-C₄ alkylene which contains 1, 2 or 3 heteroatoms selected from N, O and S, wherein at least one heteroatom is N. In some embodiments, non-limiting examples of the 5- to 12-such as 5- to 6-membered heterocyclic ring or heteroaromatic ring optionally substituted with C₁-C₆ such as C₁-C₄ alkylene formed by R^{a} and R^{b} together with the N atom to which they are attached include but are not limited to the following heterocyclic rings: * represents a position linked to other parts in a compound molecule,

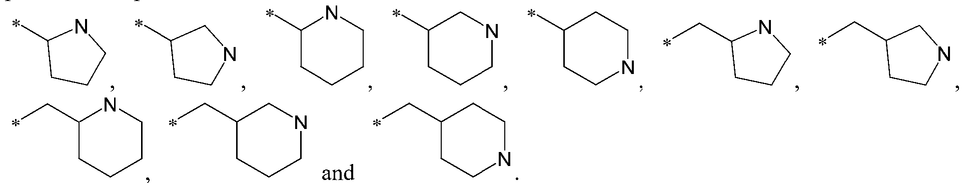

In some embodiments, R^{a}, R^{b} and R^{c} together with the N atom to which they are attached form a 5-to 12- such as 5- to 6-membered heterocyclic ring or heteroaromatic ring optionally substituted with C₁-C₆ such as C₁-C₄ alkylene, the 5- to 12- such as 5- to 6-membered heterocyclic ring or heteroaromatic ring optionally substituted with alkylene can be monocyclic, fused bicyclic, spirocyclic bicyclic or bridged bicyclic, etc. In some embodiments, R^{a}, R^{b} and R^{c} together with the N atom to which they are attached form a 5-, 6-, 7- or 8-membered monocyclic heterocyclic ring or heteroaromatic ring optionally substituted with C₁-C₆ such as C₁-C₄ alkylene which contains 1, 2 or 3 heteroatoms selected from N, O and S, wherein at least one heteroatom is N. In some embodiments, non-limiting examples of the 5- to 12- such as 5- to 6-membered heterocyclic ring or heteroaromatic ring optionally substituted with C₁-C₆ such as C₁-C₄ alkylene formed by R^{a}, R^{b} and R^{c} together with the N atom to which they are attached include but are not limited to the following heteroaromatic rings: * represents a position linked to other parts in a compound molecule,

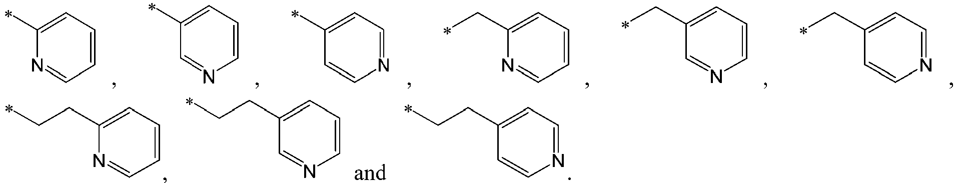

In some embodiments, R_{III} is selected from the group A, and the group A comprises groups containing at least one ionizable tertiary amine structure as shown in the following structures:

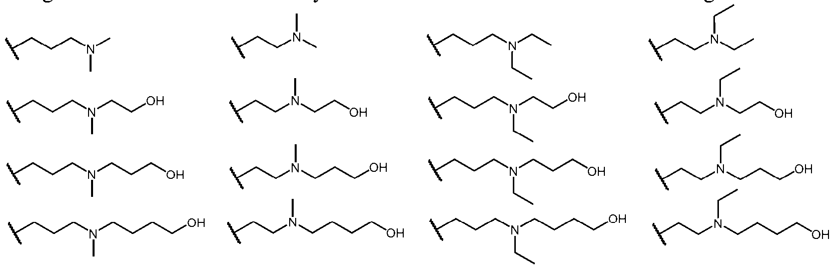

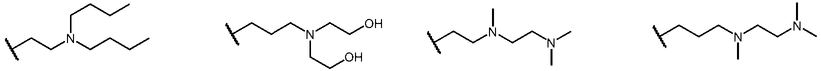

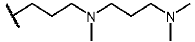

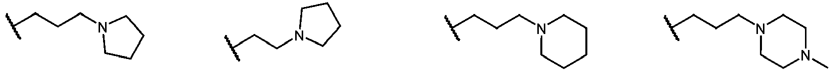

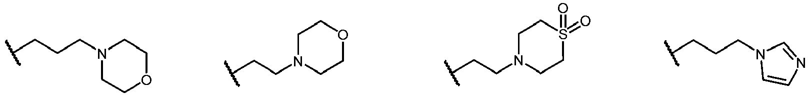

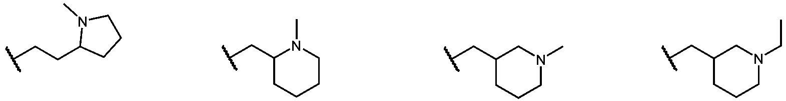

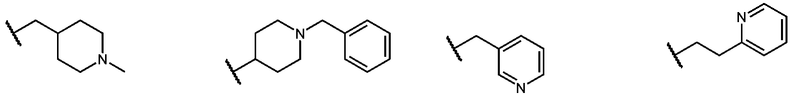

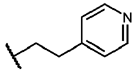

In some embodiments, R_{I} and R_{IV} are each independently selected from the group B.

In some embodiments, R_{I} and R_{IV} are each independently selected from the group B, the substituted or unsubstituted aliphatic or substituted or unsubstituted heteroaliphatic group selected from the group B has 11 to 30 carbon atoms, for example 11, 12, 13, 14, 15, 16, 17, 18, 19, 20, 21, 22, 23, 24, 25, 26, 27, 28, 29 and 30 carbon atoms. In some embodiments, the substituted or unsubstituted aliphatic or substituted or unsubstituted heteroaliphatic group selected from the group B has for example 11 to 16 carbon atoms, 11 to 25 carbon atoms, 16 to 25 carbon atoms, 16 to 30 carbon atoms or 16 to 30 carbon atoms, or the number of carbon atoms it has is in a range between any two numerical values within a range of 11 to 30.

In some embodiments, R_{I} and R_{IV} are each independently selected from the group B, the substituted or unsubstituted heteroaliphatic group selected from the group B contains 1 to 8 heteroatoms independently selected from N, O and S, for example 1, 2, 3, 4, 5, 6, 7 and 8 heteroatoms. In some embodiments, the substituted or unsubstituted heteroaliphatic group selected from the group B contains for example 1 to 2 heteroatoms, 1 to 3 heteroatoms, 1 to 4 heteroatoms or 1 to 5 heteroatoms, or has a heteroatom number being in a range between any two numerical values within a range of 1 to 8.

In some embodiments, R_{I} and R_{IV} are each independently selected from the group B, the substituted or unsubstituted heteroaliphatic group selected from the group B has 11 to 30 atoms including carbon atoms and heteroatoms such as N, O and S, for example 11, 12, 13, 14, 15, 16, 17, 18, 19, 20, 21, 22, 23, 24, 25, 26, 27, 28, 29 and 30 atoms. In some embodiments, the substituted or unsubstituted heteroaliphatic group selected from the group B has for example 11 to 16 atoms, 11 to 25 atoms, 16 to 25 atoms or 16 to 30 atoms, or the number of atoms it has is in a range between any two numerical values within a range of 11 to 30.

In some embodiments, R_{I} and R_{IV} are each independently selected from the group B, and the group B comprises substituted or unsubstituted aliphatic or substituted or unsubstituted heteroaliphatic groups having 11 to 30 carbon atoms as shown in the following structures:

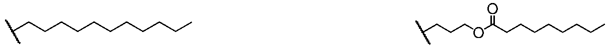

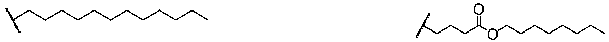

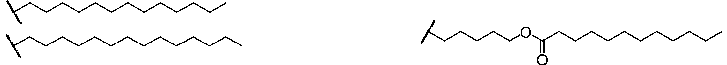

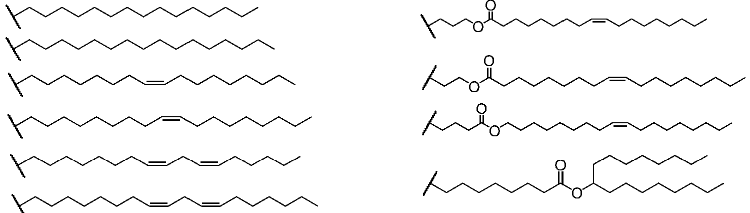

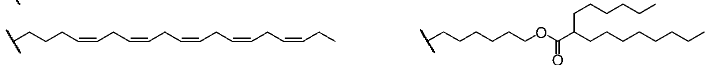

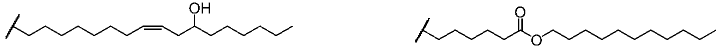

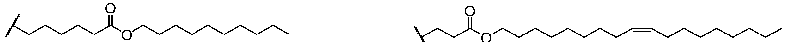

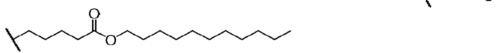

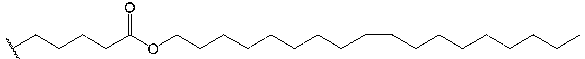

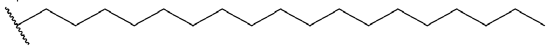

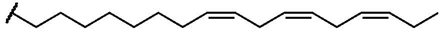

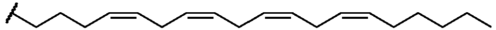

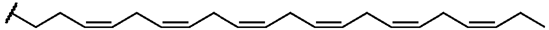

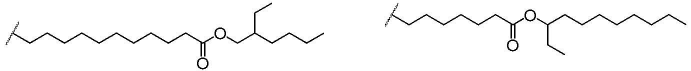

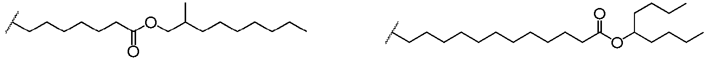

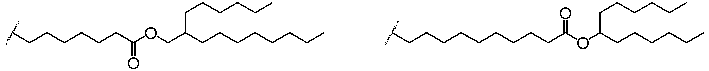

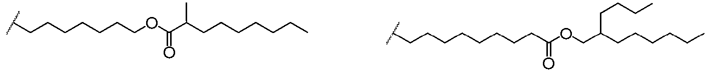

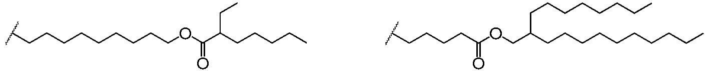

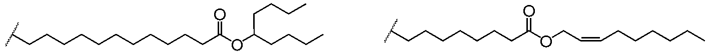

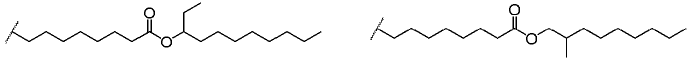

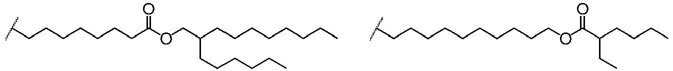

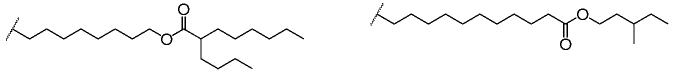

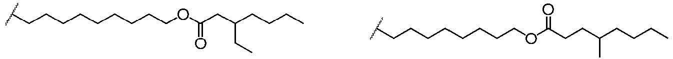

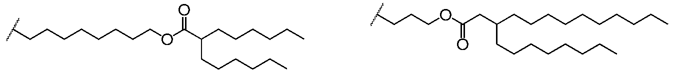

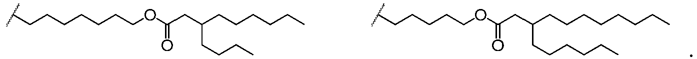

In some embodiments, one of R^{I} and R_{IV} has 16 to 30 carbon atoms or 16 to 30 total atoms, and the other has 11 to 25 carbon atoms or 11 to 25 total atoms, preferably, one of R_{I} and R_{IV} has 16 to 25 carbon atoms or 16 to 25 total atoms, and the other has 11 to 16 carbon atoms or 11 to 16 total atoms.

In some embodiments, at least one of R_{I} and R_{IV} contains at least one degradable group.

In some embodiments, the degradable group is selected from -C(O)O-, -OC(O)-, -OC(O)O-, -S-S-, -C(O)NH-, -NHC(O)-, -NHC(O)O-, -NR¹C(O)-, -C(O)NR²-, -NR³C(O)O-, -OP(O)OR⁴O-, -OCR⁵(OR⁶)O-, -CR⁷(OR⁸)O- and -CH(OR⁹)O-, wherein R¹, R ², R³, R⁴, R⁵, R⁶, R⁷, R⁸ and R⁹ are each independently substituted or unsubstituted C₁-C₁₄ such as C₁-C₁₀, C₁-C₆ and C₁-C₄ aliphatic hydrocarbyl, preferably, the degradable group is an ester group, i.e., -C(O)O- or -OC(O)-; or the degradable group comprises an ester bond.

In some embodiments, R_{I} and R_{IV} each contain at least one degradable group, for example the degradable group is an ester group, i.e., -C(O)O- or -OC(O)-; or the degradable group comprises an ester bond.

In some embodiments, R_{I} and R_{IV} each contain one degradable group, for example the degradable group is an ester group, i.e., -C(O)O- or -OC(O)-; or the degradable group comprises an ester bond.

In some embodiments, only one of R_{I} and R_{IV} contains at least one degradable group, for example the degradable group is an ester group, i.e., -C(O)O- or -OC(O)-; or the degradable group comprises an ester bond.

In some embodiments, one of R_{I} and R_{IV} contains at least one degradable group, for example the degradable group is an ester group, i.e., -C(O)O- or -OC(O)-; or the degradable group comprises an ester bond, and the other contains no degradable group.

In some embodiments, only one of R_{I} and R_{IV} contains one degradable group, for example the degradable group is an ester group, i.e., -C(O)O- or -OC(O)-; or the degradable group comprises an ester bond.

In some embodiments, one of R_{I} and R_{IV} contains one degradable group, for example the degradable group is an ester group, i.e., -C(O)O- or -OC(O)-; or the degradable group comprises an ester bond, and the other contain no degradable group.

In some embodiments, when R_{I} and/or R_{IV} contains no degradable group, R_{I} and/or R_{IV} is selected from groups as shown in the following structures:

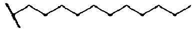

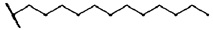

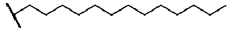

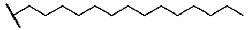

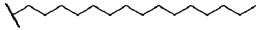

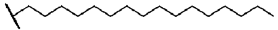

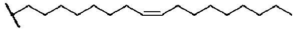

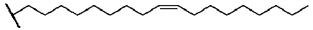

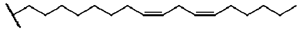

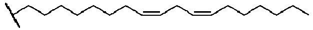

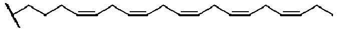

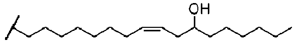

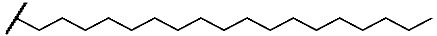

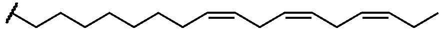

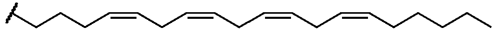

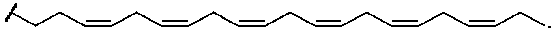

In some embodiments, when R_{I} and/or R_{IV} contains a degradable group, R_{I} and/or R_{IV} is selected from groups as shown in the following structures:

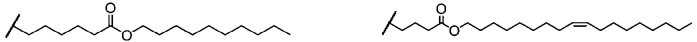

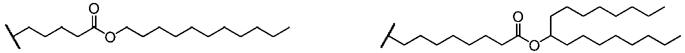

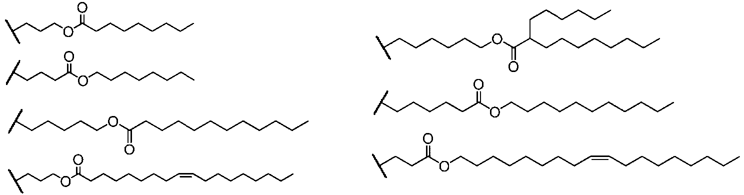

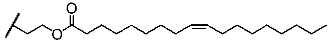

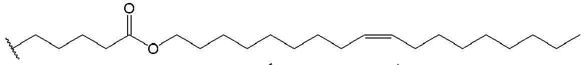

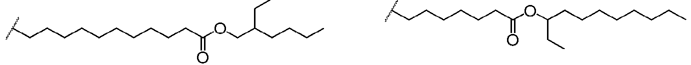

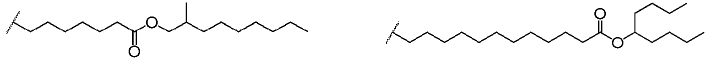

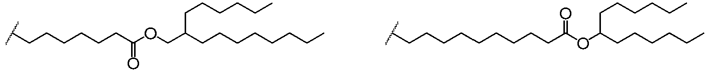

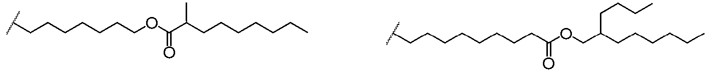

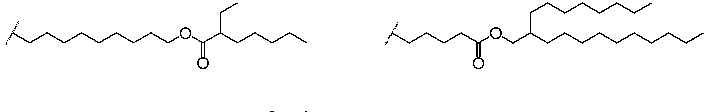

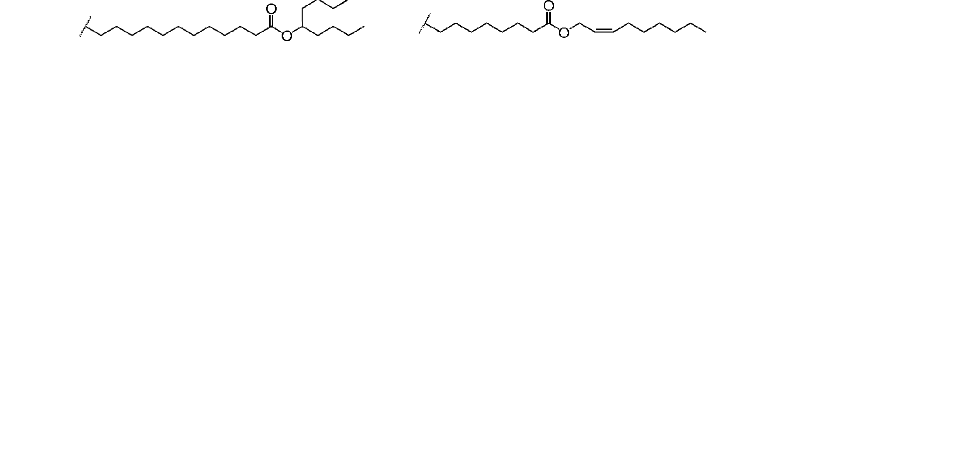

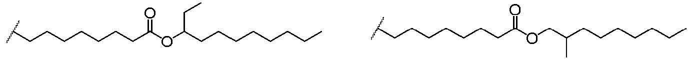

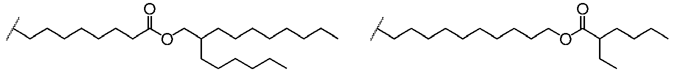

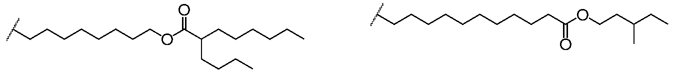

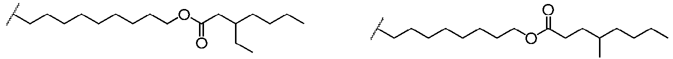

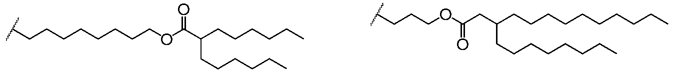

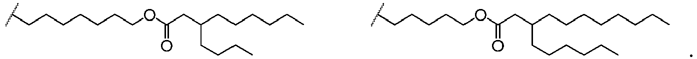

In some embodiments, when R_{I} and/or R_{IV} contains a degradable group, R_{I} and/or R_{IV} has a structure as shown below: 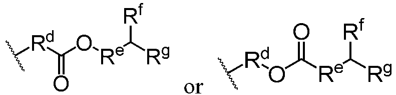
wherein R^{d} is substituted or unsubstituted hydrocarbylene or substituted or unsubstituted heterohydrocarbylene having 1 to 11 carbon atoms, and the hydrocarbylene or heterohydrocarbylene is optionally substituted with one or more substituents selected from the following group: deuterium, halogen, -NO₂ and -OH, preferably R^{d} is substituted or unsubstituted hydrocarbylene having 1 to 11 carbon atoms, for example alkylene, more preferably R^{d} is substituted or unsubstituted linear hydrocarbylene having 1 to 11 carbon atoms, for example linear alkylene, the number of carbon atoms in the linear alkylene is for example 1 to 11, 2 to 11, 2 to 10, 3 to 10, etc.;
R^{e} is absent, or is substituted or unsubstituted hydrocarbylene or substituted or unsubstituted heterohydrocarbylene having 1 to 4 carbon atoms, and the hydrocarbylene or heterohydrocarbylene is optionally substituted with one or more substituents selected from the following group: deuterium, halogen, -NO₂ and -OH, preferably R^{e} is absent, or is substituted or unsubstituted linear hydrocarbylene having 1 to 4 carbon atoms, for example linear alkylene, the number of carbon atom in the linear alkylene is for example 1 to 4, 1 to 3 and 1 to 2, for example R^{e} is absent, or is methylidene, ethylidene, propylidene or butylidene, preferably, R^{e} is absent, or is methylidene or ethylidene;
R^{f} and R^{g} are each independently substituted or unsubstituted hydrocarbyl or substituted or unsubstituted heterohydrocarbyl having 1 to 11 carbon atoms, and the hydrocarbyl or heterohydrocarbyl is optionally substituted with one or more substituents selected from the following group: deuterium, halogen, -NO₂ and -OH, preferably R^{f} and R^{g} are each independently substituted or unsubstituted hydrocarbyl having 1 to 11 carbon atoms, for example alkyl, more preferably R^{f} and R^{g} are each independently substituted or unsubstituted linear hydrocarbyl having 1 to 11 carbon atoms, for example linear alkyl, the number of the carbon atoms in the linear alkyl is for example 1 to 11, 1 to 10, 1 to 9, 1 to 8, etc., for example R^{f} and R^{g} are each independently methyl, ethyl, propyl, butyl, pentyl, hexyl, heptyl, octyl, nonyl, decyl or undecyl, and R^{f} and R^{g} can be identical or different.

In some embodiments, when R_{I} and/or R_{IV} contains a degradable group, R_{I} and/or R_{IV} is each independently selected from the group consisting of the following groups:

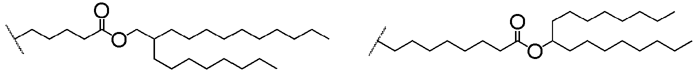

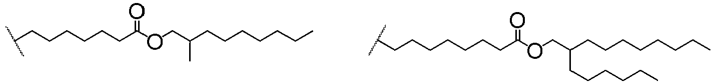

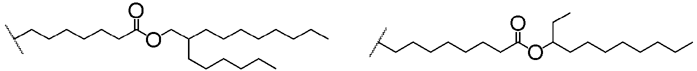

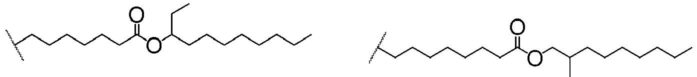

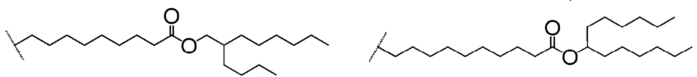

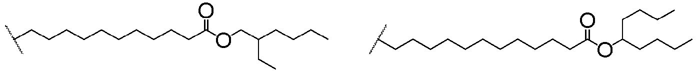

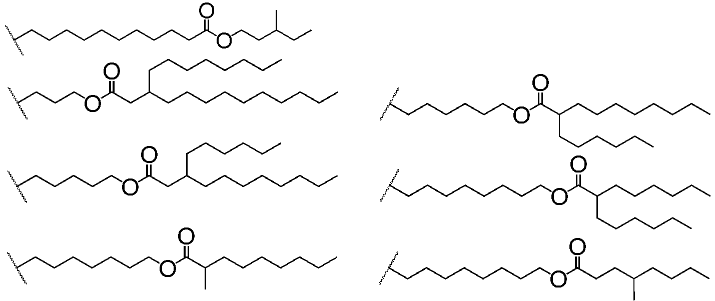

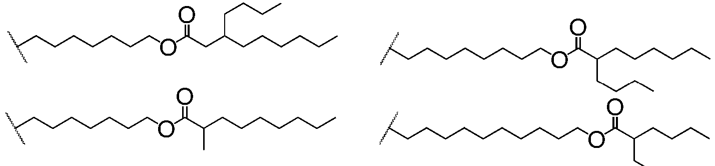

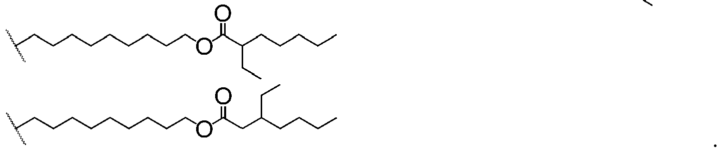

In some embodiments, R_{II} is selected from the group C.

In some embodiments, R_{II} is selected from the group C, and the substituted or unsubstituted hydrocarbyl, substituted or unsubstituted heterohydrocarbyl, substituted or unsubstituted aryl or substituted or unsubstituted heteroaryl group selected from the group C has 1 to 11 carbon atoms, for example 1, 2, 3, 4, 5, 6, 7, 8, 9 and 11 carbon atoms. In some embodiments, the substituted or unsubstituted hydrocarbyl, substituted or unsubstituted heterohydrocarbyl, substituted or unsubstituted aryl or substituted or unsubstituted heteroaryl group selected from the group C has for example 1 to 6 carbon atoms, 1 to 7 carbon atoms, 1 to 8 carbon atoms, 1 to 9 carbon atoms, 1 to 10 carbon atoms or 1 to 11 carbon atoms, or the number of carbon atoms it has is in a range between any two numerical values within a range of 1 to 11.

In some embodiments, R_{II} is selected from the group C, and the substituted or unsubstituted hydrocarbyl, substituted or unsubstituted heterohydrocarbyl, substituted or unsubstituted aryl or substituted or unsubstituted heteroaryl group selected from the group C contains 1 to 5 heteroatoms independently selected from N, O and S, for example 1, 2, 3, 4 and 5 heteroatoms. In some embodiments, the substituted or unsubstituted hydrocarbyl, substituted or unsubstituted heterohydrocarbyl, substituted or unsubstituted aryl or substituted or unsubstituted heteroaryl group selected from the group C contains for example 1 to 2 heteroatoms, 1 to 3 heteroatoms or 1 to 4 heteroatoms, or the number of carbon atoms it has is in a range between any two numerical values within a range of 1 to 5.

In some embodiments, R_{II} is selected from the group C, and the substituted or unsubstituted hydrocarbyl, substituted or unsubstituted heterohydrocarbyl, substituted or unsubstituted aryl or substituted or unsubstituted heteroaryl group selected from the group C has 1 to 11 atoms including carbon atoms and heteroatoms such as N, O and S, for example 1, 2, 3, 4, 5, 6, 7, 8, 9 and 11 atoms. In some embodiments, the substituted or unsubstituted hydrocarbyl, substituted or unsubstituted heterohydrocarbyl, substituted or unsubstituted aryl or substituted or unsubstituted heteroaryl group selected from the group C has for example 1 to 6 atoms, 1 to 7 atoms, 1 to 8 atoms, 1 to 9 atoms, 1 to 10 atoms or 1 to 11 atoms, or the number of atoms it has is in a range between any two numerical values within a range of 1 to 11.

In some embodiments, the substituted or unsubstituted hydrocarbyl, substituted or unsubstituted heterohydrocarbyl, substituted or unsubstituted aryl or substituted or unsubstituted heteroaryl group selected from the group C is optionally substituted with one or more substituents selected from the following group: deuterium, halogen, -NO₂ and -OH.

In some embodiments, the substituted or unsubstituted hydrocarbyl comprises linear, branched or cyclic alkyl, alkenyl and alkynyl. In some embodiments, the substituted or unsubstituted hydrocarbyl contains one or more for example 1, 2, 3, 4 or 5 unsaturated carbon-carbon double bond (-C=C-) and carbon-carbon triple bond (-C=C-) groups, and/or any combinations thereof. In some embodiments, the substituted or unsubstituted heterohydrocarbyl comprises linear, branched or cyclic heteroalkyl, heteroalkenyl and heteroalkynyl containing heteroatoms selected from N, O and S. In some embodiments, the heteroatom contained in the substituted or unsubstituted heterohydrocarbyl together with carbon atoms may form a main chain of a heteroaliphatic group, for example including but not limited to group structures such as -C-N-C-, -C-O-C-, -C-O-O-C, -C-S-C- and -C-S-S-C, or any combinations thereof. In some embodiments, the heteroatom contained in the substituted or unsubstituted heterohydrocarbyl may be a substituent linked to the carbon atoms, for example including but not limited to substituent structures such as -C≡N, -C=N-, -C-N=, -C=O, -C-OH, -C=S and -C-SH. In some of the embodiments, the heteroatom contained in the substituted or unsubstituted heterohydrocarbyl may be any combination of the above-mentioned listed group structures. In some embodiments, the heteroaliphatic group contains one or more for example 1, 2, 3, 4 or 5 unsaturated 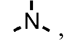 carbon-carbon double bond (-C=C-), carbon-carbon triple bond (-C≡C-), , -NH-, -NH₂, -OH, -OR^{m}', -O-, -C(O)-, -C(ORⁿ')-, -C(O)O-, -SH, -SR^{o}', -S-, -C(S)-, -C(SR^{p}')-, -C(S)O- and -P(O)- groups, wherein R^{m}', Rⁿ', R^{o}' and R^{p}' are each independently substituted or unsubstituted C₁-C₁₄ aliphatic hydrocarbyl, for example C₁-C₁₂, C₁-C₁₀, C₁-C₈, C₁-C₆ and C₁-C₄ aliphatic hydrocarbyl. Non-limiting specific examples of the substituted or unsubstituted C₁-C₁₄ aliphatic hydrocarbyl include but are not limited to methyl, ethyl, n-propyl and isopropyl, n-butyl, sec-butyl, isobutyl, tert-butyl, neopentyl, cyclopropyl, cyclobutyl, cyclopentyl, cyclohexyl, ethylidene (vinyl), propenyl, butenyl, pentenyl, 1-methyl-2-buten-1-yl, 5-hexenyl, ethynyl, 1-propynyl, 2-propynyl, etc.

In some embodiments, R_{II} is selected from the group C, and the group C comprises substituted or unsubstituted hydrocarbyl, substituted or unsubstituted heterohydrocarbyl, substituted or unsubstituted aryl or substituted or substituted heteroaryl group having fewer than 11 carbon atoms as shown in the following structures:

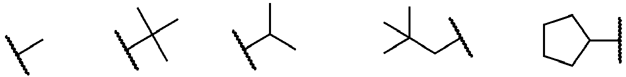

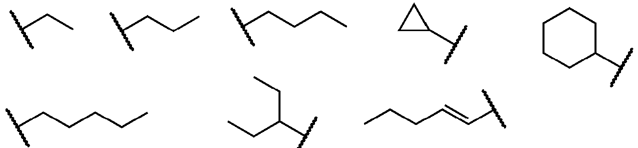

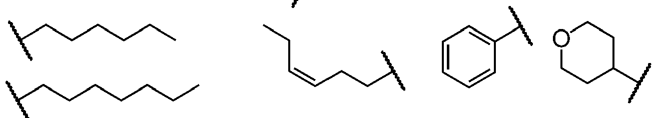

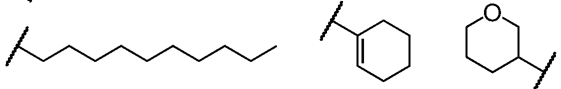

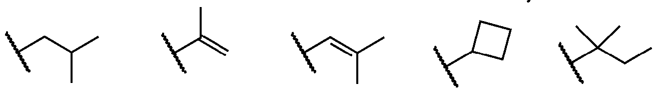

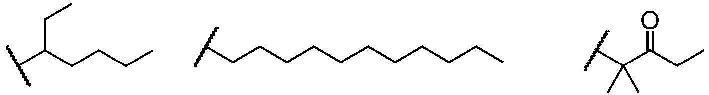

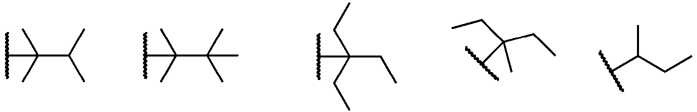

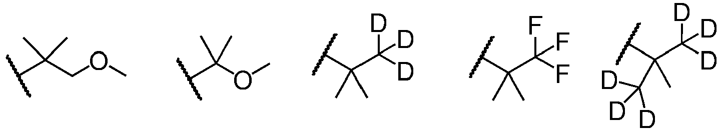

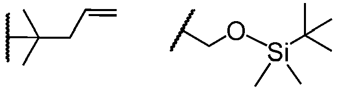

In some embodiments, R_{II} is tert-butyl.

In some embodiments, R_{III} is selected from the group A, R_{I} and R_{IV} are selected from the group B, and R_{II} is selected from the group C.

In some embodiments, R_{III} is selected from the group A, R_{I} and R_{IV} are selected from the group B, R_{II} is selected from the group C, and R_{II}' is hydrogen or is selected from the group C.

In some embodiments, R_{II}' is hydrogen or is selected from the group C, R_{III} is selected from the group A, R_{II} is selected from the group C, R_{I} and R_{IV} are selected from the group B, and the groups A, B and C are as defined herein; further, the ranges of R_{II}', R_{I}, R_{II}, R_{III} and R_{IV} can be preferably as defined for each group in these groups herein.

In some embodiments, the lipid of the present disclosure has the following structural formula (I_{A-1}), 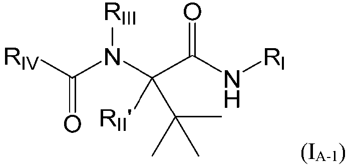 wherein R_{II}', R_{I}, R_{III} and R_{IV} are as defined in the present disclosure, for example the definition for the structural formula (I_{A}) includes the definition of each embodiment, which is not described in detail here.

In some embodiments, the lipid of the present disclosure has the following structural formula (I_{A-2}), 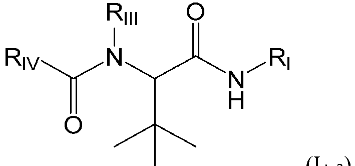 wherein R_{I}, R_{III} and R_{IV} are as defined in the present disclosure, for example the definition for the structural formula (I_{A}) includes the definition of each embodiment, which is not described in detail here.

In some embodiments, the lipid compound of the present disclosure has the following structural formula (I_{B}), 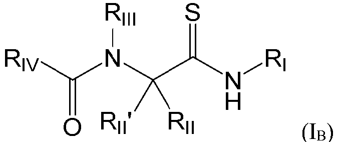 wherein R_{II}', R_{I}, R_{II}, R_{III} and R_{IV} are as defined in the present disclosure, for example the definition for the structural formula (I_{A}) includes the definition of each embodiment, which is not described in detail here.

In some embodiments, R_{II}' is hydrogen;
R_{II} is selected from the group C, for example C₁-C₆ alkyl and C₁-C₄ alkyl, including but not limited to methyl, ethyl, propyl, isopropyl, n-butyl, p-butyl, sec-butyl, tert-butyl, pentyl, hexyl, etc., also for example tert-butyl;
R_{III} is selected from the group A, for example a group having the following general formula: 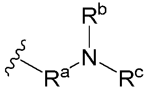
wherein R^{a}, R^{b} and R^{c} are each independently C₁-C₆ such as C₁-C₄ substituted or unsubstituted alkyl, which are optionally substituted with 1, 2 or 3 substituents selected from -OH, -SH, -NR^{d}R^{d'} or phenyl, wherein R^{d} and R^{d'} are each independently hydrogen or C₁-C₃ alkyl, for example R^{a}, R^{b} and R^{c} are each independently C₁-C₆ such as C₁-C₄ alkyl.

For example, the group A comprises groups containing at least one ionizable tertiary amine structure as shown in the following structures: 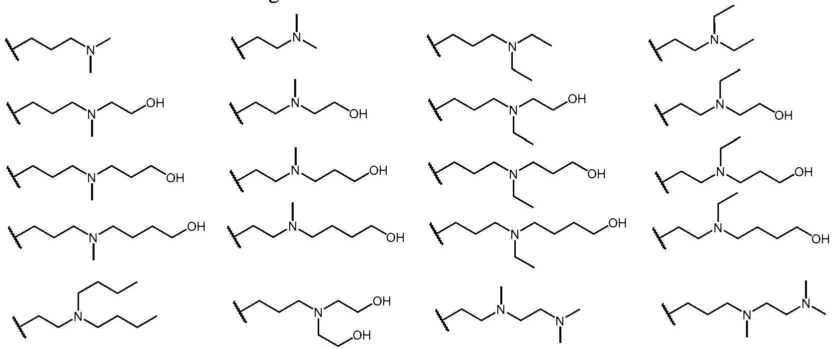 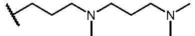 ; and
R_{I} and R_{IV} are each independently selected from the group B, at least one of R_{I} and R_{IV} contains one degradable group, for example an ester group, i.e., -C(O)O- or -OC(O)-; or the degradable group comprises an ester bond, for example the group B comprises substituted or unsubstituted aliphatic or substituted or unsubstituted heteroaliphatic groups having 11 to 30 carbon atoms as shown in the following structures:

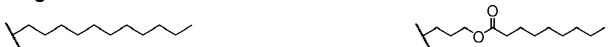

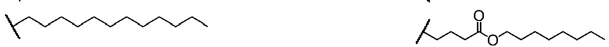

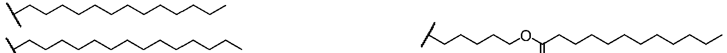

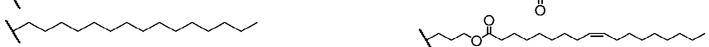

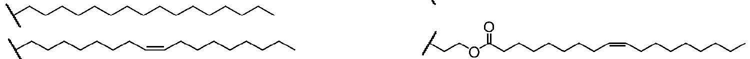

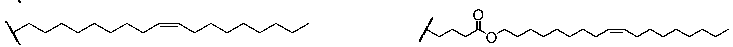

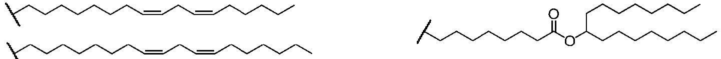

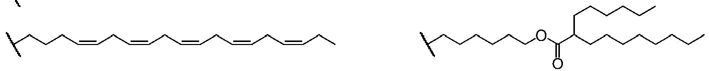

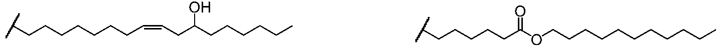

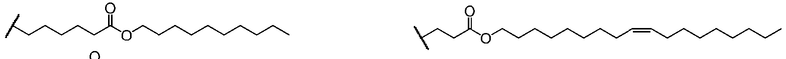

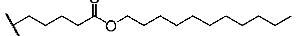

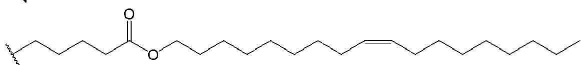

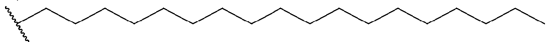

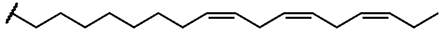

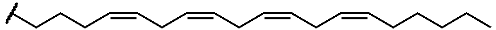

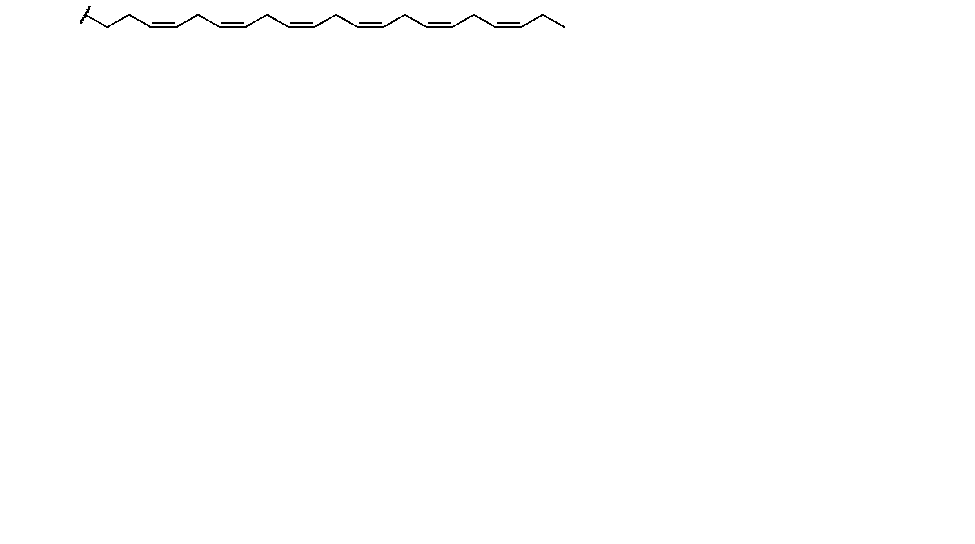

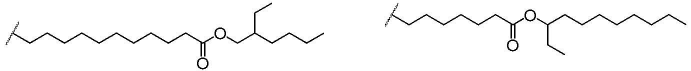

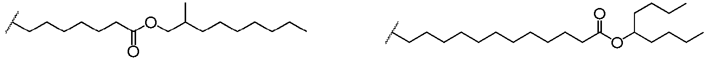

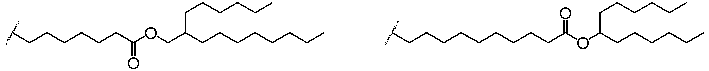

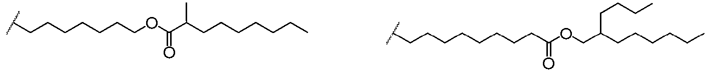

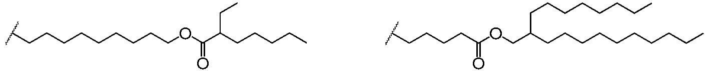

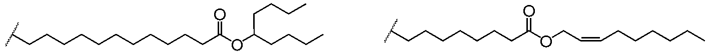

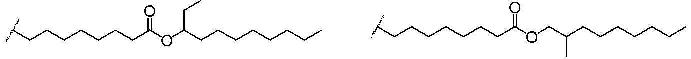

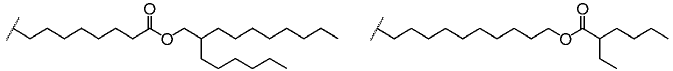

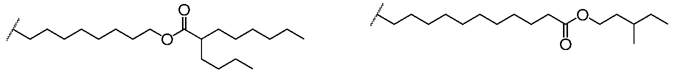

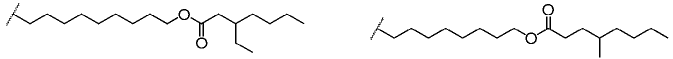

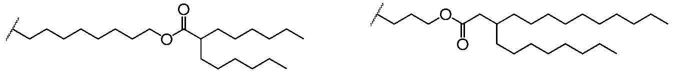

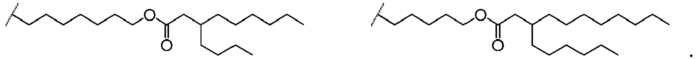

In some embodiments, the lipid compound of formula (I) of the present disclosure is selected from lipid compounds as shown in Table 1-Table 8: lipid numbers 1 to 1075.

In some embodiments, the lipid compounds of formula (I) of the present disclosure are lipid compounds as shown below: lipid numbers 16, 30, 60, 108, 113, 157, 158, 159, 160, 161, 162, 163, 166, 170, 173, 174, 175, 176, 188, 189, 194, 195, 223, 226, 227, 229, 230, 232, 233, 234, 235, 236, 237, 238, 239, 240, 284, 285, 286, 287, 292, 293, 294, 295, 304, 325, 326, 327, 401, 402, 406, 407, 408, 409, 420, 424, 431, 445, 446, 447, 583, 595, 598, 607, 613, 641, 675, 685, 914, 915, 920, 921, 922, 923, 924, 926, 927, 928, 929, 930, 931, 932, 933, 934, 935, 938, 939, 940, 941, 942, 943, 944, 945, 946, 947, 949, 950, 951, 952, 954, 955, 958, 959, 960, 961, 962, 963, 964, 966 to 1051 and 1053 to 1057.

In some embodiments, the lipid compounds of formula (I) of the present disclosure are lipid compounds as shown below: lipid numbers are 317 and 1058 to 1075.

In some embodiments, the lipid compounds of formula (I) of the present disclosure are lipid compounds as shown below:

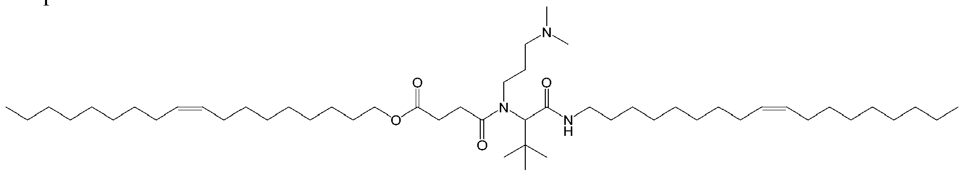

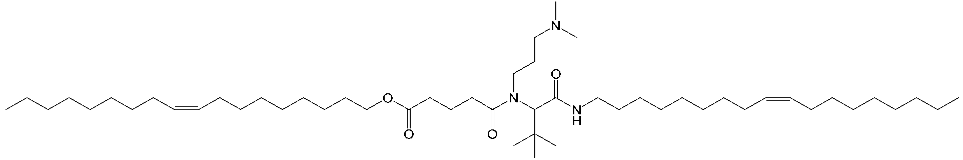

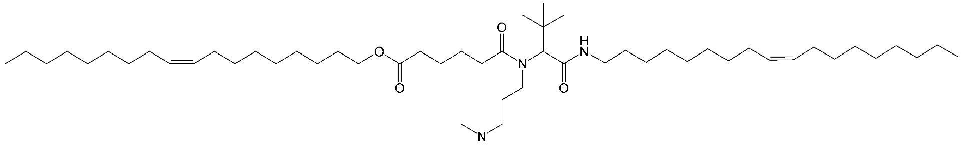

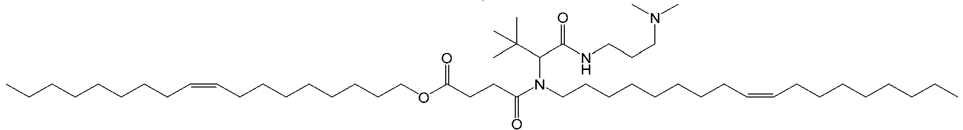

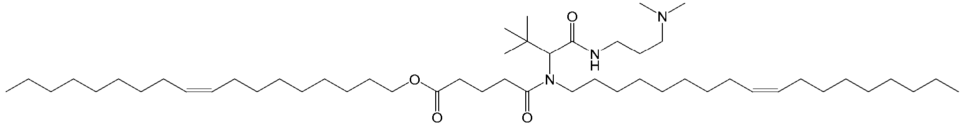

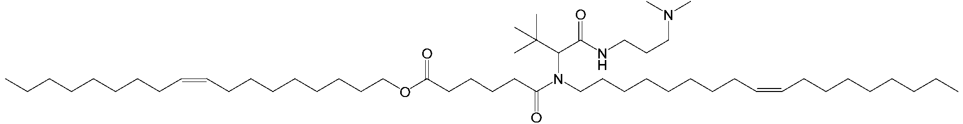

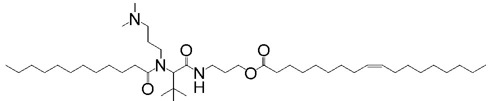

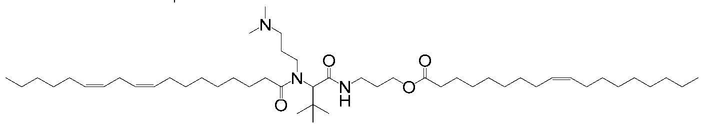

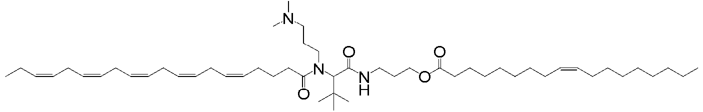

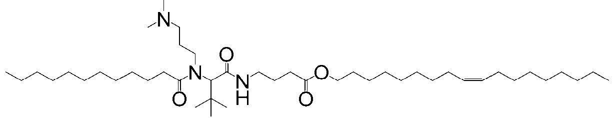

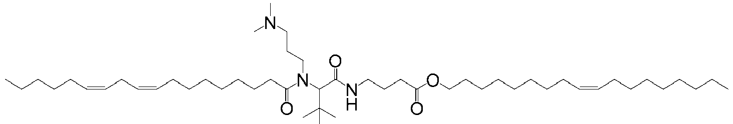

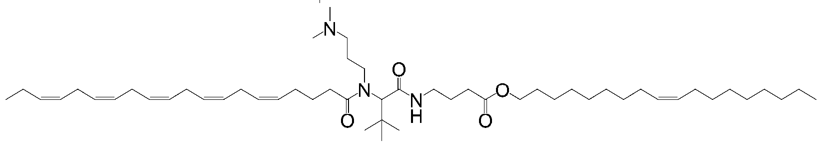

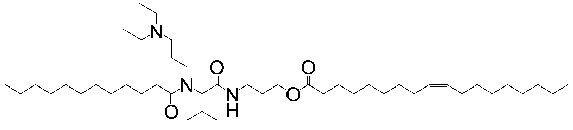

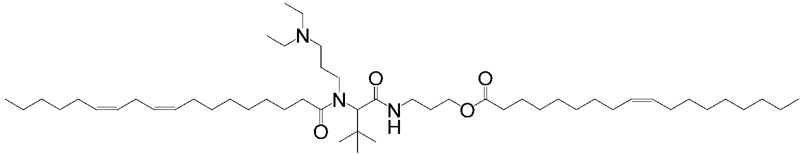

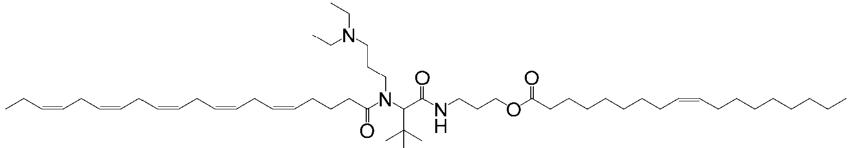

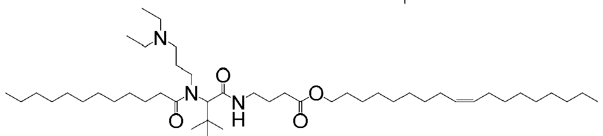

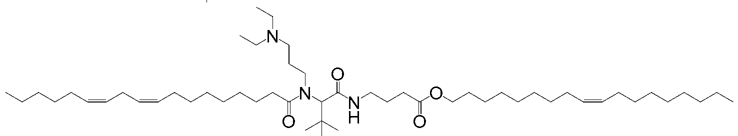

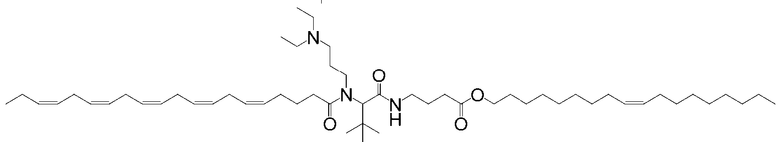

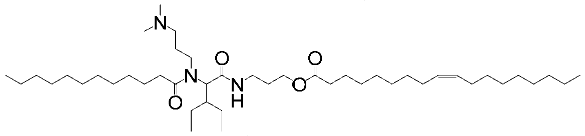

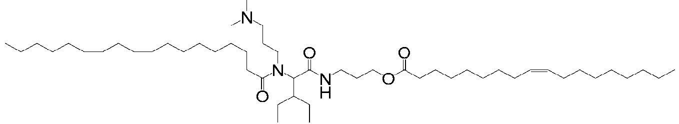

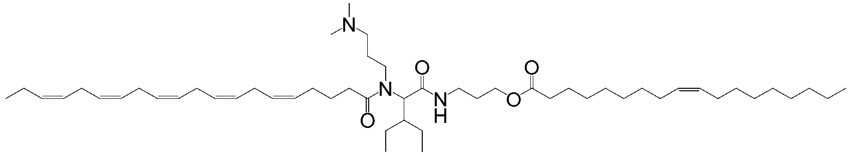

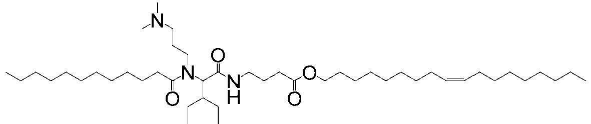

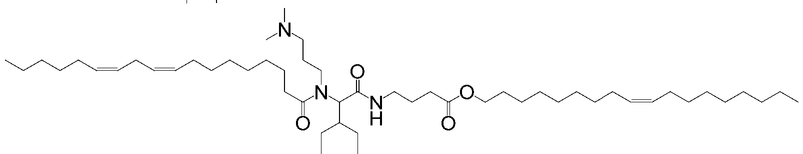

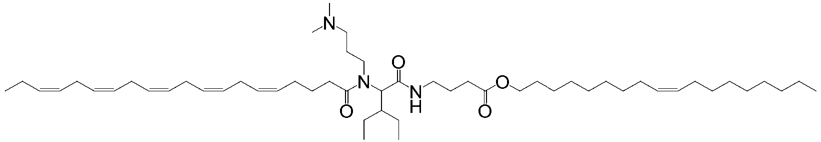

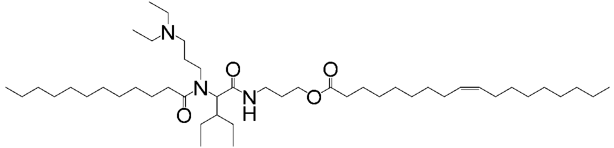

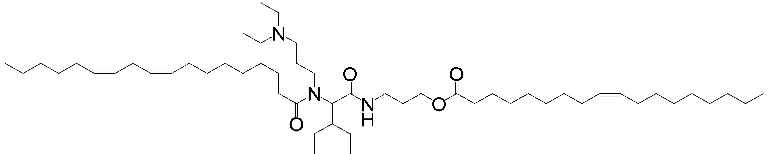

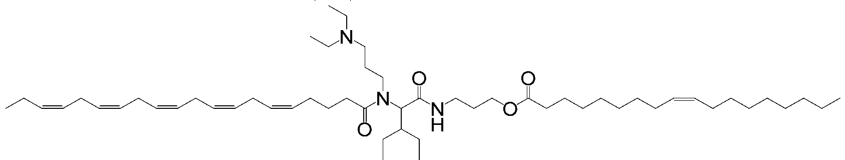

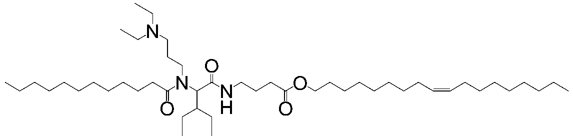

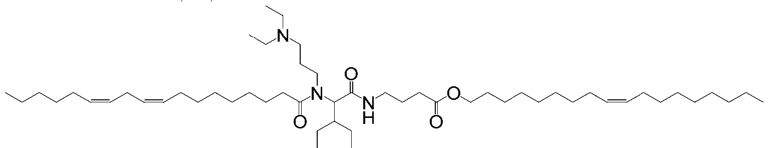

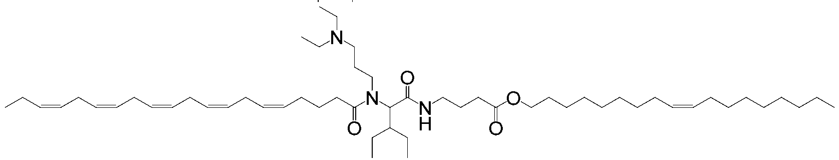

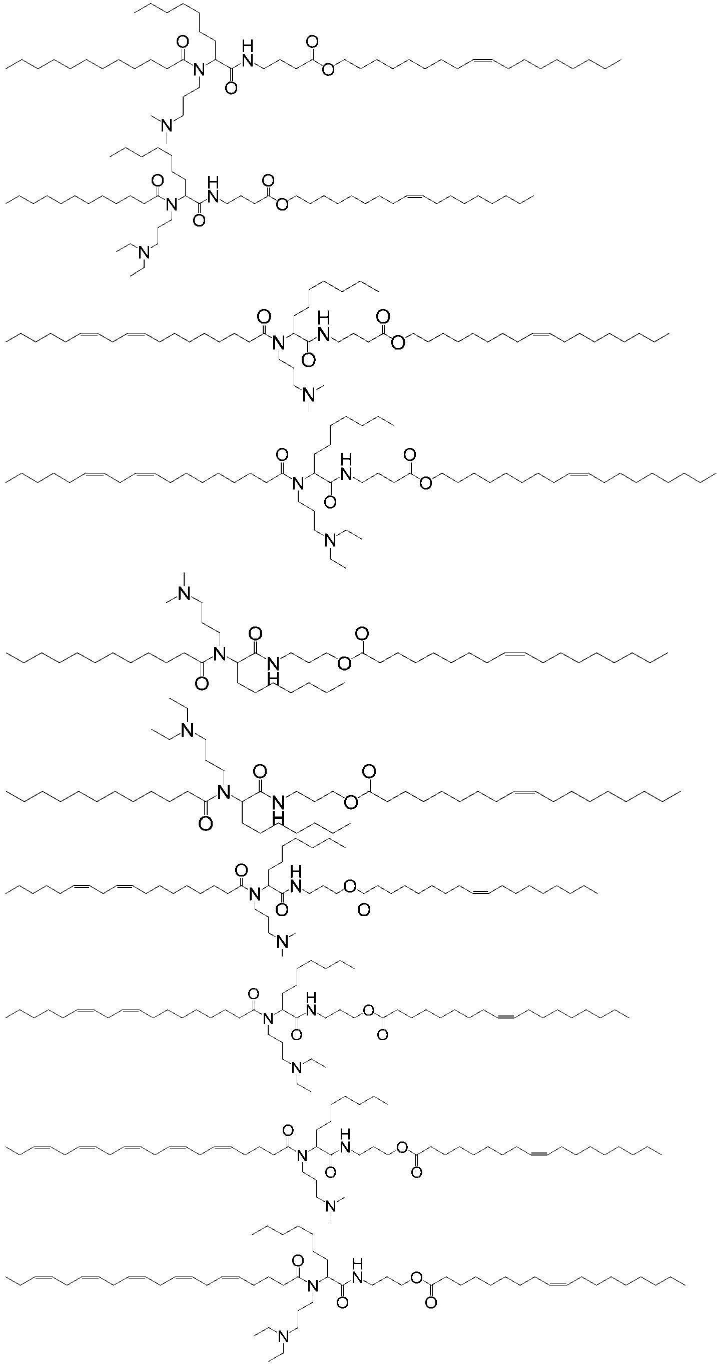

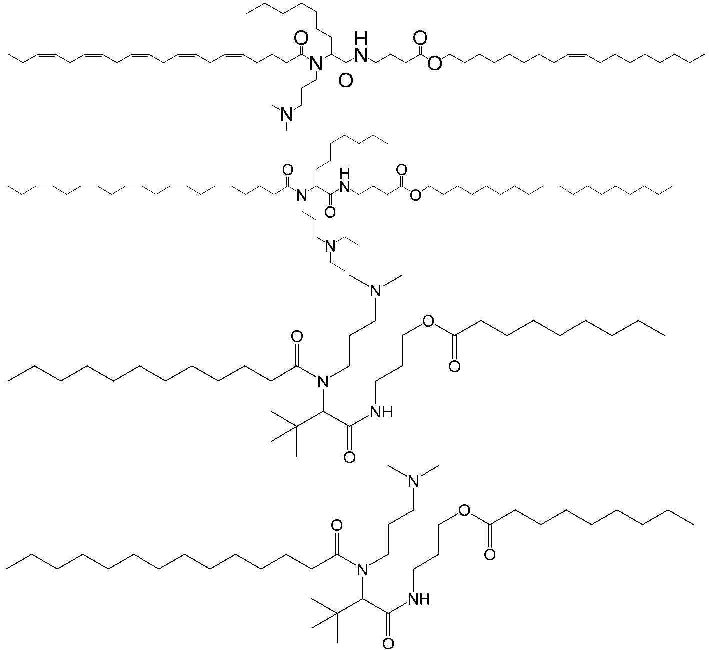

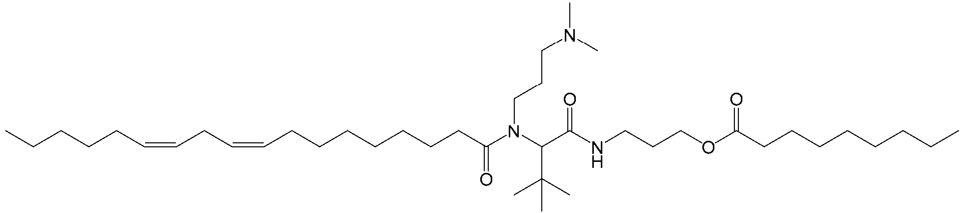

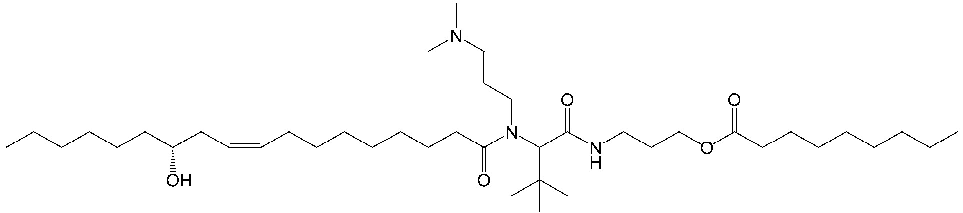

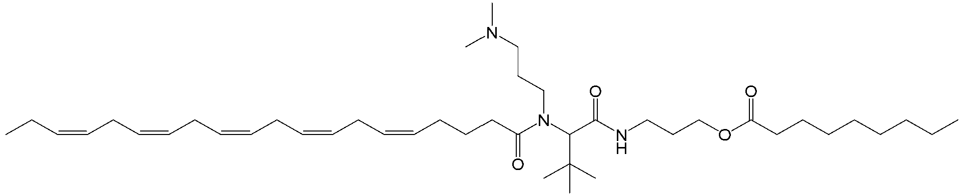

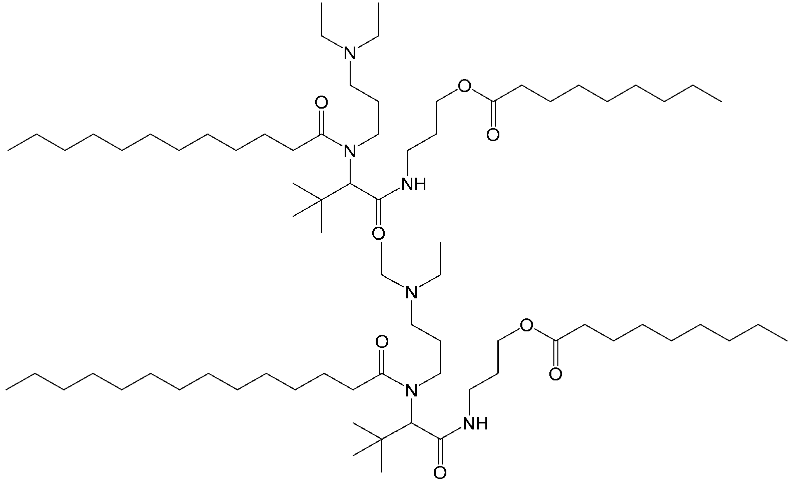

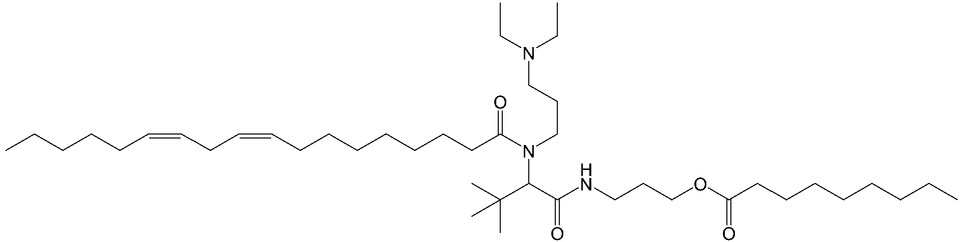

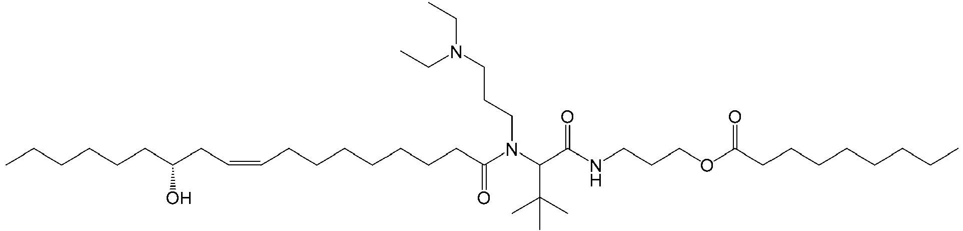

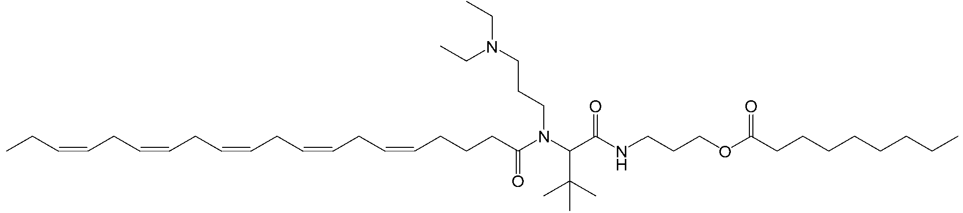

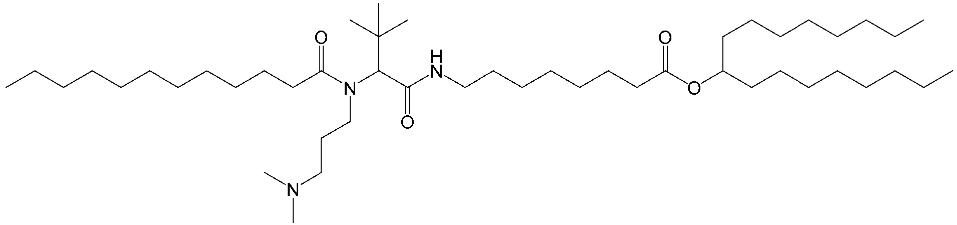

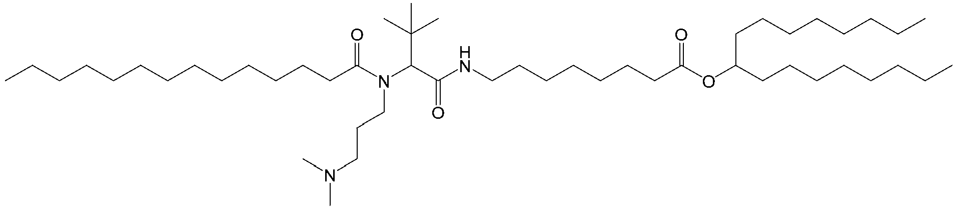

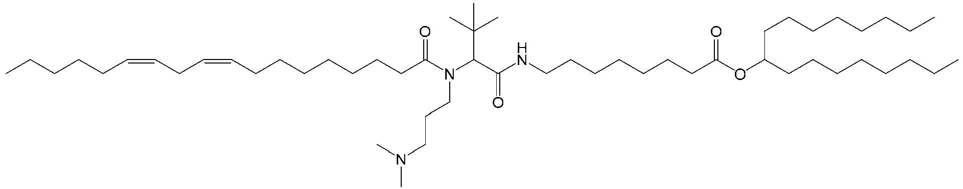

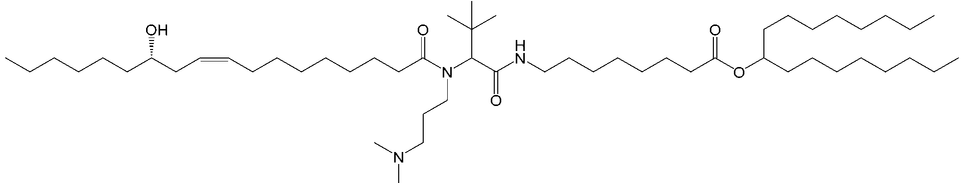

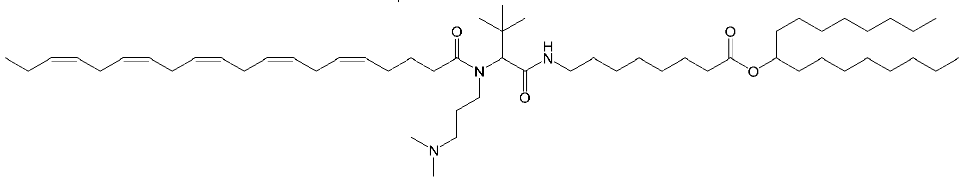

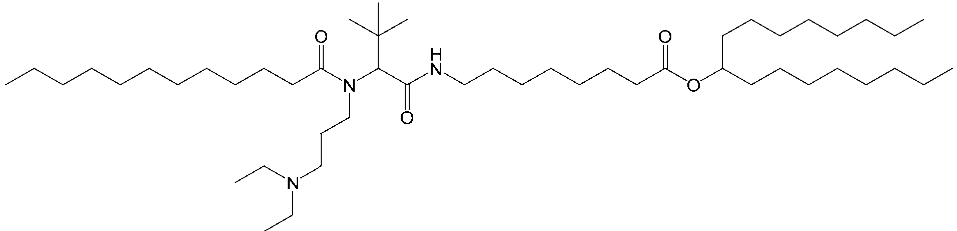

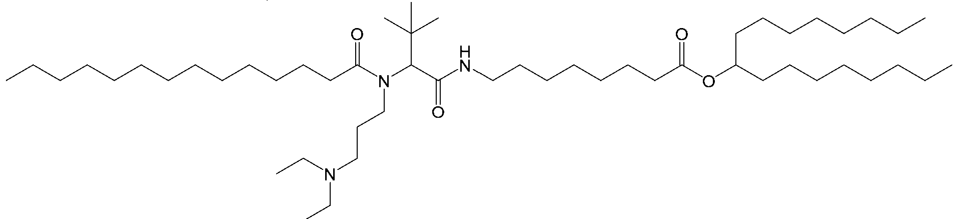

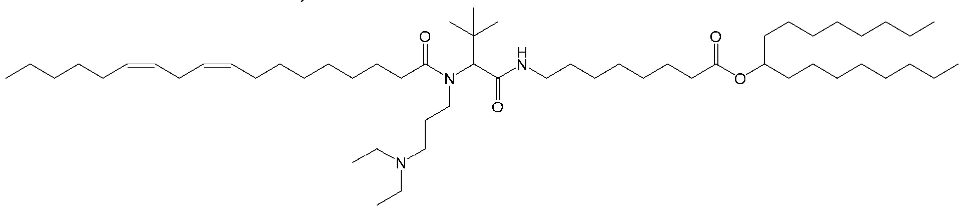

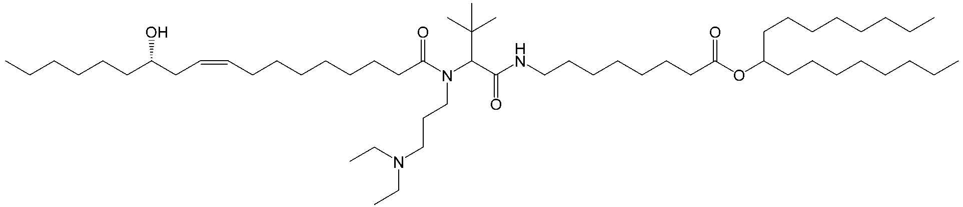

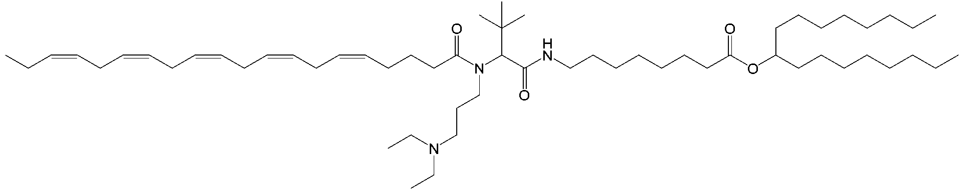

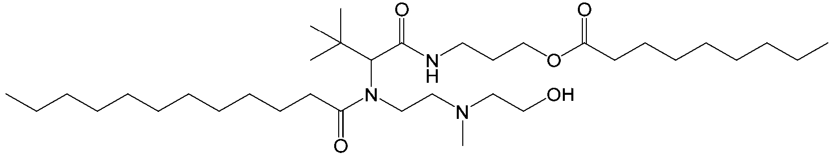

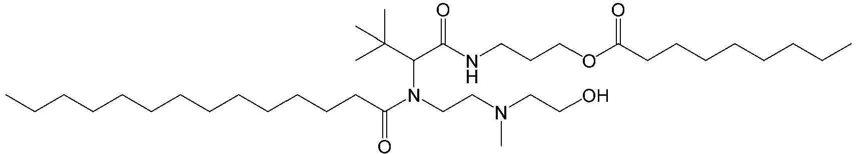

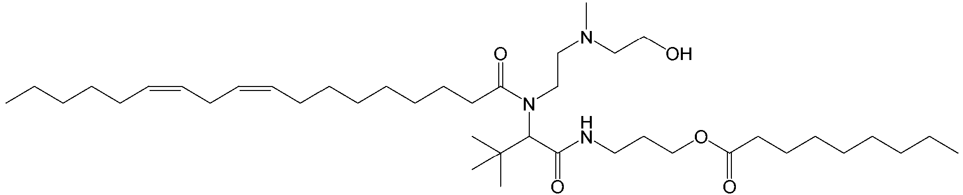

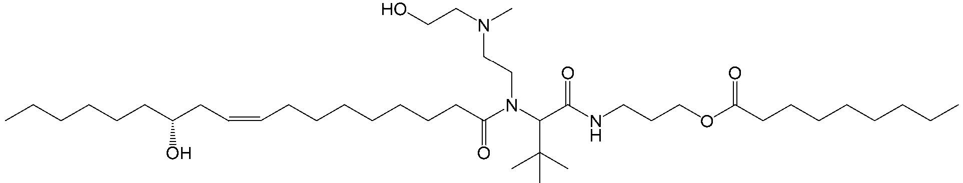

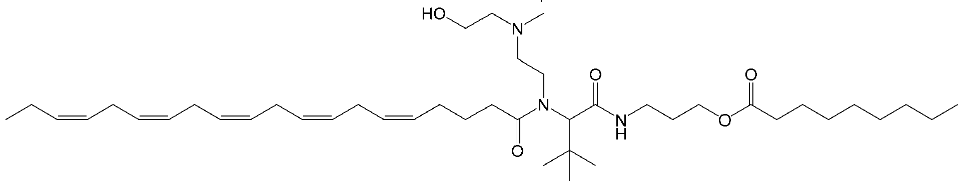

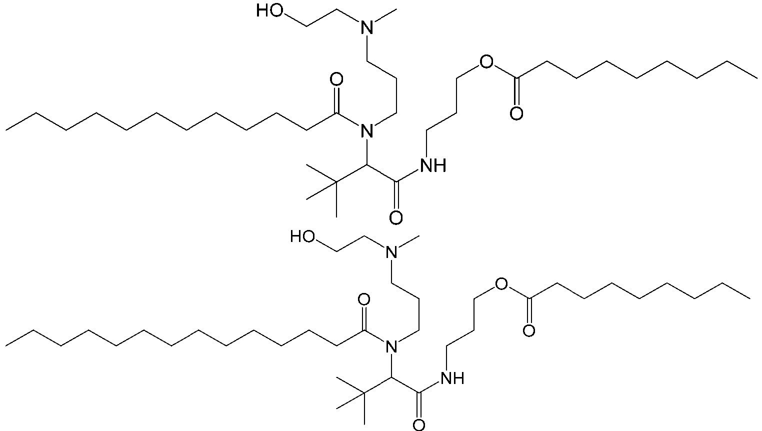

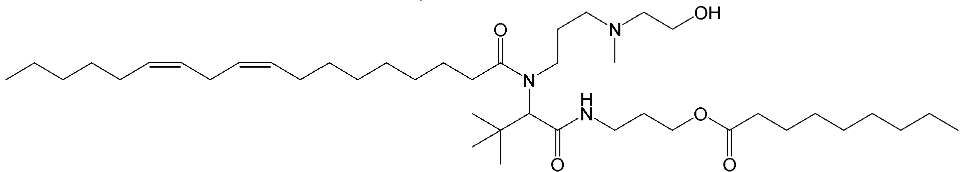

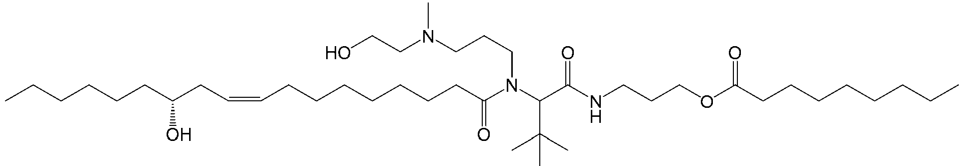

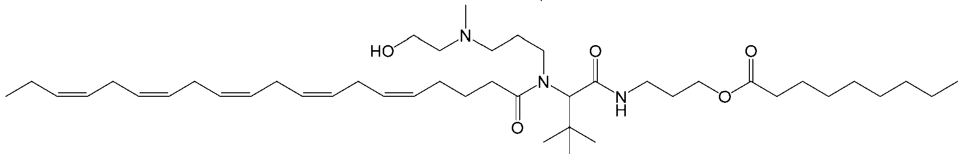

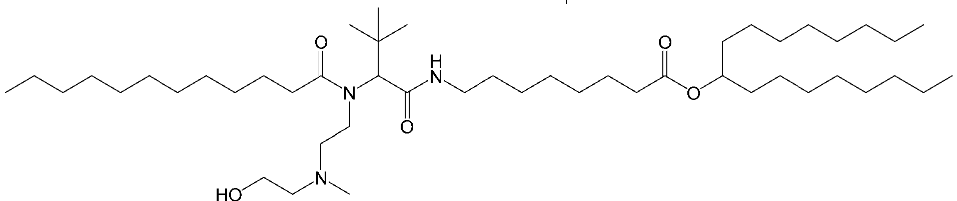

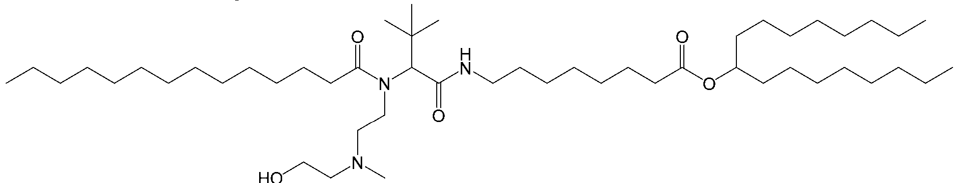

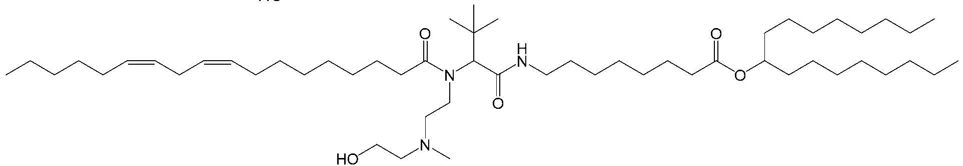

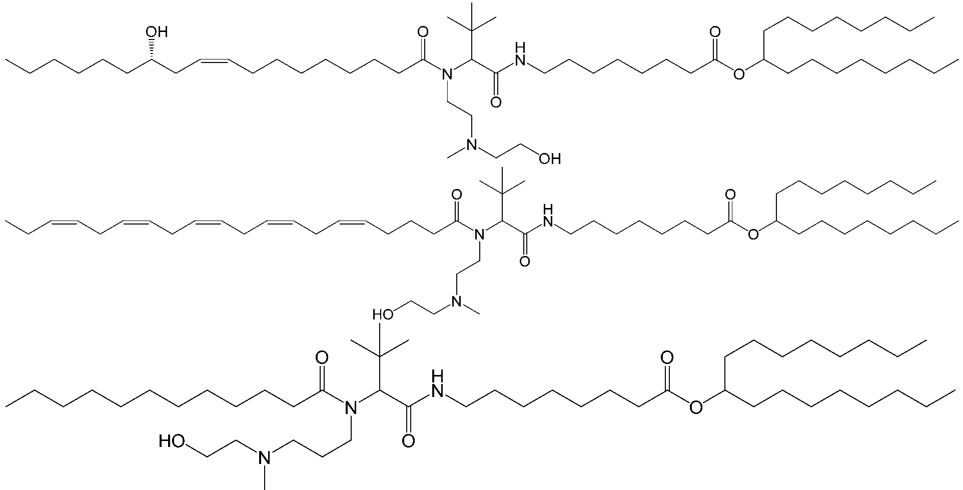

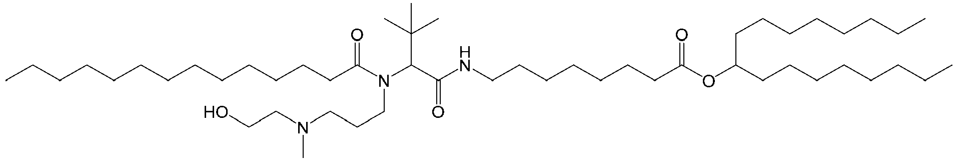

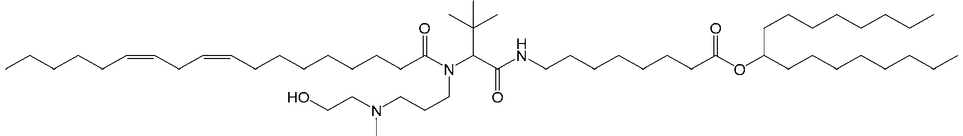

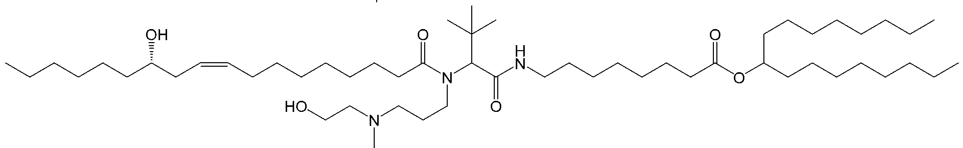

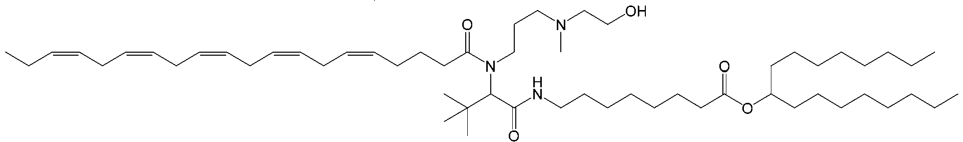

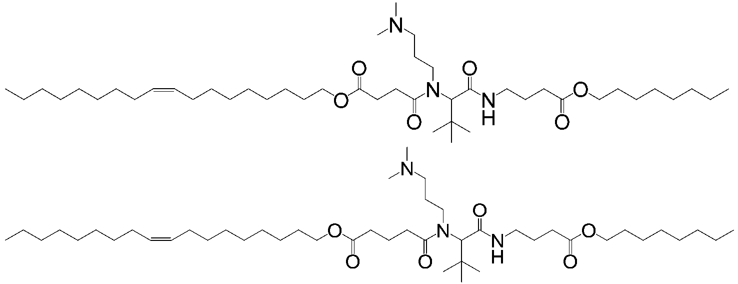

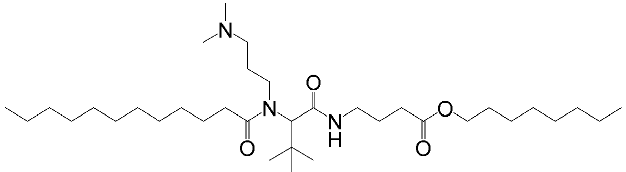

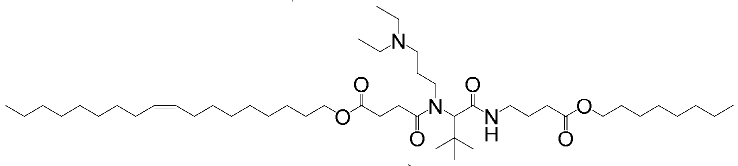

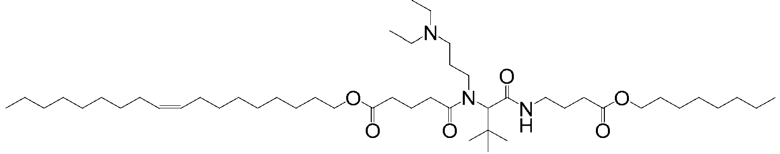

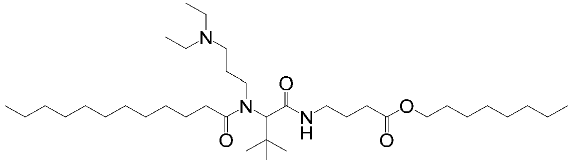

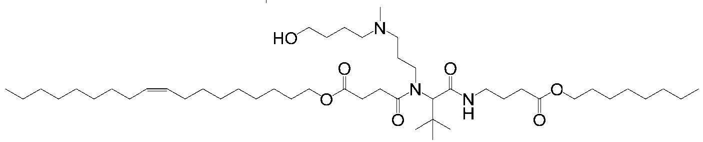

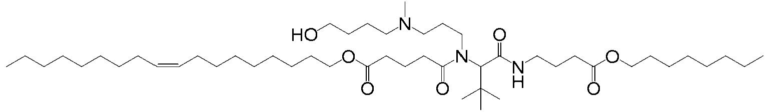

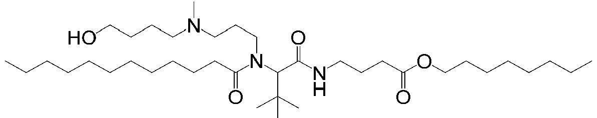

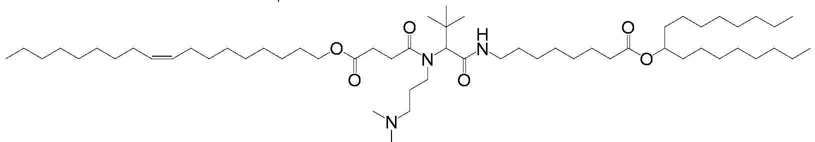

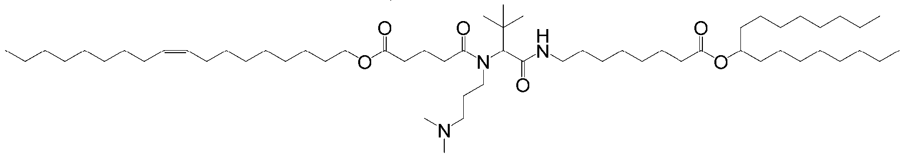

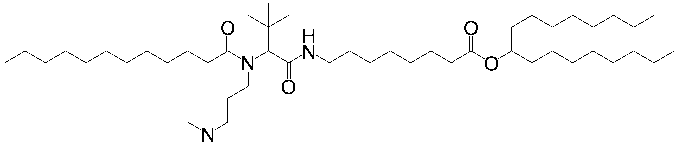

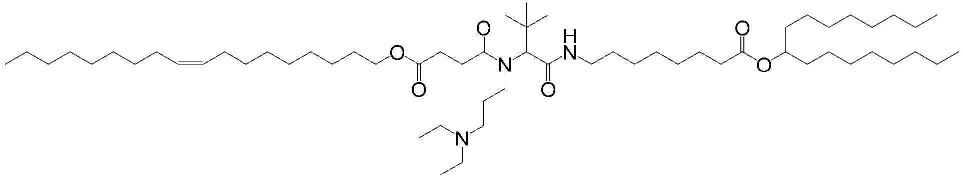

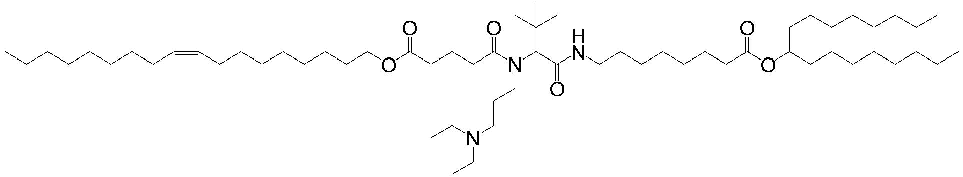

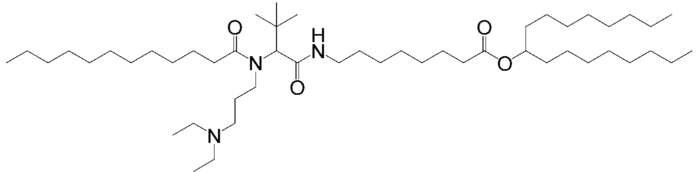

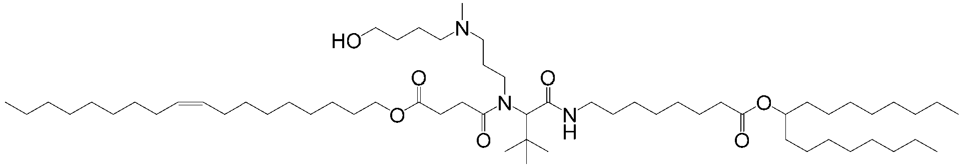

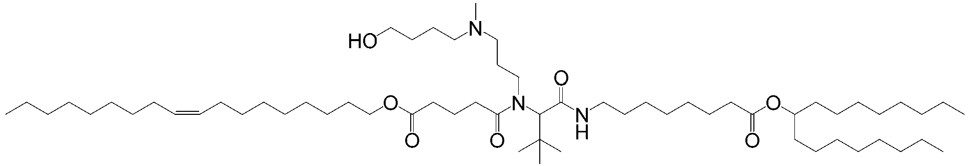

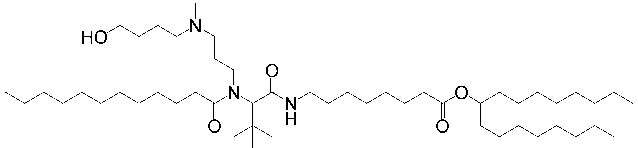

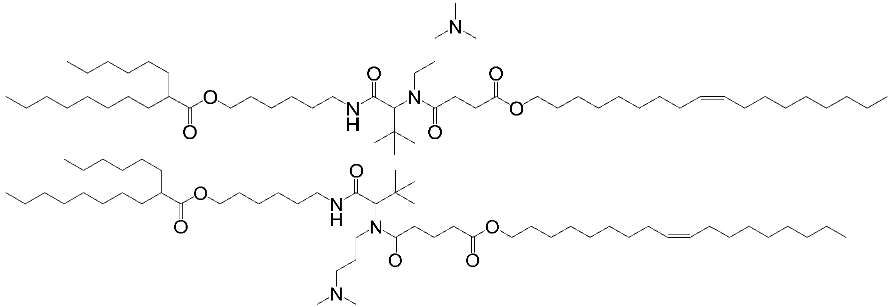

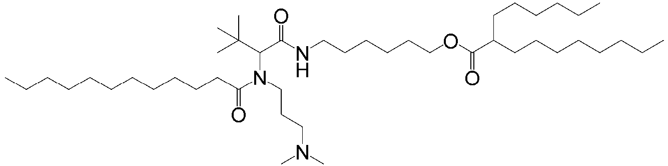

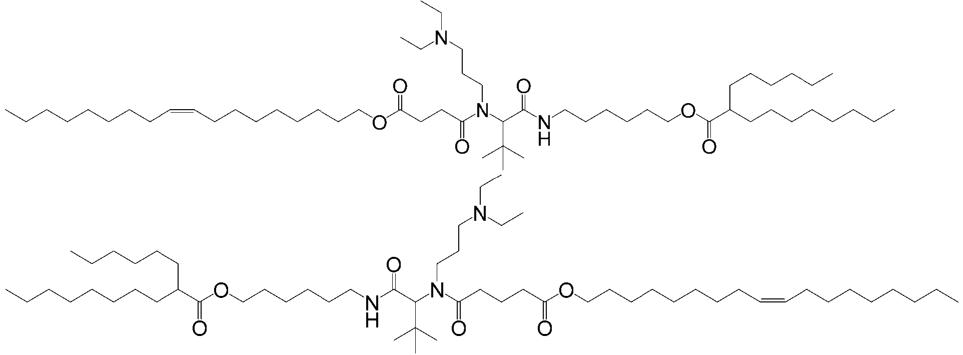

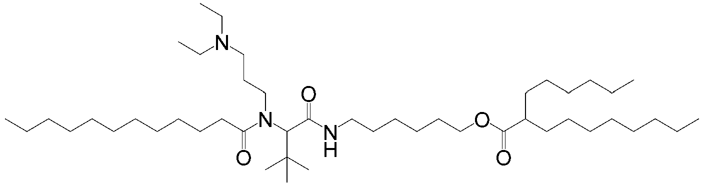

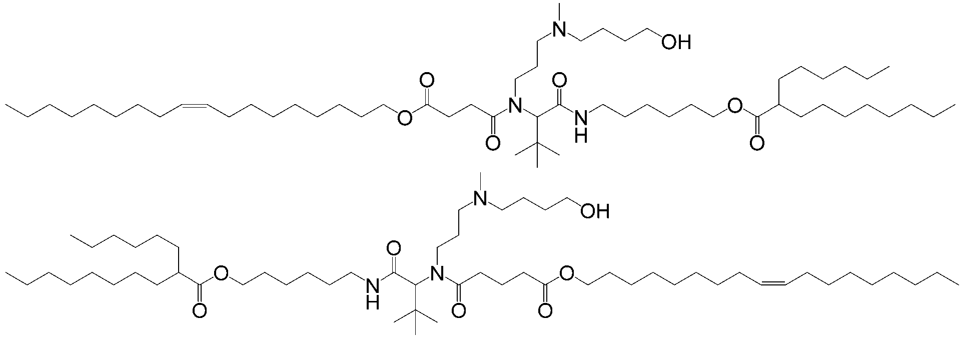

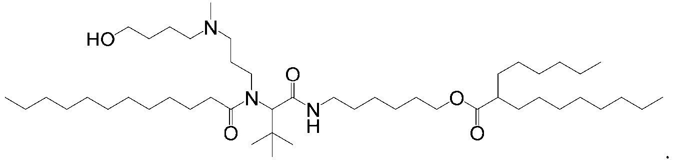

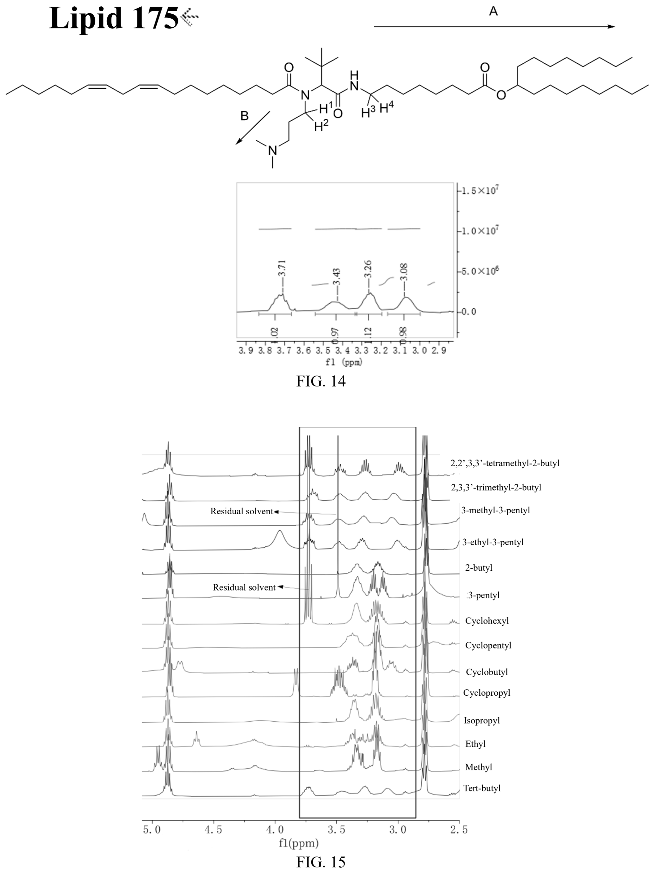

In some embodiments of the present disclosure, a series of lipids of formula (I_{A}), with the same R_{I}, R_{III} and R_{IV} groups and R_{II'} being hydrogen, are compared, and different R_{II} groups exhibit a regular change for the structures of the lipids. Furthermore, when R_{II} is tert-butyl or is of a derived structure containing tert-butyl, the configuration of its lipid has remarkable features. By taking the skeleton of lipid 175 as an example, an one-dimensional nuclear magnetic hydrogen spectrum of the lipid 175 is as shown in FIG. 14, chemical shifts δ 3.43, δ 3.71, δ 3.08 and δ 3.26 respectively correspond to H¹, H², H³ and H⁴, the peak forms of the four groups of multiple peaks appear only when the R_{II} group is tert-butyl, or a larger group containing tert-butyl (as shown in FIG. 15 and in table below). H¹, H², H³ and H⁴ have four different chemical shifts, which may be caused by limited rotation of a center carbon atom (directly linked to a R_{II} tert-butyl group) toward chains in direction A and direction B. This limited effect is highly related to the steric hindrance of the R_{II} group.

It is found by further analysis that the peak form of δ 3.71 (H¹) presents a set of sextuple peaks, showing that in addition to subject to coupling splitting of a proton on adjacent carbon, this H further contains coupling splitting of another proton on the same carbon atom, which verifies inequivalence of H¹ and H² when a substituent is tert-butyl from another perspective. In addition, it is found that when the R_{II} group is methyl, ethyl or isopropyl, this interval consists of two sets of adjacent multiple peaks, showing that when the R_{II} group is relatively small in volume, chain A and chain B relatively freely rotate. By further comparison of lipid delivery mRNA efficiencies, it is found that R_{II} is a lipid (175, 944, 945, 946, 947, etc.) with tert-butyl or a derived structure containing tert-butyl, which more easily exhibits high delivery efficiency in mRNA mouse *in vivo* delivery.

| Lipid number | R_{II} substituent on lipid 175 skeleton | Number of 3-4 ppm splitting peaks |
|---|---|---|
| 175 | Tert-butyl | 4 |
| 949 | Methyl | 2 |
| 950 | Ethyl | 2 |
| 942 | Isopropyl | 2 |
| 936 | Cyclopropyl | 2 |
| 937 | Cyclobutyl | 3 |
| 1045 | Cyclopentyl | 2 |
| 1048 | Cyclohexyl | 2 |
| 941 | 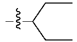 | 3 |
| 943 | 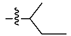 | 2 |
| 944 | 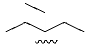 | 4 |
| 945 | 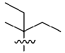 | 4 |
| 946 | 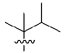 | 4 |
| 947 | 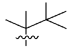 | 4 |

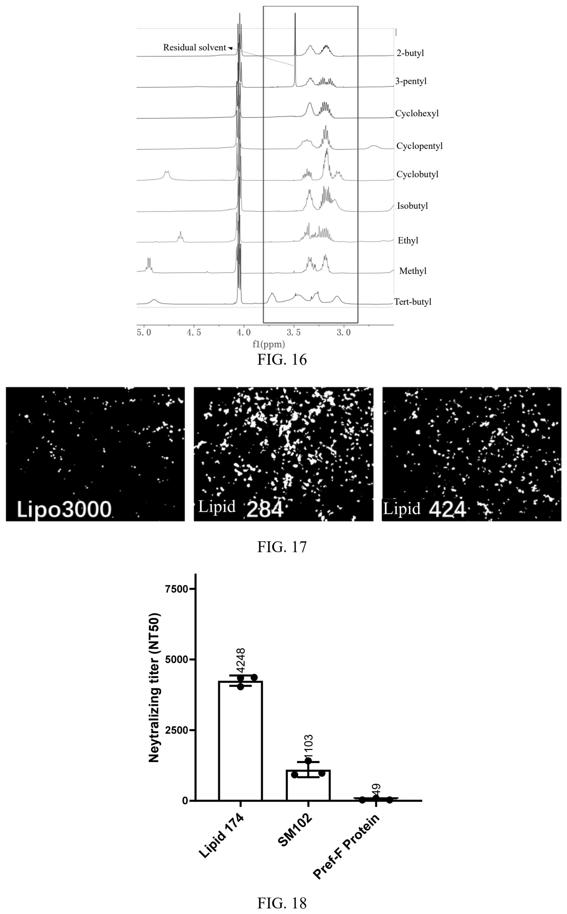

The same nuclear magnetic spectrum features can also be observed on a structure using lipid 284 as a main skeleton, as shown in FIG.16 and in table below.

| Lipid number | R_{II} substituent on lipid 284 skeleton | Number of 3-4 splitting peaks |
|---|---|---|
| 284 | Tert-butyl | 4 |
| 1039 | Methyl | 2 |
| 1041 | Ethyl | 2 |
| 960 | Isopropyl | 2 |
| 1043 | Cyclobutyl | 3 |
| 1046 | Cyclopenyl | 2 |
| 1049 | Cyclohexyl | 2 |
| 959 | 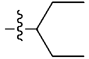 | 2 |
| 961 | 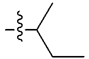 | 2 |

By preparing a lipid in which R_{II} is tert-butyl and R_{I} and R_{IV} are short-chain groups (ethyl and butyl), it is found that there are still quadruple peaks between chemical shifts 3-4 ppm in a one-dimensional nuclear magnetic spectrum (not shown), showing that the structure features on the nuclear magnetic spectrum are not related to the length of a hydrophobic lipid chain, and only related to the R_{II} group.

In some embodiments, the lipid compound of formula (I) of the present disclosure has a molecular weight in a range of about 500 g/mol to about 1400 g/mol; preferably, the lipid compound has a molecular weight in a range of about 600 g/mol to about 1200 g/mol; preferably, the lipid compound has a molecular weight in a range of about 600 g/mol to about 1000 g/mol; and more preferably, the lipid compound has a molecular weight in a range of about 700 g/mol to about 1000 g/mol.

The lipid compound of formula (I) of the present disclosure is used for nucleic acid delivery to achieve the introduction of a nucleic acid (for example DNA and RNA) into organelles, cells, tissues or organisms.

In one aspect, the lipid compound of the present disclosure is a lipid compound having a structure represented by formula (III), or an N-oxide, stereoisomer or pharmaceutically acceptable salt thereof,

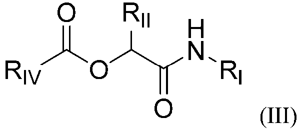

R_{I}, R_{II} and R_{IV} are each independently selected from (A) groups containing at least one tertiary amine structure, and (B) substituted or unsubstituted aliphatic or substituted or unsubstituted heteroaliphatic groups having 11 to 30 carbon atoms, provided that: one and only one of R_{I}, R_{II} and R_{IV} is the group containing at least one ionizable tertiary amine structure.

In some embodiments, R_{I} is selected from (B) substituted or unsubstituted aliphatic or substituted or unsubstituted heteroaliphatic groups having 11 to 30 carbon atoms, one of R_{II} and R_{IV} is selected from (A) groups containing at least one tertiary amine structure, and the other is selected from (B) substituted or unsubstituted aliphatic or substituted or unsubstituted heteroaliphatic groups having 11 to 30 carbon atoms.

In some embodiments, R_{I}, R_{II} and R_{IV} are as defined by corresponding groups in the lipid compound of formula (I).

In one aspect, the lipid compound of the present disclosure is a lipid compound having a structure represented by formula (IV), or an N-oxide, stereoisomer or pharmaceutically acceptable salt thereof,

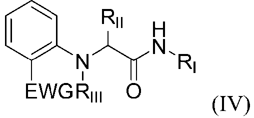

R_{I}, R_{II} and R_{III} are each independently selected from (A) groups containing at least one tertiary amine structure, and (B) substituted or unsubstituted aliphatic or substituted or unsubstituted heteroaliphatic groups having 11 to 30 carbon atoms, provided that: one and only one of R_{I}, R_{II} and R_{III} is the group containing at least one ionizable tertiary amine structure.

In some embodiments, R_{I} is selected from (B) substituted or unsubstituted aliphatic or substituted or unsubstituted heteroaliphatic groups having 11 to 30 carbon atoms, one of R_{II} and R_{III} is selected from (A) groups containing at least one tertiary amine structure, and the other is selected from (B) substituted or unsubstituted aliphatic or substituted or unsubstituted heteroaliphatic groups having 11 to 30 carbon atoms.

In some embodiments, R_{I}, R_{II} and R_{III} are as defined by corresponding groups in the lipid compound of formula (I).

In one aspect, the lipid compound of the present disclosure is a lipid compound having a structure represented by formula (V), or an N-oxide, stereoisomer or pharmaceutically acceptable salt thereof,

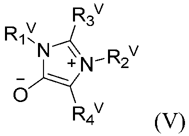

R₁^{V}, R₂^{V}, R₃^{V} and R₄^{V} are each independently selected from (A) groups containing at least one tertiary amine structure, (B) substituted or unsubstituted aliphatic or substituted or unsubstituted heteroaliphatic groups having 11 to 30 carbon atoms, and (C) substituted or unsubstituted hydrocarbyl, substituted or unsubstituted heterohydrocarbyl, substituted or unsubstituted aryl or substituted or unsubstituted heteroaryl groups having fewer than 11 carbon atoms, provided that: one and only one of R₁^{V}, R₂^{V}, R₃^{V} and R₄^{V} is the group containing at least one ionizable tertiary amine structure.

In one aspect, the lipid compound of the present disclosure is a lipid compound having a structure represented by formula (VI), or an N-oxide, stereoisomer or pharmaceutically acceptable salt thereof,

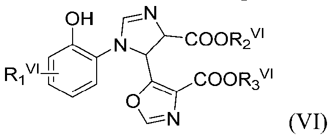

R₁^{VI}, R₂^{VI}, R₃^{V} and R₄^{VI} are each independently selected from (A) groups containing at least one tertiary amine structure, (B) substituted or unsubstituted aliphatic or substituted or unsubstituted heteroaliphatic groups having 11 to 30 carbon atoms, and (C) substituted or unsubstituted hydrocarbyl, substituted or unsubstituted heterohydrocarbyl, substituted or unsubstituted aryl or substituted or unsubstituted heteroaryl groups having fewer than 11 carbon atoms, provided that: one and only one of R₁^{VI}, R₂^{VI}, R₃^{V} and R₄^{VI} is the group containing at least one ionizable tertiary amine structure.

The lipid compound of the present disclosure is used for nucleic acid delivery to achieve the introduction of a nucleic acid (for example DNA and RNA) into organelles, cells, tissues or organisms.

### Lipid compound containing isonitrile or carboxylic acid end group

The lipid compound containing an isonitrile or carboxylic acid end group of the present disclosure can be used for preparing an ionizable lipid.

The lipid compound containing an isonitrile or carboxylic acid end group of the present disclosure is a lipid compound having a structure represented by formula (II), or a stereoisomer or pharmaceutically acceptable salt thereof, 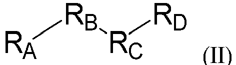
wherein R_{A} is selected from substituted or unsubstituted aliphatic groups and heteroaliphatic groups, R_{A} is optionally substituted with one or more substituents selected from the following group: deuterium, halogen, -NO₂ and -OH;
R_{B} is a degradable bond-containing degradable group selected from the following group: 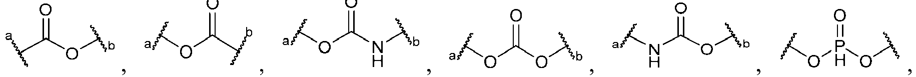 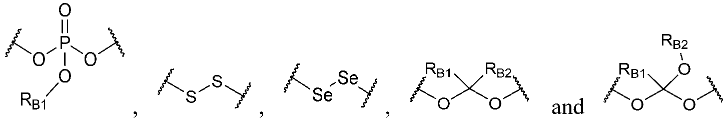 , wherein a represents a position linked to R_{A}, b represents a position linked to R_{C}, R_{B1} and R_{B2} are each independently selected from H and substituted or unsubstituted C₁₋₆ alkyl, and the alkyl is optionally substituted with one or more substituents selected from the following group: deuterium, halogen, -NO₂ and -OH;
R_{C} is selected from aliphatic groups and heteroaliphatic groups, and R_{C} is optionally substituted with one or more substituents selected from the following group: deuterium, halogen, -NO₂ and -OH; and
R_{D} is selected from -NC or -COOH.

In some embodiments, R_{D} is -NC, and the lipid compound containing an isonitrile end group of the present disclosure has a structure represented by formula (II_{A}):

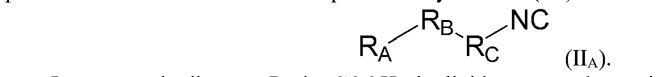

In some embodiments, R_{D} is -COOH, the lipid compound containing a carboxylic acid end group of the present disclosure has a structure represented by formula (II_{B}):

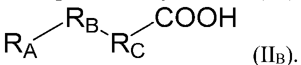

In some embodiments, R_{A} is selected from substituted or unsubstituted linear aliphatic groups having 6 to 20 carbon atoms and substituted or unsubstituted linear heteroaliphatic groups having 6 to 20 carbon atoms, the linear aliphatic groups and the linear heteroaliphatic groups are optionally substituted with one or more substituents selected from the following group: deuterium, halogen, -NO₂ and -OH.

In some embodiments, the number of carbon atoms in R_{A} is in a range between any two numerical values within a range of 6 to 20.

In some embodiments, R_{A} is selected from substituted or unsubstituted branched aliphatic groups or substituted or unsubstituted heteroapliphatic groups having a formula (II_{A}): 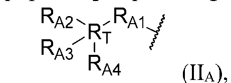 wherein,
R_{A1} is absent, or is selected from substituted or unsubstituted C₁₋₄ alkyl, and the alkyl is optionally substituted with one or more substituents selected form the following group: deuterium, halogen, -NO₂ and -OH;
a branching center group R_{T} is selected from the group consisting of the following groups: 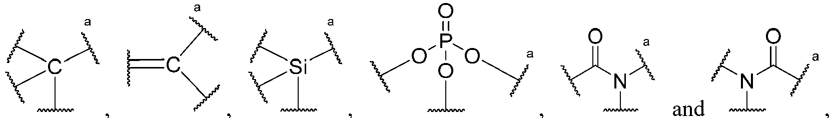 wherein a represents a position linked to R_{A1}, or a position directly linked to R_{B} when R_{A1} is absent;
R_{A2} and R_{A3} are each independently selected from substituted or unsubstituted aliphatic groups and substituted or unsubstituted heterpaliphatic groups having 1 to 12 carbon atoms, and the aliphatic groups and the heteroaliphatic groups are optionally substituted with one or more substituents selected from the following group: deuterium, halogen, -NO₂ and -OH;
R_{A4} is absent, or is selected from hydrogen, and substituted or unsubstituted aliphatic groups and substituted or unsubstituted heteroaliphatic groups having 1 to 12 carbon atoms, and the aliphatic groups and the heteroaliphatic groups are optionally substituted with one or more substituents selected from the following group: deuterium, halogen, -NO₂ and -OH; and
a sum of a carbon atom number n_{A2} of R_{A2}, a carbon atom number n_{A3} of R_{A3} and a carbon atom number n_{A4} of R_{A4} is in a range of 2 to 24.

In some embodiments, the number of carbon atoms in R_{A1} is in a range between any two numerical values within a range of 1 to 4.

In some embodiments, the number of carbon atoms in R_{A2} and R_{A3} is in a range between any two numerical values within a range of 1 to 12.

In some embodiments, the number of carbon atoms in R_{A4} is in a range between any two numerical values within a range of 1 to 12.

In some embodiments, a sum of a carbon atom number n_{A2} of R_{A2}, a carbon atom number n_{A3} of R_{A3} and a carbon atom number n_{A4} of R_{A4} is in a range of 2 to 18, for example 2, 3, 4, 5, 6, 7, 8, 9, 10, 11, 12, 13, 14, 15, 16, 17 and 18, and is in a range between any two numerical values as described above.

In some embodiments, R_{A} is selected from groups as shown below:

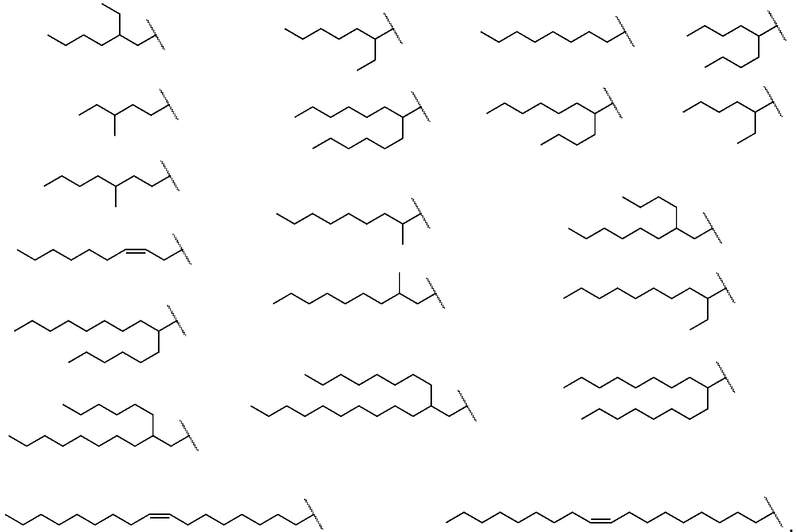

In some embodiments, R_{C} is selected from substituted or unsubstituted linear aliphatic groups having 2 to 18 carbon atoms and substituted or unsubstituted linear heteroaliphatic groups having 2 to 18 carbon atoms, the linear aliphatic group and the linear heteroaliphatic group are optionally substituted with one or more substituents selected from the following group: deuterium, halogen, -NO₂ and -OH, and R_{D} is linked to a terminal of the linear aliphatic group or the linear heteroaliphatic group.

In some embodiments, R_{C} is selected from substituted or unsubstituted branched aliphatic groups or substituted or unsubstituted branched heteroaliphatic groups having a formula (II_{c}): 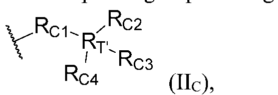 wherein,
R_{C1} is absent, or is selected from substituted or unsubstituted C₁₋₄ alkyl, and the alkyl is optionally substituted with one or more substituents selected from the following group: deuterium, halogen, -NO₂ and -OH;
a branching center group R_{T'} is selected from the group consisting of the following groups: 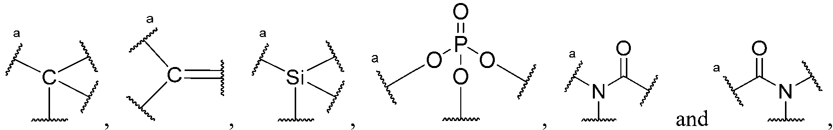 wherein a represents a position linked to R_{C1}, or a position directly linked to R_{B} when R_{C1} is absent;
R_{C2} and R_{C3} are each independently selected from substituted or unsubstituted aliphatic groups and substituted or unsubstituted heteroaliphatic groups having 1 to 12 carbon atoms, and the aliphatic group and the heteroaliphatic group are optionally substituted with one or more substituents selected from the following group: deuterium, halogen, -NO₂ and -OH;
R_{C4} is absent, or is selected from hydrogen, and substituted or unsubstituted aliphatic groups and substituted or unsubstituted heteroaliphatic groups having 1 to 12 carbon atoms, and the aliphatic groups and the heteroaliphatic groups are optionally substituted with one or more substituents selected from the following group: deuterium, halogen, -NO₂ and -OH;
A sum of a carbon atom number n_{C2} of R_{C2}, a carbon atom number n_{C3} of R_{C3} and a carbon atom number n_{C4} of R_{C4} is in a range of 2 to 24; and
R_{D} is linked to the terminal of any one and only one group in R_{C2}, R_{C3} and R_{C4}.

In some embodiments, the number of carbon atoms in R_{C1} is in a range of any two numerical values within a range of 1 to 4.

In some embodiments, the number of carbon atoms in R_{C2} and R_{C3} is in a range of any two numerical values within a range of 1 to 12.

In some embodiments, the number of carbon atoms in R_{C4} is in a range of any two numerical values within a range of 1 to 12.

In some embodiments, a sum of a carbon atom number n_{C2} of R_{C2}, a carbon atom number n_{C3} of R_{C3} and a carbon atom number n_{C4} of R_{C4} is in a range of 2 to 18, for example 2, 3, 4, 5, 6, 7, 8, 9, 10, 11, 12, 13, 14, 15, 16, 17 and 18, or is in a range between any two numerical values as described above.

In some embodiments, the R_{A} group contains one branching center carbon atom; further, the branching center carbon atom is located at the position α, β or γ of the R_{B} group.

In some embodiments, R_{C} is selected from substituted or unsubstituted linear aliphatic groups having 2 to 11 carbon atoms.

In some embodiments, R_{C} is selected from groups as shown below:

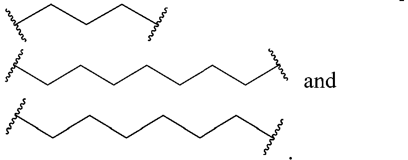

In some embodiments, R_{A} is selected form substituted or unsubstituted linear aliphatic groups having 6 to 20 carbon atoms, and the linear aliphatic group is optionally substituted with one or more substituents selected from the following group: deuterium, halogen, -NO₂ and -OH; or
R_{A} is selected from substituted or unsubstituted branched aliphatic groups or substituted or unsubstituted branched heteroaliphatic groups having a formula (II_{A'}): 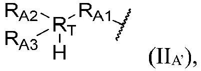
wherein,
R_{A1} is absent, or is selected from substituted or unsubstituted C₁₋₄ alkyl, the alkyl is optionally substituted with one or more substituents selected from the following group: deuterium, halogen, -NO₂ and -OH;
a branching center group R_{T} is 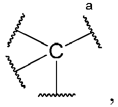 wherein a represents a position linked to R_{A1}, or a position directly linked to R_{B} when R_{A1} is absent;
R_{A2} and R_{A3} are each independently selected from substituted or unsubstituted aliphatic groups having 1 to 12 carbon atoms, and the aliphatic group is optionally substituted with one or more substituents selected from the following group: deuterium, halogen, -NO₂ and -OH;
a sum of a carbon atom number n_{A2} of R_{A2}, a carbon atom number n_{A3} of R_{A3} is in a range of 2 to 18; and
R_{B} is a degradable bond-containing degradable group selected from the following group: 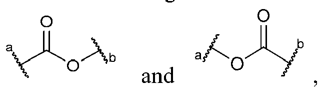 wherein a represents a position linked to R_{A}, and b represents a position linked to R_{C};
R_{C} is selected from substituted or unsubstituted linear aliphatic groups having 2 to 18 carbon atoms, and the linear aliphatic group is optionally substituted with one or more substituents selected from the following group: deuterium, halogen, -NO₂ and -OH; and
R_{D} is selected from -CN or -COOH.

In some embodiments, the lipid compounds of formula (II) of the present disclosure are lipid compounds as shown below:

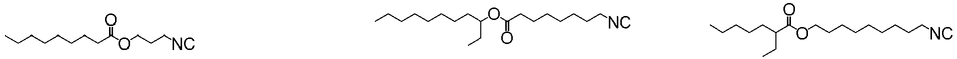

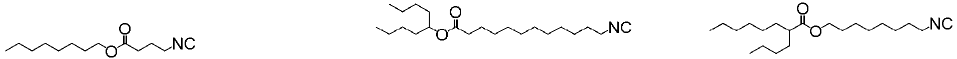

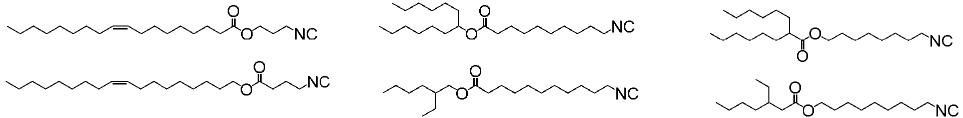

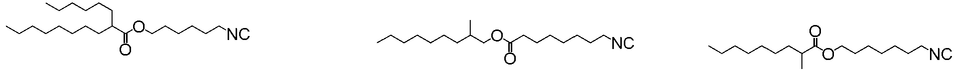

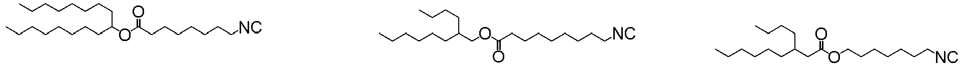

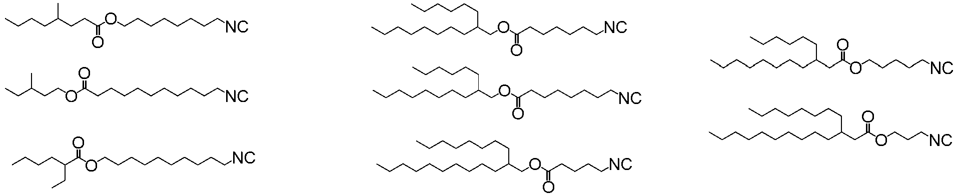

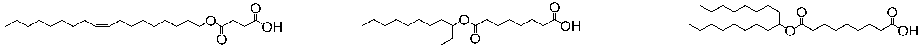

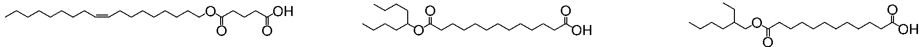

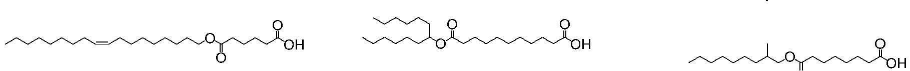

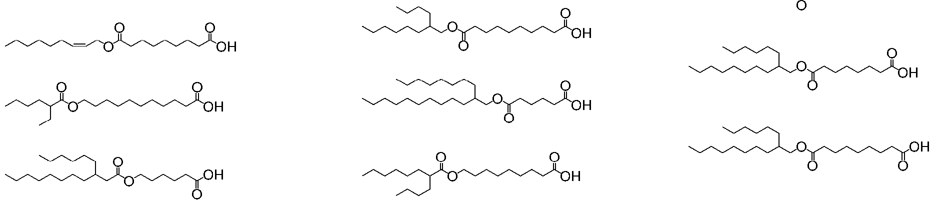

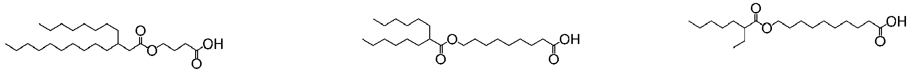

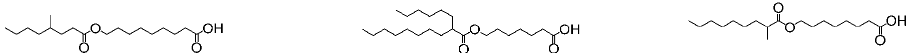

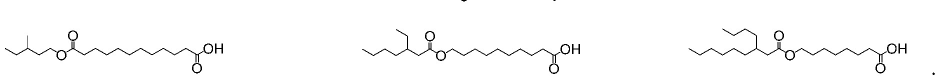

In one aspect, the present disclosure provides a preparation method of a lipid compound of formula (II), the preparation method comprising the following synthesis route:
when R_{B} is selected from 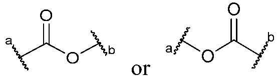 and R_{D} is selected -NC, 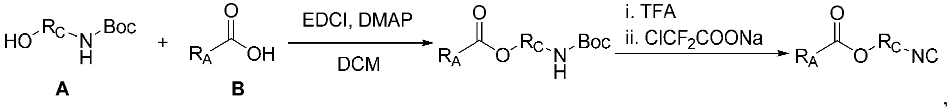 or

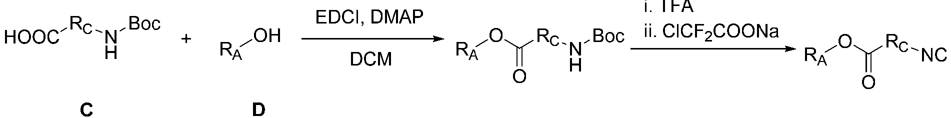

In some embodiments, the synthesis route of the lipid compound of formula (II) comprises:
N-Boc protected alcohol (1 eq) of compound A and a carboxyl acid compound (1 eq) of compound B are dissolved into a one-necked flask filled with dichloromethane (DCM), N-(3-dimethylaminopropyl)-N'-ethyl carbodiimide hydrochloride (1 eq) and 4-(dimethylamino) pyridine (0.1 eq) are then added, and the above materials are sufficiently stirred at normal temperature overnight. After the reaction is ended, a solvent is removed in vacuum, and a residue (petroleumether (PE) of 0-10% ethyl acetate (EA)) is purified by a flash column. After loading onto the column, the product is dissolved with DCM, an equal volume of trifluoroacetic acid (TFA) is slowly dropwise added, and then the above mixed solution is sufficiently stirred for 3 h. After the reaction is ended, an equal volume of saturated sodium bicarbonate solution is added, and the above reaction solution is extracted with DCM three times, dried over anhydrous sodium sulfate, filtered and undergoes rotary evaporation to remove the solvent, so as to obtain a crude product. The crude product (1 eq) is dissolved into a one-necked flask filled with dimethylformamide (DMF), sodium chlorodifluoroacetate (2 eq) and potassium carbonate (2 eq) are then added, the above system is subjected to nitrogen replacement and then heated, and subsequently stirred for 12 h in oil bath at 100°C. After the system is cooled to room temperature, dichloromethane is added, and then the system is washed with a large number of deionized water four times, washed with a saturated saline solution once, and dried over anhydrous sodium sulfate. The solvent is removed in vacuum, and the residue (PE of 0-10%EA) is purified through the flash column.

In some embodiments, the synthesis route of the lipid compound of formula (II) comprises:
N-Boc protected carboxyl acid (1 eq) of compound C and an alcohol compound (1 eq) of compound D are dissolved into a one-necked flask filled with DCM, N-(3-dimethylaminopropyl)-N'-ethyl carbodiimide hydrochloride (1 eq) and 4-(dimethylamino) pyridine (0.1 eq) are then added, and the above materials are sufficiently stirred at normal temperature overnight. After the reaction is ended, a solvent is removed in vacuum, and a residue (PE of 0-10% EA) is purified by a flash column. After loading onto the column, the product is dissolved with DCM, an equal volume of TFA is slowly dropwise added, and then the above mixed solution is sufficiently stirred for 3 h. After the reaction is ended, an equal volume of saturated sodium bicarbonate solution is added, the above reaction solution is extracted with DCM three times, dried over anhydrous sodium sulfate, filtered and undergoes rotary evaporation to remove the solvent, so as to obtain a crude product. The crude product (1 eq) is dissolved into a one-necked flask filled with DMF, sodium chlorodifluoroacetate (2 eq) and potassium carbonate (2 eq) are then added, the above system is subjected to nitrogen replacement and then heated, and subsequently stirred for 12 h in oil bath at 100°C. After the system is cooled to room temperature, dichloromethane is added, and the system is washed with a large number of deionized water four times, washed with a saturated saline solution once, and dried over anhydrous sodium sulfate. The solvent is removed in vacuum, and the residue (PE of 0-10% EA) is purified through the flash column. 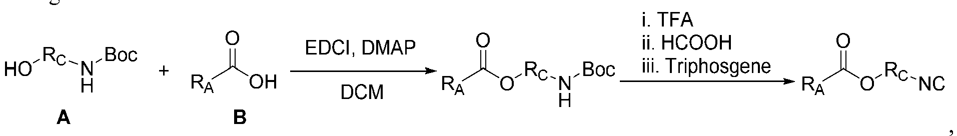 or

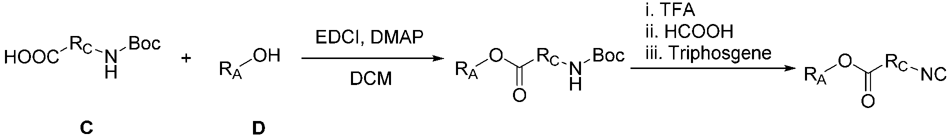

In some embodiments, the first-step reaction is the same as the previous reaction, and therefore is not described in detail here; a second-step reaction: an intermediate (2 mol/L concentration) is dissolved into a solution of 50% TFA in DCM, and the above materials react for 2 h at room temperature under the condition of stirring, and the obtained product is washed and loaded onto a column to obtain primary amine. Then, the primary amine (5 mol/L) is dissolved into a formic acid solution to react at 90°C overnight. After the reaction is ended, excess formic acid is removed by rotary evaporation, the remaining product is washed, extracted and loaded onto the column to obtain a formamide intermediate. In ice bath, formamide and 1,4-diazabicyclo[2.2.2]octane (DABCO) (3 eq) are dissolved into DCM and then a solution of triphosgene in DCM is slowly dropwise added, the above materials react for half an hour under the condition of stirring, and then the reaction solution is poured into a 0.5 M saturated sodium carbonate solution, extracted with DCM, dried over anhydrous sodium sulfate and loaded onto the column to obtain a target isonitrile compound. 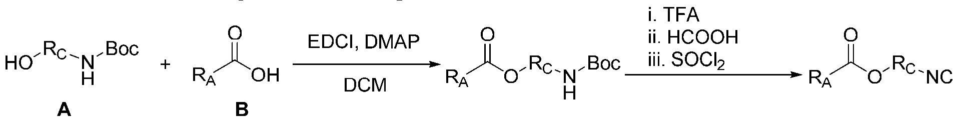 , or

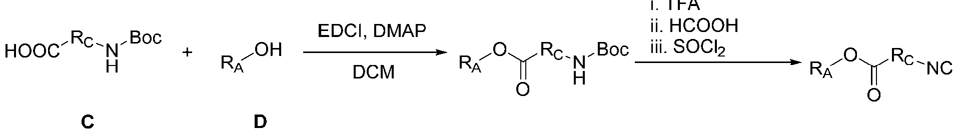

In some embodiments, the first-step reaction is the same as the previous reaction, and therefore is not described in detail; a second-step reaction: an intermediate (2 mol/L concentration) is dissolved into a solution of 50% TFA in DCM to react for 2 h at room temperature under the condition of stirring, and the obtained product is washed and loaded onto a column to obtain primary amine. Then, the primary amine (5 mol/L) is dissolved into a formic acid solution to react at 90°C overnight. After the reaction is ended, excess formic acid is removed by rotary evaporation, and the remaining product is washed, extracted and loaded onto the column to obtain a formamide intermediate. Under the protection of nitrogen, the formamide intermediate is dissolved into DMF (0.5 mol/L), the reaction temperature is controlled at -50°C, a solution (1.1 eq) of 3M SOCl₂ in DCM is dropwise added, then anhydrous sodium carbonate (2 eq) is added, the reaction temperature is gradually restored to room temperature, and then the above materials are stirred overnight. After the reaction is ended, 2-fold volume amount of water is added, and the reaction system is extracted with EA, washed with a saturated saline solution, dried and loaded onto the column to obtain a target isonitrile compound. 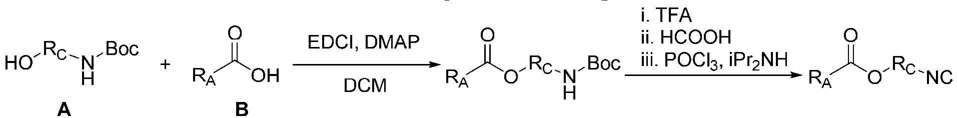 , or

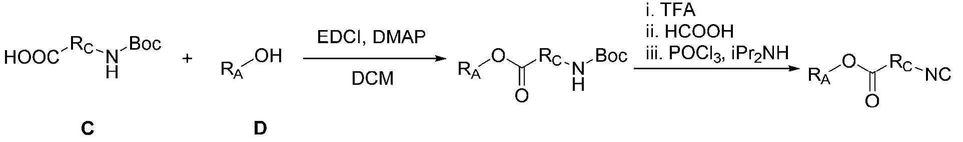

In some embodiments, the first-step reaction is the same as the previous reaction, and therefore is not described in detail; a second-step reaction: an intermediate (2 mol/L concentration) is dissolved into a solution of 50% TFA in DCM to react for 2 h at room temperature under the condition of stirring, and the obtained product is washed and loaded onto a column to obtain primary amine. Then, the primary amine (5 mol/L) is dissolved into a formic acid solution to react at 90°C overnight. After the reaction is ended, excess formic acid is removed by rotary evaporation, and the remaining product is washed, extracted and loaded onto the column to obtain a formamide intermediate. In ice bath, the formamide intermediate is dissolved into DCM (1 mol/L), diisopropylamine (3 eq) is added, then phosphorus oxychloride (1.1 eq) is slowly dropwise added, and the above materials react for 1 h at 0°C and then react for 8 h at room temperature. After the reaction is ended, a saturated sodium carbonate solution is added until pH reaches weak alkaline, the above reaction solution was stirred for 1 h at room temperature, an equal volume of water is added, and then the reaction system was extracted with DCM, dried over anhydrous sodium sulfate and loaded onto the column to obtain a target isonitrile compound. 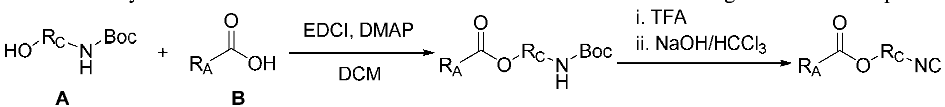 , or

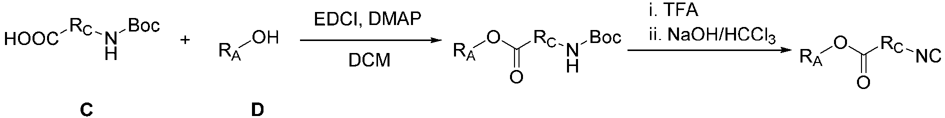

In some embodiments, the first-step reaction is the same as the previous reaction, and is not described in detail; a second-step reaction: an intermediate (2 mol/L concentration) is dissolved into a solution of 50% TFA in DCM to react for 2 h at room temperature under the condition of stirring, and the obtained product is washed and loaded onto a column to obtain primary amine. The primary amine is dissolved into DCM (4 mol/L), an equal volume of 50% NaOH aqueous solution is added, chloroform (1 eq) is added, a phase transfer catalyst benzyltriethylammonium chloride (10 g/L) is added, the reaction is refluxed overnight, and the obtained product is diluted with an equal volume of water, extracted with DCM, dried over anhydrous sodium sulfate and loaded onto the column to obtain a target isonitrile compound. 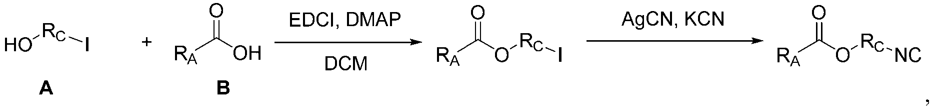 or

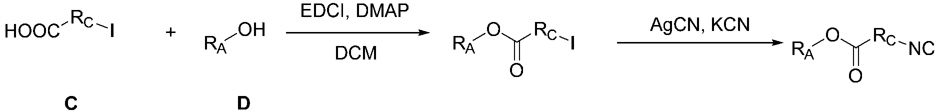

In some embodiments, the first-step reaction is the same as the previous reaction, and is not described in detail; a second-step reaction: an iodo intermediate is dissolved into tetrahydrofuran (THF), AgCN (1.1 eq) is added, and the above materials were subjected to heating reflux and violently stirred for 2 h. An equal volume of water is added, an aqueous solution of KCN (3 eq) is then dropwise added, and the above materials are stirred for 20 min. The above reaction product is subjected to rotary evaporation to remove an organic solvent, extracted with EA, dried over anhydrous sodium sulfate and loaded onto a column to obtain a target isonitrile compound.

When R_{B} is selected from 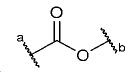 or 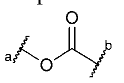 and R_{D} is selected from -COOH, 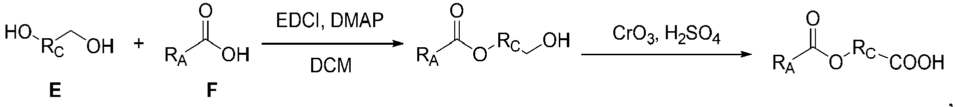 or

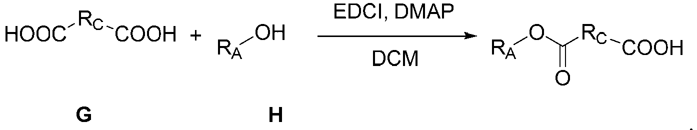

In some embodiments, the synthesis route of the lipid compound of formula (II) comprises:
Diol (1 eq) of compound E and a carboxyl acid compound (1 eq) of compound F are dissolved into a one-necked flask filled with DCM, N-(3-dimethylaminopropyl)-N'-ethyl carbodiimide hydrochloride (1 eq) and 4-(dimethylamino) pyridine (0.1 eq) are than added, and subsequently the above materials are sufficiently stirred at room temperature overnight. After the reaction is ended, a solvent is removed in vacuum, and a residue (PE of 0-10% EA) is purified through a flash column. After loading onto the column, the product is dissolved with acetone and dropwise added into a sulfuric acid aqueous solution of chromium trioxide for 1 h of reaction. The pH of the system is adjusted using a saturated sodium bicarbonate solution to be weak alkaline and then extracted with diethyl ether twice, and a water phase is acidified using sulfuric acid, extracted with diethyl ether and dried. A solvent is removed in vacuum, and a residue (PE of 0-20% EA) is purified through a flash column.

In some embodiments, the synthesis route of the lipid compound of formula (II) comprises:
A diacid (1 eq) of compound G and an alcohol compound (1 eq) of compound H are dissolved into a one-necked flask filled with DCM, N-(3-dimethylaminopropyl)-N'-ethyl carbodiimide hydrochloride (1 eq) and 4-(dimethylamino) pyridine (0.1 eq) are than added, and the above materials are sufficiently stirred at room temperature overnight. After the reaction is ended, a solvent is removed in vacuum, and a residue (PE of 0-20% EA) is purified through a flash column. 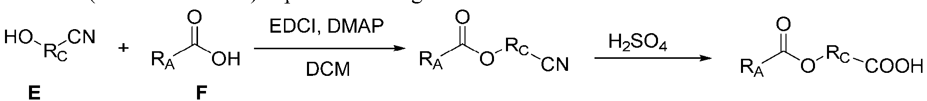 , or

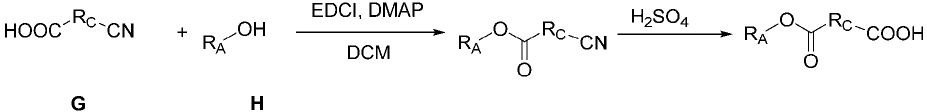

In some embodiments, the first-step reaction is the same as the previous reaction, and therefore is not described in detail; a second-step reaction: the obtained cyano intermediate is dissolved into a 20% sulfuric acid solution, the above mixed solution is heated to 80°C and reacts overnight. After the reaction is ended, a 3-fold volume of water is added, and the above system is extracted with EA, dried with anhydrous sodium sulfate and then loaded onto a column to obtain a target carboxylic acid compound. 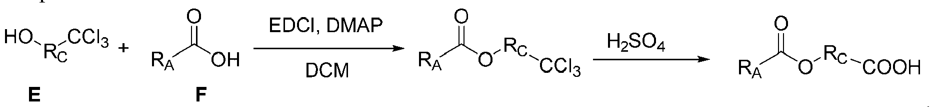 or

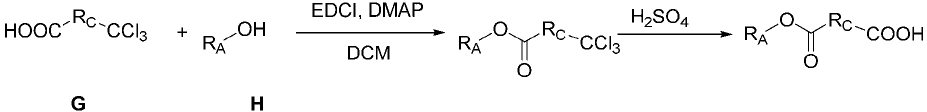

In some embodiments, the first-step reaction is the same as the previous reaction, and therefore is not described in detail; a second-step reaction: the obtained trichlorine intermediate is dissolved into a 10% sulfuric acid solution, and the above mixed solution is heated to 90°C and reacts overnight. After the reaction is ended, a 3-fold volume of water is added, and the above system is extracted with EA, dried over anhydrous sodium sulfate and then loaded onto a column to obtain a target carboxylic acid compound. 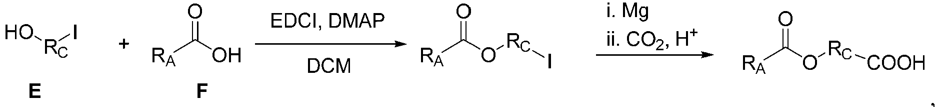 or

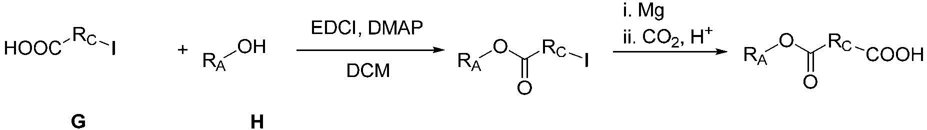

In some embodiments, the first-step reaction is the same as the previous reaction, and therefore is not described in detail; a second-step reaction: under the protection of nitrogen, magnesium debris (1 eq) is added into THF, a solution of an iodo intermediate in THF is slowly dropwise added at room temperature, and then the above mixed solution is gradually heated to reflux and reacts for 4 h. After the reaction is ended, excess carbon dioxide ice is added, and the above materials are violently stirred and react for 2 h. After the temperature is restored to room temperature, an equal volume of water is added, diluted hydrochloric acid is used to adjust the pH of the system to 2, and then the above system is extracted with EA, dried over anhydrous sodium sulfate and loaded onto a column for separation, so as to obtain a target carboxylic acid compound.

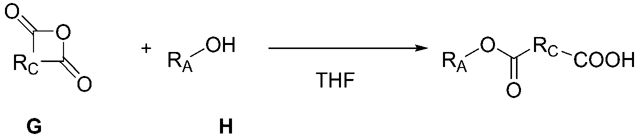

In some embodiments, anhydride is dissolved into THF (2 mol/L), alcohol (1 eq) is slowly dropwise added, the above mixed solution is heated to reflux and reacts overnight, an equal volume of water is then added, diluted hydrochloric acid is used to adjust the pH of the system to 2, and then the above system is extracted with EA, dried over anhydrous sodium sulfate and loaded onto a column for separation, so as to a target carboxylic acid compound.

### A Lipid compound containing an isonitrile or carboxylic acid end group is used for preparing an ionizable lipid

In one aspect, the present disclosure provides use of a lipid compound of formula (II) in the preparation of ionizable lipids. The ionizable lipids are lipid compounds of formulas (I), (III), (IV), (V) and (VI) of the present disclosure as shown below. 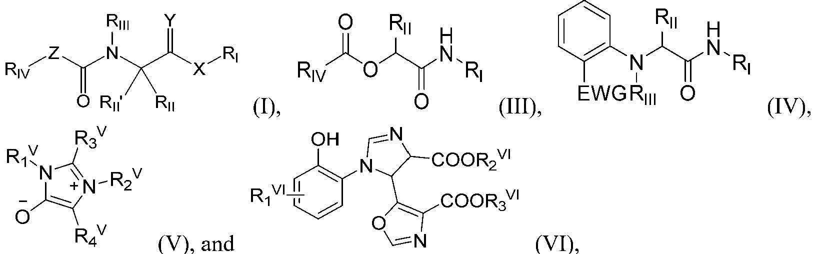 wherein each substituent in the formulas (I), (III), (IV), (V) and (VI) is as defined herein.

In one aspect, the present disclosure provides use of a lipid compound of formula (II) in the preparation of an ionizable lipid of formula (I) of the present disclosure. The preparation comprises the following Ugi reaction synthesis route: 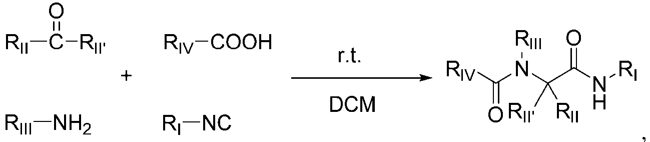 wherein R_{I}, R_{II}, R_{II}', R_{III} and R_{IV} are as defined herein, R_{I}-NC and R_{IV}-COOH are each independently selected from the lipid compounds of formula (II) of the present disclosure.

In some embodiments, the synthesis route for preparing the ionizable lipid of formula (I) of the present disclosure comprises:
Amine (1 mmol, 1.0 eq) and aldehyde or ketone (1 mmol, 1.0 eq) are dissolved into 10 mL of ethanol. After stirring for 10 min, an acid (1 mmol, 1.0 eq) is added, and then the materials are stirred for 10 min. Isocyan (1 mmol, 1.0 eq) is added, and then the obtained solution is stirred for 12 h at room temperature. A solvent is removed in vacuum, and a residue (DCM of 0-5% MeOH) is purified through a flash column to obtain a target product.

In one aspect, the present disclosure provides use of a lipid compound of formula (II) in the preparation of an ionizable lipid of formula (III) of the present disclosure. The preparation comprises the following Passerini reaction synthesis route: 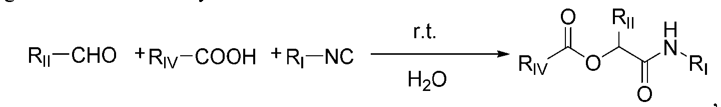 wherein R_{I}, R_{II} and R_{IV} are as defined herein, R_{I}-NC and R_{IV}-COOH are each independently selected from the lipid compounds of formula (II) of the present disclosure.

In some embodiments, the synthesis route for preparing the ionizable lipid of formula (III) of the present disclosure comprises:
An acid (1 mmol, 1.0 eq) is added into 5 mL of water, aldehyde (1 mmol, 1.0 eq) and isocyan (1 mmol, 1.0 eq) are then added, and the above materials are violently stirred at room temperature. After 4 h of reaction, a solvent is removed in vacuum, and a residue (DCM of 5% MeOH) is purified through a flash column.

In some embodiments, the preparation comprises the following synthesis route: 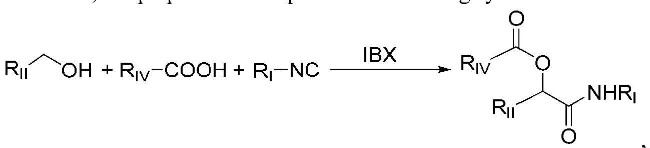 wherein R_{I}, R_{II} and R_{IV} are as defined herein, and R_{I}-NC and R_{IV}-COOH are each independently selected from the lipid compounds of formula (II) of the present disclosure.

In some embodiments, the synthesis route for preparing the ionizable lipid of formula (III) of the present disclosure comprises:
Alcohol (1.5 mmol, 1.5 eq) is added into 2 mL of THF, isocyan (1.0 mmol, 1.0 eq) and an acid (1.5 mmol, 1.5 eq) are then added, subsequently 2-iodoxybenzoic acid (2 mmol, 2.0 eq) is added, and the above materials react for 24 h at 40°C. After the reaction is ended, a solvent is removed in vacuum, and a residue (DCM of 0-5% MeOH) is purified through a flash column.

In one aspect, the present disclosure provides use of a lipid compound of formula (II) in the preparation of an ionizable lipid of formula (IV) of the present disclosure. The preparation comprises the following Ugi-Smiles reaction synthesis route: 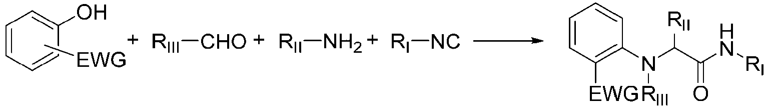 , wherein R_{I}, R_{II} and R_{III} are as defined herein, and R_{I}-NC is selected from the lipid compounds of formula (II) of the present disclosure.

In some embodiments, the synthesis route of the ionizable lipid of formula (IV) of the present disclosure comprises:
Amine (1 mmol, 1.0 eq) and aldehyde (1 mmol, 1.0 eq) are dissolved into 10 mL of toluene. The above mixed solution is stirred for 10 min, phenol (1 mmol, 1.0 eq) is then added, and subsequently the materials are stirred for 10 min. Isocyan (1 mmol, 1.0 eq) is added, and then the obtained solution is stirred for 24 h at 100°C. A solvent is removed in vacuum, and a residue (DCM of 0-5% MeOH) is purified through a flash column to obtain a target product.

In one aspect, the present disclosure provides use of a lipid compound of formula (II) in the preparation of an ionizable lipid of formula (V) of the present disclosure. The preparation comprises the following Ugi-Smiles reaction synthesis route: 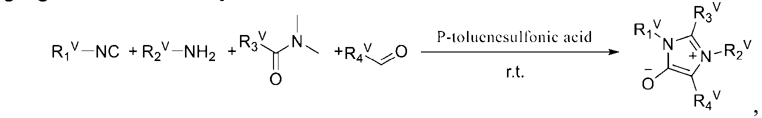 wherein R₁^{V}, R₂^{V}, R₃^{V} and R₄^{v} are as defined herein, and R₁^{IV}-NC is selected from the lipid compounds of formula (II) of the present disclosure.

In some embodiments, the synthesis route for preparing the ionizable lipid of formula (V) of the present disclosure comprises:
Aldehyde (1 mmol, 1.0 eq) is added into 2.5 mL of DMF (or other N-diformamide sources), amine (1 mmol, 1.0 eq) and isocyan (1 mmol, 1.0 eq) are then added, and the above materials are violently stirred at room temperature. After reaction for 16 h, a solvent is removed in vacuum, and a residue (DCM of 0-5% MeOH) is purified through a flash column.

In one aspect, the present disclosure provides use of a lipid compound of formula (II) in the preparation of an ionizable lipid of formula (VI) of the present disclosure. The preparation comprises the following reaction synthesis route: 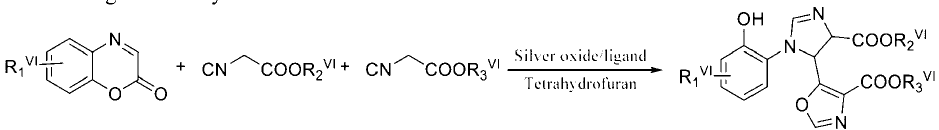 wherein R₁^{VI}, R₂^{VI} and R₃^{VI} are as defined herein, and 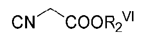 and 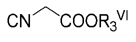 are each independently selected from the lipid compounds of formula (II) of the present disclosure.

In some embodiments, the synthesis route for preparing the ionizable lipid of formula (VI) of the present disclosure comprises:
Imidoester (1 mmol, 1.0 eq) is added into 2.5 mL of THF, silver oxide (0.1 mmol, 0.1 eq) and a ligand (0.2 mmol, 0.2 eq) are added, first isonitrile (1 mmol, 1.0 eq) is then added, the above materials react for 24 h at 30°C below zero, subsequently, second isonitrile (1 mmol, 1.0 eq) is added, and the above system reacts for 12 h at 0°C and then reacts for 12 h at room temperature. After the reaction is ended, a solvent is removed in vacuum, and a residue (DCM of 0-5% MeOH) is purified through a flash column.

### Liposome

In some aspects, the present disclosure relates to a liposome. The liposome of the present disclosure comprises the lipid compound of the present disclosure as described above. When being used for preparing the liposome of the present disclosure, the lipid compound of the present disclosure is also referred to as a cationic lipid or an ionizable lipid.

In some embodiments, in the liposome of the present disclosure, based on the total molar amount of the components constituting the liposome, the lipid compound accounts for about 10 mol% to about 90 mol%, for example about 10 mol%, 15 mol%, 20 mol%, 25 mol%, 30 mol%, 40 mol%, 45 mol%, 50 mol%, 55 mol%, 60 mol%, 65 mol%, 70 mol%, 75 mol%, 80 mol%, 85 mol%, 90 mol%, or is in a range between any two of the above. Preferably, the lipid compound accounts for about 10 mol% to about 70 mol%, more preferably, the lipid compound accounts for about 20 mol% to about 50 mol%.

In some embodiments, the liposome of the present disclosure also comprises a phospholipid and cholesterol.

In some embodiments, based on the total molar amount of the components constituting the liposome, the phospholipid accounts for about 0 mol% to about 20 mol%, or is in a range between any numerical values of about 0 mol% to about 20 mol%.

In some embodiments, based on the total molar amount of the components constituting the liposome, the cholesterol accounts for about 30 mol% to about 50 mol%, or is in a range between any numerical values of about 30 mol% to about 50 mol%.

In one aspect, the present disclosure provides use of a liposome of the present disclosure as a delivery vehicle, for example as a delivery vehicle for delivering plasmids and nucleic acids such as RNA.

In one aspect, the present disclosure provides a drug delivery system, comprising a liposome of the present disclosure as a delivery vehicle, for example for delivering plasmids and nucleic acids such as RNA.

A preparation method of a liposome has been widely known in the art. The liposome of the present disclosure can be prepared by using a conventional method known by those skilled in the art.

### Lipid nanoparticle

In some aspects, the present disclosure relates to a lipid nanoparticle. The lipid nanoparticle of the present disclosure comprises the lipid compound of the present disclosure as described above. When being used for preparing the lipid nanoparticle of the present disclosure, the lipid compound of the present disclosure can also be referred to as a cationic lipid or an ionizable lipid.

In some embodiments, in the lipid nanoparticle of the present disclosure, based on the total molar amount of the components constituting the lipid nanoparticle, the lipid compound accounts for about 10 mol% to about 90 mol%, for example about 10 mol%, about 15 mol%, about 20 mol%, about 25 mol%, about 30 mol%, about 40 mol%, about 45 mol%, about 50 mol%, about 55 mol%, about 60 mol%, about 65 mol%, about 70 mol%, about 75 mol%, about 80 mol%, about 85 mol%, about 90 mol%, or is in a range between any two of the above. Preferably, the lipid compound accounts for about 10 mol% to about 70 mol%, more preferably, the lipid compound accounts for about 20 mol% to about 50 mol%.

In some embodiments, the lipid compound of the present disclosure also comprises a phospholipid, a structural lipid or a PEG lipid.

In some embodiments, based on the total molar amount of the components constituting the lipid nanoparticle, the phospholipid accounts for about 0 mol% to about 20 mol%, or is in a range between any numerical values of about 0 mol% to about 20 mol%.

In some embodiments, based on the total molar amount of the components constituting the lipid nanoparticle, the structural lipid accounts for about 30 mol% to about 50 mol%, or is in a range between any numerical values of about 30 mol% to about 50 mol%.

In some embodiments, based on the total molar amount of the components constituting the lipid nanoparticle, the PEG lipid accounts for about 0 mol% to about 10 mol%, or is in a range between any numerical values of about 0 mol% to about 10 mol%.

In some embodiments, the lipid nanoparticle of the present disclosure also comprises a nucleic acid.

In some embodiments, an N/P ratio of the lipid compound to the nucleic acid is about 1.1:1 to 10:1, or is in a range between any numerical values of 1.1:1 to 10:1. Herein, the N/P ratio can be defined as, in the lipid nanoparticle containing the nucleic acid, a ratio of a number of N atoms in the ionizable group contained in the lipid compound (also referred to as "cationic lipid" or "ionizable lipid") to a number of P atoms in the phosphate group of the nucleic acid. The N/P ratio can be calculated based on the following: for example, 1 µg of RNA generally contains about 3 nmol of phosphate residues, provided that RNA presents a statistical distribution of bases. The "N" value of the lipid compound can be calculated according to its molecular weight and a relative content of cationic groups. If more than one lipid compound is present, the N value should be calculated based on all lipid compounds contained in the lipid nanoparticle.

In some embodiments, a ratio of the lipid compound to the nucleic acid is calculated based on a mass ratio.

In some embodiments, examples of the nucleic acid include single-stranded and double-stranded DNA, single-stranded and double-stranded RNA, and a hybridized molecule having a mixture of single-stranded and double-stranded DNA and RNA. In some embodiments, examples of the nucleic acid include any types of RNA, for example messenger RNA (mRNA), small interfering RNA (siRNA), short-hairpin RNA (shRNA), micro RNA (miRNA), guide RNA (gRNA), single-stranded guide RNA (sgRNA), CRISPR RNA (crRNA), trans-activating RNA (tracerRNA), plasmid DNA (pDNA), small circular DNA, genomic DNA (gDNA) and any fragments thereof. In some embodiments, the nucleic acid contained in the lipid nanoparticle of the present disclosure can be a mixture of one or more nucleic acid molecules.

In some embodiments, the lipid nanoparticle of the present disclosure also comprises a plasmid.

In one aspect, the present disclosure provides use of a lipid nanoparticle as a delivery vehicle, for example a delivery vehicle for delivering plasmids and nucleic acids such as RNA.

In one aspect, the present disclosure provides a drug delivery system, comprising the lipid nanoparticle of the present disclosure as a delivery vehicle, for example for delivering plasmids and nucleic acids such as RNA.

In one aspect, the present disclosure provides a lipid nanoparticle coating a nucleic acid molecule such as RNA.

In one aspect, the present disclosure provides a lipid nanoparticle coating plasmids.

The preparation method of the lipid nanoparticle has been widely known in the art. The lipid nanoparticle of the present disclosure can be prepared by using a conventional method known by those skilled in the art.

### Pharmaceutical composition

In some aspects, the present disclosure relates to a pharmaceutical composition, comprising a lipid compound of formula (I) as provided herein, or a stereoisomer or pharmaceutically acceptable salt thereof, and at least one pharmaceutically acceptable carrier or excipients. In some embodiments, the pharmaceutical composition comprises the lipid nanoparticle as provided herein, and at least one pharmaceutically acceptable carrier or excipients.

As used herein, the term "pharmaceutically acceptable carrier or excipients" refers to a carrier or excipients that can be used for preparing a pharmaceutical composition, which are often safe and non-toxic and are not unexpected biologically or in other aspects, and comprise an acceptable carrier or excipients for veterinary and human drug use. The pharmaceutically acceptable carrier or excipients as used herein comprise one and more than one of such the carrier or excipients. The used specific carrier or excipients will depend on a manner and purpose for applying the compound of the present disclosure. The proper carrier or excipients are known by those skilled in the art, and described in detail in, for example, Ansel, Howard C et al, Ansel's Pharmaceutical Dosage Forms and Drug Delivery Systems. Philadelphia: Lippincott, Williams & Wilkins, 2004; Gennaro, Alfonso R. et al., Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The excipients also comprise one or more of a buffering agent, a stabilizing agent, a surfactant, a wetting agent, a lubricating agent, an emulsifier, a suspension, a preservative, an antioxidant, a light-screening agent, a flow aid, a processing aid, a colorant, a sweetener, an aromatizing agent, a flavouring agent, a diluent and other known additives to provide the exquisite expression of a drug (i.e., the compound or pharmaceutical composition provided herein) or facilitate the production of a drug product (i.e., the drug).

The composition of the present disclosure can be prepared into multiple forms. These include, for example, liquid, semi-solid and solid dosage forms, for example a liquid solution (for example, injectable and infusional solutions), a dispersion or a suspension, a tablet, a pill, a powder, a liposome, a suppository, etc. The forms depend on expected administration manners and therapeutic application.

The pharmaceutical composition of the present disclosure can be prepared by any known pharmacological technology (for example effective formulations and administration programs). The consideration about effective formulations and administration programs as described above is known in the art, and described in standard textbooks. For example, formulations of a drug product are discussed in Hoover, John E., Remington's Pharmaceutical Sciences, Mack Publishing Co., Easton, Pennsylvania, 1975; Liberman et al, edition, Pharmaceutical Dosage Forms, Marcel Decker, New York, N.Y., 1980; and Kibbe et al, edition, Handbook of Pharmaceutical Excipients, 3rd edition, American Pharmaceutical Association, Washington, 1999.

### Examples

To provide more detailed description of the present disclosure, the following examples are proposed. Examples described herein are used for explaining a lipid compound, a preparation method, a lipid nanoparticle and a pharmaceutical composition provided in the present disclosure, and should not be construed in any way as limiting their scopes.

During the synthesis, it may be necessary and/or desirable to protect sensitive or reactive groups on any molecule of interest, which can be achieved by conventional protecting groups, for example, those described in T.W. Greene and P.G.M. Wutts, Protecting Groups in Organic Synthesis, 4th edition, John Wiley and Sons. Methods known in the art are used to conveniently and optionally remove protecting groups in subsequent stages.

The lipid compound of the present disclosure can be readily prepared by using easily obtained starting materials, reagents and conventional synthesis programs according to reaction schemes and examples or their modifications below. In these reactions, variants that are known by those skilled in the art but not involved in more detail can also be used. In addition, according to the reaction schemes and examples described herein, other methods for preparing the lipid compound of the present disclosure are apparent to those skilled in the art. Unless otherwise specified, all variables are as defined above. Generally speaking, in the chemical program, all reagents and starting materials are commercially available from commercial suppliers or easily prepared by those skilled in the art.

### Example 1

The structure of synthesized lipid isonitrile is as follows:

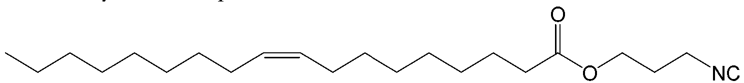

Oleic acid (2.8247 g, 1 eq), N-Boc-3-amino-1-propanol (1.7523 g, 1 eq), N-(3-dimethylaminopropyl)-N'-ethyl carbodiimide hydrochloride (1.9170 g, 1 eq) and 4-(dimethylamino) pyridine (0.1222 g, 0.1 eq) were successively dissolved into a 250 mL one-necked flask filled with 50 mL of DCM, and sufficiently stirred for 18 h at room temperature. After the reaction was ended, a solvent was removed in vacuum, and a residue (PE of 0-10% EA) was purified through a flash column. After loading onto the column, the product was dissolved with 10 mL of DCM, 10 mL of TFA was then slowly dropwise added, and subsequently the above mixed solution was sufficiently stirred for 3 h. After the reaction was ended, 20 mL of saturated sodium bicarbonate solution was added, the above mixed solution was extracted with DCM three times, dried over anhydrous sodium sulfate, filtered, and subjected to rotary evaporation to remove the solvent so as to obtain a crude product 3-aminopropyl oleate. 3-aminopropyl oleate (4.2230 g, 1 eq), sodium chlorodifluoroacetate (2.3623 g, 2 eq) and potassium carbonate (3.4553 g, 2 eq) were taken and added into a 500 mL one-necked flask, 100 mL of DMF was added into the flask, and then the above reaction system was heated after nitrogen replacement and then stirred for 12 h in oil bath at 100°C. After the system was cooled to room temperature, 100 mL of dichloromethane was added, the system was then washed with a large amount of deionized water four times, washed with a saturated saline solution once, and dried over anhydrous sodium sulfate. A solvent was removed in vacuum, and a residue was purified through the flash column (PE of 0-10% EA).
¹H NMR (500 MHz, CDCl₃, ppm): δ 5.34 (m, 2H), 4.21 (t, 2H), 3.51 (t, 2H), 2.31 (t, 2H), 2.01 (m, 6H), 1.61 (m, 2H), 1.26 (m, 20H), 0.88 (t, 3H). LCMS (APCI): 350.2 m/z (M+H⁺). Chemical formula: C₂₂H₃₉NO₂

### Example 2

The structure of synthesized lipid isonitrile is as follows:

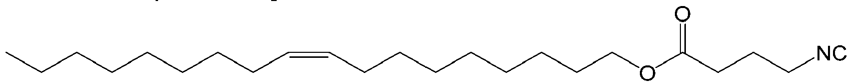

N-Boc-γ-aminobutyric acid (2.1604 g, 1 eq), cis-9-octadecenol (2.8561 g, 1 eq), N-(3-dimethylaminopropyl)-N'-ethyl carbodiimide hydrochloride (2.0378 g, 1 eq) and 4-(dimethylamino) pyridine (0.1299 g, 0.1 eq) were successively dissolved into a 250 mL one-necked flask filled with 50 mL of DCM, and sufficiently stirred for 18 h at room temperature. After the reaction is ended, a solvent was removed in vacuum, and a residue (PE of 0-10% EA) was purified through a flash column. After loading onto the column, the product was dissolved with 10 mL of DCM, 10 mL of TFA was then slowly dropwise added, and subsequently the above mixed solution was sufficiently stirred for 3 h. After the reaction was ended, 20 mL of saturated sodium bicarbonate solution was added, the above mixed solution was extracted with DCM three times, dried over anhydrous Na₂SO₄, filtered, and subjected to rotary evaporation to remove the solvent so as to obtain a crude product (Z)-dodecan-9-en-1-yl-4-aminobutyrate. (Z)-dodecan-9-en-1-yl-4-aminobutyrate (4.9320 g, 1 eq), sodium chlorodifluoroacetate (2.6457 g, 2 eq) and potassium carbonate (3.8699 g, 2 eq) were taken and added into a 500 mL one-necked flask, 100 mL of DMF was added into the flask, and then the above reaction system was heated after nitrogen replacement and then stirred for 12 h in oil bath at 100°C. After the system was cooled to room temperature, 100 mL of dichloromethane was added, the system was then washed with a large amount of deionized water four times, washed with a saturated saline solution once, and dried over anhydrous sodium sulfate. A solvent was removed in vacuum, and a residue was purified through the flash column (PE of 0-10% EA).
¹H NMR (500 MHz, CDCl₃, ppm): δ 5.34 (m, 2H), 4.06 (t, 2H), 3.16 (t, 2H), 2.35 (t, 2H), 2.01 (m, 4H), 1.80 (m, 2H), 1.60 (m, 2H), 1.29 (m, 22H), 0.88 (t, 3H). LCMS (APCI): 364.2 m/z (M+H⁺). Chemical formula: C₂₃H₄₁NO₂

### Example 3

The structure of synthesized lipid isonitrile is as follows:

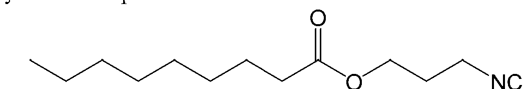

Nonanoic acid (1.6325 g, 1 eq), N-Boc-γ-amino-1-propanol (1.9136 g, 1 eq), dicyclohexylcarbodiimide (2.0216 g, 1 eq) and 4-(dimethylamino) pyridine (0.1312 g, 0.1 eq) were successively dissolved into a 250 mL one-necked flask filled with 50 mL of DCM, and sufficiently stirred for 18 h at room temperature. After the reaction is ended, a solvent was removed in vacuum, and a residue (PE of 0-10% EA) was purified through a flash column. After loading onto the column, the product was dissolved with 10 mL of DCM, 10 mL of TFA was then slowly dropwise added, and subsequently the above mixed solution was sufficiently stirred for 3 h. After the reaction was ended, 20 mL of saturated sodium bicarbonate solution was added, and the above mixed solution was extracted with DCM three times, dried over anhydrous Na₂SO₄, filtered, and subjected to rotary evaporation to remove the solvent so as to obtain a crude product 3-propyl aminononanoate. 3-propyl aminononanoate (3.5120 g, 1 eq), sodium chlorodifluoroacetate (3.1182 g, 2 eq) and potassium carbonate (4.5603 g, 2 eq) were taken and added into a 500 mL one-necked flask, 100 mL of DMF was added into the flask, and then the above reaction system was heated after nitrogen replacement and then stirred for 12 h in oil bath at 100°C. After the system was cooled to room temperature, 100 mL of dichloromethane was added, the system was then washed with a large number of deionized water four times, washed with a saturated saline solution once, and dried over anhydrous sodium sulfate. A solvent was removed in vacuum, and a residue was purified through the flash column (PE of 0-10% EA).
¹H NMR (500 MHz, CDCl₃, ppm): δ 4.21 (t, 2H), 3.51 (t, 2H), 2.31 (t, 2H), 2.03 (m, 2H), 1.61 (m, 2H), 1.29 (m, 10H), 0.88 (t, 3H). LCMS (APCI): 225.9 m/z (M+H⁺). Chemical formula: C₁₃H₂₃NO₂

### Example 4

The structure of synthesized lipid isonitrile is as follows:

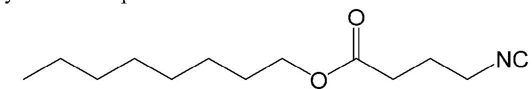

N-Boc-γ-aminobutyric acid (2.1604 g, 1 eq), n-octanol (1.3819 g, 1 eq), dicyclohexylcarbodiimide (2.0216 g, 1 eq) and 4-(dimethylamino) pyridine (0.1299 g, 0.1 eq) were successively dissolved into a 250 mL one-necked flask filled with 50 mL of DCM, and sufficiently stirred for 18 h at room temperature. After the reaction is ended, a solvent was removed in vacuum, and a residue (PE of 0-10% EA) was purified through a flash column. After loading onto the column, the product was dissolved with 10 mL of DCM, 10 mL of TFA was then slowly dropwise added, and subsequently the above mixed solution was sufficiently stirred for 3 h. After the reaction was ended, 20 mL of saturated sodium bicarbonate solution was added, and the above mixed solution was extracted with DCM three times, dried over anhydrous Na₂SO₄, filtered, and subjected to rotary evaporation to remove the solvent so as to obtain a crude product 4-octyl aminobutyrate. 4-octyl aminobutyrate (4.3068 g, 1 eq), sodium chlorodifluoroacetate (3.7796 g, 2 eq) and potassium carbonate (5.5284 g, 2 eq) were taken and added into a 500 mL one-necked flask, 100 mL of DMF was added into the flask, and then the above reaction system was heated after nitrogen replacement and then stirred for 12 h in oil bath at 100°C. After the system was cooled to room temperature, 100 mL of dichloromethane was added, and the system was then washed with a large amount of deionized water four times, washed with a saturated saline solution once, and dried over anhydrous sodium sulfate. A solvent was removed in vacuum, and a residue was purified through the flash column (PE of 0-10% EA).
¹H NMR (500 MHz, CDCl₃, ppm): δ 4.06 (t, 2H), 3.16 (t, 2H), 2.35 (t, 2H), 1.80 (m, 2H), 1.60 (m, 2H), 1.29 (m, 10H), 0.88 (t, 3H). LCMS (APCI): 225.9 m/z (M+H⁺). Chemical formula: C₁₃H₂₃NO₂

### Example 5

The structure of synthesized lipid isonitrile is as follows:

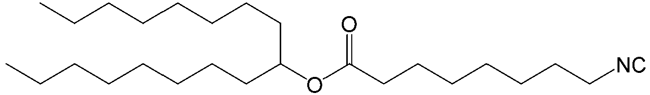

Boc-8-aminooctanoic acid (1.9179 g, 1.45 eq), 9-heptadecanol (1.3080 g, 1 eq), dicyclohexylcarbodiimide (1.0523 g, 1 eq) and 4-(dimethylamino) pyridine (0.0623 g, 0.1 eq) were successively dissolved into a 250 mL one-necked flask filled with 50 mL of DCM, and sufficiently stirred for 18 h at room temperature. After the reaction is ended, a solvent was removed in vacuum, and a residue (PE of 0-10% EA) was purified through a flash column. After loading onto the column, the product was dissolved with 10 mL of DCM, 10 mL of TFA was then slowly dropwise added, and subsequently the above mixed solution was sufficiently stirred for 3 h. After the reaction was ended, 20 mL of saturated sodium bicarbonate solution was added, and then the above mixed solution was extracted with DCM three times, dried over anhydrous Na₂SO₄, filtered, and subjected to rotary evaporation to remove the solvent so as to obtain a crude product 9-heptyl-8-aminooctanoate. 9-heptyl-8-aminooctanoate (3.1020 g, 1 eq), sodium chlorodifluoroacetate (1.4740 g, 2 eq) and potassium carbonate (2.1560 g, 2 eq) were taken and added into a 500 mL one-necked flask, 100 mL of DMF was added into the flask, and the above reaction system was heated after nitrogen replacement and then stirred for 12 h in oil bath at 100°C. After the system was cooled to room temperature, 100 mL of dichloromethane was added, and the system was then washed with a large amount of deionized water four times, washed with a saturated saline solution once, and dried over anhydrous sodium sulfate. A solvent was removed in vacuum, and a residue was purified through the flash column (PE of 0-10% EA).
¹H NMR (500 MHz, CDCl₃, ppm): δ 4.47 (m, 1H), 3.38 (t, 2H), 2.29 (t, 2H), 1.65 (m, 4H), 1.50-1.26 (m, 34H), 0.88 (t, 6H). LCMS (APCI): 408.3 m/z (M+H⁺). Chemical formula: C₂₆H₄₉NO₂

### Example 6

The structure of synthesized lipid isonitrile is as follows:

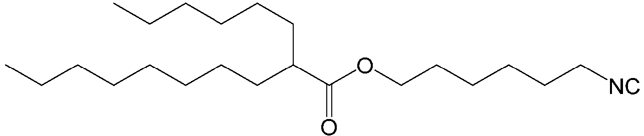

2-hexyldecanoic acid (1.7325 g, 1 eq), N-Boc-6-amino-1-hexanol (1.5386 g, 0.8 eq), dicyclohexylcarbodiimide (1.6506 g, 1 eq) and 4-(dimethylamino) pyridine (0.0977 g, 0.1 eq) were successively dissolved into a 250 mL one-necked flask filled with 50 mL of DCM, and sufficiently stirred for 18 h at room temperature. After the reaction is ended, a solvent was removed in vacuum, and a residue (PE of 0-10% EA) was purified through a flash column. After loading onto the column, the product was dissolved with 10 mL of DCM, 10 mL of TFA was then slowly dropwise added, and subsequently the above mixed solution was sufficiently stirred for 3 h. After the reaction was ended, 20 mL of saturated sodium bicarbonate solution was added, and then the above mixed solution was extracted with DCM three times, dried over anhydrous sodium sulfate, filtered, and subjected to rotary evaporation to remove the solvent so as to obtain a crude product 2-hexyl-6-aminodecanoate. 2-hexyl-6-aminodecanoate (3.0350 g, 1 eq), sodium chlorodifluoroacetate (1.7953 g, 2 eq) and potassium carbonate (2.6263 g, 2 eq) were taken and added into a 500 mL one-necked flask, 100 mL of DMF was added into the flask, and the above reaction system was heated after nitrogen replacement and then stirred for 12 h in oil bath at 100°C. After the system was cooled to room temperature, 100 mL of dichloromethane was added, and then the system was then washed with a large amount of deionized water four times, washed with a saturated saline solution once, and dried over anhydrous sodium sulfate. A solvent was removed in vacuum, and a residue was purified through the flash column (PE of 0-10% EA).
¹H NMR (500 MHz, CDCl₃, ppm): δ 4.08 (t, 2H), 3.40 (t, 2H), 2.30 (m, 1H), 1.80 (m, 2H), 1.66-1.25 (m, 32H), 0.88 (t, 6H). LCMS (APCI): 366.2 m/z (M+H⁺). Chemical formula: C₂₃H₄₃NO₂

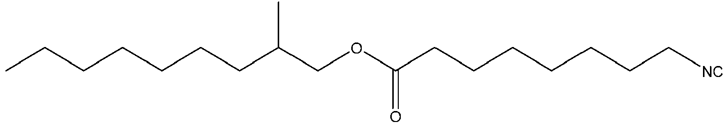

N-Boc-8-aminocaprylic acid (1.9179 g, 1.45 eq), 2-hept-2-methylethanol (0.8072 g, 1 eq), dicyclohexylcarbodiimide (1.0523 g, 1 eq) and 4-(dimethylamino) pyridine (0.0623 g, 0.1 eq) were successively dissolved into a 250 mL one-necked flask filled with 50 mL of DCM, and sufficiently stirred for 18 h at room temperature. After the reaction is ended, a solvent was removed in vacuum, and a residue (PE of 0-10% EA) was purified through a flash column. After loading onto the column, the product was dissolved with 10 mL of DCM, 10 mL of TFA was then slowly dropwise added, and subsequently the above mixed solution was sufficiently stirred for 3 h. After the reaction was ended, 20 mL of saturated sodium bicarbonate solution was added, and then the above mixed solution was extracted with DCM three times, dried over anhydrous Na₂SO₄, filtered, and subjected to rotary evaporation to remove the solvent so as to obtain a crude product 8-aminocaprylic acid heptadecane-9-ester. 8-aminocaprylic acid heptadecane-9-ester (3.1020 g, 1 eq), sodium chlorodifluoroacetate (1.4740 g, 2 eq) and potassium carbonate (2.1560 g, 2 eq) were taken and added into a 500 mL one-necked flask, 100 mL of DMF was added into the flask, and then the above reaction system was heated after nitrogen replacement and then stirred for 12 h in oil bath at 100°C. After the system was cooled to room temperature, 100 mL of dichloromethane was added, and the system was then washed with a large amount of deionized water four times, washed with a saturated saline solution once, and dried over anhydrous sodium sulfate. A solvent was removed in vacuum, and a residue was purified through the flash column (PE of 0-10% EA).

¹H NMR (500 MHz, CDCl₃, ppm): δ 4.47 (m, 2H), 3.38 (t, 2H), 2.29 (t, 2H), 2.09 (m, 1H), 1.65 (m, 4H), 1.50-1.26 (m, 18H), 0.88 (t, 3H), 0.80 (t, 3H). LCMS (APCI): 310.1 m/z (M+H⁺). Chemical formula: C₁₉H₃₅NO₂

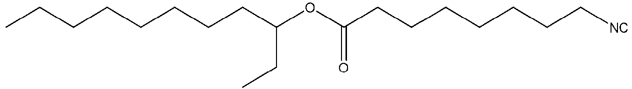

N-Boc-8-aminocaprylic acid (1.9179 g, 1.45 eq), 3-undecanol (0.8788 g, 1 eq), dicyclohexylcarbodiimide (1.0523 g, 1 eq) and 4-(dimethylamino) pyridine (0.0623 g, 0.1eq) were successively dissolved into a 250 mL one-necked flask filled with 50 mL of DCM, and sufficiently stirred for 18 h at room temperature. After the reaction is ended, a solvent was removed in vacuum, and a residue (PE of 0-10% EA) was purified through a flash column. After loading onto the column, the product was dissolved with 10 mL of DCM, 10 mL of TFA was then slowly dropwise added, and subsequently the above mixes solution was sufficiently stirred for 3 h. After the reaction was ended, 20 mL of saturated sodium bicarbonate solution was added, and then the above mixed solution was extracted with DCM three times, dried over anhydrous Na₂SO₄, filtered, and subjected to rotary evaporation to remove the solvent so as to obtain a crude product 8-aminocaprylic acid heptadecane-9-ester. 8-aminocaprylic acid heptadecane-9-ester (3.1020 g, 1 eq), sodium chlorodifluoroacetate (1.4740 g, 2 eq) and potassium carbonate (2.1560 g, 2 eq) were taken and added into a 500 mL one-necked flask, 100 mL of DMF was added into the flask, and the above reaction system was heated after nitrogen replacement and then stirred for 12 h in oil bath at 100°C. After the system was cooled to room temperature, 100 mL of dichloromethane was added, and the system was then washed with a large amount of deionized water four times, washed with a saturated saline solution once, and dried over anhydrous sodium sulfate. A solvent was removed in vacuum, and a residue was purified through the flash column (PE of 0-10% EA).
¹H NMR (500 MHz, CDCl₃, ppm): δ 4.47 (m, 1H), 3.38 (t, 2H), 2.29 (t, 2H), 1.65 (m, 4H), 1.50-1.26 (m, 22H), 0.88 (m, 6H). LCMS (APCI): 324.1 m/z (M+H⁺). Chemical formula: C₂₀H₃₇NO₂

In examples, an isonitrile compound was synthesized according to similar synthesis reaction steps, which is summarized as follows: 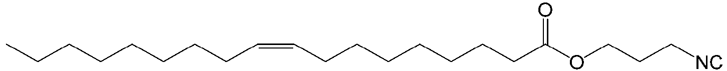
¹H NMR (500 MHz, CDCl₃, ppm): δ 5.34 (m, 2H), 4.21 (t, 2H), 3.51 (t, 2H), 2.31 (t, 2H), 2.01 (m, 6H), 1.61 (m, 2H), 1.26 (m, 20H), 0.88 (t, 3H). LCMS (APCI): 350.2 m/z (M+H⁺). Chemical formula: C₂₂H₃₉NO₂ 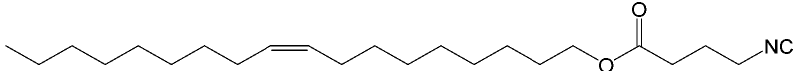
¹H NMR (500 MHz, CDCl₃, ppm): δ 5.34 (m, 2H), 4.06 (t, 2H), 3.16 (t, 2H), 2.35 (t, 2H), 2.01 (m, 4H), 1.80 (m, 2H), 1.60 (m, 2H), 1.29 (m, 22H), 0.88 (t, 3H). LCMS (APCI): 364.2 m/z (M+H⁺). Chemical formula: C₂₃H₄₁NO₂ 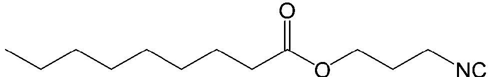
¹H NMR (500 MHz, CDCl₃, ppm): δ 4.21 (t, 2H), 3.51 (t, 2H), 2.31 (t, 2H), 2.03 (m, 2H), 1.61 (m, 2H), 1.29 (m, 10H), 0.88 (t, 3H). LCMS (APCI): 225.9 m/z (M+H⁺). Chemical formula: C₁₃H₂₃NO₂ 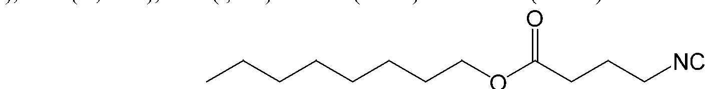
¹H NMR (500 MHz, CDCl₃, ppm): δ 4.06 (t, 2H), 3.16 (t, 2H), 2.35 (t, 2H), 1.80 (m, 2H), 1.60 (m, 2H), 1.29 (m, 10H), 0.88 (t, 3H). LCMS (APCI): 225.9 m/z (M+H⁺). Chemical formula: C₁₃H₂₃NO₂ 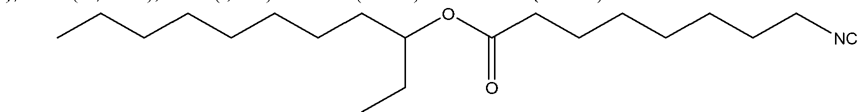
¹H NMR (500 MHz, CDCl₃, ppm): δ 4.47 (m, 1H), 3.38 (t, 2H), 2.29 (t, 2H), 1.65 (m, 4H), 1.50-1.26 (m, 22H), 0.88 (m, 6H). LCMS (APCI): 324.1 m/z (M+H⁺). Chemical formula: C₂₀H₃₇NO₂ 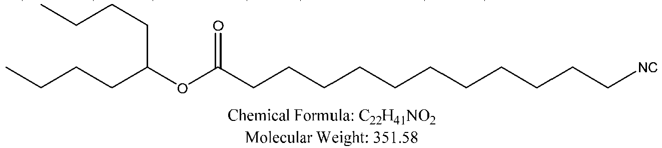
¹H NMR (500 MHz, CDCl₃, ppm): δ 4.87 (m, 1H), 3.37 (t, 2H), 2.28 (t, 2H), 1.65 (m, 4H), 1.50-1.26 (m, 26H), 0.88 (m, 6H). LCMS (APCI): 352.1 m/z (M+H⁺). Chemical formula: C₂₂H₄₁NO₂ 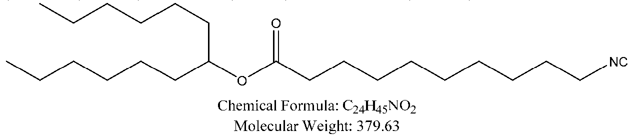
¹H NMR (500 MHz, CDCl₃, ppm): δ 4.87 (m, 1H), 3.37 (t, 2H), 2.28 (t, 2H), 1.65 (m, 4H), 1.50-1.26 (m, 30H), 0.88 (m, 6H). LCMS (APCI): 380.1 m/z (M+H⁺). Chemical formula: C₂₄H₄₅NO₂ 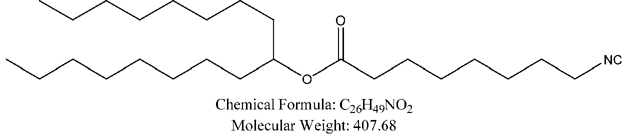
¹H NMR (500 MHz, CDCl₃, ppm): δ 4.47 (m, 1H), 3.38 (t, 2H), 2.29 (t, 2H), 1.65 (m, 4H), 1.50-1.26 (m, 34H), 0.88 (t, 6H). LCMS (APCI): 408.3 m/z (M+H⁺). Chemical formula: C₂₆H₄₉NO₂ 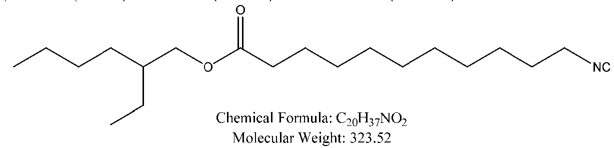
¹H NMR (500 MHz, CDCl₃, ppm): δ 3.97 (m, 2H), 3.37 (t, 2H), 2.30 (m, 2H), 1.65 (m, 5H), 1.50-1.26 (m, 20H), 0.88 (m, 6H). LCMS (APCI): 341.5 m/z (M-H⁺). Chemical formula: C₂₀H₃₇NO₂ 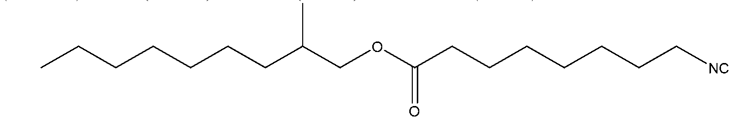
¹H NMR (500 MHz, CDCl₃, ppm): δ 4.47 (m, 2H), 3.38 (t, 2H), 2.29 (t, 2H), 2.09 (m, 1H), 1.65 (m, 4H), 1.50-1.26 (m, 18H), 0.88 (t, 3H), 0.80 (t, 3H). LCMS (APCI): 310.1 m/z (M+H⁺). Chemical formula: C₁₉H₃₅NO₂ 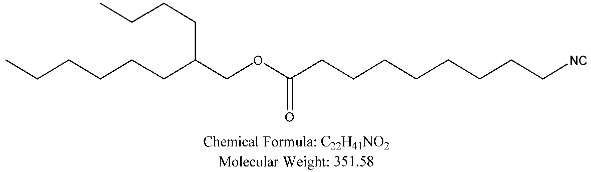
¹H NMR (500 MHz, CDCl₃, ppm): δ 3.99 (d, 2H), 3.38 (t, 2H), 2.31 (m, 2H), 1.65 (m, 5H), 1.50-1.26 (m, 24H), 0.88 (t, 6H). LCMS (APCI): 352.1 m/z (M+H⁺). Chemical formula: C₂₂H₄₁NO₂ 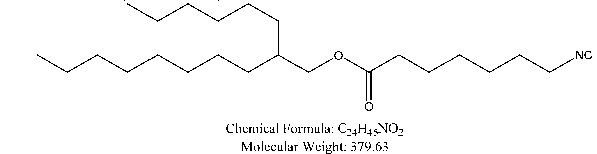
¹H NMR (500 MHz, CDCl₃, ppm): δ 3.97 (d, 2H), 3.38 (t, 2H), 2.33 (m, 2H), 1.65 (m, 5H), 1.50-1.26 (m, 28H), 0.88 (t, 6H). LCMS (APCI): 380.1 m/z (M+H⁺). Chemical formula: C₂₄H₄₅NO₄ 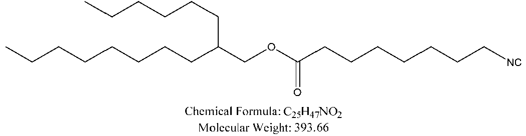
¹H NMR (500 MHz, CDCl₃, ppm): δ 3.97 (d, 2H), 3.38 (t, 2H), 2.33 (m, 2H), 1.65 (m, 5H), 1.50-1.26 (m, 30H), 0.88 (t, 6H). LCMS (APCI): 380.1 m/z (M+H⁺). Chemical formula: C₂₄H₄₅NO₄ 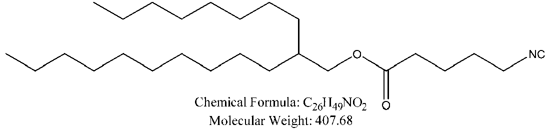
¹H NMR (500 MHz, CDCl₃, ppm): δ 3.96 (d, 2H), 3.38 (t, 2H), 2.33 (m, 2H), 1.65 (m, 5H), 1.50-1.26 (m, 32H), 0.88 (t, 6H). LCMS (APCI): 408.3 m/z (M+H⁺). Chemical formula: C₂₆H₄₉NO₂ 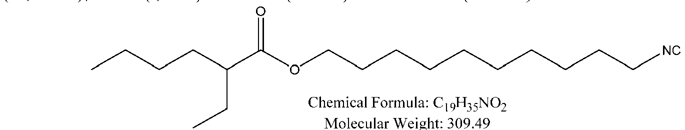
¹H NMR (500 MHz, CDCl₃, ppm): δ 4.08 (t, 2H), 3.38 (t, 2H), 2.30 (m, 1H), 1.66-1.25 (m, 24H), 0.88 (t, 6H). LCMS (APCI): 310.1 m/z (M+H⁺). Chemical formula: C₁₉H₃₅NO₂ 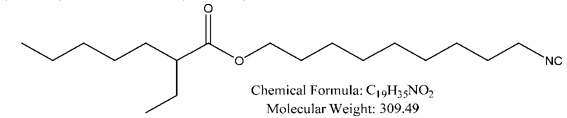
¹H NMR (500 MHz, CDCl₃, ppm): δ 4.08 (t, 2H), 3.38 (t, 2H), 2.30 (m, 1H), 1.66-1.25 (m, 24H), 0.88 (t, 6H). LCMS (APCI): 310.1 m/z (M+H⁺). Chemical formula: C₁₉H₃₅NO₂ 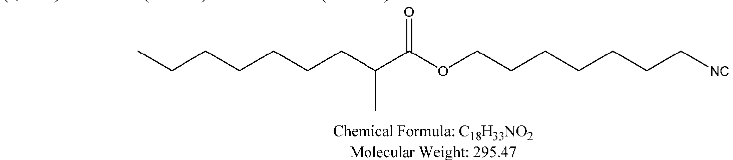
¹H NMR (500 MHz, CDCl₃, ppm): δ 4.08 (m, 2H), 3.38 (t, 2H), 2.29 (m, 3H), 1.66-1.10 (m, 23H), 0.88 (t, 3H). LCMS (APCI): 296.1 m/z (M+H⁺). Chemical formula: C₁₈H₃₃NO₂ 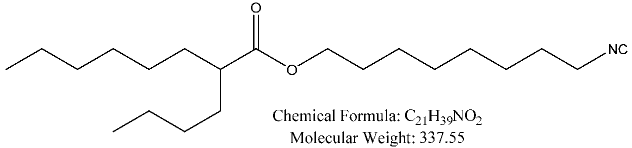
¹H NMR (500 MHz, CDCl₃, ppm): δ 4.08 (t, 2H), 3.39 (t, 2H), 2.31 (m, 1H), 1.66-1.25 (m, 28H), 0.88 (t, 6H). LCMS (APCI): 338.1 m/z (M+H⁺). Chemical formula: CH₂₁H₃₉O₂ 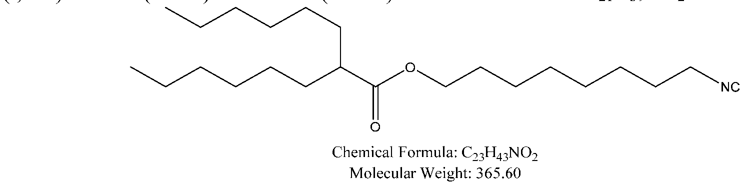
¹H NMR (500 MHz, CDCl₃, ppm): δ 4.08 (t, 2H), 3.39 (t, 2H), 2.31 (m, 1H), 1.66-1.25 (m, 32H), 0.88 (t, 6H). LCMS (APCI): 366.2 m/z (M+H⁺). Chemical formula: C₂₃H₄₃NO₂ 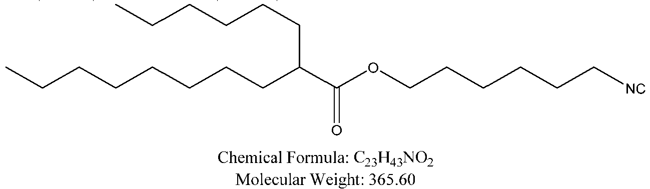
¹H NMR (500 MHz, CDCl₃, ppm): δ 4.08 (t, 2H), 3.40 (t, 2H), 2.30 (m, 1H), 1.80 (m, 2H), 1.66-1.25 (m, 32H), 0.88 (t, 6H). LCMS (APCI): 366.2 m/z (M+H⁺). Chemical formula: C₂₃H₄₃NO₂ 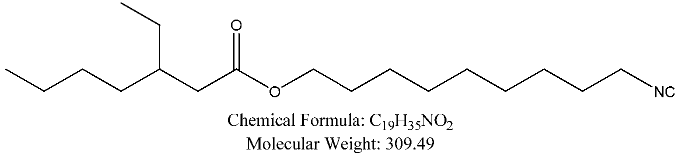
¹H NMR (500 MHz, CDCl₃, ppm): δ 4.21 (t, 2H), 3.51 (t, 2H), 2.25 (d, 2H), 1.83 (m, 1H), 1.66-1.14 (m, 22H). 0.88 (t, 6H). LCMS (APCI): 310.1 m/z (M+H⁺). Chemical formula: C₁₉H₃₅NO₂ 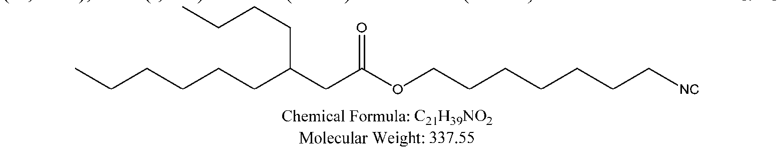
¹H NMR (500 MHz, CDCl₃, ppm): δ 4.21 (t, 2H), 3.51 (t, 2H), 2.25 (d, 2H), 1.83 (m, 1H), 1.66-1.14 (m, 26H), 0.88 (t, 6H). LCMS (APCI): 338.1 m/z (M+H⁺). Chemical formula: C₂₁H₃₉NO₂ 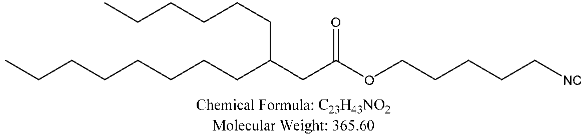
¹H NMR (500 MHz, CDCl₃, ppm): δ 4.21 (t, 2H), 3.51 (t, 2H), 2.25 (d, 2H), 1.83 (m, 1H), 1.66-1.14 (m, 30H), 0.88 (t, 6H). LCMS (APCI): 366.1 m/z (M+H⁺). Chemical formula: C₂₃H₄₃NO₂ 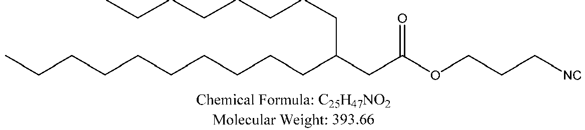
¹H NMR (500 MHz, CDCl₃, ppm): δ 4.21 (t, 2H), 3.51 (t, 2H), 2.25 (d, 2H), 2.02 (m, 2H), 1.83 (m, 1H), 1.42-1.14 (m, 32H), 0.88 (t, 6H). LCMS (APCI): 394.2 m/z (M+H⁺). Chemical formula: C₂₅H₄₇NO₂ 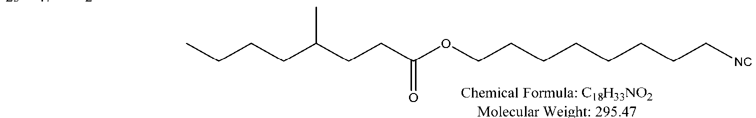
¹H NMR (500 MHz, CDCl₃, ppm): δ 4.06 (t, 2H), 3.38 (t, 2H), 2.31 (m, 2H), 1.67 (m, 6H), 1.44-1.20 (m, 15H), 0.88 (t, 6H). LCMS (APCI): 296.1 m/z (M+H⁺). Chemical formula: C₁₈H₃₃NO₂ 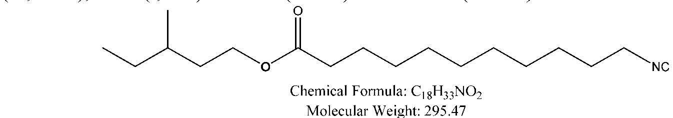
¹H NMR (500 MHz, CDCl₃, ppm): δ 4.10 (t, 2H), 3.38 (t, 2H), 2.31 (m, 2H), 1.67 (m, 5H), 1.44-1.20 (m, 16H), 0.88 (t, 6H). LCMS (APCI): 296.1 m/z (M+H⁺). Chemical formula: C₁₈H₃₃NO₂

In examples, a carboxylic acid compound was synthesized according to similar synthesis reaction steps, which is summarized as follows: 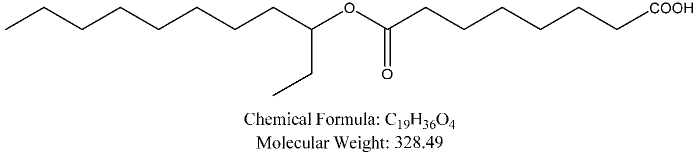
¹H NMR (500 MHz, CDCl₃, ppm): δ 4.47 (m, 1H), 2.29 (m, 4H), 1.65 (m, 4H), 1.50-1.26 (m, 20H), 0.88 (m, 6H). LCMS (APCI): 327.5 m/z (M-H⁺). Chemical formula: C₁₉H₃₆O₄ 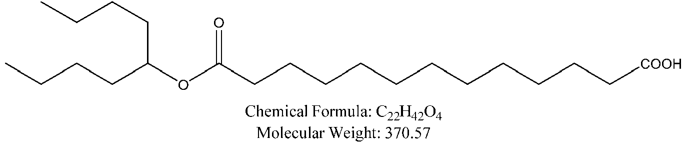
¹H NMR (500 MHz, CDCl₃, ppm): δ 4.87 (m, 1H), 2.28 (m, 4H), 1.65 (m, 4H), 1.50-1.26 (m, 26H), 0.88 (m, 6H). LCMS (APCI): 369.6 m/z (M-H⁺). Chemical formula: C₂₂H₄₂O₄ 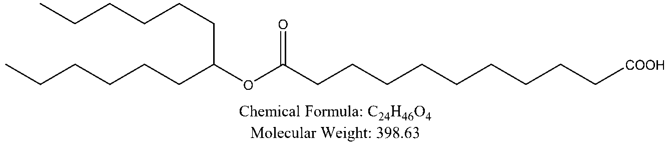
¹H NMR (500 MHz, CDCl₃, ppm): δ 4.87 (m, 1H), 2.28 (m, 4H), 1.65 (m, 4H), 1.50-1.26 (m, 30H), 0.88 (m, 6H). LCMS (APCI): 397.6 m/z (M-H⁺). Chemical formula: C₂₄H₄₆O₄ 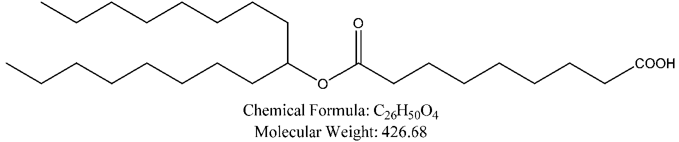
¹H NMR (500 MHz, CDCl₃, ppm): δ 4.47 (m, 1H), 2.29 (m, 4H), 1.65 (m, 4H), 1.50-1.26 (m, 34H), 0.88 (t, 6H). LCMS (APCI): 425.7 m/z (M-H⁺). Chemical formula: C₂₆H₅₀O₄ 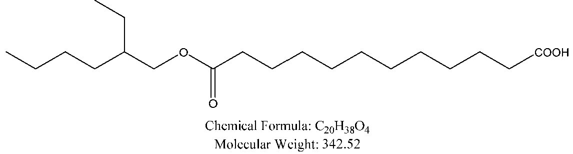
¹H NMR (500 MHz, CDCl₃, ppm): δ 3.97 (m, 2H), 2.30 (m, 4H), 1.65 (m, 5H), 1.50-1.26 (m, 20H), 0.88 (m, 6H). LCMS (APCI): 341.5 m/z (M-H⁺). Chemical formula: C₂₀H₃₈O₄ 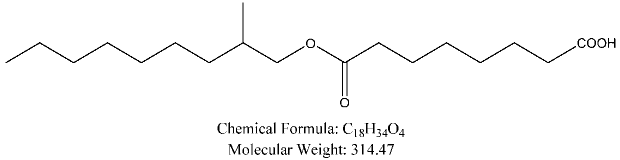
¹H NMR (500 MHz, CDCl₃, ppm): δ 4.47 (m, 2H), 2.29 (m, 4H), 2.09 (m, 1H), 1.65 (m, 4H), 1.50-1.26 (m, 16H), 0.88 (t, 3H), 0.80 (t, 3H). LCMS (APCI): 313.5 m/z (M-H⁺). Chemical formula: C₁₈H₃₄O₄ 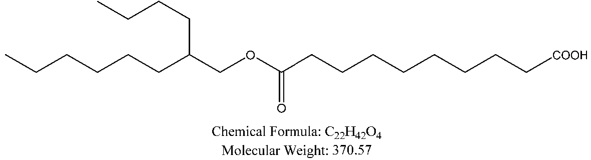
¹H NMR (500 MHz, CDCl₃, ppm): δ 3.99 (d, 2H), 2.31 (m, 4H), 1.65 (m, 5H), 1.50-1.26 (m, 24H), 0.88 (t, 6H). LCMS (APCI): 369.6 m/z (M-H⁺). Chemical formula: C₂₂H₄₂O₄ 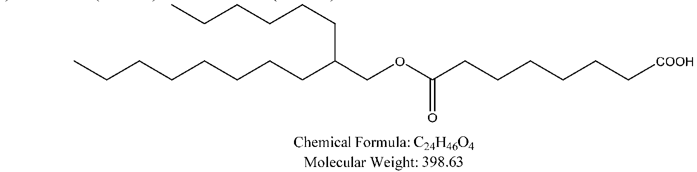
¹H NMR (500 MHz, CDCl₃, ppm): δ 3.97 (d, 2H), 2.33 (m, 4H), 1.65 (m, 5H), 1.50-1.26 (m, 28H), 0.88 (t, 6H). LCMS (APCI): 397.6 m/z (M-H⁺). Chemical formula: C₂₄H₄₆O₄ 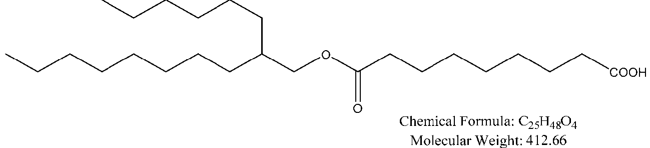
¹H NMR (500 MHz, CDCl₃, ppm): δ 3.97 (d, 2H), 2.33 (m, 4H), 1.65 (m, 5H), 1.50-1.26 (m, 30H), 0.88 (t, 6H). LCMS (APCI): 411.7 m/z (M-H⁺). Chemical formula: C₂₅H₄₈O₄ 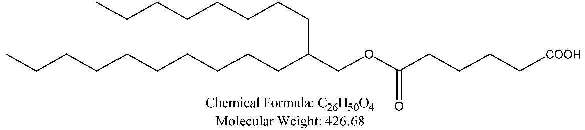
¹H NMR (500 MHz, CDCl₃, ppm): δ 3.96 (d, 2H), 2.33 (m, 4H), 1.65 (m, 5H), 1.50-1.26 (m, 32H), 0.88 (t, 6H). LCMS (APCI): 425.7 m/z (M-H⁺). Chemical formula: C₂₆H₅₀O₄ 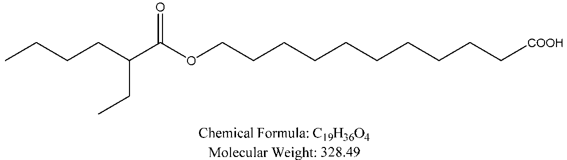
¹H NMR (500 MHz, CDCl₃, ppm): δ 4.08 (t, 2H), 2.30 (m, 3H), 1.66-1.25 (m, 24H), 0.88 (t, 6H). LCMS (APCI): 327.5 m/z (M-H⁺). Chemical formula: C₁₉H₃₆O₄ 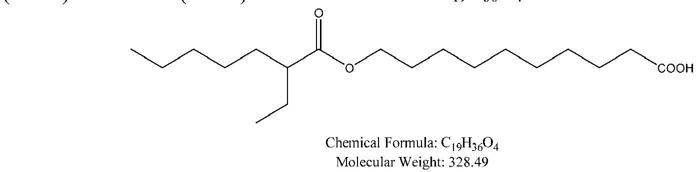
¹H NMR (500 MHz, CDCl₃, ppm): δ 4.08 (t, 2H), 2.30 (m, 3H), 1.66-1.25 (m, 24H), 0.88 (t, 6H). LCMS (APCI): 327.5 m/z (M-H⁺). Chemical formula: C₁₉H₃₆O₄ 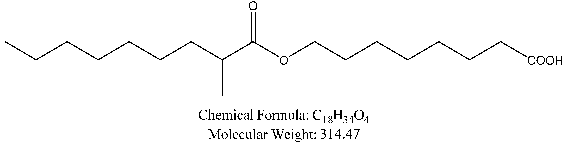
¹H NMR (500 MHz, CDCl₃, ppm): δ 4.08 (m, 2H), 2.29 (m, 3H), 1.66-1.10 (m, 25H), 0.88 (t, 3H). LCMS (APCI): 313.5 m/z (M-H⁺). Chemical formula: C₁₈H₃₄O₄ 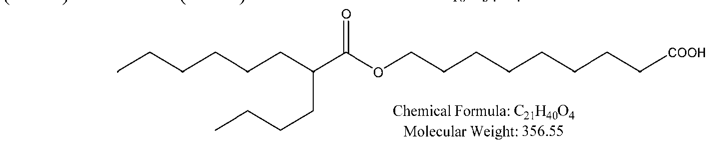
¹H NMR (500 MHz, CDCl₃, ppm): δ 4.08 (t, 2H), 2.30 (m, 3H), 1.66-1.25 (m, 28H), 0.88 (t, 6H). LCMS (APCI): 355.5 m/z (M-H⁺). Chemical formula: C₂₁H₄₀O₄ 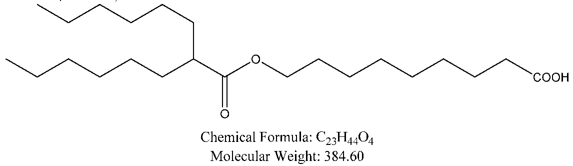
¹H NMR (500 MHz, CDCl₃, ppm): δ 4.08 (t, 2H), 2.30 (m, 3H), 1.66-1.25 (m, 32H), 0.88 (t, 6H). LCMS (APCI): 383.6 m/z (M-H⁺). Chemical formula: C₂₃H₄₄O₄ 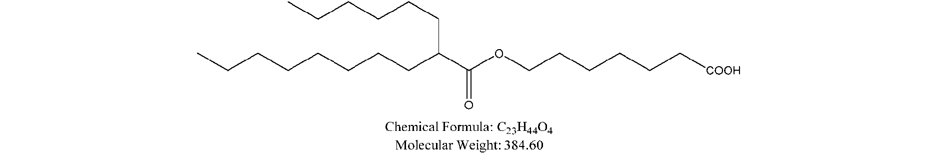
¹H NMR (500 MHz, CDCl₃, ppm): δ 4.08 (t, 2H), 2.30 (m, 3H), 1.66-1.25 (m, 32H), 0.88 (t, 6H). LCMS (APCI): 383.6 m/z (M-H⁺). Chemical formula: C₂₃H₄₄O₄ 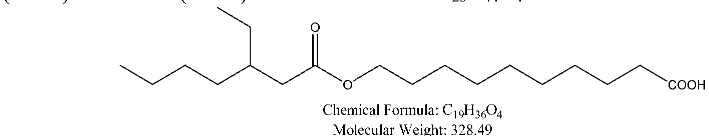
¹H NMR (500 MHz, CDCl₃, ppm): δ 4.12 (t, 2H), 2.46 (t, 2H), 2.22 (d, 2H), 1.83 (m, 1H), 1.66-1.14 (m, 22H), 0.88 (t, 6H). LCMS (APCI): 327.5 m/z (M-H⁺). Chemical formula: C₁₉H₃₆O₄ 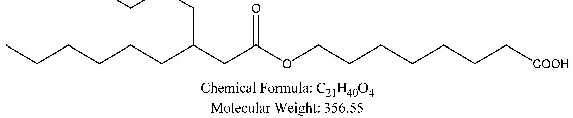
¹H NMR (500 MHz, CDCl₃, ppm): δ 4.12 (t, 2H), 2.46 (t, 2H), 2.22 (d, 2H), 1.83 (m, 1H), 1.66-1.14 (m, 26H), 0.88 (t, 6H). LCMS (APCI): 355.5 m/z (M-H⁺). Chemical formula: C₂₁H₄₀O₄ 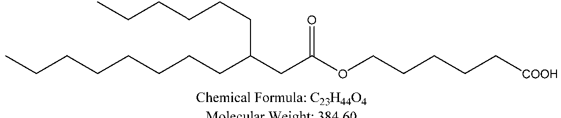
¹H NMR (500 MHz, CDCl₃, ppm): δ 4.12 (t, 2H), 2.46 (t, 2H), 2.22 (d, 2H), 1.83 (m, 1H), 1.66-1.14 (m, 30H), 0.88 (t, 6H). LCMS (APCI): 383.6 m/z (M-H⁺). Chemical formula: C₂₃H₄₄O₄ 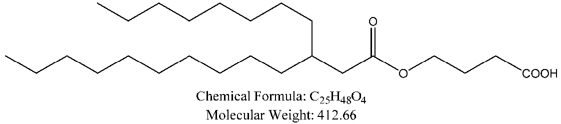
¹H NMR (500 MHz, CDCl₃, ppm): δ 4.12 (t, 2H), 2.46 (t, 2H), 2.22 (d, 2H), 1.97 (m, 2H), 1.83 (m, 1H), 1.42-1.14 (m, 32H), 0.88 (t, 6H). LCMS (APCI): 411.7 m/z (M-H⁺). Chemical formula: C₂₅H₄₈O₄ 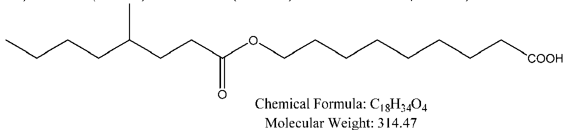
¹H NMR (500 MHz, CDCl₃, ppm): δ 4.05 (t, 2H), 2.35 (m, 4H), 1.60 (m, 6H), 1.44-1.20 (m, 15H), 0.88 (t, 6H). LCMS (APCI): 313.5 m/z (M-H⁺). Chemical formula: C₁₈H₃₄O₄ 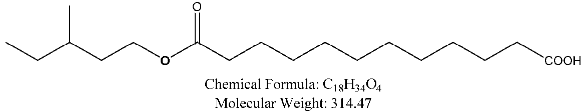
¹H NMR (500 MHz, CDCl₃, ppm): δ 4.10 (t, 2H), 2.35 (m, 4H), 1.60 (m, 5H), 1.44-1.20 (m, 16H), 0.88 (t, 6H). LCMS (APCI): 313.5 m/z (M-H⁺). Chemical formula: C₁₈H₃₄O₄

### Example 7

### Lipids 1-324 were prepared by the following steps:

(i-1) a first reactant (R_{III}-NH₂, 1 mmol, 1.0 eq) selected from Pool a and a second reactant (aldehyde R_{II}-C(O)-H, 1 mmol, 1.0 eq) selected from Pool b were dissolved into ethanol and stirred for 10 min to form a first mixture; and when the second reactant was selected from a ketone compound (R_{II}-C(O)-R_{II}') in Pool b, the amount of the second reactant increased to a three-fold equivalent (3 mmol), and meanwhile the reaction temperature was raised to 40°C in step (iii);
(ii) a third reactant (HY-C(O)-R_{IV}, 1 mmol, 1.0 eq) selected from Pool c was added into the first mixture obtained in step (i) and stirred for 10 min to form a second mixture;
(iii) 1-isocyanocyclohexene (1 mmol, 1.0 eq) was added into the second mixture obtained in step (ii) and stirred for 12 h at room temperature, followed by removing a solvent, a compound having a structure of general formula (Ia) was obtained by silica gel column chromatography (leaching gradient: from dichloromethane containing 0% methanol by volume to dichloromethane containing 10% methanol by volume, and a methanol solution containing 0.1% ammonium hydroxide);
(iv) the product (formula (Ia), 1 mmol, 1.0 eq) from the previous step was dissolved into 9.5 mL of tetrahydrofuran, 0.5 mL of concentrated hydrochloric acid was added, and the above materials were stirred for 12-18 h at room temperature. The above solution after stirring was neutralized with solid sodium bicarbonate and then filtered. A solvent was removed in vacuum, and then a residue was dissolved into a sodium bicarbonate solution of pH=10 and washed with dichloromethane. Then, a water layer was acidified and extracted with dichloromethane. Organic phases were merged and dried over anhydrous sodium sulfate. After filtration, the solvent was removed in vacuum to obtain a compound of general formula (Ib); and
(v) the product (formula (Ib), 1 mmol, 1.0 eq) from the previous step, 1-ethyl-(3-dimethylaminopropyl) carbodiimide hydrochloride (1 mmol, 1.0 eq) and 4-dimethylaminopyridine (0.1 mmol, 0.1 eq) were dissolved into 20 mL of dichloromethane, a fourth reaction (R_{I}-OH or R_{I}-NH₂, 1 mmol, 1.0 eq) selected from Pool d was then added, and the above materials were stirred overnight. The obtained solution was washed with a saturated saline solution twice, and an organic phase was dried over anhydrous sodium sulfate. The dried organic phase was filtered and a solvent was removed in vacuum, and a lipid compound having a structure of general formula (I') or (I") was obtained by silica gel column chromatography (leaching gradient: from dichloromethane containing 0% methanol by volume to dichloromethane containing 10% methanol by volume, and a methanol solution containing 0.1% ammonium hydroxide).

### A lipid 325 was prepared by the following steps:

Step 1: Amine (1 mmol, 1.0 eq) in Pool a and aldehyde (1 mmol, 1.0 eq) in Pool b were dissolved into 10 mL of methanol, and then the above solution was stirred for 10 min. Then, isocyanocyclohexene (1 mmol, 1.0 eq) was added, and subsequently the materials were stirred for 10 min again. The obtained solution was stirred for 12 h under carbon dioxide at room temperature. After a solvent was removed, a crude product containing a cyclovinylamide structure was obtained.

Step 2: The product from Step 1 was dissolved into 9.5 mL of tetrahydrofuran, 0.5 mL of concentrated hydrochloric acid was added, and then the above materials were stirred for 12-18 h at room temperature. The obtained solution was neutralized with solid sodium bicarbonate and then filtered. After a solvent was removed in vacuum, a residue was dissolved into a sodium bicarbonate solution of pH=10 and washed with dichloromethane. Then, a water layer was acidified and extracted with dichloromethane. Organic phases were merged and dried over anhydrous sodium sulfate. After filtration, a solvent was removed in vacuum to obtain a crude product.

Step 3: The crude product (1 mmol, 1.0 eq) in Step 2, 1-ethyl-(3-dimethylaminopropyl) carbodiimide hydrochloride (1 mmol, 1.0 eq) and 4-dimethylaminopyridine (0.1 mmol, 1.0 eq) were dissolved into 20 mL of dichloromethane, then a fourth component (1 mmol, 1.0 eq) from Pool d was added, and then the above materials were stirred overnight. The obtained solution was washed with a saturated saline solution twice, and an organic phase was dried over anhydrous sodium sulfate. The dried organic phase was filtered and a solvent was removed in vacuum, and a target product was obtained by silica gel column chromatography (leaching gradient: from dichloromethane containing 0% methanol by volume to dichloromethane containing 10% methanol by volume, and a methanol solution containing 0.1% ammonium hydroxide).

### Lipid 325:

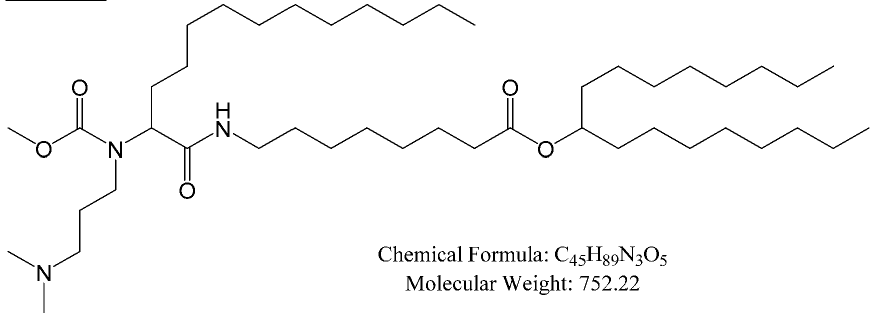

¹H NMR (500 MHz, CDCl₃, ppm): δ 4.86 (m, 1H), 4.32 (m, 1H), 3.73 (s, 3H), 3.19 (m, 4H), 2.33 (m, 10H), 1.79 (m, 2H), 1.61 (m, 2H), 1.51-1.18 (m, 58H), 0.87 (t, 9H). MS: m/z 752.9 (M+H⁺). Chemical formula: C₄₅H₈₉N₃O₅

### Lipids 326-327 were prepared by the following steps:

Step 1: Amine (1 mmol, 1.0 eq) in Pool a and aldehyde (1 mmol, 1.0 eq) in Pool b were dissolved into 10 mL of tert-butanol and stirred for 10 min. Then, isocyanocyclohexene (1 mmol, 1.0 eq) was added, and subsequently the materials were stirred for 10 min again. The obtained solution was stirred for 12 h under carbon dioxide at room temperature. After a solvent was removed, an intermediate 1 containing a cyclovinylamide structure was obtained.

Step 2: The product in Step 1 was dissolved into a solution of 30% trifluoroacetic acid in dichloromethane and stirred for 3-5 h at room temperature, and a solvent was removed in vacuum to obtain a secondary amine intermediate 2.

Step 3: The crude product (1 mmol, 1.0 eq) obtained in the previous step and triethylamine (1 mmol, 1.0 eq) were dissolved into 10 mL of dichloromethane, 10 mL of dichloromethane dissolved with 4-nitrobenzoylformylchloride formate (1 mmol, 1 eq) was dropwise added at a low temperature, and the above materials reacted for 6 h at room temperature. The above reaction solution was washed with a saturated sodium carbonate solution, and an organic phase was collected and dried. After the solvent was removed, an intermediate 3 was obtained.

Step 4: The intermediate 3 was dissolved into 9.5 mL of tetrahydrofuran, 0.5 mL of concentrated hydrochloric acid was added, and the above materials were stirred for 12-18 h at room temperature. The above reaction solution was neutralized with solid sodium bicarbonate and then filtered. After a solvent was removed in vacuum, a residue was dissolved into a sodium bicarbonate solution of pH=10 and then washed with dichloromethane. Then, a water layer was acidified and extracted with dichloromethane. Organic phases were merged and dried over anhydrous sodium sulfate. After filtration, the solvent was removed to obtain an intermediate 4.

Step 5: The intermediate 4, 1-ethyl-(3-dimethylaminopropyl) carbodiimide hydrochloride (1 mmol) and 4-dimethylaminopyridine (0.1 mmol) were dissolved into 20 mL of dichloromethane, a fourth component (1 mmol, 1.0 eq) in Pool d was then added, and the above materials were stirred overnight. The obtained solution was washed with a saturated saline solution twice, and an organic phase was dried over anhydrous sodium sulfate. The dried organic phase was filtered and a solvent was removed, and subsequently a target product was obtained by silica gel column chromatography (leaching gradient: from dichloromethane containing 0% methanol by volume to dichloromethane containing 10% methanol by volume, and a methanol solution containing 0.1% ammonium hydroxide).

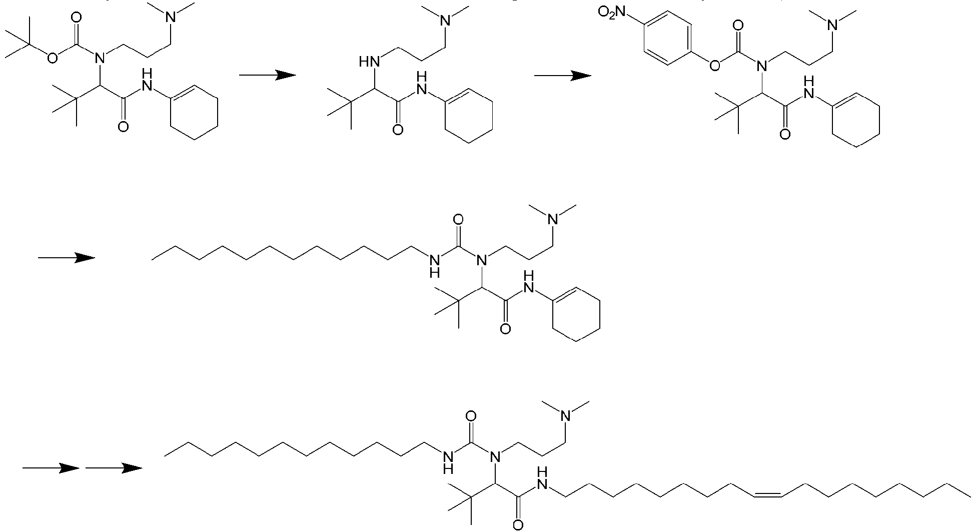

### Lipid 326:

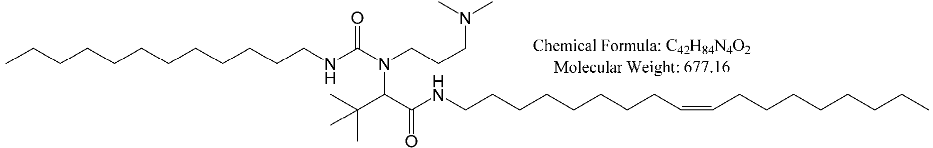

¹H NMR (500 MHz, CDCl₃, ppm): δ 5.34 (m, 2H), 4.1-3.9 (m, 1H), 3.73 (m, 1H), 3.45(m, 1H), 3.21 (m, 1H), 3.08-3.01 (m, 3H), , 2.33 (m, 8H), 2.00 (m, 4H), 1.79-1.54 (m, 6H), 1.51-1.18 (m, 49H), 0.87 (t, 6H). MS: m/z 678.8 (M+H⁺). Chemical formula: C₄₂H₈₄N₄O₂

### Lipid 327:

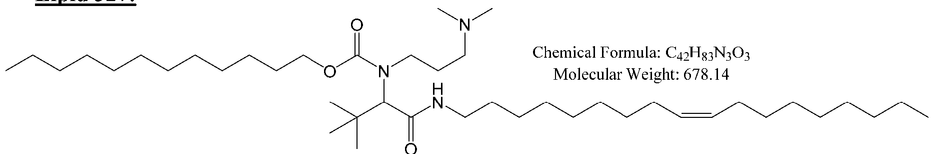

¹H NMR (500 MHz, CDCl₃, ppm): δ 5.34 (m, 2H), 4.1-3.9 (m, 3H), 3.73 (m, 1H), 3.45(m, 1H), 3.21 (m, 1H), 3.08 (m, 1H), 2.33 (m, 8H), 2.00 (m, 4H), 1.79-1.54 (m, 6H), 1.51-1.18 (m, 49H), 0.87 (t, 6H). MS: m/z 678.8 (M+H⁺). Chemical formula: C₄₂H₈₃N₃O₃

### Lipids 401-1075 were prepared by the following steps:

(i-1) a first reactant (R_{III}-NH₂, 1 mmol, 1.0 eq) selected from Pool a and a second reactant (aldehyde R_{II}-C(O)-H, 1 mmol, 1.0 eq) selected from Pool b were dissolved into ethanol and stirred for 10 min to form a first mixture; and when the second reactant was selected from a ketone compound (R_{II}-C(O)-R_{II}') in Pool b, the amount of the second reactant increased to a three-fold equivalent (3 mmol), and meanwhile the reaction temperature was raised to 40°C in step (iii);
(ii) a third reactant (HY-C(O)-R_{IV}, 1 mmol, 1.0 eq) selected from Pool c was added into the first mixture obtained in step (i) and stirred for 10 min to form a second mixture; and
(iii) a fourth reactant (R_{I}-NC, 1 mmol, 1.0 eq) selected from Pool e was added into the second mixture obtained in step (ii) and stirred for 12 h at room temperature, followed by removing a solvent, and a compound having the structure of formula (Ia) was obtained by silica gel column chromatography (leaching gradient: from dichloromethane containing 0% methanol by volume to dichloromethane containing 10% methanol by volume, and a methanol solution containing 0.1% ammonium hydroxide).

The compounds included in Pool a, Pool b, Pool c, Pool d and Pool e are specifically shown below.

### Pool a

| | | | | | |
|---|---|---|---|---|---|
| **a1** | 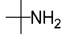 | **a17** | 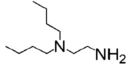 | **a28** | 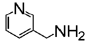 |
| **a2** | 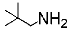 | | | | |
| | | | | **a29** | 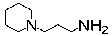 |
| **a3** | 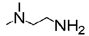 | **a18** | 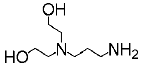 | | |
| | | | | **a30** | 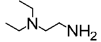 |
| **a4** | 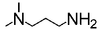 | | | | |
| | | **a19** | 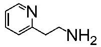 | **a31** | 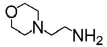 |
| **a5** | 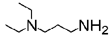 | | | | |
| | | **a20** | 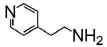 | **a32** | 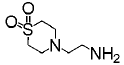 |
| **a6** | 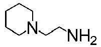 | | | | |
| | | **a21** | 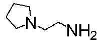 | **a33** | 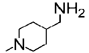 |
| **a7** | 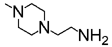 | | | | |
| **a8** | 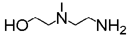 | **a22** | 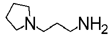 | **a34** | 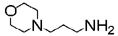 |
| **a9** | 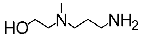 | **a23** | 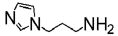 | **a35** | 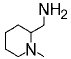 |
| **a10** | 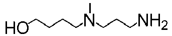 | **a24** | 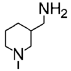 | | |
| **a11** | 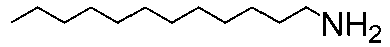 | | | **a36** | 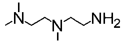 |
| **a12** | 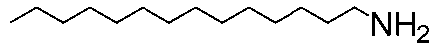 | **a25** | 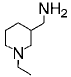 | **a37** | 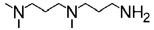 |
| **a13** | 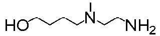 | | | | |
| **a14** | 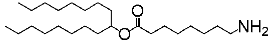 | **a26** | 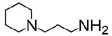 | **a38** | 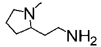 |
| **a15** | 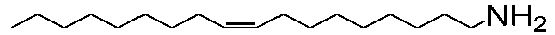 | **a27** | 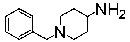 | | |

### Pool b

| | | | | | | | | | | | |
|---|---|---|---|---|---|---|---|---|---|---|---|
| **b1** | 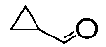 | **b2** | 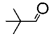 | **b16** | 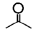 | **b26** | 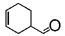 | | | **b40** | 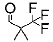 |
| **b3** | 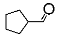 | **b4** | 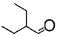 | **b17** | 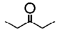 | **b27** | 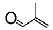 | | | **b41** | 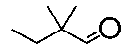 |
| | | | | **b18** | 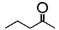 | **b28** | 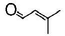 | | | **b42** | 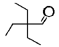 |
| **b5** | 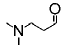 | **b6** | 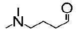 | | | **b29** | 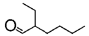 | | | | |
| | | | | **b19** | 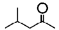 | | | | | **b43** | 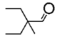 |
| **b7** | 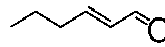 | **b8** | 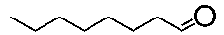 | | | **b30** | 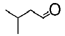 | | | | |
| | | | | **b20** | 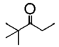 | | | | | | 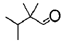 |
| **b9** | 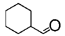 | **b10** | 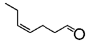 | | | **b31** | 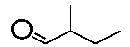 | | | | |
| | | | | **b21** | 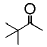 | | | | | **b44 b45** | 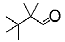 |
| **b11** | 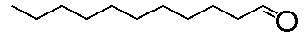 | | | **b22** | 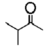 | **b32** | 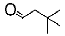 | | | | |
| **b12** | 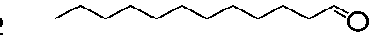 | | | | | | | | | **b46** | 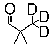 |
| | | | | **b23** | 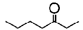 | **b33** | 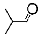 | | | | |
| **b13** | 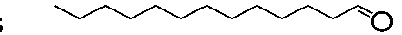 | | | | | | | | | **b47** | 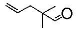 |
| | | | | **b24** | 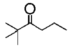 | **b34** | 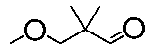 | | | | |
| **b14** | 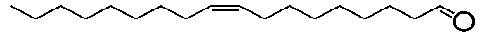 | | | | | | | | | **b48** | 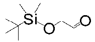 |
| **b15** | 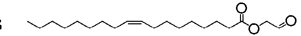 | | | **b25** | 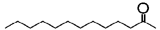 | **b35** | 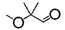 | | | **b49** | 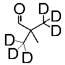 |
| | | | | | | **b36** | 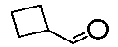 | | | | |
| | | | | | | **b37** | 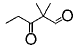 | | | **b50** | 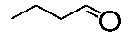 |
| | | | | | | **b38** | 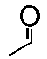 | **b39** | 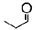 | | |

### Pool c

| | | | | | |
|---|---|---|---|---|---|
| c1 | 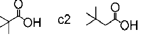 | c23 | 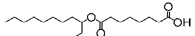 | c35 | 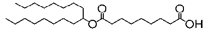 |
| c3 | 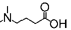 | c24 | 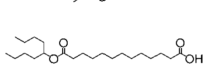 | | |
| c4 | 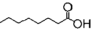 | | | c36 | 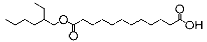 |
| c5 | 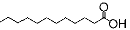 | c25 | 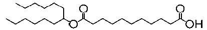 | c37 | 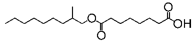 |
| c6 | 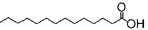 | | | | |
| c7 | 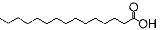 | c26 | 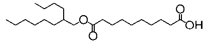 | c38 | 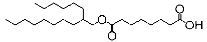 |
| c8 | 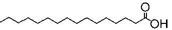 | | | | |
| c9 | 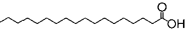 | c27 | 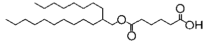 | c39 | 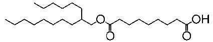 |
| c10 | 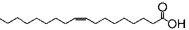 | | | | |
| c11 | 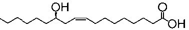 | c28 | 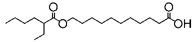 | c40 | 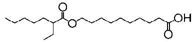 |
| | 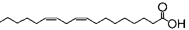 | c29 | 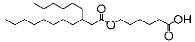 | c41 | |
| c12 | | | | | 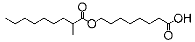 |
| c13 | 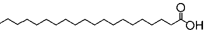 | | | | |
| c14 | 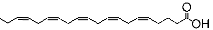 | c30 | 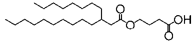 | c42 | 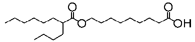 |
| c15 | 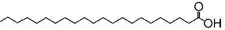 | | | | |
| c16 | 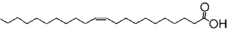 | c31 | 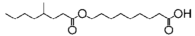 | c43 | 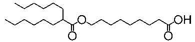 |
| c17 | 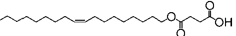 | | | | |
| c18 | 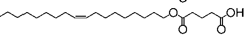 | c32 | 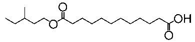 | c44 | 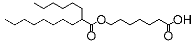 |
| c19 | 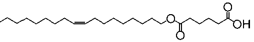 | | | | |
| c20 | 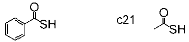 | c33 | 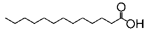 | c45 | 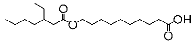 |
| c22 | 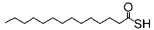 | c34 | 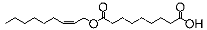 | c46 | 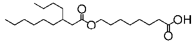 |
| c48 | 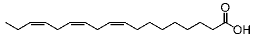 | | | c47 | 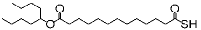 |
| c49 | 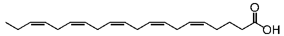 | | | | |
| c50 | 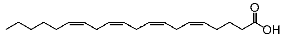 | | | | |
| c51 | 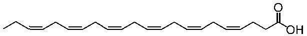 | | | | |
| c52 | 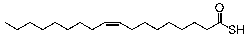 | | | | |
| c53 | 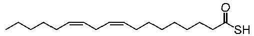 | | | | |
| c54 | 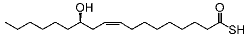 | | | | |
| c55 | 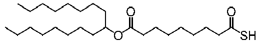 | | | | |
| c56 | 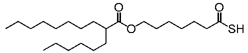 | | | | |
| c57 | 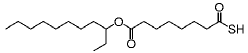 | | | | |
| c58 | 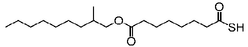 | | | | |

### Pool d

| | |
|---|---|
| **d1** | 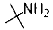 |
| **d2** | 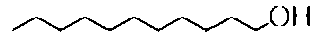 |
| **d3** | 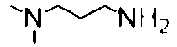 |
| **d4** | 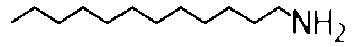 |
| **d5** | 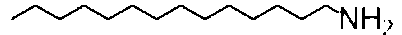 |
| **d6** | 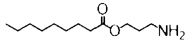 |
| **d7** | 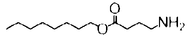 |
| **d8** | 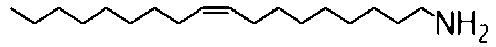 |
| **d9** | 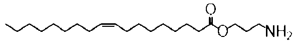 |
| **d10** | 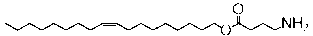 |
| **d11** | 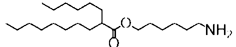 |
| **d12** | 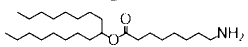 |
| **d13** | 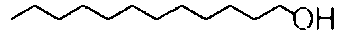 |
| **d14** | 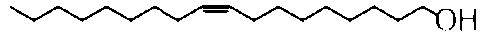 |

### Pool e

| | | | | | |
|---|---|---|---|---|---|
| **e1** | 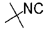 | **e12** | 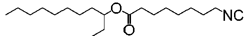 | **e22** | 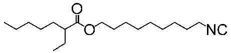 |
| **e2** | 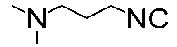 | **e13** | 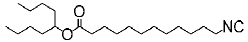 | **e23** | 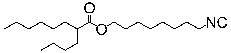 |
| | | **e14** | 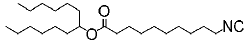 | | |
| **e3** | 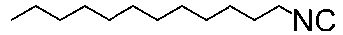 | | | **e24** | 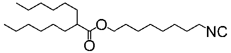 |
| **e4** | 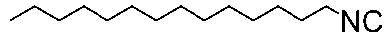 | | | | |
| | | **e15** | 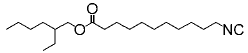 | | |
| **e5** | 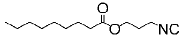 | | | **e25** | 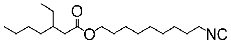 |
| **e6** | 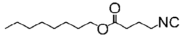 | **e16** | 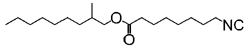 | **e26** | 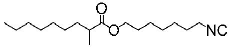 |
| **e7** | 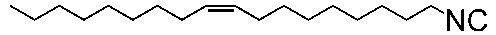 | **e17** | 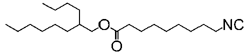 | **e27** | 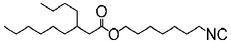 |
| **e8** | 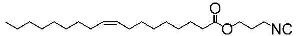 | **e18** | 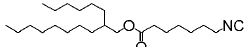 | **e28** | 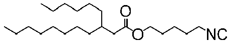 |
| **e9** | 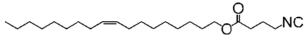 | **e19** | 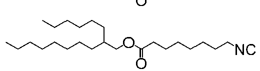 | **e29** | 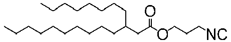 |
| **e10** | 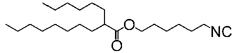 | **e20** | 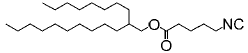 | **e30** | 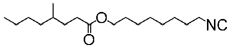 |
| **e11** | 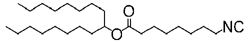 | **e21** | 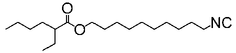 | **e31** | 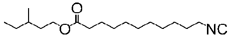 |
| | | | | **e32** | 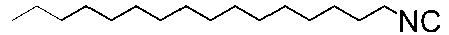 |
| | | | | **e33** | 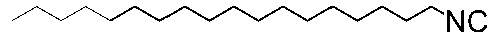 |

### Synthesis of some key reaction intermediates:

### Synthesis of 1-isocyanocyclohexene:

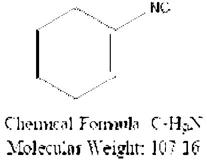

Cyclohexanone (4.9072 g, 1 eq), formamide (3.3780 g, 1.5 eq) and p-toluenesulfonic acid monohydrate (0.4755 g, 0.05 eq) were added into a 250 mL one-necked flask, 100 mL of toluene was added into the flask, and the above reaction system was subjected to condensation reflux for 24 h at 140°C. A solvent was removed by rotary evaporation, and a residue was purified through a flash column to obtain N-(1-cyclovinyl) formamide (PE of 0-60% EA).

N-(1-cyclovinyl) formamide (1.6460 g, 1 eq) and 1,4-diazabicyclo[2.2.2]octane (4.4262 g, 3 eq) were added into a 100 mL one-necked flask, 32.88 ml of dichloromethane was then added into the flask, and the above reaction system was stirred in ice water bath. Triphosgene (2.6152 g, 0.67 eq) dispersed with 28.62 ml of dichloromethane was dropwise added by a constant-pressure dropping funnel. After stirring for 2 h, the above reaction solution was washed with a saturated sodium bicarbonate solution, and a water phase was extracted with dichloromethane three times. An organic phase was dried over anhydrous sodium sulfate. A solvent was removed in vacuum, and a residue (PE of 0-10% EA) was purified by a flash column. ¹H NMR (500 MHz, CDCl₃, ppm): δ 5.92 (s, 1H), 2.22 (m, 2H), 2.10 (m, 2H), 1.69 (m, 2H), 1.57 (m, 2H). MS: m/z 108.2 (M+H⁺). Chemical formula: C₇H₉N.

### Synthesis of compound a8:

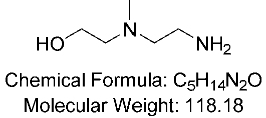

2-(methylamino) ethylamino tert-butyl formate (0.5000 g, 1 eq) and 2-bromoethanol (0.5379 g, 1.5 eq) were dissolved into 10 mL of toluene, and the above reaction system was refluxed for 5 h at 115°C at the atmosphere of nitrogen. A solvent was removed in vacuum, and a residue (DCM of 0-10% MeOH) was purified by a flash column. After loading onto the column, the product was dissolved into a solution of 50% trifluoroacetic acid in dichloromethane and reacted for 4 h. The solvent was removed in vacuum to obtain the product.

¹H NMR (500 MHz, CDCl₃, ppm): 3.86 (t, 2H), 3.38 (m, 4H), 3.22 (t, 2H), 2.87 (s, 3H). MS: m/z 119.1 (M+H⁺). Chemical formula: C₅H₁₄N₂O.

### Synthesis of compound a9:

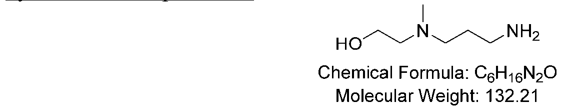

2-(methylamino) propylamino tert-butyl formate (0.5000 g, 1 eq) and 2-bromoethanol (0.4978 g, 1.5 eq) were dissolved into 10 mL of toluene, and the above reaction system was refluxed for 5 h at 115°C at the atmosphere of nitrogen. A solvent was removed in vacuum, and a residue (DCM of 0-10% MeOH) was purified by a flash column. After loading onto the column, the product was dissolved into a solution of 50% trifluoroacetic acid in dichloromethane and reacted for 4 h. The solvent was removed in vacuum to obtain the product.

¹H NMR (500 MHz, CDCl₃, ppm): 3.72 (t, 2H), 3.53 (m, 2H), 3.45 (m, 2H), 3.38 (m, 2H), 2.98 (s, 3H), 2.00 (m, 2H). MS: m/z 131.1 (M+H⁺). Chemical formula: C₆H₁₆N₂O.

### Synthesis of compound a10:

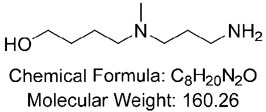

2-(methylamino) ethylamino tert-butyl formate (0.5000 g, 1 eq) and 4-bromo-1-butanol (0.6096 g, 1.5 eq) were dissolved into 10 mL of toluene, and the above reaction system was refluxed for 5 h at 115°C at the atmosphere of nitrogen. A solvent was removed in vacuum, and a residue (DCM of 0-10% MeOH) was purified by a flash column. After loading onto the column, the product was dissolved into a solution of 50% trifluoroacetic acid in dichloromethane and reacted for 4 h. The solvent was removed in vacuum to obtain the product.

¹H NMR (500 MHz, CDCl₃, ppm): 3.72 (t, 2H), 3.53 (m, 2H), 3.45 (m, 2H), 3.38 (m, 2H), 2.98 (s, 3H), 2.00 (m, 2H), 1.71 (m, 2H), 1.52 (m, 2H). MS: m/z 161.2 (M+H⁺). Chemical formula: C₈H₂₀N₂O.

### Synthesis of compound a13:

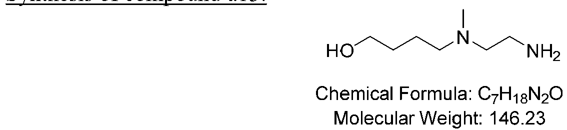

2-(methylamino) ethylamino tert-butyl formate (0.5000 g, 1 eq) and 4-bromo-1-butanol (0.6587 g, 1.5 eq) were dissolved into 10 mL of toluene, and the above reaction system was refluxed for 5 h at 115°C under the atmosphere of nitrogen. A solvent was removed in vacuum, and a residue (DCM of 0-10% MeOH) was purified by a flash column. After loading onto the column, the product was dissolved into a solution of 50% trifluoroacetic acid in dichloromethane and reacted for 4 h. The solvent was removed in vacuum to obtain the product.

¹H NMR (500 MHz, CDCl₃, ppm): 3.86 (t, 2H), 3.38 (m, 4H), 3.22 (t, 2H), 2.87 (s, 3H), 1.71 (m, 2H), 1.52 (m, 2H). MS: m/z 147.2 (M+H⁺). Chemical formula: C₇H₁₈N₂O.

### Synthesis of compound b5: 3-(dimethylamino) propanal

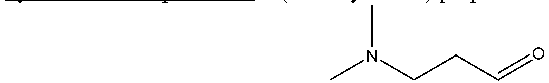

A dimethylamine aqueous solution (2 eq, 24 mmol) was added into 5 mL of water, sodium hydroxide (2 eq, 24 mmol) was then added, and then the above reaction system was stirred in ice bath. Then, 3-chloropropanal diethyl acetal (1 eq, 12 mmol) was slowly dropwise added, and the above materials were stirred for 0.5 h at 0°C and then stirred overnight at 30°C. After the reaction was ended, hydrochloric acid was used to adjust the pH of the system to be neutral, and then the system was washed with dichloromethane twice. An organic phase was dried over anhydrous magnesium sulfate, and then a solvent was removed in vacuum to obtain a crude product 3-dimethylaminopropanal diethyl acetal. 3-dimethylaminopropanal diethyl acetal (1 mmol) was dissolved into 5 mL of tetrahydrofuran, 5 mL of 2 M hydrochloric acid was then added, and the above materials reacted at 40°C overnight. The solvent was removed in vacuum to obtain 3-(dimethylamino) n-propanal hydrochloride.

¹H NMR (500 MHz, CDCl₃, ppm): δ 9.48 (s, 1H), 2.64 (s, 6H), 2.52 (m, 4H).

### Synthesis of compound b6: 4-(dimethylamino) n-butanal

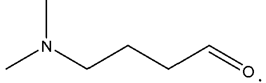

4-dimethylaminobutanal diethyl acetal (1 mmol) was dissolved into 5 mL of tetrahydrofuran, 5 mL of 2 M hydrochloric acid was then added, and the above materials reacted at 40°C overnight. A solvent was removed in vacuum to obtain 4-(dimethylamino) n-butanal hydrochloride.

¹H NMR (500 MHz, CDCl₃, ppm): δ 9.36 (s, 1H), 2.40 (m, 4H), 2.16 (s, 6H), 1.56 (m, 2H).

### Synthesis of compound b15:

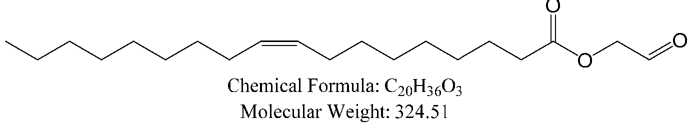

Oleic acid (9.6036 g, 34 mmol), N-(3-dimethylaminopropyl)-N'-ethyl carbodiimide hydrochloride (9.7767 g, 26.6 mmol), 1,2-glycol (10.5519 g, 170 mmol), 4-(dimethylamino) pyridine (2.0769 g, 17mmol) and N,N-diisopropylethylamine (8.7883 g, 68 mmol) were dissolved into dichloromethane (400 ml). The reaction was stirred for 18 h at room temperature under the nitrogen, and then washed with a saturated sodium bicarbonate aqueous solution. An organic layer was separated, washed with a saline solution, dried over anhydrous sodium sulfate and filtered, and then a filtrate was evaporated in vacuum. The residue was purified by silica gel chromatography (a solution of 0-50% ethyl acetate in hexane) to obtain 2-hydroxyethyl oleate. 2-hydroxyethyl oleate (4.5713 g, 14 mmol) was taken and dissolved into 200 mL of dichloromethane and then dissolved into Deiss-Martin high iodoalkane (8.9070 g, 21 mmol). The reaction was stirred for 3 h at room temperature under the nitrogen. Sodium thiosulfate pentahydrate (50 % w/v, 200 mL) was added into the reaction and stirred for 15 min again. Then, an organic layer was separated, washed with a saline solution, dried over anhydrous sodium sulfate and filtered, and then a filtrate was evaporated in vacuum. A residue was purified by a flash column (PE of 0-30% EA) to obtain 2-oxoethyl oleate.

¹H NMR (500 MHz, CDCl₃, ppm): δ 5.37 (m, 2H), 4.67 (s, 2H), 2.44 (m, 2H), 2.04 (m, 4H), 1.66 (m, 2H), 1.37-1.27 (m, 20H), 0.88 (t, 3H). MS: m/z 325.1 (M+H⁺). Chemical formula: C₂₀H₃₆O₃.

### Synthesis of compound c17:

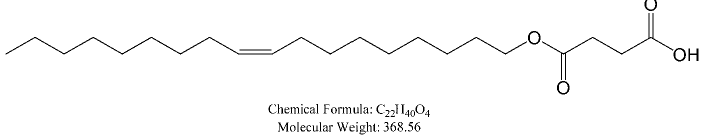

Oleyl alcohol (4.0000 g, 1 eq) and 4-(dimethylamino) pyridine (0.1820 g, 0.1 eq) were dissolved into 100 mL of super-dried dichloromethane, and then the reaction system was subjected to nitrogen replacement three times. Succinic anhydride (1.7891 g, 1.2 eq) dissolved into 50 mL of super-dried dichloromethane was dropwise added through a constant-pressure dropping funnel. The above materials reacted overnight at room temperature, a solvent was removed in vacuum, and a residue (PE of 0-20% EA) was purified through a flash column. ¹H NMR (500 MHz, CDCl₃, ppm): δ 5.37 (m, 2H), 4.09 (t, 2H), 2.69 (m, 2H), 2.62 (m, 2H), 2.02 (m, 4H), 1.62 (m, 2H), 1.37-1.27 (m, 22H), 0.88 (t, 3H). MS: m/z 368.0 (M-H⁺). Chemical formula: C₂₂H₄₀O₄.

### Synthesis of compound c18:

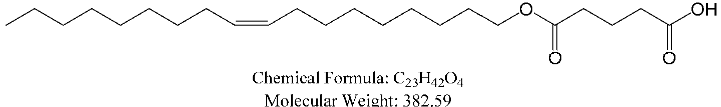

Oleyl alcohol (4.0000 g, 1 eq) and 4-(dimethylamino) pyridine (0.1820 g, 0.1 eq) were dissolved into 100 mL of super-dried dichloromethane, and then the reaction system was subjected to nitrogen replacement three times. Glutaric anhydride (2.0399 g, 1.2 eq) dissolved into 50 mL of super-dried dichloromethane was dropwise added through a constant-pressure dropping funnel. The above materials reacted overnight at room temperature, a solvent was removed in vacuum, and a residue (PE of 0-20% EA) was purified through a flash column.

¹H NMR (500 MHz, CDCl₃, ppm): δ 5.37 (m, 2H), 4.09 (t, 2H), 2.64 (m, 2H), 2.43 (m, 2H), 2.02 (m, 6H), 1.62 (m, 2H), 1.37-1.27 (m, 22H), 0.88 (t, 3H). MS: m/z 382.1 (M-H⁺). Chemical formula: C₂₃H₄₂O₄.

### Synthesis of compound c19:

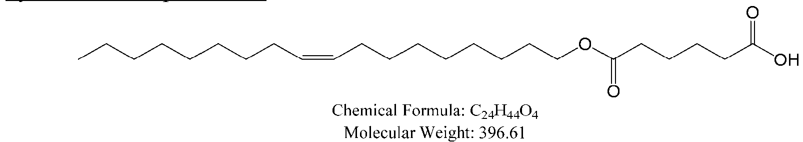

Oleyl alcohol (3.0025 g, 1 eq), N-ethyldiisopropylamine (3.4346 g, 2.5 eq), dicyclohexylcarbodiimide (2.1933 g, 1 eq) and 4-(dimethylamino) pyridine (0.1299 g, 0.1 eq) were dissolved into 100 mL of super-dried dichloromethane, and then the reaction system subjected to nitrogen replacement three times. Hexane diacid (1.2561 g, 1 eq) dissolved into 50 mL of super-dried dichloromethane was dropwise added through a constant-pressure dropping funnel. The above system reacted overnight at room temperature, washed with a saturated sodium bicarbonate solution twice, washed with a saturated saline solution and then dried over anhydrous sodium sulfate. The obtained reaction solution was filtered, a solvent was removed in vacuum, and a residue (PE of 0-20% EA) was purified through a flash column.

¹H NMR (500 MHz, CDCl₃, ppm): δ 5.37 (m, 2H), 4.09 (t, 2H), 2.44 (m, 2H), 2.33 (m, 2H), 2.02 (m, 2H), 1.62 (m, 6H), 1.37-1.27 (m, 22H), 0.88 (t, 3H). MS: m/z 396.1 (M-H⁺). Chemical formula: C₂₄H₄₄O₄.

### Synthesis of compound c22:

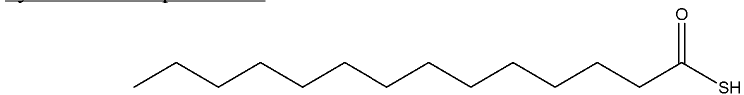

A sodium bisulfide solid (2.0 eq, 10 mmol, 0.56 g) and 6 ml of methanol were added into a 50 ml round-bottom flask. The above materials were stirred in ice bath at 0°C, and then myristoyl chloride (5 mmol) was slowly dropwise added through a constant-pressure dropping funnel. The above system reacted for 1 h at 0°C and then reacted for 2 h at room temperature. The reactants were poured into water, acidified with a 1 N HCl solution (10 ml) and then extracted with 4*15 ml of ethyl acetate. Organic phases were merged, washed with a saturated saline solution twice, and dried over anhydrous magnesium sulfate. The anhydrous magnesium sulfate was removed by suction filtration, and a solvent was removed by rotary evaporation to obtain a yellow oily substance.

¹H NMR (500 MHz, CDCl₃, ppm): δ 5.0 (s, 1H), 2.30 (t, 2H), 1.62 (m, 2H), 1.36-1.18 (m, 20H), 0.88 (t, 3H).

### Synthesis of compound d5:

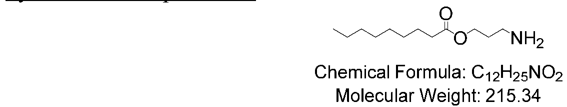

Nonoic acid (1.6325 g, 1 eq), N-Boc-γ-amino-1-propanol (1.9136 g, 1 eq), dicyclohexylcarbodiimide (2.0216 g, 1 eq) and 4-(dimethylamino) pyridine (0.1312 g, 0.1 eq) were successively dissolved into a 250 mL one-necked flask filled with 50 mL of DCM and sufficiently stirred for 18 h at room temperature. After the reaction was ended, a solvent was removed in vacuum, and a residue (PE of 0-10% EA) was purified by a flash column. After loading onto the column, the product was dissolved with 10 mL of DCM, 10 mL of TFA was then slowly dropwise added, and subsequently the above mixed solution was sufficiently stirred for 3 h. After the reaction was ended, 20 mL of saturated sodium bicarbonate solution was added, the above reaction system was extracted with DCM three times, dried over anhydrous Na₂SO₄ and filtered, and then the solvent was removed by rotary evaporation to obtain compound d5.

¹H NMR (500 MHz, CDCl₃, ppm): δ 4.21 (t, 2H), 2.69 (t, 2H), 2.31 (t, 2H), 2.03 (m, 2H), 1.61 (m, 2H), 1.29 (m, 10H), 0.88 (t, 3H). Chemical formula: C₁₂H₂₅NO₂.

### Synthesis of compound d7:

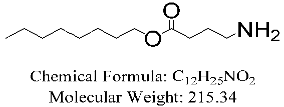

N-Boc-γ-aminobutyric acid (2.1604 g, 1 eq), n-octanol (1.3819 g, 1 eq), dicyclohexylcarbodiimide (2.0216 g, 1 eq) and 4-(dimethylamino) pyridine (0.1299 g, 0.1 eq) were successively dissolved into a 250 mL one-necked flask filled with 50 mL of DCM and sufficiently stirred for 18 h at room temperature. After the reaction was ended, a solvent was removed in vacuum, and a residue (PE of 0-10% EA) was purified by a flash column. After loading onto the column, the product was dissolved with 10 mL of DCM, 10 mL of TFA was then slowly dropwise added, and subsequently the above mixed solution was stirred for 3 h. After the reaction was ended, 20 mL of saturated sodium bicarbonate solution was added, the above reaction system was extracted with DCM three times, dried over anhydrous Na₂SO₄ and filtered, and then the solvent was removed by rotary evaporation to obtain compound d7.

¹H NMR (500 MHz, CDCl₃, ppm): δ 4.06 (t, 2H), 2.69 (t, 2H), 2.35 (t, 2H), 1.80 (m, 2H), 1.60 (m, 2H), 1.29 (m, 10H), 0.88 (t, 3H). Chemical formula: C₁₂H₂₅NO₂.

### Synthesis of compound d9:

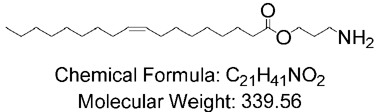

Oleic acid (2.8247 g, 1 eq), N-Boc-3-amino-1-propanol (1.7523 g, 1 eq), N-(3-dimethylaminopropyl)-N'-ethyl carbodiimide hydrochloride (1.9170 g, 1 eq) and 4-(dimethylamino) pyridine (0.1222 g, 0.1 eq) were successively dissolved into a 250 mL one-necked flask filled with 50 mL of DCM and sufficiently stirred for 18 h at room temperature. After the reaction was ended, a solvent was removed in vacuum, and a residue (PE of 0-10% EA) was purified by a flash column. After loading onto the column, the product was dissolved with 10 mL of DCM, 10 mL of TFA was then slowly dropwise added, and subsequently the above mixed solution was stirred for 3 h. After the reaction was ended, 20 mL of saturated sodium bicarbonate solution was added, the above reaction system was extracted with DCM three times, dried over anhydrous Na₂SO₄ and filtered, and then the solvent was removed by rotary evaporation to obtain compound d9. ¹H NMR (500 MHz, CDCl₃, ppm): δ 5.34 (m, 2H), 4.21 (t, 2H), 2.69 (t, 2H), 2.31 (t, 2H), 2.01 (m, 6H), 1.61 (m, 2H), 1.26 (m, 20H), 0.88 (t, 3H). Chemical formula: C₂₁H₄₁NO₂.

### Synthesis of compound d10:

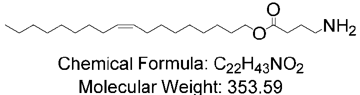

N-Boc-γ-aminobutyric acid (2.1604 g, 1 eq), cis-9-octadecenol (2.8561 g, 1 eq), N-(3-dimethylaminopropyl)-N'-ethyl carbodiimide hydrochloride (2.0378 g, 1 eq) and 4-(dimethylamino) pyridine (0.1299 g, 0.1 eq) were successively dissolved into a 250 mL one-necked flask filled with 50 mL of DCM, and sufficiently stirred for 18 h at room temperature. After the reaction was ended, a solvent was removed in vacuum, and a residue (PE of 0-10% EA) was purified through a flash column. After loading onto the column, the product was dissolved with 10 mL of DCM, 10 mL of TFA was then slowly dropwise added, and subsequently the above mixed solution was sufficiently stirred for 3 h. After the reaction was ended, 20 mL of saturated sodium bicarbonate solution was added, the above reaction system was extracted with DCM three times, dried over anhydrous Na₂SO₄ and filtered, and then the solvent was removed by rotary evaporation to obtain compound d10.

¹H NMR (500 MHz, CDCl₃, ppm): δ 5.34 (m, 2H), 4.06 (t, 2H), 2.69 (t, 2H), 2.35 (t, 2H), 2.01 (m, 4H), 1.80 (m, 2H), 1.60 (m, 2H), 1.29 (m, 22H), 0.88 (t, 3H). Chemical formula: C₂₂H₄₃NO₂.

### Synthesis of compound d11:

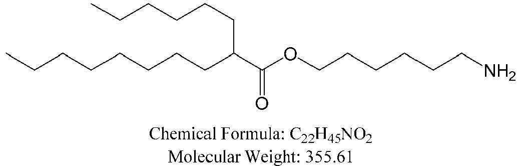

2-hexyldecanoic acid (1.7325 g, 1 eq), N-Boc-6-amino-1-hexanol (1.5386 g, 0.8 eq), dicyclohexylcarbodiimide (1.6506 g, 1 eq) and 4-(dimethylamino) pyridine (0.0977 g, 0.1 eq) were successively dissolved into a 250 mL one-necked flask filled with 50 mL of DCM, and sufficiently stirred for 18 h at room temperature. After the reaction was ended, a solvent was removed in vacuum, and a residue (PE of 0-10% EA) was purified through a flash column. After loading onto the column, the product was dissolved with 10 mL of DCM, 10 mL of TFA was slowly dropwise added, and then the above materials were sufficiently stirred for 3 h. After the reaction was ended, 20 mL of saturated sodium bicarbonate solution was added, the above system was extracted with DCM three times, dried over anhydrous Na₂SO₄ and filtered, and then the solvent was removed by rotary evaporation to obtain compound d11.

¹H NMR (500 MHz, CDCl₃, ppm): δ 4.08 (t, 2H), 2.69 (t, 2H), 2.30 (m, 1H), 1.80 (m, 2H), 1.66-1.25 (m, 32H), 0.88 (t, 6H). Chemical formula: C₂₂H₄₅NO₂.

### Synthesis of compound d12 (a14):

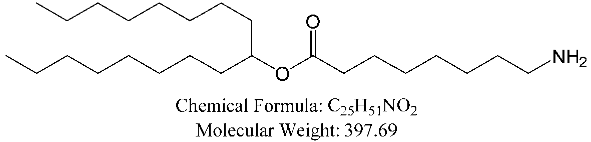

Boc-8-aminooctanoic acid (1.9179 g, 1.45 eq), 9-heptadecanol (1.3080 g, 1 eq), dicyclohexylcarbodiimide (1.0523 g, 1 eq) and 4-(dimetylamino) pyridine (0.0623 g, 0.1 eq) were successively dissolved into a 250 mL one-necked flask filled with 50 mL of DCM, and sufficiently stirred for 18 h at room temperature. After the reaction was ended, a solvent was removed in vacuum, and a residue (PE of 0-10% EA) was removed by a flash column. After loading onto the column, the product was dissolved with 10 mL of DCM, 10 mL of TFA was then slowly dropwise added, and subsequently the above mixed solution was sufficiently stirred for 3 h. After the reaction was ended, 20 mL of saturated sodium bicarbonate solution was added, the above system was extracted with DCM three times, dried over anhydrous Na₂SO₄ and filtered, and then the solvent was removed by rotary evaporation to obtain compound d12.

¹H NMR (500 MHz, CDCl₃, ppm): δ 4.47 (m, 1H), 2.69 (t, 2H), 2.29 (t, 2H), 1.65 (m, 4H), 1.50-1.26 (m, 34H), 0.88 (t, 6H). Chemical formula: C₂₅H₅₁NO₂.

Table 1 to Table 8 list four reactant structures corresponding to each lipid, each group of reactant compositions corresponds to one group of R₁, R_{II} (R_{II}'), R_{III} and R_{IV} structures in formula (I).

**Table 1**

| Lipid | First reactant | Second reactant | Third reactant | Fourth reactant | Lipid | First reactant | Second reactant | Third reactant | Fourth reactant | Lipid | First reactant | Second reactant | Third reactant | Fourth reactant | Lipid | First reactant | Second reactant | Third reactant | Fourth reactant |
|---|---|---|---|---|---|---|---|---|---|---|---|---|---|---|---|---|---|---|---|
| 1 | a4 | b9 | c10 | d8 | 41 | a7 | b2 | c4 | d8 | 81 | a4 | b2 | c12 | d3 | 121 | a15 | b4 | c14 | d3 |
| 2 | a4 | b3 | c10 | d1 | 42 | a7 | b2 | c5 | d8 | 82 | a4 | b2 | c14 | d3 | 122 | a4 | b8 | c5 | d10 |
| 3 | a4 | b1 | c10 | d2 | 43 | a7 | b2 | c14 | d8 | 83 | a4 | b2 | c5 | d9 | 123 | a5 | b8 | c5 | d10 |
| 4 | a4 | b2 | c5 | d8 | 44 | a5 | b9 | c4 | d1 | 84 | a4 | b2 | c12 | d9 | 124 | a4 | b8 | c5 | d3 |
| 5 | a4 | b2 | c6 | d8 | 45 | a5 | b9 | c5 | d1 | 85 | a4 | b2 | c15 | d9 | 125 | a5 | b8 | c5 | d3 |
| 6 | a4 | b2 | c7 | d8 | 46 | a5 | b9 | c14 | d1 | 86 | a4 | b2 | c5 | d10 | 126 | a15 | b8 | c5 | d3 |
| 7 | a4 | b2 | c8 | d8 | 47 | a5 | b9 | c4 | d8 | 87 | a4 | b2 | c12 | d10 | 127 | a4 | b8 | c12 | d3 |
| 8 | a4 | b2 | c12 | d8 | 48 | a5 | b9 | c5 | d8 | 88 | a4 | b2 | c15 | d10 | 128 | a5 | b8 | c12 | d3 |
| 9 | a4 | b2 | c10 | d6 | 49 | a5 | b9 | c14 | d8 | 89 | a5 | b2 | c5 | d3 | 129 | a15 | b8 | c12 | d3 |
| 10 | a4 | b2 | c9 | d6 | 50 | a6 | b9 | c4 | d1 | 90 | a5 | b2 | c12 | d3 | 130 | a4 | b8 | c14 | d3 |
| 11 | a4 | b2 | c13 | d8 | 51 | a6 | b9 | c5 | d1 | 91 | a5 | b2 | c14 | d3 | 131 | a5 | b8 | c14 | d3 |
| 12 | a4 | b2 | c17 | d8 | 52 | a6 | b9 | c14 | d1 | 92 | a5 | b2 | c6 | d9 | 132 | a15 | b8 | c14 | d3 |
| 13 | a4 | b2 | c18 | d8 | 53 | a6 | b9 | c4 | d8 | 93 | a5 | b2 | c12 | d9 | 133 | a4 | b8 | c12 | d10 |
| 14 | a4 | b2 | c16 | d8 | 54 | a6 | b9 | c5 | d8 | 94 | a5 | b2 | c14 | d9 | 134 | a5 | b8 | c12 | d10 |
| 15 | a4 | b2 | c15 | d8 | 55 | a6 | b9 | c14 | d8 | 95 | a5 | b2 | c5 | d10 | 135 | a4 | b8 | c5 | d9 |
| 16 | a4 | b2 | c11 | d8 | 56 | a7 | b9 | c4 | d1 | 96 | a5 | b2 | c12 | d10 | 136 | a5 | b8 | c5 | d9 |
| 17 | a4 | b2 | c19 | d8 | 57 | a7 | b9 | c5 | d1 | 97 | a5 | b2 | c14 | d10 | 137 | a4 | b8 | c12 | d9 |
| 18 | a15 | b2 | c3 | d8 | 58 | a7 | b9 | c14 | d1 | 98 | a15 | b2 | c5 | d3 | 138 | a5 | b8 | c12 | d9 |
| 19 | a4 | b15 | c1 | d8 | 59 | a7 | b9 | c4 | d8 | 99 | a15 | b2 | c12 | d3 | 139 | a4 | b8 | c14 | d9 |
| 20 | a4 | b15 | c2 | d8 | 60 | a7 | b9 | c5 | d8 | 100 | a15 | b2 | c14 | d3 | 140 | a5 | b8 | c14 | d9 |
| 21 | a1 | b15 | c3 | d8 | 61 | a7 | b9 | c14 | d8 | 101 | a4 | b2 | c5 | d3 | 141 | a4 | b8 | c14 | d10 |
| 22 | a2 | b15 | c3 | d8 | 62 | a5 | b10 | c4 | d8 | 102 | a4 | b4 | c12 | d3 | 142 | a5 | b8 | c14 | d10 |
| 23 | a15 | b2 | c17 | d3 | 63 | a6 | b10 | c4 | d8 | 103 | a4 | b4 | c14 | d3 | 143 | a4 | b2 | c5 | d8 |
| 24 | a15 | b2 | c18 | d3 | 64 | a7 | b10 | c4 | d8 | 104 | a4 | b4 | c5 | d9 | 144 | a4 | b2 | c6 | d8 |
| 25 | a15 | b2 | c19 | d3 | 65 | a5 | b7 | c4 | d1 | 105 | a4 | b4 | c12 | d9 | 145 | a4 | b2 | c12 | d8 |
| 26 | a5 | b2 | c4 | d1 | 66 | a6 | b7 | c4 | d1 | 106 | a4 | b4 | c14 | d9 | 146 | a4 | b2 | c11 | d4 |
| 27 | a5 | b2 | c5 | d1 | 67 | a7 | b7 | c4 | d1 | 107 | a4 | b4 | c5 | d10 | 147 | a4 | b2 | c14 | d4 |
| 28 | a5 | b2 | c14 | d1 | 68 | a5 | b7 | c5 | d1 | 108 | a4 | b4 | c12 | d10 | 148 | a5 | b2 | c5 | d4 |
| 29 | a5 | b2 | c4 | d8 | 69 | a6 | b7 | c5 | d1 | 109 | a4 | b4 | c14 | d10 | 149 | a5 | b2 | c6 | d4 |
| 30 | a5 | b2 | c5 | d8 | 70 | a7 | b7 | c5 | d1 | 110 | a5 | b4 | c5 | d3 | 150 | a5 | b2 | c12 | d4 |
| 31 | a5 | b2 | c14 | d8 | 71 | a5 | b7 | c14 | d1 | 111 | a5 | b4 | c12 | d3 | 151 | a5 | b2 | c11 | d4 |
| 32 | a6 | b2 | c4 | d1 | 72 | a6 | b7 | c14 | d1 | 112 | a5 | b4 | c14 | d3 | 152 | a5 | b2 | c14 | d4 |
| 33 | a6 | b2 | c5 | d1 | 73 | a7 | b7 | c14 | d1 | 113 | a5 | b4 | c5 | d9 | 153 | a4 | b2 | c5 | d5 |
| 34 | a6 | b2 | c14 | d1 | 74 | a5 | b10 | c5 | d8 | 114 | a5 | b4 | c12 | d9 | 154 | a4 | b2 | c6 | d5 |
| 35 | a6 | b2 | c14 | d8 | 75 | a6 | b10 | c5 | d8 | 115 | a5 | b4 | c14 | d9 | 155 | a4 | b2 | c12 | d5 |
| 36 | a6 | b2 | c5 | d8 | 76 | a7 | b10 | c5 | d8 | 116 | a5 | b4 | c5 | d10 | 156 | a4 | b2 | c11 | d5 |
| 37 | a6 | b2 | c14 | d8 | 77 | a5 | b10 | c14 | d8 | 117 | a5 | b4 | c12 | d10 | 157 | a3 | b2 | c14 | d5 |
| 38 | a7 | b2 | c4 | d1 | 78 | a6 | b10 | c14 | d8 | 118 | a5 | b4 | c14 | d10 | 158 | a5 | b2 | c5 | d5 |
| 39 | a7 | b2 | c5 | d1 | 79 | a7 | b10 | c14 | d8 | 119 | a5 | b4 | c5 | d3 | 159 | a5 | b2 | c6 | d5 |
| 40 | a7 | b2 | c14 | d1 | 80 | a4 | b2 | c5 | d 3 | 120 | a5 | b4 | c12 | d3 | 160 | a5 | b2 | c12 | d5 |

**Table 2**

| Lipid | First reactant | Second reactant | Third reactant | Fourth reactant | Lipid | First reactant | Second reactant | Third reactant | Fourth reactant | Lipid | First reactant | Second reactant | Third reactant | Fourth reactant | Lipid | First reactant | Second reactant | Third reactant | Fourth reactant |
|---|---|---|---|---|---|---|---|---|---|---|---|---|---|---|---|---|---|---|---|
| 161 | a5 | b2 | c11 | d5 | 202 | a9 | b2 | c14 | d5 | 243 | a4 | b2 | c5 | d11 | 284 | a4 | b2 | c6 | d11 |
| 162 | a5 | b2 | c14 | d5 | 203 | a8 | b2 | c5 | d6 | 244 | a5 | b2 | c17 | d11 | 285 | a4 | b2 | c12 | d11 |
| 163 | a4 | b2 | c5 | d6 | 204 | a8 | b2 | c6 | d6 | 245 | a5 | b2 | c18 | d11 | 286 | a4 | b2 | c11 | d11 |
| 164 | a4 | b2 | c6 | d6 | 205 | a8 | b2 | c12 | d6 | 246 | a5 | b2 | c5 | d11 | 287 | a4 | b2 | c14 | d11 |
| 165 | a4 | b2 | c12 | d6 | 206 | a8 | b2 | c11 | d6 | 247 | a10 | b2 | c17 | d11 | 288 | a4 | b2 | c6 | d7 |
| 166 | a4 | b2 | c11 | d6 | 207 | a8 | b2 | c14 | d6 | 248 | a10 | b2 | c18 | d11 | 289 | a4 | b2 | c12 | d7 |
| 167 | a4 | b2 | c14 | d6 | 208 | a9 | b2 | c5 | d6 | 249 | a10 | b2 | c5 | d11 | 290 | a4 | b2 | c11 | d7 |
| 168 | a5 | b2 | c5 | d6 | 209 | a9 | b2 | c6 | d6 | 250 | a11 | b2 | c3 | d8 | 291 | a4 | b2 | c14 | d7 |
| 169 | a5 | b2 | c6 | d6 | 210 | a9 | b2 | c12 | d6 | 251 | a15 | b2 | c5 | d3 | 292 | a5 | b2 | c6 | d11 |
| 170 | a5 | b2 | c12 | d6 | 211 | a9 | b2 | c11 | d6 | 252 | a4 | b2 | c10 | d4 | 293 | a5 | b2 | c12 | d11 |
| 171 | a5 | b2 | c11 | d6 | 212 | a9 | b2 | c14 | d6 | 253 | a11 | b14 | c3 | d1 | 294 | a5 | b2 | c11 | d11 |
| 172 | a5 | b2 | c14 | d6 | 213 | a8 | b2 | c5 | d12 | 254 | a 1 | b14 | c5 | d3 | 295 | a5 | b2 | c14 | d11 |
| 173 | a4 | b2 | c5 | d12 | 214 | a8 | b2 | c6 | d12 | 255 | a4 | b14 | c1 | d4 | 296 | a5 | b2 | c6 | d7 |
| 174 | a4 | b2 | c6 | d12 | 215 | a8 | b2 | c12 | d12 | 256 | a15 | b2 | c3 | d4 | 297 | a5 | b2 | c12 | d7 |
| 175 | a4 | b2 | c12 | d12 | 216 | a8 | b2 | c11 | d12 | 257 | a11 | b14 | c1 | d3 | 298 | a5 | b2 | c11 | d7 |
| 176 | a4 | b2 | c11 | d12 | 217 | a8 | b2 | c14 | d12 | 258 | a4 | b14 | c5 | d1 | 299 | a5 | b2 | c14 | d7 |
| 177 | a4 | b2 | c14 | d12 | 218 | a9 | b2 | c5 | d12 | 259 | a1 | b14 | c3 | d4 | 300 | a12 | b2 | c3 | d12 |
| 178 | a5 | b2 | c5 | d12 | 219 | a9 | b2 | c6 | d12 | 260 | a11 | b2 | c10 | d3 | 301 | a14 | b2 | c6 | d3 |
| 179 | a5 | b2 | c6 | d12 | 220 | a9 | b2 | c12 | d12 | 261 | a1 | b6 | c5 | d8 | 302 | a14 | b2 | c12 | d3 |
| 180 | a5 | b2 | c12 | d12 | 221 | a9 | b2 | c11 | d12 | 262 | a1 | b6 | c10 | d4 | 303 | a12 | b2 | c3 | d5 |
| 181 | a5 | b2 | c11 | d12 | 222 | a9 | b2 | c14 | d12 | 263 | a11 | b6 | c1 | d8 | 304 | a4 | b16 | c6 | d12 |
| 182 | a5 | b2 | c14 | d12 | 223 | a4 | b2 | c17 | d7 | 264 | a11 | b6 | c10 | d1 | 305 | a4 | b21 | c6 | d12 |
| 183 | a8 | b2 | c5 | d4 | 224 | a4 | b2 | c18 | d7 | 265 | a15 | b6 | c1 | d4 | 306 | a4 | b19 | c6 | d8 |
| 184 | a8 | b2 | c6 | d4 | 225 | a4 | b2 | c5 | d7 | 266 | a15 | b6 | c5 | d1 | 307 | a4 | b22 | c6 | d8 |
| 185 | a8 | b2 | c12 | d4 | 226 | a5 | b2 | c17 | d7 | 267 | a12 | b2 | c3 | d8 | 308 | a4 | b18 | c6 | d8 |
| 186 | a8 | b2 | c11 | d4 | 227 | a5 | b2 | c18 | d7 | 268 | a15 | b2 | c7 | d3 | 309 | a4 | b23 | c6 | d8 |
| 187 | a8 | b2 | c14 | d4 | 228 | a5 | b2 | c5 | d7 | 269 | a4 | b2 | c10 | d5 | 310 | a4 | b17 | c6 | d8 |
| 188 | a9 | b2 | c5 | d4 | 229 | a10 | b2 | c17 | d7 | 270 | a12 | b14 | c3 | d1 | 311 | a4 | b25 | c6 | d8 |
| 189 | a9 | b2 | c6 | d4 | 230 | a10 | b2 | c18 | d7 | 271 | a 1 | b14 | c7 | d3 | 312 | a4 | b20 | c6 | d8 |
| 190 | a9 | b2 | c12 | d4 | 231 | a10 | b2 | c5 | d7 | 272 | a4 | b14 | c1 | d5 | 313 | a4 | b24 | c6 | d8 |
| 191 | a9 | b2 | c11 | d4 | 232 | a4 | b2 | c17 | d12 | 273 | a15 | b2 | c3 | d5 | 314 | a4 | b15 | c20 | d8 |
| 192 | a9 | b2 | c14 | d4 | 233 | a4 | b2 | c18 | d12 | 274 | a12 | b14 | c1 | d3 | 315 | a4 | b15 | c21 | d8 |
| 193 | a8 | b2 | c5 | d5 | 234 | a4 | b2 | c5 | d12 | 275 | a4 | b14 | c7 | d1 | 316 | a4 | b2 | c22 | d8 |
| 194 | a8 | b2 | c6 | d5 | 235 | a5 | b2 | c17 | d12 | 276 | a1 | b14 | c3 | d5 | 317 | a4 | b2 | c22 | d12 |
| 195 | a8 | b2 | c12 | d5 | 236 | a5 | b2 | c18 | d12 | 277 | a12 | b2 | c10 | d3 | 318 | a4 | b10 | c10 | d13 |
| 196 | a8 | b2 | c11 | d5 | 237 | a5 | b2 | c5 | d12 | 278 | a1 | b6 | c7 | d8 | 319 | a4 | b2 | c6 | d14 |
| 197 | a8 | b2 | c14 | d5 | 238 | a10 | b2 | c17 | d12 | 279 | a1 | b6 | c10 | d5 | 320 | a3 | b2 | c5 | d8 |
| 198 | a9 | b2 | c5 | d5 | 239 | a10 | b2 | c18 | d12 | 280 | a12 | b6 | c1 | d8 | 321 | a8 | b2 | c5 | d8 |
| 199 | a9 | b2 | c6 | d5 | 240 | a10 | b2 | c5 | d12 | 281 | a12 | b6 | c10 | d1 | 322 | a13 | b2 | c5 | d8 |
| 200 | a9 | b2 | c12 | d5 | 241 | a4 | b2 | c17 | d 11 | 282 | a15 | b6 | c1 | d5 | 323 | a9 | b2 | c5 | d8 |
| 201 | a9 | b2 | c11 | d5 | 242 | a4 | b2 | c18 | d 11 | 283 | a15 | b6 | c7 | d1 | 324 | a10 | b2 | c5 | d8 |

**Table 3**

| Lipid | First reactant | Second reactant | Third reactant | Fourth reactant | Lipid | First reactant | Second reactant | Third reactant | Fourth reactant | Lipid | First reactant | Second reactant | Third reactant | Fourth reactant |
|---|---|---|---|---|---|---|---|---|---|---|---|---|---|---|
| 401 | a4 | b2 | c23 | e12 | 441 | a4 | b2 | c39 | e19 | 481 | a17 | b1 | c38 | e12 |
| 402 | a4 | b2 | c23 | e11 | 442 | a4 | b2 | c39 | e10 | 482 | a17 | b2 | c38 | e12 |
| 403 | a4 | b2 | c23 | e16 | 443 | a4 | b2 | c44 | e12 | 483 | a17 | b30 | c38 | e12 |
| 404 | a4 | b2 | c23 | e18 | 444 | a4 | b2 | c44 | e11 | 484 | a17 | b32 | c38 | e12 |
| 405 | a4 | b2 | c23 | e19 | 445 | a4 | b2 | c44 | e16 | 485 | a17 | b4 | c38 | e12 |
| 406 | a4 | b2 | c23 | e10 | 446 | a4 | b2 | c44 | e18 | 486 | a17 | b3 | c38 | e12 |
| 407 | a4 | b2 | c25 | e12 | 447 | a4 | b2 | c44 | e19 | 487 | a17 | b26 | c38 | e12 |
| 408 | a4 | b2 | c25 | e11 | 448 | a4 | b2 | c44 | e10 | 488 | a17 | b9 | c38 | e12 |
| 409 | a4 | b2 | c25 | e16 | 449 | a3 | b1 | c38 | e12 | 489 | a18 | b1 | c38 | e12 |
| 410 | a4 | b2 | c25 | e18 | 450 | a3 | b2 | c38 | e12 | 490 | a18 | b2 | c38 | e12 |
| 411 | a4 | b2 | c25 | e19 | 451 | a3 | b30 | c38 | e12 | 491 | a18 | b30 | c38 | e12 |
| 412 | a4 | b2 | c25 | e10 | 452 | a3 | b32 | c38 | e12 | 492 | a18 | b32 | c38 | e12 |
| 413 | a4 | b2 | c35 | e12 | 453 | a3 | b4 | c38 | e12 | 493 | a18 | b4 | c38 | e12 |
| 414 | a4 | b2 | c35 | e11 | 454 | a3 | b3 | c38 | e12 | 494 | a18 | b3 | c38 | e12 |
| 415 | a4 | b2 | c35 | e16 | 455 | a3 | b26 | c38 | e12 | 495 | a18 | b26 | c38 | e12 |
| 416 | a4 | b2 | c35 | e18 | 456 | a3 | b9 | c38 | e12 | 496 | a18 | b9 | c38 | e12 |
| 417 | a4 | b2 | c35 | e19 | 457 | a30 | b1 | c38 | e12 | 505 | a34 | b1 | c38 | e12 |
| 418 | a4 | b2 | c35 | e10 | 458 | a30 | b2 | c38 | e12 | 506 | a34 | b2 | c38 | e12 |
| 419 | a4 | b2 | c37 | e12 | 459 | a30 | b30 | c38 | e12 | 507 | a34 | b30 | c38 | e12 |
| 420 | a4 | b2 | c37 | e11 | 460 | a30 | b32 | c38 | e12 | 508 | a34 | b32 | c38 | e12 |
| 421 | a4 | b2 | c37 | e16 | 461 | a30 | b4 | c38 | e12 | 509 | a34 | b4 | c38 | e12 |
| 422 | a4 | b2 | c37 | e18 | 462 | a30 | b3 | c38 | e12 | 510 | a34 | b3 | c38 | e12 |
| 423 | a4 | b2 | c37 | e19 | 463 | a30 | b26 | c38 | e12 | 511 | a34 | b26 | c38 | e12 |
| 424 | a4 | b2 | c37 | e10 | 464 | a30 | b9 | c38 | e12 | 512 | a34 | b9 | c38 | e12 |
| 425 | a4 | b2 | c26 | e12 | 465 | a4 | b1 | c38 | e12 | 513 | a6 | b27 | c38 | e12 |
| 426 | a4 | b2 | c26 | e11 | 466 | a4 | b2 | c38 | e12 | 514 | a6 | b2 | c38 | e12 |
| 427 | a4 | b2 | c26 | e16 | 467 | a4 | b30 | c38 | e12 | 515 | a6 | b30 | c38 | e12 |
| 428 | a4 | b2 | c26 | e18 | 468 | a4 | b32 | c38 | e12 | 516 | a6 | b32 | c38 | e12 |
| 429 | a4 | b2 | c26 | e19 | 469 | a4 | b4 | c38 | e12 | 517 | a6 | b4 | c38 | e12 |
| 430 | a4 | b2 | c26 | e10 | 470 | a4 | b3 | c38 | e12 | 518 | a6 | b29 | c38 | e12 |
| 431 | a4 | b2 | c38 | e12 | 471 | a4 | b26 | c38 | e12 | 519 | a6 | b28 | c38 | e12 |
| 432 | a4 | b2 | c38 | e11 | 472 | a4 | b9 | c38 | e12 | 520 | a6 | b10 | c38 | e12 |
| 433 | a4 | b2 | c38 | e16 | 473 | a5 | b1 | c38 | e12 | 521 | a26 | b27 | c38 | e12 |
| 434 | a4 | b2 | c38 | e18 | 474 | a5 | b2 | c38 | e12 | 522 | a26 | b2 | c38 | e12 |
| 435 | a4 | b2 | c38 | e19 | 475 | a5 | b30 | c38 | e12 | 523 | a26 | b30 | c38 | e12 |
| 436 | a4 | b2 | c38 | e10 | 476 | a5 | b32 | c38 | e12 | 524 | a26 | b32 | c38 | e12 |
| 437 | a4 | b2 | c39 | e12 | 477 | a5 | b4 | c38 | e12 | 525 | a26 | b4 | c38 | e12 |
| 438 | a4 | b2 | c39 | e11 | 478 | a5 | b3 | c38 | e12 | 526 | a26 | b29 | c38 | e12 |
| 439 | a4 | b2 | c39 | e16 | 479 | a5 | b26 | c38 | e12 | 527 | a26 | b28 | c38 | e12 |
| 440 | a4 | b2 | c39 | e18 | 480 | a5 | b9 | c38 | e12 | 528 | a26 | b10 | c38 | e12 |

**Table 4**

| Lipid | First reactant | Second reactant | Third reactant | Fourth reactant | Lipid | First reactant | Second reactant | Third reactant | Fourth reactant | Lipid | First reactant | Second reactant | Third reactant | Fourth reactant |
|---|---|---|---|---|---|---|---|---|---|---|---|---|---|---|
| 529 | a27 | b27 | c38 | e12 | 569 | a22 | b27 | c38 | e12 | 609 | a4 | b2 | c26 | e15 |
| 530 | a27 | b2 | c38 | e12 | 570 | a22 | b2 | c38 | e12 | 610 | a4 | b2 | c38 | e15 |
| 531 | a27 | b30 | c38 | e12 | 571 | a22 | b30 | c38 | e12 | 611 | a4 | b2 | c39 | e15 |
| 532 | a27 | b32 | c38 | e12 | 572 | a22 | b32 | c38 | e12 | 612 | a4 | b2 | c44 | e15 |
| 533 | a27 | b4 | c38 | e12 | 573 | a22 | b4 | c38 | e12 | 613 | a4 | b2 | c35 | e20 |
| 534 | a27 | b29 | c38 | e12 | 574 | a22 | b29 | c38 | e12 | 614 | a4 | b2 | c37 | e20 |
| 535 | a27 | b28 | c38 | e12 | 575 | a22 | b28 | c38 | e12 | 615 | a4 | b2 | c26 | e20 |
| 536 | a27 | b10 | c38 | e12 | 576 | a22 | b10 | c38 | e12 | 616 | a4 | b2 | c38 | e20 |
| 537 | a28 | b27 | c38 | e12 | 577 | a4 | b2 | c24 | el4 | 617 | a4 | b2 | c39 | e20 |
| 538 | a28 | b2 | c38 | e12 | 578 | a4 | b2 | c36 | el4 | 618 | a4 | b2 | c44 | e20 |
| 539 | a28 | b30 | c38 | e12 | 579 | a4 | b2 | c27 | el4 | 619 | a4 | b2 | c35 | e29 |
| 540 | a28 | b32 | c38 | e12 | 580 | a4 | b2 | c42 | el4 | 620 | a4 | b2 | c37 | e29 |
| 541 | a28 | b4 | c38 | e12 | 581 | a4 | b2 | c23 | el4 | 621 | a4 | b2 | c26 | e29 |
| 542 | a28 | b29 | c38 | e12 | 582 | a4 | b2 | c25 | el4 | 622 | a4 | b2 | c38 | e29 |
| 543 | a28 | b28 | c38 | e12 | 583 | a4 | b2 | c24 | e15 | 623 | a4 | b2 | c39 | e29 |
| 544 | a28 | b10 | c38 | e12 | 584 | a4 | b2 | c36 | e15 | 624 | a4 | b2 | c44 | e29 |
| 545 | a19 | b27 | c38 | e12 | 585 | a4 | b2 | c27 | e15 | 625 | a4 | b2 | c5 | e12 |
| 546 | a19 | b2 | c38 | e12 | 586 | a4 | b2 | c42 | e15 | 627 | a4 | b2 | c5 | e16 |
| 547 | a19 | b30 | c38 | e12 | 587 | a4 | b2 | c23 | e15 | 628 | a4 | b2 | c5 | e18 |
| 548 | a19 | b32 | c38 | e12 | 588 | a4 | b2 | c25 | e15 | 629 | a4 | b2 | c5 | e19 |
| 549 | a19 | b4 | c38 | e12 | 589 | a4 | b2 | c24 | e20 | 631 | a4 | b2 | c5 | e14 |
| 550 | a19 | b29 | c38 | e12 | 590 | a4 | b2 | c36 | e20 | 632 | a4 | b2 | c5 | e15 |
| 551 | a19 | b28 | c38 | e12 | 591 | a4 | b2 | c27 | e20 | 633 | a4 | b2 | c5 | e20 |
| 552 | a19 | b10 | c38 | e12 | 592 | a4 | b2 | c42 | e20 | 634 | a4 | b2 | c5 | e29 |
| 553 | a20 | b27 | c38 | e12 | 593 | a4 | b2 | c23 | e20 | 635 | a4 | b2 | c33 | e12 |
| 554 | a20 | b2 | c38 | e12 | 594 | a4 | b2 | c25 | e20 | 636 | a4 | b2 | c33 | e11 |
| 555 | a20 | b30 | c38 | e12 | 595 | a4 | b2 | c24 | e29 | 637 | a4 | b2 | c33 | e16 |
| 556 | a20 | b32 | c38 | e12 | 596 | a4 | b2 | c36 | e29 | 638 | a4 | b2 | c33 | e18 |
| 557 | a20 | b4 | c38 | e12 | 597 | a4 | b2 | c27 | e29 | 639 | a4 | b2 | c33 | e19 |
| 558 | a20 | b29 | c38 | e12 | 598 | a4 | b2 | c42 | e29 | 640 | a4 | b2 | c33 | e10 |
| 559 | a20 | b28 | c38 | e12 | 599 | a4 | b2 | c23 | e29 | 641 | a4 | b2 | c33 | e14 |
| 560 | a20 | b10 | c38 | e12 | 600 | a4 | b2 | c25 | e29 | 642 | a4 | b2 | c33 | e15 |
| 561 | a21 | b27 | c38 | e12 | 601 | a4 | b2 | c35 | e14 | 643 | a4 | b2 | c33 | e20 |
| 562 | a21 | b2 | c38 | e12 | 602 | a4 | b2 | c37 | el4 | 644 | a4 | b2 | c33 | e29 |
| 563 | a21 | b30 | c38 | e12 | 603 | a4 | b2 | c26 | el4 | 645 | a4 | b2 | c6 | e12 |
| 564 | a21 | b32 | c38 | e12 | 604 | a4 | b2 | c38 | el4 | 647 | a4 | b2 | c6 | e16 |
| 565 | a21 | b4 | c38 | e12 | 605 | a4 | b2 | c39 | el4 | 648 | a4 | b2 | c6 | e18 |
| 566 | a21 | b29 | c38 | e12 | 606 | a4 | b2 | c44 | el4 | 649 | a4 | b2 | c6 | e19 |
| 567 | a21 | b28 | c38 | e12 | 607 | a4 | b2 | c35 | e15 | 651 | a4 | b2 | c6 | e14 |
| 568 | a21 | b10 | c38 | e12 | 608 | a4 | b2 | c37 | e15 | 652 | a4 | b2 | c6 | e15 |

**Table 5**

| Lipid | First reactant | Second reactant | Third reactant | Fourth reactant | Lipid | First reactant | Second reactant | Third reactant | Fourth reactant | Lipid | First reactant | Second reactant | Third reactant | Fourth reactant |
|---|---|---|---|---|---|---|---|---|---|---|---|---|---|---|
| 653 | a4 | b2 | c6 | e20 | 695 | a4 | b2 | c11 | e12 | 735 | a4 | b2 | c24 | e7 |
| 654 | a4 | b2 | c6 | e29 | 697 | a4 | b2 | c11 | e16 | 736 | a4 | b2 | c36 | e7 |
| 655 | a4 | b2 | c7 | e12 | 698 | a4 | b2 | c11 | el8 | 737 | a4 | b2 | c27 | e7 |
| 656 | a4 | b2 | c7 | e11 | 699 | a4 | b2 | c11 | e19 | 738 | a4 | b2 | c42 | e7 |
| 657 | a4 | b2 | c7 | e16 | 699 | a4 | b2 | c11 | e19 | 739 | a4 | b2 | c23 | e7 |
| 658 | a4 | b2 | c7 | e18 | 701 | a4 | b2 | c11 | el4 | 740 | a4 | b2 | c25 | e7 |
| 659 | a4 | b2 | c7 | e19 | 702 | a4 | b2 | c11 | el5 | 741 | a4 | b2 | c35 | e6 |
| 660 | a4 | b2 | c7 | e10 | 703 | a4 | b2 | c11 | e20 | 742 | a4 | b2 | c37 | e6 |
| 661 | a4 | b2 | c7 | e14 | 704 | a4 | b2 | c11 | e29 | 743 | a4 | b2 | c26 | e6 |
| 662 | a4 | b2 | c7 | e15 | 705 | a4 | b2 | c24 | e3 | 744 | a4 | b2 | c38 | e6 |
| 663 | a4 | b2 | c7 | e20 | 706 | a4 | b2 | c36 | e3 | 745 | a4 | b2 | c39 | e6 |
| 664 | a4 | b2 | c7 | e29 | 707 | a4 | b2 | c27 | e3 | 746 | a4 | b2 | c44 | e6 |
| 665 | a4 | b2 | c8 | e12 | 708 | a4 | b2 | c42 | e3 | 747 | a4 | b2 | c35 | e4 |
| 666 | a4 | b2 | c8 | e11 | 709 | a4 | b2 | c23 | e3 | 748 | a4 | b2 | c37 | e4 |
| 667 | a4 | b2 | c8 | e16 | 710 | a4 | b2 | c25 | e3 | 749 | a4 | b2 | c26 | e4 |
| 668 | a4 | b2 | c8 | e18 | 711 | a4 | b2 | c24 | e33 | 750 | a4 | b2 | c38 | e4 |
| 669 | a4 | b2 | c8 | e19 | 712 | a4 | b2 | c36 | e33 | 751 | a4 | b2 | c39 | e4 |
| 670 | a4 | b2 | c8 | e10 | 713 | a4 | b2 | c27 | e33 | 752 | a4 | b2 | c44 | e4 |
| 671 | a4 | b2 | c8 | e14 | 714 | a4 | b2 | c42 | e33 | 753 | a4 | b2 | c35 | e32 |
| 672 | a4 | b2 | c8 | e15 | 715 | a4 | b2 | c23 | e33 | 754 | a4 | b2 | c37 | e32 |
| 673 | a4 | b2 | c8 | e20 | 716 | a4 | b2 | c25 | e33 | 755 | a4 | b2 | c26 | e32 |
| 674 | a4 | b2 | c8 | e29 | 717 | a4 | b2 | c24 | e4 | 756 | a4 | b2 | c38 | e32 |
| 675 | a4 | b2 | c10 | e12 | 718 | a4 | b2 | c36 | e4 | 757 | a4 | b2 | c39 | e32 |
| 676 | a4 | b2 | c10 | e11 | 719 | a4 | b2 | c27 | e4 | 758 | a4 | b2 | c44 | e32 |
| 677 | a4 | b2 | c10 | e16 | 720 | a4 | b2 | c42 | e4 | 759 | a4 | b2 | c35 | e7 |
| 678 | a4 | b2 | c10 | e18 | 721 | a4 | b2 | c23 | e4 | 760 | a4 | b2 | c37 | e7 |
| 679 | a4 | b2 | c10 | e19 | 722 | a4 | b2 | c25 | e4 | 761 | a4 | b2 | c26 | e7 |
| 680 | a4 | b2 | c10 | e10 | 723 | a4 | b2 | c35 | e4 | 762 | a4 | b2 | c38 | e7 |
| 681 | a4 | b2 | c10 | e14 | 724 | a4 | b2 | c37 | e4 | 763 | a4 | b2 | c39 | e7 |
| 682 | a4 | b2 | c10 | e15 | 725 | a4 | b2 | c26 | e4 | 764 | a4 | b2 | c44 | e7 |
| 683 | a4 | b2 | c10 | e20 | 726 | a4 | b2 | c38 | e4 | 765 | a4 | b2 | c24 | e12 |
| 684 | a4 | b2 | c10 | e29 | 726 | a4 | b2 | c38 | e4 | 766 | a4 | b2 | c24 | e11 |
| 685 | a4 | b2 | c12 | e12 | 727 | a4 | b2 | c39 | e4 | 767 | a4 | b2 | c24 | e16 |
| 687 | a4 | b2 | c12 | e16 | 728 | a4 | b2 | c44 | e4 | 768 | a4 | b2 | c24 | e18 |
| 688 | a4 | b2 | c12 | e18 | 729 | a4 | b2 | c24 | e32 | 769 | a4 | b2 | c24 | e19 |
| 689 | a4 | b2 | c12 | e19 | 730 | a4 | b2 | c36 | e32 | 770 | a4 | b2 | c24 | e10 |
| 691 | a4 | b2 | c12 | e14 | 731 | a4 | b2 | c27 | e32 | 771 | a4 | b2 | c24 | e13 |
| 692 | a4 | b2 | c12 | e15 | 732 | a4 | b2 | c42 | e32 | 772 | a4 | b2 | c36 | e12 |
| 693 | a4 | b2 | c12 | e20 | 733 | a4 | b2 | c23 | e32 | 773 | a4 | b2 | c36 | e11 |
| 694 | a4 | b2 | c12 | e29 | 734 | a4 | b2 | c25 | e32 | 774 | a4 | b2 | c36 | e16 |

**Table 6**

| Lipid | First reactant | Second reactant | Third reactant | Fourth reactant | Lipid | First reactant | Second reactant | Third reactant | Fourth reactant | Lipid | First reactant | Second reactant | Third reactant | Fourth reactant |
|---|---|---|---|---|---|---|---|---|---|---|---|---|---|---|
| 775 | a4 | b2 | c36 | e18 | 815 | a5 | b2 | c26 | e11 | 857 | a31 | b33 | c6 | e10 |
| 776 | a4 | b2 | c36 | e19 | 816 | a5 | b2 | c26 | e16 | 858 | a3 I | b2 | c6 | e10 |
| 777 | a4 | b2 | c36 | e10 | 817 | a5 | b2 | c26 | e18 | 859 | a3 I | b31 | c6 | e10 |
| 778 | a4 | b2 | c36 | e13 | 818 | a5 | b2 | c26 | e19 | 860 | a17 | b33 | c6 | e10 |
| 779 | a4 | b2 | c27 | e12 | 819 | a5 | b2 | c26 | e10 | 861 | a17 | b2 | c6 | e10 |
| 780 | a4 | b2 | c27 | e11 | 820 | a5 | b2 | c26 | e13 | 862 | a17 | b31 | c6 | e10 |
| 781 | a4 | b2 | c27 | e16 | 821 | a3 | b33 | c6 | e10 | 863 | a34 | b33 | c6 | e10 |
| 782 | a4 | b2 | c27 | e18 | 822 | a3 | b2 | c6 | e10 | 864 | a34 | b2 | c6 | e10 |
| 783 | a4 | b2 | c27 | e19 | 823 | a3 | b31 | c6 | e10 | 865 | a34 | b31 | c6 | e10 |
| 784 | a4 | b2 | c27 | e10 | 824 | a19 | b33 | c6 | e10 | 866 | a18 | b33 | c6 | e10 |
| 785 | a4 | b2 | c27 | e13 | 825 | a19 | b2 | c6 | e10 | 867 | a18 | b2 | c6 | e10 |
| 786 | a4 | b2 | c26 | e12 | 826 | a19 | b31 | c6 | e10 | 868 | a18 | b31 | c6 | e10 |
| 787 | a4 | b2 | c26 | e11 | 827 | a30 | b33 | c6 | e10 | 869 | a4 | b2 | c34 | e13 |
| 788 | a4 | b2 | c26 | e16 | 828 | a30 | b2 | c6 | e10 | 870 | a4 | b2 | c31 | e13 |
| 789 | a4 | b2 | c26 | e18 | 829 | a30 | b31 | c6 | e10 | 871 | a4 | b2 | c32 | e13 |
| 790 | a4 | b2 | c26 | e19 | 830 | a20 | b33 | c6 | e10 | 872 | a4 | b2 | c5 | e13 |
| 791 | a4 | b2 | c26 | e10 | 831 | a20 | b2 | c6 | e10 | 873 | a4 | b2 | c33 | e13 |
| 792 | a4 | b2 | c26 | e13 | 832 | a20 | b31 | c6 | e10 | 874 | a4 | b2 | c6 | e13 |
| 793 | a5 | b2 | c24 | e12 | 833 | a4 | b33 | c6 | e10 | 875 | a4 | b2 | c7 | e13 |
| 794 | a5 | b2 | c24 | e11 | 835 | a4 | b31 | c6 | e10 | 876 | a4 | b2 | c8 | e13 |
| 795 | a5 | b2 | c24 | e16 | 836 | a21 | b33 | c6 | e10 | 877 | a4 | b2 | c10 | e13 |
| 796 | a5 | b2 | c24 | e18 | 837 | a21 | b2 | c6 | e10 | 878 | a4 | b2 | c12 | e13 |
| 797 | a5 | b2 | c24 | e19 | 838 | a21 | b31 | c6 | e10 | 879 | a4 | b2 | c34 | e30 |
| 798 | a5 | b2 | c24 | e10 | 839 | a5 | b33 | c6 | e10 | 880 | a4 | b2 | c31 | e30 |
| 799 | a5 | b2 | c24 | e13 | 841 | a5 | b31 | c6 | e10 | 881 | a4 | b2 | c32 | e30 |
| 800 | a5 | b2 | c36 | e12 | 842 | a22 | b33 | c6 | e10 | 882 | a4 | b2 | c5 | e30 |
| 801 | a5 | b2 | c36 | e11 | 843 | a22 | b2 | c6 | e10 | 883 | a4 | b2 | c33 | e30 |
| 802 | a5 | b2 | c36 | e16 | 844 | a22 | b31 | c6 | e10 | 884 | a4 | b2 | c6 | e30 |
| 803 | a5 | b2 | c36 | e18 | 845 | a6 | b33 | c6 | e10 | 885 | a4 | b2 | c7 | e30 |
| 804 | a5 | b2 | c36 | e19 | 846 | a6 | b2 | c6 | e10 | 886 | a4 | b2 | c8 | e30 |
| 805 | a5 | b2 | c36 | e10 | 847 | a6 | b31 | c6 | e10 | 887 | a4 | b2 | c10 | e30 |
| 806 | a5 | b2 | c36 | e13 | 848 | a23 | b33 | c6 | e10 | 888 | a4 | b2 | c12 | e30 |
| 807 | a5 | b2 | c27 | e12 | 849 | a23 | b2 | c6 | e10 | 889 | a4 | b2 | c34 | e31 |
| 808 | a5 | b2 | c27 | e11 | 850 | a23 | b31 | c6 | e10 | 890 | a4 | b2 | c31 | e31 |
| 809 | a5 | b2 | c27 | e16 | 851 | a29 | b33 | c6 | e10 | 891 | a4 | b2 | c32 | e31 |
| 810 | a5 | b2 | c27 | e18 | 852 | a29 | b2 | c6 | e10 | 892 | a4 | b2 | c5 | e31 |
| 811 | a5 | b2 | c27 | e19 | 853 | a29 | b31 | c6 | e10 | 893 | a4 | b2 | c33 | e31 |
| 812 | a5 | b2 | c27 | e10 | 854 | a32 | b33 | c6 | e10 | 894 | a4 | b2 | c6 | e31 |
| 813 | a5 | b2 | c27 | e13 | 855 | a32 | b2 | c6 | e10 | 895 | a4 | b2 | c7 | e31 |
| 814 | a5 | b2 | c26 | e12 | 856 | a32 | b31 | c6 | e10 | 896 | a4 | b2 | c8 | e31 |

**Table 7**

| Lipid | First reactant | Second reactant | Third reactant | Fourth reactant | Lipid | First reactant | Second reactant | Third reactant | Fourth reactant |
|---|---|---|---|---|---|---|---|---|---|
| 897 | a4 | b2 | c10 | e31 | 945 | a4 | b43 | c12 | e11 |
| 898 | a4 | b2 | c12 | e31 | 946 | a4 | b44 | c12 | e11 |
| 899 | a4 | b2 | c34 | e3 | 947 | a4 | b45 | c12 | e11 |
| 900 | a4 | b2 | c31 | e3 | 949 | a4 | b38 | c12 | e11 |
| 901 | a4 | b2 | c32 | e3 | 950 | a4 | b39 | c12 | e11 |
| 902 | a4 | b2 | c34 | e4 | 951 | a4 | b16 | c12 | e11 |
| 903 | a4 | b2 | c31 | e4 | 952 | a4 | bl7 | c12 | e11 |
| 904 | a4 | b2 | c32 | e4 | 953 | a4 | b48 | c6 | e10 |
| 905 | a4 | b2 | c34 | e32 | 954 | a4 | b40 | c6 | e10 |
| 906 | a4 | b2 | c31 | e32 | 955 | a4 | b46 | c6 | e10 |
| 907 | a4 | b2 | c32 | e32 | 956 | a4 | b49 | c6 | e10 |
| 908 | a4 | b2 | c34 | e7 | 958 | a4 | b47 | c6 | e10 |
| 909 | a4 | b2 | c31 | e7 | 959 | a4 | b4 | c6 | e10 |
| 910 | a4 | b2 | c32 | e7 | 960 | a4 | b33 | c6 | e10 |
| 911 | a4 | b2 | c34 | e33 | 961 | a4 | b31 | c6 | e10 |
| 912 | a4 | b2 | c31 | e33 | 962 | a4 | b4 | c6 | e11 |
| 913 | a4 | b2 | c32 | e33 | 963 | a4 | b33 | c6 | e11 |
| 918 | a4 | b2 | c47 | e15 | 964 | a4 | b31 | c6 | e11 |
| 919 | a4 | b2 | c47 | e29 | 965 | a4 | b49 | c12 | e11 |
| 920 | a22 | b2 | c6 | e11 | | | | | |
| 921 | a22 | b2 | c12 | e11 | | | | | |
| 922 | a22 | b2 | c6 | e10 | | | | | |
| 923 | a22 | b2 | c12 | e10 | | | | | |
| 924 | a22 | b2 | c14 | e10 | | | | | |
| 925 | a22 | b30 | c6 | e10 | | | | | |
| 926 | a24 | b2 | c6 | e10 | | | | | |
| 927 | a24 | b2 | c6 | e11 | | | | | |
| 928 | a25 | b2 | c6 | e10 | | | | | |
| 929 | a25 | b2 | c6 | e11 | | | | | |
| 934 | a4 | b34 | c12 | e11 | | | | | |
| 935 | a4 | b35 | c12 | e11 | | | | | |
| 936 | a4 | b1 | c12 | e11 | | | | | |
| 937 | a4 | b36 | c12 | e11 | | | | | |
| 938 | a4 | b41 | c12 | e11 | | | | | |
| 939 | a4 | b30 | c12 | e11 | | | | | |
| 940 | a4 | b32 | c12 | e11 | | | | | |
| 941 | a4 | b4 | c12 | e11 | | | | | |
| 942 | a4 | b33 | c12 | e11 | | | | | |
| 943 | a4 | b31 | c12 | e11 | | | | | |
| 944 | a4 | b42 | c12 | e11 | | | | | |

**Table 8**

| Lipid | First reactant | Second reactant | Third reactant | Fourth reactant | Lipid | First reactant | Second reactant | Third reactant | Fourth reactant | Lipid | First reactant | Second reactant | Third reactant | Fourth reactant |
|---|---|---|---|---|---|---|---|---|---|---|---|---|---|---|
| 966 | a4 | b33 | c48 | e10 | 1003 | a4 | b2 | c48 | e16 | 1040 | a4 | b38 | c6 | e11 |
| 967 | a4 | b2 | c48 | e10 | 1004 | a4 | b32 | c48 | e16 | 1041 | a4 | b39 | c6 | e10 |
| 968 | a4 | b32 | c48 | e10 | 1005 | a4 | b4 | c48 | e16 | 1042 | a4 | b39 | c6 | e11 |
| 969 | a4 | b4 | c48 | e10 | 1006 | a4 | b33 | c49 | e11 | 1043 | a4 | b36 | c6 | e10 |
| 970 | a4 | b33 | c48 | e12 | 1007 | a4 | b2 | c49 | e11 | 1044 | a4 | b36 | c6 | e11 |
| 971 | a4 | b2 | c48 | e12 | 1008 | a4 | b32 | c49 | e11 | 1045 | a4 | b3 | c12 | e11 |
| 972 | a4 | b32 | c48 | e12 | 1009 | a4 | b4 | c49 | e11 | 1046 | a4 | b3 | c6 | e10 |
| 973 | a4 | b4 | c48 | e12 | 1010 | a4 | b33 | c49 | e16 | 1047 | a4 | b3 | c6 | e11 |
| 974 | a4 | b33 | c49 | e10 | 1011 | a4 | b2 | c49 | e16 | 1048 | a4 | b9 | c12 | e11 |
| 975 | a4 | b2 | c49 | e10 | 1012 | a4 | b32 | c49 | e16 | 1049 | a4 | b9 | c6 | e10 |
| 976 | a4 | b32 | c49 | e10 | 1013 | a4 | b4 | c49 | e16 | 1050 | a4 | b9 | c6 | e11 |
| 977 | a4 | b4 | c49 | e10 | 1014 | a4 | b33 | c50 | e11 | 1051 | a4 | b18 | c12 | e11 |
| 978 | a4 | b33 | c49 | e12 | 1015 | a4 | b2 | c50 | e11 | 1053 | a4 | b17 | c6 | e11 |
| 979 | a4 | b2 | c49 | e12 | 1016 | a4 | b32 | c50 | e11 | 1054 | a4 | b18 | c6 | e11 |
| 980 | a4 | b32 | c49 | e12 | 1017 | a4 | b4 | c50 | e11 | 1055 | a4 | b16 | c6 | e10 |
| 981 | a4 | b4 | c49 | e12 | 1018 | a4 | b33 | c50 | e16 | 1056 | a4 | b17 | c6 | e10 |
| 982 | a4 | b33 | c50 | e10 | 1019 | a4 | b2 | c50 | e16 | 1057 | a4 | b18 | c6 | e10 |
| 983 | a4 | b2 | c50 | e10 | 1020 | a4 | b32 | c50 | e16 | 1058 | a4 | b2 | c22 | e10 |
| 984 | a4 | b32 | c50 | e10 | 1021 | a4 | b4 | c50 | e16 | 1059 | a4 | b2 | c22 | e11 |
| 985 | a4 | b4 | c50 | e10 | 1022 | a4 | b33 | c51 | e11 | 1060 | a4 | b2 | c53 | e11 |
| 986 | a4 | b33 | c50 | e12 | 1023 | a4 | b2 | c51 | e11 | 1061 | a4 | b2 | c55 | e11 |
| 987 | a4 | b2 | c50 | e12 | 1024 | a4 | b32 | c51 | e11 | 1062 | a4 | b2 | c55 | e10 |
| 988 | a4 | b32 | c50 | e12 | 1025 | a4 | b4 | c51 | e11 | 1063 | a4 | b2 | c57 | e12 |
| 989 | a4 | b4 | c50 | e12 | 1026 | a4 | b33 | c51 | e16 | 1064 | a4 | b2 | c57 | e11 |
| 990 | a4 | b33 | c51 | e10 | 1027 | a4 | b2 | c51 | e16 | 1065 | a4 | b2 | c57 | e10 |
| 991 | a4 | b2 | c51 | e10 | 1028 | a4 | b32 | c51 | e16 | 1066 | a4 | b2 | c52 | e12 |
| 992 | a4 | b32 | c51 | e10 | 1029 | a4 | b4 | c51 | e16 | 1067 | a4 | b2 | c53 | e12 |
| 993 | a4 | b4 | c51 | e10 | 1030 | a36 | b2 | c6 | e10 | 1068 | a4 | b2 | c58 | e11 |
| 994 | a4 | b33 | c51 | e12 | 1031 | a36 | b2 | c6 | e11 | 1069 | a4 | b2 | c58 | e10 |
| 995 | a4 | b2 | c51 | e12 | 1032 | a37 | b2 | c6 | e10 | 1070 | a4 | b2 | c52 | e16 |
| 996 | a4 | b32 | c51 | e12 | 1033 | a37 | b2 | c6 | e11 | 1071 | a4 | b2 | c53 | e16 |
| 997 | a4 | b4 | c51 | e12 | 1034 | a38 | b2 | c6 | e10 | 1072 | a4 | b2 | c56 | e11 |
| 998 | a4 | b33 | c48 | e11 | 1035 | a38 | b2 | c6 | e11 | 1073 | a4 | b2 | c56 | e10 |
| 999 | a4 | b2 | c48 | e11 | 1036 | a4 | b50 | c6 | e10 | 1074 | a4 | b2 | c53 | e10 |
| 1000 | a4 | b32 | c48 | e11 | 1037 | a4 | b2 | c5 | e10 | 1075 | a4 | b2 | c54 | e10 |
| 1001 | a4 | b4 | c48 | e11 | 1038 | a4 | b2 | c7 | e10 | | | | | |
| 1002 | a4 | b33 | c48 | e16 | 1039 | a4 | b38 | c6 | e10 | | | | | |

The structural formulas of some synthesized lipids in this example are as follows:

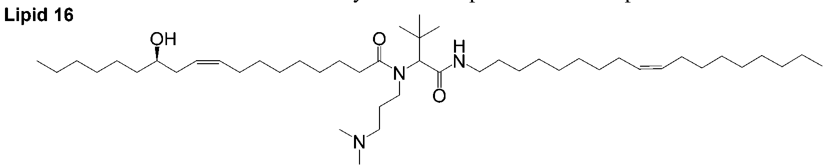

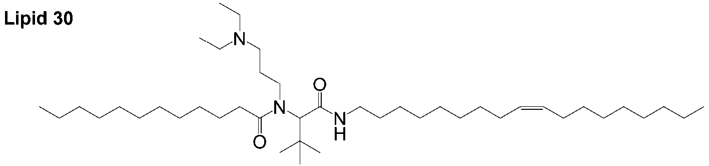

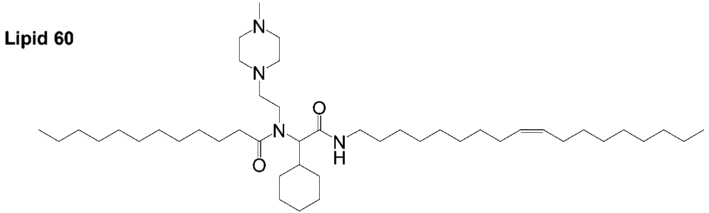

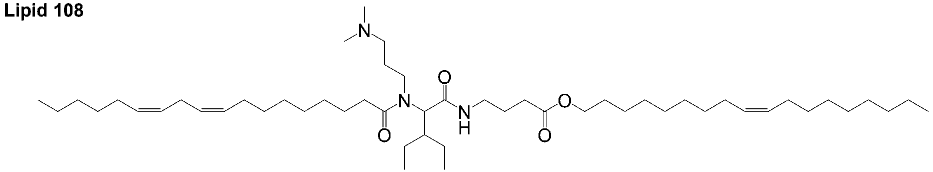

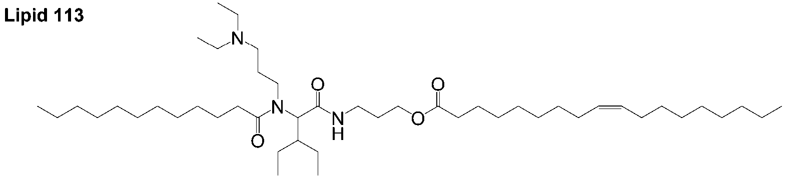

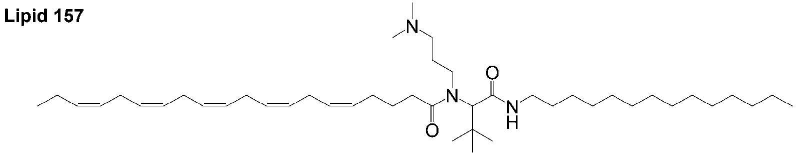

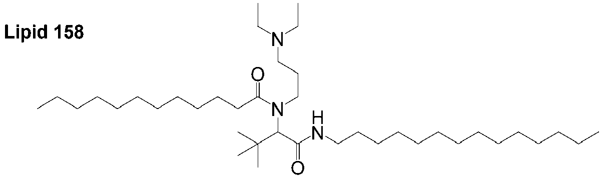

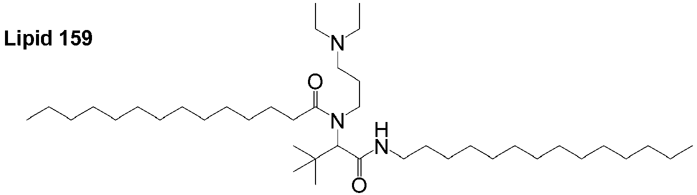

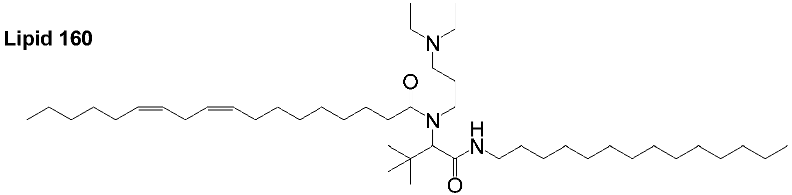

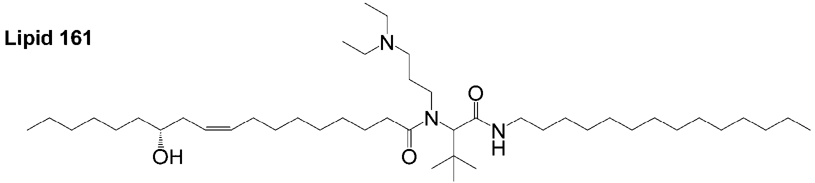

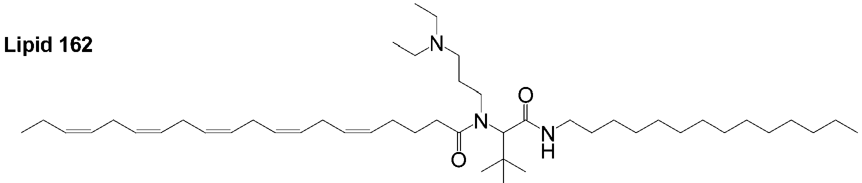

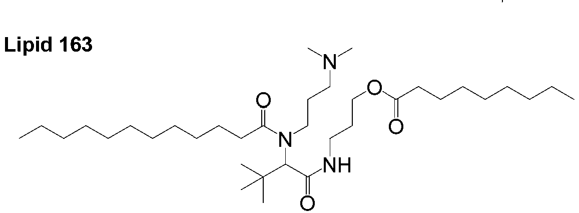

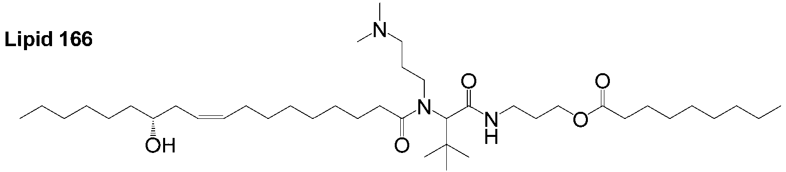

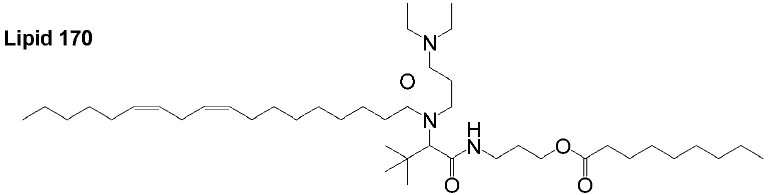

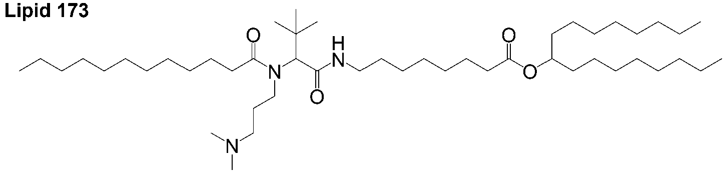

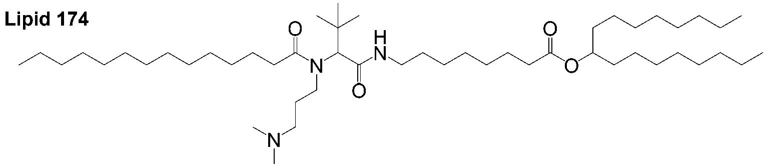

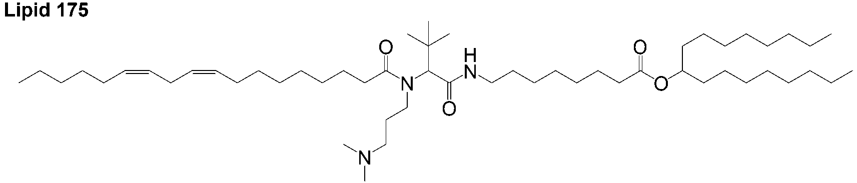

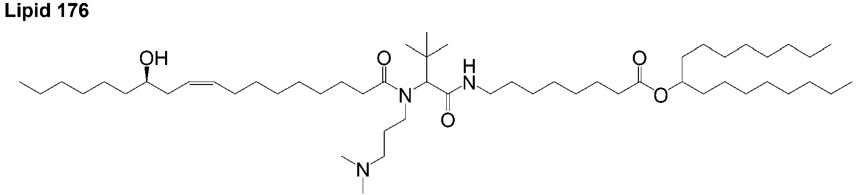

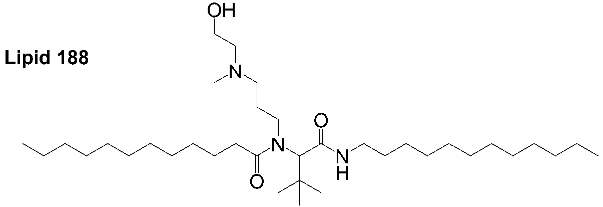

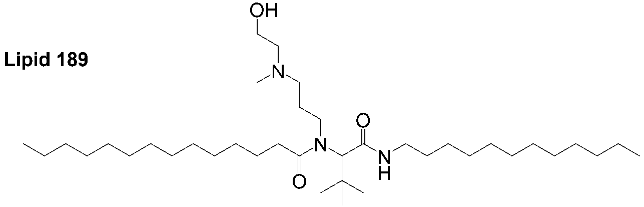

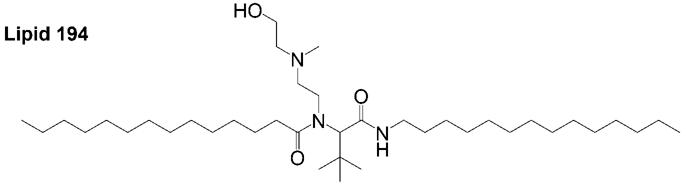

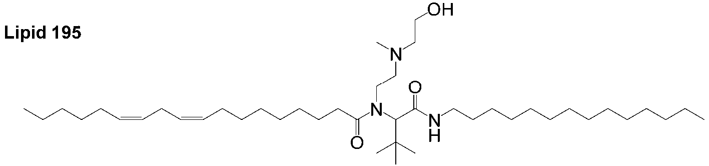

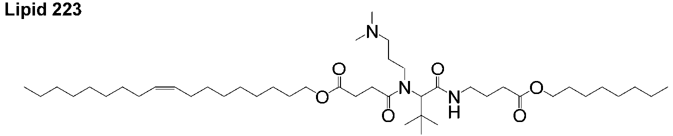

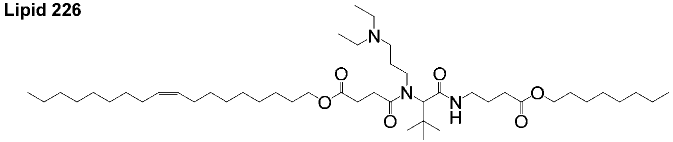

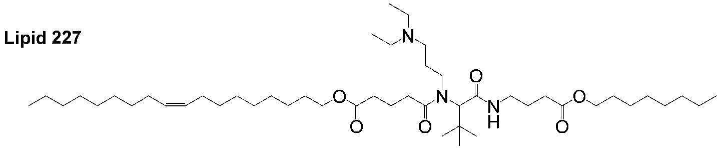

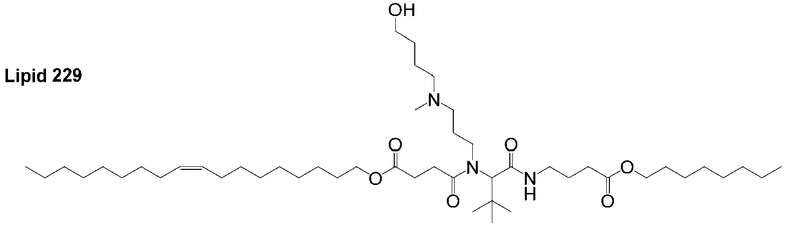

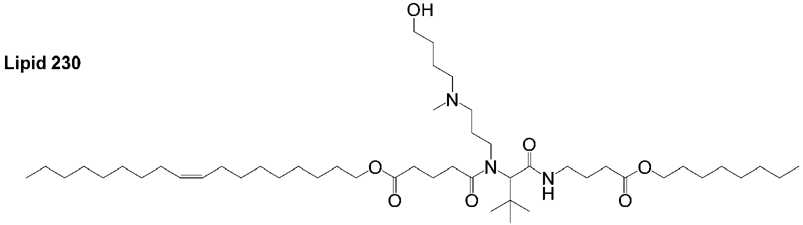

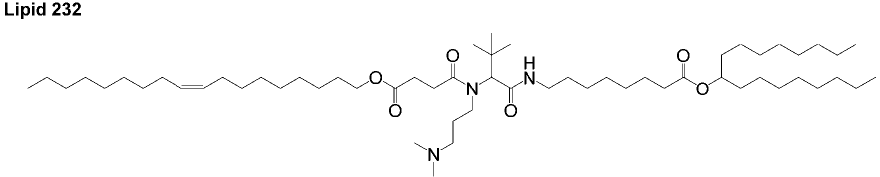

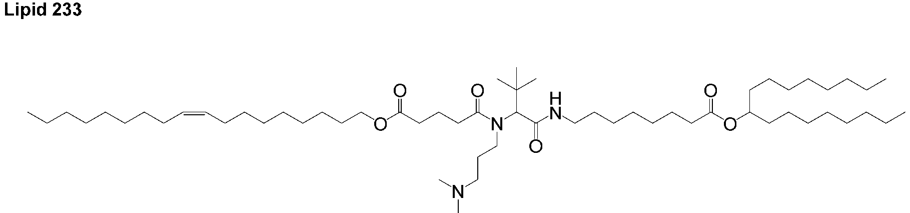

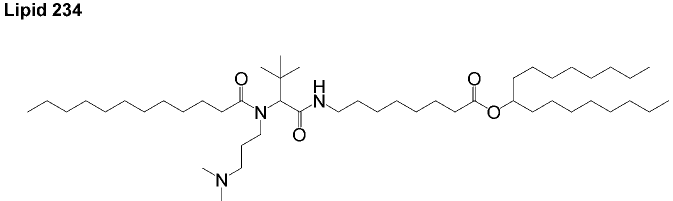

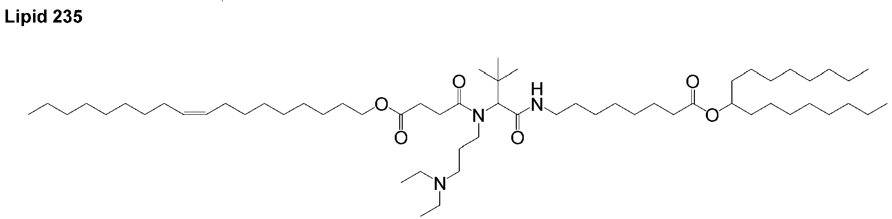

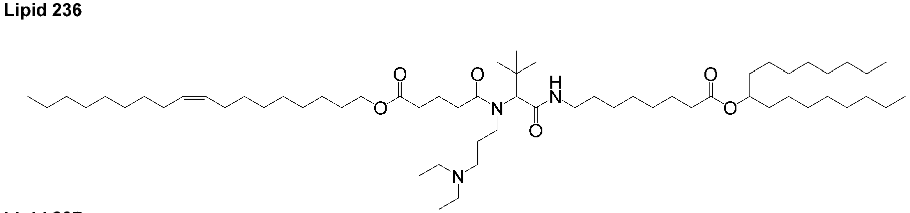

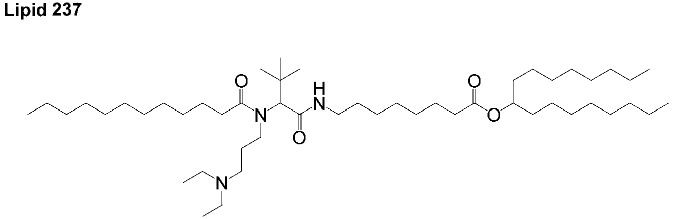

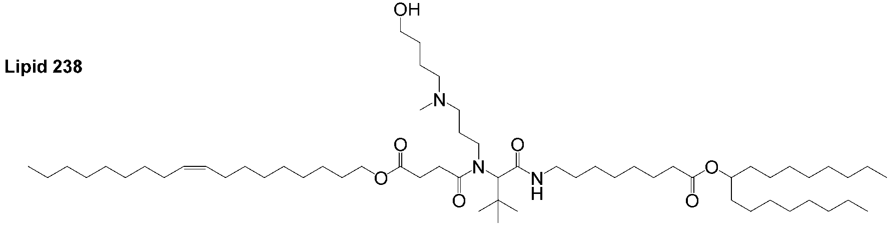

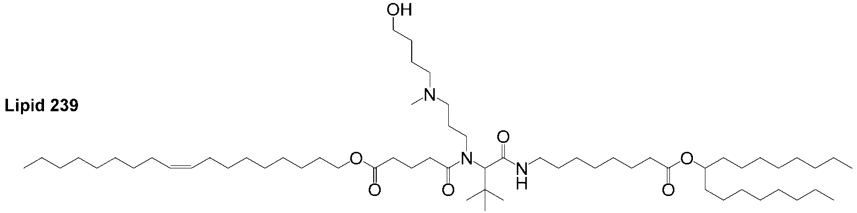

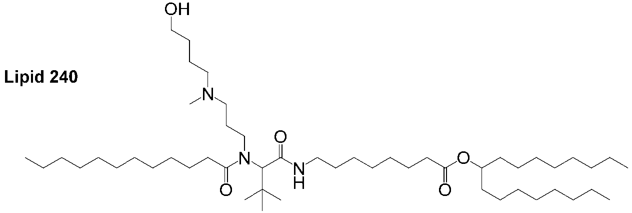

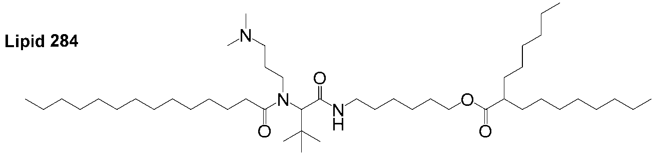

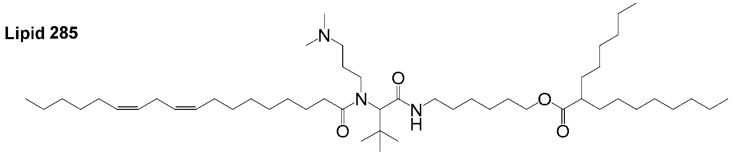

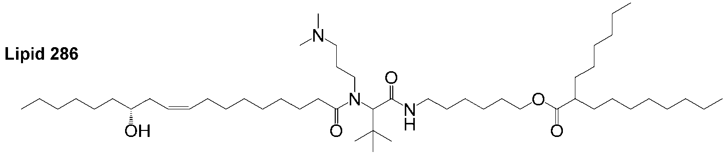

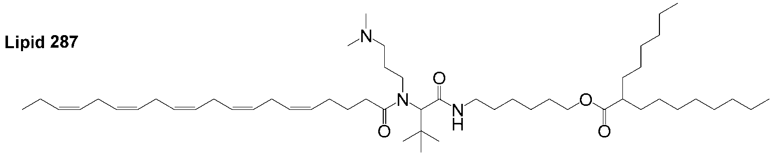

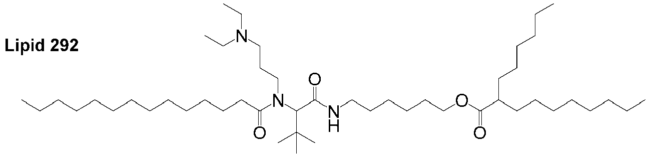

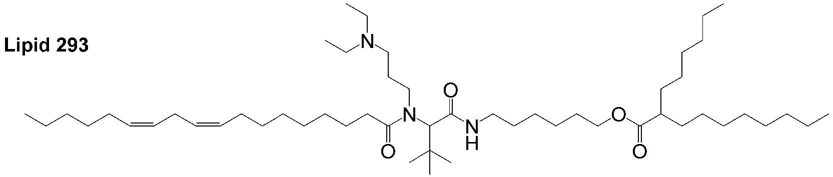

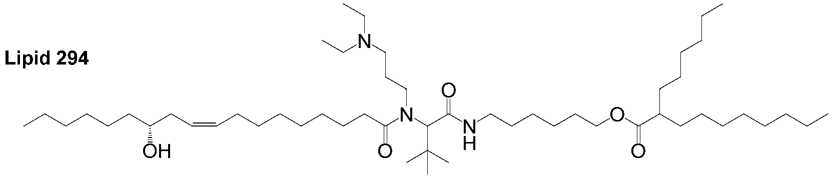

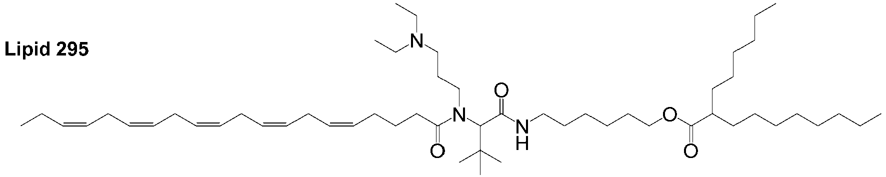

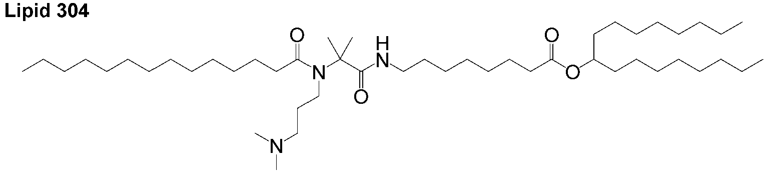

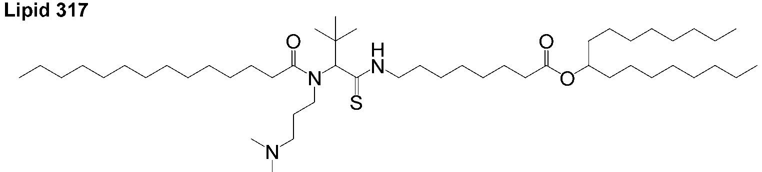

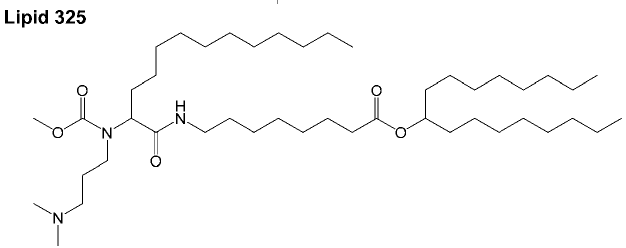

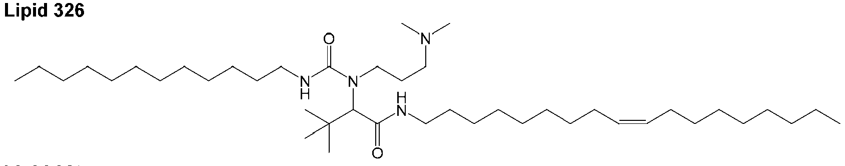

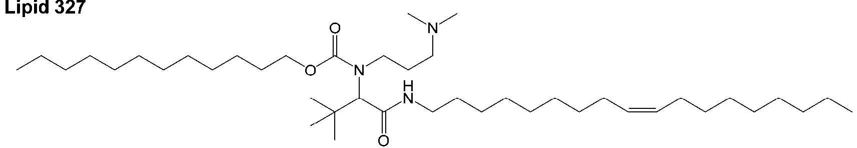

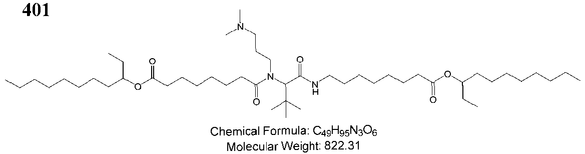

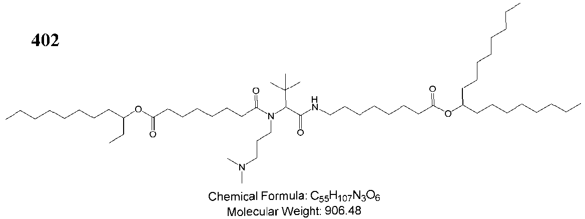

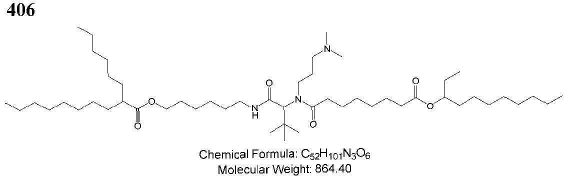

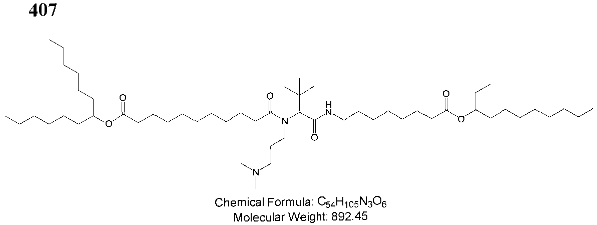

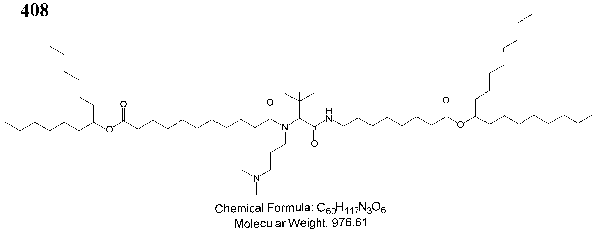

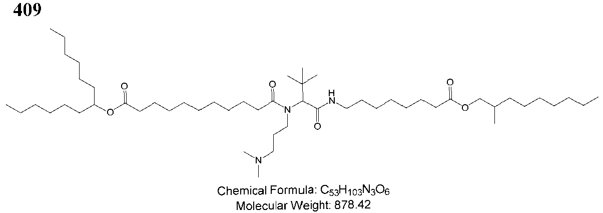

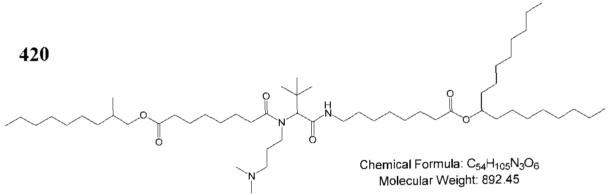

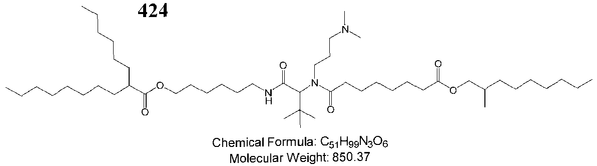

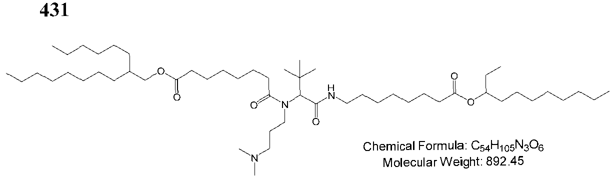

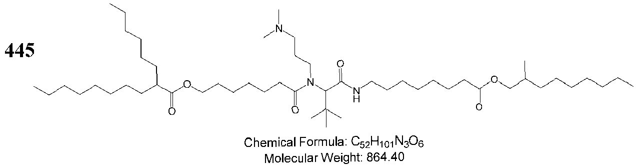

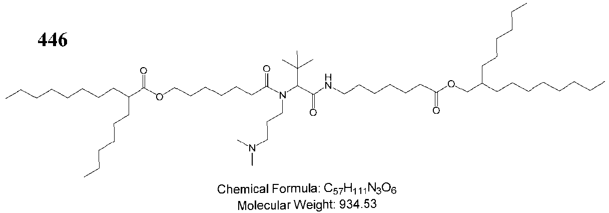

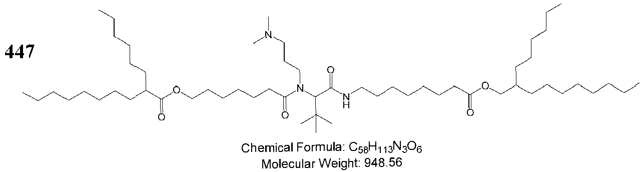

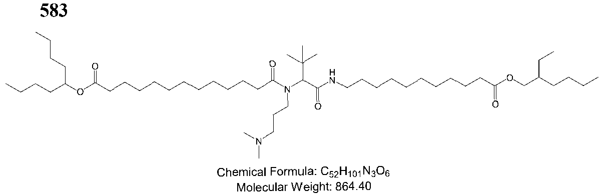

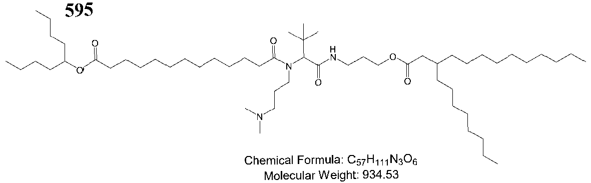

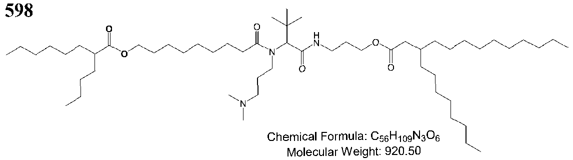

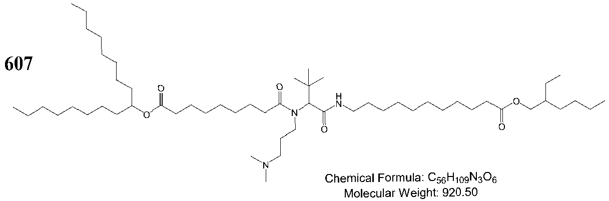

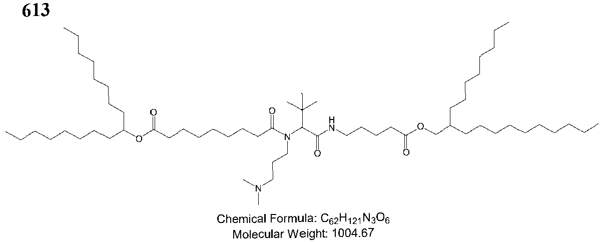

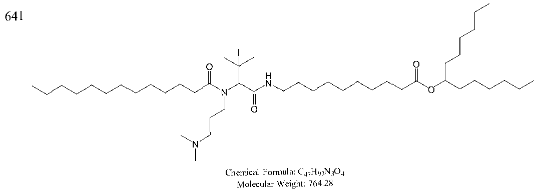

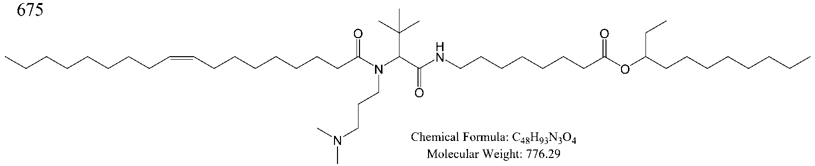

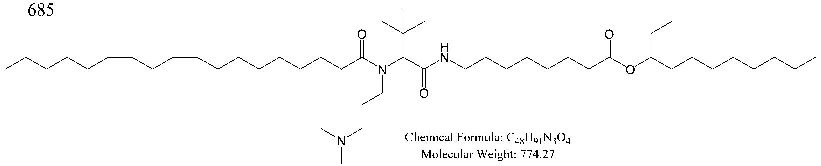

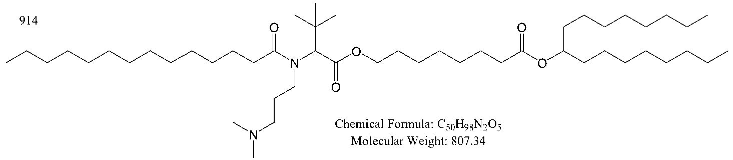

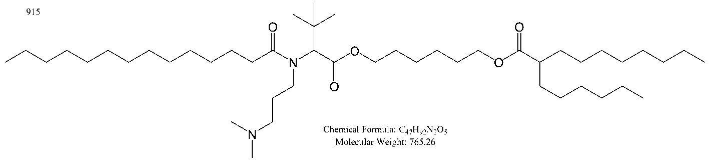

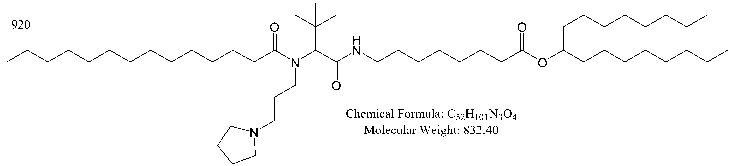

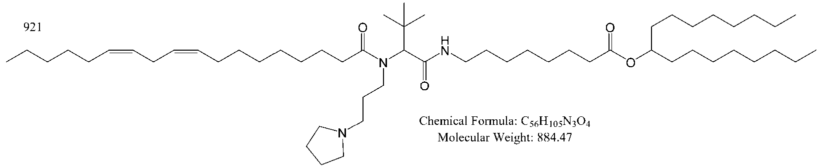

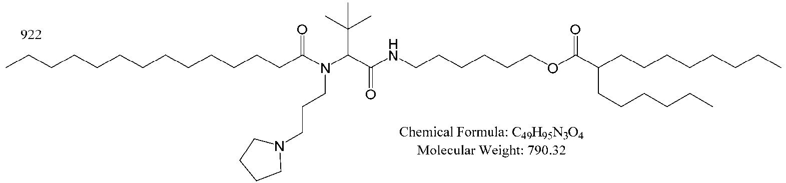

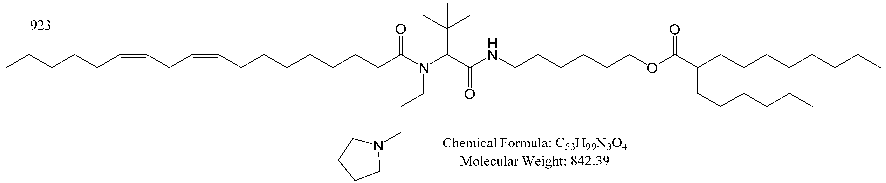

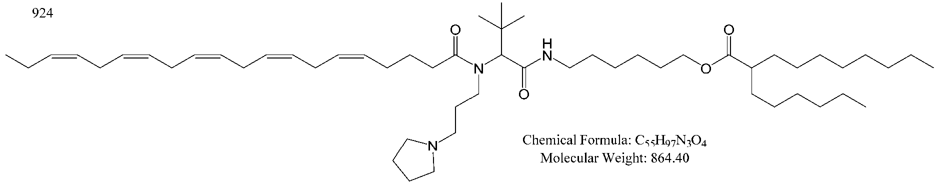

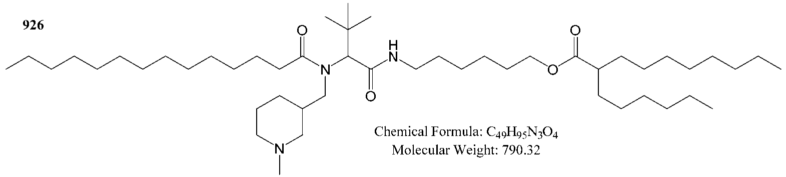

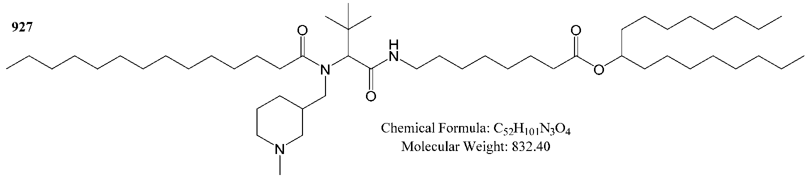

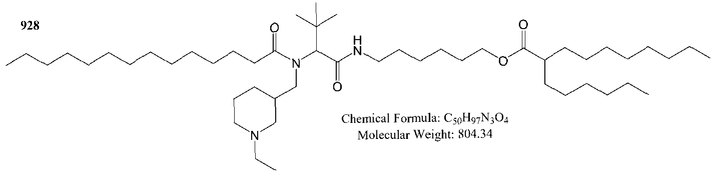

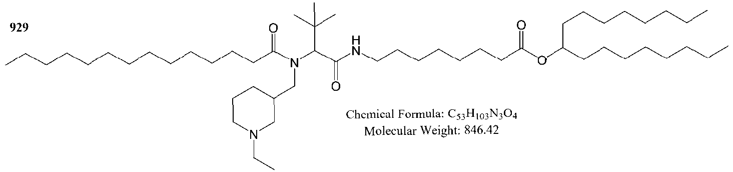

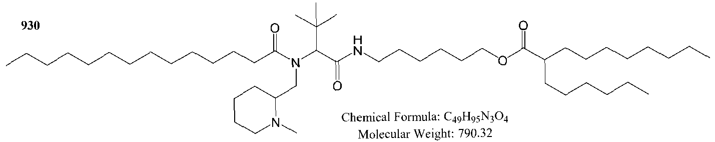

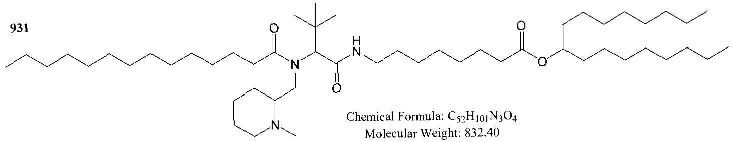

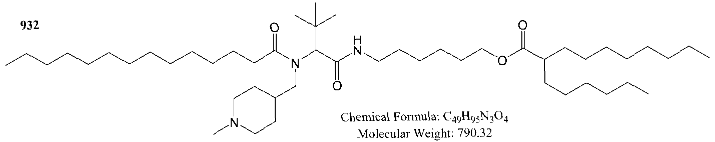

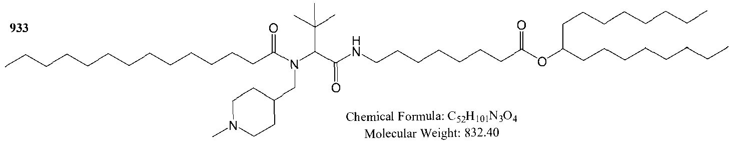

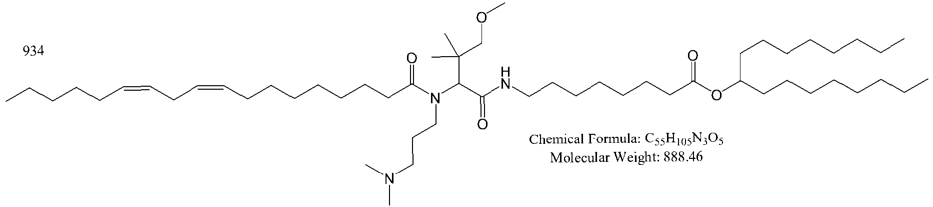

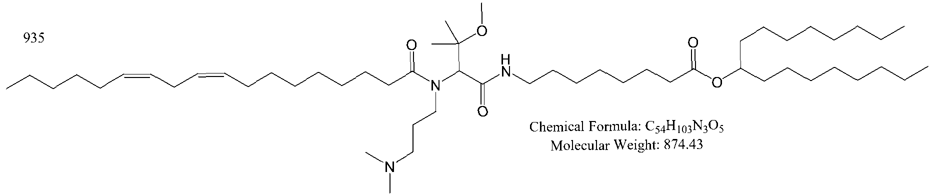

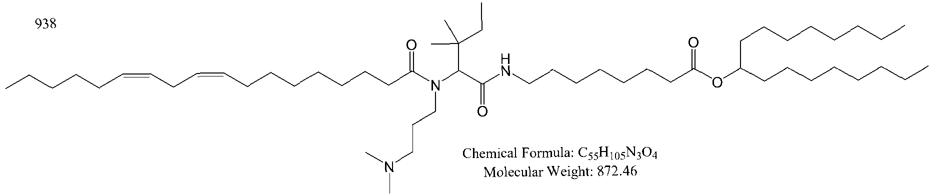

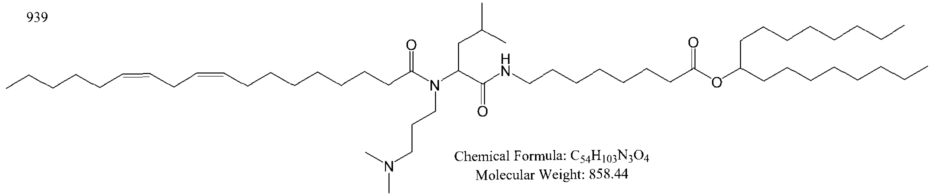

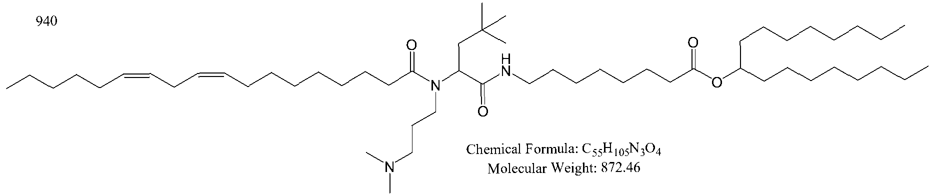

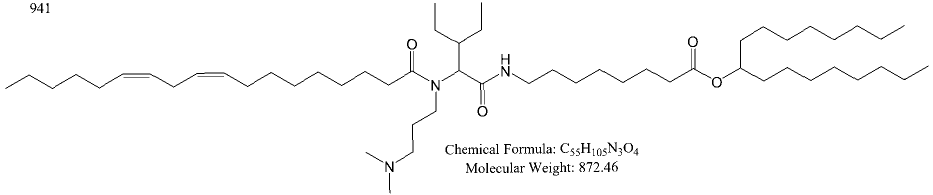

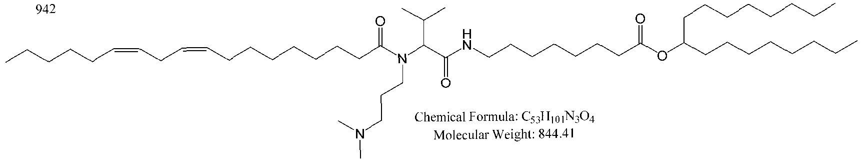

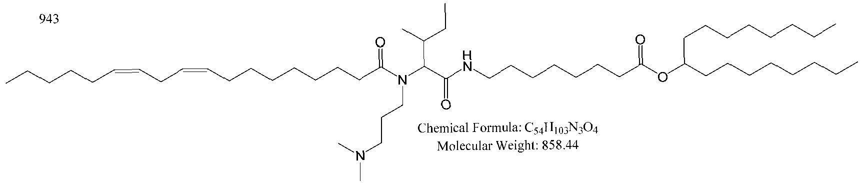

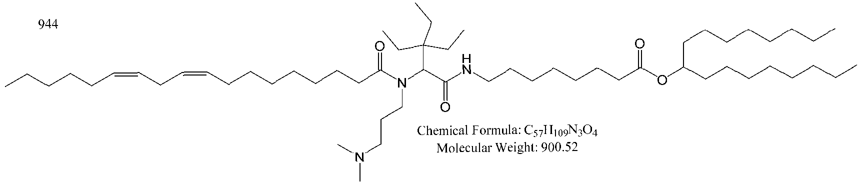

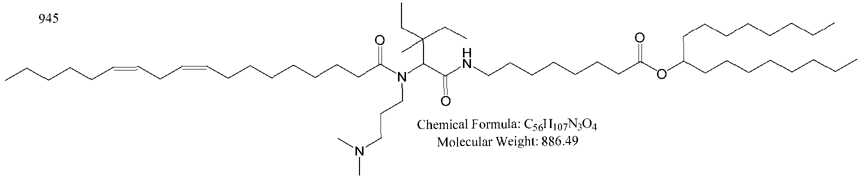

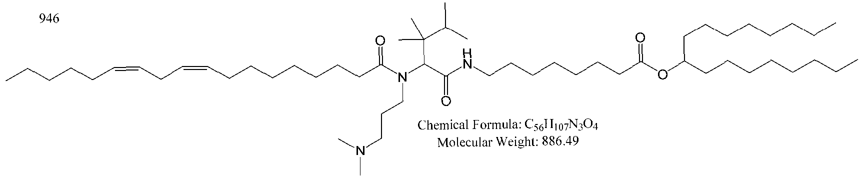

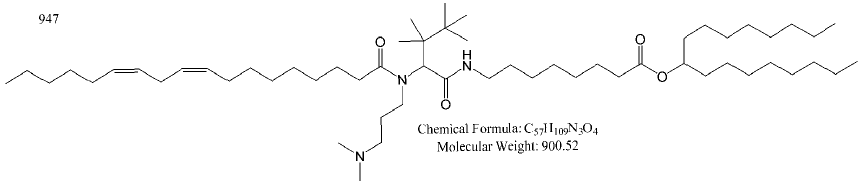

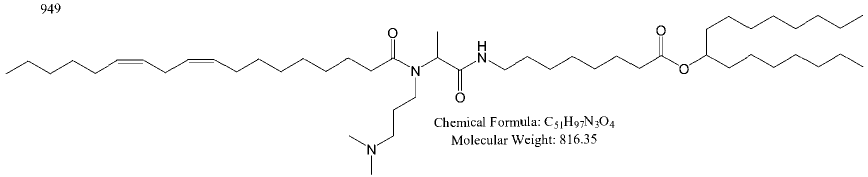

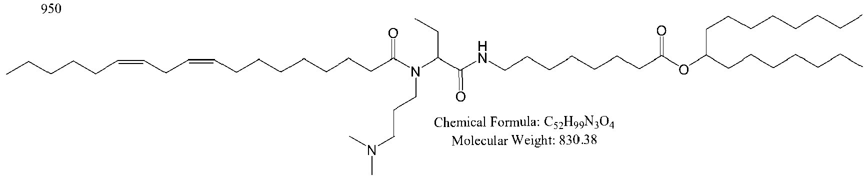

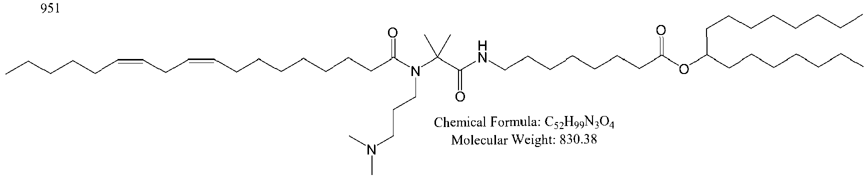

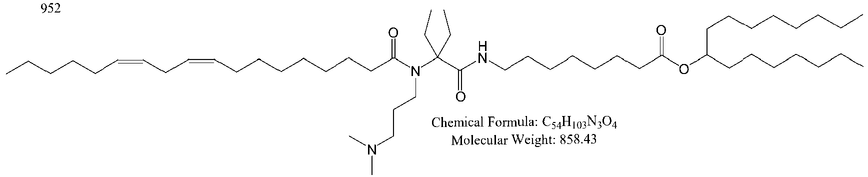

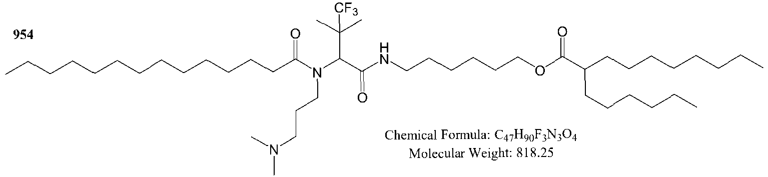

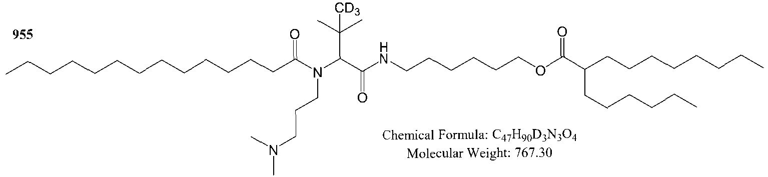

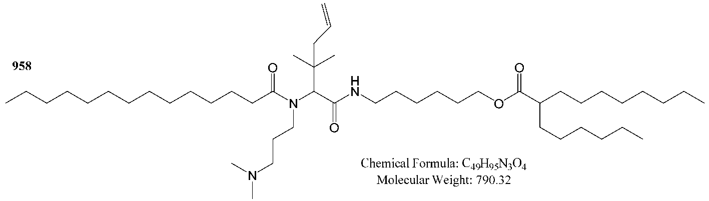

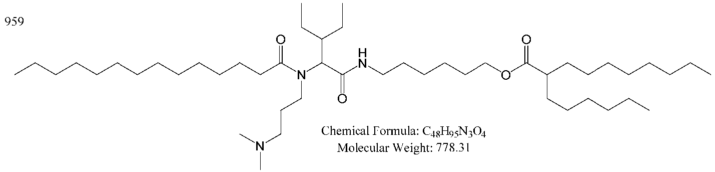

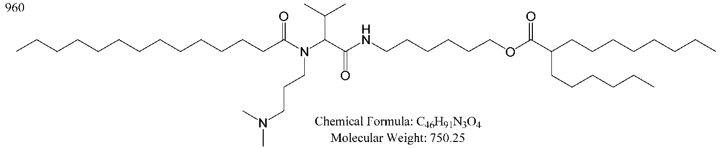

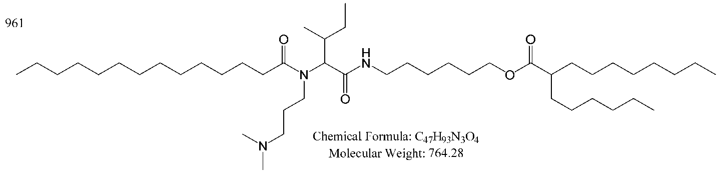

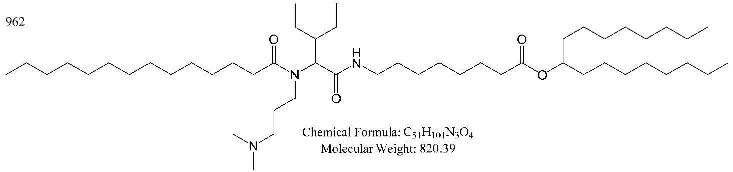

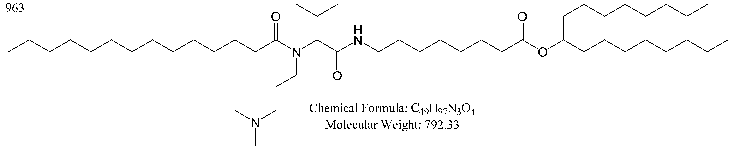

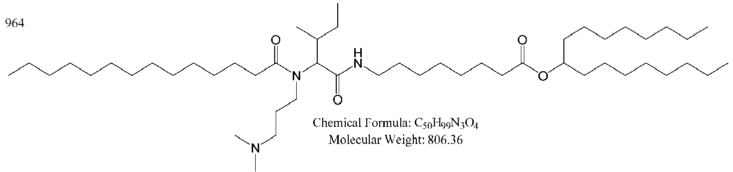

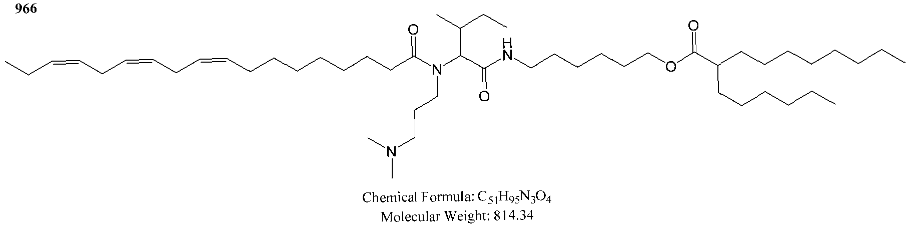

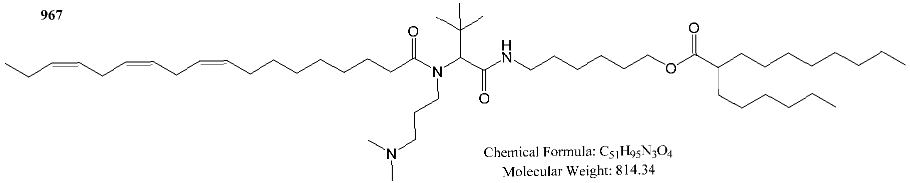

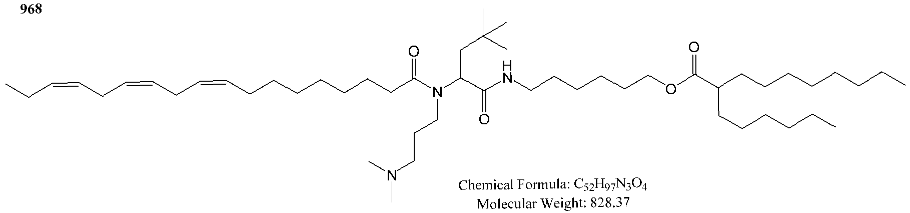

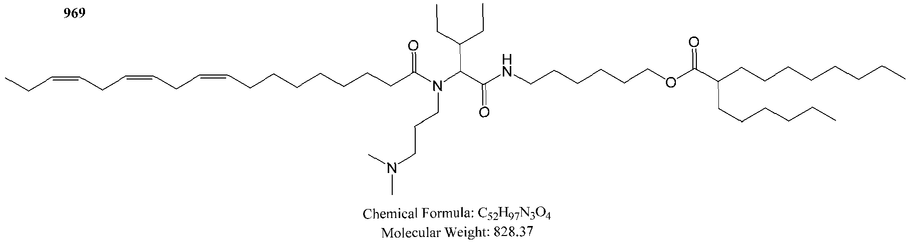

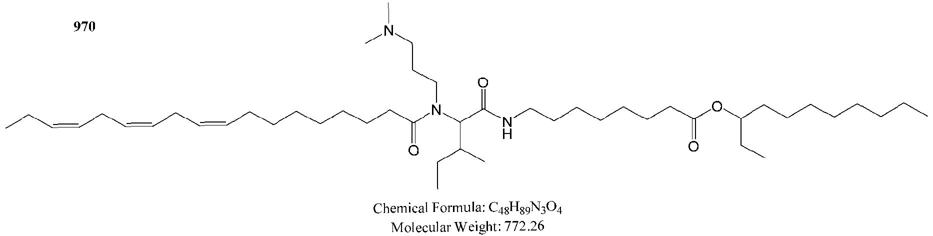

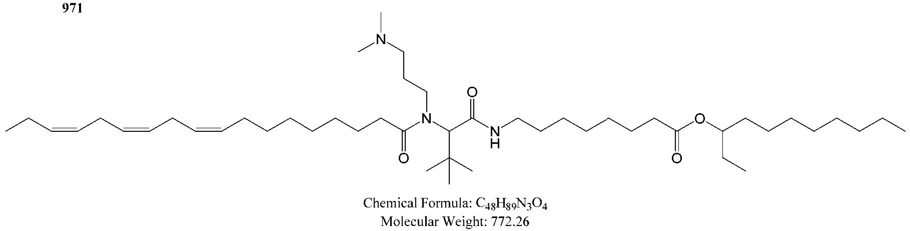

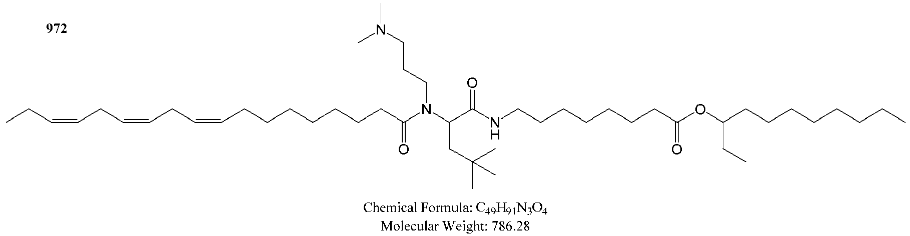

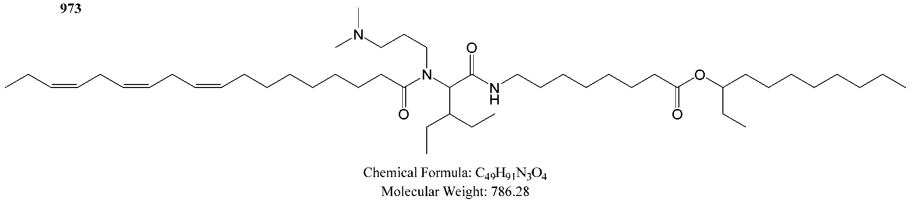

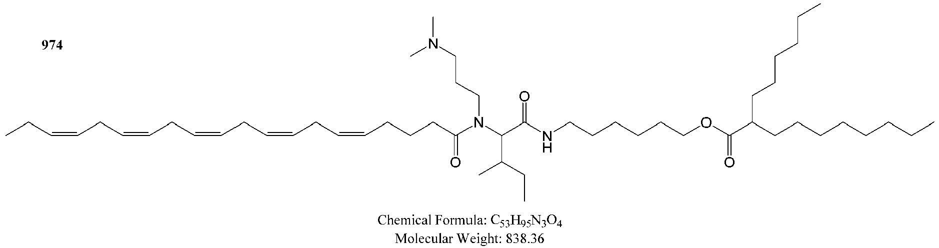

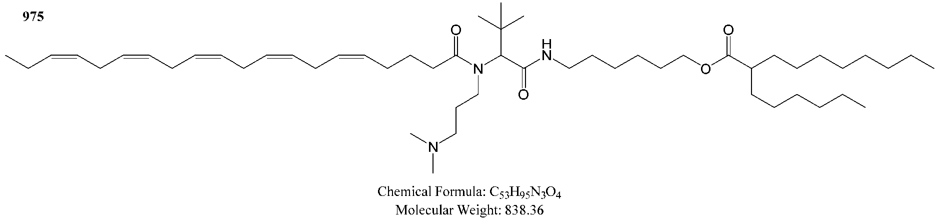

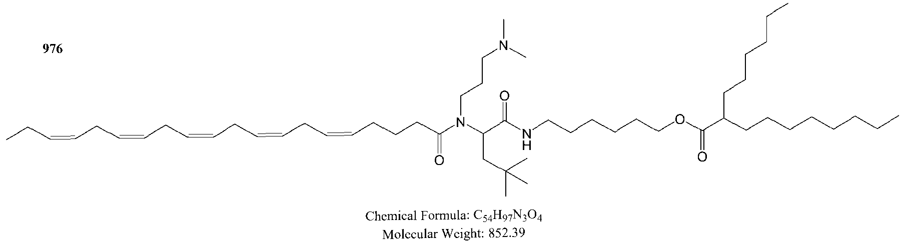

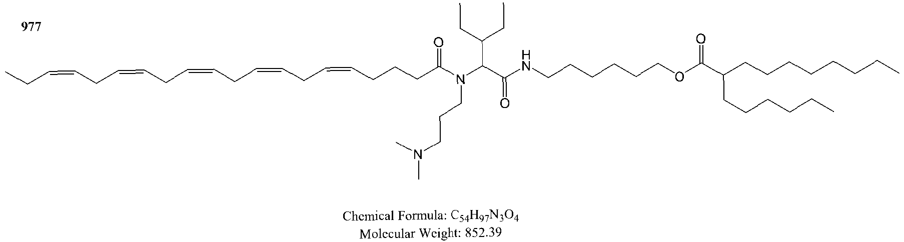

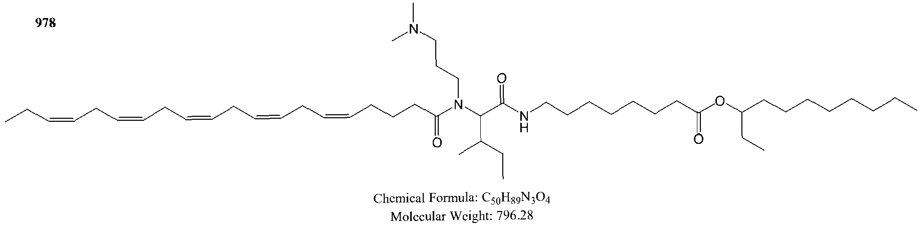

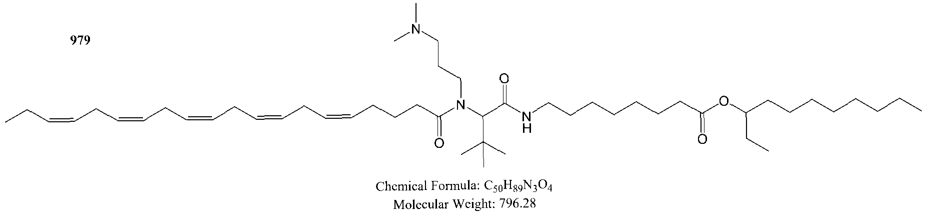

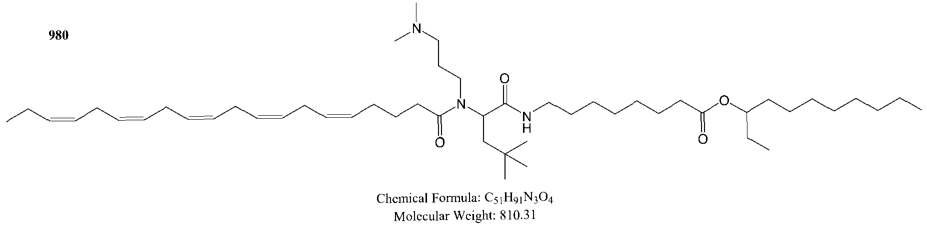

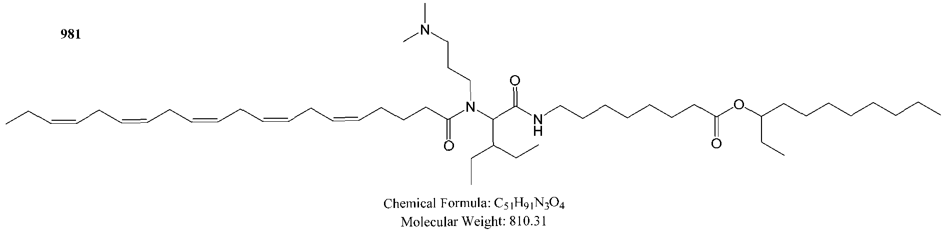

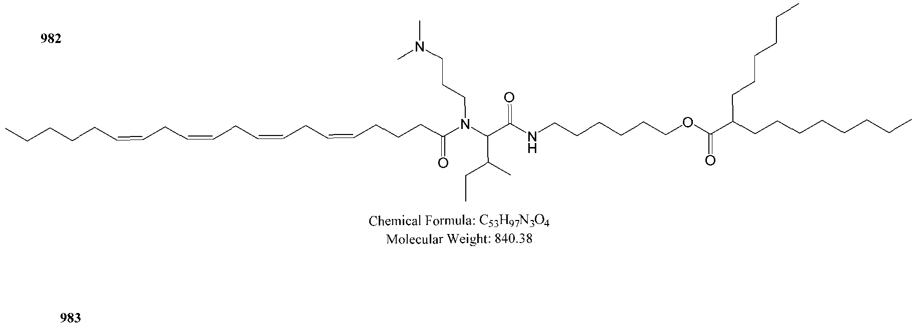

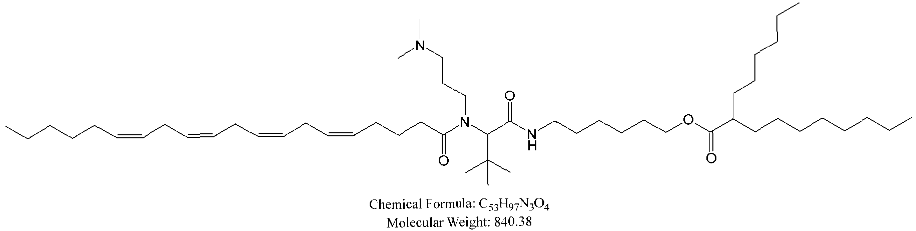

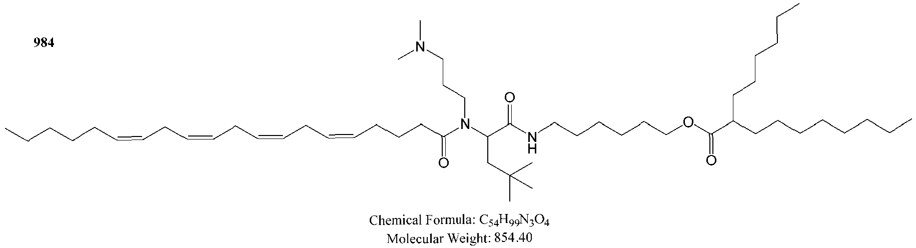

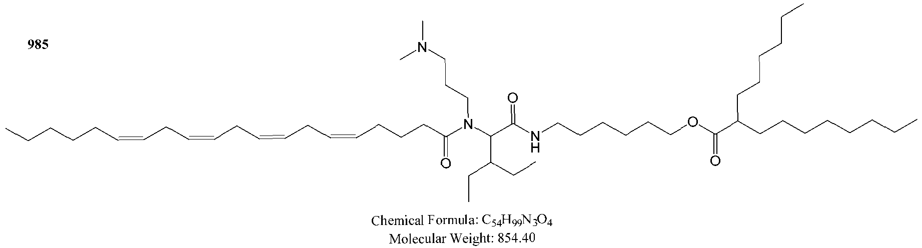

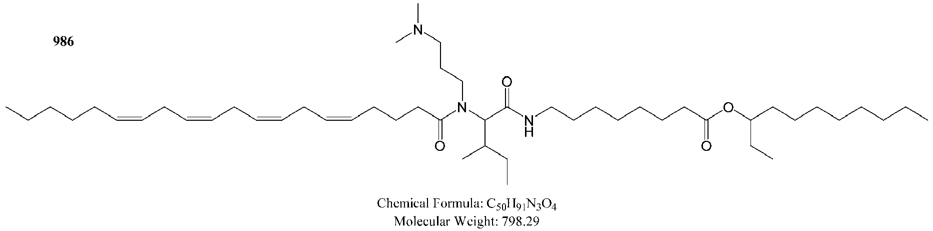

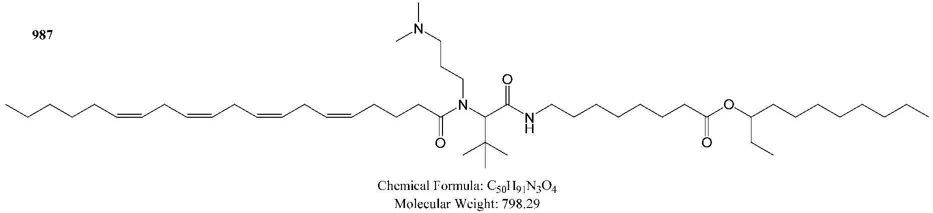

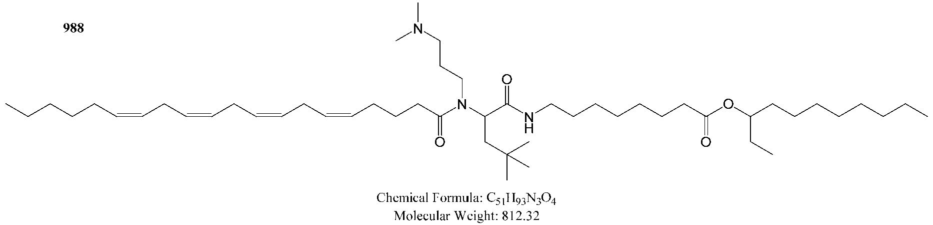

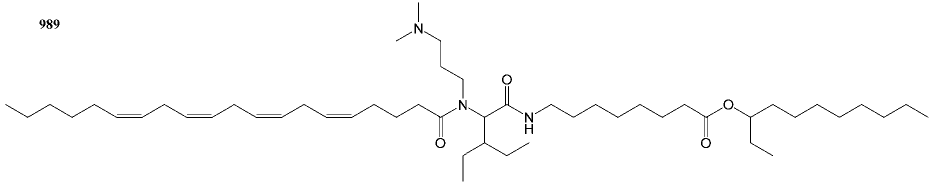

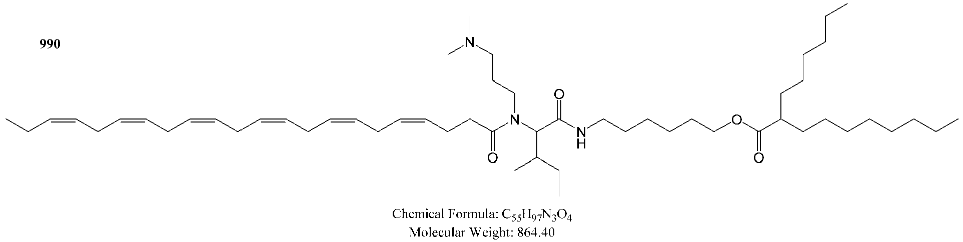

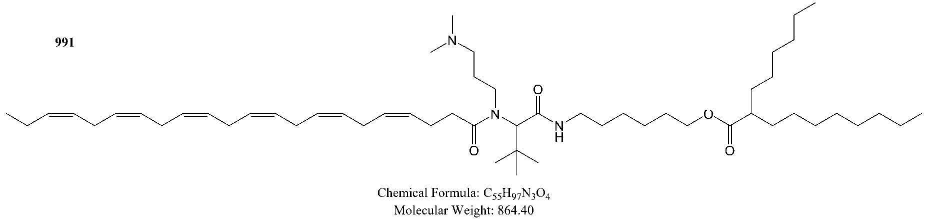

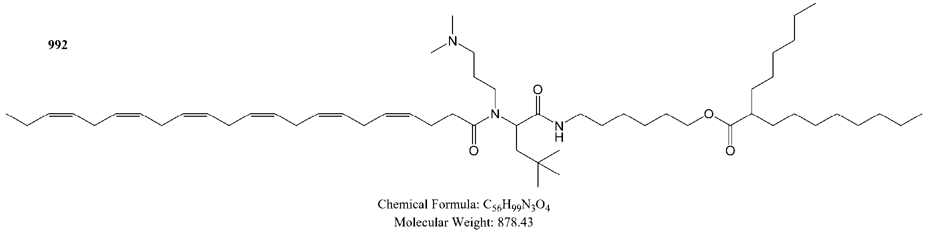

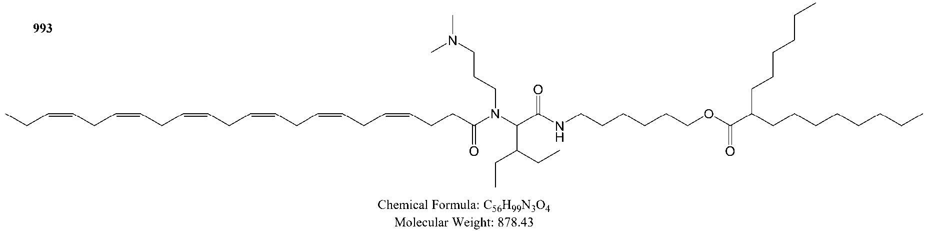

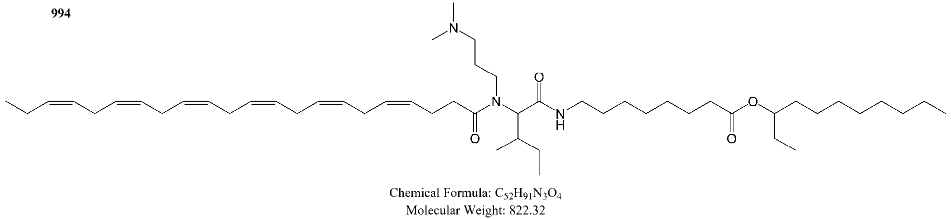

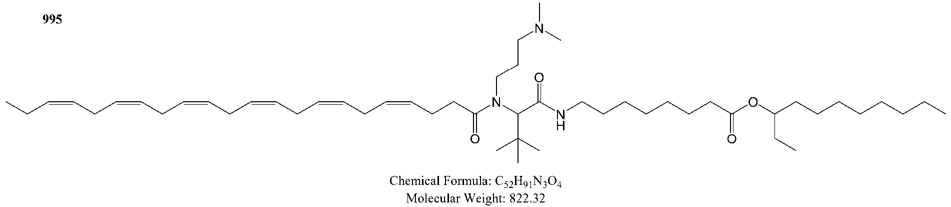

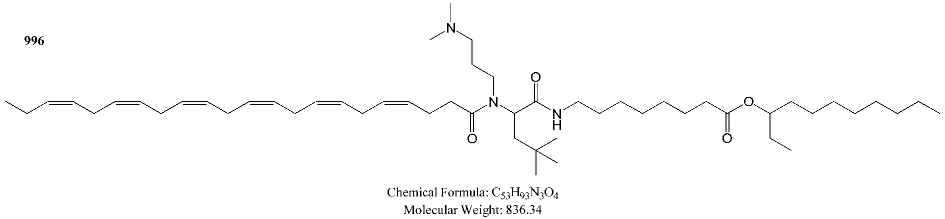

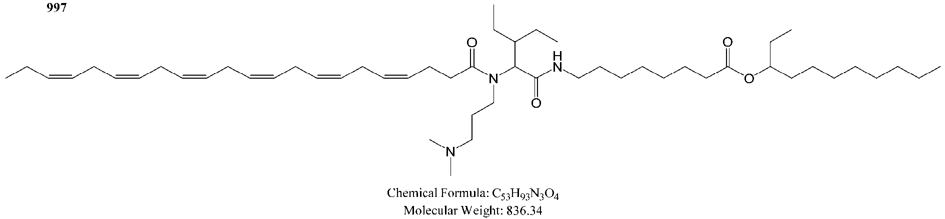

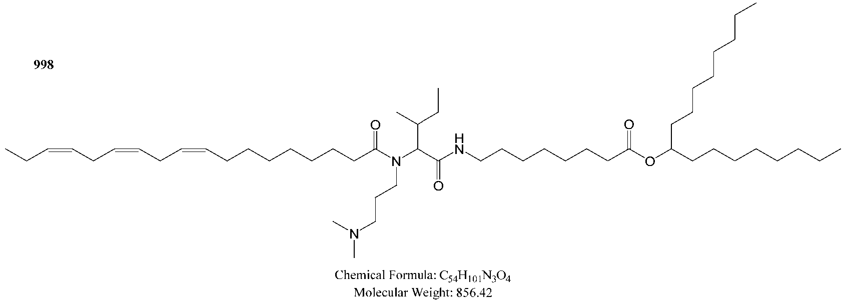

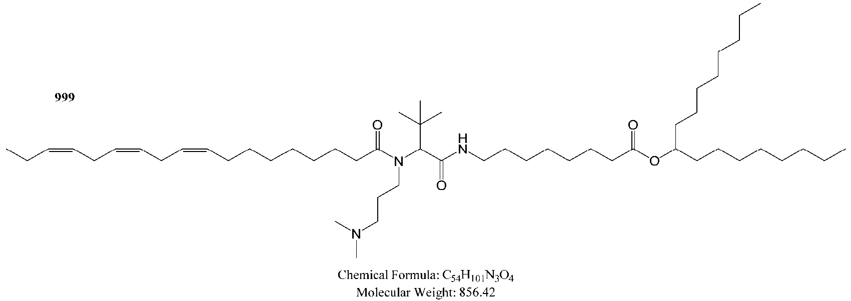

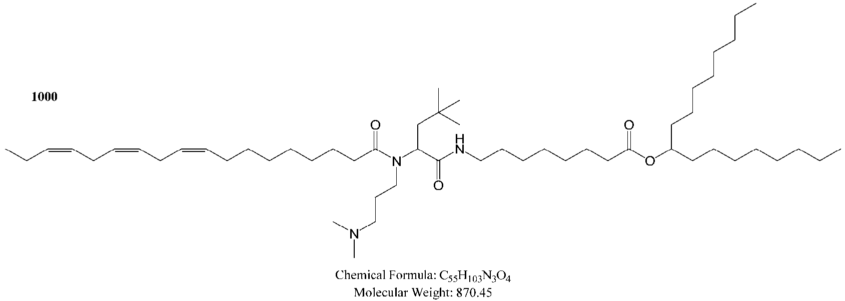

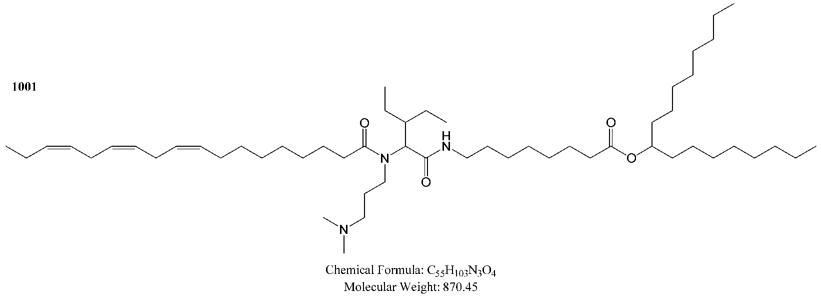

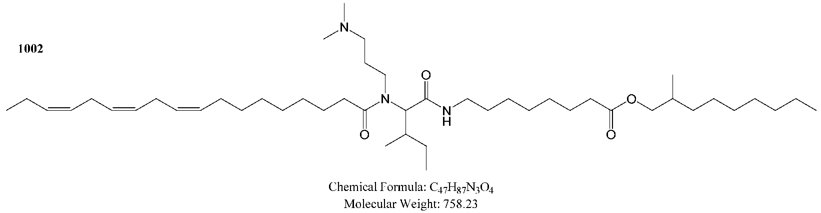

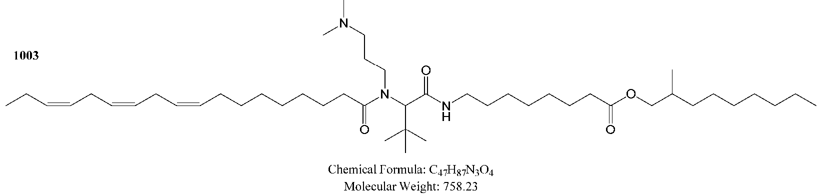

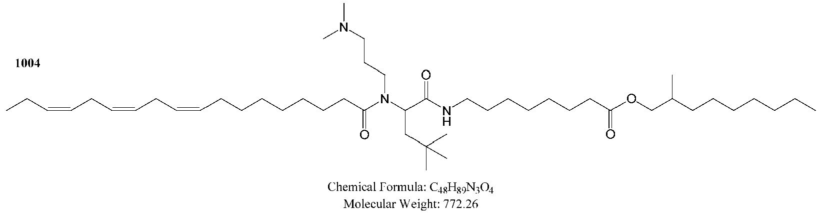

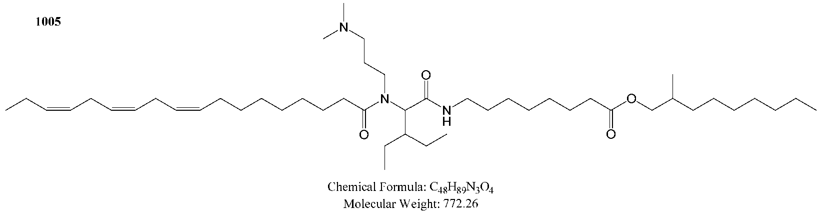

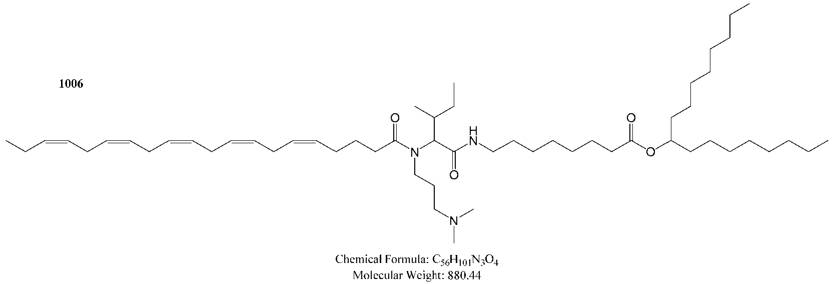

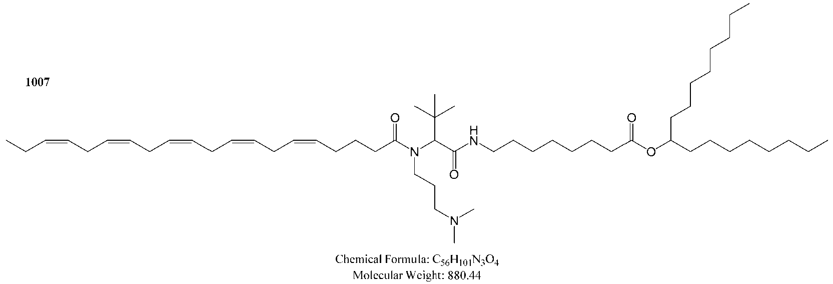

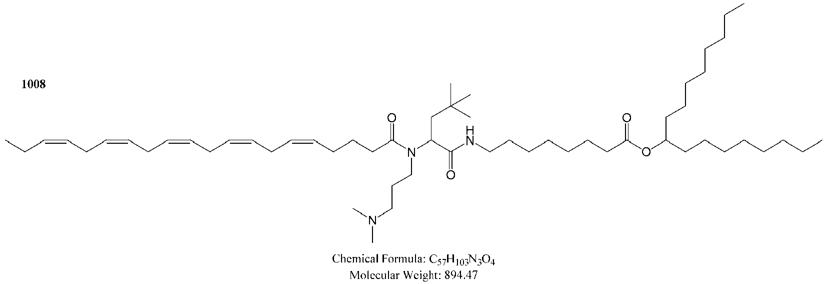

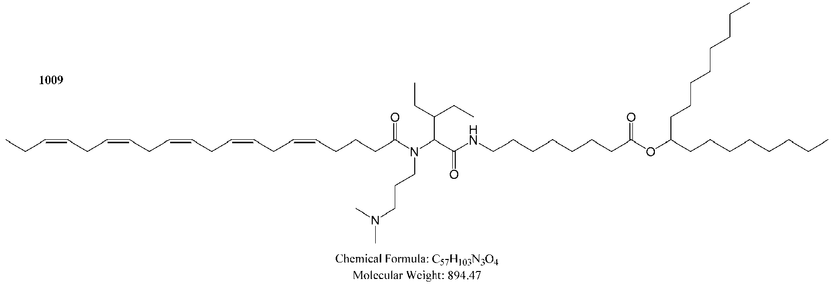

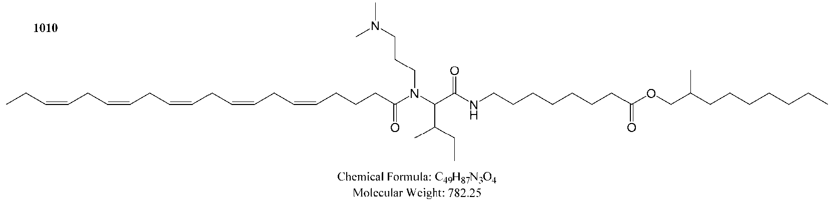

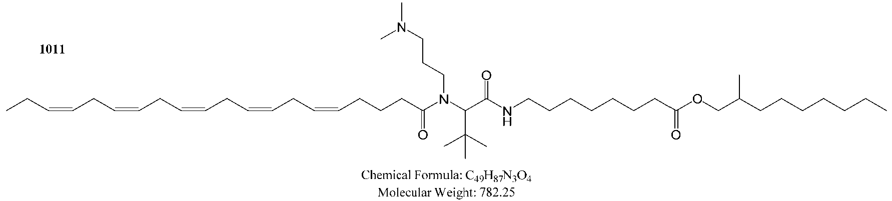

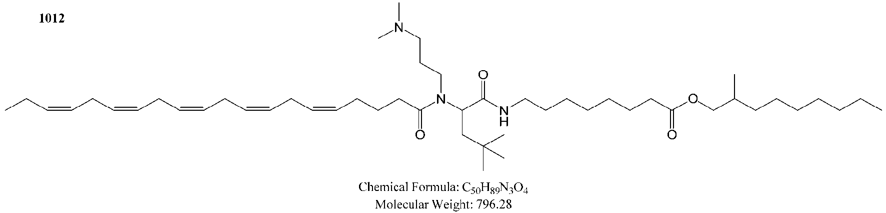

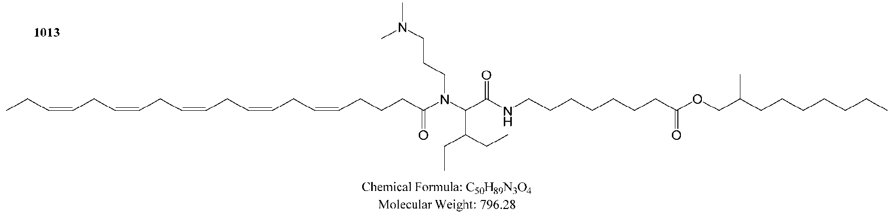

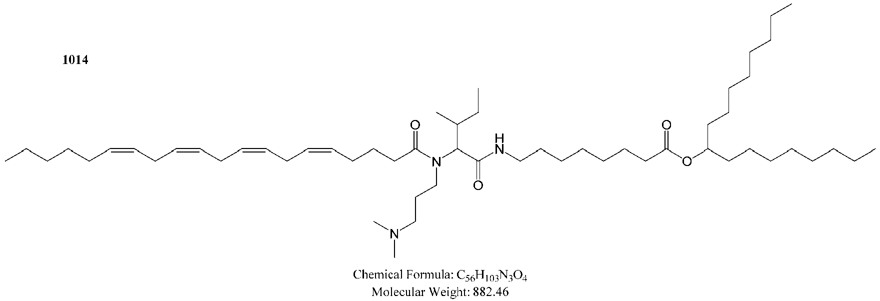

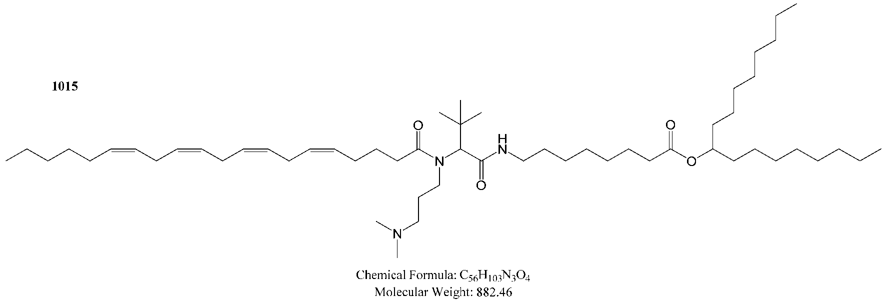

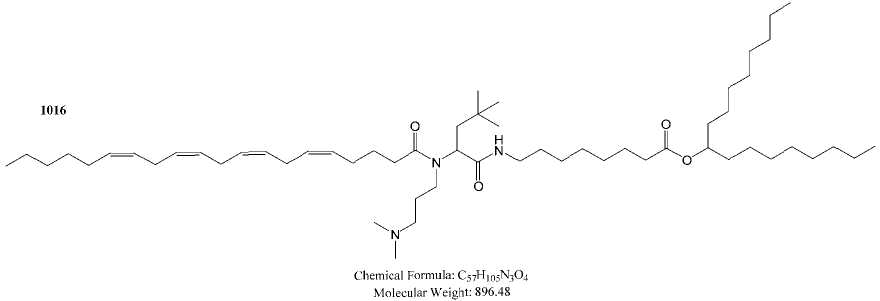

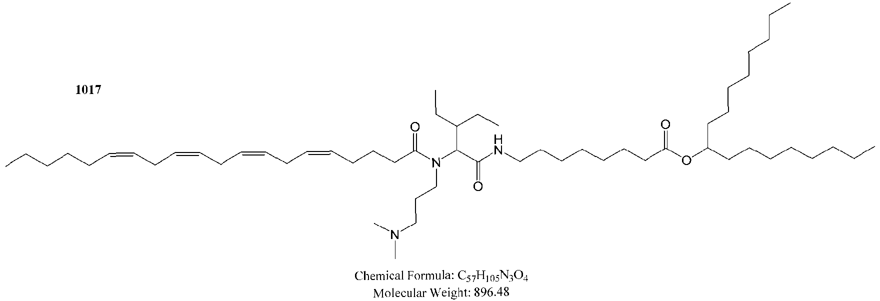

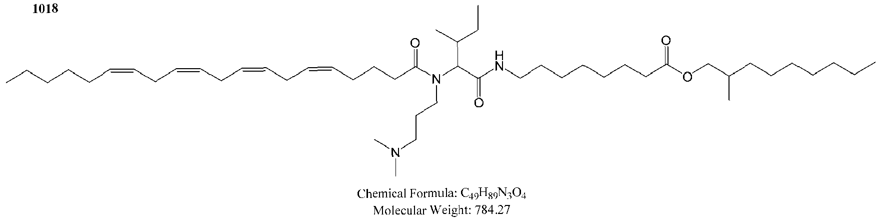

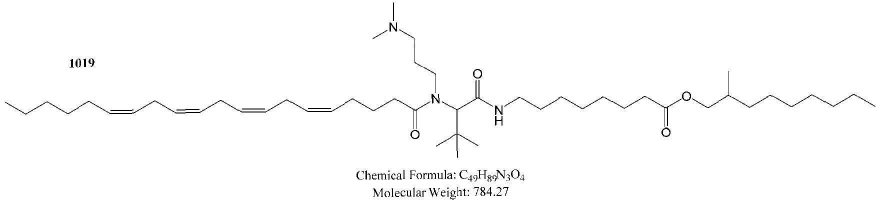

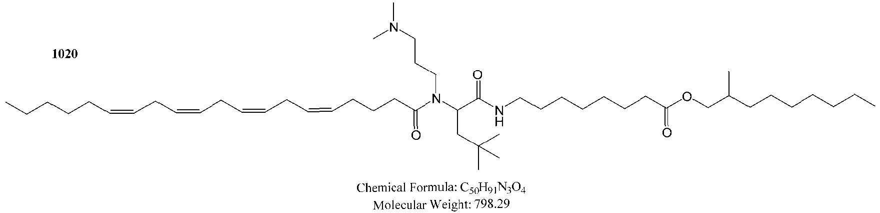

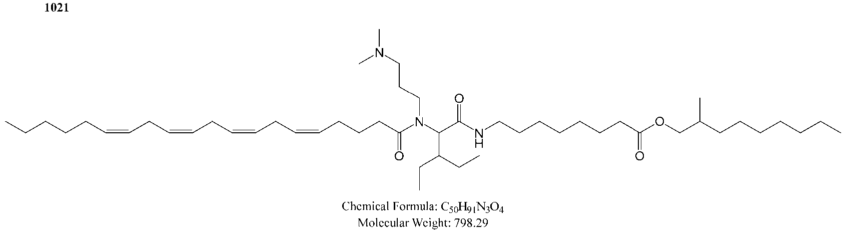

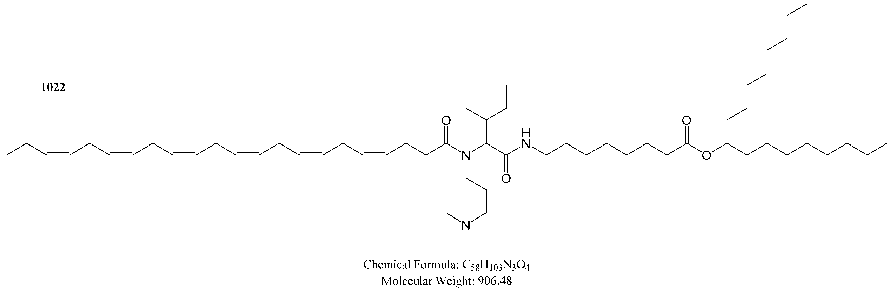

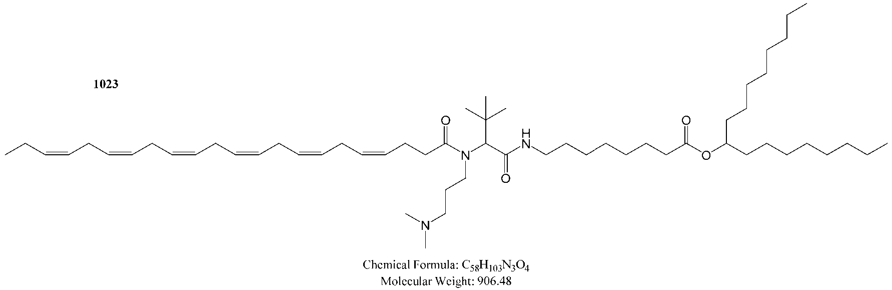

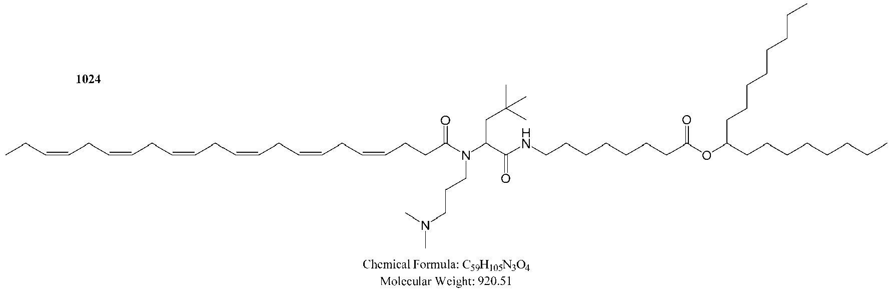

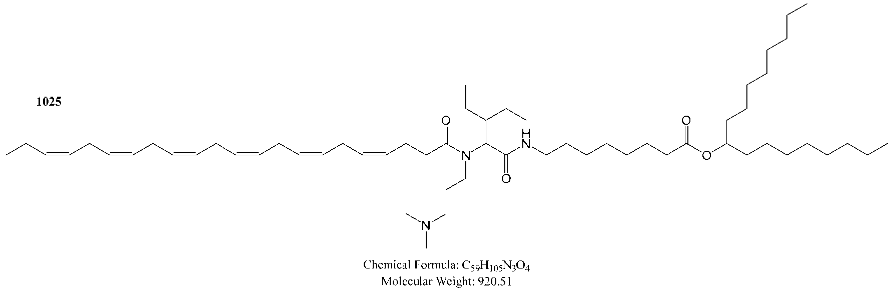

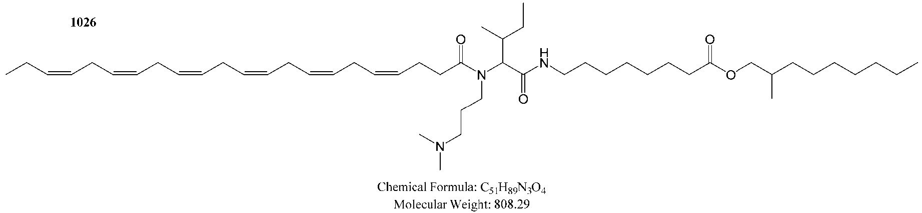

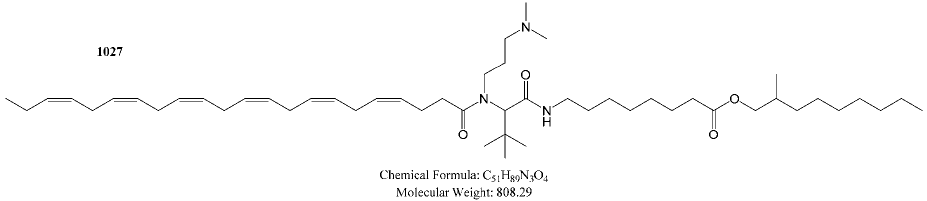

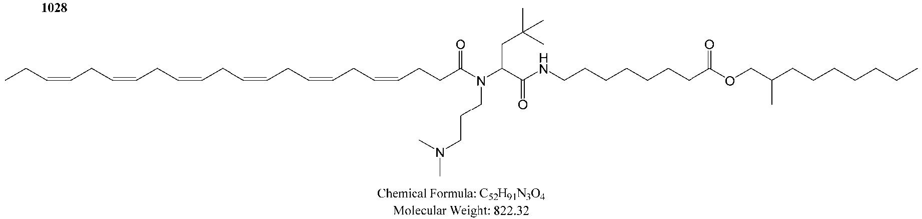

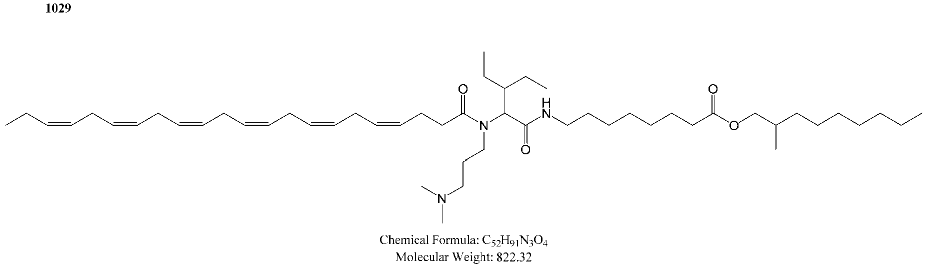

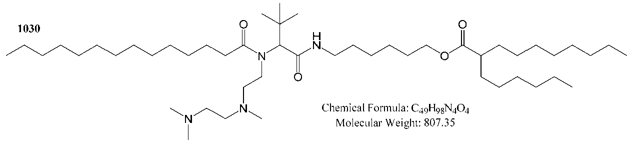

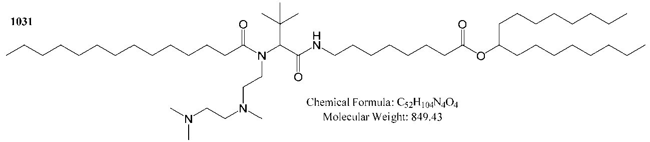

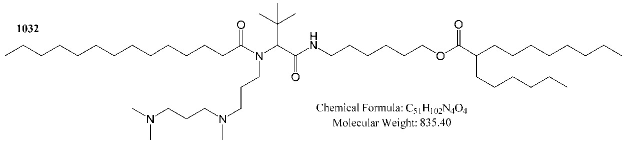

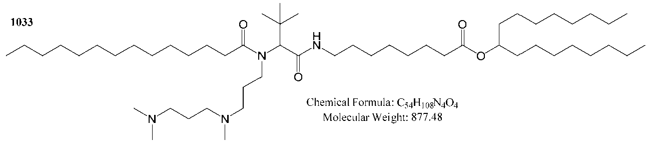

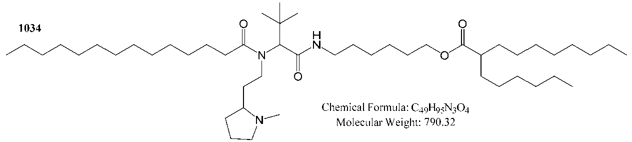

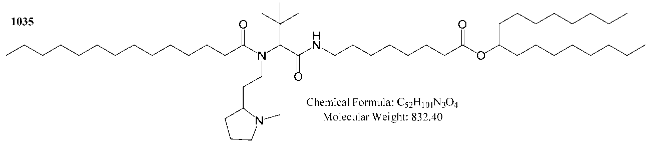

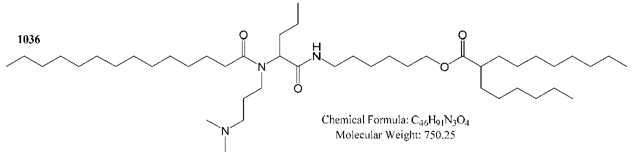

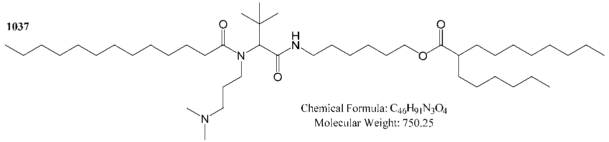

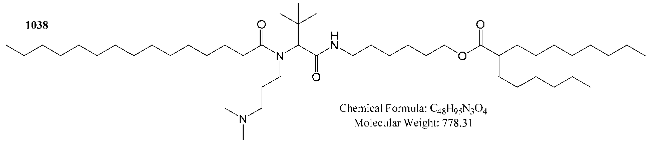

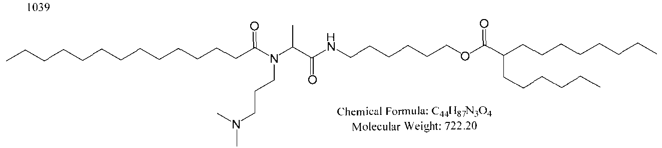

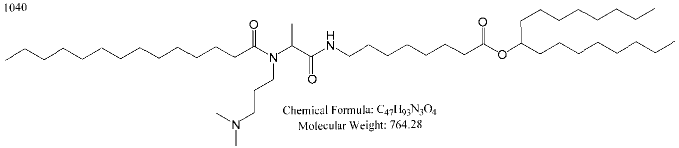

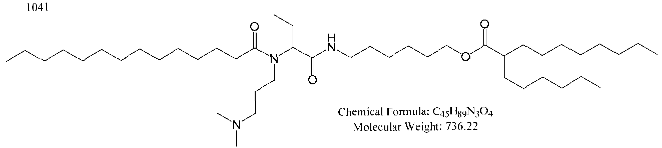

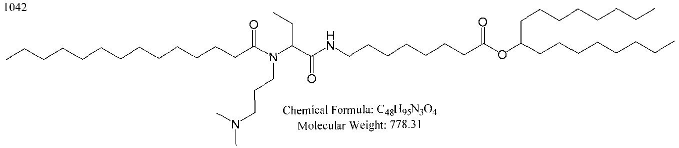

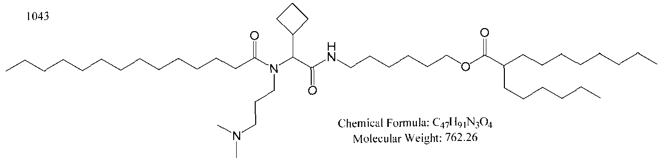

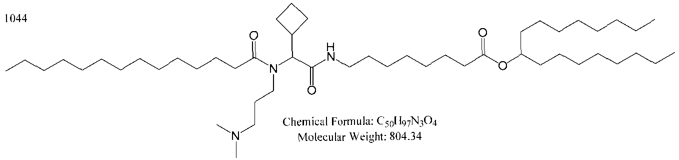

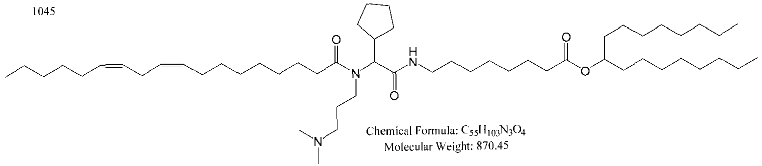

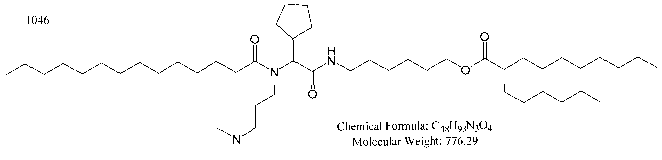

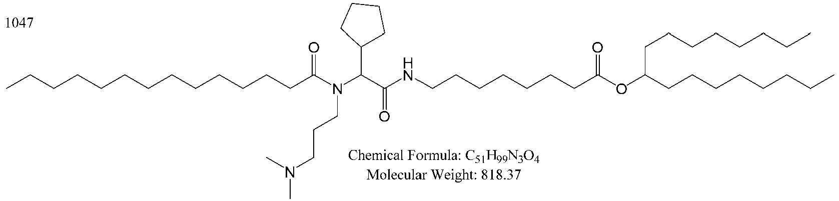

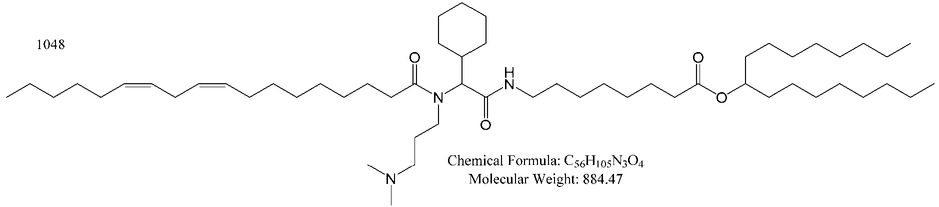

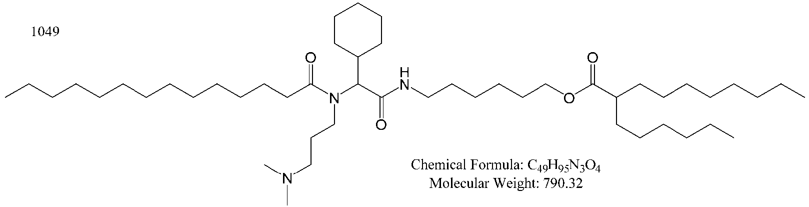

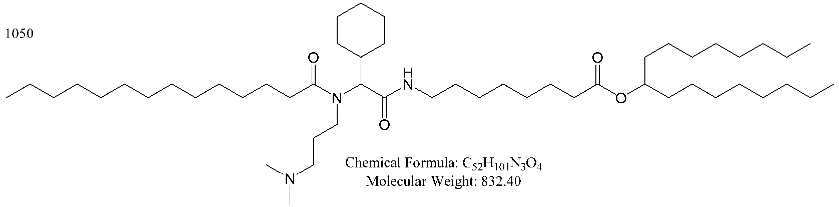

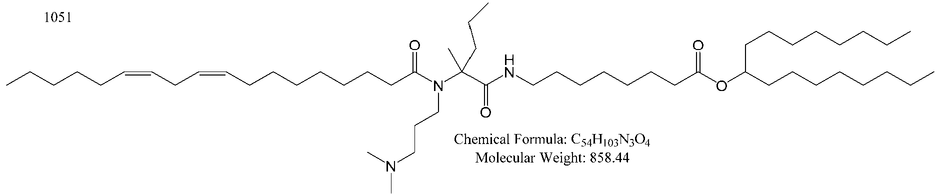

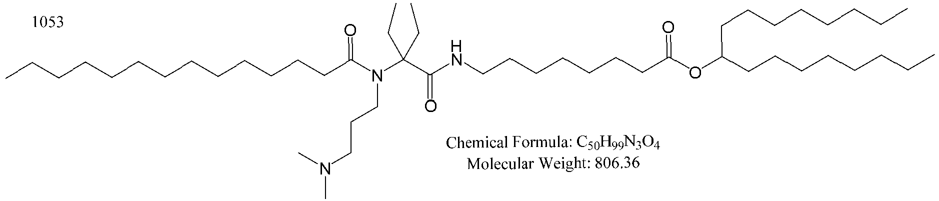

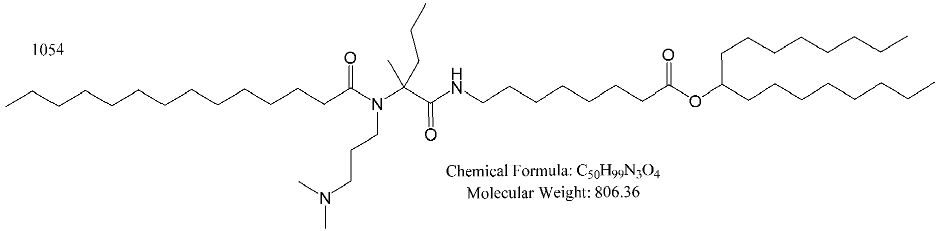

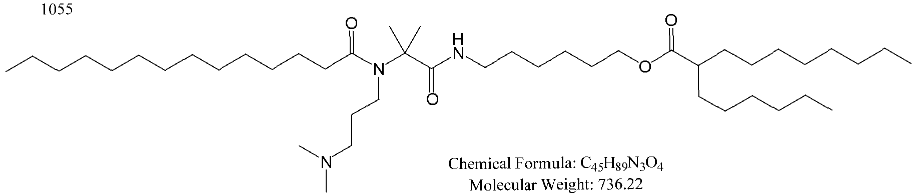

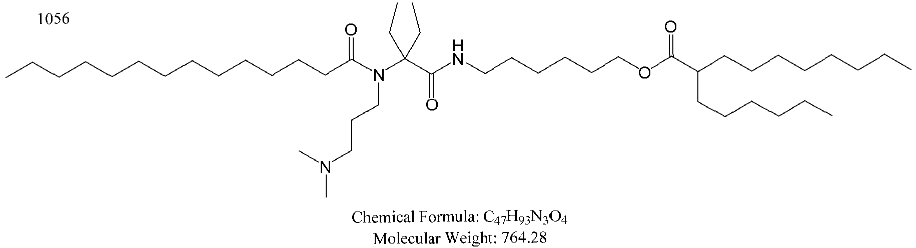

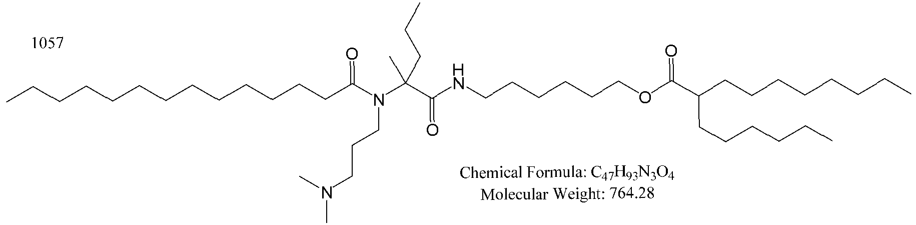

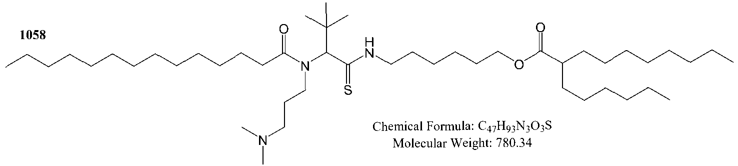

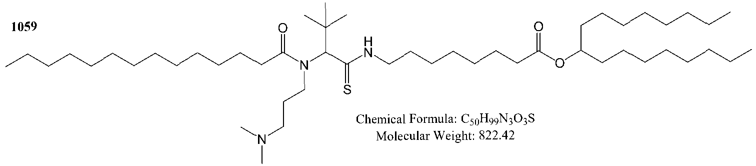

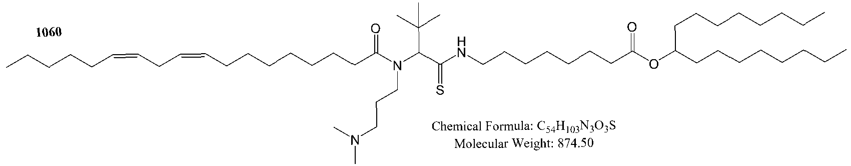

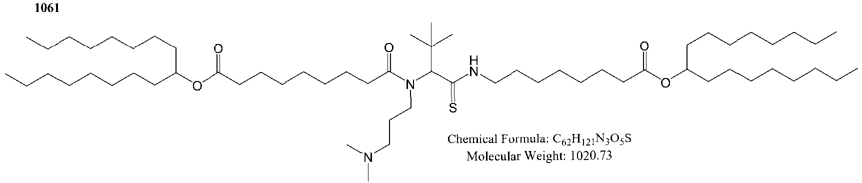

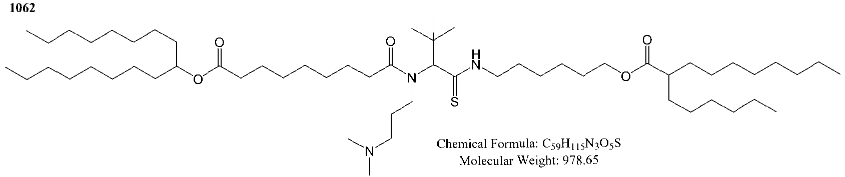

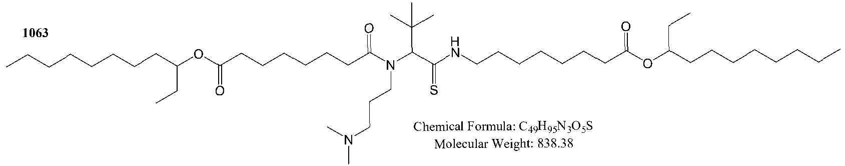

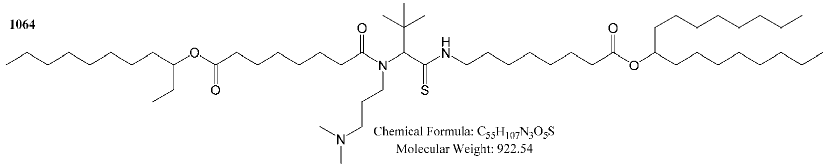

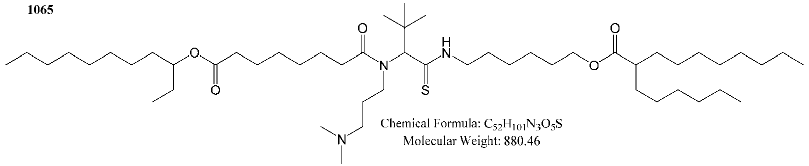

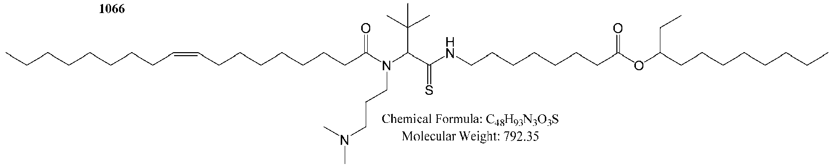

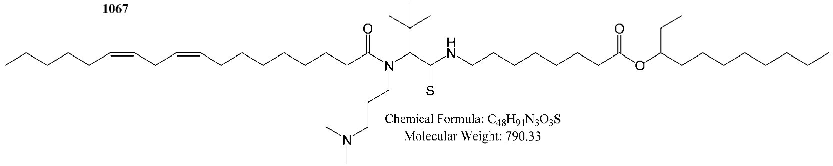

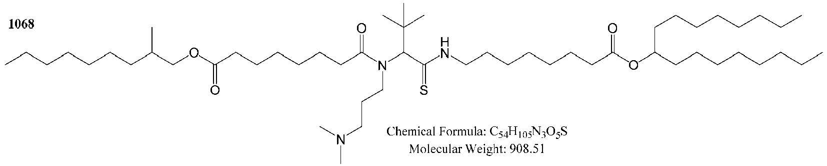

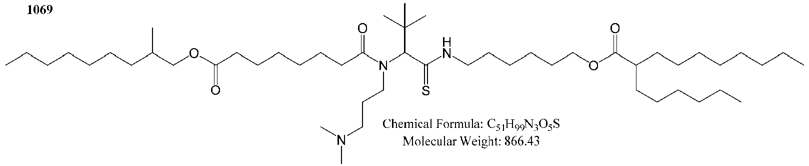

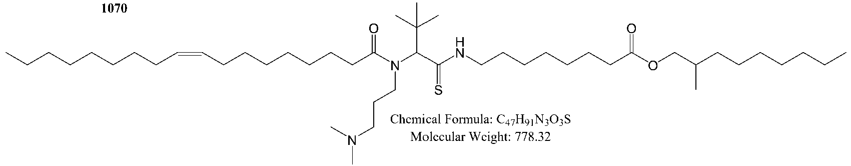

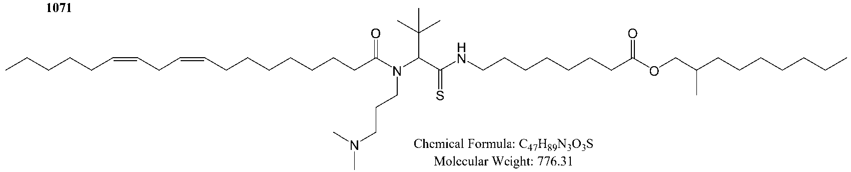

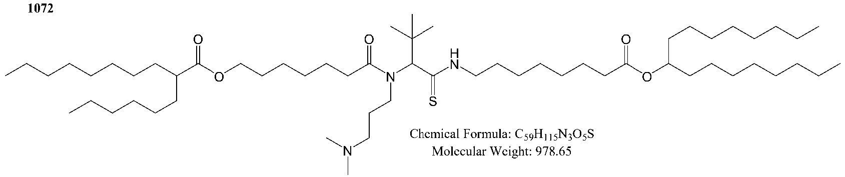

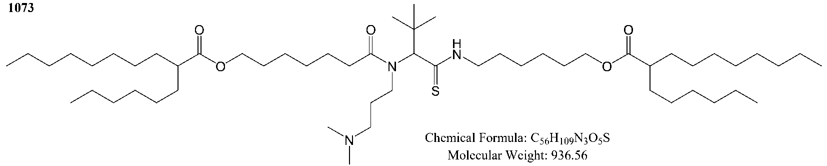

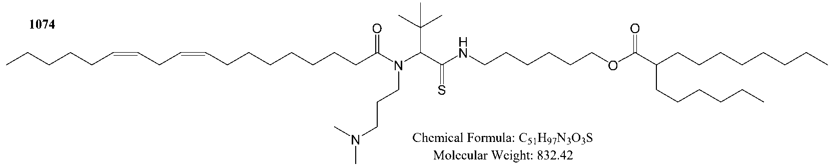

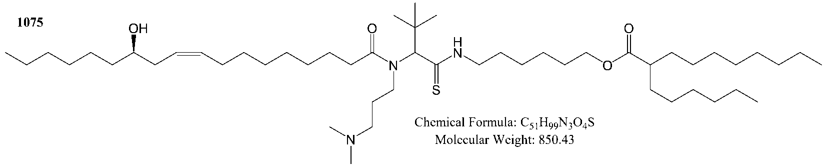

The structure information of synthesized lipids 1-1075 in this example is as follows:
Lipid 16: ¹H NMR (500 MHz, CDCl₃, ppm): δ 5.35 (m, 4H), 4.91 (s, 1H), 3.43 (s, 1H) 3.26 (t, 2H), 3.08 (t, 2H), 2.23 (m, 6H), 2.00 (s, 8H), 1.67 (t, 2H), 1.47-1.29 (m, 49H), 1.04 (s, 9H), 0.87 (t, 6H). MS: m/z 770.8 (M+H⁺). Chemical formula: C₄₇H₉₁N₃O₃
Lipid 30: ¹H NMR (500 MHz, CDCl₃, ppm): δ 5.33 (m, 2H), 4.23-3.92 (br, 1H), 3.71 (m, 1H), 3.42(m, 1H), 3.22 (m, 1H), 3.08 (t, 1H), 2.51-2.15 (m, 8H), 1.99 (m, 4H), 1.65 (m, 6H), 1.51-1.18 (m, 38H), 1.05-0.99 (m, 15H), 0.88 (m, 6H). MS: m/z 677.3 (M+H⁺). Chemical formula: C₄₃H₈₅N₃O₂
Lipid 60: ¹H NMR (500 MHz, CDCl₃, ppm): δ 5.33 (m, 2H), 4.19-3.81 (br, 1H), 3.71 (br, 1H), 3.42(br, 1H), 3.22 (br, 1H), 3.06 (br, 1H), 2.64 (m, 2H), 2.30 (m, 10H), 2.26 (m, 1H), 2.14 (t, 3H), 2.00 (m, 4H), 1.51-1.18 (m, 52H), 0.87 (t, 6H). MS: m/z 715.8 (M+H⁺). Chemical formula: C₄₅H₈₆N₄O₂
Lipid 108: ¹H NMR (500 MHz, CDCl₃, ppm): δ 5.35 (m, 6H), 4.18 (br, 1H), 4.04 (m, 2H), 3.76 (br, 1H), 3.40 (br, 1H), 3.24 (br, 1H), 3.08 (br, 1H), 2.77 (m, 2H), 2.69 (m, 4H), 2.32 (s, 2H), 2.23 (m, 6H), 2.00 (s, 8H), 1.71 (br, 2H), 1.67 (t, 2H), 1.51-1.18 (m, 45H), 0.87 (m, 12H). MS: m/z 828.9 (M+H⁺). Chemical formula: C₅₂H₉₇N₃O₄
Lipid 113: ¹H NMR (500 MHz, CDCl₃, ppm): δ 5.35 (m, 2H), 4.18 (br, 1H), 4.04 (m, 2H), 3.76 (br, 1H), 3.40 (br, 1H), 3.24 (br, 1H), 3.08 (br, 1H), 3.01 (m, 4H), 2.69 (m, 4H), 2.32 (s, 2H), 2.00 (s, 4H), 1.71 (br, 2H), 1.67 (t, 2H), 1.51-1.18 (m, 45H), 1.15 (t, 6H), 0.87 (m, 12H). MS: m/z 762.9 (M+H⁺). Chemical formula: C₄₇H₉₁N₃O₄
Lipid 157: ¹H NMR (500 MHz, CDCl₃, ppm): δ 5.39-5.31 (m, 10H), 4.36-3.95 (br, 1H), 3.76 (br, 1H), 3.40 (br, 1H), 3.24 (br, 1H), 3.08 (br, 1H), 2.84 (m, 8H), 2.63-2.14 (m, 10H), 2.03 (m, 4H), 1.71 (br, 2H), 1.67 (m, 2H), 1.52-1.17 (m, 24H), 1.03 (s, 9H), 0.97 (m, 3H), 0.86 (m, 3H). MS: m/z 697.2 (M+H⁺). Chemical formula: C₄₅H₈₁N₃O₂
Lipid 158: ¹H NMR (500 MHz, CDCl₃, ppm): δ 4.53-3.92 (br, 1H), 3.71 (m, 1H), 3.42(m, 1H), 3.22 (m, 1H), 3.08 (m, 1H), 2.63-2.14 (m, 8H), 1.66 (m, 2H), 1.51-1.18 (m, 42H), 1.03 (s, 9H), 0.97 (m, 6H), 0.87 (m, 6H). MS: m/z 623.1 (M+H⁺). Chemical formula: C₃₉H₇₉N₃O₂
Lipid 159: ¹H NMR (500 MHz, CDCl₃, ppm): δ 4.53-3.92 (br, 1H), 3.71 (m, 1H), 3.42(m, 1H), 3.22 (m, 1H), 3.08 (m, 1H), 2.63-2.14 (m, 8H), 1.66 (m, 2H), 1.51-1.18 (m, 46H), 1.03 (s, 9H), 0.97 (m, 6H), 0.87 (m, 6H). MS: m/z 651.1 (M+H⁺). Chemical formula: C₄₁H₈₃N₃O₂
Lipid 160: ¹H NMR (500 MHz, CDCl₃, ppm): δ 5.35 (m, 4H), 4.56-3.93 (br, 1H), 3.76 (br, 1H), 3.40 (br, 1H), 3.24 (br, 1H), 3.08 (br, 1H), 2.79 (m, 2H), 2.63-2.14 (m, 8H), 2.03 (m, 4H), 1.67 (m, 2H), 1.58-1.13 (m, 40H), 1.03 (s, 9H), 0.97 (m, 6H), 0.87 (t, 6H). MS: m/z 703.2 (M+H⁺). Chemical formula: C₄₅H₈₇N₃O₂
Lipid 161: ¹H NMR (500 MHz, CDCl₃, ppm): δ 5.35 (m, 2H), 4.88 (br, 1H), 3.76 (br, 1H), 3.61 (br, 1H), 3.40 (br, 1H), 3.24 (br, 1H), 3.08 (br, 1H), 2.60 (m, 4H), 2.42 (m, 2H), 2.32 (s, 2H), 2.00 (m, 4H), 1.67 (t, 2H), 1.51-1.18 (m, 44H), 1.12 (m, 6H), 1.03 (s, 9H), 0.87 (t, 6H). MS: m/z 720.8 (M+H⁺). Chemical formula: C₄₅H₈₉N₃O₃
Lipid 162: ¹H NMR (500 MHz, CDCl₃, ppm): δ 5.39-5.31 (m, 10H), 4.36-3.95 (br, 1H), 3.76 (br, 1H), 3.40 (br, 1H), 3.24 (br, 1H), 3.08 (br, 1H), 2.84 (m, 8H), 2.63-2.14 (m, 8H), 2.04 (m, 4H), 1.71 (br, 2H), 1.67 (m, 2H), 1.52-1.17 (m, 24H), 1.03 (s, 9H), 0.97 (m,9H), 0.86 (m, 3H). MS: m/z 725.2 (M+H⁺). Chemical formula: C₄₇H₈₅N₃O₂
Lipid 163: ¹H NMR (500 MHz, CDCl₃, ppm): δ 4.32 (br, 1H), 4.13 (t, 2H), 3.71 (br, 1H), 3.42 (br, 1H), 3.22 (br, 1H), 3.08 (br, 1H), 2.32 (m, 4H), 2.23 (m, 6H), 2.05 (t, 2H), 1.67 (m, 8H), 1.51-1.18 (m, 26H), 1.04 (s, 9H), 0.87 (t, 6H). MS: m/z 596.1 (M+H⁺). Chemical formula: C₃₅H₆₉N₃O₄
Lipid 166: ¹H NMR (500 MHz, CDCl₃, ppm): δ 5.35 (m, 2H), 4.32 (br, 1H), 4.13 (t, 2H), 3.71 (br, 1H), 3.53 (s, 1H), 3.42 (br, 1H), 3.22 (br, 1H), 3.08 (br, 1H), 2.77 (t, 2H), 2.23 (m, 6H), 2.00 (m, 4H), 1.67 (m, 2H), 1.51-1.18 (m, 40H), 1.04 (s, 9H), 0.87 (t, 6H). MS: m/z 694.6 (M+H⁺). Chemical formula: C₄₁H₇₉N₃O₅
Lipid 170: ¹H NMR (500 MHz, CDCl₃, ppm): δ 5.35 (m, 4H), 4.32 (br, 1H), 4.13 (t, 2H), 3.71 (br, 1H), 3.42 (br, 1H), 3.22 (br, 1H), 3.08 (br, 1H), 3.00 (m, 4H), 2.77 (t, 2H), 2.00 (m, 4H), 1.67 (m, 8H), 1.51-1.18 (m, 36H), 1.04 (s, 9H), 0.87 (t, 6H). MS: m/z 704.6 (M+H⁺). Chemical formula: C₄₃H₈₁N₃O₄
Lipid 173: ¹H NMR (500 MHz, CDCl₃, ppm): δ 4.86 (m, 2H), 4.13 (t, 2H), 3.71 (br, 1H), 3.42 (br, 1H), 3.22 (br, 1H), 3.08 (br, 1H), 2.32 (m, 4H), 2.23 (m, 6H), 2.05 (t, 2H), 1.67 (m, 8H), 1.51-1.18 (m, 48H), 1.04 (s, 9H), 0.87 (t, 9H). MS: m/z 778.9 (M+H⁺). Chemical formula: C₄₈H₉₅N₃O₄
Lipid 174: ¹H NMR (500 MHz, CDCl₃, ppm): δ 4.86 (m, 2H), 4.13 (t, 2H), 3.71 (br, 1H), 3.42 (br, 1H), 3.22 (br, 1H), 3.08 (br, 1H), 2.32 (m, 4H), 2.23 (m, 6H), 2.05 (t, 2H), 1.67 (m, 8H), 1.51-1.18 (m, 52H), 1.04 (s, 9H), 0.87 (t, 9H). MS: m/z 806.9 (M+H⁺). Chemical formula: C₅₀H₉₉N₃O₄
Lipid 175: ¹H NMR (500 MHz, CDCl₃, ppm): δ 5.35 (m, 4H), 4.86 (m, 2H), 4.13 (t, 2H), 3.71 (br, 1H), 3.42 (br, 1H), 3.22 (br, 1H), 3.08 (br, 1H), 2.77 (t, 2H), 2.23 (m, 6H), 2.00 (m, 4H), 1.67 (m, 8H), 1.51-1.18 (m, 52H), 1.04 (s, 9H), 0.87 (t, 9H). MS: m/z 858.9 (M+H⁺). Chemical formula: C₅₄H₁₀₃N₃O₄
Lipid 176: ¹H NMR (500 MHz, CDCl₃, ppm): δ 5.35 (m, 2H), 4.86 (m, 2H), 4.13 (t, 2H), 3.71 (br, 1H), 3.53 (s, 1H), 3.42 (br, 1H), 3.22 (br, 1H), 3.08 (br, 1H), 2.77 (t, 2H), 2.23 (m, 6H), 2.00 (m, 4H), 1.67 (m, 2H), 1.51-1.18 (m, 62H), 1.04 (s, 9H), 0.87 (t, 9H). MS: m/z 877.1 (M+H⁺). Chemical formula: C₅₄H₁₀₅N₃O₅
Lipid 177: ¹H NMR (500 MHz, CDCl₃, ppm): δ 5.35 (m, 10H), 4.86 (m, 2H), 4.13 (t, 2H), 3.71 (br, 1H), 3.42 (br, 1H), 3.22 (br, 1H), 3.08 (br, 1H), 2.74 (m, 8H), 2.23 (m, 6H), 2.00 (m, 4H), 1.67 (m, 6H), 1.51-1.18 (m, 40H), 1.04 (s, 9H), 0.87 (t, 9H). MS: m/z 881.1 (M+H⁺). Chemical formula: C₅₆H₁₀₁N₃O₄
Lipid 178: ¹H NMR (500 MHz, CDCl₃, ppm): δ 4.86 (m, 2H), 4.13 (t, 2H), 3.71 (br, 1H), 3.42 (br, 1H), 3.22 (br, 1H), 3.08 (br, 1H), 3.00 (m, 4H), 2.32 (m, 4H), 2.05 (t, 2H), 1.67 (m, 8H), 1.51-1.18 (m, 54H), 1.04 (s, 9H), 0.87 (t, 9H). MS: m/z 807.1 (M+H⁺). Chemical formula: C₅₀H₉₉N₃O₄
Lipid 179: ¹H NMR (500 MHz, CDCl₃, ppm): δ 4.86 (m, 2H), 4.13 (t, 2H), 3.71 (br, 1H), 3.42 (br, 1H), 3.22 (br, 1H), 3.08 (br, 1H), 3.00 (m, 4H), 2.32 (m, 4H), 2.05 (t, 2H), 1.67 (m, 8H), 1.51-1.18 (m, 56H), 1.04 (s, 9H), 0.87 (t, 9H). MS: m/z 835.1 (M+H⁺). Chemical formula: C₅₂H₁₀₃N₃O₄
Lipid 180: ¹H NMR (500 MHz, CDCl₃, ppm): δ 5.35 (m, 4H), 4.86 (m, 2H), 4.13 (t, 2H), 3.71 (br, 1H), 3.42 (br, 1H), 3.22 (br, 1H), 3.08 (br, 1H), 3.00 (m, 4H), 2.77 (t, 2H), 2.00 (m, 4H), 1.67 (m, 8H), 1.51-1.18 (m, 58H), 1.04 (s, 9H), 0.87 (t, 9H). MS: m/z 887.1 (M+H⁺). Chemical formula: C₅₆H₁₀₇N₃O₄
Lipid 181: ¹H NMR (500 MHz, CDCl₃, ppm): δ 5.35 (m, 2H), 4.86 (m, 2H), 4.13 (t, 2H), 3.71 (br, 1H), 3.53 (s, 1H), 3.42 (br, 1H), 3.22 (br, 1H), 3.08 (br, 1H), 3.00 (m, 4H), 2.77 (t, 2H), 2.00 (m, 4H), 1.67 (m, 2H), 1.51-1.18 (m, 68H), 1.04 (s, 9H), 0.87 (t, 9H). MS: m/z 905.1 (M+H⁺). Chemical formula: C₅₆H₁₀₉N₃O₅
Lipid 182: ¹H NMR (500 MHz, CDCl₃, ppm): δ 5.35 (m, 10H), 4.86 (m, 2H), 4.13 (t, 2H), 3.71 (br, 1H), 3.42 (br, 1H), 3.22 (br, 1H), 3.08 (br, 1H), 3.00 (m, 4H), 2.74 (m, 8H), 2.00 (m, 4H), 1.67 (m, 6H), 1.51-1.18 (m, 46H), 1.04 (s, 9H), 0.87 (t, 9H). MS: m/z 909.1 (M+H⁺). Chemical formula: C₅₈H₁₀₅N₃O₄
Lipid 188: ¹H NMR (500 MHz, CDCl₃, ppm): δ 4.24 (br, 1H), 3.73-3.05 (br, 6H), 2.62-2.11 (m, 9H), 1.76-1.53 (m, 6H), 1.43-1.18 (m, 34H), 1.04 (s, 9H), 0.87 (m, 6H). MS: m/z 597.1 (M+H⁺). Chemical formula: C₃₆H₇₃N₃O₃
Lipid 189: ¹H NMR (500 MHz, CDCl₃, ppm): δ 4.24 (br, 1H), 3.73-3.05 (br, 6H), 2.62-2.11 (m, 9H), 1.76-1.53 (m, 6H), 1.43-1.18 (m, 38H), 1.04 (s, 9H), 0.87 (m, 6H). MS: m/z 625.1 (M+H⁺). Chemical formula: C₃₈H₇₇N₃O₃
Lipid 194: ¹H NMR (500 MHz, CDCl₃, ppm): δ 4.24 (br, 1H), 3.73-3.05 (br, 6H), 2.62-2.11 (m, 9H), 1.76-1.53 (m, 4H), 1.43-1.18 (m, 42H), 1.04 (s, 9H), 0.87 (t, 6H). MS: m/z 639.1 (M+H⁺). Chemical formula: C₃₉H₇₉N₃O₃
Lipid 195: ¹H NMR (500 MHz, CDCl₃, ppm): δ 5.34 (m, 4H), 4.24 (br, 1H), 3.73-3.05 (br, 6H), 2.83-2.11 (m, 11H), 2.03 (m, 4H), 1.76-1.53 (m, 8H), 1.43-1.18 (m, 32H), 1.04 (s, 9H), 0.87 (t, 6H). MS: m/z 691.2 (M+H⁺). Chemical formula: C₄₃H₈₃N₃O₃
Lipid 223: ¹H NMR (500 MHz, CDCl₃, ppm): δ 5.35 (m, 2H), 4.13 (br, 1H), 4.05 (m, 4H), 3.72 (br, 1H), 3.42 (br, 1H), 3.30 (br, 1H), 3.17 (br, 1H), 2.55 (m, 4H), 2.39 (m, 2H), 2.31 (m, 2H), 2.24 (m, 6H), 2.02 (m, 4H), 1.82 (m, 2H), 1.61 (m, 6H), 1.51-1.18 (m, 32H), 1.02 (s, 9H), 0.88 (t, 6H). MS: m/z 764.8 (M+H⁺). Chemical formula: C₄₅H₈₅N₃O₆
Lipid 226: ¹H NMR (500 MHz, CDCl₃, ppm): δ 5.35 (m, 2H), 4.16 (br, 1H), 4.05 (m, 4H), 3.73 (br, 1H), 3.40 (br, 1H), 3.30 (br, 1H), 3.16 (br, 1H), 2.69 (m, 4H), 2.66 (m, 4H), 2.58 (m, 2H), 2.53 (m, 2H), 2.32 (t, 2H), 2.02 (m, 4H), 1.82 (m, 2H), 1.61 (m, 4H), 1.51-1.18 (m, 32H), 1.11 (m, 6H), 1.06 (s, 9H), 0.88 (t, 6H). MS: m/z 792.8 (M+H⁺). Chemical formula: C₄₇H₈₉N₃O₆
Lipid 227: ¹H NMR (500 MHz, CDCl₃, ppm): δ 5.35 (m, 2H), 4.13 (br, 1H), 4.05 (m, 4H), 3.70 (br, 1H), 3.41 (br, 1H), 3.34 (br, 1H), 3.14 (br, 1H), 2.83 (m, 2H), 2.61 (m, 6H), 2.39 (m, 2H), 2.32 (m, 2H), 2.02 (m, 4H), 1.91 (m, 2H), 1.82 (m, 2H), 1.61 (m, 6H), 1.51-1.18 (m, 32H), 1.11 (m, 6H), 1.06 (s, 9H), 0.88 (t, 6H). MS: m/z 806.9 (M+H⁺). Chemical formula: C₄₈H₉₁N₃O₆
Lipid 229: ¹H NMR (500 MHz, CDCl₃, ppm): δ 5.35 (m, 2H), 4.16 (br, 1H), 4.05 (m, 4H), 3.94 (m, 2H), 3.38 (br, 1H), 3.27 (br, 1H), 3.03 (br, 1H), 2.95 (br, 1H), 2.83 (m, 2H), 2.74 (m, 2H), 2.68 (m, 4H), 2.44 (m, 2H), 2.02 (m, 4H), 1.97 (m, 3H), 1.87 (m, 4H), 1.61 (m, 4H), 1.51-1.18 (m, 36H), 1.11 (s, 9H), 0.88 (t, 6H). MS: m/z 822.9 (M+H⁺). Chemical formula: C₄₈H₉₁N₃O₇
Lipid 230: ¹H NMR (500 MHz, CDCl₃, ppm): δ 5.35 (m, 2H), 4.16 (br, 1H), 4.05 (m, 4H), 3.90 (m, 2H), 3.39 (br, 1H), 3.26 (br, 1H), 3.03 (br, 1H), 2.97 (br, 1H), 2.83 (m, 2H), 2.74 (m, 2H), 2.69 (m, 2H), 2.44 (m, 2H), 2.35 (m, 2H), 2.12 (m, 2H), 2.02 (m, 4H), 1.95 (m, 3H), 1.87 (m, 4H), 1.61 (m, 4H), 1.51-1.18 (m, 36H), 1.11 (s, 9H), 0.88 (t, 6H). MS: m/z 836.9 (M+H⁺). Chemical formula: C₄₉H₉₃N₃O₇
Lipid 232: ¹H NMR (500 MHz, CDCl₃, ppm): δ 5.35 (m, 2H), 4.86 (m, 1H), 4.17 (br, 1H), 4.06 (m, 2H), 3.76 (br, 1H), 3.41 (br, 1H), 3.27 (br, 1H), 3.08 (br, 2H), 2.70 (m, 4H), 2.34 (m, 2H), 2.32 (m, 2H), 2.26 (m, 6H), 2.02 (m, 4H), 1.61 (m, 4H), 1.51-1.18 (m, 58H), 1.05 (s, 9H), 0.88 (t, 9H). MS: m/z 947.1 (M+H⁺). Chemical formula: C₅₈H₁₁₁N₃O₆
Lipid 233: ¹H NMR (500 MHz, CDCl₃, ppm): δ 5.35 (m, 2H), 4.86 (m, 1H), 4.13 (br, 1H), 4.06 (m, 2H), 3.71 (br, 1H), 3.41 (br, 1H), 3.27 (br, 1H), 3.07 (br, 1H), 2.54 (m, 2H), 2.40 (m, 4H), 2.27 (m, 4H), 2.24 (m, 6H), 2.01 (m, 4H), 1.61 (m, 4H), 1.51-1.18 (m, 60H), 1.04 (s, 9H), 0.88 (t, 9H). MS: m/z 961.2 (M+H⁺). Chemical formula: C₅₉H₁₁₃N₃O₆
Lipid 234: ¹H NMR (500 MHz, CDCl₃, ppm): δ 4.86 (m, 1H), 4.16 (br, 1H), 3.72 (br, 1H), 3.45 (br, 1H), 3.26 (br, 1H), 3.07 (br, 1H), 2.47 (m, 2H), 2.39 (m, 2H), 2.30 (m, 6H), 2.27 (m, 2H), 1.66 (m, 2H), 1.60 (m, 2H), 1.51-1.18 (m, 54H), 1.03 (s, 9H), 0.88 (t, 9H). MS: m/z 778.9 (M+H⁺). Chemical formula: C₄₈H₉₅N₃O₄
Lipid 235: ¹H NMR (500 MHz, CDCl₃, ppm): δ 5.35 (m, 2H), 4.86 (m, 1H), 4.16 (br, 1H), 4.05 (m, 2H), 3.74 (br, 1H), 3.41 (br, 1H), 3.26 (br, 1H), 3.07 (br, 1H), 2.70 (m, 4H), 2.61 (m, 4H), 2.51 (m, 2H), 2.26 (m, 2H), 2.02 (m, 4H), 1.61 (m, 4H), 1.51-1.18 (m, 60H), 1.12 (m, 6H), 1.04 (s, 9H), 0.88 (t, 9H). MS: m/z 975.2 (M+H⁺). Chemical formula: C₆₀H₁₁₅N₃O₆
Lipid 236: ¹H NMR (500 MHz, CDCl₃, ppm): δ 5.35 (m, 2H), 4.86 (m, 1H), 4.13 (br, 1H), 4.05 (m, 2H), 3.69 (br, 1H), 3.42 (br, 1H), 3.27 (br, 1H), 3.06 (br, 1H), 2.70 (m, 4H), 2.57 (m, 2H), 2.51 (m, 2H), 2.44 (m, 2H), 2.39 (m, 2H), 2.27 (m, 2H), 2.02 (m, 4H), 1.61 (m, 4H), 1.51-1.18 (m, 60H), 1.12 (m, 6H), 1.04 (s, 9H), 0.88 (t, 9H). MS: m/z 989.2 (M+H⁺). Chemical formula: C₆₁H₁₁₇N₃O₆
Lipid 237: ¹H NMR (500 MHz, CDCl₃, ppm): δ 4.86 (m, 1H), 4.13 (br, 1H), 3.72 (br, 1H), 3.45 (br, 1H), 3.24 (br, 1H), 3.07 (br, 1H), 2.56 (m, 4H), 2.42 (m, 2H), 2.30 (m, 2H), 2.24 (m, 2H), 1.80 (m, 2H), 1.66 (m, 2H), 1.61 (m, 2H), 1.51-1.18 (m, 52H), 1.12 (m, 6H), 1.02 (s, 9H), 0.88 (t, 9H). MS: m/z 806.9 (M+H⁺). Chemical formula: C₅₀H₉₉N₃O₄
Lipid 238: ¹H NMR (500 MHz, CDCl₃, ppm): δ 5.35 (m, 2H), 4.86 (m, 1H), 4.16 (br, 1H), 4.05 (m, 2H), 3.92 (m, 2H), 3.39 (br, 1H), 3.26 (br, 1H), 3.03 (br, 1H), 2.97 (br, 1H), 2.83 (m, 2H), 2.72 (m, 2H), 2.68 (m, 4H), 2.45 (m, 2H), 2.02 (m, 4H), 1.98 (m, 3H), 1.86 (m, 4H), 1.62 (m, 4H), 1.51-1.18 (m, 60H), 1.12 (s, 9H), 0.88 (t, 9H). MS: m/z 1005.2 (M+H⁺). Chemical formula: C₆₁H₁₁₇N₃O₇
Lipid 239: ¹H NMR (500 MHz, CDCl₃, ppm): δ 5.34 (m, 2H), 4.85 (m, 1H), 4.13 (br, 1H), 4.05 (m, 2H), 3.94 (m, 2H), 3.35 (br, 1H), 3.13 (br, 1H), 2.97 (br, 1H), 2.90 (br, 1H), 2.70 (m, 4H), 2.40 (m, 2H), 2.27 (m, 4H), 2.11 (m, 2H), 2.02 (m, 4H), 1.95 (m, 3H), 1.61 (m, 4H), 1.51 (m, 2H), 1.49-1.18 (m, 60H), 1.06 (s, 9H), 0.88 (t, 9H). MS: m/z 1019.2 (M+H⁺). Chemical formula: C₆₂H₁₁₉N₃O₇
Lipid 240: ¹H NMR (500 MHz, CDCl₃, ppm): δ 4.86 (m, 1H), 4.16 (br, 1H), 3.93 (m, 2H), 3.71 (br, 1H), 3.45 (br, 1H), 3.28 (br, 1H), 3.04 (br, 1H), 2.82 (m, 2H), 2.73 (m, 2H), 2.43 (m, 2H), 2.30 (m, 2H), 1.98 (m, 3H), 1.62 (m, 6H), 1.51-1.18 (m, 56H), 1.12 (s, 9H), 0.88 (t, 9H). MS: m/z 836.9 (M+H⁺). Chemical formula: C₅₁H₁₀₁N₃O₅
Lipid 241: ¹H NMR (500 MHz, CDCl₃, ppm): δ 5.35 (m, 2H), 4.16 (br, 1H), 4.10 (m, 2H), 4.06 (m, 2H), 3.73 (br, 1H), 3.44 (br, 1H), 3.25 (br, 1H), 3.08 (br, 1H), 2.68 (m, 4H), 2.38 (m, 2H), 2.29 (m, 1H), 2.24 (m, 6H), 2.02 (m, 4H), 1.80 (m, 2H), 1.72 (m, 4H), 1.61 (m, 4H), 1.51-1.18 (m, 48H), 1.02 (s, 9H), 0.88 (t, 9H). MS: m/z 905.1 (M+H⁺). Chemical formula: C₅₅H₁₀₅N₃O₆
Lipid 242: ¹H NMR (500 MHz, CDCl₃, ppm): δ 5.35 (m, 2H), 4.16 (br, 1H), 4.10 (m, 2H), 4.06 (m, 2H), 3.73 (br, 1H), 3.45 (br, 1H), 3.25 (br, 1H), 3.08 (br, 1H), 2.72 (m, 2H), 2.58 (m, 2H), 2.38 (m, 2H), 2.29 (m, 1H), 2.24 (m, 6H), 2.12 (m, 2H), 2.02 (m, 4H), 1.80 (m, 2H), 1.72 (m, 4H), 1.61 (m, 4H), 1.51-1.18 (m, 48H), 1.02 (s, 9H), 0.88 (t, 9H). MS: m/z 919.1 (M+H⁺). Chemical formula: C₅₆H₁₀₇N₃O₆
Lipid 243: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 2H), 2.28 (m, 1H), 2.24 (m, 6H), 2.18 (m, 2H), 1.80 (m, 2H), 1.72 (m, 4H), 1.60 (m, 4H), 1.51-1.18 (m, 42H), 1.02 (s, 9H), 0.88 (t, 9H). MS: m/z 736.8 (M+H⁺). Chemical formula: C₄₅H₈₉N₃O₄
Lipid 244: ¹H NMR (500 MHz, CDCl₃, ppm): δ 5.35 (m, 2H), 4.16 (br, 1H), 4.10 (m, 2H), 4.06 (m, 2H), 3.73 (br, 1H), 3.44 (br, 1H), 3.25 (br, 1H), 3.08 (br, 1H), 2.68 (m, 4H), 2.60 (m, 4H), 2.38 (m, 2H), 2.29 (m, 1H), 2.02 (m, 4H), 1.80 (m, 2H), 1.72 (m, 4H), 1.61 (m, 4H), 1.51-1.18 (m, 48H), 1.12 (t, 6H), 1.02 (s, 9H), 0.88 (t, 9H). MS: m/z 933.1 (M+H⁺). Chemical formula: C₅₇H₁₀₉N₃O₆
Lipid 245: ¹H NMR (500 MHz, CDCl₃, ppm): δ 5.35 (m, 2H), 4.16 (br, 1H), 4.10 (m, 2H), 4.06 (m, 2H), 3.73 (br, 1H), 3.44 (br, 1H), 3.26 (br, 1H), 3.08 (br, 1H), 2.72 (m, 2H), 2.60 (m, 4H), 2.54 (m, 2H), 2.38 (m, 2H), 2.29 (m, 1H), 2.12 (m, 2H), 2.02 (m, 4H), 1.81 (m, 2H), 1.72 (m, 4H), 1.61 (m, 4H), 1.51-1.18 (m, 48H), 1.12 (t, 6H), 1.02 (s, 9H), 0.88 (t, 9H). MS: m/z 947.1 (M+H⁺). Chemical formula: C₅₈H₁₁₁N₃O₆
Lipid 246: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.60 (m, 4H), 2.44 (m, 2H), 2.27 (m, 1H), 2.18 (m, 2H), 1.80 (m, 2H), 1.72 (m, 4H), 1.60 (m, 4H), 1.51-1.18 (m, 42H), 1.12 (t, 6H), 1.02 (s, 9H), 0.88 (t, 9H). MS: m/z 764.9 (M+H⁺). Chemical formula: C₄₇H₉₃N₃O₄
Lipid 284: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 2H), 2.28 (m, 1H), 2.24 (m, 6H), 2.18 (m, 2H), 1.80 (m, 2H), 1.72 (m, 4H), 1.60 (m, 4H), 1.51-1.18 (m, 46H), 1.02 (s, 9H), 0.88 (t, 9H). MS: m/z 764.9 (M+H⁺). Chemical formula: C₄₇H₉₃N₃O₄
Lipid 285: ¹H NMR (500 MHz, CDCl₃, ppm): δ 5.35 (m, 4H), 4.13 (br, 1H), 4.05 (m, 2H), 3.73 (br, 1H), 3.47 (br, 1H), 3.22 (br, 1H), 3.08 (br, 1H), 2.77 (t, 2H), 2.45 (m, 2H), 2.28 (m, 1H), 2.24 (m, 6H), 2.18 (m, 2H), 2.00 (m, 4H) 1.80 (m, 2H), 1.72 (m, 4H), 1.60 (m, 4H), 1.51-1.18 (m, 40H), 1.02 (s, 9H), 0.88 (t, 9H). MS: m/z 816.9 (M+H⁺). Chemical formula: C₅₁H₉₇N₃O₄
Lipid 286: ¹H NMR (500 MHz, CDCl₃, ppm): δ 5.34 (m, 2H), 4.13 (br, 1H), 4.05 (m, 2H), 3.73 (br, 1H), 3.53 (s, 1H), 3.47 (br, 1H), 3.22 (br, 1H), 3.08 (br, 1H), 2.45 (m, 2H), 2.28 (m, 1H), 2.24 (m, 6H), 2.18 (m, 2H), 2.00 (m, 4H), 1.80 (m, 2H), 1.72 (m, 4H), 1.60 (m, 4H), 1.51-1.18 (m, 46H), 1.02 (s, 9H), 0.88 (t, 9H). MS: m/z 834.9 (M+H⁺). Chemical formula: C₅₁H₉₉N₃O₅
Lipid 287: ¹H NMR (500 MHz, CDCl₃, ppm): δ 5.34 (m, 10H), 4.13 (br, 1H), 4.05 (m, 2H), 3.73 (br, 1H), 3.47 (br, 1H), 3.22 (br, 1H), 3.08 (br, 1H), 2.74 (m, 8H), 2.45 (m, 2H), 2.28 (m, 1H), 2.24 (m, 6H), 2.18 (m, 2H), 2.00 (m, 4H), 1.80 (m, 2H), 1.72 (m, 4H), 1.60 (m, 4H), 1.51-1.18 (m, 26H), 1.02 (s, 9H), 0.88 (t, 9H). MS: m/z 838.9 (M+H⁺). Chemical formula: C₅₃H₉₅N₃O₄
Lipid 292: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.60 (m, 4H), 2.44 (m, 2H), 2.27 (m, 1H), 2.18 (m, 2H), 1.80 (m, 2H), 1.72 (m, 4H), 1.60 (m, 4H), 1.51-1.18 (m, 46H), 1.12 (t, 6H), 1.02 (s, 9H), 0.88 (t, 9H). MS: m/z 792.9 (M+H⁺). Chemical formula: C₄₉H₉₇N₃O₄
Lipid 293: ¹H NMR (500 MHz, CDCl₃, ppm): δ 5.35 (m, 4H), 4.13 (br, 1H), 4.05 (m, 2H), 3.73 (br, 1H), 3.47 (br, 1H), 3.22 (br, 1H), 3.08 (br, 1H), 2.77 (t, 2H), 2.60 (m, 4H), 2.45 (m, 2H), 2.28 (m, 1H), 2.18 (m, 2H), 2.00 (m, 4H) 1.80 (m, 2H), 1.72 (m, 4H), 1.60 (m, 4H), 1.51-1.18 (m, 40H), 1.12 (t, 6H), 1.02 (s, 9H), 0.88 (t, 9H). MS: m/z 845.0 (M+H⁺). Chemical formula: C₅₃H₁₀₁N₃O₄
Lipid 294: ¹H NMR (500 MHz, CDCl₃, ppm): δ 5.34 (m, 2H), 4.13 (br, 1H), 4.05 (m, 2H), 3.73 (br, 1H), 3.53 (s, 1H), 3.47 (br, 1H), 3.22 (br, 1H), 3.08 (br, 1H), 2.60 (m, 4H), 2.45 (m, 2H), 2.28 (m, 1H), 2.18 (m, 2H), 2.00 (m, 4H), 1.80 (m, 2H), 1.72 (m, 4H), 1.60 (m, 4H), 1.51-1.18 (m, 54H), 1.12 (t, 6H), 1.02 (s, 9H), 0.88 (t, 9H). MS: m/z 863.1 (M+H⁺). Chemical formula: C₅₃H₁₀₃N₃O₅
Lipid 295: ¹H NMR (500 MHz, CDCl₃, ppm): δ 5.34 (m, 10H), 4.13 (br, 1H), 4.05 (m, 2H), 3.73 (br, 1H), 3.47 (br, 1H), 3.22 (br, 1H), 3.08 (br, 1H), 2.74 (m, 8H), 2.60 (m, 4H), 2.45 (m, 2H), 2.28 (m, 1H), 2.18 (m, 2H), 2.00 (m, 4H), 1.80 (m, 2H), 1.72 (m, 4H), 1.60 (m, 4H), 1.51-1.18 (m, 26H), 1.12 (t, 6H), 1.02 (s, 9H), 0.88 (t, 9H). MS: m/z 867.0 (M+H⁺). Chemical formula: C₅₅H₉₉N₃O₄
Lipid 304: ¹H NMR (500 MHz, CDCl₃, ppm): 5.73 (m, 1H), 4.87 (m, 1H), 3.42 (m, 2H), 3.19 (m, 2H), 2.39 (s, 2H), 2.34-2.21 (m, 10H), 1.83 (m, 2H), 1.62 (m, 4H), 1.48 (m, 12H), 1.37-1.18 (m, 50H), 0.88 (m, 9H). MS: m/z 779.2 (M+H⁺). Chemical formula: C₄₈H₉₅N₃O₄
Lipid 314: ¹H NMR (500 MHz, CDCl₃, ppm): δ 7.94-7.57 (m, 5H), 5.34 (m, 4H), 4.15 (br, 1H), 4.06 (m, 2H), 3.67 (br, 1H), 3.58 (br, 2H), 3.09 (br, 1H), 2.32 (m, 4H), 2.23 (m, 6H), 2.17 (m, 2H), 2.00 (m, 8H), 1.67 (m, 8H), 1.51-1.18 (m, 40H), 0.87 (t, 6H). MS: m/z 839.1 (M+H⁺). Chemical formula: C₅₂H₉₁N₃O₃S
Lipid 315: ¹H NMR (500 MHz, CDCl₃, ppm): δ 5.34 (m, 4H), 4.15 (br, 1H), 4.06 (m, 2H), 3.67 (br, 1H), 3.58 (br, 2H), 3.09 (br, 1H), 2.32 (m, 7H), 2.23 (m, 6H), 2.17 (m, 2H), 2.00 (m, 8H), 1.67 (m, 8H), 1.51-1.18 (m, 40H), 0.87 (t, 6H). MS: m/z 777.1 (M+H⁺). Chemical formula: C₄₇H₈₉N₃O₃S
Lipid 316: ¹H NMR (500 MHz, CDCl₃, ppm): δ 5.34 (m, 2H), 4.15 (s, 1H), 3.67(br, 1H), 3.58 (br, 2H), 3.09 (br, 1H), 2.32 (m, 4H), 2.23 (m, 6H), 2.00 (m, 4H), 1.67 (m, 8H), 1.51-1.18 (m, 40H), 1.16 (s, 9H), 0.87 (t, 6H). MS: m/z 692.8 (M+H⁺). Chemical formula: C₄₃H₈₅N₃OS
Lipid 317: ¹H NMR (500 MHz, CDCl₃, ppm):δ 4.86 (m, 1H), 4.15 (s, 1H), 3.67 (br, 1H), 3.58 (br, 2H), 3.09 (br, 1H), 2.32 (m, 4H), 2.23 (m, 6H), 2.05 (t, 2H), 1.67 (m, 8H), 1.51-1.18 (m, 54H), 1.16 (s, 9H), 0.87 (t, 9H). MS: m/z 823.1 (M+H⁺). Chemical formula: C₅₀H₉₉N₃O₃S
Lipid 401: ¹H NMR (500 MHz, CDCl₃, ppm): δ 5.90 (m, 1H), 4.89 (m, 2H), 3.73 (br, 1H), 3.47 (br, 1H), 3.22 (br, 1H), 3.08 (br, 1H), 2.45 (m, 6H), 2.24 (m, 6H), 1.72 (m, 6H), 1.60 (m, 2H), 1.51-1.18 (m, 46H), 1.02 (s, 9H), 0.88 (t, 12H). MS: m/z 822.7. Chemical formula: C₄₉H₉₅N₃O₆
Lipid 402: ¹H NMR (500 MHz, CDCl₃, ppm): δ 5.90 (m, 1H), 4.89 (m, 2H), 3.73 (br, 1H), 3.47 (br, 1H), 3.22 (br, 1H), 3.08 (br, 1H), 2.45 (m, 6H), 2.24 (m, 6H), 1.72 (m, 6H), 1.60 (m, 2H), 1.51-1.18 (m, 58H), 1.02 (s, 9H), 0.88 (t, 12H). MS: m/z 906.7. Chemical formula: C₅₅H₁₀₇N₃O₆
Lipid 403: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.72 (m, 6H), 1.60 (m, 2H), 1.51-1.18 (m, 46H), 1.02 (s, 9H), 0.88 (t, 9H), 0.80 (t, 3H). MS: m/z 808.7. Chemical formula: C₄₈H₉₃N₃O₆
Lipid 404: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 56H), 1.02 (s, 9H), 0.88 (t, 12H). MS: m/z 878.7. Chemical formula: C₅₃H₁₀₃N₃O₆
Lipid 405: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 58H), 1.02 (s, 9H), 0.88 (t, 12H). MS: m/z 892.7. Chemical formula: C₅₄H₁₀₅N₃O₆
Lipid 406: ¹H NMR (500 MHz, CDCl₃, ppm): δ 5.88 (m, 1H), 4.80 (m, 1H), 4.05 (m, 2H), 3.73 (br, 1H), 3.47 (br, 1H), 3.22 (br, 1H), 3.08 (br, 1H), 2.45 (m, 6H), 2.24 (m, 6H), 2.13 (m, 1H), 1.72 (m, 6H), 1.60 (m, 2H), 1.51-1.18 (m, 50H), 1.02 (s, 9H), 0.88 (t, 12H). MS: m/z 864.7. Chemical formula: C₅₂H₁₀₁N₃O₆
Lipid 407: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 6H), 2.24 (m, 2H), 1.72 (m, 6H), 1.60 (m, 2H), 1.51-1.18 (m, 60H), 1.02 (s, 9H), 0.88 (t, 12H). MS: m/z 892.7. Chemical formula: C₅₄H₁₀₅N₃O₆
Lipid 408: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 6H), 2.24 (m, 2H), 1.72 (m, 6H), 1.60 (m, 2H), 1.51-1.18 (m, 72H), 1.02 (s, 9H), 0.88 (t, 12H). MS: m/z 976.7. Chemical formula: C₆₀H₁₁₇N₃O₆
Lipid 409: ¹H NMR (500 MHz, CDCl₃, ppm): δ 4.85 (m, 2H), 3.93 (m, 1H), 3.84 (m, 1H), 3.73 (br, 1H), 3.49 (br, 1H), 3.26 (br, 1H), 3.07 (br, 1H), 2.45 (m, 6H), 2.24 (m, 2H), 2.09 (m, 1H), 1.72 (m, 6H), 1.60 (m, 2H), 1.51-1.18 (m, 56H), 1.02 (s, 9H), 0.88 (m, 12H). MS: m/z 878.7. Chemical formula: C₅₃H₁₀₃N₃O₆
Lipid 410: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 66H), 1.02 (s, 9H), 0.88 (t, 12H). MS: m/z 948.7. Chemical formula: C₅₈H₁₁₃N₃O₆
Lipid 411: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 68H), 1.02 (s, 9H), 0.88 (t, 12H). MS: m/z 962.7. Chemical formula: C₅₉H₁₁₅N₃O₆
Lipid 412: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.72 (m, 6H), 1.60 (m, 2H), 1.51-1.18 (m, 64H), 1.02 (s, 9H), 0.88 (t, 12H). MS: m/z 934.7. Chemical formula: C₅₇H₁₁₁N₃O₆
Lipid 413: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 6H), 2.24 (m, 2H), 1.72 (m, 6H), 1.60 (m, 2H), 1.51-1.18 (m, 64H), 1.02 (s, 9H), 0.88 (t, 12H). MS: m/z 920.7. Chemical formula: C₅₆H₁₀₉N₃O₆
Lipid 414: ¹H NMR (500 MHz, CDCl₃, ppm): δ 4.86 (m, 2H), 3.73 (br, 1H), 3.64 (s, 1H), 3.43 (br, 1H), 3.25 (br, 1H), 3.06 (br, 1H), 2.45 (m, 6H), 2.24 (m, 6H), 1.60 (m, 6H), 1.51-1.18 (m, 74H), 1.02 (s, 9H), 0.88 (t, 12H). MS: m/z 1004.7. Chemical formula: C₆₂H₁₂₁N₃O₆
Lipid 415: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.72 (m, 6H), 1.60 (m, 2H), 1.51-1.18 (m, 60H), 1.02 (s, 9H), 0.88 (t, 9H), 0.80 (t, 3H). MS: m/z 906.7. Chemical formula: C₅₅H₁₀₇N₃O₆
Lipid 416: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 70H), 1.02 (s, 9H), 0.88 (t, 12H). MS: m/z 976.7. Chemical formula: C₆₀H₁₁₇N₃O₆
Lipid 417: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 72H), 1.02 (s, 9H), 0.88 (t, 12H). MS: m/z 990.7. Chemical formula: C₆₁H₁₁₉N₃O₆
Lipid 418: ¹H NMR (500 MHz, CDCl₃, ppm): δ 5.95 (m, 1H), 4.85 (m, 1H), 4.05 (m, 2H), 3.73 (br, 1H), 3.47 (br, 1H), 3.22 (br, 1H), 3.08 (br, 1H), 2.45 (m, 6H), 2.24 (m, 6H), 1.72 (m, 6H), 1.60 (m, 2H), 1.51-1.18 (m, 66H), 1.02 (s, 9H), 0.88 (t, 12H). MS: m/z 962.7. Chemical formula: C₅₉H₁₁₅N₃O₆
Lipid 419: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.72 (m, 6H), 1.60 (m, 2H), 1.51-1.18 (m, 48H), 1.02 (s, 9H), 0.88 (t, 9H), 0.80 (t, 3H). MS: m/z 808.7. Chemical formula: C₄₈H₉₃N₃O₆
Lipid 420: ¹H NMR (500 MHz, CDCl₃, ppm): δ5.88 (m, 1H), 4.86 (m, 2H), 3.97 (m, 1H), 3.82 (m, 1H), 3.69 (br, 1H), 3.44 (br, 1H), 3.25 (br, 1H), 3.08 (br, 1H), 2.45 (m, 6H), 2.24 (m, 2H), 2.09 (m, 1H), 1.72 (m, 6H), 1.60 (m, 2H), 1.51-1.18 (m, 58H), 1.02 (s, 9H), 0.88 (m, 12H). MS: m/z 892.7. Chemical formula: C₅₄H₁₀₅N₃O₆
Lipid 421: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 2H), 1.72 (m, 6H), 1.60 (m, 2H), 1.51-1.18 (m, 44H), 1.02 (s, 9H), 0.88 (t, 6H), 0.80 (t, 6H). MS: m/z 794.7. Chemical formula: C₄₇H₉₁N₃O₆
Lipid 422: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.89 (m, 1H), 1.72 (m, 6H), 1.60 (m, 2H), 1.51-1.18 (m, 54H), 1.02 (s, 9H), 0.88 (t, 9H) , 0.80 (t, 3H). MS: m/z 864.7. Chemical formula: C₅₂H₁₀₁N₃O₆
Lipid 423: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.89 (m, 1H), 1.72 (m, 6H), 1.60 (m, 2H), 1.51-1.18 (m, 56H), 1.02 (s, 9H), 0.88 (t, 9H) , 0.80 (t, 3H). MS: m/z 878.7. Chemical formula: C₅₃H₁₀₃N₃O₆
Lipid 424: ¹H NMR (500 MHz, CDCl₃, ppm): δ 5.88 (m, 1H), 4.89 (m, 1H), 4.05 (t, 2H), 3.97 (m, 1H), 3.82 (m, 1H), 3.69 (br, 1H), 3.44 (br, 1H), 3.25 (br, 1H), 3.08 (br, 1H), 2.45 (m, 6H), 2.24 (m, 2H), 2.13 (m, 1H), 2.09 (m, 1H), 1.72 (m, 6H), 1.60 (m, 2H), 1.51-1.18 (m, 50H), 1.02 (s, 9H), 0.88 (m, 12H). MS: m/z 850.7. Chemical formula: C₅₁H₉₉N₃O₆
Lipid 425: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 54H), 1.02 (s, 9H), 0.88 (t, 12H). MS: m/z 864.7. Chemical formula: C₅₂H₁₀₁N₃O₆
Lipid 426: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 66H), 1.02 (s, 9H), 0.88 (t, 12H). MS: m/z 948.7. Chemical formula: C₅₈H₁₁₃N₃O₆
Lipid 427: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.89 (m, 1H), 1.72 (m, 6H), 1.60 (m, 2H), 1.51-1.18 (m, 50H), 1.02 (s, 9H), 0.88 (t, 9H), 0.80 (t, 3H). MS: m/z 850.7. Chemical formula: C₅₁H₉₉N₃O₆
Lipid 428: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 60H), 1.02 (s, 9H), 0.88 (t, 12H). MS: m/z 920.7. Chemical formula: C₅₆H₁₀₉N₃O₆
Lipid 429: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 62H), 1.02 (s, 9H), 0.88 (t, 12H). MS: m/z 934.7. Chemical formula: C₅₇H₁₁₁N₃O₆
Lipid 430: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.89 (m, 1H), 1.72 (m, 6H), 1.60 (m, 2H), 1.51-1.18 (m, 58H), 1.02 (s, 9H), 0.88 (t, 12H). MS: m/z 906.7. Chemical formula: C₅₅H₁₀₇N₃O₆
Lipid 431: ¹H NMR (500 MHz, CDCl₃, ppm): δ 4.85 (m, 2H), 3.93 (m, 1H), 3.84 (m, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 58H), 1.02 (s, 9H), 0.88 (t, 12H). MS: m/z 892.7. Chemical formula: C₅₄H₁₀₅N₃O₆
Lipid 432: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 70H), 1.02 (s, 9H), 0.88 (t, 12H). MS: m/z 976.7. Chemical formula: C₆₀H₁₁₇N₃O₆
Lipid 433: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.89 (m, 1H), 1.72 (m, 6H), 1.60 (m, 2H), 1.51-1.18 (m, 54H), 1.02 (s, 9H), 0.88 (t, 9H), 0.80 (t, 3H). MS: m/z 878.7. Chemical formula: C₅₃H₁₀₃N₃O₆
Lipid 434: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 64H), 1.02 (s, 9H), 0.88 (t, 12H). MS: m/z 948.7. Chemical formula: C₅₈H₁₁₃N₃O₆
Lipid 435: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 66H), 1.02 (s, 9H), 0.88 (t, 12H). MS: m/z 962.7. Chemical formula: C₅₉H₁₁₅N₃O₆
Lipid 436: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.89 (m, 1H), 1.72 (m, 6H), 1.60 (m, 2H), 1.51-1.18 (m, 62H), 1.02 (s, 9H), 0.88 (t, 12H). MS: m/z 934.7. Chemical formula: C₅₇H₁₁₁N3O6
Lipid 437: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 60H), 1.02 (s, 9H), 0.88 (t, 12H). MS: m/z 906.7. Chemical formula: C₅₅H₁₀₇N₃O₆
Lipid 438: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 72H), 1.02 (s, 9H), 0.88 (t, 12H). MS: m/z 990.7. Chemical formula: C₆₁H₁₁₉N₃O₆
Lipid 439: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.89 (m, 1H), 1.72 (m, 6H), 1.60 (m, 2H), 1.51-1.18 (m, 56H), 1.02 (s, 9H), 0.88 (t, 9H), 0.80 (t, 3H). MS: m/z 892.7. Chemical formula: C₅₄H₁₀₅N₃O₆
Lipid 440: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 66H), 1.02 (s, 9H), 0.88 (t, 12H). MS: m/z 962.7. Chemical formula: C₅₉H₁₁₅N₃O₆
Lipid 441: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 68H), 1.02 (s, 9H), 0.88 (t, 12H). MS: m/z 976.7. Chemical formula: C₆₀H₁₁₇N₃O₆
Lipid 442: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.89 (m, 1H), 1.72 (m, 6H), 1.60 (m, 2H), 1.51-1.18 (m, 64H), 1.02 (s, 9H), 0.88 (t, 12H). MS: m/z 948.7. Chemical formula: C₅₈H₁₁₃N₃O₆
Lipid 443: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.72 (m, 6H), 1.60 (m, 2H), 1.51-1.18 (m, 56H), 1.02 (s, 9H), 0.88 (t, 12H). MS: m/z 878.7. Chemical formula: C₅₃H₁₀₃N₃O₆
Lipid 444: ¹H NMR (500 MHz, CDCl₃, ppm): δ 5.95 (m, 1H), 4.85 (m, 1H), 4.05 (m, 2H), 3.73 (br, 1H), 3.47 (br, 1H), 3.22 (br, 1H), 3.08 (br, 1H), 2.45 (m, 6H), 2.24 (m, 6H), 1.72 (m, 6H), 1.60 (m, 2H), 1.51-1.18 (m, 66H), 1.02 (s, 9H), 0.88 (t, 12H). MS: m/z 962.7. Chemical formula: C₅₉H₁₁₅N₃O₆
Lipid 445: ¹H NMR (500 MHz, CDCl₃, ppm): δ 4.05 (t, 2H), 3.97 (d, 2H), 3.73 (br, 1H), 3.47 (br, 1H), 3.22 (br, 1H), 3.08 (br, 1H), 2.45 (m, 1H), 2.29 (t, 4H), 2.21 (s, 6H), 1.76-1.55 (m, 13H), 1.51-1.18 (m, 46H), 1.02 (s, 9H), 0.88 (m, 12H). MS: m/z 864.7. Chemical formula: C₅₂H₁₀₁N₃O₆
Lipid 446: ¹H NMR (500 MHz, CDCl₃, ppm): δ 4.05 (t, 2H), 3.97 (d, 2H), 3.73 (br, 1H), 3.47 (br, 1H), 3.22 (br, 1H), 3.08 (br, 1H), 2.45 (m, 1H), 2.29 (t, 4H), 2.21 (s, 6H), 1.76-1.55 (m, 13H), 1.51-1.18 (m, 56H), 1.02 (s, 9H), 0.88 (t, 12H). MS: m/z 934.7. Chemical formula: C₅₇H₁₁₁N₃O₆
Lipid 447: ¹H NMR (500 MHz, CDCl₃, ppm): δ 4.05 (t, 2H), 3.97 (d, 2H), 3.73 (br, 1H), 3.47 (br, 1H), 3.22 (br, 1H), 3.08 (br, 1H), 2.45 (m, 1H), 2.29 (t, 4H), 2.21 (s, 6H), 1.76-1.55 (m, 13H), 1.51-1.18 (m, 58H), 1.02 (s, 9H), 0.88 (t, 12H). MS: m/z 948.7. Chemical formula: C₅₈H₁₁₃N₃O₆
Lipid 448: ¹H NMR (500 MHz, CDCl₃, ppm): δ 5.95 (m, 1H), 4.05 (m, 4H), 3.73 (br, 1H), 3.47 (br, 1H), 3.22 (br, 1H), 3.08 (br, 1H), 2.45 (m, 6H), 2.24 (m, 2H), 2.13 (m, 2H), 1.72 (m, 6H), 1.60 (m, 2H), 1.51-1.18 (m, 62H), 1.02 (s, 9H), 0.88 (t, 12H). MS: m/z 920.7. Chemical formula: C₅₆H₁₀₉N₃O₆
Lipid 577: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 6H), 2.24 (m, 2H), 1.72 (m, 6H), 1.60 (m, 2H), 1.51-1.18 (m, 64H), 1.02 (s, 9H), 0.88 (t, 12H). MS: m/z 920.7. Chemical formula: C₅₆H₁₀₉N₃O₆
Lipid 578: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 56H), 1.02 (m, 12H), 0.88 (t, 9H). MS: m/z 892.7. Chemical formula: C₅₄H₁₀₅N₃O₆
Lipid 579: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 70H), 1.02 (s, 9H), 0.88 (t, 12H). MS: m/z 976.7. Chemical formula: C₆₀H₁₁₇N₃O₆
Lipid 580: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.72 (m, 6H), 1.60 (m, 2H), 1.51-1.18 (m, 60H), 1.02 (s, 9H), 0.88 (t, 12H). MS: m/z 906.7. Chemical formula: C₅₅H₁₀₇N₃O₆
Lipid 581: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 6H), 2.24 (m, 2H), 1.72 (m, 6H), 1.60 (m, 2H), 1.51-1.18 (m, 58H), 1.02 (s, 9H), 0.88 (t, 12H). MS: m/z 878.7. Chemical formula: C₅₃H₁₀₃N₃O₆
Lipid 582: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 6H), 2.24 (m, 2H), 1.72 (m, 6H), 1.60 (m, 2H), 1.51-1.18 (m, 68H), 1.02 (s, 9H), 0.88 (t, 12H). MS: m/z 948.7. Chemical formula: C₅₈H₁₁₃N₃O₆
Lipid 583: ¹H NMR (500 MHz, CDCl₃, ppm): δ 4.86 (m, 2H), 4.00 (m, 2H), 3.73 (br, 1H), 3.47 (br, 1H), 3.22 (br, 1H), 3.08 (br, 1H), 2.45 (m, 6H), 2.24 (m, 2H), 1.76-1.55 (m, 17H), 1.51-1.18 (m, 54H), 1.02 (s, 9H), 0.88 (t, 12H). MS: m/z 864.7. Chemical formula: C₅₂H₁₀₁N₃O₆
Lipid 584: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 52H), 1.02 (m, 15H), 0.88 (t, 6H). MS: m/z 836.7. Chemical formula: C₅₀H₉₇N₃O₆
Lipid 585: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 68H), 1.02 (m, 12H), 0.88 (t, 9H). MS: m/z 920.7. Chemical formula: C₅₆H₁₀₉N₃O₆
Lipid 586: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.89 (m, 1H), 1.72 (m, 6H), 1.60 (m, 2H), 1.51-1.18 (m, 58H), 1.02 (m, 12H), 0.88 (t, 9H). MS: m/z 850.7. Chemical formula: C₅₁H₉₉N₃O₆
Lipid 587: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 56H), 1.02 (m, 12H), 0.88 (t, 9H). MS: m/z 822.7. Chemical formula: C₄₉H₉₅N₃O₆
Lipid 588: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 66H), 1.02 (m, 12H), 0.88 (t, 9H). MS: m/z 892.7. Chemical formula: C₅₄H₁₀₅N₃O₆
Lipid 589: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 74H), 1.02 (s, 9H), 0.88 (t, 12H). MS: m/z 948.7. Chemical formula: C₅₈H₁₁₃N₃O₆
Lipid 590: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 64H), 1.02 (m, 12H), 0.88 (t, 9H). MS: m/z 920.7. Chemical formula: C₅₆H₁₀₉N₃O₆
Lipid 591: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 80H), 1.02 (s, 9H), 0.88 (t, 12H). MS: m/z 1004.7. Chemical formula: C₆₂H₁₂₁N₃O₆
Lipid 592: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.89 (m, 1H), 1.72 (m, 6H), 1.60 (m, 2H), 1.51-1.18 (m, 70H), 1.02 (s, 9H), 0.88 (t, 12H). MS: m/z 934.7. Chemical formula: C₅₇H₁₁₁N₃O₆
Lipid 593: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 68H), 1.02 (s, 9H), 0.88 (t, 12H).MS: m/z 906.7. Chemical formula: C₅₅H₁₀₇N₃O₆
Lipid 594: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 78H), 1.02 (s, 9H), 0.88 (t, 12H). MS: m/z 976.7. Chemical formula: C₆₀H₁₁₇N₃O₆
Lipid 595: ¹H NMR (500 MHz, CDCl₃, ppm): δ 4.89 (m, 2H), 4.11 (m, 2H), 3.73 (br, 1H), 3.37 (br, 2H), 3.08 (br, 1H), 2.30 (m, 3H), 2.24 (m, 6H), 1.84 (m, 4H), 1.67 (m, 6H), 1.54 (m, 4H), 1.51-1.18 (m, 58H), 1.02 (s, 9H), 0.88 (t, 12H). MS: m/z 934.7. Chemical formula: C₅₇H₁₁₁N₃O₆
Lipid 596: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 4H), 1.89 (m, 2H), 1.72 (m, 6H), 1.60 (m, 2H), 1.51-1.18 (m, 60H), 1.02 (m, 12H), 0.88 (t, 9H). MS: m/z 906.7. Chemical formula: C₅₅H₁₀₇N₃O₆
Lipid 597: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 4H), 1.89 (m, 2H), 1.72 (m, 6H), 1.60 (m, 2H), 1.51-1.18 (m, 76H), 1.02 (s, 9H), 0.88 (t, 12H). MS: m/z 990.7. Chemical formula: C₆₁H₁₁₉N₃O₆
Lipid 598: ¹H NMR (500 MHz, CDCl₃, ppm): δ 4.89 (m, 2H), 4.11 (m, 2H), 3.73 (br, 1H), 3.37 (br, 2H), 3.08 (br, 1H), 2.30 (m, 3H), 2.24 (m, 6H), 1.84 (m, 4H), 1.67 (m, 6H), 1.54 (m, 4H), 1.51-1.18 (m, 56H), 1.02 (s, 9H), 0.88 (t, 12H). MS: m/z 920.7. Chemical formula: C₅₆H₁₀₉N₃O₆
Lipid 599: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 4H), 1.89 (m, 1H), 1.72 (m, 6H), 1.60 (m, 2H), 1.51-1.18 (m, 64H), 1.02 (s, 9H), 0.88 (t, 12H). MS: m/z 892.7. Chemical formula: C₅₄H₁₀₅N₃O₆
Lipid 600: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 4H), 1.89 (m, 1H), 1.72 (m, 6H), 1.60 (m, 2H), 1.51-1.18 (m, 74H), 1.02 (s, 9H), 0.88 (t, 12H). MS: m/z 962.7. Chemical formula: C₅₉H₁₁₅N₃O₆
Lipid 601: ¹H NMR (500 MHz, CDCl₃, ppm): δ 4.89 (m, 3H), 3.73 (br, 1H), 3.47 (br, 1H), 3.22 (br, 1H), 3.08 (br, 1H), 2.45 (m, 6H), 2.24 (m, 2H), 1.72 (m, 6H), 1.60 (m, 2H), 1.51-1.18 (m, 72H), 1.02 (s, 9H), 0.88 (t, 12H). MS: m/z 976.7. Chemical formula: C₆₀H₁₁₇N₃O₆
Lipid 602: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.72 (m, 6H), 1.60 (m, 2H), 1.51-1.18 (m, 54H), 1.02 (s, 9H), 0.88 (t, 9H), 0.80 (t, 3H). MS: m/z 864.7. Chemical formula: C₅₂H₁₀₁N₃O₆
Lipid 603: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 62H), 1.02 (s, 9H), 0.88 (t, 12H). MS: m/z 920.7. Chemical formula: C₅₆H₁₀₉N₃O₆
Lipid 604: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 66H), 1.02 (s, 9H), 0.88 (t, 12H). MS: m/z 948.7. Chemical formula: C₅₈H₁₁₃N₃O₆
Lipid 605: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 68H), 1.02 (s, 9H), 0.88 (t, 12H). MS: m/z 962.7. Chemical formula: C₅₉H₁₁₅N₃O₆
Lipid 606: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.72 (m, 6H), 1.60 (m, 2H), 1.51-1.18 (m, 64H), 1.02 (s, 9H), 0.88 (t, 12H). MS: m/z 934.7. Chemical formula: C₅₇H₁₁₁N₃O₆
Lipid 607: ¹H NMR (500 MHz, CDCl₃, ppm): δ 4.86 (m, 2H), 4.00 (m, 2H), 3.73 (br, 1H), 3.47 (br, 1H), 3.22 (br, 1H), 3.08 (br, 1H), 2.45 (m, 6H), 2.24 (m, 2H), 1.76-1.55 (m, 17H),1.51-1.18 (m, 62H), 1.02 (m, 12H), 0.88 (t, 9H). MS: m/z 920.7. Chemical formula: C₅₆H₁₀₉N₃O₆
Lipid 608: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.89 (m, 1H), 1.72 (m, 6H), 1.60 (m, 2H), 1.51-1.18 (m, 52H), 1.02 (m, 12H), 0.88 (t, 6H), 0.80 (t, 3H). MS: m/z 808.7. Chemical formula: C₄₈H₉₃N₃O₆
Lipid 609: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 60H), 1.02 (m, 12H), 0.88 (t, 9H). MS: m/z 864.7. Chemical formula: C₅₂H₁₀₁N₃O₆
Lipid 610: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 64H), 1.02 (m, 12H), 0.88 (t, 9H). MS: m/z 892.7. Chemical formula: C₅₄H₁₀₅N₃O₆
Lipid 611: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 66H), 1.02 (m, 12H), 0.88 (t, 9H). MS: m/z 906.7. Chemical formula: C₅₅H₁₀₇N₃O₆
Lipid 612: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.89 (m, 1H), 1.72 (m, 6H), 1.60 (m, 2H), 1.51-1.18 (m, 62H), 1.02 (m, 12H), 0.88 (t, 9H). MS: m/z 878.7. Chemical formula: C₅₃H₁₀₃N₃O₆
Lipid 613: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 82H), 1.02 (s, 9H), 0.88 (t, 12H). MS: m/z 1004.7. Chemical formula: C₆₂H₁₂N₃O₆
Lipid 614: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.89 (m, 1H), 1.72 (m, 6H), 1.60 (m, 2H), 1.51-1.18 (m, 64H), 1.02 (s, 9H), 0.88 (t, 9H), 0.80 (t, 3H). MS: m/z 892.7. Chemical formula: C₅₄H₁₀₅N₃O₆
Lipid 615: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 72H), 1.02 (s, 9H), 0.88 (t, 12H). MS: m/z 948.7. Chemical formula: C₅₈H₁₁₃N₃O₆
Lipid 616: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 76H), 1.02 (s, 9H), 0.88 (t, 12H). MS: m/z 976.7. Chemical formula: C₆₀H₁₁₇N₃O₆
Lipid 617: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 78H), 1.02 (s, 9H), 0.88 (t, 12H). MS: m/z 990.7. Chemical formula: C₆₁H₁₁₉N₃O₆
Lipid 618: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.89 (m, 1H), 1.72 (m, 6H), 1.60 (m, 2H), 1.51-1.18 (m, 74H), 1.02 (s, 9H), 0.88 (t, 12H). MS: m/z 962.7. Chemical formula: C₅₉H₁₁₅N₃O₆
Lipid 619: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 4H), 1.89 (m, 1H), 1.72 (m, 6H), 1.60 (m, 2H), 1.51-1.18 (m, 78H), 1.02 (s, 9H), 0.88 (t, 12H). MS: m/z 990.7. Chemical formula: C₆₁H₁₁₉N₃O₆
Lipid 620: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 4H), 2.09 (m, 1H), 1.89 (m, 1H), 1.72 (m, 6H), 1.60 (m, 2H), 1.51-1.18 (m, 60H), 1.02 (s, 9H), 0.88 (t, 9H), 0.80 (t, 3H). MS: m/z 878.7. Chemical formula: C₅₃H₁₀₃N₃O₆
Lipid 621: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 4H), 1.89 (m, 2H), 1.72 (m, 6H), 1.60 (m, 2H), 1.51-1.18 (m, 68H), 1.02 (s, 9H), 0.88 (t, 12H). MS: m/z 934.7. Chemical formula: C₅₇H₁₁₁N₃O₆
Lipid 622: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 4H), 1.89 (m, 2H), 1.72 (m, 6H), 1.60 (m, 2H), 1.51-1.18 (m, 72H), 1.02 (s, 9H), 0.88 (t, 12H). MS: m/z 962.7. Chemical formula: C₅₉H₁₁₅N₃O₆
Lipid 623: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 4H), 1.89 (m, 2H), 1.72 (m, 6H), 1.60 (m, 2H), 1.51-1.18 (m, 74H), 1.02 (s, 9H), 0.88 (t, 12H). MS: m/z 976.7. Chemical formula: C₆₀H₁₁₇N₃O₆
Lipid 624: ¹H NMR (500 MHz, CDCl₃, ppm): δ 4.13 (br, 1H), 4.05 (m, 4H), 3.73 (br, 1H), 3.47 (br, 1H), 3.22 (br, 1H), 3.08 (br, 1H), 2.45 (m, 6H), 2.24 (m, 4H), 2.13 (m, 1H), 1.89 (m, 1H), 1.72 (m, 6H), 1.60 (m, 2H), 1.51-1.18 (m, 70H), 1.02 (s, 9H), 0.88 (t, 12H). MS: m/z 948.7. Chemical formula: C₅₈H₁₁₃N₃O₆
Lipid 625: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 36H), 1.02 (s, 9H), 0.88 (t, 9H). MS: m/z 694.7. Chemical formula: C₄₂H₈₃N₃O₄
Lipid 626: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 48H), 1.02 (s, 9H), 0.88 (t, 9H). MS: m/z 778.7. Chemical formula: C₄₈H₉₅N₃O₄
Lipid 627: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.09 (m, 1H), 1.80 (m, 2H), 1.72 (m, 4H), 1.60 (m, 4H), 1.51-1.18 (m, 34H), 1.02 (s, 9H), 0.88 (t, 6H), 0.80 (t, 3H). MS: m/z 680.7. Chemical formula: C₄₁H₈₁N₃O₄
Lipid 628: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 44H), 1.02 (s, 9H), 0.88 (t, 9H). MS: m/z 750.7. Chemical formula: C₄₆H₉₁N₃O₄
Lipid 629: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 46H), 1.02 (s, 9H), 0.88 (t, 9H). MS: m/z 764.7. Chemical formula: C₄₇H₉₃N₃O₄
Lipid 630: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 2H), 2.28 (m, 1H), 2.24 (m, 6H), 2.18 (m, 2H), 1.80 (m, 2H), 1.72 (m, 4H), 1.60 (m, 4H), 1.51-1.18 (m, 42H), 1.02 (s, 9H), 0.88 (t, 9H). MS: m/z 736.7. Chemical formula: C₄₅H₈₉N₃O₄
Lipid 631: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 44H), 1.02 (s, 9H), 0.88 (t, 9H). MS: m/z 750.7. Chemical formula: C₄₆H₉₁N₃O₄
Lipid 632: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 36H), 1.02 (m, 12H), 0.88 (t, 6H). MS: m/z 694.7. Chemical formula: C₄₂H₈₃N₃O₄
Lipid 633: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 48H), 1.02 (s, 9H), 0.88 (t, 9H). MS: m/z 778.7. Chemical formula: C₄₈H₉₅N₃O₄
Lipid 634: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 2H), 2.24 (m, 6H), 2.18 (m, 2H), 1.93 (m, 1H), 1.84 (m, 2H), 1.65 (m, 4H), 1.51-1.18 (m, 46H), 1.02 (s, 9H), 0.88 (t, 9H). MS: m/z 764.7. Chemical formula: C₄₇H₉₃N₃O₄
Lipid 635: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 38H), 1.02 (s, 9H), 0.88 (t, 9H). MS: m/z 708.7. Chemical formula: C₄₃H₈₅N₃O₄
Lipid 636: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 50H), 1.02 (s, 9H), 0.88 (t, 9H). MS: m/z 792.7. Chemical formula: C₄₉H₉₇N₃O₄
Lipid 637: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.09 (m, 1H), 1.80 (m, 2H), 1.72 (m, 4H), 1.60 (m, 4H), 1.51-1.18 (m, 36H), 1.02 (s, 9H), 0.88 (t, 6H), 0.80 (t, 3H). MS: m/z 694.7. Chemical formula: C₄₂H₈₃N₃O₄
Lipid 638: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 46H), 1.02 (s, 9H), 0.88 (t, 9H). MS: m/z 764.7. Chemical formula: C₄₇H₉₃N₃O₄
Lipid 639: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 48H), 1.02 (s, 9H), 0.88 (t, 9H). MS: m/z 778.7. Chemical formula: C₄₈H₉₅N₃O₄
Lipid 640: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 2H), 2.28 (m, 1H), 2.24 (m, 6H), 2.18 (m, 2H), 1.80 (m, 2H), 1.72 (m, 4H), 1.60 (m, 4H), 1.51-1.18 (m, 44H), 1.02 (s, 9H), 0.88 (t, 9H). MS: m/z 750.7. Chemical formula: C₄₆H₉₁N₃O₄
Lipid 641: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 46H), 1.02 (s, 9H), 0.88 (t, 9H). MS: m/z 764.7. Chemical formula: C₄₇H₉₃N₃O₄
Lipid 642: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 38H), 1.02 (m, 12H), 0.88 (t, 6H). MS: m/z 708.7. Chemical formula: C₄₃H₈₅N₃O₄
Lipid 643: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 50H), 1.02 (s, 9H), 0.88 (t, 9H). MS: m/z 792.7. Chemical formula: C₄₉H₉₇N₃O₄
Lipid 644: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 2H), 2.24 (m, 6H), 2.18 (m, 2H), 1.93 (m, 1H), 1.84 (m, 2H), 1.65 (m, 4H), 1.51-1.18 (m, 48H), 1.02 (s, 9H), 0.88 (t, 9H). MS: m/z 778.7. Chemical formula: C₄₈H₉₅N₃O₄
Lipid 645: ¹H NMR (500 MHz, CDCl₃, ppm): δ 5.90 (m, 1H), 4.81 (m, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 40H), 1.02 (s, 9H), 0.88 (t, 9H). MS: m/z 722.7. Chemical formula: C₄₄H₈₇N₃O₄
Lipid 646: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 52H), 1.02 (s, 9H), 0.88 (t, 9H). MS: m/z 806.7. Chemical formula: C₅₀H₉₉N₃O₄
Lipid 647: ¹H NMR (500 MHz, CDCl₃, ppm): δ 5.88 (m, 1H), 4.91 (m, 1H), 3.97 (m, 1H), 3.82 (m, 1H), 3.69 (br, 1H), 3.44 (br, 1H), 3.25 (br, 1H), 3.08 (br, 1H), 2.45 (m, 4H), 2.24 (m, 6H), 2.18 (m, 2H), 2.09 (m, 1H), 1.80 (m, 2H), 1.72 (m, 4H), 1.60 (m, 4H), 1.51-1.18 (m, 34H), 1.02 (s, 9H), 0.88 (m, 9H). MS: m/z 708.7. Chemical formula: C₄₃H₈₅N₃O₄
Lipid 648: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 48H), 1.02 (s, 9H), 0.88 (t, 9H). MS: m/z 778.7. Chemical formula: C₄₈H₉₅N₃O₄
Lipid 649: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 50H), 1.02 (s, 9H), 0.88 (t, 9H). MS: m/z 792.7. Chemical formula: C₄₉H₉₇N₃O₄
Lipid 650: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 2H), 2.28 (m, 1H), 2.24 (m, 6H), 2.18 (m, 2H), 1.80 (m, 2H), 1.72 (m, 4H), 1.60 (m, 4H), 1.51-1.18 (m, 46H), 1.02 (s, 9H), 0.88 (t, 9H). MS: m/z 764.7. Chemical formula: C₄₇H₉₃N₃O₄
Lipid 651: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 48H), 1.02 (s, 9H), 0.88 (t, 9H). MS: m/z 778.7. Chemical formula: C₄₈H₉₅N₃O₄
Lipid 652: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 40H), 1.02 (m, 12H), 0.88 (t, 6H). MS: m/z 722.7. Chemical formula: C₄₄H₈₇N₃O₄
Lipid 653: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 52H), 1.02 (s, 9H), 0.88 (t, 9H). MS: m/z 7806.7. Chemical formula: C₅₀H₉₉N₃O₄
Lipid 654: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 2H), 2.24 (m, 6H), 2.18 (m, 2H), 1.93 (m, 1H), 1.84 (m, 2H), 1.65 (m, 4H), 1.51-1.18 (m, 52H), 1.02 (s, 9H), 0.88 (t, 9H). MS: m/z 764.7. Chemical formula: C₄₉H₉₉N₃O₄
Lipid 655: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 42H), 1.02 (s, 9H), 0.88 (t, 9H). MS: m/z 694.7. Chemical formula: C₄₅H₈₉N₃O₄
Lipid 656: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 54H), 1.02 (s, 9H), 0.88 (t, 9H). MS: m/z 820.7. Chemical formula: C₅₁H₁₀₁N₃O₄
Lipid 657: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.09 (m, 1H), 1.80 (m, 2H), 1.72 (m, 4H), 1.60 (m, 4H), 1.51-1.18 (m, 40H), 1.02 (s, 9H), 0.88 (t, 6H), 0.80 (t, 3H). MS: m/z 722.7. Chemical formula: C₄₄H₈₇N₃O₄
Lipid 658: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 50H), 1.02 (s, 9H), 0.88 (t, 9H). MS: m/z 792.7. Chemical formula: C₄₉H₉₇N₃O₄
Lipid 659: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 52H), 1.02 (s, 9H), 0.88 (t, 9H). MS: m/z 806.7. Chemical formula: C₅₀H₉₉N₃O₄
Lipid 660: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 2H), 2.28 (m, 1H), 2.24 (m, 6H), 2.18 (m, 2H), 1.80 (m, 2H), 1.72 (m, 4H), 1.60 (m, 4H), 1.51-1.18 (m, 48H), 1.02 (s, 9H), 0.88 (t, 9H). MS: m/z 778.7. Chemical formula: C₄₈H₉₅N₃O₄
Lipid 661: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 50H), 1.02 (s, 9H), 0.88 (t, 9H). MS: m/z 792.7. Chemical formula: C₄₉H₉₇N₃O₄
Lipid 662: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 42H), 1.02 (m, 12H), 0.88 (t, 6H). MS: m/z 736.7. Chemical formula: C₄₅H₈₉N₃O₄
Lipid 663: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 54H), 1.02 (s, 9H), 0.88 (t, 9H). MS: m/z 820.7. Chemical formula: C₅₁H₁₀₁N₃O₄
Lipid 664: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 2H), 2.24 (m, 6H), 2.18 (m, 2H), 1.93 (m, 1H), 1.84 (m, 2H), 1.65 (m, 4H), 1.51-1.18 (m, 52H), 1.02 (s, 9H), 0.88 (t, 9H). MS: m/z 806.7. Chemical formula: C₅₀H₉₉N₃O₄
Lipid 665: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 42H), 1.02 (s, 9H), 0.88 (t, 9H). MS: m/z 750.7. Chemical formula: C₄₆H₉₁N₃O₄
Lipid 666: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 56H), 1.02 (s, 9H), 0.88 (t, 9H). MS: m/z 834.7. Chemical formula: C₅₂H₁₀₃N₃O₄
Lipid 667: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.09 (m, 1H), 1.80 (m, 2H), 1.72 (m, 4H), 1.60 (m, 4H), 1.51-1.18 (m, 42H), 1.02 (s, 9H), 0.88 (t, 6H), 0.80 (t, 3H). MS: m/z 736.7. Chemical formula: C₄₅H₈₉N₃O₄
Lipid 668: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 52H), 1.02 (s, 9H), 0.88 (t, 9H). MS: m/z 750.7. Chemical formula: C₅₀H₉₉N₃O₄
Lipid 669: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 54H), 1.02 (s, 9H), 0.88 (t, 9H). MS: m/z 820.7. Chemical formula: C₅₁H₁₀₁N₃O₄
Lipid 670: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 2H), 2.28 (m, 1H), 2.24 (m, 6H), 2.18 (m, 2H), 1.80 (m, 2H), 1.72 (m, 4H), 1.60 (m, 4H), 1.51-1.18 (m, 50H), 1.02 (s, 9H), 0.88 (t, 9H). MS: m/z 792.7. Chemical formula: C₄₆H₉₇N₃O₄
Lipid 671: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 52H), 1.02 (s, 9H), 0.88 (t, 9H). MS: m/z 806.7. Chemical formula: C₅₀H₉₉N₃O₄
Lipid 672: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 44H), 1.02 (m, 12H), 0.88 (t, 6H). MS: m/z 750.7. Chemical formula: C₄₆H₉₁N₃O₄
Lipid 673: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 3H), 1.72 (m, 4H), 1.60 (m, 4H), 1.51-1.18 (m, 56H), 1.02 (s, 9H), 0.88 (t, 9H). MS: m/z 834.7. Chemical formula: C₅₂H₁₀₃N₃O₄
Lipid 674, 230307-E10: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 2H), 2.24 (m, 6H), 2.18 (m, 2H), 1.93 (m, 1H), 1.84 (m, 2H), 1.65 (m, 4H), 1.51-1.18 (m, 54H), 1.02 (s, 9H), 0.88 (t, 9H). MS: m/z 820.7. Chemical formula: C₅₁H₁₀₁N₃O₄
Lipid 675: ¹H NMR (500 MHz, CDCl₃, ppm): δ 5.92 (m, 1H), 5.34 (m, 2H), 4.81 (m, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.00 (m, 4H), 1.80 (m, 2H), 1.72 (m, 4H), 1.60 (m, 4H), 1.51-1.18 (m, 40H), 1.02 (s, 9H), 0.88 (t, 9H). MS: m/z 776.7. Chemical formula: C₄₈H₉₃N₃O₄
Lipid 676: ¹H NMR (500 MHz, CDCl₃, ppm): δ 5.34 (m, 2H), 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.00 (m, 4H), 1.80 (m, 2H), 1.72 (m, 4H), 1.60 (m, 4H), 1.51-1.18 (m, 52H), 1.02 (s, 9H), 0.88 (t, 9H). MS: m/z 860.7. Chemical formula: C₅₄H₁₀₅N₃O₄
Lipid 677: ¹H NMR (500 MHz, CDCl₃, ppm): δ 5.88 (m, 1H), 5.34 (m, 2H), 4.91 (m, 1H), 3.97 (m, 1H), 3.82 (m, 1H), 3.69 (br, 1H), 3.44 (br, 1H), 3.25 (br, 1H), 3.08 (br, 1H), 2.45 (m, 4H), 2.24 (m, 6H), 2.18 (m, 2H), 2.09 (m, 5H), 1.80 (m, 2H), 1.72 (m, 4H), 1.60 (m, 4H), 1.51-1.18 (m, 36H), 1.02 (s, 9H), 0.88 (m, 9H). MS: m/z 762.7. Chemical formula: C₄₇H₉₁N₃O₄
Lipid 678: ¹H NMR (500 MHz, CDCl₃, ppm): δ 5.34 (m, 2H), 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.00 (m, 4H), 1.80 (m, 3H), 1.72 (m, 4H), 1.60 (m, 4H), 1.51-1.18 (m, 48H), 1.02 (s, 9H), 0.88 (t, 9H). MS: m/z 832.7. Chemical formula: C₅₂H₁₀₁N₃O₄
Lipid 679: ¹H NMR (500 MHz, CDCl₃, ppm): δ 5.34 (m, 2H), 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.00 (m, 4H), 1.80 (m, 3H), 1.72 (m, 4H), 1.60 (m, 4H), 1.51-1.18 (m, 50H), 1.02 (s, 9H), 0.88 (t, 9H). MS: m/z 846.7. Chemical formula: C₅₃H₁₀₃N₃O₄
Lipid 680: ¹H NMR (500 MHz, CDCl₃, ppm): δ 5.34 (m, 2H), 4.13 (br, 1H), 4.05 (m, 2H), 3.73 (br, 1H), 3.47 (br, 1H), 3.22 (br, 1H), 3.08 (br, 1H), 2.45 (m, 2H), 2.28 (m, 1H), 2.24 (m, 6H), 2.18 (m, 2H), 2.00 (m, 4H), 1.80 (m, 2H), 1.72 (m, 4H), 1.60 (m, 4H), 1.51-1.18 (m, 46H), 1.02 (s, 9H), 0.88 (t, 9H). MS: m/z 818.7. Chemical formula: C₅₁H₉₉N₃O₄
Lipid 681: ¹H NMR (500 MHz, CDCl₃, ppm): δ 5.34 (m, 2H), 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.00 (m, 4H), 1.80 (m, 2H), 1.72 (m, 4H), 1.60 (m, 4H), 1.51-1.18 (m, 48H), 1.02 (s, 9H), 0.88 (t, 9H). MS: m/z 832.7. Chemical formula: C₅₂H₁₀₁N₃O₄
Lipid 682: ¹H NMR (500 MHz, CDCl₃, ppm): δ 5.34 (m, 2H), 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.00 (m, 4H), 1.80 (m, 3H), 1.72 (m, 4H), 1.60 (m, 4H), 1.51-1.18 (m, 40H), 1.02 (m, 12H), 0.88 (t, 6H). MS: m/z 776.7. Chemical formula: C₄₈H₉₃N₃O₄
Lipid 683: ¹H NMR (500 MHz, CDCl₃, ppm): δ 5.34 (m, 2H), 4.89 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.00 (m, 4H), 1.80 (m, 3H), 1.72 (m, 4H), 1.60 (m, 4H), 1.51-1.18 (m, 52H), 1.02 (s, 9H), 0.88 (t, 9H). MS: m/z 860.7. Chemical formula: C₅₄H₁₀₅N₃O₄
Lipid 684: ¹H NMR (500 MHz, CDCl₃, ppm): δ 5.34 (m, 2H), 4.13 (br, 1H), 4.05 (m, 2H), 3.73 (br, 1H), 3.47 (br, 1H), 3.22 (br, 1H), 3.08 (br, 1H), 2.45 (m, 2H), 2.24 (m, 6H), 2.18 (m, 2H), 2.00 (m, 4H), 1.93 (m, 1H), 1.84 (m, 2H), 1.65 (m, 4H), 1.51-1.18 (m, 50H), 1.02 (s, 9H), 0.88 (t, 9H). MS: m/z 846.7. Chemical formula: C₅₃H₁₀₃N₃O₄
Lipid 685: ¹H NMR (500 MHz, CDCl₃, ppm): δ 5.89 (m, 1H), 5.34 (m, 4H), 4.81 (m, 1H), 3.73 (br, 1H), 3.47 (br, 1H), 3.22 (br, 1H), 3.08 (br, 1H), 2.80 (m, 2H), 2.45 (m, 4H), 2.24 (m, 6H), 2.18 (m, 2H), 2.00 (m, 4H), 1.80 (m, 2H), 1.72 (m, 4H), 1.60 (m, 4H), 1.51-1.18 (m, 34H), 1.02 (s, 9H), 0.88 (t, 9H). MS: m/z 774.7. Chemical formula: C₄₈H₉₁N₃O₄
Lipid 686: ¹H NMR (500 MHz, CDCl₃, ppm): δ 5.34 (m, 4H), 4.89 (m, 1H), 4.13 (br, 1H), 3.73 (br, 1H), 3.47 (br, 1H), 3.22 (br, 1H), 3.08 (br, 1H), 2.80 (m, 2H), 2.45 (m, 4H), 2.24 (m, 6H), 2.18 (m, 2H), 2.00 (m, 4H), 1.80 (m, 2H), 1.72 (m, 4H), 1.60 (m, 4H), 1.51-1.18 (m, 46H), 1.02 (s, 9H), 0.88 (t, 9H). MS: m/z 858.7. Chemical formula: C₅₄H₁₀₃N₃O₄
Lipid 687: ¹H NMR (500 MHz, CDCl₃, ppm): δ 5.88 (m, 1H), 5.34 (m, 4H), 4.91 (m, 1H), 3.97 (m, 1H), 3.82 (m, 1H), 3.69 (br, 1H), 3.44 (br, 1H), 3.25 (br, 1H), 3.08 (br, 1H), 2.80 (m, 2H), 2.45 (m, 4H), 2.24 (m, 6H), 2.18 (m, 2H), 2.09 (m, 5H), 1.80 (m, 2H), 1.72 (m, 4H), 1.60 (m, 4H), 1.51-1.18 (m, 30H), 1.02 (s, 9H), 0.88 (m, 9H). MS: m/z 760.7. Chemical formula: C₄₇H₈₉N₃O₄
Lipid 688: ¹H NMR (500 MHz, CDCl₃, ppm): δ 5.34 (m, 4H), 4.89 (m, 2H), 4.13 (br, 1H), 3.73 (br, 1H), 3.47 (br, 1H), 3.22 (br, 1H), 3.08 (br, 1H), 2.80 (m, 2H), 2.45 (m, 4H), 2.24 (m, 6H), 2.18 (m, 2H), 2.00 (m, 4H), 1.80 (m, 3H), 1.72 (m, 4H), 1.60 (m, 4H), 1.51-1.18 (m, 42H), 1.02 (s, 9H), 0.88 (t, 9H). MS: m/z 830.7. Chemical formula: C₅₂H₉₉N₃O₄
Lipid 689: ¹H NMR (500 MHz, CDCl₃, ppm): δ 5.34 (m, 4H), 4.89 (m, 2H), 4.13 (br, 1H), 3.73 (br, 1H), 3.47 (br, 1H), 3.22 (br, 1H), 3.08 (br, 1H), 2.80 (m, 2H), 2.45 (m, 4H), 2.24 (m, 6H), 2.18 (m, 2H), 2.00 (m, 4H), 1.80 (m, 3H), 1.72 (m, 4H), 1.60 (m, 4H), 1.51-1.18 (m, 44H), 1.02 (s, 9H), 0.88 (t, 9H). MS: m/z 844.7. Chemical formula: C₅₃H₁₀₁N₃O₄
Lipid 690: ¹H NMR (500 MHz, CDCl₃, ppm): δ 5.34 (m, 4H), 4.13 (br, 1H), 4.05 (m, 2H), 3.73 (br, 1H), 3.47 (br, 1H), 3.22 (br, 1H), 3.08 (br, 1H), 2.80 (m, 2H), 2.45 (m, 2H), 2.28 (m, 1H), 2.24 (m, 6H), 2.18 (m, 2H), 2.00 (m, 4H), 1.80 (m, 2H), 1.72 (m, 4H), 1.60 (m, 4H), 1.51-1.18 (m, 40H), 1.02 (s, 9H), 0.88 (t, 9H). MS: m/z 816.7. Chemical formula: C₅₁H₉₇N₃O₄
Lipid 691: ¹H NMR (500 MHz, CDCl₃, ppm): δ 5.34 (m, 4H), 4.89 (m, 1H), 4.13 (br, 1H), 3.73 (br, 1H), 3.47 (br, 1H), 3.22 (br, 1H), 3.08 (br, 1H), 2.80 (m, 2H), 2.45 (m, 4H), 2.24 (m, 6H), 2.18 (m, 2H), 2.00 (m, 4H), 1.80 (m, 2H), 1.72 (m, 4H), 1.60 (m, 4H), 1.51-1.18 (m, 42H), 1.02 (s, 9H), 0.88 (t, 9H). MS: m/z 830.7. Chemical formula: C₅₂H₉₉N₃O₄
Lipid 692: ¹H NMR (500 MHz, CDCl₃, ppm): δ 5.34 (m, 4H), 4.89 (m, 2H), 4.13 (br, 1H), 3.73 (br, 1H), 3.47 (br, 1H), 3.22 (br, 1H), 3.08 (br, 1H), 2.80 (m, 2H), 2.45 (m, 4H), 2.24 (m, 6H), 2.18 (m, 2H), 2.00 (m, 4H), 1.80 (m, 3H), 1.72 (m, 4H), 1.60 (m, 4H), 1.51-1.18 (m, 34H), 1.02 (m, 12H), 0.88 (t, 6H). MS: m/z 774.7. Chemical formula: C₄₈H₉₁N₃O₄
Lipid 693: ¹H NMR (500 MHz, CDCl₃, ppm): δ 5.34 (m, 4H), 4.89 (m, 2H), 4.13 (br, 1H), 3.73 (br, 1H), 3.47 (br, 1H), 3.22 (br, 1H), 3.08 (br, 1H), 2.80 (m, 2H), 2.45 (m, 4H), 2.24 (m, 6H), 2.18 (m, 2H), 2.00 (m, 4H), 1.80 (m, 3H), 1.72 (m, 4H), 1.60 (m, 4H), 1.51-1.18 (m, 46H), 1.02 (s, 9H), 0.88 (t, 9H). MS: m/z 858.7. Chemical formula: C₅₄H₁₀₃N₃O₄
Lipid 694: ¹H NMR (500 MHz, CDCl₃, ppm): δ 5.34 (m, 4H), 4.13 (br, 1H), 4.05 (m, 2H), 3.73 (br, 1H), 3.47 (br, 1H), 3.22 (br, 1H), 3.08 (br, 1H), 2.80 (m, 2H), 2.45 (m, 2H), 2.24 (m, 6H), 2.18 (m, 2H), 2.00 (m, 4H), 1.93 (m, 1H), 1.84 (m, 2H), 1.65 (m, 4H), 1.51-1.18 (m, 44H), 1.02 (s, 9H), 0.88 (t, 9H). MS: m/z 844.7. Chemical formula: C₅₃H₁₀₁N₃O₄
Lipid 765: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 6H), 2.24 (m, 2H), 1.72 (m, 6H), 1.60 (m, 2H), 1.51-1.18 (m, 56H), 1.02 (s, 9H), 0.88 (t, 12H). MS: m/z 864.7. Chemical formula: C₅₂H₁₀₁N₃O₆
Lipid 766: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 6H), 2.24 (m, 2H), 1.72 (m, 6H), 1.60 (m, 2H), 1.51-1.18 (m, 68H), 1.02 (s, 9H), 0.88 (t, 12H). MS: m/z 948.7. Chemical formula: C₅₈H₁₁₃N₃O₆
Lipid 767: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.72 (m, 6H), 1.60 (m, 2H), 1.51-1.18 (m, 52H), 1.02 (s, 9H), 0.88 (t, 9H), 0.80 (t, 3H). MS: m/z 850.7. Chemical formula: C₅₁H₉₉N₃O₆
Lipid 768: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 62H), 1.02 (s, 9H), 0.88 (t, 12H). MS: m/z 920.7. Chemical formula: C₅₆H₁₀₉N₃O₆
Lipid 769: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 64H), 1.02 (s, 9H), 0.88 (t, 12H). MS: m/z 934.7. Chemical formula: C₅₇H₁₁₁N₃O₆
Lipid 770: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 3H), 1.72 (m, 6H), 1.60 (m, 2H), 1.51-1.18 (m, 60H), 1.02 (s, 9H), 0.88 (t, 12H). MS: m/z 906.7. Chemical formula: C₅₅H₁₀₇N₃O₆
Lipid 771: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.45 (m, 6H), 2.24 (m, 2H), 1.72 (m, 6H), 1.60 (m, 2H), 1.51-1.18 (m, 60H), 1.02 (s, 9H), 0.88 (t, 12H). MS: m/z 892.7. Chemical formula: C₅₄H₁₀₅N₃O₆
Lipid 772: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 54H), 1.02 (m, 12H), 0.88 (t, 9H). MS: m/z 836.7. Chemical formula: C₅₀H₉₇N₃O₆
Lipid 773: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 66H), 1.02 (m, 12H), 0.88 (t, 9H). MS: m/z 892.7. Chemical formula: C₅₄H₁₀₅N₃O₆
Lipid 774: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.89 (m, 1H), 1.72 (m, 6H), 1.60 (m, 2H), 1.51-1.18 (m, 50H), 1.02 (m, 12H), 0.88 (t, 6H), 0.80 (t, 3H). MS: m/z 822.7. Chemical formula: C₄₉H₉₅N₃O₆
Lipid 775: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 60H), 1.02 (m, 12H), 0.88 (t, 9H). MS: m/z 892.7. Chemical formula: C₅₄H₁₀₅N₃O₆
Lipid 776: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 62H), 1.02 (m, 12H), 0.88 (t, 9H). MS: m/z 906.7. Chemical formula: C₅₅H₁₀₇N₃O₆
Lipid 777: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 3H), 1.89 (m, 1H), 1.72 (m, 6H), 1.60 (m, 2H), 1.51-1.18 (m, 58H), 1.02 (m, 12H), 0.88 (t, 9H). MS: m/z 878.7. Chemical formula: C₅₃H₁₀₃N₃O₆
Lipid 778: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 58H), 1.02 (m, 12H), 0.88 (t, 9H). MS: m/z 864.7. Chemical formula: C₅₂H₁₀₁N₃O₆
Lipid 779: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 62H), 1.02 (s, 9H), 0.88 (t, 12H). MS: m/z 920.7. Chemical formula: C₅₆H₁₀₉N₃O₆
Lipid 780: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 74H), 1.02 (s, 9H), 0.88 (t, 12H). MS: m/z 1004.7. Chemical formula: C₆₂H₁₂₁N₃O₆
Lipid 781: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 2.09 (m, 1H), 1.89 (m, 1H), 1.72 (m, 6H), 1.60 (m, 2H), 1.51-1.18 (m, 58H), 1.02 (s, 9H), 0.88 (t, 9H), 0.80 (t, 3H). MS: m/z 906.7. Chemical formula: C₅₅H₁₀₇N₃O₆
Lipid 782: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 68H), 1.02 (s, 9H), 0.88 (t, 12H). MS: m/z 976.7. Chemical formula: C₆₀H₁₁₇N₃O₆
Lipid 783: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 2H), 1.72 (m, 6H), 1.60 (m, 2H), 1.51-1.18 (m, 70H), 1.02 (s, 9H), 0.88 (t, 12H). MS: m/z 990.7. Chemical formula: C₆₁H₁₁₉N₃O₆
Lipid 784: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 3H), 1.89 (m, 1H), 1.72 (m, 6H), 1.60 (m, 2H), 1.51-1.18 (m, 66H), 1.02 (s, 9H), 0.88 (t, 12H). MS: m/z 962.7. Chemical formula: C₅₉H₁₁₅N₃O₆
Lipid 785: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.45 (m, 6H), 2.24 (m, 2H), 1.89 (m, 1H), 1.72 (m, 6H), 1.60 (m, 2H), 1.51-1.18 (m, 66H), 1.02 (s, 9H), 0.88 (t, 12H). MS: m/z 948.7. Chemical formula: C₅₈H₁₁₃N₃O₆
Lipid 786: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.72 (m, 6H), 1.60 (m, 2H), 1.51-1.18 (m, 52H), 1.02 (s, 9H), 0.88 (t, 12H). MS: m/z 850.7. Chemical formula: C₅₁H₉₉N₃O₆
Lipid 787: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.72 (m, 6H), 1.60 (m, 2H), 1.51-1.18 (m, 64H), 1.02 (s, 9H), 0.88 (t, 12H). MS: m/z 934.7. Chemical formula: C₅₇H₁₁₁N₃O₆
Lipid 788: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 2H), 1.72 (m, 6H), 1.60 (m, 2H), 1.51-1.18 (m, 48H), 1.02 (s, 9H), 0.88 (t, 9H), 0.80 (t, 3H). MS: m/z 836.7. Chemical formula: C₅₀H₉₇N₃O₆
Lipid 789: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.89 (m, 1H), 1.72 (m, 6H), 1.60 (m, 2H), 1.51-1.18 (m, 58H), 1.02 (s, 9H), 0.88 (t, 12H). MS: m/z 906.7. Chemical formula: C₅₅H₁₀₇N₃O₆
Lipid 790: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.89 (m, 1H), 1.72 (m, 6H), 1.60 (m, 2H), 1.51-1.18 (m, 60H), 1.02 (s, 9H), 0.88 (t, 12H). MS: m/z 920.7. Chemical formula: C₅₆H₁₀₉N₃O₆
Lipid 791: ¹H NMR (500 MHz, CDCl₃, ppm): δ 4.13 (br, 1H), 4.05 (m, 4H), 3.73 (br, 1H), 3.47 (br, 1H), 3.22 (br, 1H), 3.08 (br, 1H), 2.45 (m, 6H), 2.24 (m, 3H), 2.13 (m, 1H), 1.72 (m, 6H), 1.60 (m, 2H), 1.51-1.18 (m, 56H), 1.02 (s, 9H), 0.88 (t, 12H). MS: m/z 892.7. Chemical formula: C₅₄H₁₀₅N₃O₆
Lipid 792: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.45 (m, 6H), 2.24 (m, 2H), 2.13 (m, 1H), 1.72 (m, 6H), 1.60 (m, 2H), 1.51-1.18 (m, 56H), 1.02 (s, 9H), 0.88 (t, 12H). MS: m/z 878.7. Chemical formula: C₅₃H₁₀₃N₃O₆
Lipid 793: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.68 (m, 4H), 2.24 (m, 2H), 1.72 (m, 6H), 1.60 (m, 2H), 1.51-1.18 (m, 56H), 1.08 (s, 6H), 1.02 (s, 9H), 0.88 (t, 12H). MS: m/z 892.7. Chemical formula: C₅₄H₁₀₅N₃O₆
Lipid 794: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.68 (m, 4H), 2.24 (m, 2H), 1.72 (m, 6H), 1.60 (m, 2H), 1.51-1.18 (m, 68H), 1.08 (s, 6H), 1.02 (s, 9H), 0.88 (t, 12H). MS: m/z 976.7. Chemical formula: C₆₀H₁₁₇N₃O₆
Lipid 795: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.68 (m, 4H), 2.24 (m, 2H), 2.09 (m, 1H), 1.72 (m, 6H), 1.60 (m, 2H), 1.51-1.18 (m, 52H), 1.08 (s, 6H), 1.02 (s, 9H), 0.88 (t, 9H), 0.80 (t, 3H). MS: m/z 878.7. Chemical formula: C₅₃H₁₀₃N₃O₆
Lipid 796: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.68 (m, 4H), 2.24 (m, 2H), 1.89 (m, 1H), 1.72 (m, 6H), 1.60 (m, 2H), 1.51-1.18 (m, 62H), 1.08 (s, 6H), 1.02 (s, 9H), 0.88 (t, 12H). MS: m/z 948.7. Chemical formula: C₅₈H₁₁₃N₃O₆
Lipid 797: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.68 (m, 4H), 2.24 (m, 2H), 1.89 (m, 1H), 1.72 (m, 6H), 1.60 (m, 2H), 1.51-1.18 (m, 64H), 1.08 (s, 6H), 1.02 (s, 9H), 0.88 (t, 12H). MS: m/z 962.7. Chemical formula: C₅₉H₁₁₅N₃O₆
Lipid 798: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.68 (m, 4H), 2.24 (m, 3H), 1.72 (m, 6H), 1.60 (m, 2H), 1.51-1.18 (m, 60H), 1.08 (s, 6H), 1.02 (s, 9H), 0.88 (t, 12H). MS: m/z 920.7. Chemical formula: C₅₆H₁₀₉N₃O₆
Lipid 799: ¹H NMR (500 MHz, CDCl₃, ppm): δ 4.89 (m, 2H), 4.13 (br, 1H), 3.73 (br, 1H), 3.47 (br, 1H), 3.22 (br, 1H), 3.08 (br, 1H), 2.68 (m, 4H), 2.24 (m, 2H), 1.72 (m, 6H), 1.60 (m, 2H), 1.51-1.18 (m, 60H), 1.08 (s, 6H), 1.02 (s, 9H), 0.88 (t, 12H). MS: m/z 920.7. Chemical formula: C₅₆H₁₀₉N₃O₆
Lipid 800: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.68 (m, 4H), 2.24 (m, 2H), 1.89 (m, 1H), 1.72 (m, 6H), 1.60 (m, 2H), 1.51-1.18 (m, 54H), 1.08 (s, 6H), 1.02 (m, 12H), 0.88 (t, 9H). MS: m/z 864.7. Chemical formula: C₅₂H₁₀₁N₃O₆
Lipid 801: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.68 (m, 4H), 2.24 (m, 2H), 1.89 (m, 1H), 1.72 (m, 6H), 1.60 (m, 2H), 1.51-1.18 (m, 66H), 1.08 (s, 6H), 1.02 (m, 12H), 0.88 (t, 9H). MS: m/z 920.7. Chemical formula: C₅₆H₁₀₉N₃O₆
Lipid 802: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.68 (m, 4H), 2.24 (m, 2H), 2.09 (m, 1H), 1.89 (m, 1H), 1.72 (m, 6H), 1.60 (m, 2H), 1.51-1.18 (m, 50H), 1.08 (s, 6H), 1.02 (m, 12H), 0.88 (t, 6H), 0.80 (t, 3H). MS: m/z 850.7. Chemical formula: C₅₁H₉₉N₃O₆
Lipid 803: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.68 (m, 4H), 2.24 (m, 2H), 1.89 (m, 2H), 1.72 (m, 6H), 1.60 (m, 2H), 1.51-1.18 (m, 60H), 1.08 (s, 6H), 1.02 (m, 12H), 0.88 (t, 9H). MS: m/z 920.7. Chemical formula: C₅₆H₁₀₉N₃O₆
Lipid 804: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.68 (m, 4H), 2.24 (m, 2H), 1.89 (m, 2H), 1.72 (m, 6H), 1.60 (m, 2H), 1.51-1.18 (m, 62H), 1.08 (s, 6H), 1.02 (m, 12H), 0.88 (t, 9H). MS: m/z 934.7. Chemical formula: C₅₇H₁₁₁N₃O₆
Lipid 805: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.68 (m, 4H), 2.24 (m, 3H), 1.89 (m, 1H), 1.72 (m, 6H), 1.60 (m, 2H), 1.51-1.18 (m, 58H), 1.08 (s, 6H), 1.02 (m, 12H), 0.88 (t, 9H). MS: m/z 906.7. Chemical formula: C₅₅H₁₀₇N₃O₆
Lipid 806: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.68 (m, 4H), 2.24 (m, 2H), 1.89 (m, 1H), 1.72 (m, 6H), 1.60 (m, 2H), 1.51-1.18 (m, 58H), 1.08 (s, 6H), 1.02 (m, 12H), 0.88 (t, 9H). MS: m/z 892.7. Chemical formula: C₅₄H₁₀₅N₃O₆
Lipid 807: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.68 (m, 4H), 2.24 (m, 2H), 1.89 (m, 1H), 1.72 (m, 6H), 1.60 (m, 2H), 1.51-1.18 (m, 62H), 1.08 (s, 6H), 1.02 (s, 9H), 0.88 (t, 12H). MS: m/z 948.7. Chemical formula: C₅₈H₁₁₃N₃O₆
Lipid 808: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.68 (m, 4H), 2.24 (m, 2H), 1.89 (m, 1H), 1.72 (m, 6H), 1.60 (m, 2H), 1.51-1.18 (m, 74H), 1.08 (s, 6H), 1.02 (s, 9H), 0.88 (t, 12H). MS: m/z 1032.7. Chemical formula: C₆₄H₁₂₅N₃O₆
Lipid 809: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.68 (m, 4H), 2.24 (m, 2H), 2.09 (m, 1H), 1.89 (m, 1H), 1.72 (m, 6H), 1.60 (m, 2H), 1.51-1.18 (m, 58H), 1.08 (s, 6H), 1.02 (s, 9H), 0.88 (t, 9H), 0.80 (t, 3H). MS: m/z 934.7. Chemical formula: C₅₇H₁₁₁N₃O₆
Lipid 810: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.68 (m, 4H), 2.24 (m, 2H), 1.89 (m, 2H), 1.72 (m, 6H), 1.60 (m, 2H), 1.51-1.18 (m, 68H), 1.08 (s, 6H), 1.02 (s, 9H), 0.88 (t, 12H). MS: m/z 1004.7. Chemical formula: C₆₂H₁₂₁N₃O₆
Lipid 811: ¹H NMR (500 MHz, CDCl₃, ppm): δ 4.89 (m, 4H), 4.13 (br, 1H), 3.73 (br, 1H), 3.47 (br, 1H), 3.22 (br, 1H), 3.08 (br, 1H), 2.68 (m, 4H), 2.24 (m, 2H), 1.89 (m, 2H), 1.72 (m, 6H), 1.60 (m, 2H), 1.51-1.18 (m, 70H), 1.08 (s, 6H), 1.02 (s, 9H), 0.88 (t, 12H). MS: m/z 1018.7. Chemical formula: C₆₃H₁₂₃N₃O₆
Lipid 812: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.68 (m, 4H), 2.24 (m, 3H), 1.89 (m, 1H), 1.72 (m, 6H), 1.60 (m, 2H), 1.51-1.18 (m, 66H), 1.08 (s, 6H), 1.02 (s, 9H), 0.88 (t, 12H). MS: m/z 990.7. Chemical formula: C₆₁H₁₁₉N₃O₆
Lipid 813: ¹H NMR (500 MHz, CDCl₃, ppm): δ 4.89 (m, 3H), 4.13 (br, 1H), 3.73 (br, 1H), 3.47 (br, 1H), 3.22 (br, 1H), 3.08 (br, 1H), 2.68 (m, 4H), 2.24 (m, 2H), 1.89 (m, 1H), 1.72 (m, 6H), 1.60 (m, 2H), 1.51-1.18 (m, 66H), 1.08 (s, 6H), 1.02 (s, 9H), 0.88 (t, 12H). MS: m/z 976.7. Chemical formula: C₆₀H₁₁₇N₃O₆
Lipid 814: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.68 (m, 4H), 2.24 (m, 2H), 2.13 (m, 1H), 1.72 (m, 6H), 1.60 (m, 2H), 1.51-1.18 (m, 52H), 1.08 (s, 6H), 1.02 (s, 9H), 0.88 (t, 12H). MS: m/z 878.7. Chemical formula: C₅₃H₁₀₃N₃O₆
Lipid 815: ¹H NMR (500 MHz, CDCl₃, ppm): δ 4.89 (m, 1H), 4.13 (br, 1H), 4.05 (m, 2H), 3.73 (br, 1H), 3.47 (br, 1H), 3.22 (br, 1H), 3.08 (br, 1H), 2.68 (m, 4H), 2.24 (m, 2H), 2.13 (m, 1H), 1.72 (m, 6H), 1.60 (m, 2H), 1.51-1.18 (m, 64H), 1.08 (s, 6H), 1.02 (s, 9H), 0.88 (t, 12H). MS: m/z 962.7. Chemical formula: C₅₉H₁₁₅N₃O₆
Lipid 816: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.68 (m, 4H), 2.24 (m, 2H), 2.13 (m, 2H), 1.72 (m, 6H), 1.60 (m, 2H), 1.51-1.18 (m, 48H), 1.08 (s, 6H), 1.02 (s, 9H), 0.88 (t, 9H), 0.80 (t, 3H). MS: m/z 864.7. Chemical formula: C₅₂H₁₀₁N₃O₆
Lipid 817: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.68 (m, 4H), 2.24 (m, 2H), 2.13 (m, 1H), 1.89 (m, 1H), 1.72 (m, 6H), 1.60 (m, 2H), 1.51-1.18 (m, 58H), 1.08 (s, 6H), 1.02 (s, 9H), 0.88 (t, 12H). MS: m/z 934.7. Chemical formula: C₅₇H₁₁₁N₃O₆
Lipid 818: ¹H NMR (500 MHz, CDCl₃, ppm): δ 4.89 (m, 2H), 4.13 (br, 1H), 4.05 (m, 2H), 3.73 (br, 1H), 3.47 (br, 1H), 3.22 (br, 1H), 3.08 (br, 1H), 2.68 (m, 4H), 2.24 (m, 2H), 2.13 (m, 1H), 1.89 (m, 1H), 1.72 (m, 6H), 1.60 (m, 2H), 1.51-1.18 (m, 60H), 1.08 (s, 6H), 1.02 (s, 9H), 0.88 (t, 12H). MS: m/z 948.7. Chemical formula: C₅₈H₁₁₃N₃O₆
Lipid 819: ¹H NMR (500 MHz, CDCl₃, ppm): δ 4.13 (br, 1H), 4.05 (m, 4H), 3.73 (br, 1H), 3.47 (br, 1H), 3.22 (br, 1H), 3.08 (br, 1H), 2.68 (m, 4H), 2.24 (m, 3H), 2.13 (m, 1H), 1.72 (m, 6H), 1.60 (m, 2H), 1.51-1.18 (m, 56H), 1.08 (s, 6H), 1.02 (s, 9H), 0.88 (t, 12H). MS: m/z 920.7. Chemical formula: C₅₆H₁₀₉N₃O₆
Lipid 820: ¹H NMR (500 MHz, CDCl₃, ppm): δ 4.89 (m, 1H), 4.05 (m, 2H), 4.13 (br, 1H), 3.73 (br, 1H), 3.47 (br, 1H), 3.22 (br, 1H), 3.08 (br, 1H), 2.68 (m, 4H), 2.24 (m, 2H), 2.13 (m, 1H), 1.72 (m, 6H), 1.60 (m, 2H), 1.51-1.18 (m, 56H), 1.08 (s, 6H), 1.02 (s, 9H), 0.88 (t, 12H). MS: m/z 906.7. Chemical formula: C₅₅H₁₀₇N₃O₆
Lipid 869: ¹H NMR (500 MHz, CDCl₃, ppm): δ 5.60 (m, 2H), 4.89 (m, 1H), 4.69 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.00 (m, 4H), 1.60 (m, 4H), 1.51-1.18 (m, 48H), 1.02 (s, 9H), 0.88 (t, 9H). MS: m/z 834.7. Chemical formula: C₅₀H₉₅N₃O₆
Lipid 870: ¹H NMR (500 MHz, CDCl₃, ppm): δ 4.89 (m, 1H), 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.00 (m, 4H), 1.60 (m, 4H), 1.51-1.18 (m, 40H), 1.02 (s, 9H), 0.88 (t, 12H). MS: m/z 836.7. Chemical formula: C₅₀H₉₇N₃O₆
Lipid 871: ¹H NMR (500 MHz, CDCl₃, ppm): δ 4.89 (m, 1H), 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.00 (m, 4H), 1.60 (m, 4H), 1.51-1.18 (m, 40H), 1.02 (m, 12H), 0.88 (t, 9H). MS: m/z 836.7. Chemical formula: C₅₀H₉₇N₃O₆
Lipid 872: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 40H), 1.02 (s, 9H), 0.88 (t, 9H). MS: m/z 722.7. Chemical formula: C₄₄H₈₇N₃O₄
Lipid 873: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 42H), 1.02 (s, 9H), 0.88 (t, 9H). MS: m/z 736.7. Chemical formula: C₄₅H₈₉N₃O₄
Lipid 874: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 44H), 1.02 (s, 9H), 0.88 (t, 9H). MS: m/z 750.7. Chemical formula: C₄₆H₉₁N₃O₄
Lipid 875: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 46H), 1.02 (s, 9H), 0.88 (t, 9H). MS: m/z 764.7. Chemical formula: C₄₇H₉₃N₃O₄
Lipid 876: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.80 (m, 2H), 1.72 (m, 4H), 1.60 (m, 4H), 1.51-1.18 (m, 48H), 1.02 (s, 9H), 0.88 (t, 9H). MS: m/z 778.7. Chemical formula: C₄₈H₉₅N₃O₆
Lipid 877: ¹H NMR (500 MHz, CDCl₃, ppm): δ 5.34 (m, 2H), 4.89 (m, 1H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.00 (m, 4H), 1.80 (m, 2H), 1.72 (m, 4H), 1.60 (m, 4H), 1.51-1.18 (m, 44H), 1.02 (s, 9H), 0.88 (t, 9H). MS: m/z 804.7. Chemical formula: C₅₀H₉₇N₃O₄
Lipid 878: ¹H NMR (500 MHz, CDCl₃, ppm): δ 5.34 (m, 4H), 4.89 (m, 1H), 4.13 (br, 1H), 3.73 (br, 1H), 3.47 (br, 1H), 3.22 (br, 1H), 3.08 (br, 1H), 2.80 (m, 2H), 2.45 (m, 4H), 2.24 (m, 6H), 2.18 (m, 2H), 2.00 (m, 4H), 1.80 (m, 2H), 1.72 (m, 4H), 1.60 (m, 4H), 1.51-1.18 (m, 38H), 1.02 (s, 9H), 0.88 (t, 9H). MS: m/z 802.7. Chemical formula: C₅₀H₉₅N₃O₄
Lipid 879: ¹H NMR (500 MHz, CDCl₃, ppm): δ 5.60 (m, 2H), 4.69 (m, 2H), 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.00 (m, 4H), 1.60 (m, 4H), 1.51-1.18 (m, 39H), 1.02 (s, 9H), 0.88 (t, 9H). MS: m/z 778.7. Chemical formula: C₄₆H₈₇N₃O₆
Lipid 880: ¹H NMR (500 MHz, CDCl₃, ppm): δ 4.23 (m, 4H), 4.13 (br, 1H), 3.73 (br, 1H), 3.47 (br, 1H), 3.22 (br, 1H), 3.08 (br, 1H), 2.45 (m, 6H), 2.24 (m, 6H), 2.00 (m, 2H), 1.88 (m, 4H), 1.65 (m, 6H), 1.51-1.18 (m, 34H), 1.02 (s, 9H), 0.88 (t, 12H). MS: m/z 780.7. Chemical formula: C₄₆H₈₉N₃O₆
Lipid 881: ¹H NMR (500 MHz, CDCl₃, ppm): δ 4.23 (m, 4H), 4.13 (br, 1H), 3.73 (br, 1H), 3.47 (br, 1H), 3.22 (br, 1H), 3.08 (br, 1H), 2.45 (m, 6H), 2.24 (m, 6H), 2.00 (m, 2H), 1.88 (m, 4H), 1.65 (m, 6H), 1.51-1.18 (m, 34H), 1.02 (m, 12H), 0.88 (t, 9H). MS: m/z 780.7. Chemical formula: C₄₆H₈₉N₃O₄
Lipid 882: ¹H NMR (500 MHz, CDCl₃, ppm): δ 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.88 (m, 2H), 1.72 (m, 2H), 1.60 (m, 4H), 1.51-1.18 (m, 33H), 1.02 (s, 9H), 0.88 (t, 9H). MS: m/z 666.7. Chemical formula: C₄₀H₇₉N₃O₄
Lipid 883: ¹H NMR (500 MHz, CDCl₃, ppm): δ 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.88 (m, 2H), 1.72 (m, 2H), 1.60 (m, 4H), 1.51-1.18 (m, 35H), 1.02 (s, 9H), 0.88 (t, 9H). MS: m/z 680.7. Chemical formula: C₄₁H₈₁N₃O₄
Lipid 884: ¹H NMR (500 MHz, CDCl₃, ppm): δ 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.88 (m, 2H), 1.72 (m, 2H), 1.60 (m, 4H), 1.51-1.18 (m, 37H), 1.02 (s, 9H), 0.88 (t, 9H). MS: m/z 694.7. Chemical formula: C₄₂H₈₃N₃O₄
Lipid 885: ¹H NMR (500 MHz, CDCl₃, ppm): δ 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.88 (m, 2H), 1.72 (m, 2H), 1.60 (m, 4H), 1.51-1.18 (m, 39H), 1.02 (s, 9H), 0.88 (t, 9H). MS: m/z 708.7. Chemical formula: C₄₃H₈₅N₃O₄
Lipid 886: ¹H NMR (500 MHz, CDCl₃, ppm): δ 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.88 (m, 2H), 1.72 (m, 2H), 1.60 (m, 4H), 1.51-1.18 (m, 41H), 1.02 (s, 9H), 0.88 (t, 9H). MS: m/z 722.7. Chemical formula: C₄₄H₈₇N₃O₄
Lipid 887: ¹H NMR (500 MHz, CDCl₃, ppm): δ 5.34 (m, 2H), 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.00 (m, 4H), 1.88 (m, 2H), 1.72 (m, 2H), 1.60 (m, 4H), 1.51-1.18 (m, 37H), 1.02 (s, 9H), 0.88 (t, 9H). MS: m/z 748.7. Chemical formula: C₄₆H₈₉N₃O₄
Lipid 888: ¹H NMR (500 MHz, CDCl₃, ppm): δ 5.34 (m, 4H), 4.23 (m, 2H), 4.13 (br, 1H), 3.73 (br, 1H), 3.47 (br, 1H), 3.22 (br, 1H), 3.08 (br, 1H), 2.80 (m, 2H), 2.45 (m, 4H), 2.24 (m, 6H), 2.18 (m, 2H), 2.00 (m, 4H), 1.88 (m, 2H), 1.72 (m, 2H), 1.60 (m, 4H), 1.51-1.18 (m, 31H), 1.02 (s, 9H), 0.88 (t, 9H). MS: m/z 746.7. Chemical formula: C₄₆H₈₇N₃O₄
Lipid 889: ¹H NMR (500 MHz, CDCl₃, ppm): δ 5.60 (m, 2H), 4.69 (m, 2H), 4.13 (br, 1H), 4.06 (m, 2H), 3.73 (br, 1H), 3.47 (br, 1H), 3.22 (br, 1H), 3.08 (br, 1H), 2.45 (m, 4H), 2.24 (m, 6H), 2.00 (m, 4H), 1.60 (m, 4H), 1.51-1.18 (m, 39H), 1.02 (m, 12H), 0.88 (t, 6H). MS: m/z 778.7. Chemical formula: C₄₆H₈₇N₃O₄
Lipid 890: ¹H NMR (500 MHz, CDCl₃, ppm): δ 4.23 (m, 4H), 4.13 (br, 1H), 3.73 (br, 1H), 3.47 (br, 1H), 3.22 (br, 1H), 3.08 (br, 1H), 2.45 (m, 6H), 2.24 (m, 6H), 2.00 (m, 2H), 1.88 (m, 4H), 1.65 (m, 6H), 1.51-1.18 (m, 34H), 1.02 (m, 12H), 0.88 (t, 9H). MS: m/z 780.7. Chemical formula: C₄₆H₈₉N₃O₄
Lipid 891: ¹H NMR (500 MHz, CDCl₃, ppm): δ 4.23 (m, 4H), 4.13 (br, 1H), 3.73 (br, 1H), 3.47 (br, 1H), 3.22 (br, 1H), 3.08 (br, 1H), 2.45 (m, 6H), 2.24 (m, 6H), 2.00 (m, 2H), 1.88 (m, 4H), 1.65 (m, 6H), 1.51-1.18 (m, 34H), 1.02 (m, 15H), 0.88 (t, 6H). MS: m/z 780.7. Chemical formula: C₄₆H₈₉N₃O₄
Lipid 892: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 4H), 2.24 (m, 6H), 2.18 (m, 2H), 1.60 (m, 6H), 1.51-1.18 (m, 35H), 1.02 (m, 12H), 0.88 (m, 6H). MS: m/z 666.7. Chemical formula: C₄₀H₇₉N₃O₄
Lipid 893: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 4H), 2.24 (m, 6H), 2.18 (m, 2H), 1.60 (m, 6H), 1.51-1.18 (m, 37H), 1.02 (m, 12H), 0.88 (m, 6H). MS: m/z 680.7. Chemical formula: C₄₁H₈₁N₃O₄
Lipid 894: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 4H), 2.24 (m, 6H), 2.18 (m, 2H), 1.60 (m, 6H), 1.51-1.18 (m, 39H), 1.02 (m, 12H), 0.88 (m, 6H). MS: m/z 694.7. Chemical formula: C₄₂H₈₃N₃O₄
Lipid 895: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 4H), 2.24 (m, 6H), 2.18 (m, 2H), 1.60 (m, 6H), 1.51-1.18 (m, 41H), 1.02 (m, 12H), 0.88 (m, 6H). MS: m/z 708.7. Chemical formula: C₄₃H₈₅N₃O₄
Lipid 896: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 4H), 2.24 (m, 6H), 2.18 (m, 2H), 1.60 (m, 6H), 1.51-1.18 (m, 43H), 1.02 (m, 12H), 0.88 (m, 6H). MS: m/z 722.7. Chemical formula: C₄₄H₈₇N₃O₄
Lipid 897: ¹H NMR (500 MHz, CDCl₃, ppm): δ 5.34 (m, 2H), 4.13 (br, 1H), 4.05 (m, 2H), 3.73 (br, 1H), 3.47 (br, 1H), 3.22 (br, 1H), 3.08 (br, 1H), 2.45 (m, 4H), 2.24 (m, 6H), 2.18 (m, 2H), 2.00 (m, 4H), 1.60 (m, 6H), 1.51-1.18 (m, 39H), 1.02 (m, 12H), 0.88 (m, 6H). MS: m/z 748.7. Chemical formula: C₄₆H₈₉N₃O₄
Lipid 898: ¹H NMR (500 MHz, CDCl₃, ppm): δ 5.34 (m, 4H), 4.13 (br, 1H), 4.05 (m, 2H), 3.73 (br, 1H), 3.47 (br, 1H), 3.22 (br, 1H), 3.08 (br, 1H), 2.80 (m, 2H), 2.45 (m, 4H), 2.24 (m, 6H), 2.18 (m, 2H), 2.00 (m, 4H), 1.60 (m, 6H), 1.51-1.18 (m, 33H), 1.02 (m, 12H), 0.88 (m, 6H). MS: m/z 746.7. Chemical formula: C₄₆H₈₇N₃O₄
Lipid 899: ¹H NMR (500 MHz, CDCl₃, ppm): δ 5.60 (m, 2H), 4.69 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.00 (m, 4H), 1.60 (m, 4H), 1.51-1.18 (m, 36H), 1.02 (s, 9H), 0.88 (t, 6H). MS: m/z 678.7. Chemical formula: C₄₁H₇₉N₃O₄
Lipid 900: ¹H NMR (500 MHz, CDCl₃, ppm): δ 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.88 (m, 2H), 1.72 (m, 2H), 1.60 (m, 4H), 1.51-1.18 (m, 35H), 1.02 (s, 9H), 0.88 (t, 9H). MS: m/z 680.7. Chemical formula: C₄₁H₈₁N₃O₄
Lipid 901: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 4H), 2.24 (m, 6H), 2.18 (m, 2H), 1.60 (m, 6H), 1.51-1.18 (m, 37H), 1.02 (m, 12H), 0.88 (m, 6H). MS: m/z 680.7. Chemical formula: C₄₁H₈₁N₃O₄
Lipid 902: ¹H NMR (500 MHz, CDCl₃, ppm): δ 5.60 (m, 2H), 4.69 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.00 (m, 4H), 1.60 (m, 4H), 1.51-1.18 (m, 40H), 1.02 (s, 9H), 0.88 (t, 6H). MS: m/z 706.7. Chemical formula: C₄₃H₈₃N₃O₄
Lipid 903: ¹H NMR (500 MHz, CDCl₃, ppm): δ 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.88 (m, 2H), 1.72 (m, 2H), 1.60 (m, 4H), 1.51-1.18 (m, 39H), 1.02 (s, 9H), 0.88 (t, 9H). MS: m/z 708.7. Chemical formula: C₄₃H₈₅N₃O₄
Lipid 904: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 4H), 2.24 (m, 6H), 2.18 (m, 2H), 1.60 (m, 6H), 1.51-1.18 (m, 41H), 1.02 (m, 12H), 0.88 (m, 6H). MS: m/z 708.7. Chemical formula: C₄₃H₈₅N₃O₄
Lipid 905: ¹H NMR (500 MHz, CDCl₃, ppm): δ 5.60 (m, 2H), 4.69 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.00 (m, 4H), 1.60 (m, 4H), 1.51-1.18 (m, 44H), 1.02 (s, 9H), 0.88 (t, 6H). MS: m/z 734.7. Chemical formula: C₄₅H₈₇N₃O₄
Lipid 906: ¹H NMR (500 MHz, CDCl₃, ppm): δ 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.88 (m, 2H), 1.72 (m, 2H), 1.60 (m, 4H), 1.51-1.18 (m, 43H), 1.02 (s, 9H), 0.88 (t, 9H). MS: m/z 736.7. Chemical formula: C₄₅H₈₉N₃O₄
Lipid 907: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 4H), 2.24 (m, 6H), 2.18 (m, 2H), 1.60 (m, 6H), 1.51-1.18 (m, 45H), 1.02 (m, 12H), 0.88 (m, 6H). MS: m/z 736.7. Chemical formula: C₄₅H₈₉N₃O₄
Lipid 908: ¹H NMR (500 MHz, CDCl₃, ppm): δ 5.60 (m, 2H), 5.34 (m, 2H), 4.69 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.00 (m, 8H), 1.60 (m, 4H), 1.51-1.18 (m, 40H), 1.02 (s, 9H), 0.88 (t, 6H). MS: m/z 760.7. Chemical formula: C₄₇H₈₉N₃O₄
Lipid 909: ¹H NMR (500 MHz, CDCl₃, ppm): δ 5.34 (m, 2H), 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 2.00 (m, 4H), 1.88 (m, 2H), 1.72 (m, 2H), 1.60 (m, 4H), 1.51-1.18 (m, 39H), 1.02 (s, 9H), 0.88 (t, 9H). MS: m/z 762.7. Chemical formula: C₄₇H₉₁N₃O₄
Lipid 910: ¹H NMR (500 MHz, CDCl₃, ppm): δ 5.34 (m, 2H), 4.13 (br, 1H), 4.05 (m, 2H), 3.73 (br, 1H), 3.47 (br, 1H), 3.22 (br, 1H), 3.08 (br, 1H), 2.45 (m, 4H), 2.24 (m, 6H), 2.18 (m, 2H), 2.00 (m, 4H), 1.60 (m, 6H), 1.51-1.18 (m, 41H), 1.02 (m, 12H), 0.88 (m, 6H). MS: m/z 762.7. Chemical formula: C₄₇H₉₁N₃O₄
Lipid 911: ¹H NMR (500 MHz, CDCl₃, ppm): δ 5.60 (m, 2H), 4.69 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.00 (m, 4H), 1.60 (m, 4H), 1.51-1.18 (m, 48H), 1.02 (s, 9H), 0.88 (t, 6H). MS: m/z 762.7. Chemical formula: C₄₇H₉₁N₃O₄
Lipid 912: ¹H NMR (500 MHz, CDCl₃, ppm): δ 4.23 (m, 2H), 4.13 (br, 1H), 3.73 (br, 1H), 3.47 (br, 1H), 3.22 (br, 1H), 3.08 (br, 1H), 2.45 (m, 4H), 2.24 (m, 6H), 2.18 (m, 2H), 1.88 (m, 2H), 1.72 (m, 2H), 1.60 (m, 4H), 1.51-1.18 (m, 47H), 1.02 (s, 9H), 0.88 (t, 9H). MS: m/z 764.7. Chemical formula: C₄₇H₉₃N₃O₄
Lipid 913: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 4H), 2.24 (m, 6H), 2.18 (m, 2H), 1.60 (m, 6H), 1.51-1.18 (m, 49H), 1.02 (m, 12H), 0.88 (m, 6H). MS: m/z 764.7. Chemical formula: C₄₇H₉₃N₃O₄
Lipid 914: ¹H NMR (500 MHz, CDCl₃, ppm): δ 4.86 (m, 2H), 4.06 (m, 2H), 3.07 (m, 2H), 2.32 (m, 4H), 2.23 (m, 6H), 2.05 (t, 2H), 1.67 (m, 8H), 1.51-1.18 (m, 54H), 1.04 (s, 9H), 0.87 (t, 9H). MS: m/z 807.7. Chemical formula: C₅₀H₉₈N₂O₅
Lipid 915: ¹H NMR (500 MHz, CDCl₃, ppm): δ 4.13 (br, 1H), 4.05 (m, 4H), 3.07 (m, 2H), 2.45 (m, 2H), 2.28 (m, 1H), 2.24 (m, 6H), 2.18 (m, 2H), 1.80 (m, 2H), 1.72 (m, 4H), 1.60 (m, 4H), 1.51-1.18 (m, 46H), 1.02 (s, 9H), 0.88 (t, 9H). MS: m/z 765.7. Chemical formula: C₄₇H₉₂N₂O₅
Lipid 916: ¹H NMR (500 MHz, CDCl₃, ppm): δ 4.86 (m, 2H), 3.90 (m, 2H), 3.18 (m, 2H), 3.07 (m, 2H), 2.32 (m, 4H), 2.23 (m, 6H), 2.05 (t, 2H), 1.67 (m, 8H), 1.51-1.18 (m, 58H), 1.04 (s, 9H), 0.87 (t, 9H). MS: m/z 864.7. Chemical formula: C₅₃H₁₀₅N₃O₅
Lipid 917: ¹H NMR (500 MHz, CDCl₃, ppm): δ 4.86 (m, 2H), 3.18 (m, 4H), 3.07 (m, 2H), 2.32 (m, 4H), 2.23 (m, 6H), 2.05 (t, 2H), 1.67 (m, 8H), 1.51-1.18 (m, 54H), 1.04 (s, 9H), 0.87 (t, 9H). MS: m/z 835.7. Chemical formula: C₅₁H₁₀₂N₄O₄
Lipid 918: ¹H NMR (500 MHz, CDCl₃, ppm): δ 4.89 (m, 3H), 3.73 (br, 1H), 3.47 (br, 1H), 3.22 (br, 2H), 3.08 (br, 1H), 2.45 (m, 6H), 2.24 (m, 2H), 1.89 (m, 1H), 1.72 (m, 6H), 1.60 (m, 2H), 1.51-1.18 (m, 62H), 1.02 (m, 12H), 0.88 (t, 9H). MS: m/z 864.7. Chemical formula: C₅₂H₁₀₁N₃O₅S
Lipid 919: ¹H NMR (500 MHz, CDCl₃, ppm): δ 4.89 (m, 1H), 4.05 (m, 2H), 3.73 (br, 1H), 3.47 (br, 1H), 3.22 (br, 2H), 3.08 (br, 1H), 2.45 (m, 6H), 2.24 (m, 4H), 1.89 (m, 1H), 1.72 (m, 6H), 1.60 (m, 2H), 1.51-1.18 (m, 70H), 1.02 (s, 9H), 0.88 (t, 12H). MS: m/z 934.7. Chemical formula: C₅₇H₁₁₁N₃O₅S
Lipid 920: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 8H), 2.18 (m, 2H), 1.80 (m, 2H), 1.72 (m, 4H), 1.60 (m, 8H), 1.51-1.18 (m, 52H), 1.02 (s, 9H), 0.88 (t, 9H). MS: m/z 832.7. Chemical formula: C₅₂H₁₀₁N₃O₄
Lipid 921: ¹H NMR (500 MHz, CDCl₃, ppm): δ 5.34 (m, 4H), 4.89 (m, 1H), 4.13 (br, 1H), 3.73 (br, 1H), 3.47 (br, 1H), 3.22 (br, 1H), 3.08 (br, 1H), 2.80 (m, 2H), 2.45 (m, 8H), 2.18 (m, 2H), 2.00 (m, 4H), 1.80 (m, 2H), 1.72 (m, 4H), 1.60 (m, 8H), 1.51-1.18 (m, 46H), 1.02 (s, 9H), 0.88 (t, 9H). MS: m/z 884.7. Chemical formula: C₅₂H₁₀₅N₃O₄
Lipid 922: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 6H), 2.28 (m, 1H), 2.18 (m, 2H), 1.80 (m, 2H), 1.72 (m, 4H), 1.60 (m, 8H), 1.51-1.18 (m, 46H), 1.02 (s, 9H), 0.88 (t, 9H). MS: m/z 790.7. Chemical formula: C₄₉H₉₅N₃O₄
Lipid 923: ¹H NMR (500 MHz, CDCl₃, ppm): δ 5.34 (m, 4H), 4.13 (br, 1H), 4.05 (m, 2H), 3.73 (br, 1H), 3.47 (br, 1H), 3.22 (br, 1H), 3.08 (br, 1H), 2.80 (m, 2H), 2.45 (m, 6H), 2.28 (m, 1H), 2.18 (m, 2H), 2.00 (m, 4H), 1.80 (m, 2H), 1.72 (m, 4H), 1.60 (m, 8H), 1.51-1.18 (m, 40H), 1.02 (s, 9H), 0.88 (t, 9H). MS: m/z 842.7. Chemical formula: C₅₃H₉₉N₃O₄
Lipid 924: ¹H NMR (500 MHz, CDCl₃, ppm): δ 5.34 (m, 10H), 4.13 (br, 1H), 4.05 (m, 2H), 3.73 (br, 1H), 3.47 (br, 1H), 3.22 (br, 1H), 3.08 (br, 1H), 2.80 (m, 8H), 2.45 (m, 6H), 2.28 (m, 1H), 2.18 (m, 2H), 2.00 (m, 4H), 1.80 (m, 2H), 1.72 (m, 4H), 1.60 (m, 8H), 1.51-1.18 (m, 28H), 1.02 (s, 9H), 0.88 (t, 9H). MS: m/z 842.7. Chemical formula: C₅₃H₉₉N₃O₄
Lipid 925: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 6H), 2.28 (m, 1H), 2.18 (m, 2H), 1.80 (m, 4H), 1.72 (m, 4H), 1.60 (m, 8H), 1.51-1.18 (m, 47H), 1.02 (s, 6H), 0.88 (t, 9H). MS: m/z 790.7. Chemical formula: C₄₉H₉₅N₃O₄
Lipid 926: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 4H), 2.28 (m, 1H), 2.18 (m, 6H), 1.80 (m, 2H), 1.72 (m, 4H), 1.60 (m, 2H), 1.51-1.18 (m, 44H), 1.02 (s, 9H), 0.88 (t, 9H). MS: m/z 790.7, Chemical formula: C₄₉H₉₅N₃O₄
Lipid 927: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 8H), 2.18 (m, 6H), 1.80 (m, 2H), 1.72 (m, 4H), 1.60 (m, 8H), 1.51-1.18 (m, 48H), 1.02 (s, 9H), 0.88 (t, 9H). MS: m/z 832.7. Chemical formula: C₅₂H₁₀₁N₃O₄
Lipid 928: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 6H), 2.28 (m, 1H), 2.18 (m, 3H), 1.80 (m, 2H), 1.72 (m, 4H), 1.60 (m, 2H), 1.51-1.18 (m, 44H), 1.02 (m, 12H), 0.88 (t, 9H). MS: m/z 804.7, Chemical formula: C₅₀H₉₇N₃O₄
Lipid 929: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 10H), 2.18 (m, 3H), 1.80 (m, 2H), 1.72 (m, 4H), 1.60 (m, 8H), 1.51-1.18 (m, 48H), 1.02 (m, 12H), 0.88 (t, 9H). MS: m/z 846.7. Chemical formula: C₅₃H₁₀₃N₃O₄
Lipid 930: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.96 (m, 1H), 2.45 (m, 2H), 2.28 (m, 4H), 2.18 (m, 2H), 1.80 (m, 2H), 1.72 (m, 4H), 1.60 (m, 2H), 1.51-1.18 (m, 44H), 1.02 (s, 9H), 0.88 (t, 9H). MS: m/z 790.7, Chemical formula: C₄₉H₉₅N₃O₄
Lipid 931: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.96 (m, 1H), 2.45 (m, 6H), 2.28 (m, 3H), 2.18 (m, 3H), 1.80 (m, 2H), 1.72 (m, 4H), 1.60 (m, 8H), 1.51-1.18 (m, 48H), 1.02 (s, 9H), 0.88 (t, 9H). MS: m/z 832.7. Chemical formula: C₅₂H₁₀₁N₃O₄
Lipid 932: ¹H NMR (500 MHz, CDCl₃, ppm): δ 4.13 (br, 1H), 4.05 (m, 2H), 3.73 (br, 1H), 3.47 (br, 1H), 3.22 (br, 1H), 3.08 (br, 1H), 2.45 (m, 4H), 2.28 (m, 1H), 2.18 (m, 6H), 1.80 (m, 2H), 1.72 (m, 4H), 1.60 (m, 2H), 1.51-1.18 (m, 44H), 1.02 (s, 9H), 0.88 (t, 9H). MS: m/z 790.7, Chemical formula: C₄₉H₉₅N₃O₄
Lipid 933: ¹H NMR (500 MHz, CDCl₃, ppm): δ 4.89 (m, 1H), 4.13 (br, 1H), 3.73 (br, 1H), 3.47 (br, 1H), 3.22 (br, 1H), 3.08 (br, 1H), 2.45 (m, 8H), 2.18 (m, 6H), 1.80 (m, 2H), 1.72 (m, 4H), 1.60 (m, 8H), 1.51-1.18 (m, 48H), 1.02 (s, 9H), 0.88 (t, 9H). MS: m/z 832.7. Chemical formula: C₅₂H₁₀₁N₃O₄
Lipid 934: ¹H NMR (500 MHz, CDCl₃, ppm): δ 5.34 (m, 6H), 4.84 (m, 2H), 3.71 (br, 1H), 3.43 (br, 1H), 3.26 (m, 4H), 3.08 (br, 1H), 2.77 (m, 2H), 2.27 (m, 6H), 2.22 (m, 6H), 2.04 (m, 4H), 1.87 (m, 6H), 1.60 (m, 8H), 1.51-1.18 (m, 42H), 1.02 (s, 6H), 0.88 (t, 9H). MS: m/z 888.7. Chemical formula: C55H105N3O5
Lipid 935: ¹H NMR (500 MHz, CDCl₃, ppm): δ 5.34 (m, 4H), 4.84 (m, 2H), 3.71 (br, 1H), 3.43 (br, 1H), 3.26 (m, 4H), 3.08 (br, 1H), 2.77 (m, 2H), 2.27 (m, 6H), 2.22 (m, 6H), 2.04 (m, 4H), 1.87 (m, 6H), 1.60 (m, 8H), 1.51-1.18 (m, 48H), 0.88 (t, 9H). MS: m/z 874.7. Chemical formula: C₅₄H₁₀₃N₃O₅
Lipid 936: ¹H NMR (500 MHz, CDCl₃, ppm): δ 6.62 (t, 1H), 5.34 (m, 4H), 4.86 (m, 1H), 3.82 (d, 1H), 3.48 (m, 2H), 3.18 (m, 2H), 2.77 (t, 2H), 2.38 (td, 2H), 2.22 (m, 6H), 2.27 (t, 4H), 2.04 (m, 4H), 1.80 (m, 2H), 1.60 (m, 4H), 1.51-1.18 (m, 51H), 0.88 (t, 9H), 0.78 (m, 1H), 0.58 (m, 1H), 0.37 (m, 1H), 0.24 (m, 1H). MS: m/z 842.7. Chemical formula: C₅₃H₉₉N₃O₄
Lipid 937: ¹H NMR (500 MHz, CDCl₃, ppm): δ 6.69 (t, 1H), 5.34 (m, 4H), 4.86 (m, 1H), 3.35 (br, 1H), 3.18 (m, 2H), 3.05 (br, 1H), 2.90 (br, 1H), 2.77 (t, 2H), 2.38 (td, 2H), 2.22 (m, 6H), 2.27 (t, 4H), 2.04 (m, 4H), 1.80 (m, 2H), 1.60 (m, 6H), 1.51-1.18 (m, 61H), 0.88 (t, 9H). MS: m/z 856.7. Chemical formula: C₅₄H₁₀₁N₃O₄
Lipid 938: ¹H NMR (500 MHz, CDCl₃, ppm): δ 5.91 (s, 1H), 5.34 (m, 4H), 4.94 (m, 1H), 4.84 (m, 1H), 3.71 (br, 1H), 3.43 (br, 1H), 3.26 (br, 1H), 3.08 (br, 1H), 2.77 (m, 2H), 2.27 (m, 6H), 2.22 (m, 6H), 2.04 (m, 6H), 1.60 (m, 6H), 1.51-1.18 (m, 50H), 1.02 (s, 6H), 0.88 (m, 12H). MS: m/z 872.7. Chemical formula: C₅₅H₁₀₅N₃O₄
Lipid 939: ¹H NMR (500 MHz, CDCl₃, ppm): δ 6.64 (s, 1H), 5.34 (m, 4H), 4.84 (m, 2H), 3.32 (br, 1H), 3.26 (br, 1H), 3.16 (br, 2H), 2.77 (m, 2H), 2.38 (m, 2H), 2.27 (m, 4H), 2.22 (s, 6H), 2.04 (m, 4H), 1.77 (m, 2H), 1.62 (m, 6H), 1.51-1.18 (m, 51H), 0.90 (d, 3H), 0.88 (m, 12H). MS: m/z 858.7. Chemical formula: C₅₄H₁₀₃N₃O₄
Lipid 940: ¹H NMR (500 MHz, CDCl₃, ppm): δ 6.64 (s, 1H), 5.34 (m, 4H), 4.84 (m, 2H), 3.71 (m, 2H), 3.34 (br, 1H), 3.18 (br, 1H), 3.09 (br, 1H), 2.77 (m, 2H), 2.33 (m, 2H), 2.27 (s, 6H), 2.04 (m, 4H), 1.62 (m, 4H), 1.51-1.18 (m, 57H), 0.88 (m, 18H). MS: m/z 872.7. Chemical formula: C₅₅H₁₀₅N₃O₄
Lipid 941: ¹H NMR (500 MHz, CDCl₃, ppm): δ5.34 (m, 4H), 4.84 (m, 1H), 3.71 (m, 2H), 3.49 (s, 1H), 3.30 (br, 1H), 3.21 (br, 1H), 3.12 (br, 1H), 2.77 (m, 2H), 2.33 (m, 2H), 2.27 (s, 6H), 2.04 (m, 4H), 1.62 (m, 4H), 1.51-1.18 (m, 60H), 0.88 (m, 9H), 0.77 (t, 6H). MS: m/z 872.7. Chemical formula: C₅₅H₁₀₅N₃O₄
Lipid 942: ¹H NMR (500 MHz, CDCl₃, ppm): δ 5.34 (m, 4H), 4.84 (m, 1H), 3.33 (m, 2H), 3.18 (m, 2H), 2.77 (m, 2H), 2.38 (m, 3H), 2.27 (m, 3H), 2.22 (s, 6H), 2.04 (m, 4H), 1.65 (m, 8H), 1.51-1.18 (m, 50H), 0.96 (d, 3H), 0.88 (t, 9H), 0.80 (d, 3H). MS: m/z 844.7. Chemical formula: C₅₃H₁₀₁N₃O₄
Lipid 943: ¹H NMR (500 MHz, CDCl₃, ppm): δ 5.34 (m, 4H), 4.84 (m, 1H), 3.33 (m, 2H), 3.18 (m, 2H), 2.77 (m, 2H), 2.38 (m, 3H), 2.27 (m, 3H), 2.22 (s, 6H), 2.04 (m, 4H), 1.65 (m, 8H), 1.51-1.18 (m, 52H), 0.88 (m, 15H).MS: m/z 858.7. Chemical formula: C₅₄H₁₀₃N₃O₄
Lipid 944: ¹H NMR (500 MHz, CDCl₃, ppm): δ 6.07 (s, 1H), 5.34 (m, 4H), 4.84 (m, 2H), 3.71 (br, 1H), 3.43 (br, 1H), 3.26 (br, 1H), 3.08 (br, 1H), 2.77 (m, 2H), 2.27 (m, 6H), 2.22 (m, 6H), 2.04 (m, 4H), 1.87 (m, 6H), 1.60 (m, 8H), 1.51-1.18 (m, 48H), 1.02 (s, 9H), 0.88 (t, 9H). MS: m/z 900.7. Chemical formula: C₅₇H₁₀₉N₃O₄
Lipid 945: ¹H NMR (500 MHz, CDCl₃, ppm): δ 6.07 (s, 1H), 5.34 (m, 4H), 4.84 (m, 2H), 3.71 (br, 1H), 3.43 (br, 1H), 3.26 (br, 1H), 3.08 (br, 1H), 2.77 (m, 2H), 2.27 (m, 6H), 2.22 (m, 6H), 2.04 (m, 4H), 1.87 (m, 6H), 1.60 (m, 8H), 1.51-1.18 (m, 46H), 1.02 (m, 9H), 0.88 (t, 9H). MS: m/z 886.7. Chemical formula: C₅₆H₁₀₇N₃O₄
Lipid 946: ¹H NMR (500 MHz, CDCl₃, ppm): δ 5.99 (s, 1H), 5.34 (m, 4H), 5.13 (m, 1H), 4.84 (m, 1H), 3.71 (br, 1H), 3.47 (br, 1H), 3.25 (br, 1H), 3.05 (br, 1H), 2.77 (m, 2H), 2.27 (m, 6H), 2.22 (m, 6H), 2.04 (m, 6H), 1.65 (m, 8H), 1.51-1.18 (m, 47H), 1.01 (d, 6H), 0.88 (m, 15H). MS: m/z 886.7. Chemical formula: C₅₆H₁₀₇N₃O₄
Lipid 947: ¹H NMR (500 MHz, CDCl₃, ppm): δ 5.99 (s, 1H), 5.34 (m, 4H), 5.13 (m, 1H), 4.84 (m, 1H), 3.71 (br, 1H), 3.47 (br, 1H), 3.25 (br, 1H), 3.05 (br, 1H), 2.77 (m, 2H), 2.27 (m, 6H), 2.22 (m, 6H), 2.04 (m, 6H), 1.65 (m, 8H), 1.51-1.18 (m, 46H), 1.01 (d, 6H), 0.88 (m, 18H). MS: m/z 900.7. Chemical formula: C₅₇H₁₀₉N₃O₄
Lipid 948: ¹H NMR (500 MHz, CDCl₃, ppm): δ 5.34 (m, 4H), 4.86 (m, 1H), 4.12 (m, 2H), 3.42 (m, 2H), 3.18 (m, 2H), 2.77 (m, 2H), 2.31 (m, 5H), 2.22 (m, 8H), 2.04 (m, 4H), 1.79 (m, 2H), 1.65 (m, 4H), 1.51-1.18 (m, 50H), 0.88 (m, 9H). MS: m/z 802.7. Chemical formula: C₅₀H₉₅N₃O₄
Lipid 949: ¹H NMR (500 MHz, CDCl₃, ppm): δ 6.64 (s, 1H), 5.34 (m, 4H), 4.93 (m, 1H), 4.84 (m, 1H), 3.32 (m, 2H), 3.15 (m, 2H), 2.77 (m, 2H), 2.40 (m, 2H), 2.27 (m, 10H), 2.04 (m, 6H), 1.65 (m, 6H), 1.51-1.18 (m, 57H), 0.88 (m, 9H). MS: m/z 816.7. Chemical formula: C₅₁H₉₇N₃O₄
Lipid 950: ¹H NMR (500 MHz, CDCl₃, ppm): δ 6.64 (s, 1H), 5.34 (m, 4H), 4.93 (m, 1H), 4.84 (m, 1H), 3.32 (m, 2H), 3.15 (m, 2H), 2.77 (m, 2H), 2.40 (m, 2H), 2.27 (m, 10H), 2.04 (m, 6H), 1.65 (m, 6H), 1.51-1.18 (m, 56H), 0.88 (m, 12H). MS: m/z 830.7. Chemical formula: C₅₂H₉₉N₃O₄
Lipid 951: ¹H NMR (500 MHz, CDCl₃, ppm): δ 5.34 (m, 4H), 4.86 (m, 1H), 3.42 (m, 2H), 3.18 (m, 2H), 2.77 (m, 2H), 2.31 (m, 5H), 2.22 (m, 8H), 2.04 (m, 4H), 1.79 (m, 2H), 1.65 (m, 4H), 1.51-1.18 (m, 56H), 0.88 (m, 9H). MS: m/z 830.7. Chemical formula: C₅₂H₉₉N₃O₄
Lipid 952: ¹H NMR (500 MHz, CDCl₃, ppm): δ 5.34 (m, 4H), 4.86 (m, 1H), 3.42 (m, 2H), 3.18 (m, 2H), 2.77 (m, 2H), 2.31 (m, 5H), 2.22 (m, 8H), 2.04 (m, 4H), 1.79 (m, 2H), 1.65 (m, 4H), 1.51-1.18 (m, 54H), 0.88 (m, 15H). MS: m/z 858.7. Chemical formula: C₅₄H₁₀₃N₃O₄
Lipid 953: ¹H NMR (500 MHz, CDCl₃, ppm): δ 4.19 (m, 2H), 4.05 (t, 2H), 3.33 (m, 2H), 3.18 (m, 2H), 2.31 (m, 5H), 2.22 (m, 6H), 1.65 (m, 8H), 1.51-1.18 (m, 50H), 0.88 (m, 9H). MS: m/z 738.7. Chemical formula: C₄₄H₈₇N₃O₅
Lipid 954: ¹H NMR (500 MHz, CDCl₃, ppm): δ 4.05 (t, 2H), 3.33 (m, 2H), 3.18 (m, 2H), 2.31 (m, 5H), 2.22 (m, 6H), 1.65 (m, 8H), 1.51-1.18 (m, 48H), 1.02 (s, 6H), 0.88 (m, 9H). MS: m/z 818.7. Chemical formula: C₄₇H₉₀F₃N₃O₄
Lipid 955: ¹H NMR (500 MHz, CDCl₃, ppm): δ 4.05 (t, 2H), 3.33 (m, 2H), 3.18 (m, 2H), 2.31 (m, 5H), 2.22 (m, 6H), 1.65 (m, 8H), 1.51-1.18 (m, 48H), 1.02 (s, 6H), 0.88 (m, 9H). MS: m/z 767.7. Chemical formula: C₄₇H₉₀D₃N₃O₄
Lipid 956: ¹H NMR (500 MHz, CDCl₃, ppm): δ 4.05 (t, 2H), 3.33 (m, 2H), 3.18 (m, 2H), 2.31 (m, 5H), 2.22 (m, 6H), 1.65 (m, 8H), 1.51-1.18 (m, 48H), 1.02 (s, 3H), 0.88 (m, 9H). MS: m/z 770.7. Chemical formula: C₄₇H₈₇D₆N₃O₄
Lipid 957: ¹H NMR (500 MHz, CDCl₃, ppm): δ 4.05 (t, 2H), 3.33 (m, 2H), 3.18 (m, 2H), 2.31 (m, 5H), 2.22 (m, 6H), 1.65 (m, 8H), 1.51-1.18 (m, 48H), 0.88 (m, 9H). MS: m/z 773.7. Chemical formula: C₄₇H₈₄D₉N₃O₄
Lipid 958: ¹H NMR (500 MHz, CDCl₃, ppm): δ 6.14 (s, 1H), 5.73 (s, 1H), 5.13 (s, 1H), 4.05 (t, 2H), 3.33 (m, 2H), 3.18 (m, 2H), 2.31 (m, 5H), 2.22 (m, 6H), 2.00 (m, 2H), 1.65 (m, 8H), 1.51-1.18 (m, 48H), 1.02 (s, 6H), 0.88 (m, 9H). MS: m/z 790.7. Chemical formula: C₄₉H₉₅N₃O₄
Lipid 959: ¹H NMR (500 MHz, CDCl₃, ppm): δ 4.05 (t, 2H), 3.71 (m, 2H), 3.33 (m, 2H), 3.18 (m, 2H), 2.33 (m, 2H), 2.27 (s, 6H), 1.62 (m, 4H), 1.51-1.18 (m, 60H), 0.88 (m, 9H), 0.77 (t, 6H). MS: m/z 778.7. Chemical formula: C₄₈H₉₅N₃O₄
Lipid 960: ¹H NMR (500 MHz, CDCl₃, ppm): δ 4.05 (t, 2H), 3.33 (m, 2H), 3.18 (m, 2H), 2.38 (m, 3H), 2.27 (m, 3H), 2.22 (s, 6H), 1.65 (m, 8H), 1.51-1.18 (m, 50H), 0.96 (d, 3H), 0.88 (t, 9H), 0.80 (d, 3H). MS: m/z 750.7. Chemical formula: C₄₆H₉₁N₃O₄
Lipid 961: ¹H NMR (500 MHz, CDCl₃, ppm): δ 4.05 (t, 2H), 3.33 (m, 2H), 3.18 (m, 2H), 2.38 (m, 3H), 2.27 (m, 3H), 2.22 (s, 6H), 1.65 (m, 8H), 1.51-1.18 (m, 52H), 0.88 (m, 15H). MS: m/z 764.7. Chemical formula: C₄₇H₉₃N₃O₄
Lipid 962: ¹H NMR (500 MHz, CDCl₃, ppm): δ 4.84 (m, 1H), 3.71 (m, 2H), 3.49 (s, 1H), 3.30 (br, 1H), 3.21 (br, 1H), 3.12 (br, 1H), 2.33 (m, 2H), 2.27 (s, 6H), 1.62 (m, 4H), 1.51-1.18 (m, 66H), 0.88 (m, 9H), 0.77 (t, 6H). MS: m/z 820.7. Chemical formula: C₅₁H₁₀₁N₃O₄
Lipid 963: ¹H NMR (500 MHz, CDCl₃, ppm): δ 4.84 (m, 1H), 3.33 (m, 2H), 3.18 (m, 2H), 2.38 (m, 3H), 2.27 (m, 3H), 2.22 (s, 6H), 1.65 (m, 8H), 1.51-1.18 (m, 56H), 0.96 (d, 3H), 0.88 (t, 9H), 0.80 (d, 3H). MS: m/z 792.7. Chemical formula: C₄₉H₉₇N₃O₄
Lipid 964: ¹H NMR (500 MHz, CDCl₃, ppm): δ 4.84 (m, 1H), 3.33 (m, 2H), 3.18 (m, 2H), 2.38 (m, 3H), 2.27 (m, 3H), 2.22 (s, 6H), 1.65 (m, 8H), 1.51-1.18 (m, 58H), 0.88 (m, 15H). MS: m/z 806.7. Chemical formula: C₅₀H₉₉N₃O₄
Lipid 965: ¹H NMR (500 MHz, CDCl₃, ppm): δ 5.99 (s, 1H), 5.34 (m, 4H), 5.13 (m, 1H), 4.84 (m, 1H), 3.71 (br, 1H), 3.47 (br, 1H), 3.25 (br, 1H), 3.05 (br, 1H), 2.77 (m, 2H), 2.27 (m, 6H), 2.22 (m, 6H), 2.04 (m, 6H), 1.65 (m, 8H), 1.51-1.18 (m, 47H), 0.88 (m, 15H). MS: m/z 892.7. Chemical formula: C₅₆H₁₀₁D₆N₃O₄.

The structure information of lipids synthesized in examples 966-1059 is as follows:
Lipid 967: ¹H NMR (500 MHz, CDCl₃, ppm): δ 5.97 (s, 1H), 5.34 (m, 6H), 4.88 (s, 1H), 4.05 (t, 2H), 3.70 (br, 1H), 3.47 (br, 1H), 3.25 (br, 1H), 3.07 (br, 1H), 2.77 (t, 4H), 2.44-2.22 (m, 11H), 2.04 (m, 4H), 1.61-1.18 (m, 44H), 1.02 (s, 9H), 0.88 (t, 9H).
Lipid 970: ¹H NMR (500 MHz, CDCl₃, ppm): δ 5.97 (s, 1H), 5.34 (m, 6H), 4.86 (m, 2H), 3.72 (br, 1H), 3.45 (br, 1H), 3.23 (br, 1H), 3.05 (br, 1H), 2.77 (t, 4H), 2.44-2.22 (m, 13H), 2.04 (m, 4H), 1.61-1.18 (m, 43H), 1.02 (s, 3H), 0.88 (t, 9H).
Lipid 977: ¹H NMR (500 MHz, CDCl₃, ppm): δ 5.97 (s, 1H), 5.34 (m, 10H), 4.88 (s, 1H), 4.05 (t, 2H), 3.70 (br, 1H), 3.47 (br, 1H), 3.25 (br, 1H), 3.07 (br, 1H), 2.77 (t, 8H), 2.44-2.22 (m, 12H), 2.04 (m, 4H), 1.61-1.18 (m, 40H), 1.02 (s, 6H), 0.88 (t, 9H).
Lipid 983: ¹H NMR (500 MHz, CDCl₃, ppm): δ 5.97 (s, 1H), 5.34 (m, 8H), 4.88 (s, 1H), 4.05 (t, 2H), 3.70 (br, 1H), 3.47 (br, 1H), 3.25 (br, 1H), 3.07 (br, 1H), 2.77 (t, 6H), 2.44-2.22 (m, 11H), 2.04 (m, 4H), 1.61-1.18 (m, 42H), 1.02 (s, 9H), 0.88 (t, 9H).
Lipid 1024: ¹H NMR (500 MHz, CDCl₃, ppm): δ 5.97 (s, 1H), 5.34 (m, 12H), 4.86 (m, 2H), 3.72 (br, 1H), 3.45 (br, 1H), 3.23 (br, 1H), 3.05 (br, 1H), 2.77 (t, 10H), 2.44-2.22 (m, 12H), 2.04 (m, 4H), 1.61-1.18 (m, 44H), 1.02 (s, 9H), 0.88 (t, 9H).
Lipid 1034: ¹H NMR (500 MHz, CDCl₃, ppm): δ 5.97 (s, 1H), 4.88 (s, 1H), 4.05 (t, 2H), 3.70 (br, 1H), 3.47 (br, 1H), 3.25 (br, 1H), 3.07 (br, 1H), 2.44-2.22 (m, 9H), 1.61 (m, 8H), 1.51-1.18 (m, 52H), 1.02 (s, 9H), 0.88 (t, 9H).
Lipid 1035: ¹H NMR (500 MHz, CDCl₃, ppm): δ 6.04 (s, 1H), 4.84 (m, 2H), 3.72 (br, 1H), 3.45 (br, 1H), 3.23 (br, 1H), 3.08 (br, 1H), 2.44-2.22 (m, 10H), 1.61 (m, 6H), 1.51-1.18 (m, 60H), 1.02 (s, 9H), 0.88 (t, 9H).
Lipid 1036: ¹H NMR (500 MHz, CDCl₃, ppm): δ 6.74 (s, 1H), 4.73 (s, 1H), 4.05 (t, 2H), 3.45-3.05 (m, 4H), 2.44-2.22 (m, 11H), 1.51-1.18 (m, 60H), 0.88 (t, 12H).
Lipid 1037: ¹H NMR (500 MHz, CDCl₃, ppm): δ 5.97 (s, 1H), 4.88 (s, 1H), 4.05 (t, 2H), 3.70 (br, 1H), 3.47 (br, 1H), 3.25 (br, 1H), 3.07 (br, 1H), 2.44-2.22 (m, 11H), 1.61 (m, 8H), 1.51-1.18 (m, 44H), 1.02 (s, 9H), 0.88 (t, 9H).
Lipid 1038: ¹H NMR (500 MHz, CDCl₃, ppm): δ 5.97 (s, 1H), 4.88 (s, 1H), 4.05 (t, 2H), 3.70 (br, 1H), 3.47 (br, 1H), 3.25 (br, 1H), 3.07 (br, 1H), 2.44-2.22 (m, 11H), 1.61 (m, 8H), 1.51-1.18 (m, 52H), 1.02 (s, 9H), 0.88 (t, 9H).
Lipid 1039: ¹H NMR (500 MHz, CDCl₃, ppm): δ 6.67 (s, 1H), 4.95 (s, 1H), 4.05 (t, 2H), 3.33 (m, 2H), 3.18 (m, 2H), 2.44-2.22 (m, 11H), 1.61 (m, 8H), 1.51-1.18 (m, 51H), 0.88 (t, 9H).
Lipid 1040: ¹H NMR (500 MHz, CDCl₃, ppm): δ 6.04 (s, 1H), 4.84 (m, 2H), 3.33 (m, 2H), 3.18 (m, 2H), 2.44-2.22 (m, 12H), 1.61 (m, 6H), 1.51-1.18 (m, 59H), 0.88 (t, 9H).
Lipid 1041: ¹H NMR (500 MHz, CDCl₃, ppm): δ 6.74 (s, 1H), 4.62 (s, 1H), 4.05 (t, 2H), 3.33 (m, 2H), 3.18 (m, 2H), 2.44-2.22 (m, 11H), 1.61 (m, 8H), 1.51-1.18 (m, 50H), 0.88 (m, 12H).
Lipid 1042: ¹H NMR (500 MHz, CDCl₃, ppm): δ 6.04 (s, 1H), 4.84 (m, 2H), 3.33 (m, 2H), 3.18 (m, 2H), 2.44-2.22 (m, 12H), 1.61 (m, 6H), 1.51-1.18 (m, 58H), 0.88 (m, 12H).
Lipid 1043: ¹H NMR (500 MHz, CDCl₃, ppm): δ 6.72 (s, 1H), 4.77 (s, 1H), 4.05 (t, 2H), 3.37 (m, 1H), 3.16 (m, 3H), 3.04 (m, 1H), 2.44-2.22 (m, 11H), 1.61 (m, 8H), 1.51-1.18 (m, 54H), 0.88 (t, 9H).
Lipid 1044: ¹H NMR (500 MHz, CDCl₃, ppm): δ 6.71 (s, 1H), 4.87 (m, 1H), 4.78 (m, 1H), 3.37 (m, 1H), 3.17 (m, 3H), 3.04 (m, 1H), 2.44-2.22 (m, 12H), 1.61 (m, 6H), 1.51-1.18 (m, 62H), 0.88 (t, 9H).
Lipid 1045: ¹H NMR (500 MHz, CDCl₃, ppm): δ 5.34 (m, 4H), 4.86 (t, 1H), 3.36 (m, 2H), 3.16 (m, 2H), 2.77 (t, 2H), 2.44-2.22 (m, 13H), 2.04 (m, 4H), 1.82 (m, 2H), 1.61-1.18 (m, 62H), 0.88 (t, 9H).
Lipid 1046: ¹H NMR (500 MHz, CDCl₃, ppm): δ 4.05 (t, 2H), 3.37 (m, 2H), 3.16 (m, 2H), 2.44-2.22 (m, 12H), 1.82 (m, 2H), 1.61-1.18 (m, 60H), 0.88 (t, 9H).
Lipid 1047: ¹H NMR (500 MHz, CDCl₃, ppm): δ4.84 (m, 1H), 3.34 (m, 2H), 3.16 (m, 2H), 2.44-2.22 (m, 14H), 1.82 (m, 2H), 1.61-1.18 (m, 66H), 0.88 (t, 9H).
Lipid 1048: ¹H NMR (500 MHz, CDCl₃, ppm): δ 5.34 (m, 4H), 4.86 (t, 1H), 3.36 (m, 2H), 3.16 (m, 2H), 2.77 (t, 2H), 2.44-2.22 (m, 13H), 2.04 (m, 4H), 1.82 (m, 2H), 1.61-1.18 (m, 64H), 0.88 (t, 9H).
Lipid 1049: ¹H NMR (500 MHz, CDCl₃, ppm): δ 4.05 (t, 2H), 3.37 (m, 2H), 3.16 (m, 2H), 2.44-2.22 (m, 12H), 1.82 (m, 2H), 1.61-1.18 (m, 62H), 0.88 (t, 9H).
Lipid 1050: ¹H NMR (500 MHz, CDCl₃, ppm): δ4.84 (m, 1H), 3.34 (m, 2H), 3.16 (m, 2H), 2.44-2.22 (m, 14H), 1.82 (m, 2H), 1.61-1.18 (m, 68H), 0.88 (t, 9H).
Lipid 1051: ¹H NMR (500 MHz, CDCl₃, ppm): δ 5.97 (s, 1H), 5.34 (m, 4H), 4.86 (m, 2H), 3.45 (m, 2H), 3.08 (m, 2H), 2.77 (t, 2H), 2.44-2.22 (m, 12H), 2.04 (m, 4H), 1.61-1.18 (m, 57H), 0.88 (m, 12H).
Lipid 1053: ¹H NMR (500 MHz, CDCl₃, ppm): δ 4.84 (m, 1H), 3.45 (m, 2H), 3.08 (m, 2H), 2.44-2.22 (m, 12H), 1.61-1.18 (m, 66H), 0.88 (m, 15H).
Lipid 1054: ¹H NMR (500 MHz, CDCl₃, ppm): δ 4.84 (m, 1H), 3.45 (m, 2H), 3.08 (m, 2H), 2.44-2.22 (m, 12H), 1.61 -1.18 (m, 63H), 0.88 (m, 12H).
Lipid 1055: ¹H NMR (500 MHz, CDCl₃, ppm): δ 5.97 (s, 1H), 4.88 (s, 1H), 4.05 (t, 2H), 3.70 (br, 1H), 3.47 (br, 1H), 3.25 (br, 1H), 3.07 (br, 1H), 2.44-2.22 (m, 11H), 1.61-1.18 (m, 62H), 0.88 (t, 9H).
Lipid 1056: ¹H NMR (500 MHz, CDCl₃, ppm): δ 5.97 (s, 1H), 4.88 (s, 1H), 4.05 (t, 2H), 3.70 (br, 1H), 3.47 (br, 1H), 3.25 (br, 1H), 3.07 (br, 1H), 2.44-2.22 (m, 11H), 1.61-1.18 (m, 60H), 0.88 (m, 15H).
Lipid 1057: ¹H NMR (500 MHz, CDCl₃, ppm): δ 5.97 (s, 1H), 4.88 (s, 1H), 4.05 (t, 2H), 3.45 (m, 2H), 3.08 (m, 2H), 2.44-2.22 (m, 11H), 1.61 (m, 8H), 1.51-1.18 (m, 49H), 0.88 (m, 12H).
Lipid 1058: ¹H NMR (500 MHz, CDCl₃, ppm): δ 11.06 (s, 1H), 4.18 (s, 1H), 4.05 (t, 2H), 3.70 (m, 3H), 3.09 (br, 1H), 2.44-2.22 (m, 11H), 1.61 (m, 8H), 1.51-1.18 (m, 48H), 1.02 (s, 9H), 0.88 (t, 9H).
Lipid 1059: ¹H NMR (500 MHz, CDCl₃, ppm): δ 11.06 (s, 1H), 4.84 (m, 1H),4.18 (s, 1H), 3.70 (m, 3H), 3.09 (br, 1H), 2.70-2.22 (m, 12H), 1.61 (m, 6H), 1.51-1.18 (m, 56H), 1.02 (s, 9H), 0.88 (t, 9H).

Example 8 Preparation and physicochemical detection of lipid nanoparticle (LNP)

### Preparation of LNP

Step 1. Preparation of a lipid solution and a nucleic acid solution: unless otherwise specified, all aqueous solutions involved in examples were prepared from deionized water without nucleases. An ionizable lipid, a phospholipid (disaturated phosphatidylcholine (DSPC) or dioleoylphosphatidylethanolamine (DOPE)), cholesterol and a PEG lipid (DMG-PEG2000) were mixed in a molar ratio of 50/10/38.5/1.5 (containing DSPC, formulation 1) or 36.5/16/46.5/2.5 (containing DOPE, formulation 2) and dissolved into an ethanol solution. Meanwhile, a nucleic acid (a mass ratio of ionizable lipid/nucleic acid was 10/1) was dissolved into a citrate buffer solution of pH=3, so that a final volume ratio of the ethanol solution to the citrate solution was 1/3.

Step 2. Preparation of LNP by mixing the lipid solution with the nucleic acid solution: the lipid ethanol solution and the nucleic acid aqueous solution that were prepared in Step 1 were respectively put into a sterile syringe and placed in a Precision Nanosystem Ignite instrument, the extrusion flow speeds of the ethanol solution and the water phase solution were respectively set as 3 ml/min and 9 ml/min in instrument program settings. A microfluidic chip was inserted into the instrument and LNP preparation was initiated, and then a solution mixed by the chip was collected.

Step 2.1. Preparation of an LNP solution coating nucleic acid in a porous plate: a solution of an ionizable lipid, a phospholipid (DSPC or DOPE), cholesterol and a PEG lipid (DMG-PEG2000) in ethanol was added into each well of a 96-well deep plate, so that a molar ratio of four component lipids in each well was 50/10/38.5/1.5 (containing DSPC, formulation 1) or 36.5/16/46.5/2.5 (containing DOPE, formulation 2). Then, a citrate buffer solution (pH=3, the mass of nucleic acid added into each well was 1/10 that of the ionizable lipid in this well) that was three times as ethanol in volume and dissolved with a nucleic acid molecule was quickly added into the well plate through a multi-channel pipette or an automatic liquid dispensing system and rapidly mixed, so as to obtain the LNP solution coating nucleic acid.

Step 3. Purification of the LNP solution: the LNP solution collected in Step 2 or Step 2.1 was subjected to dialysis-concentration-filtration operations, so as to obtain a final LNP solution coating nucleic acid for subsequent testing.

### Particle size detection of LNP

The LNP solution coating nucleic acid obtained in Step 3 was diluted so that the final lipid concentration was between 0.01 mg/ml and 0.1 mg/ml. The diluted solution was added into a sample reservoir, and then subjected to particle size detection in a Brookhaven NanoBrook Omni instrument. Data is shown in Tables 9-12.

The following Tables 9-12 list particle sizes of LNPs after coating mRNA (luciferase).

### Determination of total nucleic acid and measurement of encapsulation efficiency

The content of nucleic acids in an LNP sample was determined through a Quant-iT^{™} RiboGreen^{®} RNA detection kit (Thermofisher). The detection of total RNA concentration: 5 µl of to-be-detected sample was placed into 2 ml of ep tube, 245 µl of 1xTE Buffer was added, and the above materials were evenly mixed to obtain a "diluted sample". 100 µl of the above "diluted sample" was taken, 100 µl of 10% TritonX-100 solution was added, the above materials were sufficiently and evenly mixed, and then the obtained mixture was demulsified for 15 min in metal bath under the conditions of 37°C and 300 rpm; and 80 µl of 1xTE Buffer was added into an ELISA plate, 20 µl of demulsified sample was taken and added into the ELISA plate to be evenly mixed, and then 100 µl of fluorescence dye (2000-fold dilution) solution was added.

Detection of free RNA concentration: 90 µl of 1xTE Buffer was added into the ELISA plate, 10 µl of the above "diluted sample" was taken, and 100 µl of fluorescence dye (2000-fold dilution) solution was added.

Meanwhile, a series of standard solutions having different concentrations were set in the ELISA plate as quantitative standard curves. The samples were loaded onto the ELISA plate to detect fluorescence signals (with an excitation wavelength of 480 nm and an emission detection wavelength of 520). A corresponding RNA concentration was obtained after the fluorescence signal in each well was converted via the standard curve.

Calculation method of encapsulation efficiency: $EE \left(%\right) = \frac{Concentration of total mRNA - concentration of free mRN A}{Concentration of total mRNA} ∗ 100 %$

**Table 9**

| Name | Particle size (nm.d) | PDI | Name | Particle size (nm.d) | PDI |
|---|---|---|---|---|---|
| Lipid 1 | 165.54±2.42 | 0.21 | Lipid 124 | 99.57±4.81 | 0.18 |
| Lipid 5 | 162.01±0.48 | 0.07 | Lipid 125 | 121.29±5.36 | 0.13 |
| Lipid 6 | 140.23±3.78 | 0.14 | Lipid 126 | 152.95±1.37 | 0.19 |
| Lipid 7 | 94.89±2.86 | 0.18 | Lipid 127 | 119.03±2.57 | 0.08 |
| Lipid 8 | 95.86±4.65 | 0.19 | Lipid 128 | 136.8±2.9 | 0.07 |
| Lipid 9 | 77.95±8.33 | 0.17 | Lipid 132 | 132.29±6.18 | 0.16 |
| Lipid 10 | 113.3±3.58 | 0.17 | Lipid 133 | 155.06±1.13 | 0.16 |
| Lipid 11 | 104.91±4.75 | 0.24 | Lipid 134 | 155.61±2.36 | 0.12 |
| Lipid 12 | 105.24±9.75 | 0.22 | Lipid 135 | 156.47±2.27 | 0.23 |
| Lipid 15 | 121.49±0.86 | 0.15 | Lipid 136 | 179.57±8.23 | 0.26 |
| Lipid 16 | 128.73±1.97 | 0.26 | Lipid 137 | 169.47±0.96 | 0.25 |
| Lipid 17 | 169.19±3.62 | 0.19 | Lipid 150 | 162.24±3.74 | 0.25 |
| Lipid 18 | 162.01±6.54 | 0.22 | Lipid 151 | 174.21±4.73 | 0.21 |
| Lipid 19 | 70.75±5.83 | 0.25 | Lipid 152 | 174.09±1.34 | 0.26 |
| Lipid 20 | 167.54±3.15 | 0.67 | Lipid 153 | 178.71±13.69 | 0.13 |
| Lipid 23 | 167.54±3.15 | 0.67 | Lipid 154 | 138.2±5.9 | 0.23 |
| Lipid 24 | 281.65±13.76 | 0.25 | Lipid 155 | 141.04±3.5 | 0.19 |
| Lipid 25 | 152.95±1.37 | 0.19 | Lipid 159 | 136.93±2.95 | 0.22 |
| Lipid 26 | 148.92±3.75 | 0.24 | Lipid 160 | 201.12±47.7 | 0.23 |
| Lipid 27 | 271.22±7.67 | 0.23 | Lipid 161 | 253.06±4.58 | 0.27 |
| Lipid 34 | 135.74±2.65 | 0.24 | Lipid 162 | 261.64±5.92 | 0.27 |
| Lipid 35 | 284.48±7.6 | 0.22 | Lipid 163 | 200.94±5.63 | 0.17 |
| Lipid 36 | 126.74±5.99 | 0.28 | Lipid 164 | 206.39±0.86 | 0.24 |
| Lipid 45 | 181.16±7.06 | 0.22 | Lipid 174 | 202.44±2.28 | 0.17 |
| Lipid 46 | 133.1±3.10 | 0.27 | Lipid 175 | 178.88±3.61 | 0.18 |
| Lipid 47 | 127.59±2.30 | 0.29 | Lipid 186 | 156.53±5.52 | 0.21 |
| Lipid 63 | 174.41±2.13 | 0.25 | Lipid 187 | 190.27±3.15 | 0.19 |
| Lipid 64 | 167.84±4.18 | 0.24 | Lipid 189 | 188.99±4.55 | 0.19 |
| Lipid 65 | 169.93±2.88 | 0.26 | Lipid 190 | 211.32±8.91 | 0.17 |
| Lipid 72 | 132.12±0.27 | 0.16 | Lipid 192 | 209.01±1.53 | 0.18 |
| Lipid 73 | 99.44±9.56 | 0.18 | Lipid 193 | 191.34±8.95 | 0.21 |
| Lipid 74 | 127.99±2.01 | 0.27 | Lipid 195 | 204.79±3.09 | 0.19 |
| Lipid 81 | 183.1±1.41 | 0.12 | Lipid 196 | 207.19±10.61 | 0.13 |
| Lipid 82 | 163.32±7.69 | 0.15 | Lipid 198 | 178±4.42 | 0.15 |
| Lipid 83 | 182.11±1.23 | 0.17 | Lipid 199 | 158±3.53 | 0.17 |
| Lipid 90 | 169.05±1.64 | 0.13 | Lipid 201 | 241.11±18.01 | 0.26 |
| Lipid 91 | 169.09±3.56 | 0.15 | Lipid 202 | 299.59±11.81 | 0.26 |
| Lipid 92 | 168.43±3.31 | 0.19 | Lipid 203 | 124.67±1.87 | 0.23 |
| Lipid 93 | 173.31±3.05 | 0.23 | Lipid 204 | 194.44±4.2 | 0.18 |
| Lipid 94 | 139.13±7.65 | 0.16 | Lipid 205 | 214.58±3.02 | 0.17 |
| Lipid 95 | 136.39±3.8 | 0.18 | Lipid 206 | 189.94±1.09 | 0.19 |
| Lipid 105 | 140.32±1.2 | 0.19 | Lipid 207 | 210.34±0.43 | 0.18 |
| Lipid 106 | 274.91±12.99 | 0.30 | Lipid 208 | 155.83±1.5 | 0.15 |
| Lipid 107 | 211.71±6.11 | 0.31 | Lipid 209 | 207.85±1.08 | 0.16 |
| Lipid 117 | 209.62±1.49 | 0.19 | Lipid 210 | 177.75±11.33 | 0.23 |
| Lipid 118 | 187.73±5.96 | 0.23 | Lipid 211 | 186.97±13.51 | 0.24 |
| Lipid 119 | 236.05±2.18 | 0.26 | Lipid 212 | 160.39±14.96 | 0.18 |
| Lipid 123 | 128.44±9.48 | 0.21 | Lipid 213 | 222.09±1.13 | 0.17 |

**Table 10**

| Name | Particle size (nm.d) | PDI | Name | Particle size (nm.d) | PDI |
|---|---|---|---|---|---|
| Lipid 214 | 225.01±2.31 | 0.19 | Lipid 262 | 176.35±2.84 | 0.20 |
| Lipid 215 | 125.06±2.72 | 0.30 | Lipid 263 | 154.44±1.23 | 0.22 |
| Lipid 216 | 216.87±0.51 | 0.17 | Lipid 264 | 152.61±1.49 | 0.19 |
| Lipid 217 | 144.23±0.52 | 0.15 | Lipid 265 | 246.22±13.58 | 0.22 |
| Lipid 218 | 147.21±2.34 | 0.17 | Lipid 266 | 112.66±2.27 | 0.21 |
| Lipid 219 | 186.37±2.57 | 0.20 | Lipid 267 | 121.55±5.39 | 0.23 |
| Lipid 220 | 155.24±3.11 | 0.18 | Lipid 268 | 239.79±17.53 | 0.12 |
| Lipid 221 | 152.33±0.59 | 0.18 | Lipid 269 | 207.12±13.07 | 0.17 |
| Lipid 222 | 187.08±5.27 | 0.20 | Lipid 270 | 211.42±20.54 | 0.15 |
| Lipid 223 | 190.71±1.15 | 0.19 | Lipid 271 | 186.54±8.8 | 0.165 |
| Lipid 224 | 210.98±5.97 | 0.20 | Lipid 272 | 170.34±4.13 | 0.126 |
| Lipid 225 | 231.81±5.01 | 0.17 | Lipid 273 | 161.75±6.58 | 0.133 |
| Lipid 226 | 207.34±2.23 | 0.19 | Lipid 274 | 160.62±7.28 | 0.155 |
| Lipid 227 | 228.59±2.26 | 0.19 | Lipid 275 | 163.03±8.21 | 0.165 |
| Lipid 228 | 167.26±1.19 | 0.16 | Lipid 276 | 161.06±8.27 | 0.155 |
| Lipid 229 | 223.57±2.83 | 0.16 | Lipid 277 | 165.17±5.47 | 0.171 |
| Lipid 230 | 208.58±19.21 | 0.17 | Lipid 278 | 190.5712.49 | 0.249 |
| Lipid 231 | 182.60±7.68 | 0.23 | Lipid 279 | 196.74±0.83 | 0.18 |
| Lipid 232 | 127.29±11.35 | 0.28 | Lipid 280 | 180.44±2.26 | 0.17 |
| Lipid 233 | 145.87±4.73 | 0.28 | Lipid 281 | 159.41±26.92 | 0.24 |
| Lipid 234 | 240.23±1.39 | 0.17 | Lipid 282 | 69.68±4.62 | 0.23 |
| Lipid 235 | 245.71±2.64 | 0.18 | Lipid 283 | 187.67±2.32 | 0.15 |
| Lipid 236 | 194.99±3.33 | 0.20 | Lipid 294 | 150.04±0.93 | 0.26 |
| Lipid 237 | 234.57±1.84 | 0.14 | Lipid 295 | 162.45±2.28 | 0.16 |
| Lipid 238 | 154.82±0.78 | 0.17 | Lipid 296 | 149.35±0.60 | 0.14 |
| Lipid 239 | 159.52±1.82 | 0.17 | Lipid 297 | 129.02±1.46 | 036 |
| Lipid 240 | 204.35±3.72 | 0.19 | Lipid 298 | 143.37±1.81 | 0.26 |
| Lipid 241 | 168.33±2.59 | 0.19 | Lipid 299 | 134.33±6.27 | 0.25 |
| Lipid 242 | 165.47±0.76 | 0.17 | Lipid 300 | 125.84±1.86 | 0.28 |
| Lipid 243 | 204.53±5.62 | 0.19 | Lipid 301 | 148.93±1.57 | 0.26 |
| Lipid 244 | 207.68±0.93 | 0.18 | Lipid 302 | 150.21±2.42 | 0.14 |
| Lipid 245 | 218.21±6.97 | 0.19 | Lipid 309 | 132.53±2.29 | 0.22 |
| Lipid 246 | 225.64±9.49 | 0.18 | Lipid 310 | 181.16±2.41 | 0.12 |
| Lipid 247 | 128.73±1.97 | 0.26 | Lipid 311 | 166.73±0.13 | 0.14 |
| Lipid 248 | 186.5±2.23 | 0.08 | Lipid 312 | 125.72±8.89 | 0.30 |
| Lipid 249 | 168.59±1.51 | 0.18 | Lipid 313 | 145.31±2.18 | 0.22 |
| Lipid 250 | 140.67±1.14 | 0.23 | Lipid 314 | 143.02±5.01 | 0.22 |
| Lipid 251 | 157.41±4.11 | 0.21 | Lipid 315 | 174.91±1.49 | 0.18 |
| Lipid 252 | 207.07±24.67 | 0.22 | Lipid 316 | 176.62±5.09 | 0.19 |
| Lipid 253 | 257.86±29.8 | 0.10 | Lipid 317 | 162.23±0.89 | 0.23 |
| Lipid 254 | 211.63±3.53 | 0.21 | Lipid 318 | 196.93±1.78 | 0.19 |
| Lipid 255 | 186.02±8.84 | 0.21 | Lipid 319 | 101.42±4.09 | 0.19 |
| Lipid 256 | 179.06±9.69 | 0.19 | Lipid 320 | 164.99±1.21 | 0.17 |
| Lipid 257 | 251.52±4.27 | 0.18 | Lipid 321 | 91.75±4.15 | 0.20 |
| Lipid 258 | 171.79±2.34 | 0.21 | Lipid 322 | 113.37±0.73 | 0.22 |
| Lipid 259 | 178.34±3.11 | 0.20 | Lipid 323 | 110.25±4.23 | 0.20 |
| Lipid 260 | 197.34±14.23 | 0.18 | Lipid 326 | 111.42±4.09 | 0.18 |
| Lipid 261 | 208.09±16.04 | 0.23 | Lipid 327 | 104.99±1.21 | 0.17 |

**Table 11**

| LNP (Lipid number) | Particle size (nm.d) | PDI | LNP (Lipid number) | Particle size (nm.d) | PDI | LNP (Lipid number) | Particle size (nm.d) | PDI |
|---|---|---|---|---|---|---|---|---|
| 401 | 93.6 ± 0.84 | 0.19 | 456 | 77 ± 1.44 | 0.26 | 579 | 127.3 ± 0.28 | 0.10 |
| 402 | 84.3 ± 3.42 | 0.25 | 457 | 79.9 ± 2.93 | 0.26 | 581 | 106.3 ± 2.49 | 0.20 |
| 403 | 93.7 ± 0.92 | 0.19 | 458 | 74 ± 0.47 | 0.23 | 582 | 115 ± 1.86 | 0.19 |
| 404 | 95.3 ± 0.81 | 0.20 | 459 | 94.9 ± 0.75 | 0.26 | 585 | 111.2 ± 1.39 | 0.17 |
| 405 | 94.7 ± 0.62 | 0.19 | 460 | 124.7 ± 1.63 | 0.24 | 586 | 147 ± 2.02 | 0.13 |
| 406 | 95.9 ± 0.75 | 0.17 | 461 | 128 ± 0.72 | 0.21 | 587 | 151 ± 0.05 | 0.16 |
| 407 | 97.4 ± 0.64 | 0.18 | 462 | 126.5 ± 0.88 | 0.24 | 589 | 122.5 ± 1.52 | 0.15 |
| 408 | 101.7 ± 5.09 | 0.18 | 464 | 243.5 ± 74.8 | 0.09 | 590 | 142.1 ± 0.78 | 0.11 |
| 409 | 95 ± 0.75 | 0.25 | 465 | 93.9 ± 10.89 | 0.10 | 591 | 143.7 ± 0.16 | 0.10 |
| 410 | 91.6 ± 0.38 | 0.22 | 468 | 98.5 ± 11.48 | 0.12 | 594 | 118.2 ± 0.76 | 0.13 |
| 411 | 92.3 ± 2.18 | 0.21 | 475 | 91.7 ± 0.08 | 0.26 | 597 | 113.5 ± 0.6 | 0.21 |
| 412 | 88.7 ± 1.98 | 0.25 | 476 | 92.6 ± 24.4 | 0.22 | 599 | 100.2 ± 0.64 | 0.2 |
| 413 | 97.4 ± 0.91 | 0.25 | 477 | 141.4 ± 18.97 | 0.16 | 602 | 98.7 ± 0.76 | 0.24 |
| 414 | 99.2 ± 1.34 | 0.20 | 480 | 93.3 ± 0.34 | 0.27 | 603 | 100.2 ± 0.13 | 0.22 |
| 415 | 96.5 ± 0.98 | 0.23 | 481 | 92.6 ± 2.27 | 0.29 | 605 | 84.2 ± 1.13 | 0.21 |
| 416 | 87.8 ± 0.51 | 0.25 | 491 | 103.7 ± 10.8 | 0.27 | 606 | 112.6 ± 0.49 | 0.20 |
| 418 | 101.7 ± 1.73 | 0.29 | 492 | 125.3 ± 6.91 | 0.21 | 609 | 116.7 ± 1.15 | 0.18 |
| 419 | 86.7 ± 0.96 | 0.26 | 495 | 91.8 ± 0.06 | 0.24 | 610 | 120.6 ± 1.06 | 0.16 |
| 420 | 110.3 ± 1.65 | 0.22 | 497 | 91.8 ± 1.17 | 0.26 | 611 | 122.7 ± 0.79 | 0.14 |
| 421 | 96.1 ± 0.83 | 0.26 | 499 | 106 ± 0.56 | 0.24 | 613 | 91.7 ± 0.25 | 0.06 |
| 422 | 94.5 ± 0.77 | 0.26 | 501 | 108.4 ± 0.78 | 0.21 | 614 | 107.2 ± 0.97 | 0.23 |
| 424 | 153 ± 2.11 | 0.11 | 503 | 73.1 ± 0.63 | 0.22 | 615 | 108.7 ± 0.67 | 0.19 |
| 425 | 94.4 ± 1.86 | 0.25 | 504 | 71.8 ± 1.19 | 0.26 | 618 | 109.3 ± 1.65 | 0.17 |
| 426 | 79.5 ± 0.79 | 0.26 | 505 | 69.4 ± 0.92 | 0.26 | 619 | 112.6 ± 0.44 | 0.17 |
| 427 | 112.9 ± 0.65 | 0.25 | 506 | 107.1 ± 0.62 | 0.24 | 621 | 91.7 ± 2.26 | 0.24 |
| 428 | 111.7 ± 2.92 | 0.26 | 513 | 88.8 ± 0.23 | 0.21 | 622 | 138.9 ± 0.8 | 0.16 |
| 429 | 117.5 ± 3.56 | 0.28 | 516 | 86.8 ± 0.55 | 0.26 | 623 | 140.5 ± 0.55 | 0.14 |
| 430 | 90.5 ± 1.67 | 0.23 | 517 | 151.1 ± 4.19 | 0.10 | 625 | 102.9 ± 2.92 | 0.21 |
| 431 | 114 ± 2.51 | 0.26 | 521 | 108.1 ± 1.48 | 0.11 | 626 | 136.7 ± 1.19 | 0.14 |
| 433 | 93.4 ± 1.15 | 0.24 | 522 | 111.1 ± 1.2 | 0.10 | 627 | 139.1 ± 0.37 | 0.14 |
| 434 | 95.7 ± 0.99 | 0.23 | 523 | 113.5 ± 0.92 | 0.11 | 628 | 101.4 ± 0.52 | 0.20 |
| 436 | 92.2 ± 0.47 | 0.18 | 524 | 152.6 ± 1.49 | 0.12 | 629 | 135.8 ± 0.69 | 0.16 |
| 437 | 91.3 ± 0.93 | 0.20 | 525 | 80.3 ± 3.26 | 0.26 | 630 | 119 ± 1.21 | 0.16 |
| 438 | 93.2 ± 1.63 | 0.20 | 527 | 87.9 ± 1.11 | 0.21 | 633 | 139.8 ± 1.78 | 0.14 |
| 440 | 88.7 ± 0.67 | 0.27 | 528 | 88.3 ± 6.17 | 0.18 | 634 | 117.1 ± 0.28 | 0.11 |
| 441 | 90 ± 0.98 | 0.22 | 529 | 80.5 ± 2.12 | 0.23 | 635 | 116.5 ± 0.49 | 0.19 |
| 442 | 88.1 ± 1.21 | 0.27 | 530 | 84.7 ± 1.12 | 0.22 | 637 | 112.5 ± 20.92 | 0.18 |
| 444 | 90 ± 0.55 | 0.20 | 536 | 82.5 ± 1.07 | 0.20 | 638 | 133.6 ± 0.37 | 0.14 |
| 445 | 94.7 ± 0.95 | 0.24 | 562 | 90 ± 0.5 | 0.21 | 639 | 132.9 ± 0.48 | 0.13 |
| 446 | 92.2 ± 0.76 | 0.19 | 563 | 91 ± 0.57 | 0.20 | 642 | 102.2 ± 0.36 | 0.20 |
| 447 | 88.9 ± 0.82 | 0.25 | 565 | 118.7 ± 0.55 | 0.19 | 643 | 72.9 ± 4.46 | 0.25 |
| 448 | 87.7 ± 12.1 | 0.27 | 566 | 80.8 ± 0.57 | 0.25 | 645 | 126.4 ± 1.33 | 0.23 |
| 449 | 111.9 ± 0.91 | 0.20 | 567 | 82 ± 0.55 | 0.25 | 646 | 102.5 ± 0.81 | 0.24 |
| 450 | 113.7 ± 0.18 | 0.10 | 570 | 82.8 ± 0.73 | 0.22 | 647 | 100.9 ± 3.01 | 0.26 |
| 451 | 91.1 ± 0.31 | 0.23 | 571 | 84.2 ± 0.65 | 0.21 | 650 | 135.4 ± 0.2 | 0.17 |
| 452 | 94.5 ± 4.93 | 0.14 | 574 | 150.7 ± 0.95 | 0.10 | 654 | 131.5 ± 0.56 | 0.15 |
| 453 | 104.3 ± 9.4 | 0.19 | 577 | 92.6 ± 5.71 | 0.21 | 655 | 132.6 ± 0.98 | 0.16 |
| 454 | 105.5 ± 1.32 | 0.14 | 578 | 126.4 ± 0.42 | 0.13 | 657 | 121.4 ± 0.78 | 0.17 |

**Table 12**

| LNP (Lipid number) | Particle size (nm.d) | PDI | LNP (Lipid number) | Particle size (nm.d) | PDI | LNP (Lipid number) | Particle size (nm.d) | PDI |
|---|---|---|---|---|---|---|---|---|
| 658 | 114.4 ± 1.86 | 0.18 | 730 | 135.9 ± 0.47 | 0.20 | 890 | 134.1 ± 0.84 | 0.15 |
| 659 | 118.1 ± 0.45 | 0.18 | 734 | 166.5 ± 1.59 | 0.20 | 893 | 155.7 ± 0.94 | 0.13 |
| 661 | 77 ± 2.4 | 0.25 | 735 | 169.7 ± 1.15 | 0.19 | 897 | 137.2 ± 1.26 | 0.14 |
| 662 | 90.1 ± 0.02 | 0.25 | 738 | 157.1 ± 2.04 | 0.17 | 898 | 139.7 ± 0.37 | 0.16 |
| 663 | 94.4 ± 2 | 0.27 | 741 | 108.5 ± 1.27 | 0.17 | 903 | 146.8 ± 2.37 | 0.12 |
| 665 | 90.5 ± 0.22 | 0.45 | 742 | 111 ± 1.64 | 0.15 | 904 | 142 ± 0.08 | 0.16 |
| 666 | 70.9 ± 1.1 | 0.24 | 743 | 114.3 ± 0 | 0.13 | 907 | 140.2 ± 2.47 | 0.14 |
| 667 | 87.7 ± 0.27 | 0.00 | 745 | 130.3 ± 2.49 | 0.15 | 909 | 146.9 ± 1.51 | 0.12 |
| 669 | 104.4 ± 11.86 | 0.19 | 746 | 135.2 ± 0.62 | 0.15 | 910 | 143.8 ± 0.77 | 0.14 |
| 670 | 79.5 ± 3.95 | 0.26 | 749 | 137.3 ± 1.89 | 0.13 | 913 | 135.2 ± 0.42 | 0.13 |
| 671 | 87.4 ± 0.77 | 0.26 | 750 | 133.5 ± 1.02 | 0.16 | 914 | 149.6 ± 1.89 | 0.14 |
| 672 | 98.5 ± 0.12 | 0.00 | 751 | 135.5 ± 0 | 0.12 | 915 | 143.3 ± 0.83 | 0.12 |
| 673 | 97.1 ± 1.53 | 0.22 | 753 | 100.4 ± 0.95 | 0.18 | 918 | 141.4 ± 0.33 | 0.11 |
| 674 | 108.1 ± 1.98 | 0.22 | 754 | 102.3 ± 0.03 | 0.19 | 919 | 139.3 ± 1.06 | 0.12 |
| 675 | 112 ± 0.65 | 0.21 | 757 | 126.5 ± 1.11 | 0.24 | 920 | 112.8 ± 0.52 | 0.2 |
| 677 | 90.1 ± 2.39 | 0.24 | 758 | 124.2 ± 0.48 | 0.22 | 921 | 82.6 ± 1.73 | 0.22 |
| 678 | 99.8 ± 2.09 | 0.30 | 765 | 104.7 ± 1.42 | 0.17 | 921 | 140.7 ± 0 | 0.14 |
| 679 | 104 ± 1.37 | 0.28 | 766 | 107.5 ± 2.17 | 0.16 | 922 | 99.8 ± 0.79 | 0.23 |
| 682 | 120 ± 0.25 | 0.00 | 769 | 122.8 ± 1.09 | 0.15 | 923 | 81.9 ± 0.92 | 0.24 |
| 683 | 103.7 ± 0.27 | 0.00 | 770 | 120.6 ± 2.31 | 0.16 | 924 | 91 ± 0.88 | 0.21 |
| 685 | 284.6 ± 233.17 | 0.22 | 780 | 183.2 ± 2.04 | 0.20 | 925 | 85 ± 3.37 | 0.24 |
| 686 | 124.2 ± 0.8 | 0.16 | 781 | 179.1 ± 0.1 | 0.19 | 926 | 98.7 ± 1.26 | 0.21 |
| 687 | 122.6 ± 1.45 | 0.16 | 784 | 127.4 ± 0.11 | 0.18 | 927 | 118 ± 0.96 | 0.16 |
| 689 | 176.8 ± 2.98 | 0.21 | 785 | 127.6 ± 0.38 | 0.15 | 928 | 131.5 ± 0.22 | 0.09 |
| 690 | 90 ± 5.23 | 0.20 | 788 | 127.2 ± 2.9 | 0.18 | 935 | 159.7 ± 0.42 | 0.26 |
| 691 | 100.4 ± 5.95 | 0.18 | 789 | 121.4 ± 0.4 | 0.18 | 936 | 160.5 ± 2.71 | 0.19 |
| 693 | 226.7 ± 1.99 | 0.23 | 796 | 99.2 ± 1 | 0.18 | 939 | 115.4 ± 0.71 | 0.22 |
| 694 | 199.6 ± 5.06 | 0.24 | 797 | 101.2 ± 0.69 | 0.19 | 940 | 116.8 ± 0.31 | 0.23 |
| 695 | 141.2 ± 1.51 | 0.19 | 807 | 102.4 ± 2 | 0.19 | 942 | 135.8 ± 2.13 | 0.25 |
| 697 | 113.5 ± 0.24 | 8.93 | 808 | 106.4 ± 3.19 | 0.19 | 943 | 210.5 ± 0.96 | 0.18 |
| 698 | 143.1 ± 0.44 | 0.17 | 811 | 93.8 ± 0.74 | 0.28 | 944 | 212.5 ± 0.09 | 0.19 |
| 701 | 68.7 ± 5.19 | 0.28 | 812 | 95.3 ± 1.22 | 0.26 | 946 | 143.8 ± 0.61 | 0.12 |
| 702 | 228 ± 0.66 | 0.25 | 815 | 86.4 ± 0.75 | 0.25 | 947 | 116.9 ± 0.52 | 0.21 |
| 703 | 136.9 ± 0.54 | 0.19 | 816 | 87.9 ± 2.65 | 0.23 | 949 | 115.8 ± 0.26 | 0.19 |
| 705 | 142.2 ± 0.35 | 0.11 | 819 | 231.6 ± 4.63 | 0.26 | 952 | 133.6 ± 1.17 | 0.16 |
| 706 | 168 ± 1.67 | 0.11 | 820 | 240.9 ± 0.73 | 0.23 | 953 | 131.3 ± 2.17 | 0.17 |
| 707 | 164.7 ± 1.72 | 0.14 | 823 | 151.3 ± 0.38 | 0.18 | 958 | 98.5 ± 1.21 | 0.18 |
| 713 | 189.4 ± 3.62 | 0.25 | 827 | 150.5 ± 1.14 | 0.16 | 959 | 96.1 ± 1.06 | 0.21 |
| 714 | 163.3 ± 2.6 | 0.14 | 833 | 154.9 ± 1.33 | 0.20 | 962 | 149.4 ± 1.48 | 0.14 |
| 715 | 158.1 ± 0.01 | 0.13 | 837 | 91 ± 0.11 | 0.11 | 963 | 152.3 ± 0.29 | 0.16 |
| 718 | 213.4 ± 2.21 | 0.29 | 838 | 90.8 ± 1.03 | 0.11 | | | |
| 719 | 217.9 ± 7.06 | 0.29 | 841 | 145.6 ± 1.13 | 0.16 | | | |
| 722 | 265.1 ± 4.53 | 0.29 | 847 | 137.7 ± 0.21 | 0.16 | | | |
| 723 | 274.1 ± 1.84 | 0.28 | 866 | 124.5 ± 0.9 | 0.13 | | | |
| 725 | 170.8 ± 4.28 | 0.21 | 867 | 126.3 ± 1.04 | 0.14 | | | |
| 726 | 171 ± 2.57 | 0.13 | 871 | 189.5 ± 1.95 | 0.24 | | | |
| 727 | 176.1 ± 0.21 | 0.13 | 885 | 170.5 ± 1.71 | 0.12 | | | |
| 729 | 144.4 ± 1.59 | 0.19 | 888 | 136.6 ± 1.25 | 0.18 | | | |

### Example 9 In vitro mRNA delivery

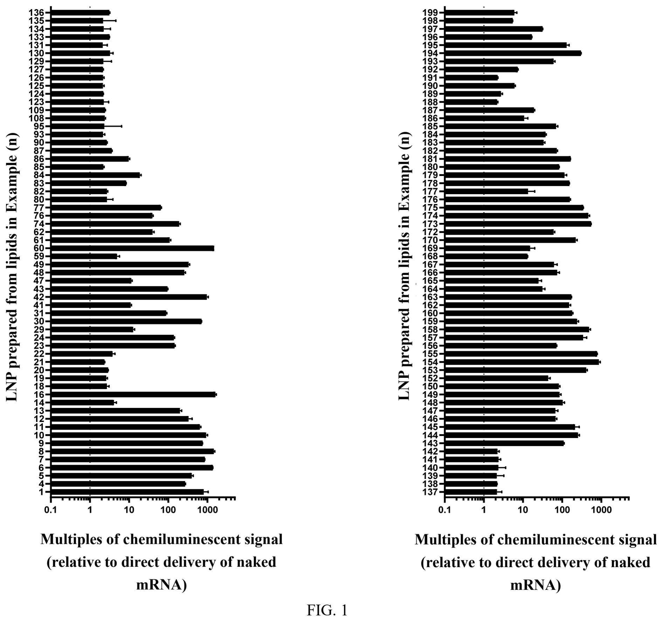

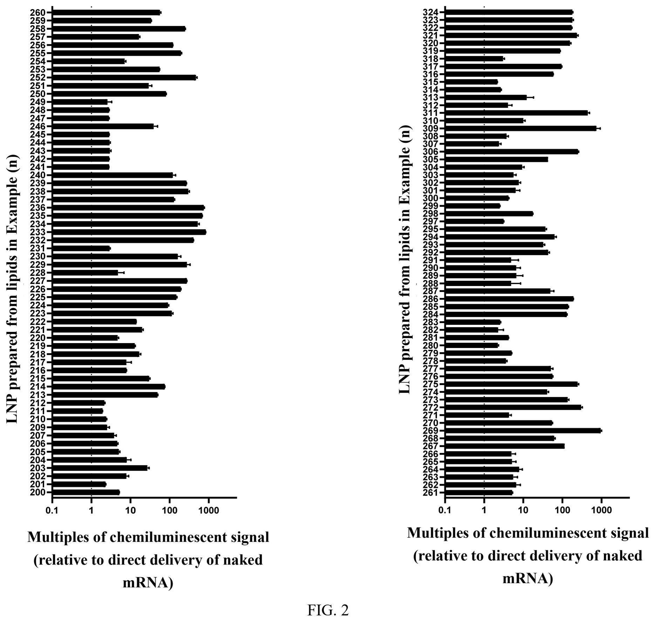

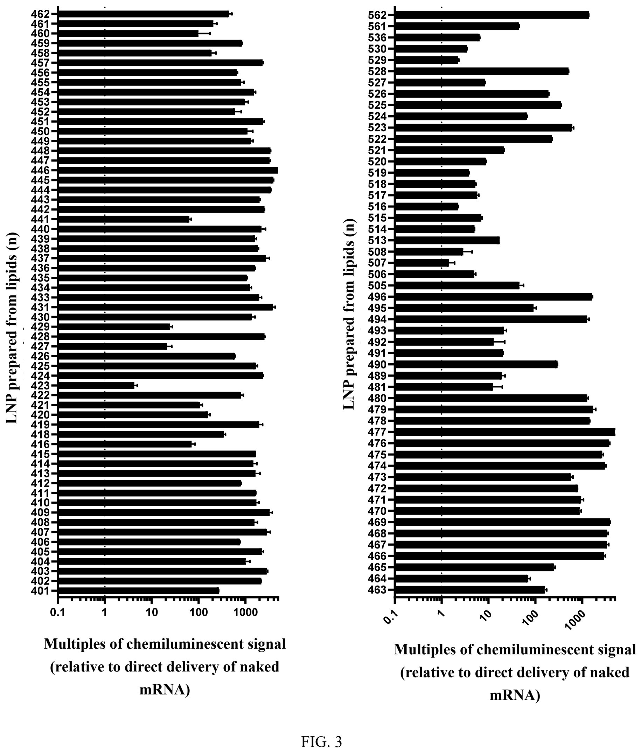

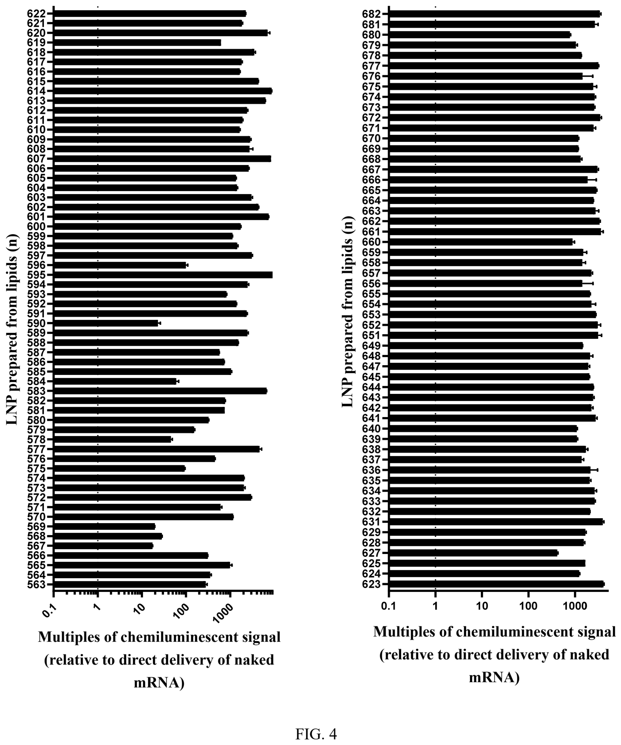

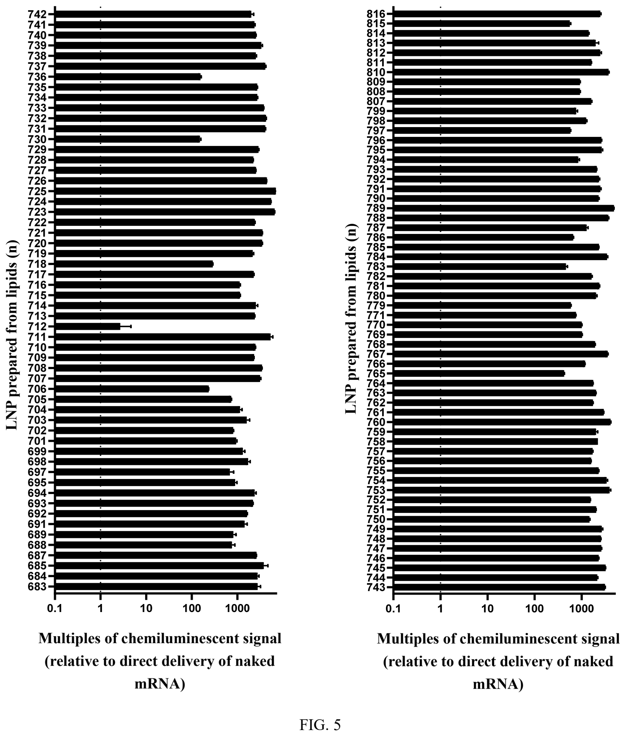

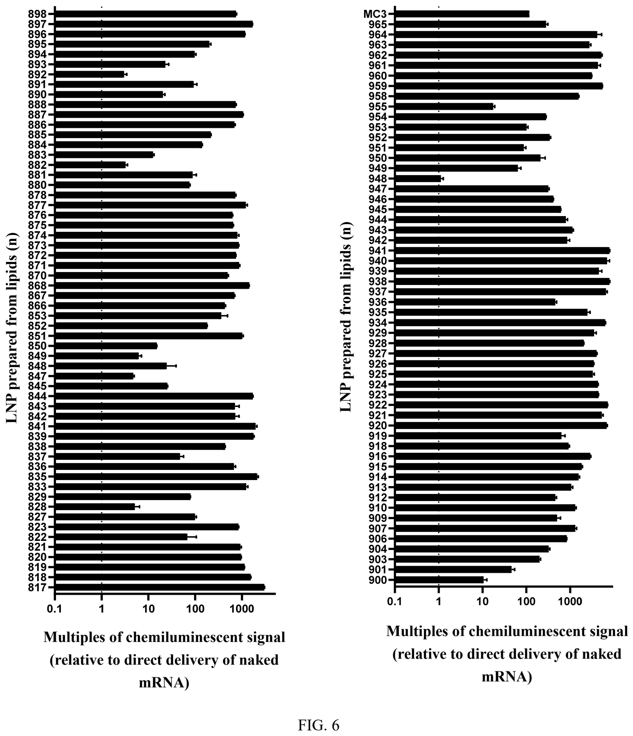

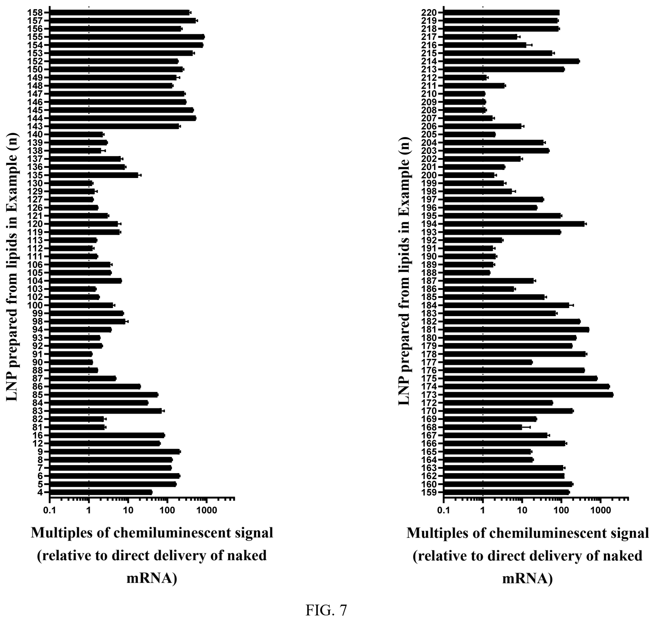

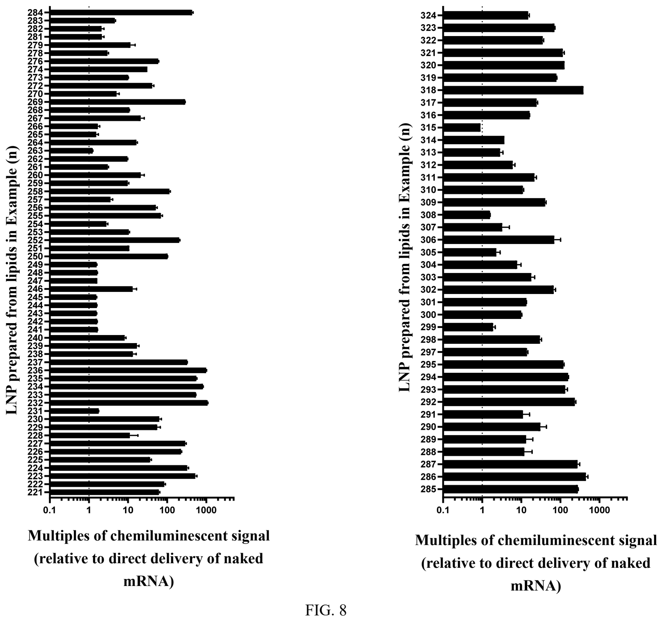

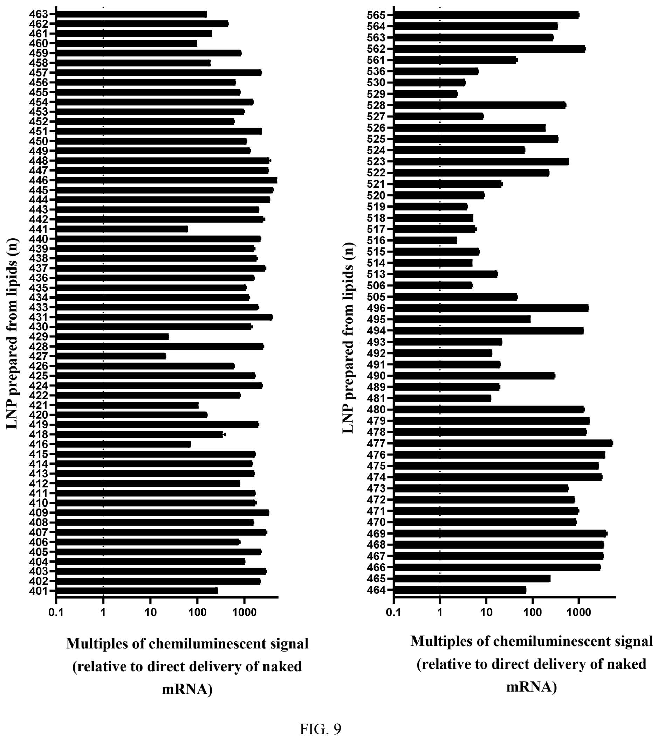

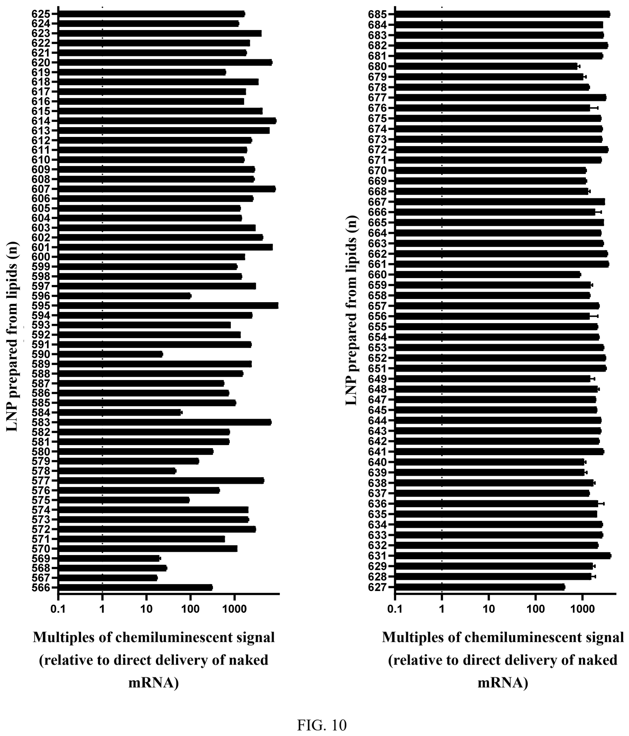

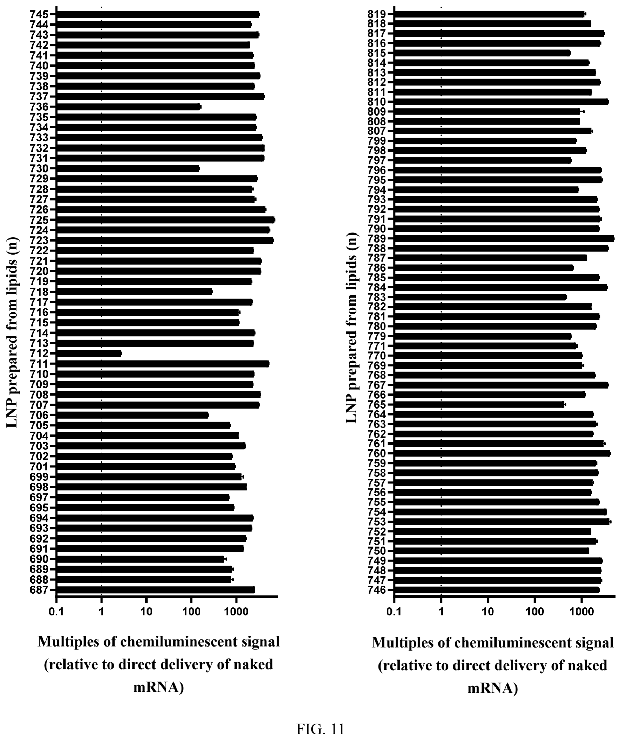

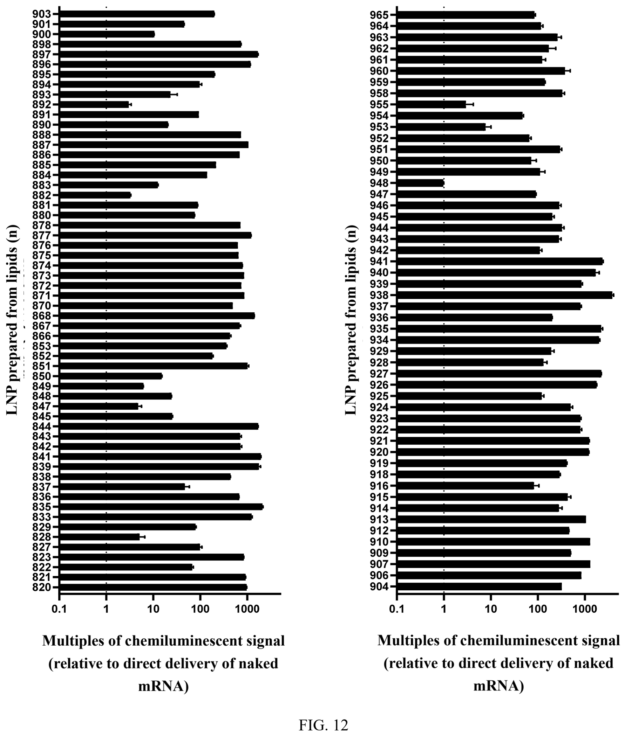

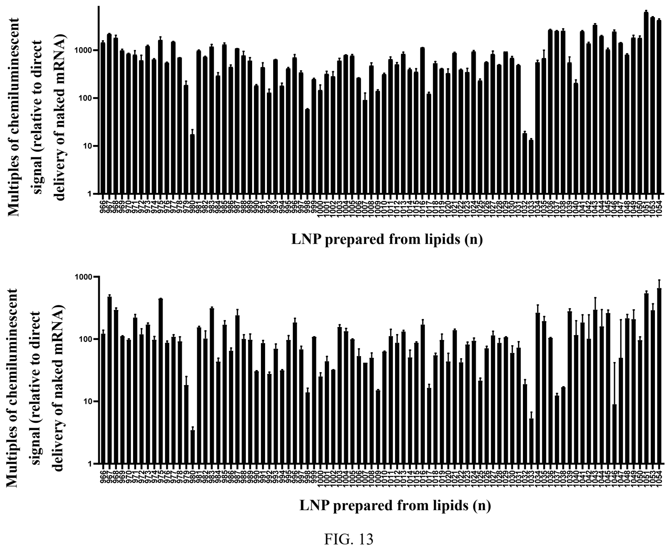

Human embryonic kidney cells (293T cells) and human non-small cell lung cancer cell lines (A549 cells) were obtained from the Chinese Type Culture Collection Center and cultured in DMEM high glucose culture medium containing 10% fetal bovine serum under the environment of 37°C and 5% CO₂. The cells were inoculated in a 96-well cell culture plate in a density of 32,000 cells per well. LNPs coating mRNA expressing luciferase (Trilink, USA) were taken and added into the wells at a dose of 100 ng of mRNA per well. Meanwhile, control wells were set on each culture plate, and 100 ng of naked mRNA that was not coated by LNPs was added into each well. The cell culture plate was placed in a cell incubator for 24 h of co-culture under the conditions of 37°C and 5% CO₂, and then the transfection effect of mRNA was detected through a One-LumiTM firefly luciferase reporter gene detection kit. The transfection results of LNPs in 293T cells are shown in FIG. 1 to FIG. 6 and FIG. 13 (upper), and the transfection results of LNPs in A549 cells are shown in FIG. 7 to FIG. 12 and FIG. 13 (lower). The transfection efficiency is expressed as a ratio of chemiluminescent intensity of LNP group to chemiluminescent intensity of control group (direct delivery of naked luciferase mRNA that is not coated by LNP).

By analysis on in vitro transfection effect, it is found that all the LNPs prepared by most of the lipids can successfully achieve luciferase expression, which is several times or even thousands of times higher than that of control group, in 293T cells. However, the abilities of LNPs prepared by the same ionizable lipid to deliver 293T cells and A549 cells vary. All the LNPs composed of some lipids, for example lipids 6, 9, 153, 154, 155, 157, 174 and 236, show relatively high delivery efficiency in the two cells; while LNPs composed of some lipids, for example lipids 238, 275 and 277, show selective and efficient delivery of Flue mRNA for 293T cells, and LNPs composed of for example lipids 85 and 318 show selective and efficient delivery of Flue mRNA for A549T cells.

By further analysis, it is found that many lipids have similar structures, which is manifested as having similar R_{I}, R_{II}, R_{III} and R_{IV} groups, however, the positions of the four groups in formula (I) are different, for example for lipids 251, 252, 253, 255, 257, 259, 260, 261, 262, 264 and 266, they are isomers with lipid 4, but the positions of tertiary amine or hydrophobic long-chain groups in the lipids are different from each other. By comparing their transfection effects corresponding to LNP, it is found that when R_{III} is a group containing an ionizable tertiary amine structure and the LNPs composed of lipid 4 and lipid 252 in which R_{I} and R_{IV} are hydrophobic long-chain groups have the highest transfection efficiency in 293T cells; similarly, lipid 5 and lipids 269, 270, 272, 274, 276, 277, 278, 279, 281 and 283 are isomers of each other, wherein the transfection abilities of LNPs composed of lipid 5 and lipid 269 in 293T and A549 cells are all significantly higher than those of LNPs composed of other lipids. Their common features are as follows: the R_{III} group is the ionizable tertiary amine group, and R_{I} and R_{IV} are hydrophobic long-chain groups.

### Example 10 mRNA delivery in mice

The animal experiments were reviewed and approved by the Ethics Committee of Zhejiang Medical College and the Ethics Committee of Zhejiang University of Technology. 6- to 8-week-old male C57Bl/6 mice were randomly grouped with 3 mice in each group. The mice in each group received a lipid nanoparticle coating mRNA expressing a luciferase via tail vein injection at a dose of 0.25 mg/kg mouse body weight (calculated by the mass of coated mRNA). After 6-hour injection of lipid nanoparticles was received, a luciferase substrate (D-Luciferin dissolved into sterile PBS) was intraperitoneally injected into the mice at a dose of 150 mg/kg mouse body weight, and the chemiluminescence in anesthetized mice was observed in an aminal IVIS imaging system (IVIS) within 10 to 15 min after injection. The mRNA delivery effect of the lipid nanoparticle is quantitatively compared through a total chemiluminiscence quantity (Total Flux p/s) of a mouse whole body within unit time (shown in Table 5). The results show that the LNP (formulation 2) composed of these lipids successively delivering mRNA expressing the luciferase *in vitro* can also successively achieve the delivery of mRNA expressing the luciferase in the animal, the delivery abilities of multiple lipids are stronger than the delivery ability of LNP composed of SM102 (formulation 1).

Table 13 and Table 14 show whole body chemiluminescent signals when LNPs (formulation 2) composed of the lipids in some examples deliver Flue mRNA in mice. The delivery dose of Flue mRNA was 0.25 mg/kg. 6 h after LNP injection, 150 mg/kg of D-luciferin was intraperitoneally injected into the mice, and then the whole body chemiluminescent signals were collected in the IVIS imaging system after 10 min.

**Table 13: The effects of LNPs prepared by using the following ionizable lipids to deliver mRNA expressing luciferase in mice**

| Name | Chemiluminescent signal (p/s) | Name | Chemiluminescent signal (p/s) |
|---|---|---|---|
| Lipid 1 | 8.32E+07 | Lipid 157 | 4.26E+09 |
| Lipid 4 | 7.14E+08 | Lipid 158 | 3.83E+08 |
| Lipid 5 | 1.19E+09 | Lipid 159 | 2.30E+09 |
| Lipid 6 | 3.96E+07 | Lipid 160 | 1.07E+10 |
| Lipid 7 | 3.64E+08 | Lipid 162 | 6.66E+09 |
| Lipid 8 | 4.32E+09 | Lipid 163 | 1.73E+09 |
| Lipid 9 | 6.22E+08 | Lipid 166 | 2.98E+09 |
| Lipid 10 | 1.49E+08 | Lipid 170 | 5.52E+08 |
| Lipid 11 | 1.32E+08 | Lipid 173 | 5.48E+08 |
| Lipid 12 | 6.06E+07 | Lipid 174 | 3.15E+09 |
| Lipid 13 | 4.06E+08 | Lipid 175 | 7.07E+09 |
| Lipid 16 | 6.94E+09 | Lipid 176 | 9.19E+08 |
| Lipid 23 | 2.33E+08 | Lipid 178 | 9.50E+08 |
| Lipid 24 | 1.21E+08 | Lipid 179 | 2.60E+09 |
| Lipid 30 | 1.35E+08 | Lipid 181 | 3.86E+09 |
| Lipid 31 | 1.85E+09 | Lipid 186 | 7.02E+08 |
| Lipid 42 | 1.10E+09 | Lipid 188 | 1.92E+10 |
| Lipid 48 | 1.88E+09 | Lipid 189 | 3.32E+08 |
| Lipid 49 | 3.48E+09 | Lipid 194 | 3.84E+09 |
| Lipid 60 | 4.10E+07 | Lipid 195 | 1.43E+09 |
| Lipid 61 | 2.45E+08 | Lipid 223 | 1.26E+10 |
| Lipid 62 | 6.40E+07 | Lipid 224 | 9.35E+08 |
| Lipid 74 | 3.57E+08 | Lipid 225 | 6.58E+08 |
| Lipid 75 | 3.07E+08 | Lipid 226 | 9.30E+09 |
| Lipid 79 | 1.10E+09 | Lipid 227 | 4.78E+09 |
| Lipid 86 | 3.23E+09 | Lipid 229 | 1.48E+10 |
| Lipid 87 | 3.47E+09 | Lipid 230 | 7.65E+09 |
| Lipid 88 | 3.64E+08 | Lipid 232 | 1.48E+08 |
| Lipid 93 | 1.26E+08 | Lipid 233 | 7.91E+08 |
| Lipid 94 | 1.82E+08 | Lipid 234 | 1.16E+10 |
| Lipid 105 | 2.55E+08 | Lipid 235 | 3.22E+09 |
| Lipid 107 | 6.37E+08 | Lipid 236 | 2.98E+09 |
| Lipid 108 | 4.23E+09 | Lipid 237 | 3.86E+09 |
| Lipid 113 | 3.39E+08 | Lipid 238 | 1.03E+08 |
| Lipid 116 | 1.01E+09 | Lipid 239 | 7.76E+08 |
| Lipid 143 | 1.13E+10 | Lipid 240 | 5.76E+08 |
| Lipid 144 | 1.32E+10 | Lipid 284 | 2.42E+10 |
| Lipid 145 | 9.95E+08 | Lipid 285 | 2.91E+10 |
| Lipid 153 | 1.61E+09 | Lipid 286 | 4.78E+10 |
| Lipid 154 | 4.78E+09 | Lipid 287 | 1.23E+10 |
| Lipid 155 | 5.58E+09 | SM102 | 3.93E+09 |

**Table 14: The effects of LNPs prepared by using the following ionizable lipids to deliver mRNA expressing luciferase in mice**

| Lipid name | Chemiluminescent signal (p/s) | Lipid name | Chemiluminescent signal (p/s) |
|---|---|---|---|
| 401 | 5.92E+10 | 695 | 5.34E+09 |
| 402 | 4.20E+10 | 914 | 4.04E+09 |
| 406 | 1.04E+11 | 915 | 5.79E+09 |
| 407 | 8.00E+10 | 916 | 5.23E+08 |
| 408 | 1.03E+11 | 919 | 2.45E+08 |
| 409 | 3.08E+10 | 921 | 7.83E+10 |
| 420 | 6.69E+09 | 923 | 9.90E+10 |
| 424 | 8.40E+ 10 | 924 | 1.01E+11 |
| 431 | 5.11E+10 | 926 | 5.48E+10 |
| 445 | 2.15E+09 | 928 | 1.13E+11 |
| 446 | 8.22E+10 | 929 | 2.49E+10 |
| 447 | 1.80E+10 | 934 | 1.88E+10 |
| 583 | 1.20E+10 | 937 | 1.01E+08 |
| 595 | 9.60E+10 | 939 | 8.00E+09 |
| 598 | 1.86E+09 | 940 | 1.69E+09 |
| 607 | 1.08E+11 | 941 | 1.96E+10 |
| 613 | 9.90E+10 | 942 | 8.15E+08 |
| 620 | 3.99E+09 | 943 | 1.04E+10 |
| 631 | 3.81E+09 | 944 | 2.74E+10 |
| 641 | 2.46E+10 | 945 | 1.18E+10 |
| 645 | 2.65E+09 | 946 | 5.05E+10 |
| 651 | 1.93E+09 | 947 | 3.71E+09 |
| 653 | 3.75E+09 | 954 | 4.93E+10 |
| 661 | 3.52E+08 | 958 | 9.75E+10 |
| 665 | 1.13E+09 | 959 | 2.51E+10 |
| 675 | 5.31E+10 | 961 | 1.61E+08 |
| 683 | 1.47E+08 | 962 | 7.10E+09 |
| 685 | 1.08E+11 | 964 | 3.56E+08 |
| 687 | 4.75E+09 | SM102 | 3.93E+09 |
| 789 | 1.14E+11 | 1034 | 2.06E+10 |
| 930 | 6.94E+09 | 1035 | 8.16E+10 |
| 931 | 6.13E+07 | 1037 | 2.43E+10 |
| 949 | 6.88E+05 | 1038 | 3.75E+10 |
| 950 | 3.41E+07 | 1039 | 3.31E+06 |
| 967 | 8.01E+10 | 1041 | 3.31E+06 |
| 983 | 4.11E+09 | 1046 | 1.81E+08 |
| 1024 | 4.21E+08 | 1049 | 1.62E+09 |

### Example 11 In vitro eGFP plasmid transfection

293T cells were inoculated into a 96-well cell culture plate at a density of 40,000 cells per well, LNPs prepared from lipid 284 and lipid 424 was used to coat plasmid DNA expressing eGFP, lipofectamine 3000 was used as a control group, 100 ng of plasmid DNA coated by the above vehicle was given to cells per well, the expression of eGFP was observed through a fluorescence microscope after co-culture for 48 h. The results in FIG. 17 show that all lipid LNPs can effectively transfect plasmid DNA *in vitro,* and delivery efficiencies and positive cell ratios exceed those in control group lipofectamine3000.

### Example 12 Optimization of lipid 284 LNP formulation

For lipid 284, the effect of an LNP composition on physicochemical properties and delivery efficiency of nanoparticles was studied by changing the N/P of an ionizable lipid to mRNA in the LNP composition. 5 LNPs with N/P being from 2.8 to 6.0 were respectively prepared by using each lipid ratio in formulation 2 as described above. The physicochemical parameters of the LNPs are shown in Table 15, showing that with a change in N/P, the particle size and mRNA encapsulation efficiency of the overall LNP have no significant change, however, its *in vivo* delivery efficiency significantly increases with an increase in N/P at the same mRNA dose. Based on the same lipid composition ratio in formulation 2, the physicochemical properties of two LNPs with N/P=2.8 and 6.0 were compared after DOPE was replaced with DOPC. The results show that in the DOPC formulation, an increase in N/P reduces the final nano size to a certain extent.

**Table 15**

| Formulation | | Diam.(nm) | PDI | Encapsulation efficiency (%) | *In vivo* delivery (p/s)^{a} |
|---|---|---|---|---|---|
| 1 | Lipid 284, DOPE-N/P=2.8 | 75.72 | 0.146 | 92.91% | 2.42E+10 |
| 2 | Lipid 284, DOPE-N/P=3.6 | 75.39 | 0.170 | 96.84% | 6.94E+10 |
| 3 | Lipid 284, DOPE-N/P=4.4 | 71.93 | 0.166 | 94.73% | 4.15E+10 |
| 4 | Lipid 284, DOPE-N/P=5.2 | 71.87 | 0.139 | 96.59% | 1.67E+11 |
| 5 | Lipid 284, DOPE-N/P=6.0 | 88.77 | 0.120 | 95.83% | 7.33E+11 |
| 6 | Lipid 284, DOPC-N/P=2.8 | 102.17 | 0.217 | 96.79% | - |
| 7 | Lipid 284, DOPC-N/P=6.0 | 89.00 | 0.140 | 96.13% | - |

^{a} LNPs containing 5 µg of luc mRNA were injected into C57Bl//6 mice via a tail vein, a substrate D-luciferin was given via intraperitoneal injection after 6 h, and the chemiluminescent values of abdominal positions of mice were recorded through the IVIS system after 10 min.

### Example 13 Lipid LNP delivery gene editing system

LNPs were prepared by using a composition ratio in formulation 2 and simultaneously coating spCas9 mRNA and sgRNA (sgGFP sequence: CAACTACAAGACCCGCGCCG) with lipid 284, wherein N/P was 6, and a mass ratio of mRNA to sgRNA was 3/1. By DLS detection, the particle size of LNP was about 130 nm, PDI was 0.15, and the RNA encapsulation efficiency was greater than 95%. An HEK 293/eGFP-aavs1 stable cell line stably expressing a green fluorescence protein (GFP) was inoculated into a 96-well cell culture plate at a density of 40,000 cells per well. After incubation for 24 h, LNPs containing 200 ng of total RNA were added into each well, cells were collected after 48 h and the knockout efficiency cells of GFP were detected on a flow cytometer. The results show that average 41.2% of 293T cells has significant knockdown of GFP expression, indicating that LNP-Cas9 sgGFP transfection using an enhanced green fluorescent protein (EGFP) gene as a target can achieve the cleavage of the EGFP gene, and is repaired through non-homologous terminal junction (NHEJ), thereby generating frameshift mutations to cause the knockdown effect of GFP expression.

### Example 14 Preparation and research of respiratory syncytial virus (RSV) vaccine

An RSV antigen mRNA sequence with a pre-fusion F protein conformation was prepared by a cap analogue co-transcription method, the sequence information is as shown in sequence 1 (SEQ ID NO: 1), and the amino acid sequence of the expressed antigen protein is as shown in sequence 2 (SEQ ID NO: 2). LNPs containing RSV-mRNA were prepared by respectively using lipid 174 and sm102 in a lipid ratio of formulation 1, wherein N/P was 6. The particle sizes and mRNA encapsulation efficiency data of two LNPs are as follows: lipid 174 has a particle size of 77.6 nm and an encapsulation efficiency of 96.2%; and sm102 has a particle size of 96.1 nm and an encapsulation efficiency of 97.9%. The LNP preparation encapsulating RSV mRNA and an F protein antigen with a pre-fusion conformation were intramuscularly administrated to BALB/c mice at a dose of 10 µg per mouse, with immunization once every three weeks for two immunizations in total. 14 days after secondary immunization, mouse serum was collected and submitted to CRO for detection of RSV true virus neutralizing antibody titers. As shown in FIG. 18, compared with direct inoculation of antigen proteins or an SM102 lipid delivery system, the LNPs prepared from lipid 174 can efficiently stimulate the mouse to generate a neutralizing antibody targeting RSV.

Sequences used herein are as follows:

Sequence 1 (SEQ ID NO: 1)
AATAAACTAGTATTCTTCTGGTCCCCACAGACTCAGAGAGAACCCGCCACCATGGAGCTGCTGATCCTG
AAGGCCAACGCTATCACCACCATCCTGACCGCCGTGACCTTCTGCTTCGCCTCTGGCCAGAACATAACA
GAGGAATTCTACCAGTCTACCTGTTCTGCTGTTTCCAAGGGCTACCTGAGCGCCCTGCGGACAGGCTGG
TACACCAGCGTGATCACAATCGAGCTGTCTAATATCAAGGAGAACAAGTGCAATGGTACAGATGCCAAG
GTGAAGCTGATCAAGCAGGAGCTAGACAAGTACAAGAATGCCGTGACCGAACTGCAGCTCCTGATGCAG
AGCACACCCCCCTGTAACAACAGAGCCAGAAGGGAACTGCCACGGTTTATGAACTACACCCTGAACAAT
GCCAAGAAGACAAACGTGACCCTCTCTAAGAAAAGAAAGCGCAGATTCCTCGGCTTCCTGCTGGGCGTG
GGAAGCGCCTGCGCCAGCGGAGTGGCCGTGTCTAAAGTGCTGCACCTGGAAGGCGAGGTGAACAAGATT

AAGAGCGCTCTGCTGAGCACCAACAAGGCCGTCGTGTCCCTGAGCAATGGAGTGTCCGTGCTGACCATC
AAGGTGCTGGATCTGAAAAACTACATTGACAAGCAACTGCTGCCTATCGTGAACAAACAAAGCTGTAGC
ATCAGCAATATCGAAACAGTGATCGAGTTCCAGCAAAAGAACAACCGGCTGCTGGAGATCACAAGAGAA
TTCAGCGTCAATGCTGGCGTGACCACCCCTGTGAGCACCTACATGCTGACCAACAGCGAGCTGCTGAGC
CTGATAAATGACATGCCTATCACAAACGATCAGAAAAAGCTTATGAGCAACAACGTGCAGATCGTGCGG
CAGCAGAGCTACAGCATCATGTCTATCATCAAGGAAGAAGTGCTGGCCTACGTGGTCCAGCTGCCTCTG
TATGGCGTGATCGACACCCCTTGCTGGAAGCTGCACACCAGCCCTCTGTGTACAACCAACACAAAGGAA
GGCTCTAACATCTGCCTGACCAGAACGGATAGAGGCTGGTATTGCGACAACGCCGGCAGCGTGTCCTTT
TTCCCCCAGGCCGAGACATGCAAGGTGCAGTCTAACAGAGTGTTCTGTGATACCATGAACAGCCTGACC
CTGCCTTCTGAGATCAACCTGTGCAACGTCGACATCTTCAACCCCAAGTACGATTGCAAGATCATGACC
AGCAAAACAGACGTGTCCAGCAGCGTGATTACATCTCTGGGAGCCATCGTGAGCTGCTACGGAAAGACC
AAGTGCACAGCTAGCAACAAAAATAGAGGCATCATCAAAACCTTTAGCAACGGCTGCGACTACGTGAGC
AACAAAGGCATGGACACCGTGAGCGTTGGCAACACCCTATACTACGTGAACAAACAGGAGGGCAAGTCC
CTGTACGTCAAGGGCGAGCCTATCATCAACTTCTACGACCCCCTGGTGTTTCCTAGCAGCGAGTTCGAC
GCCAGCATCAGCCAGGTGAACGAGAAGATCAACCAGAGCCTGGCCTTCATCCGGAAGAGCGACGAGCTG
CTGCACAACGTGAATGCTGGCAAGTCCACCACCAACATCATGATCACAACTATCATCATCGTGATCATC
GTGATCCTGTTGAGCCTCATCGCCGTGGGCCTGCTGCTGTATTGCAAGGCTAGGTCCACCCCCGTGACA
CTGTCTAAGGATCAGCTGAGCGGCATCAACAATATCGCCTTTTCCAACTGACTCGAGCTGGTACTGCAT
GCACGCAATGCTAGCTGCCCCTTTCCCGTCCTGGGTACCCCGAGTCTCCCCCACCTCGGGTCCCAGGTA
TGCTCCCACCTCCACCTGCCCCACTCACCACCTCTGCTAGTTCCAGACACCTCCCAAGCACGCAGCAAT
GCAGCTCAAAACGCTTAGCCTAGCCACACCCCCACGGGAAACAGCAGTGATTAACCTTTAGCAATAAAC
GAAAGTTTAACTAAGCTATACTAACCCCAGGGTTGGTCAATTTCGTGCCAGCCACACCCTGGAGCTAGC
GGAT-polyA tail 120nt

Sequence 2 (SEQ ID NO: 2)
MELLILKANAITTILTAVTFCFASGQNITEEFYQSTCSAVSKGYLSALRTGWYTSVITIELSNIKENKC
NGTDAKVKLIKQELDKYKNAVTELQLLMQSTPPCNNRARRELPRFMNYTLNNAKKTNVTLSKKRKRRFL
GFLLGVGSACASGVAVSKVLHLEGEVNKIKSALLSTNKAVVSLSNGVSVLTIKVLDLKNYIDKQLLPIV
NKQSCSISNIETVIEFQQKNNRLLEITREFSVNAGVTTPVSTYMLTNSELLSLINDMPITNDQKKLMSN
NVQIVRQQSYSIMSIIKEEVLAYVVQLPLYGVIDTPCWKLHTSPLCTTNTKEGSNICLTRTDRGWYCDN
AGSVSFFPQAETCKVQSNRVFCDTMNSLTLPSEINLCNVDIFNPKYDCKIMTSKTDVSSSVITSLGAIV
SCYGKTKCTASNKNRGIIKTFSNGCDYVSNKGMDTVSVGNTLYYVNKQEGKSLYVKGEPIINFYDPLVF
PSSEFDASISQVNEKINQSLAFIRKSDELLHNVNAGKSTTNIMITTIIIVIIVILLSLIAVGLLLYCKA
RSTPVTLSKDQLSGINNIAFSN

Although various improvements have been described herein with reference to specific embodiments of the present disclosure, it should be understood that this description is merely illustrative, but should not be explained as limiting any claimed protective scopes of the present disclosure. Therefore, any claimed protective scopes and contents of the present disclosure are defined only by the terms of the attached claims in their current forms or as modified during the examination or as implemented in any continuing application. In addition, it should be understood that, unless otherwise specified, the features of any specific embodiments discussed herein may be combined with one or more features of any one or more embodiments discussed or considered in other ways herein.

## Claims

1. A lipid compound of formula (I), or an N-oxide, stereoisomer, or pharmaceutically acceptable salt thereof, 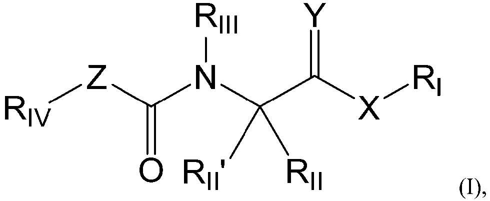 wherein X is O or NH; Y is O or S; Z is absent, or Z is O, NH, or S;
R_{II}' is hydrogen, or substituted or unsubstituted hydrocarbyl, substituted or unsubstituted heterohydrocarbyl, substituted or unsubstituted aryl, or substituted or unsubstituted heteroaryl having 1 to 11 carbon atoms; and
R_{I}, R_{II}, R_{III}, and R_{IV} are each independently selected from a group (A) comprising a group containing at least one ionizable tertiary amine structure, a group (B) comprising a substituted or unsubstituted aliphatic or substituted or unsubstituted heteroaliphatic group having 11 to 30 carbon atoms, and a group (C) comprising a substituted or unsubstituted hydrocarbyl, substituted or unsubstituted heterohydrocarbyl, substituted or unsubstituted aryl, or substituted or unsubstituted heteroaryl group having fewer than 11 carbon atoms,
provided that:
(1) when any one of R_{I}, R_{II}, R_{III}, and R_{IV} is a group containing at least one ionizable tertiary amine structure, any two of the remaining three are identical or different substituted or unsubstituted hydrocarbyl, substituted or unsubstituted heterohydrocarbyl, substituted or unsubstituted aryl, or substituted or unsubstituted heteroaryl groups having fewer than 11 carbon atoms, and the other is a substituted or unsubstituted aliphatic or substituted or unsubstituted heteroaliphatic group having 11 to 30 carbon atoms; or any two of the remaining three are identical or different substituted or unsubstituted aliphatic or substituted or unsubstituted heteroaliphatic groups having 11 to 30 carbon atoms, and the other is a substituted or unsubstituted hydrocarbyl, substituted or unsubstituted heterohydrocarbyl, substituted or unsubstituted aryl, or substituted or unsubstituted heteroaryl group having fewer than 11 carbon atoms;
or
(2) when any two of R_{I}, R_{II}, R_{III}, and R_{IV} are identical or different groups containing at least one ionizable tertiary amine structure, one of the remaining two is a substituted or unsubstituted hydrocarbyl, substituted or unsubstituted heterohydrocarbyl, substituted or unsubstituted aryl, or substituted or unsubstituted heteroaryl group having fewer than 11 carbon atoms, and the other is a substituted or unsubstituted aliphatic or substituted or unsubstituted heteroaliphatic group having 11 to 30 carbon atoms.

2. The lipid compound, or the N-oxide, stereoisomer, or pharmaceutically acceptable salt thereof according to claim 1, wherein only one of R_{I}, R_{II}, R_{III}, and R_{IV} is a group containing at least one ionizable tertiary amine structure.

3. The lipid compound, or the N-oxide, stereoisomer, or pharmaceutically acceptable salt thereof according to claim 1 or 2, wherein R_{II}' is hydrogen, or substituted or unsubstituted hydrocarbyl such as C₁-C₄ alkyl, substituted or unsubstituted heterohydrocarbyl, substituted or unsubstituted aryl, or substituted or unsubstituted heteroaryl having 1 to 11 carbon atoms;
the group A comprises a group containing at least one ionizable tertiary amine structure, wherein the group containing at least one ionizable tertiary amine structure has 3 to 11 carbon atoms and is represented by the following general formula, 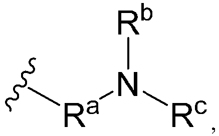
wherein R^{a} is substituted or unsubstituted C₁-C₆ alkylene; R^{b} and R^{c} are each independently substituted or unsubstituted C₁-C₆ alkyl, substituted or unsubstituted C₂-C₆ alkenyl, or substituted or unsubstituted C₂-C₆ alkynyl, which are optionally substituted with 1, 2, or 3 substituents selected from -OH, -SH, -NR^{d}R^{d}', or phenyl, wherein R^{d} and R^{d}' are each independently hydrogen or C₁-C₃ alkyl; or R^{b} and R^{c}, together with the N atom to which they are attached, form a 5- to 12-membered heterocyclic ring or heteroaromatic ring, the 5- to 12-membered heterocyclic ring or heteroaromatic ring containing 1, 2, or 3 heteroatoms independently selected from N, O, and S, wherein at least one heteroatom is N, and the 5-to 12-membered heterocyclic ring or heteroaromatic ring is optionally substituted with one or more C₁-C₆ alkyls or oxo (=O); or R^{a} and R^{b}, together with the N atom to which they are attached, form a 5-to 12-membered heterocyclic ring or heteroaromatic ring optionally substituted with C₁-C₆ alkylene, the 5- to 12-membered heterocyclic ring or heteroaromatic ring optionally substituted with C₁-C₆ alkylene comprising 1, 2, or 3 heteroatoms independently selected from N, O, and S, wherein at least one heteroatom is N, and the 5- to 12-membered heterocyclic ring or heteroaromatic ring optionally substituted with C₁-C₆ alkylene is optionally substituted with one or more C₁-C₆ alkyls or oxo (=O); or R^{a}, R^{b}, and R^{c}, together with the N atom to which they are attached, form a 5- to 12-membered heterocyclic ring or heteroaromatic ring optionally substituted with C₁-C₆ alkylene, the 5- to 12-membered heterocyclic ring or heteroaromatic ring optionally substituted with C₁-C₆ alkylene containing 1, 2, or 3 heteroatoms independently selected from N, O, and S, wherein at least one heteroatom is N, and the 5- to 12-membered heterocyclic ring or heteroaromatic ring optionally substituted with C₁-C₆ alkylene is optionally substituted with one or more C₁-C₆ alkyls or oxo (=O);
the group B comprises a substituted or unsubstituted aliphatic or substituted or unsubstituted heteroaliphatic group having 11 to 30 carbon atoms, wherein the substituted or unsubstituted aliphatic or substituted or unsubstituted heteroaliphatic group having 11 to 30 carbon atoms optionally contains 1, 2, 3, 4, 5, 6, 7, 8, 9, 10, 11, or 12 groups independently selected from -C=C-, -C≡C-, 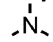 , -NH-, -NH₂, -OH, -OR^{m}, -O-, -C(O)-, -C(ORⁿ)-, -C(O)O-, -SH, -SR^{o}, -S-, -C(S)-, -C(SR^{p})-, -C(S)O-, and -P(O)-, wherein R^{m}, Rⁿ, R^{o}, and R^{p} are each independently substituted or unsubstituted C₁-C₁₄ aliphatic hydrocarbyl; and
the group C comprises a substituted or unsubstituted hydrocarbyl, substituted or unsubstituted heterohydrocarbyl, substituted or unsubstituted aryl, or substituted or unsubstituted heteroaryl group having 1 to 11 carbon atoms, wherein the substituted or unsubstituted hydrocarbyl, substituted or unsubstituted heterohydrocarbyl, substituted or unsubstituted aryl, or substituted or unsubstituted heteroaryl group having 1 to 11 carbon atoms optionally contains 1, 2, 3, 4, or 5 groups independently 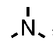 selected from -C=C-, -C=C-, , -NH-, -NH₂, -OH, -OR^{m'}, -O-, -C(O)-, -C(OR^{n'})- , -C(O)O-, -SH, -SR^{o'}, -S-, -C(S)-, -C(SR^{p'})-, -C(S)O-, and -P(O)-, wherein R^{m'}, R^{n'}, R^{o'}, and R^{p'} are each independently substituted or unsubstituted C₁-C₁₄ aliphatic hydrocarbyl; and the hydrocarbyl, heterohydrocarbyl, aryl, or heteroaryl group is optionally substituted with one or more substituents selected from the following group: deuterium, halogen, -NO₂, and -OH,
R_{I}, R_{II}, R_{III}, and R_{IV} satisfy the following conditions:
R_{I} is selected from the group A, R_{II} and R_{III} are selected from the group B, and R_{IV} is selected from the group C; or
R_{I} is selected from the group A, R_{II} and R_{IV} are selected from the group B, and R_{III} is selected from the group C; or
R_{I} is selected from the group A, R_{III} and R_{IV} are selected from the group B, and R_{II} is selected from the group C; or
R_{II} is selected from the group A, R_{I} and R_{III} are selected from the group B, and R_{IV} is selected from the group C; or
R_{II} is selected from the group A, R_{I} and R_{IV} are selected from the group B, and R_{III} is selected from the group C; or
R_{II} is selected from the group A, R_{III} and R_{IV} are selected from the group B, and R_{I} is selected from the group C; or
R_{III} is selected from the group A, R_{I} and R_{II} are selected from the group B, and R_{IV} is selected from the group C; or
R_{III} is selected from the group A, R_{I} and R_{IV} are selected from the group B, and R_{II} is selected from the group C; or
R_{III} is selected from the group A, R_{II} and R_{IV} are selected from the group B, and R_{I} is selected from the group C; or
R_{IV} is selected from the group A, R_{I} and R_{II} are selected from the group B, and R_{III} is selected from the group C; or
R_{IV} is selected from the group A, R_{I} and R_{III} are selected from the group B, and R_{II} is selected from the group C; or
R_{IV} is selected from the group A, R_{II} and R_{III} are selected from the group B, and R_{I} is selected from the group C.

4. The lipid compound, or the N-oxide, stereoisomer, or pharmaceutically acceptable salt thereof according to any one of claims 1 to 3, wherein R_{I}, R_{II}, R_{III}, and R_{IV} each independently optionally comprise at least one degradable group, preferably, R_{I}, R_{II}, R_{III} and R_{IV} also optionally comprise at least one degradable group when being each independently selected from the group B.

5. The lipid compound, or the N-oxide, stereoisomer, or pharmaceutically acceptable salt thereof according to claim 4, wherein the degradable group is selected from -C(O)O-, -OC(O)-, -OC(O)O-, -S-S-, -C(O)NH-, -NHC(O)-, -NHC(O)O-, -NR¹C(O)-, -C(O)NR²-, -NR³C(O)O-, -OP(O)OR⁴O-, -OCR⁵(OR⁶)O-, -CR⁷(OR⁸)O-, and -CH(OR⁹)O-,
wherein R¹, R², R³, R⁴, R⁵, R⁶, R⁷, R⁸, and R⁹ are each independently substituted or unsubstituted C₁-C₁₄ aliphatic hydrocarbyl.

6. The lipid compound, or the N-oxide, stereoisomer, or pharmaceutically acceptable salt thereof according to any one of claims 1 to 5, wherein
when R_{III} and R_{IV} are selected from the group B, one of R_{III} and R_{IV} has 16 to 30 carbon atoms, and the other has 11 to 25 carbon atoms;
when R_{II} and R_{IV} are selected from the group B, one of R_{II} and R_{IV} has 16 to 30 carbon atoms, and the other has 11 to 25 carbon atoms;
when R_{I} and R_{IV} are selected from the group B, one of R_{I} and R_{IV} has 16 to 30 carbon atoms, and the other has 11 to 25 carbon atoms;
when R_{I} and R_{II} are selected from the group B, one of R_{I} and R_{II} has 16 to 30 carbon atoms, and the other has 11 to 25 carbon atoms; and/or
when R_{I} and R_{III} are selected from the group B, one of R_{I} and R_{III} has 16 to 30 carbon atoms, and the other has 11 to 25 carbon atoms.

7. The lipid compound, or the N-oxide, stereoisomer, or pharmaceutically acceptable salt thereof according to any one of claims 1 to 6, wherein
when R_{III} and R_{IV} are selected from the group B, one of R_{III} and R_{IV} has 16 to 25 carbon atoms, and the other has 11 to 16 carbon atoms;
when R_{II} and R_{IV} are selected from the group B, one of R_{II} and R_{IV} has 16 to 25 carbon atoms, and the other has 11 to 16 carbon atoms;
when R_{I} and R_{IV} are selected from the group B, one of R_{I} and R_{IV} has 16 to 25 carbon atoms, and the other has 11 to 16 carbon atoms;
when R_{I} and R_{II} are selected from the group B, one of R_{I} and R_{II} has 16 to 25 carbon atoms, and the other has 11 to 16 carbon atoms; and/or
when R_{I} and R_{III} are selected from the group B, one of R_{I} and R_{III} has 16 to 25 carbon atoms, and the other has 11 to 16 carbon atoms.

8. The lipid compound, or the N-oxide, stereoisomer, or pharmaceutically acceptable salt thereof according to any one of claims 1 to 7, wherein
when R_{III} and R_{IV} are selected from the group B, at least one of R_{III} and R_{IV} comprises at least one degradable group;
when R_{II} and R_{IV} are selected from the group B, at least one of R_{II} and R_{IV} comprises at least one degradable group;
when R_{I} and R_{IV} are selected from the group B, at least one of R_{I} and R_{IV} comprises at least one degradable group;
when R_{I} and R_{II} are selected from the group B, at least one of R_{I} and R_{II} comprises at least one degradable group; and/or
when R_{I} and R_{III} are selected from the group B, at least one of R_{I} and R_{III} comprises at least one degradable group.

9. The lipid compound, or the N-oxide, stereoisomer, or pharmaceutically acceptable salt thereof according to any one of claims 1 to 8, wherein
when R_{III} and R_{IV} are selected from the group B, only one of R_{III} and R_{IV} comprises one degradable group;
when R_{II} and R_{IV} are selected from the group B, only one of R_{II} and R_{IV} comprises one degradable group;
when R_{I} and R_{IV} are selected from the group B, only one of R_{I} and R_{IV} comprises one degradable group; when R_{I} and R_{II} are selected from the group B, only one of R_{I} and R_{II} comprises one degradable group; and/or
when R_{I} and R_{III} are selected from the group B, only one of R_{I} and R_{III} comprises one degradable group.

10. The lipid compound, or the N-oxide, stereoisomer, or pharmaceutically acceptable salt thereof according to any one of claims 1 to 9, wherein the group A comprises groups as shown in the following structures:

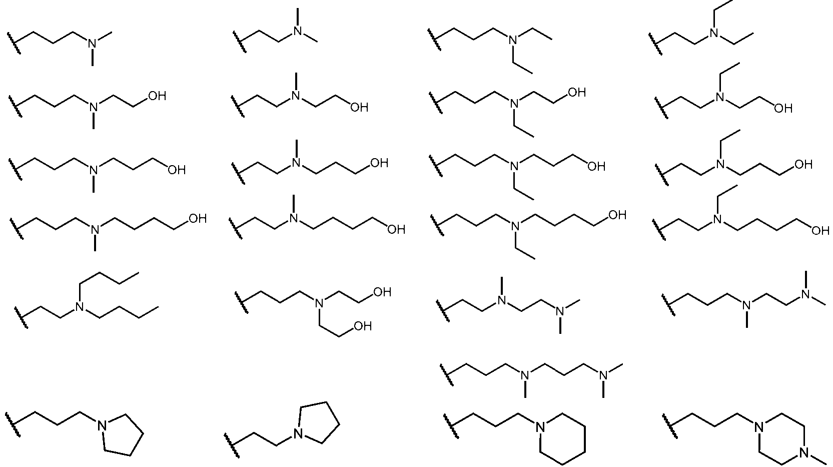

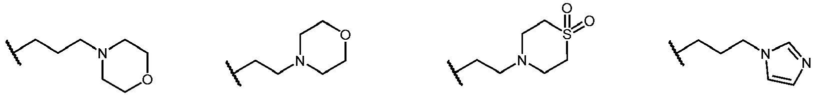

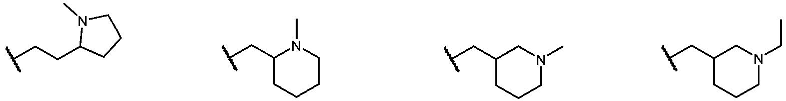

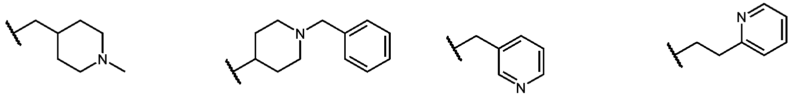

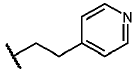

11. The lipid compound, or the N-oxide, stereoisomer, or pharmaceutically acceptable salt thereof according to any one of claims 1 to 10, wherein the group B comprises groups as shown in the following structures:

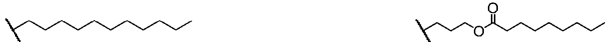

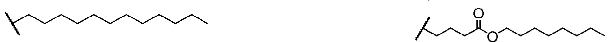

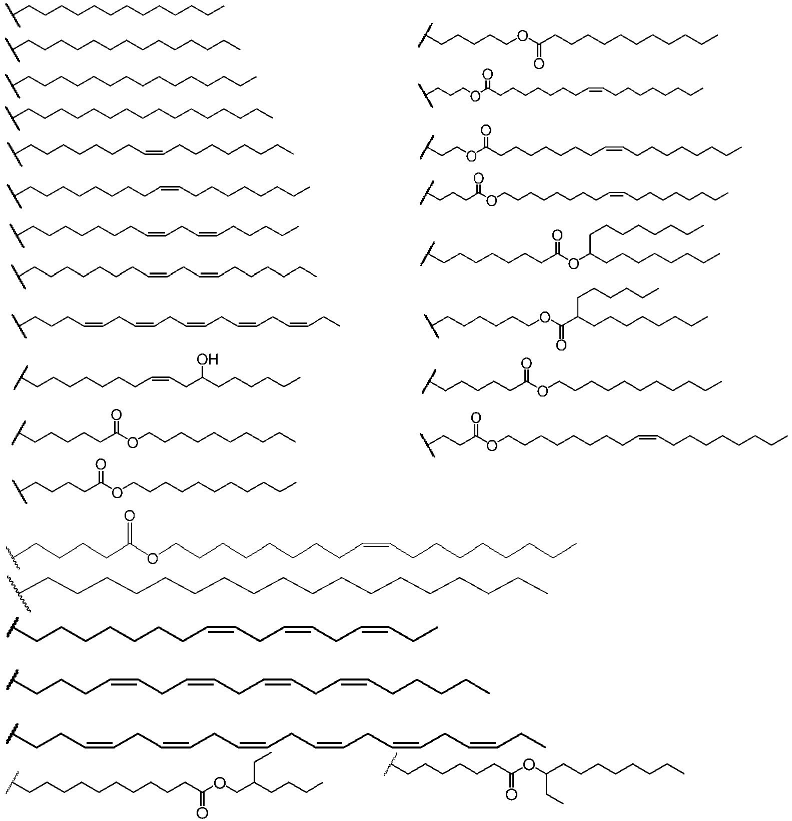

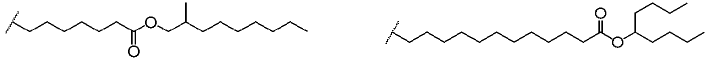

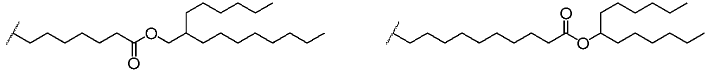

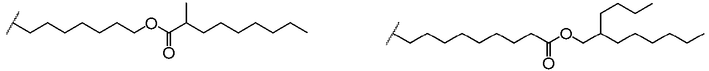

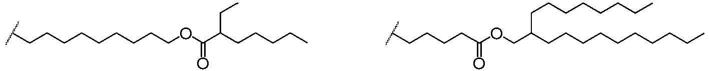

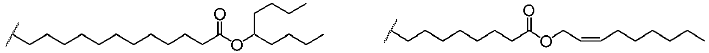

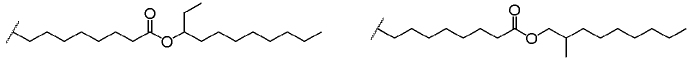

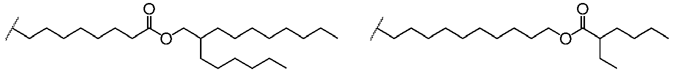

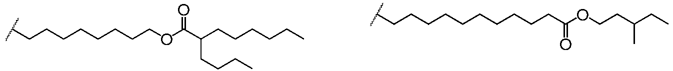

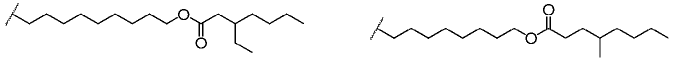

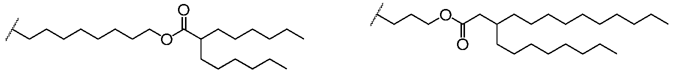

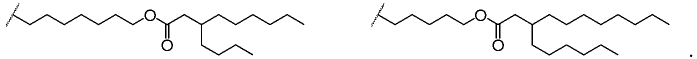

12. The lipid compound, or the N-oxide, stereoisomer, or pharmaceutically acceptable salt thereof according to any one of claims 1 to 11, wherein the group C comprises groups as shown in the following structures:

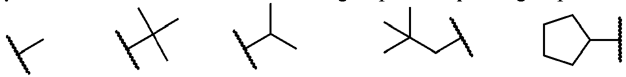

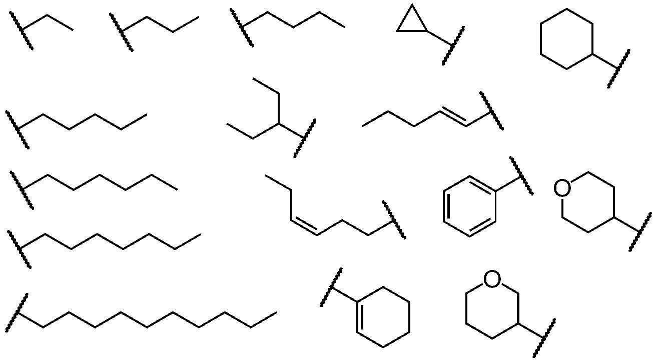

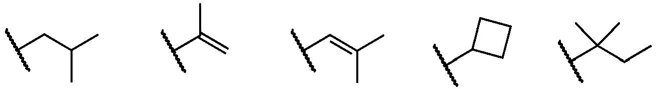

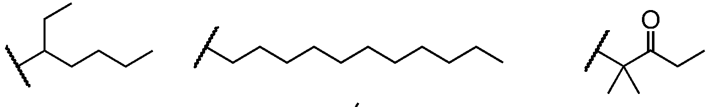

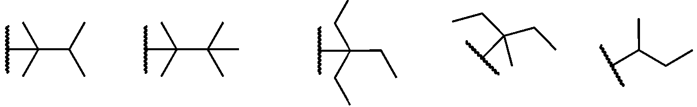

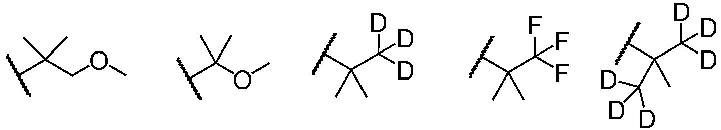

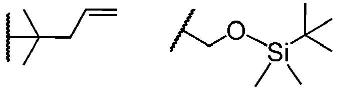

13. The lipid compound, or the N-oxide, stereoisomer, or pharmaceutically acceptable salt thereof according to any one of claims 1 to 12, wherein the lipid compound has the following general structural formula (I_{A}):

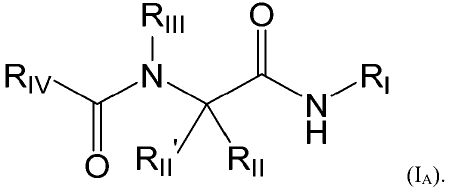

14. The lipid compound, or the N-oxide, stereoisomer, or pharmaceutically acceptable salt thereof according to claim 13, wherein R_{II}' is hydrogen, or substituted or unsubstituted hydrocarbyl, substituted or unsubstituted heterohydrocarbyl, substituted or unsubstituted aryl, or substituted or unsubstituted heteroaryl having 1 to 11 carbon atoms; and
R_{III} is selected from the group A, R_{I} and R_{IV} are selected from the group B, and R_{II} is selected from the group C.

15. The lipid compound, or the N-oxide, stereoisomer, or pharmaceutically acceptable salt thereof according to claim 13 or 14, wherein R_{II}' is hydrogen or C₁-C₆ alkyl.

16. The lipid compound, or the N-oxide, stereoisomer, or pharmaceutically acceptable salt thereof according to any one of claims 13 to 15, wherein R_{III} is selected from the group A, and the group comprising at least one ionizable tertiary amine structure in the group A is a group having the following general formula: 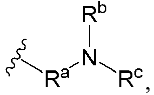 wherein R^{a} is substituted or unsubstituted C₁-C₆, such as C₁-C₄, alkylene; R^{b} and R^{c} are each independently substituted or unsubstituted C₁-C₆, such as C₁-C₄, alkyl, substituted or unsubstituted C₂-C₆, such as C₂-C₄, alkenyl, or substituted or unsubstituted C₂-C₆, such as C₂-C₄, alkynyl, which are optionally substituted with 1, 2, or 3 substituents selected from -OH, -SH, -NR^{d}R^{d'}, or phenyl, wherein R^{d} and R^{d'} are each independently hydrogen or C₁-C₃ alkyl; or R^{b} and R^{c}, together with the N atom to which they are attached, form a 5- to 12-membered heterocyclic ring or heteroaromatic ring, the 5- to 12-membered heterocyclic ring or heteroaromatic ring containing 1, 2, or 3 heteroatoms independently selected from N, O, and S, wherein at least one heteroatom is N, and the 5- to 12-membered heterocyclic ring or heteroaromatic ring is optionally substituted with one or more C₁-C₆, such as C₁-C₄, alkyls or oxo (=O); or R^{a} and R^{b}, together with the N atom to which they are attached, form a 5- to 12-membered heterocyclic ring or heteroaromatic ring optionally substituted with C₁-C₆, such as C₁-C₄, alkylene, the 5- to 12-membered heterocyclic ring or heteroaromatic ring optionally substituted with C₁-C₆, such as C₁-C₄, alkylene comprising 1, 2, or 3 heteroatoms independently selected from N, O, and S, wherein at least one heteroatom is N, and the 5- to 12-membered heterocyclic ring or heteroaromatic ring optionally substituted with C₁-C₆, such as C₁-C₄, alkylene is optionally substituted with one or more C₁-C₆, such as C₁-C₄, alkyls or oxo (=O); or R^{a}, R^{b}, and R^{c}, together with the N atom to which they are attached, form a 5- to 12-membered heterocyclic ring or heteroaromatic ring optionally substituted with C₁-C₆, such as C₁-C₄, alkylene, the 5- to 12-membered heterocyclic ring or heteroaromatic ring optionally substituted with C₁-C₆, such as C₁-C₄, alkylene comprising 1, 2, or 3 heteroatoms independently selected from N, O, and S, wherein at least one heteroatom is N, and the 5- to 12-membered heterocyclic ring or heteroaromatic ring optionally substituted with C₁-C₆, such as C₁-C₄, alkylene is optionally substituted with one or more C₁-C₆, such as C₁-C₄, alkyls or oxo (=O).

17. The lipid compound, or the N-oxide, stereoisomer, or pharmaceutically acceptable salt thereof according to any one of claims 13 to 16, wherein R_{III} is selected from the group A, and the group A comprises groups as shown in the following structures and containing at least one ionizable tertiary amine structure:

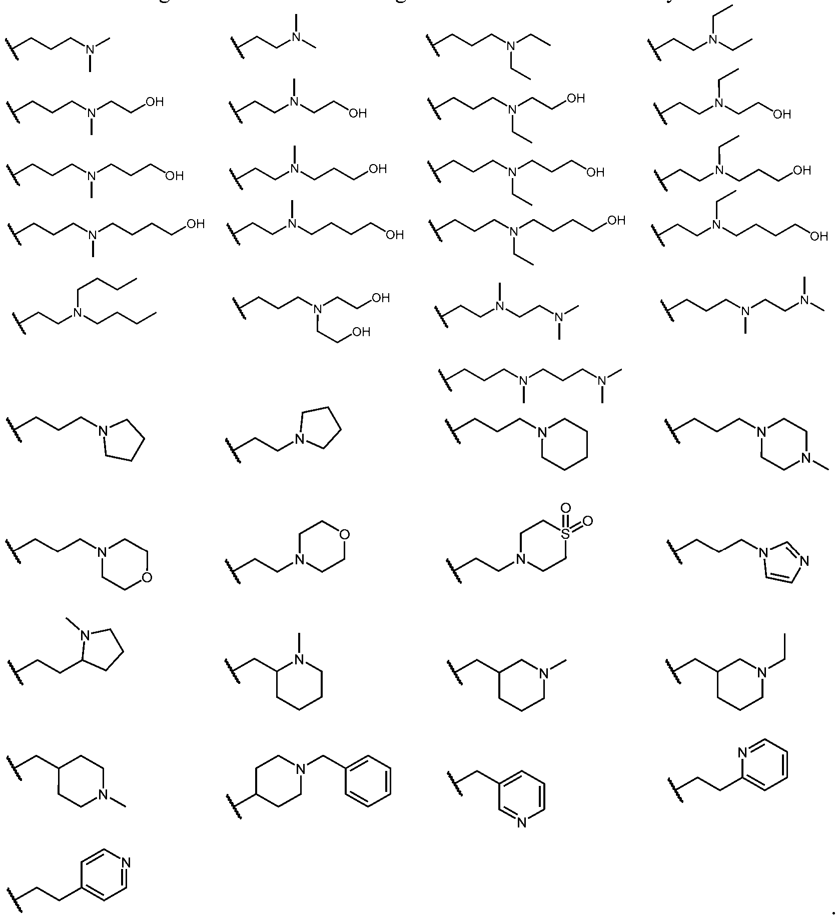

18. The lipid compound, or the N-oxide, stereoisomer, or pharmaceutically acceptable salt thereof according to any one of claims 13 to 17, wherein R_{I} and R_{IV} are each independently selected from the group B, wherein the substituted or unsubstituted aliphatic group selected from the group B has 11 to 30 carbon atoms, and the substituted or unsubstituted heteroaliphatic group selected from the group B has 11 to 30 atoms comprising carbon atoms and heteroatoms such as N, O, and S.

19. The lipid compound, or the N-oxide, stereoisomer, or pharmaceutically acceptable salt thereof according to any one of claims 13 to 18, wherein R_{I} and R_{IV} are each independently selected from the group B, and the group B comprises a substituted or unsubstituted aliphatic or substituted or unsubstituted heteroaliphatic group having 11 to 30 carbon atoms as shown in the following structures:

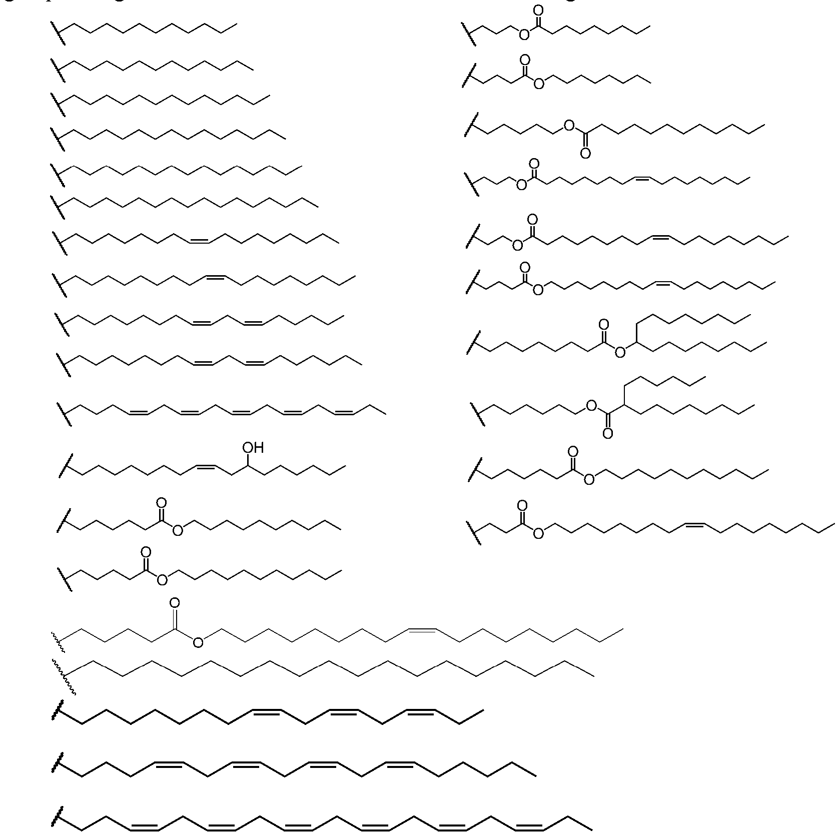

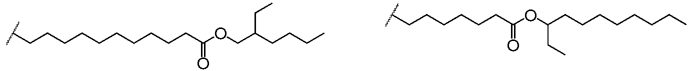

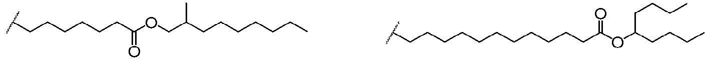

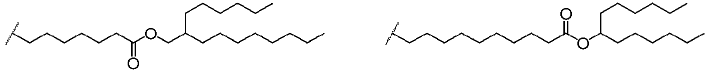

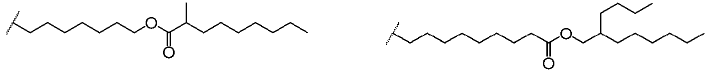

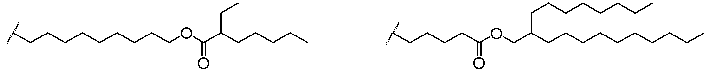

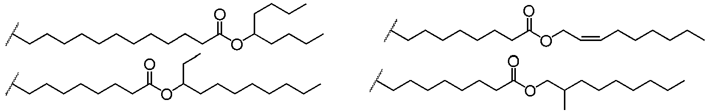

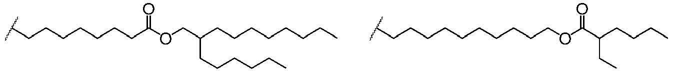

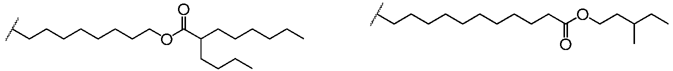

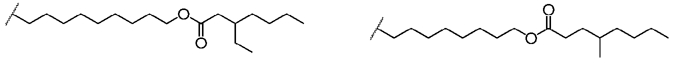

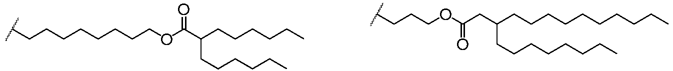

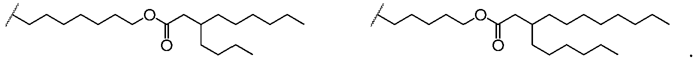

20. The lipid compound, or the N-oxide, stereoisomer, or pharmaceutically acceptable salt thereof according to any one of claims 13 to 19, wherein at least one of R_{I} and R_{IV} comprises at least one degradable group; optionally, the degradable group is selected from -C(O)O-, -OC(O)-, -OC(O)O-, -S-S-, -C(O)NH-, -NHC(O)-, -NHC(O)O-, -NR¹C(O)-, -C(O)NR²-, -NR³C(O)O-, -OP(O)OR⁴O-, -OCR⁵(OR⁶)O-, -CR⁷(OR⁸)O-, and -CH(OR⁹)O-, wherein R¹, R², R³, R⁴, R⁵, R⁶, R⁷, R⁸, and R⁹ are each independently substituted or unsubstituted C₁-C₁₄ aliphatic hydrocarbyl; preferably, the degradable group is an ester group, i.e., -C(O)O- or -OC(O)-, or the degradable group comprises an ester bond.

21. The lipid compound, or the N-oxide, stereoisomer, or pharmaceutically acceptable salt thereof according to any one of claims 13 to 20, wherein R_{I} and R_{IV} each comprise at least one degradable group, for example, the degradable group is an ester group, i.e., -C(O)O- or -OC(O)-, or the degradable group comprises an ester bond.

22. The lipid compound, or the N-oxide, stereoisomer, or pharmaceutically acceptable salt thereof according to any one of claims 13 to 21, wherein one of R_{I} and R_{IV} comprises at least one degradable group, for example, the degradable group is an ester group, i.e., -C(O)O- or -OC(O)-, or the degradable group comprises an ester bond; and the other of R_{I} and R_{IV} does not comprise any degradable group.

23. The lipid compound, or the N-oxide, stereoisomer, or pharmaceutically acceptable salt thereof according to any one of claims 13 to 22, wherein when R_{I} and/or R_{IV} does not comprise a degradable group, R_{I} and/or R_{IV} is selected from groups as shown in the following structures:

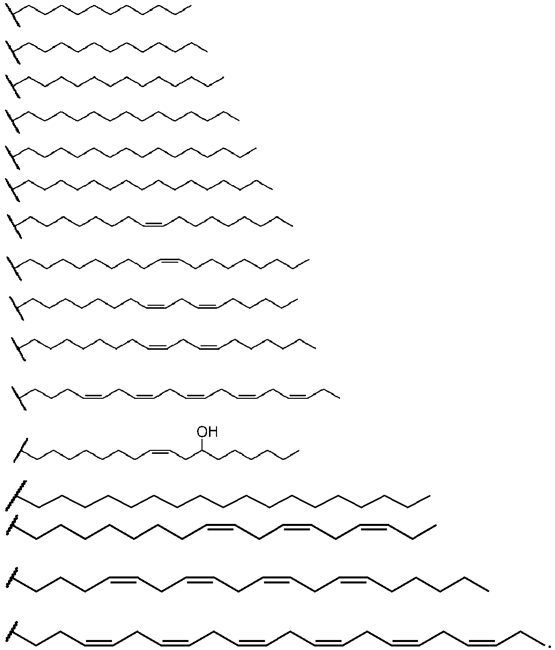

24. The lipid compound, or the N-oxide, stereoisomer, or pharmaceutically acceptable salt thereof according to any one of claims 13 to 23, wherein when R_{I} and/or R_{IV} comprises a degradable group, R_{I} and/or R_{IV} is selected from groups as shown in the following structures:

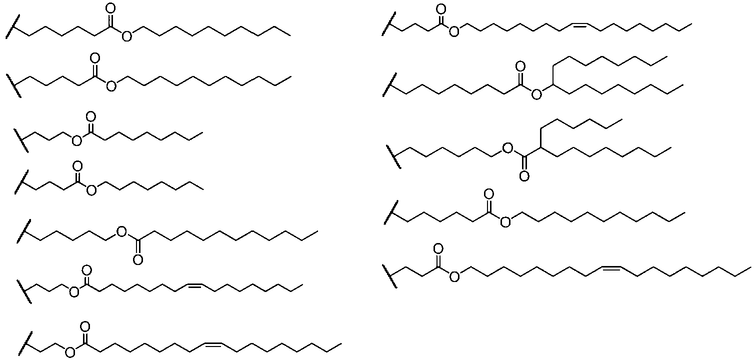

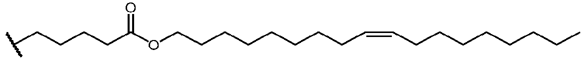

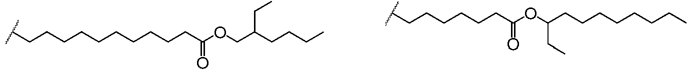

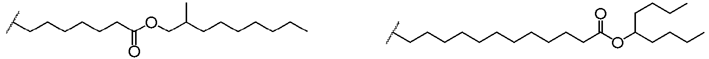

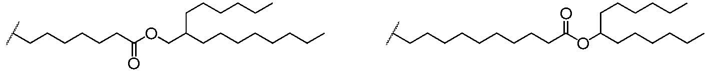

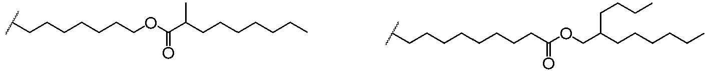

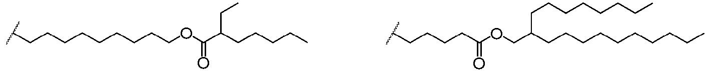

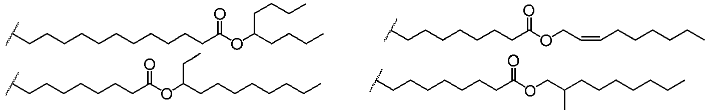

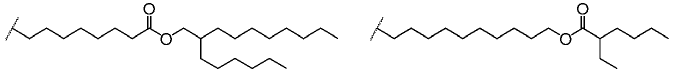

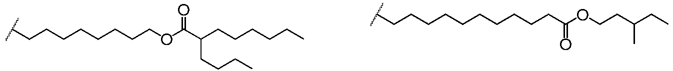

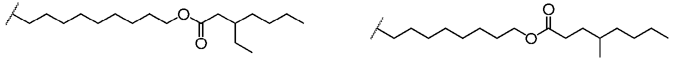

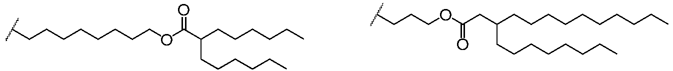

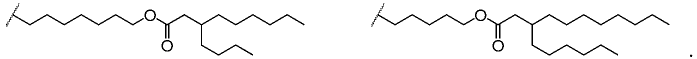

25. The lipid compound, or the N-oxide, stereoisomer, or pharmaceutically acceptable salt thereof according to any one of claims 13 to 23, wherein when R_{I} and/or R_{IV} comprises a degradable group, R_{I} and/or R_{IV} has a structure as shown in the following general formula: 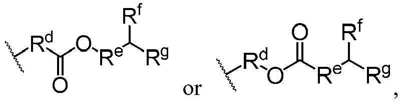
wherein R^{d} is substituted or unsubstituted hydrocarbylene or substituted or unsubstituted heterohydrocarbylene having 1 to 11 carbon atoms, and the hydrocarbylene or heterohydrocarbylene is optionally substituted with one or more substituents selected from the following group: deuterium, halogen, -NO₂, and -OH;
R^{e} is absent or is substituted or unsubstituted hydrocarbylene or substituted or unsubstituted heterohydrocarbylene having 1 to 4 carbon atoms, and the hydrocarbylene or heterohydrocarbylene is optionally substituted with one or more substituents selected from the following group: deuterium, halogen, -NO₂, and -OH; and
R^{f} and R^{g} are each independently substituted or unsubstituted hydrocarbyl or substituted or unsubstituted heterohydrocarbyl having 1 to 11 carbon atoms, and the hydrocarbyl or heterohydrocarbyl is optionally substituted with one or more substituents selected from the following group: deuterium, halogen, -NO₂, and -OH.

26. The lipid compound, or the N-oxide, stereoisomer, or pharmaceutically acceptable salt thereof according to any one of claims 13 to 23, wherein when R_{I} and/or R_{IV} comprises a degradable group, R_{I} and/or R_{IV} is each independently selected from the group consisting of the following groups:

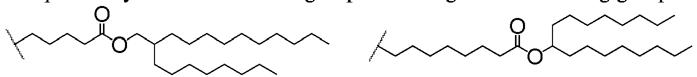

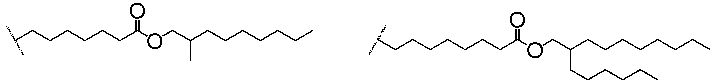

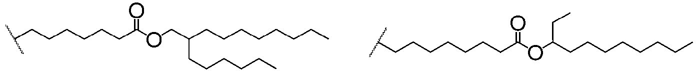

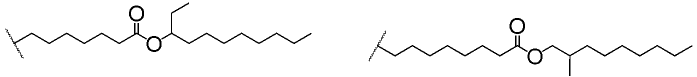

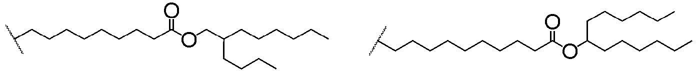

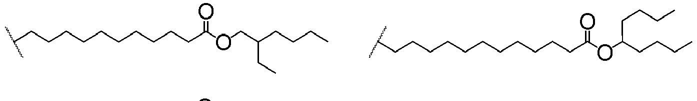

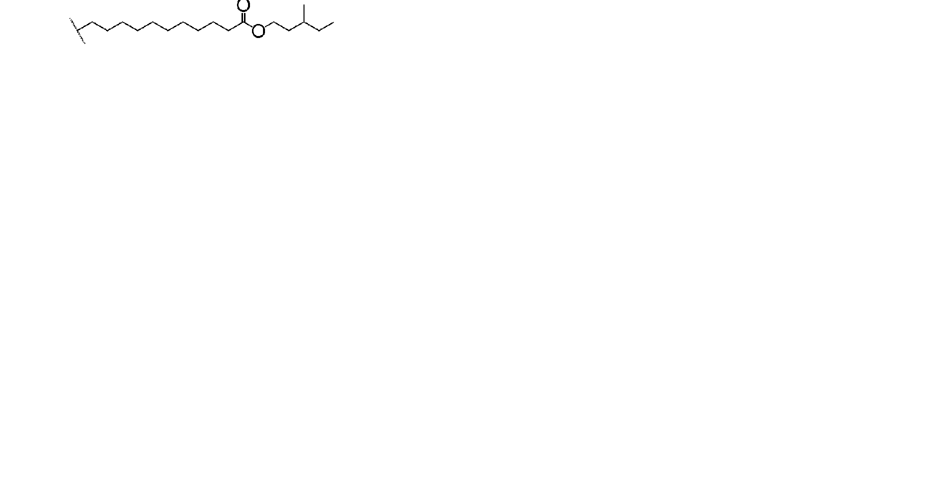

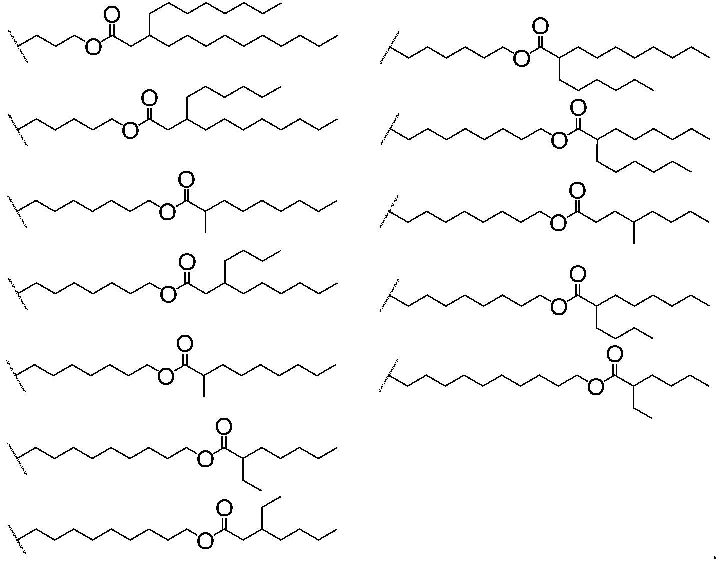

27. The lipid compound, or the N-oxide, stereoisomer, or pharmaceutically acceptable salt thereof according to any one of claims 13 to 26, wherein R_{II} is selected from the group C, and the substituted or unsubstituted hydrocarbyl, substituted or unsubstituted heterohydrocarbyl, substituted or unsubstituted aryl, or substituted or unsubstituted heteroaryl group in the group C has 1 to 11 carbon atoms.

28. The lipid compound, or the N-oxide, stereoisomer, or pharmaceutically acceptable salt thereof according to any one of claims 13 to 27, wherein R_{II} is selected from the group C, and the group C comprises a substituted or unsubstituted hydrocarbyl, substituted or unsubstituted heterohydrocarbyl, substituted or unsubstituted aryl, or substituted or unsubstituted heteroaryl group having fewer than 11 carbon atoms as shown in the following structures:

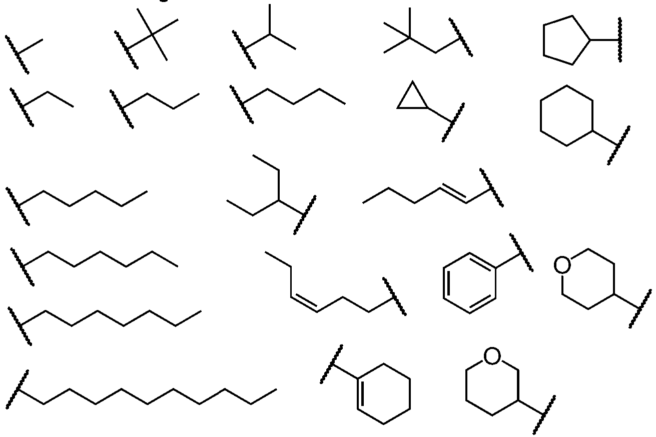

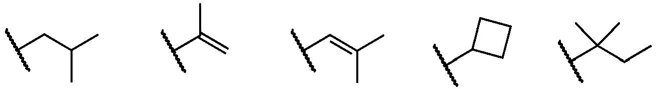

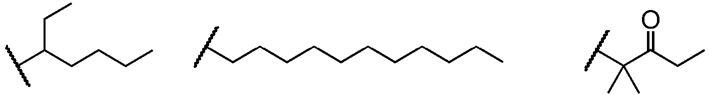

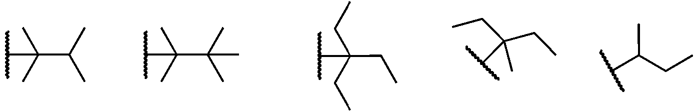

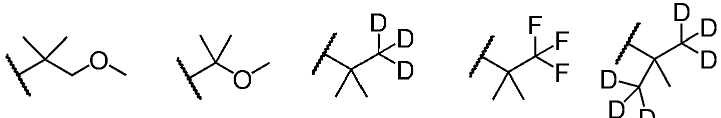

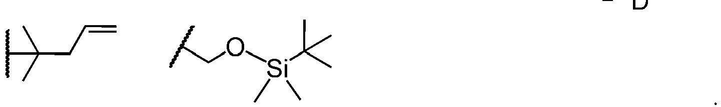

29. The lipid compound, or the N-oxide, stereoisomer, or pharmaceutically acceptable salt thereof according to any one of claims 13 to 28, wherein the lipid compound has the following general structural formula (I_{A-1}):

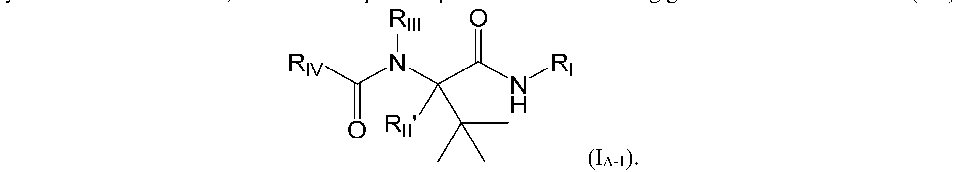

30. The lipid compound, or the N-oxide, stereoisomer, or pharmaceutically acceptable salt thereof according to any one of claims 13 to 28, wherein the lipid compound has the following general structural formula (I_{A-2}):

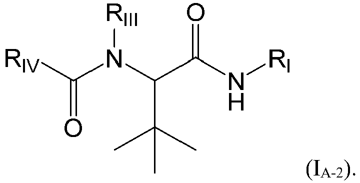

31. The lipid compound, or the N-oxide, stereoisomer, or pharmaceutically acceptable salt thereof according to any one of claims 1 to 12, wherein the lipid compound has the following general structural formula (I_{B}):

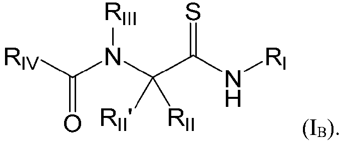

32. The lipid compound, or the N-oxide, stereoisomer, or pharmaceutically acceptable salt thereof according to claim 31, wherein
R_{II}' is hydrogen;
R_{II} is selected from the group C, for example, C₁-C₆ alkyl, C₁-C₄ alkyl, or for example, tertiary butyl; and
R_{III} is selected from the group A, for example, having a group of the following general formula; 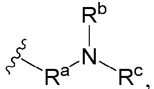
wherein R^{a}, R^{b}, and R^{c} are each independently substituted or unsubstituted C₁-C₆, such as C₁-C₄, alkyl, optionally substituted with 1, 2, or 3 substituents selected from -OH, -SH, -NR^{d}R^{d'}, or phenyl, wherein R^{d} and R^{d}' are each independently hydrogen or C₁-C₃ alkyl; and
R_{I} and R_{IV} are each independently selected from the group B, and at least one of R_{I} and R_{IV} comprises one degradable group, for example, an ester group, i.e., -C(O)O- or -OC(O)-; or the degradable group comprises an ester bond, preferably, which, for example, is selected from groups as shown in the following structures:

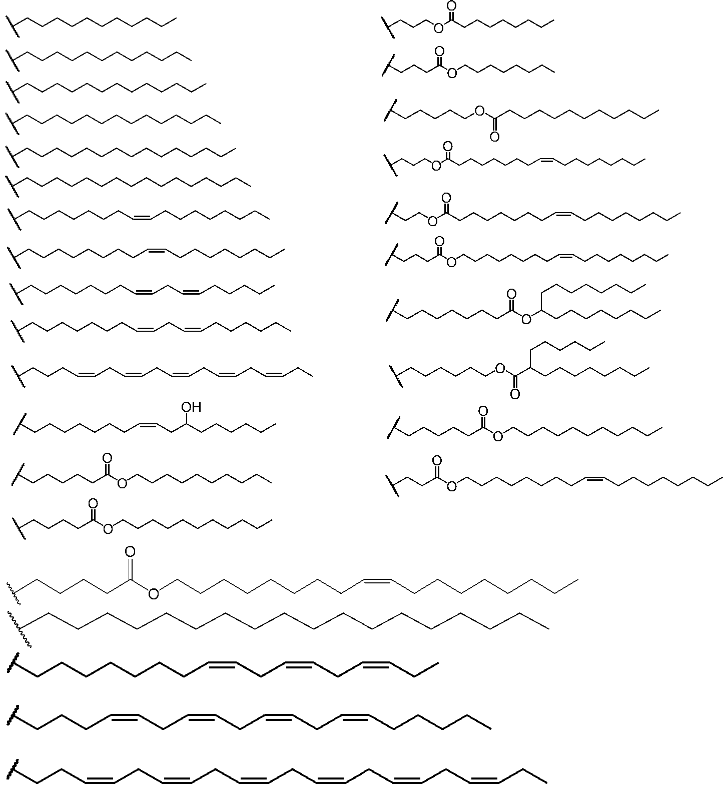

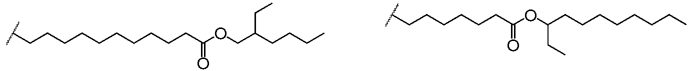

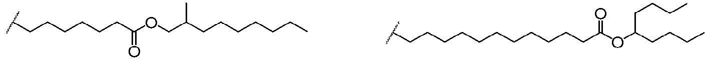

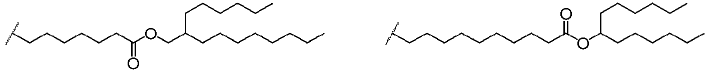

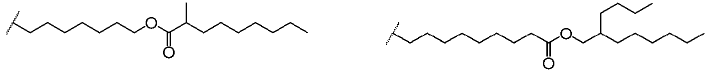

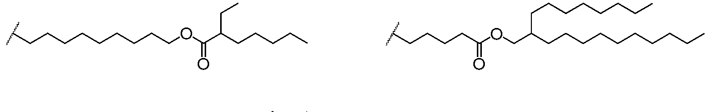

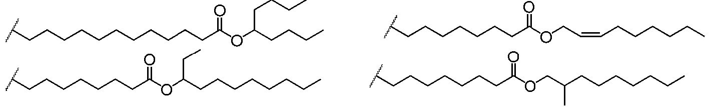

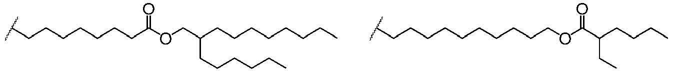

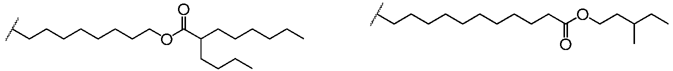

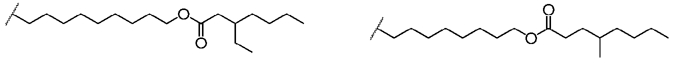

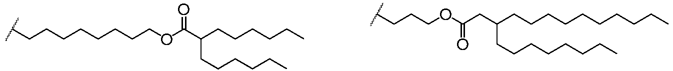

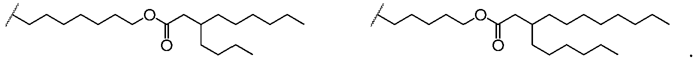

33. The lipid compound, or the N-oxide, stereoisomer, or pharmaceutically acceptable salt thereof according to claim 1, wherein the lipid compound is selected from lipid compounds as shown below:

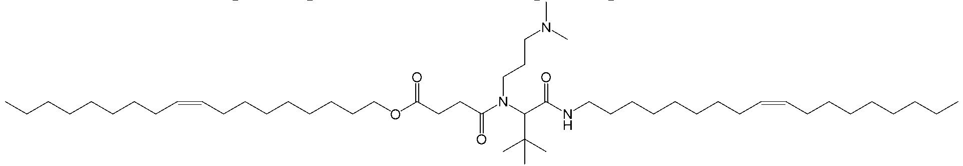

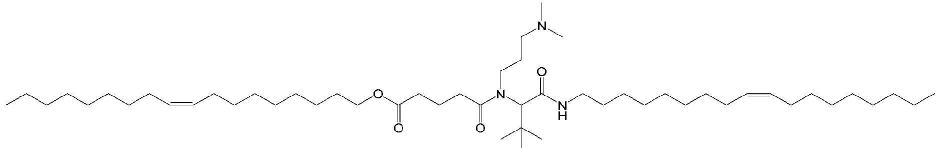

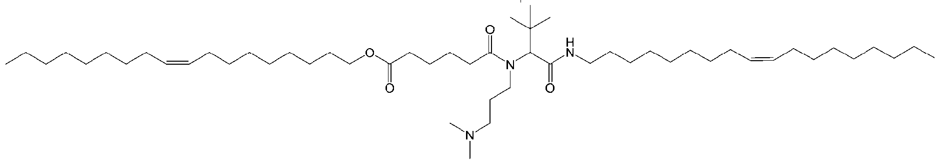

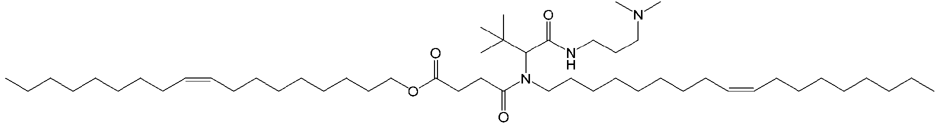

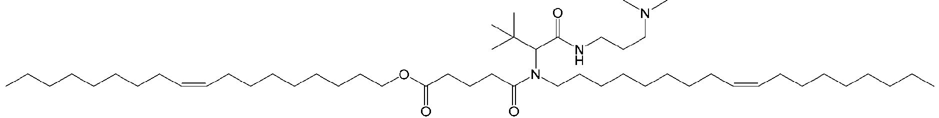

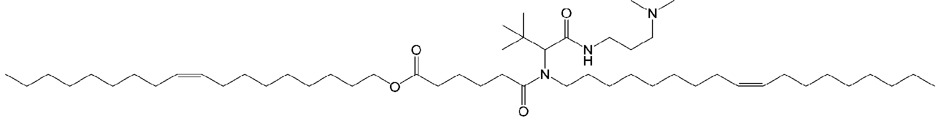

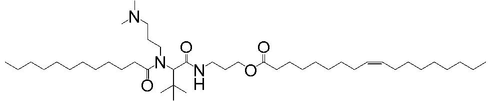

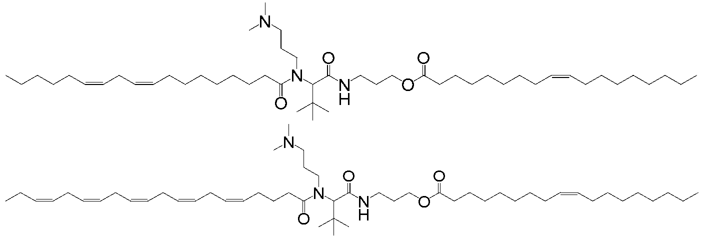

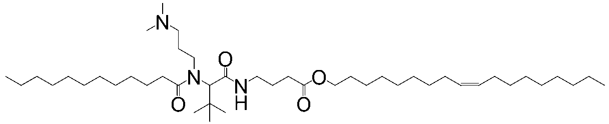

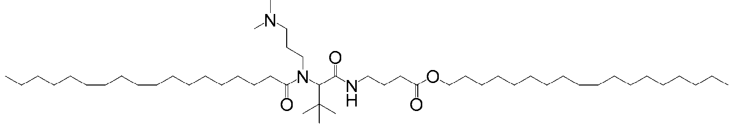

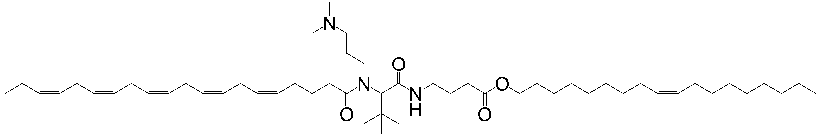

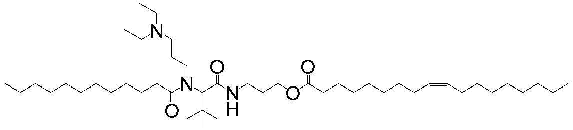

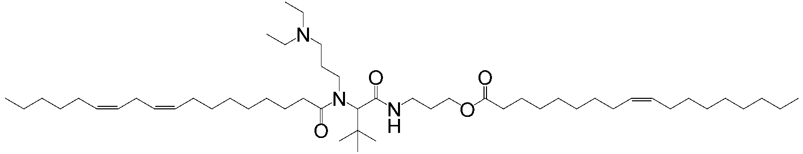

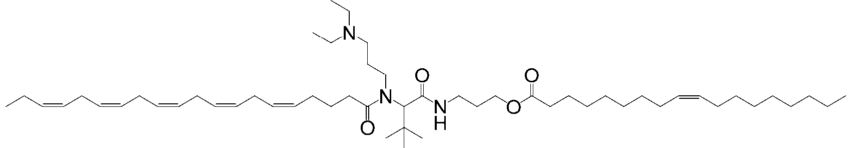

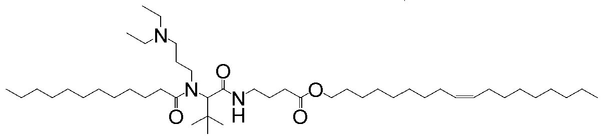

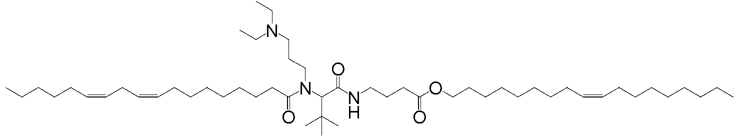

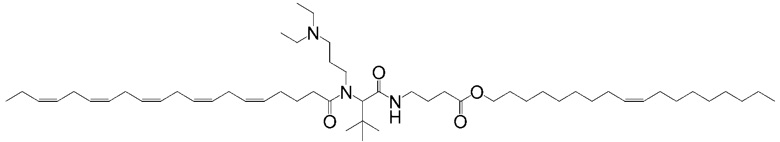

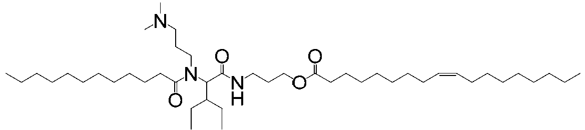

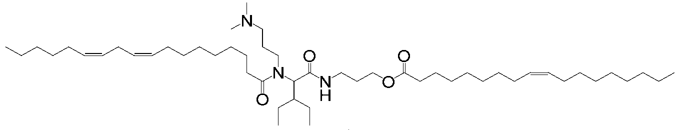

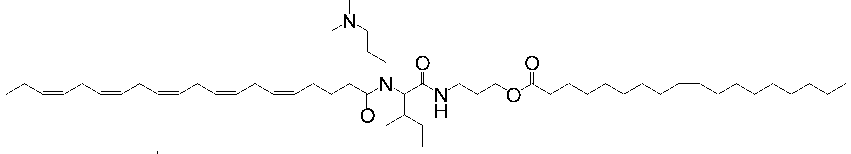

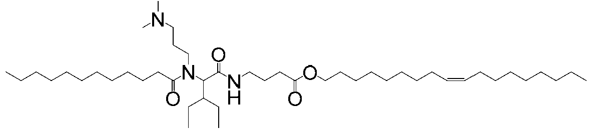

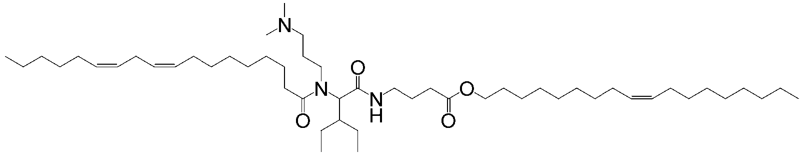

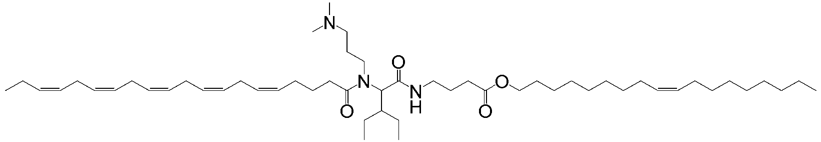

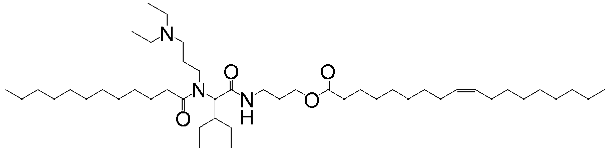

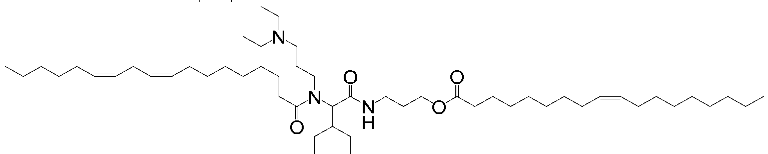

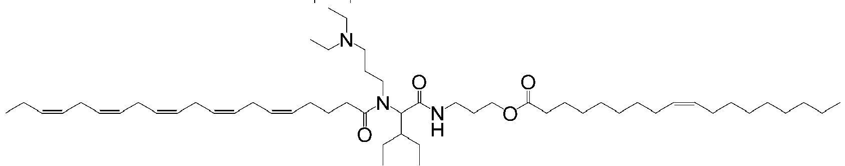

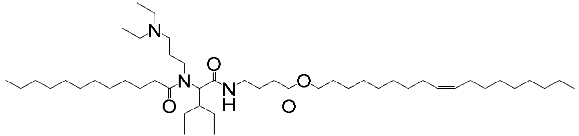

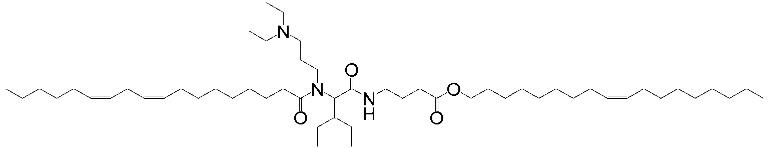

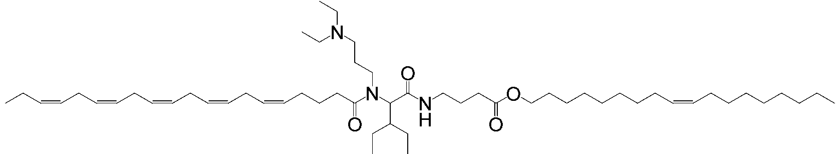

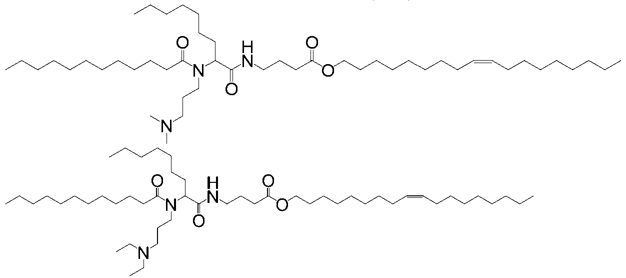

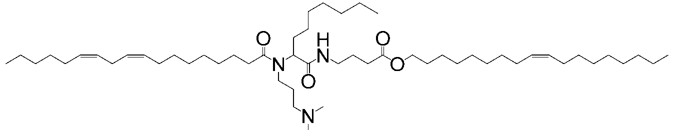

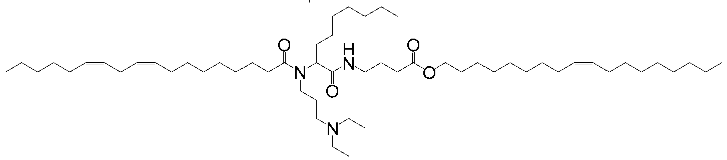

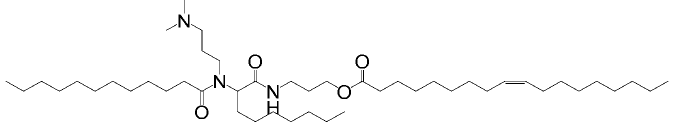

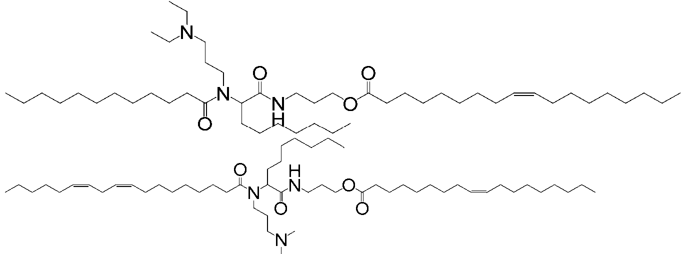

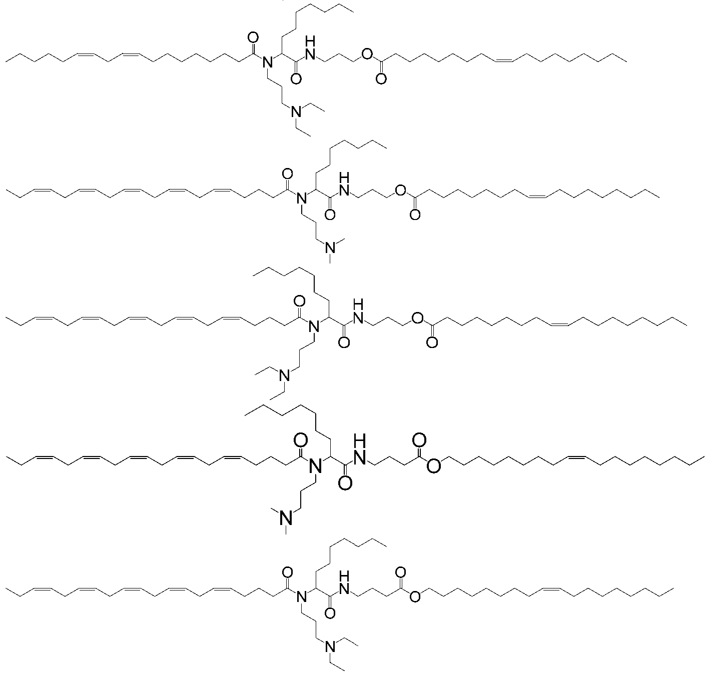

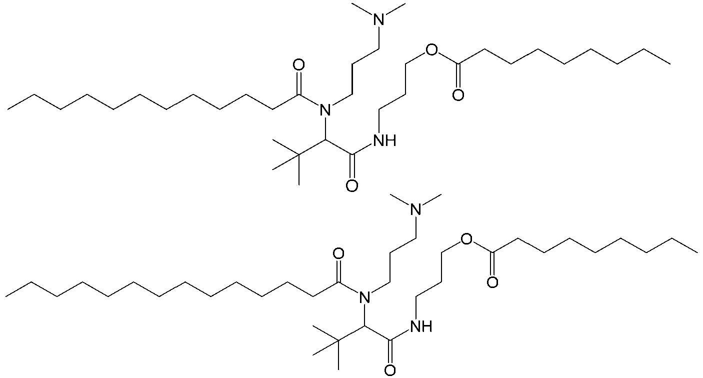

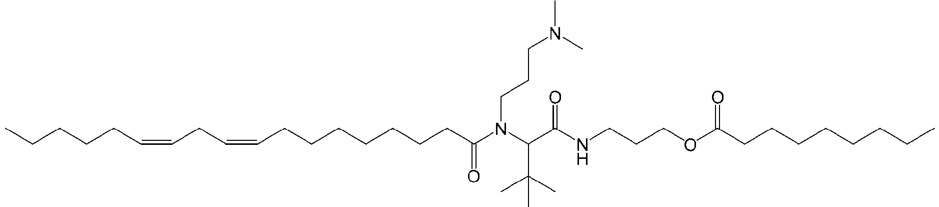

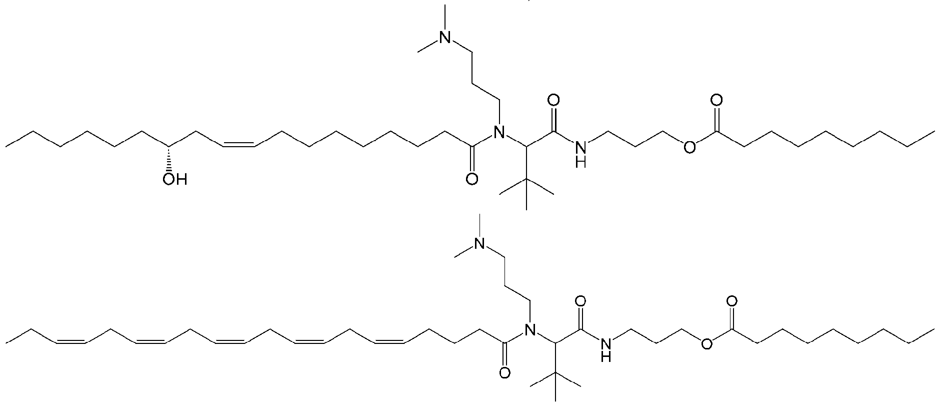

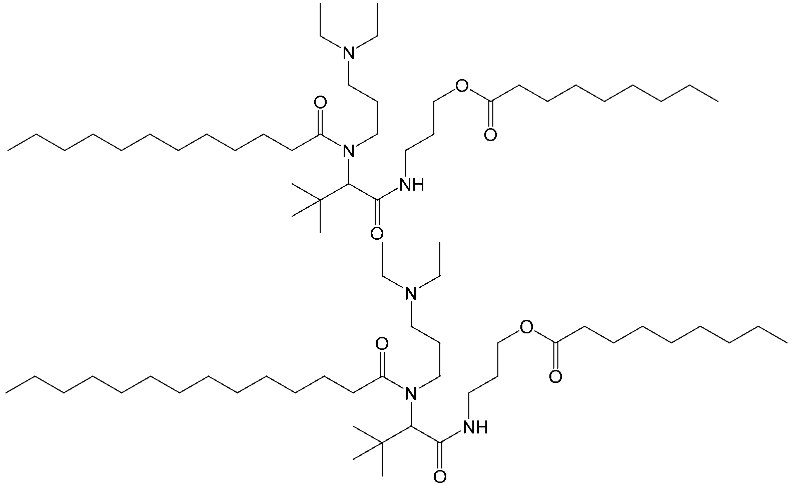

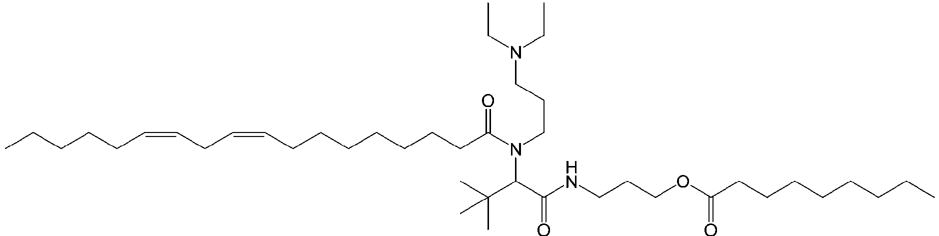

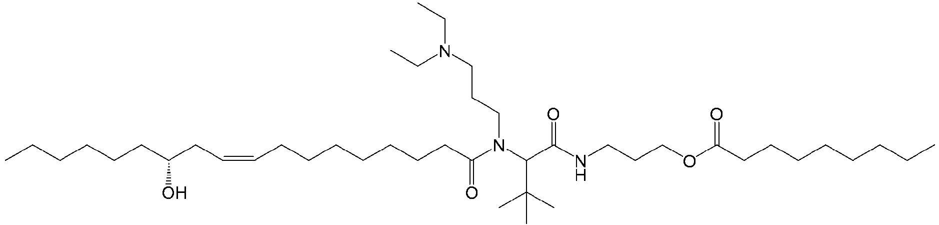

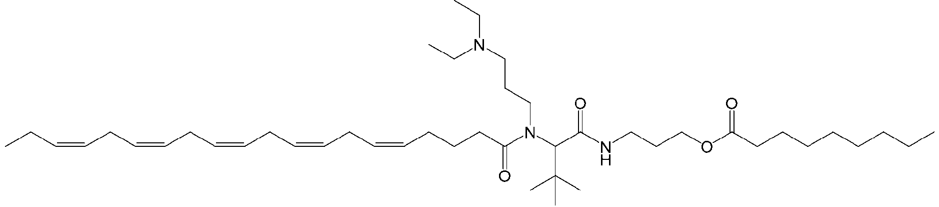

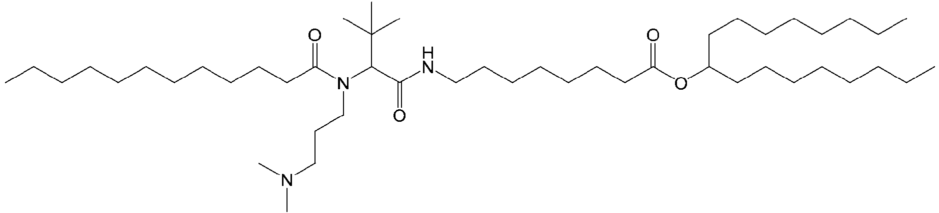

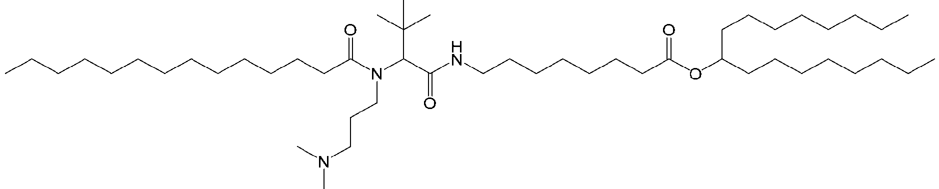

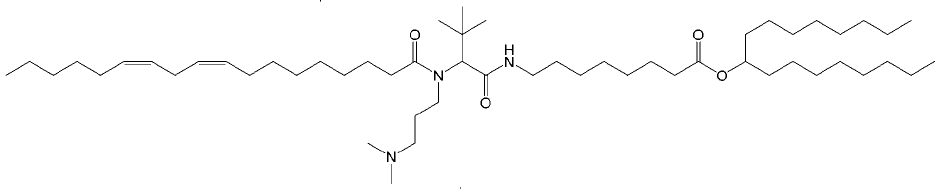

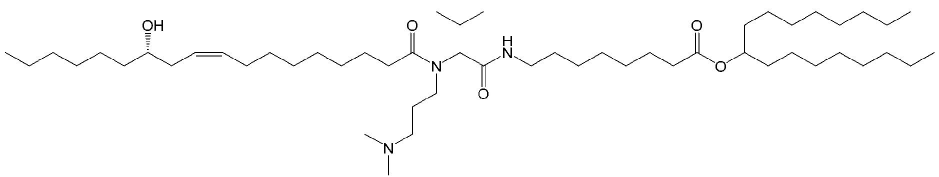

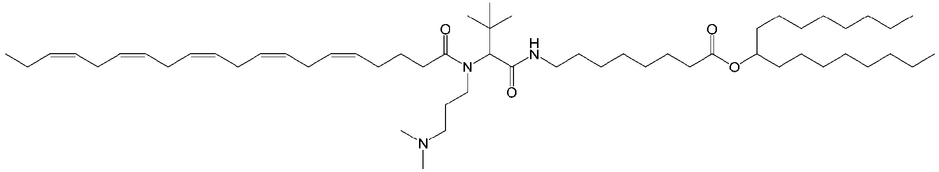

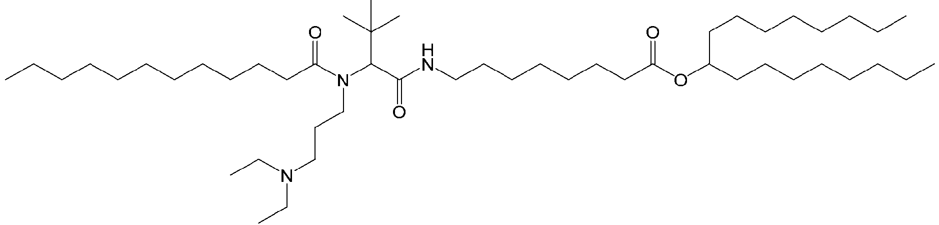

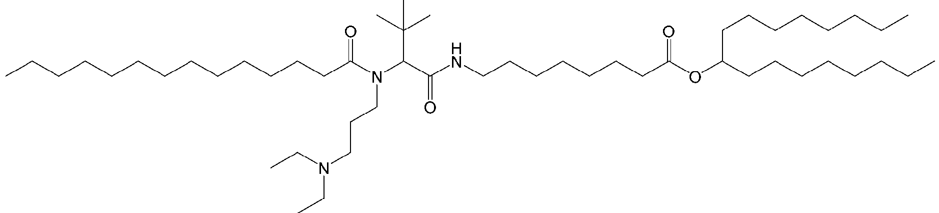

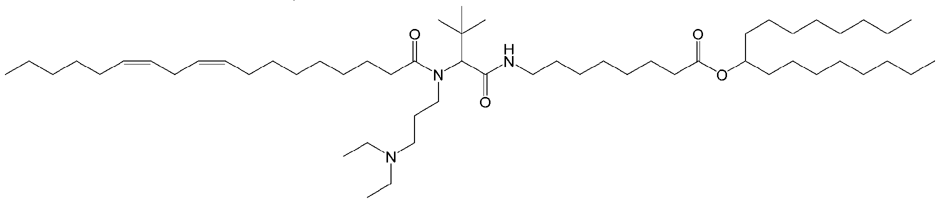

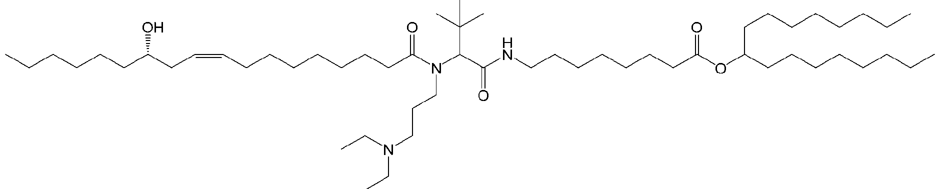

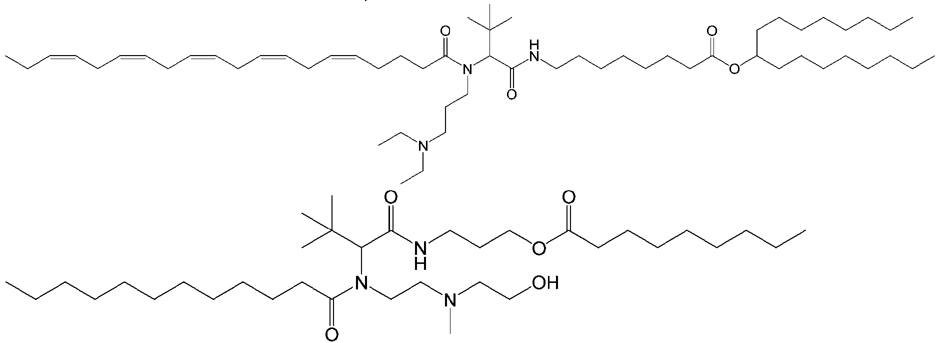

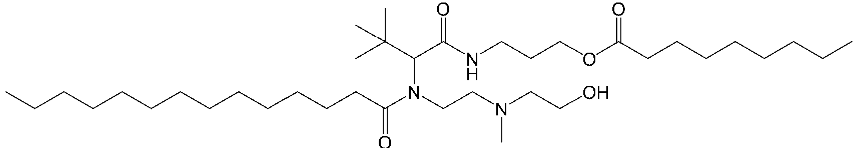

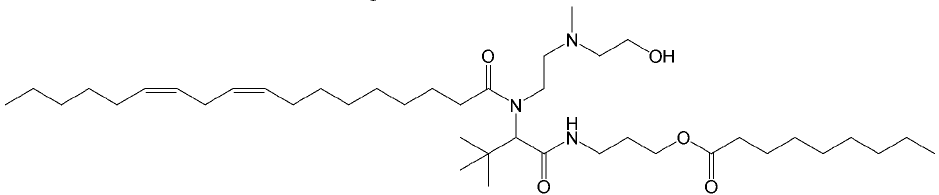

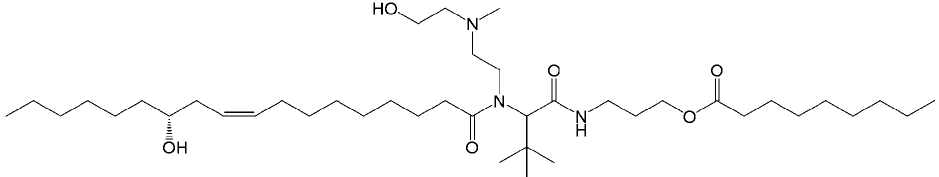

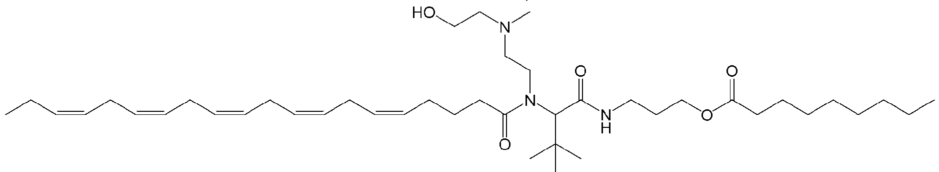

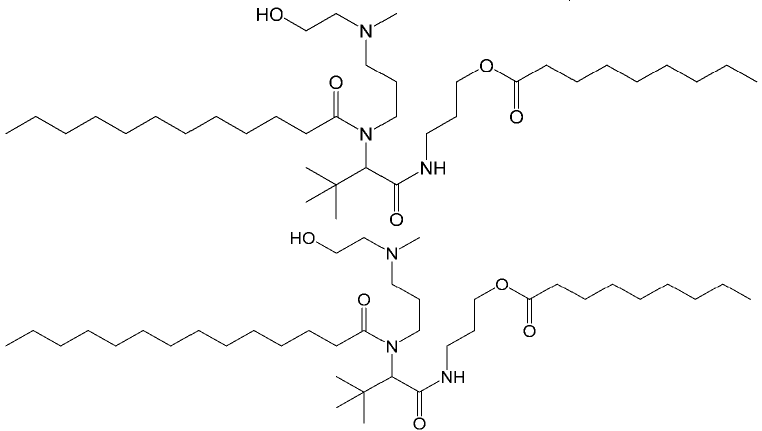

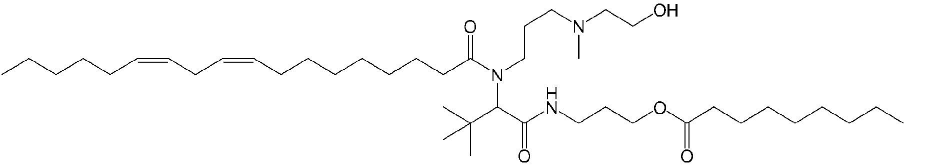

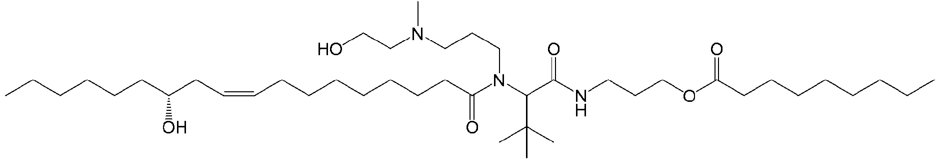

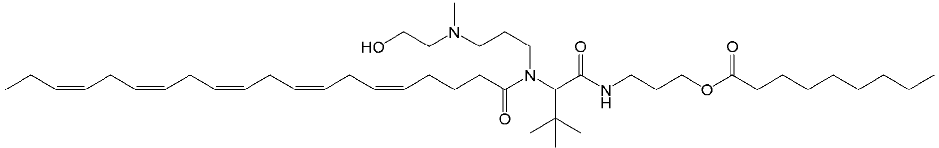

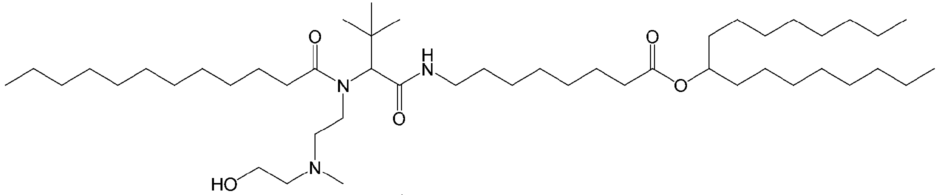

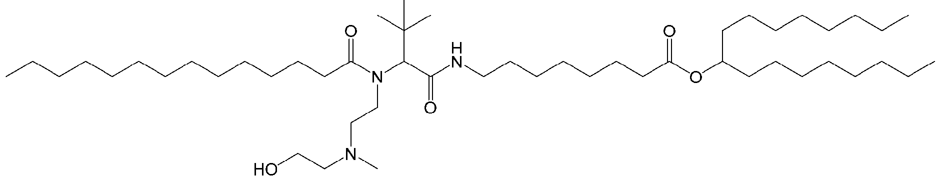

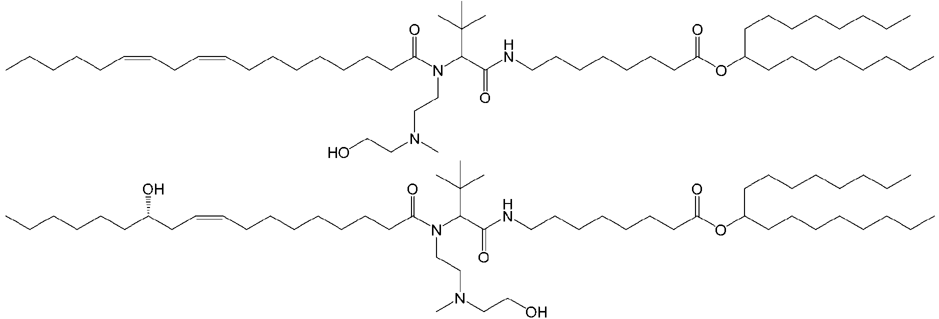

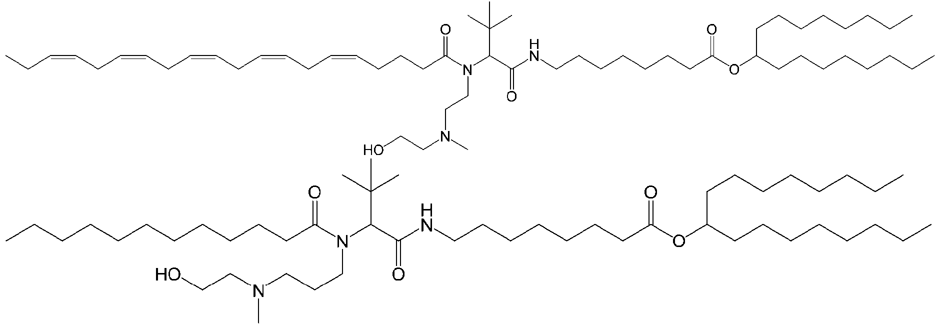

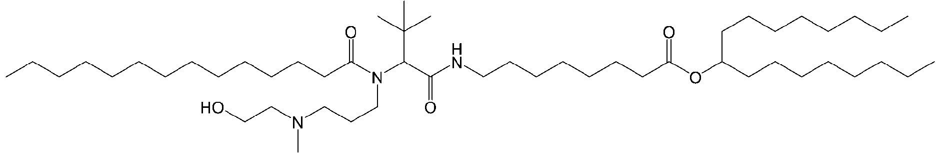

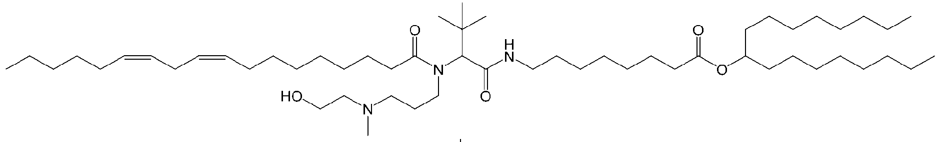

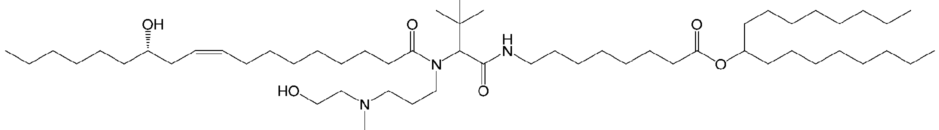

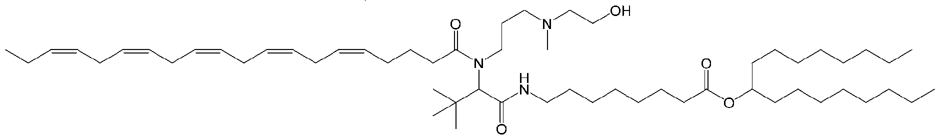

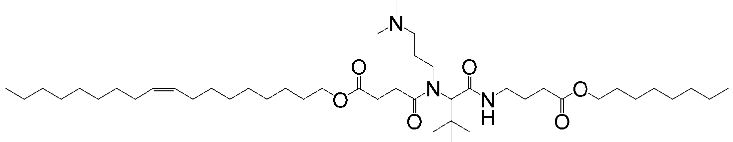

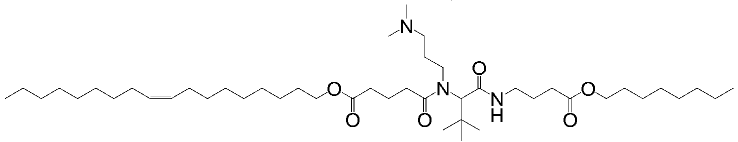

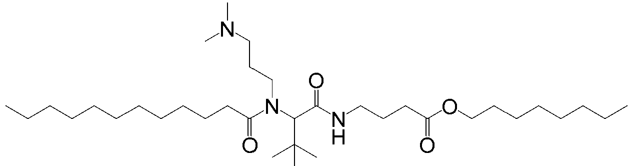

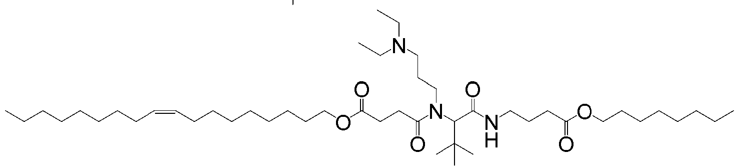

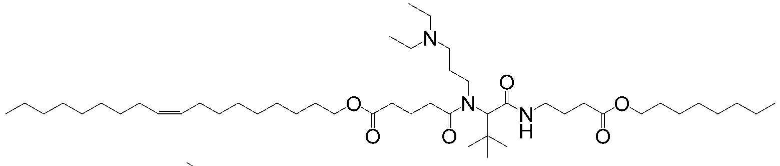

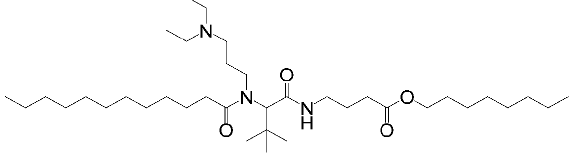

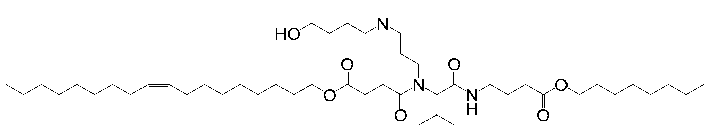

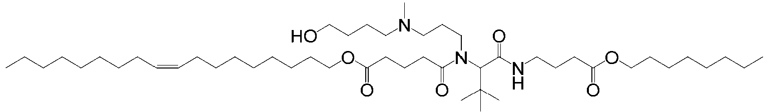

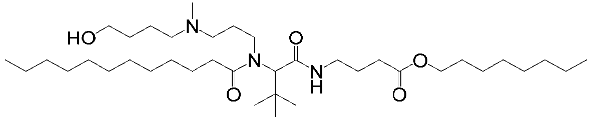

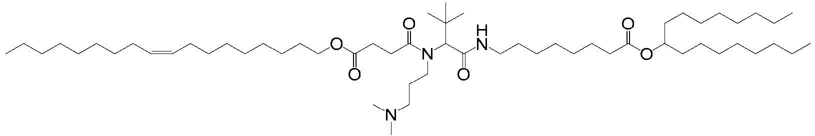

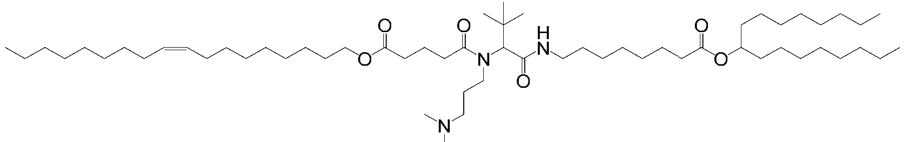

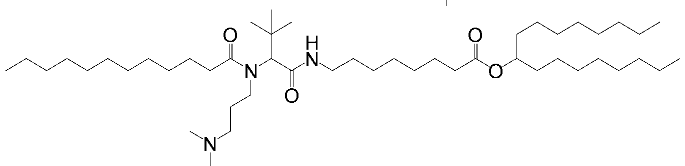

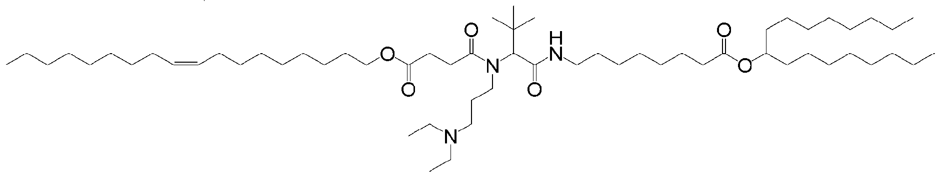

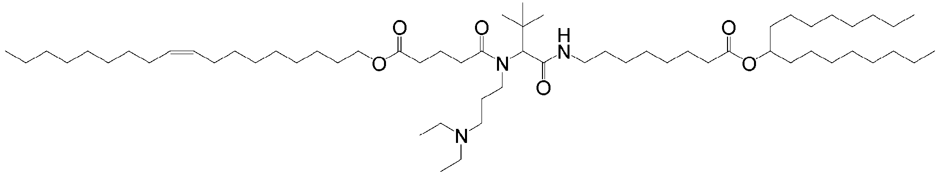

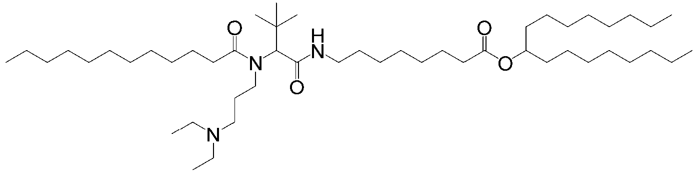

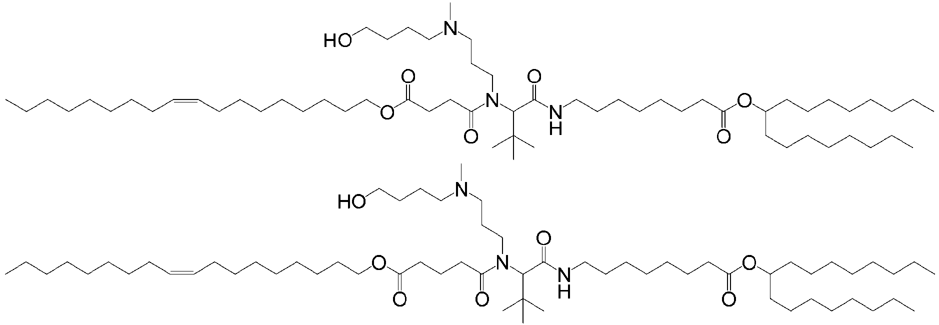

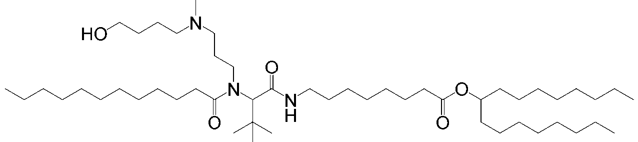

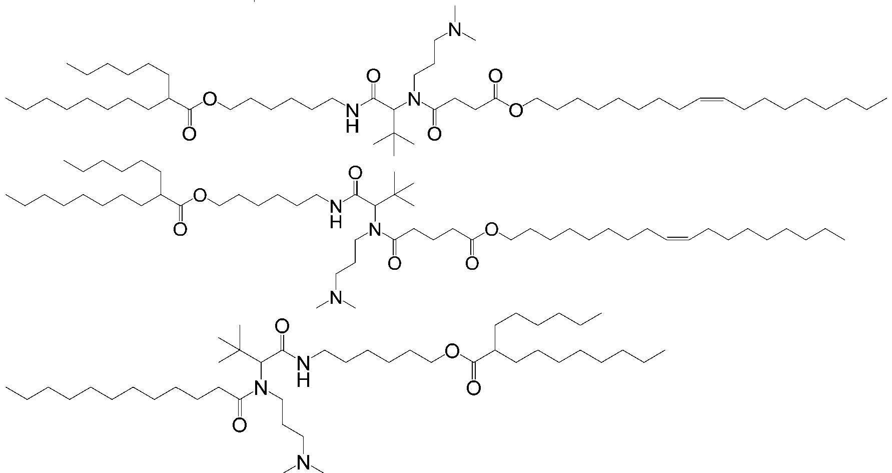

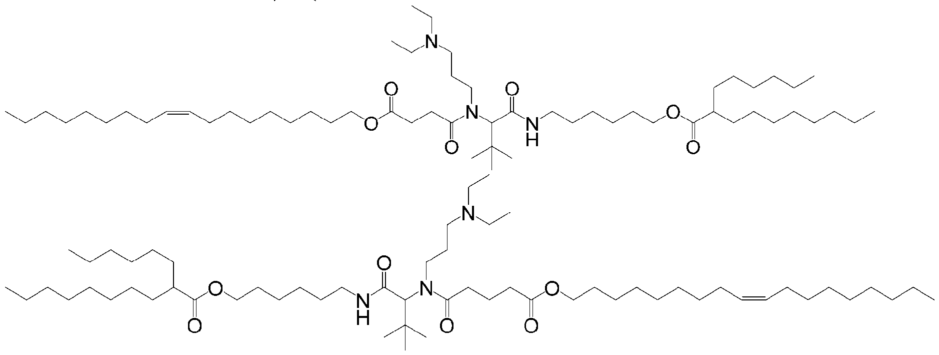

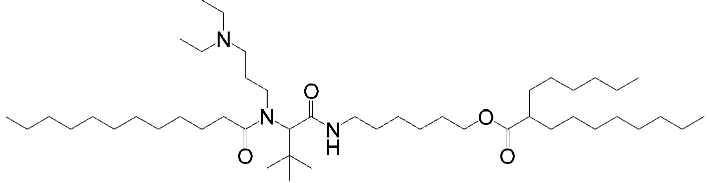

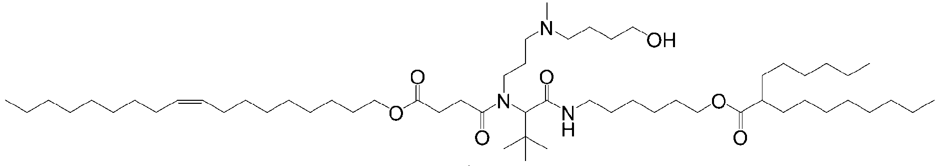

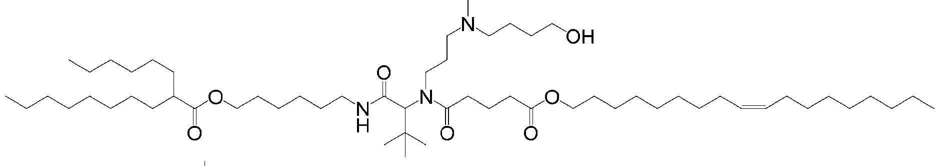

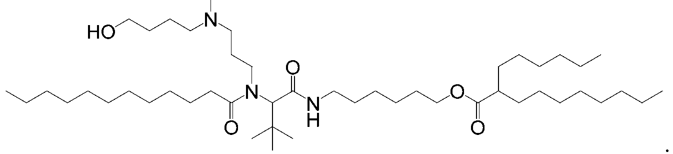

34. The lipid compound, or the N-oxide, stereoisomer, or pharmaceutically acceptable salt thereof according to claim 1, wherein the lipid compound is selected from lipid compounds having lipid numbers 1 to 1075, as shown in Tables 1 to 8.

35. The lipid compound, or the N-oxide, stereoisomer, or pharmaceutically acceptable salt thereof according to claim 1, wherein the lipid compound is selected from the following lipid compounds having lipid numbers 16, 30, 60, 108, 113, 157, 158, 159, 160, 161, 162, 163, 166, 170, 173, 174, 175, 176, 188, 189, 194, 195, 223, 226, 227, 229, 230, 232, 233, 234, 235, 236, 237, 238, 239, 240, 284, 285, 286, 287, 292, 293, 294, 295, 304, 325, 326, 327, 401, 402, 406, 407, 408, 409, 420, 424, 431, 445, 446, 447, 583, 595, 598, 607, 613, 641, 675, 685, 914, 915, 920, 921, 922, 923, 924, 926, 927, 928, 929, 930, 931, 932, 933, 934, 935, 938, 939, 940, 941, 942, 943, 944, 945, 946, 947, 949, 950, 951, 952, 954, 955, 958, 959, 960, 961, 962, 963, 964, 966 to 1051, and 1053 to 1057.

36. The lipid compound, or the N-oxide, stereoisomer, or pharmaceutically acceptable salt thereof according to claim 1, wherein the lipid compound is selected from lipid compounds having lipid numbers 317 and 1058 to 1075, as shown below.

37. The lipid compound, or the N-oxide, stereoisomer, or pharmaceutically acceptable salt thereof according to any one of claims 1 to 36, wherein the lipid compound has a molecular weight in a range of 500 g/mol to 1400 g/mol; preferably, the lipid compound has a molecular weight in a range of 700 g/mol to 1200 g/mol; and more preferably, the lipid compound has a molecular weight in a range of 700 g/mol to 1000 g/mol.

38. The lipid compound, or the N-oxide, stereoisomer, or pharmaceutically acceptable salt thereof according to any one of claims 1 to 37, for use in nucleic acid delivery.

39. Use of the lipid compound, or the N-oxide, stereoisomer, or pharmaceutically acceptable salt thereof according to any one of claims 1 to 38 in preparation of a liposome and/or lipid nanoparticle.

40. A liposome, comprising the lipid compound, or the N-oxide, stereoisomer, or pharmaceutically acceptable salt thereof according to any one of claims 1 to 37.

41. The liposome according to claim 40, wherein based on the total molar amount of components constituting the liposome, the lipid compound accounts for about 10 mol% to about 90 mol%, preferably about 10 mol% to about 70 mol%, and more preferably about 20 mol% to about 50 mol%.

42. The liposome according to claim 40 or 41, wherein the liposome further comprises a phospholipid and cholesterol.

43. The liposome according to claim 42, wherein based on the total molar amount of the components constituting the liposome, the phospholipid accounts for about 0 mol% to about 20 mol%, or any value within the range of about 0 mol% to about 20 mol%.

44. The liposome according to claim 42 or 43, wherein based on the total molar amount of the components constituting the liposome, the cholesterol accounts for about 30 mol% to about 50 mol%, or is in a range between any numerical values of about 30 mol% to about 50 mol%.

45. A lipid nanoparticle, comprising the lipid compound, or the N-oxide, stereoisomer, or pharmaceutically acceptable salt thereof according to any one of claims 1 to 37.

46. The lipid nanoparticle according to claim 45, wherein based on the total molar amount of components constituting the lipid nanoparticle, the lipid compound accounts for about 10 mol% to about 90 mol%, preferably about 10 mol% to about 70 mol%, and more preferably about 20 mol% to about 50 mol%.

47. The lipid nanoparticle according to claim 45 or 46, wherein the lipid nanoparticle further comprises a phospholipid, a structural lipid, and a PEG lipid.

48. The lipid nanoparticle according to claim 47, wherein based on the total molar amount of the components constituting the lipid nanoparticle, the phospholipid accounts for about 0 mol% to about 20 mol%.

49. The lipid nanoparticle according to claim 47 or 48, wherein based on the total molar amount of the components constituting the lipid nanoparticle, the structural lipid accounts for about 30 mol% to about 50 mol%.

50. The lipid nanoparticle according to any one of claims 47 to 49, wherein based on the total molar amount of the components constituting the lipid nanoparticle, the PEG lipid accounts for about 0 mol% to about 10 mol%.

51. The lipid nanoparticle according to any one of claims 47 to 50, wherein the lipid nanoparticle further comprises a plasmid or nucleic acid.

52. The lipid nanoparticle according to claim 51, wherein an N/P ratio of the lipid compound to the nucleic acid is about 1.1:1 to 10:1.

53. The lipid nanoparticle according to claim 51 or 52, wherein the nucleic acid is RNA.

54. Use of the liposome according to any one of claims 40 to 44 or the lipid nanoparticle according to any one of claims 45 to 50 as a delivery vehicle.

55. The use according to claim 54, wherein the delivery vehicle is used for delivering a plasmid or nucleic acid.

56. A pharmaceutical composition, comprising the lipid compound according to any one of claims 1 to 37, the liposome according to any one of claims 40 to 44, or the lipid nanoparticle according to any one of claims 45 to 50, and a pharmaceutically acceptable carrier or excipients.

57. A lipid compound of formula (II), or a stereoisomer or pharmaceutically acceptable salt thereof, 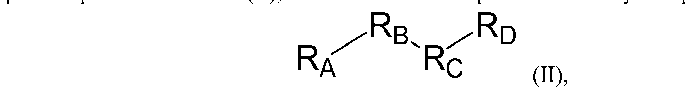
wherein R_{A} is selected from a substituted or unsubstituted aliphatic and heteroaliphatic group, and R_{A} is optionally substituted with one or more substituents selected from the following group consisting of deuterium, halogen, -NO₂, and -OH;
R_{B} is a degradable group comprising a degradable bond selected from the following group: 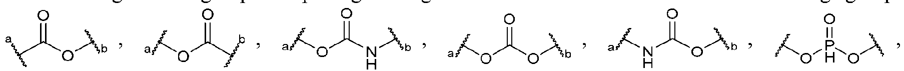 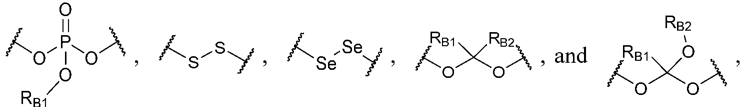 wherein a represents a position linked to R_{A}; b represents a position linked to R_{C}; and R_{B1} and R_{B2} are each independently selected from H and substituted or unsubstituted C₁₋₆ alkyl, and the alkyl is optionally substituted with one or more substituents selected from the following group: deuterium, halogen, -NO₂, and -OH;
R_{C} is selected from a substituted or unsubstituted aliphatic and heteroaliphatic group, and R_{C} is optionally substituted with one or more substituents selected from the following group: deuterium, halogen, -NO₂, and -OH; and
R_{D} is selected from -NC or -COOH.

58. The lipid compound of formula (II), or the stereoisomer or pharmaceutically acceptable salt thereof according to claim 57, wherein
(i) R_{A} is selected from a substituted or unsubstituted linear aliphatic group having 6 to 20 carbon atoms and a substituted or unsubstituted linear heteroaliphatic group having 6 to 20 carbon atoms, and the linear aliphatic group and the linear heteroaliphatic group are optionally substituted with one or more substituents selected from the following group consisting of deuterium, halogen, -NO₂, and -OH; or
(ii) R_{A} is selected from a substituted or unsubstituted branched aliphatic or substituted or unsubstituted branched heteroaliphatic group having formula (II_{A}): 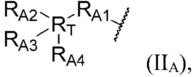 wherein
R_{A1} is absent or is selected from substituted or unsubstituted C₁₋₄ alkyl, and the alkyl is optionally substituted with one or more substituents selected from the following group: deuterium, halogen, -NO₂, and -OH;
a branching center group R_{T} is selected from the group consisting of: 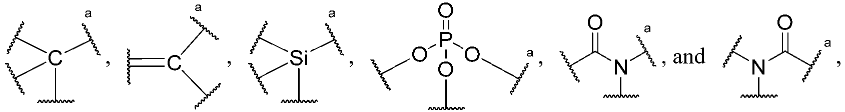
wherein a represents a position linked to R_{A1}, or a position directly linked to R_{B} when R_{A1} is absent;
R_{A2} and R_{A3} are each independently selected from a substituted or unsubstituted aliphatic and substituted or unsubstituted heteroaliphatic group having 1 to 12 carbon atoms, and the aliphatic group and the heteroaliphatic group are optionally substituted with one or more substituents selected from the following group: deuterium, halogen, -NO₂, and -OH;
R_{A4} is absent or is selected from hydrogen or a substituted or unsubstituted aliphatic and substituted or unsubstituted heteroaliphatic group having 1 to 12 carbon atoms, and the aliphatic group and the heteroaliphatic group are optionally substituted with one or more substituents selected from the following group consisting of deuterium, halogen, -NO₂, and -OH; and
a sum of a carbon atom number n_{A2} of R_{A2}, a carbon atom number n_{A3} of R_{A3} and a carbon atom number n_{A4} of R_{A4} is in a range of 2 to 24.

59. The lipid compound of formula (II), or the stereoisomer or pharmaceutically acceptable salt thereof according to claim 57 or 58, wherein
(i) R_{C} is selected from a substituted or unsubstituted linear aliphatic group having 2 to 18 carbon atoms and a substituted or unsubstituted linear heteroaliphatic group having 2 to 18 carbon atoms, wherein the linear aliphatic group and the linear heteroaliphatic group are optionally substituted with one or more substituents selected from the following group: deuterium, halogen, -NO₂, and -OH; and R_{D} is linked to a terminal of the linear aliphatic group or linear heteroaliphatic group; or
(ii) R_{C} is selected from a substituted or unsubstituted branched aliphatic or substituted or unsubstituted branched heteroaliphatic group having formula (II_{C}): 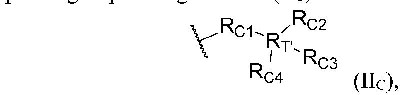 wherein
R_{C1} is absent or is selected from substituted or unsubstituted C₁₋₄ alkyl, and the alkyl is optionally substituted with one or more substituents selected from the following group: deuterium, halogen, -NO₂, and -OH;
a branching center group R_{T'} is selected from the group consisting of: 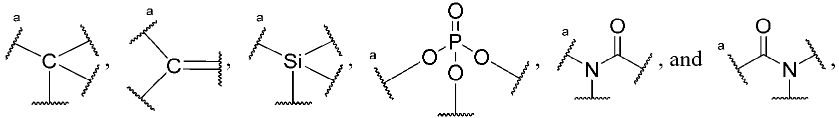
wherein a represents a position linked to R_{C1}, or a position directly linked to R_{B} when R_{C1} is absent;
R_{C2} and R_{C3} are each independently selected from a substituted or unsubstituted aliphatic and substituted or unsubstituted heteroaliphatic group having 1 to 12 carbon atoms, and the aliphatic group and the heteroaliphatic group are optionally substituted with one or more substituents selected from the following group consisting of deuterium, halogen, -NO₂, and -OH;
R_{C4} is absent or is selected from hydrogen or a substituted or unsubstituted aliphatic and substituted or unsubstituted heteroaliphatic group having 1 to 12 carbon atoms, and the aliphatic group and the heteroaliphatic group are optionally substituted with one or more substituents selected from the following group consisting of deuterium, halogen, -NO₂, and -OH;
a sum of a carbon atom number n_{C2} of R_{C2}, a carbon atom number n_{C3} of R_{C3} and a carbon atom number n_{C4} of R_{C4} is in a range of 2 to 24; and;
R_{D} is linked to a terminal of any one and only one group in R_{C2}, R_{C3}, and R_{C4}.

60. The lipid compound of formula (II), or the stereoisomer or pharmaceutically acceptable salt thereof according to any one of claims 57 to 59, wherein
R_{A} is selected from a substituted or unsubstituted linear aliphatic group having 6 to 20 carbon atoms, and the linear aliphatic group is optionally substituted with one or more substituents selected from the following group consisting of deuterium, halogen, -NO₂, and -OH; or
R_{A} is selected from a substituted or unsubstituted branched aliphatic or substituted or unsubstituted branched heteroaliphatic group having formula (II_{A'}):
wherein 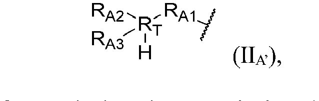
R_{A1} is absent or is selected from substituted or unsubstituted C₁₋₂ alkyl, and the alkyl is optionally substituted with one or more substituents selected from the following group consisting of deuterium, halogen, -NO₂, and -OH;
the branching center group R_{T} is 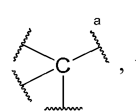 wherein a represents a position linked to R_{A1}, or a position directly linked to R_{B} when R_{A1} is absent;
R_{A2} and R_{A3} are each independently selected from a substituted or unsubstituted aliphatic group having 3 to 12 carbon atoms, and the aliphatic group is optionally substituted with one or more substituents selected from the following group consisting of deuterium, halogen, -NO₂, and -OH;
a sum of a carbon atom number n_{A2} of R_{A2} and a carbon atom number n_{A3} of R_{A3} is in a range of 6 to 18; R_{B} is a degradable group comprising a degradable bond selected from the following group consisting of 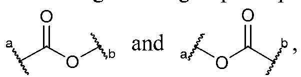
wherein a represents a position linked to R_{A}, and b represents a position linked to R_{C};
Rc is selected from a substituted or unsubstituted linear aliphatic group having 2 to 18 carbon atoms, and the linear aliphatic group is optionally substituted with one or more substituents selected from the following group: deuterium, halogen, -NO₂, and -OH; and
R_{D} is selected from -NC or -COOH.

61. The lipid compound of formula (II), or the stereoisomer or pharmaceutically acceptable salt thereof according to any one of claims 57 to 60, wherein the lipid compound is selected from lipid compounds as shown in the following structures:

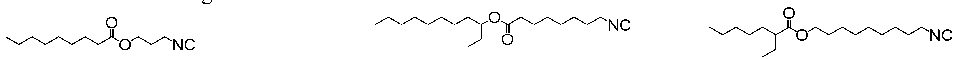

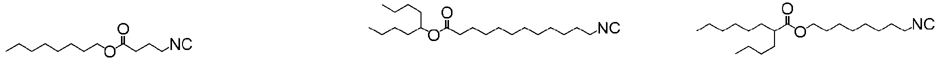

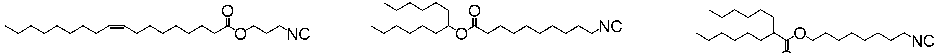

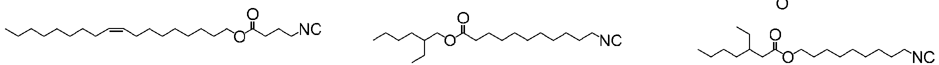

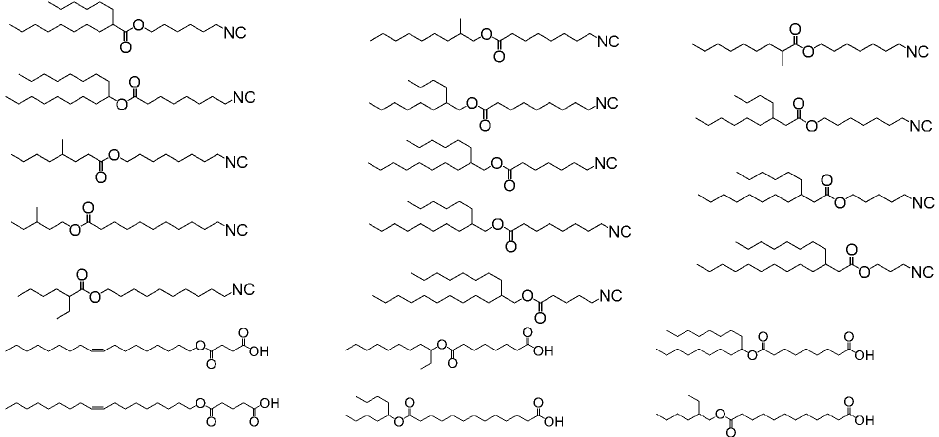

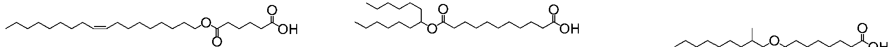

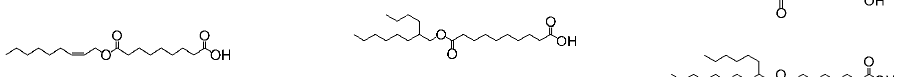

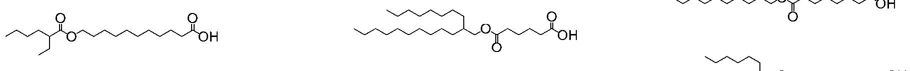

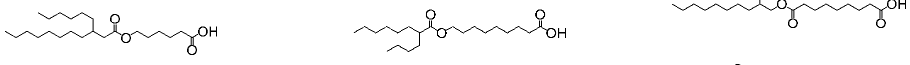

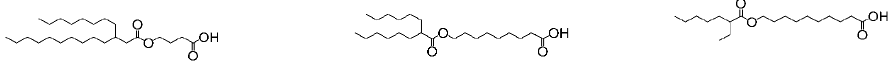

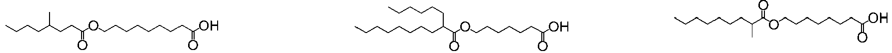

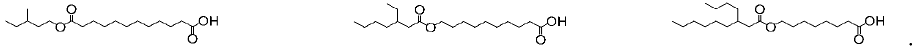

62. A preparation method of the lipid compound of formula (II), or the stereoisomer or pharmaceutically acceptable salt thereof according to claim 60 or 61, wherein the preparation method comprises the following syntehtis route: when Rₚis selected from -NC, 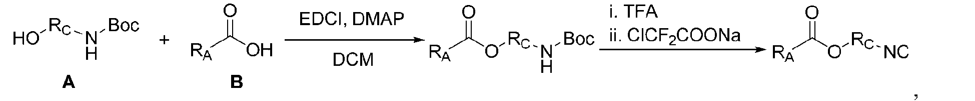 or 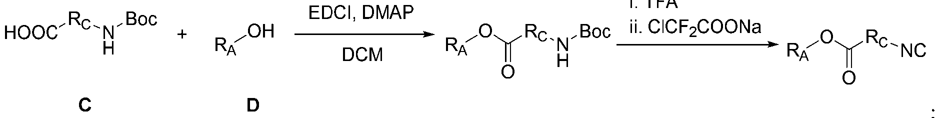 or 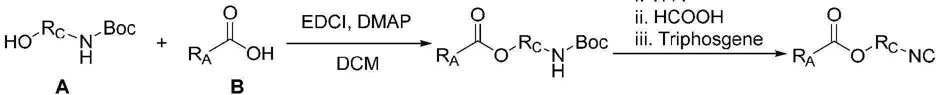 or 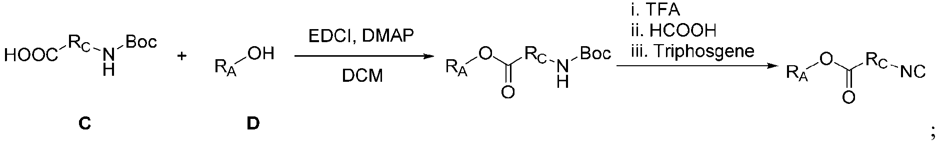 or 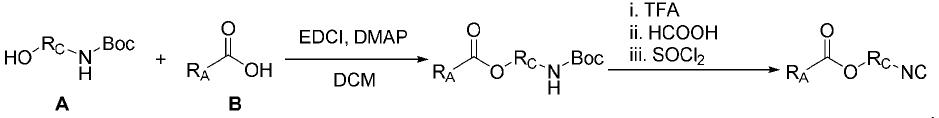 or 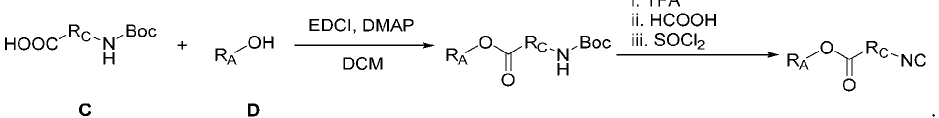 or 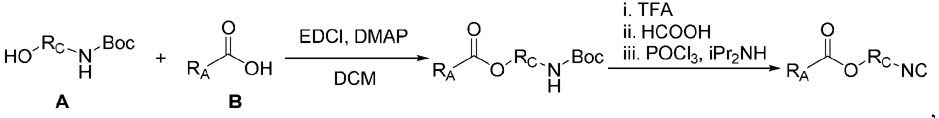 or 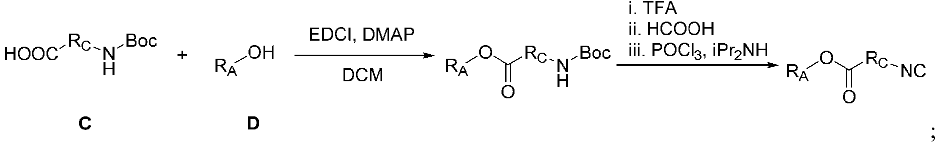 or 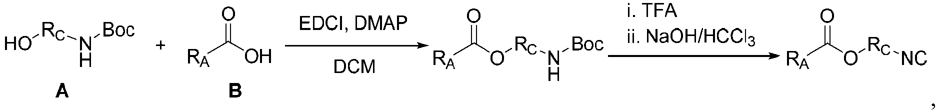 or 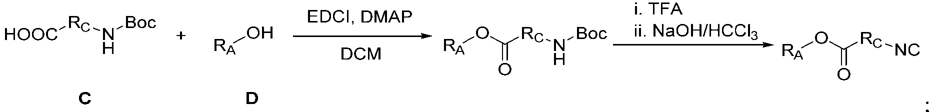 or 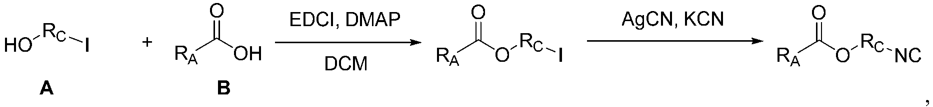 or 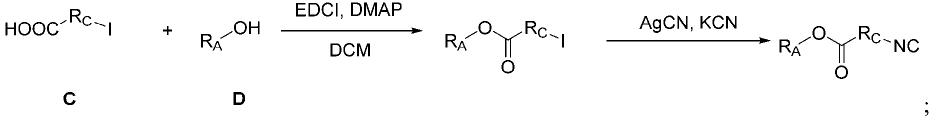 and
when R_{D} is selected from -COOH, 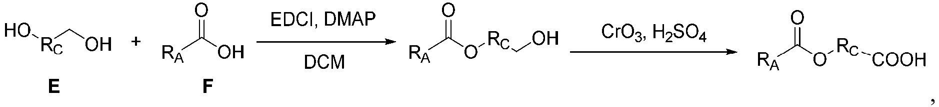 or 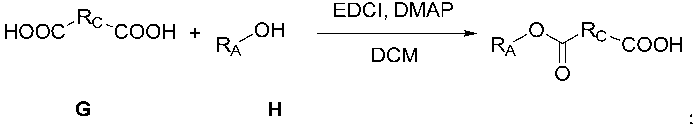 or 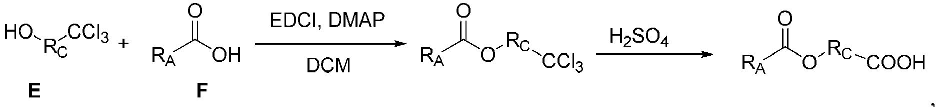 or 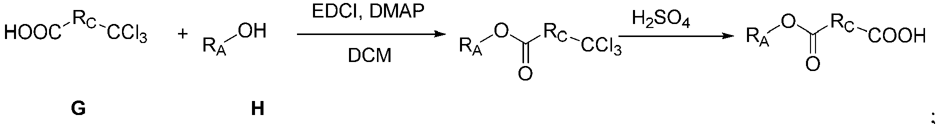 or 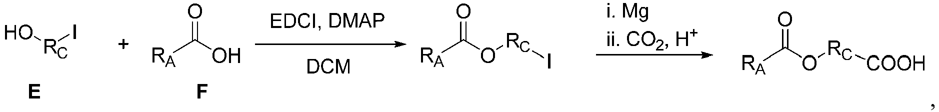 or 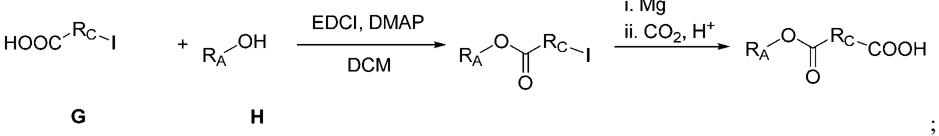 or

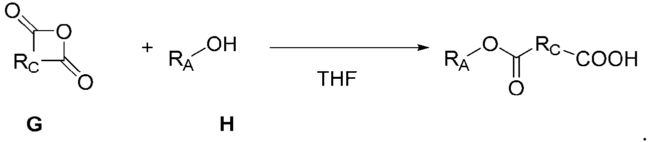

63. Use of the lipid compound of formula (II), or the stereoisomer or pharmaceutically acceptable salt thereof according to any one of claims 57 to 61 in preparation of an ionizable lipid.

64. The use according to claim 63, wherein the ionizable lipid is the lipid compound of formula (I) according to any one of claims 1 to 37.

65. The use according to claim 64, wherein the preparation comprises the following Ugi reaction synthesis route: 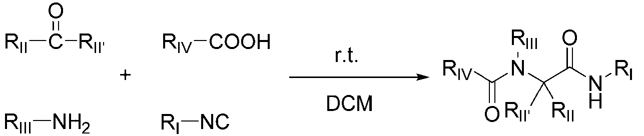
wherein R_{I}, R_{II}, R_{II}', R_{III}, and R_{IV} are as defined in any one of claims 1 to 37; and
R_{I}-NC and R_{IV}-COOH are each independently selected from the lipid compound of formula (II) according to any one of claims 57 to 61.

66. The use according to claim 63, wherein the ionizable lipid is a lipid compound of formula (III): 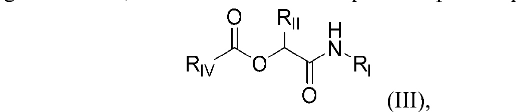 wherein R_{I}, R_{II}, and R_{IV} are each independently selected from a group (A) comprising a group comprising at least one ionizable tertiary amine structure, and a group (B) comprising a substituted or unsubstituted aliphatic or substituted or unsubstituted heteroaliphatic group having 11 to 30 carbon atoms, provided that: only one of R_{I}, R_{II}, and R_{IV} is a group comprising at least one ionizable tertiary amine structure.

67. The use according to claim 66, wherein the preparation comprises the following Passerini reaction synthesis route: 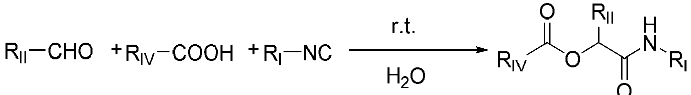 wherein R_{I}-NC and R_{IV}-COOH are each independently selected from the lipid compound of formula (II) according to any one of claims 57 to 61.

68. The use according to claim 66, wherein the preparation comprises the following reaction synthesis route: 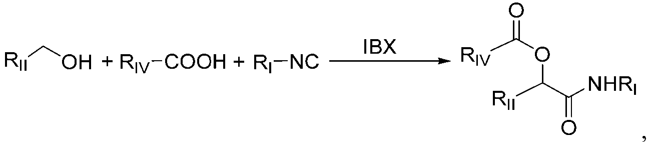 wherein R_{I}-NC and R_{IV}-COOH are each independently selected from the lipid compound of formula (II) according to any one of claims 57 to 61.

69. The use according to claim 63, wherein the ionizable lipid is a lipid compound of formula (IV): 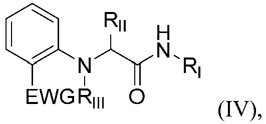 wherein R_{I}, R_{II}, and R_{III} are each independently selected from a group (A) comprising a group comprising at least one ionizable tertiary amine structure, and a group (B) comprising a substituted or unsubstituted aliphatic or substituted or unsubstituted heteroaliphatic group having 11 to 30 carbon atoms, provided that: only one of R_{I}, R_{II}, and R_{III} is a group comprising at least one ionizable tertiary amine structure.

70. The use according to claim 69, wherein the preparation comprises the following Ugi-Smiles reaction synthesis route: 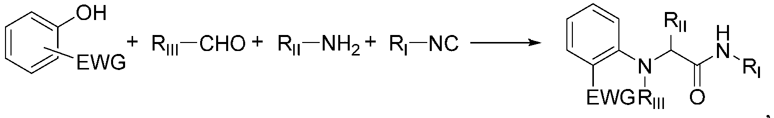 wherein R_{I}-NC is selected from the lipid compound of formula (II) according to any one of claims 57 to 61.

71. The use according to claim 63, wherein the ionizable lipid is a lipid compound of formula (V): 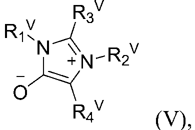 wherein R₁^{V}, R₂^{V}, R₃^{V}, and R₄^{V} are each independently selected from a group (A) comprising a group comprising at least one ionizable tertiary amine structure, a group (B) comprising a substituted or unsubstituted aliphatic or substituted or unsubstituted heteroaliphatic group having 11 to 30 carbon atoms, and a group (C) comprising a substituted or unsubstituted hydrocarbyl, substituted or unsubstituted heterohydrocarbyl, substituted or unsubstituted aryl, or substituted or unsubstituted heteroaryl group having fewer than 11 carbon atoms, provided that: only one of R₁^{V}, R₂^{V}, R₃^{V}, and R₄^{V} is the group comprising at least one ionizable tertiary amine structure.

72. The use according to claim 71, wherein the preparation comprises the following reaction synthesis route: 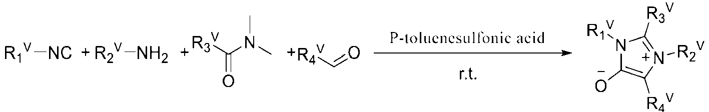 wherein R₁^{V}-NC is selected from the lipid compound of formula (II) according to any one of claims 57 to 61.

73. The use according to claim 63, wherein the ionizable lipid is a lipid compound of formula (VI): 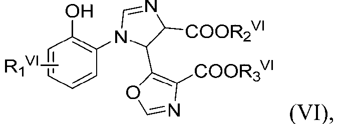 wherein R₁^{VI}, R₂^{VI}, and R₃^{VI} are each independently selected from a group (A) comprising a group comprising at least one ionizable tertiary amine structure, a group (B) comprising a substituted or unsubstituted aliphatic or substituted or unsubstituted heteroaliphatic group having 11-30 carbon atoms, and a group (C) comprising a substituted or unsubstituted hydrocarbyl, substituted or unsubstituted heterohydrocarbyl, substituted or unsubstituted aryl, or substituted or unsubstituted heteroaryl group having fewer than 11 carbon atoms, provided that: only one of R₁^{VI}, R₂^{VI}, and R₃^{VI} is the group comprising at least one ionizable tertiary amine structure.

74. The use according to claim 73, wherein the preparation comprises the following synthetic reaction route: 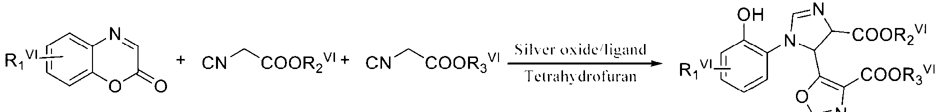 wherein 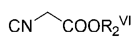 and 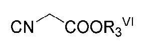 are each independently selected from the lipid compound of formula (II) according to any one of claims 57 to 61.

75. A lipid compound of formula (III), (IV), (V), or (VI), or the N-oxide, stereoisomer, or pharmaceutically acceptable salt thereof, for use in nucleic acid delivery.

76. Use of a lipid compound of formula (III), (IV), (V), or (VI), or an N-oxide, stereoisomer, or a pharmaceutically acceptable salt thereof in preparation of a liposome and/or a lipid nanoparticle.

77. A liposome, comprising a lipid compound of formula (III), (IV), (V), or (VI).

78. The liposome according to claim 77, wherein the liposome further comprises a phospholipid and cholesterol.

79. A lipid nanoparticle, comprising the lipid compound of formula (III), (IV), (V), or (VI).

80. The lipid nanoparticle according to claim 79, wherein the lipid nanoparticle further comprises a phospholipid, a structural lipid, and a PEG lipid.

81. The lipid nanoparticle according to claim 80, wherein the lipid nanoparticle further comprises a plasmid or nucleic acid.

82. Use of the liposome according to claim 77 or 78 or the lipid nanoparticle according to any one of claims 79 to 81 as a delivery vehicle.

83. The use according to claim 82, wherein the delivery vehicle is used for delivering a plasmid or nucleic acid.

84. A pharmaceutical composition, comprising the lipid compound of formula (III), (IV), (V), or (VI), the liposome according to claim 77 or 78, or the lipid nanoparticle according to any one of claims 79 to 81, and a pharmaceutically acceptable carrier or excipients.
